# EUROPÄISCHE PATENTANMELDUNG

(11) **EP 4 756 860 A2**
(43) Veröffentlichungstag der Anmeldung: **10.06.2026**
(21) Anmeldenummer: 26174440.3
(22) Anmeldetag: 15.12.2021
(51) Int. Cl.: H01J 35/24

(54) **INDOLO[3.2.1-JK]CARBAZOLE-6-CARBONITRIL-DERIVATE ALS BLAU FLUORESZIERENDE EMITTER ZUR VERWENDUNG IN OLEDS**

(30) Priorität: 18.12.2020 EP 20215734
(62) Teilanmeldung aus: 21820651.4
(71) Anmelder: UDC Ireland Limited, Ballycoolin, Dublin 15 (IE)
(72) Erfinder: STOESSEL, Philipp, 64293 Darmstadt (DE)
(74) Vertreter: Maiwald GmbH

(57) **Zusammenfassung**

Die vorliegende Erfindung betrifft aromatische Verbindungen, die sich für die Verwendung in elektronischen Vorrichtungen eignen, sowie elektronische Vorrichtungen, insbesondere organischen Elektrolumineszenzvorrichtungen, enthaltend diese Verbindungen.

## Beschreibung

Die vorliegende Erfindung betrifft aromatische Verbindungen für die Verwendung in elektronischen Vorrichtungen, insbesondere in organischen Elektrolumineszenzvorrichtungen, sowie elektronische Vorrichtungen, insbesondere organische Elektrolumineszenzvorrichtungen, enthaltend diese aromatische Verbindungen.

In organischen Elektrolumineszenzvorrichtungen werden als emittierende Materialien häufig phosphoreszierende metallorganische Komplexe oder fluoreszierende Verbindungen eingesetzt. Generell gibt es bei Elektrolumineszenzvorrichtungen immer noch Verbesserungsbedarf.

Aus US 2010/0051928, WO 2010/104047 A1, US 2014/319507 A1, WO 2017/175690, US 2019/0393439, CN 110452226 A, WO 2019/132506 A1 und WO 2020/064666 A1 sind polycyclische Verbindungen bekannt, die in organischen Elektrolumineszenzvorrichtungen eingesetzt werden können.

Generell besteht bei diesen heterocyclischen Verbindungen, beispielsweise für die Verwendung als Emitter, insbesondere als fluoreszierender Emitter, noch Verbesserungsbedarf, insbesondere in Bezug auf die Lebensdauer, die Farbreinheit, aber auch in Bezug auf die Effizienz und die Betriebsspannung der Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung eignen, und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung Verbindungen zur Verfügung zu stellen, die zu hoher Lebensdauer, guter Effizienz und geringer Betriebsspannung führen.

Weiterhin sollten die Verbindungen eine ausgezeichnete Verarbeitbarkeit aufweisen, wobei die Verbindungen insbesondere eine gute Löslichkeit zeigen sollten.

Eine weitere Aufgabe der vorliegenden Erfindung kann darin gesehen werden, Verbindungen bereitzustellen, welche sich für den Einsatz in einer phosphoreszierenden oder fluoreszierenden Elektrolumineszenzvorrichtungen eignen, insbesondere als Emitter. Insbesondere ist es eine Aufgabe der vorliegenden Erfindung, Emitter bereitzustellen, welche sich für rote, grüne oder blaue Elektrolumineszenzvorrichtungen, vorzugweise für blaue Elektrolumineszenzvorrichtungen eignen.

Weiterhin sollten die Verbindungen, insbesondere bei ihrem Einsatz als Emitter in organischen Elektrolumineszenzvorrichtungen, zu Vorrichtungen führen, die eine ausgezeichnete Farbreinheit aufweisen.

Eine weitere Aufgabe kann darin gesehen werden, elektronische Vorrichtungen mit einer ausgezeichneten Leistungsfähigkeit möglichst kostengünstig und in konstanter Qualität bereitzustellen

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, unten näher beschriebene Verbindungen diese Aufgabe lösen, sich sehr gut für die Verwendung in Elektrolumineszenzvorrichtungen eignen und zu organischen Elektrolumineszenzvorrichtungen führen, die insbesondere in Bezug auf die Lebensdauer, der Farbreinheit, der Effizienz und der Betriebsspannung sehr gute Eigenschaften vorweisen. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine Verbindung umfassend mindestens eine Struktur der Formel (I), vorzugsweise eine Verbindung gemäß der Formel (I), 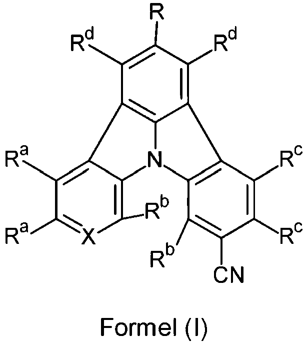 wobei für die verwendeten Symbole gilt:
- X: steht bei jedem Auftreten gleich oder verschieden für N, C-CN, C-Y-R^{y} oder CR^{b}, vorzugsweise für N, C-CN, oder C-Y-R^{y}, besonders bevorzugt für C-CN,
- Y: ist bei jedem Auftreten gleich oder verschieden CO, P(=O)R^{a}, SO, SO₂, C(O)O, C(S)O, C(O)S, C(=O)NR^{a}, C(=O)NAr, vorzugsweise CO, P(=O)R^{a}, SO, SO₂, besonders bevorzugt CO;
- R: ist bei jedem Auftreten gleich oder verschieden H, D, OH, F, Cl, Br, I, CN, NO₂, N(Ar)₂, N(R^{e})₂, C(=O)N(Ar)₂, C(=O)N(R^{e})₂, C(Ar)₃, C(R^{e})₃, Si(Ar)₃, Si(R^{e})₃, B(Ar)₂, B(R^{e})₂, C(=O)Ar, C(=O)R^{e}, P(=O)(Ar)₂, P(=O)( R^{e})₂, P(Ar)₂, P(R^{e})₂, S(=O)Ar, S(=O)R^{e}, S(=O)₂Ar, S(=O)₂R^{e}, OSO₂Ar, OSO₂R^{e}, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R^{e} substituiert sein kann, wobei eine oder mehrere nicht benachbarte CH₂-Gruppen durch R^{e}C=CR^{e}, C≡C, Si(R^{e})2, C=O, C=S, C=Se, C=NR^{e}, -C(=O)O-, -C(=O)NR^{e}-, NR^{e}, P(=O)( R^{e}), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R^{e} substituiert sein kann, oder eine Aryloxy- oder Heteroaryloxygruppe mit 5 bis 60 aromatischen Ringatomen, die durch einen oder mehrere Reste R^{e} substituiert sein kann, oder eine Arylthio- oder Heteroarylthiogruppe mit 5 bis 60 aromatischen Ringatomen, die durch einen oder mehrere Reste R^{e} substituiert sein kann, oder eine Diarylamino-, Arylheteroarylamino-, Diheteroarylaminogruppe mit 5 bis 60 aromatischen Ringatomen, die durch einen oder mehrere Reste R^{e} substituiert sein kann, oder eine Arylalkyl- oder Heteroarylalkylgruppe mit 5 bis 60 aromatischen Ringatomen und 1 bis 10 C-Atomen im Alkylrest, die durch einen oder mehrere Reste R^{e} substituiert sein kann; dabei kann ein Rest R mit einer weiteren Gruppe, vorzugsweise R^{d} ein Ringsystem bilden;
- Ar: ist bei jedem Auftreten gleich oder verschieden ein aromatisches oder heteroaromatisches Ringsystem mit 5 bis 60 aromatischen Ringatomen, das mit einem oder mehreren Resten R^{e} substituiert sein kann, dabei können zwei Reste Ar, welche an dasselbe C-Atom, Si-Atom, N-Atom, P-Atom oder B-Atom binden, auch durch eine Einfachbindung oder eine Brücke, ausgewählt aus B(R^{e}), C(R^{e})₂, Si(R^{e})2, C=O, C=NR^{e}, C=C(R^{e})₂, O, S, S=O, SO₂, N(R^{e}), P(R^{e}) und P(=O)R^{a}, miteinander verbrückt sein;
- R^{a}, R^{b}, R^{c}, R^{d}, R^{e}: ist bei jedem Auftreten gleich oder verschieden H, D, OH, F, Cl, Br, I, CN, NO₂, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R^{e})₂, S(=O)Ar', S(=O)R¹, S(=O)₂Ar', S(=O)₂R¹, OSO₂Ar', OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C- Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR', P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; dabei können zwei Reste R^{a}, R^{b}, R^{c}, R^{d}, R^{e} auch miteinander oder einer weiteren Gruppe, vorzugsweise R oder R^{y} ein Ringsystem bilden;
- R^{y}: ist bei jedem Auftreten gleich oder verschieden C(Ar')₃, C(R¹)₃, Si(Ar')₃, Si(R¹)₃, N(Ar')₂, N(R¹)₂, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, wobei eine mit dem Rest Y verbundene CH₂-Gruppe nicht durch C=O, C=S, C=Se, C=NR¹, -C(=O)O-, -C(=O)NR¹-, P(=O)(R¹), SO oder SO₂ ersetzt sein kann, wobei eine mit dem Rest Y verbundene CH₂-Gruppe besonders bevorzugt nicht durch Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein kann, oder ein aromatisches oder heteroaromatisches Ringsystem mit 5 bis 60 aromatischen Ringatomen, das jeweils durch einen oder mehrere Reste R¹ substituiert sein kann; dabei können zwei Reste R^{y} auch miteinander oder ein Rest R^{y} mit einem Rest R^{a}, R^{b}, R^{c}, R^{d}, R^{e} ein Ringsystem bilden, vorzugsweise einem Rest R^{a};
- Ar': ist bei jedem Auftreten gleich oder verschieden ein aromatisches oder heteroaromatisches Ringsystem mit 5 bis 60 aromatischen Ringatomen, das mit einem oder mehreren Resten R¹ substituiert sein kann, dabei können zwei Reste Ar', welche an dasselbe C-Atom, Si-Atom, N-Atom, P-Atom oder B-Atom binden, auch durch eine Einfachbindung oder eine Brücke, ausgewählt aus B(R¹), C(R¹)2, Si(R¹)₂, C=O, C=NR¹, C=C(R¹)₂, O, S, S=O, SO₂, N(R¹), P(R¹) und P(=O)R¹, miteinander verbrückt sein;
- R¹: ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch -R²C=CR²-, -C=C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei oder mehrere, vorzugsweise benachbarte Reste R¹ miteinander ein Ringsystem bilden, dabei können einer oder mehrere Reste R¹ mit einem weiteren Teil der Verbindung ein Ringsystem bilden;
- Ar": ist bei jedem Auftreten gleich oder verschieden ein aromatisches oder heteroaromatisches Ringsystem mit 5 bis 30 aromatischen Ringatomen, das mit einem oder mehreren Resten R² substituiert sein kann, dabei können zwei Reste Ar", welche an dasselbe C-Atom, Si-Atom, N-Atom, P-Atom oder B-Atom binden, auch durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verbrückt sein;
- R²: ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann, dabei können zwei oder mehrere, vorzugsweise benachbarte Substituenten R² miteinander ein Ringsystem bilden.

Vorzugsweise kann vorgesehen sein, dass mindestens ein vorzugsweise mindestens zwei der Reste R, R^{a}, R^{b}, R^{c}, R^{d}, R^{e} ungleich H sind, vorzugsweise ungleich H, D, OH, NO₂, F, Cl, Br, I. Demgemäß ist R vorzugsweise ausgewählt aus CN, N(Ar)₂, N(R^{e})₂, C(=O)N(Ar)₂, C(=O)N(R^{e})₂, C(Ar)₃, C(R^{e})₃, Si(Ar)₃, Si(R^{e})₃, B(Ar)₂, B(R^{e})₂, C(=O)Ar, C(=O)R^{e}, P(=O)(Ar)2, P(=O)( R^{e})₂, P(Ar)2, P(R^{e})₂, S(=O)Ar, S(=O)R^{e}, S(=O)₂Ar, S(=O)₂R^{e}, OSO₂Ar, OSO₂R^{e}, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R^{e} substituiert sein kann, wobei eine oder mehrere nicht benachbarte CH₂-Gruppen durch R^{e}C=CR^{e}, C=C, Si(R^{e})2, C=O, C=S, C=Se, C=NR^{e}, -C(=O)O-, -C(=O)NR^{e}-, NR^{e}, P(=O)( R^{e}), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R^{e} substituiert sein kann, oder eine Aryloxy- oder Heteroaryloxygruppe mit 5 bis 60 aromatischen Ringatomen, die durch einen oder mehrere Reste R^{e} substituiert sein kann, oder eine Arylthio- oder Heteroarylthiogruppe mit 5 bis 60 aromatischen Ringatomen, die durch einen oder mehrere Reste R^{e} substituiert sein kann, oder eine Diarylamino-, Arylheteroarylamino-, Diheteroarylaminogruppe mit 5 bis 60 aromatischen Ringatomen, die durch einen oder mehrere Reste R^{e} substituiert sein kann, oder eine Arylalkyl- oder Heteroarylalkylgruppe mit 5 bis 60 aromatischen Ringatomen und 1 bis 10 C-Atomen im Alkylrest, die durch einen oder mehrere Reste R^{e} substituiert sein kann; dabei kann ein Rest R mit einer weiteren Gruppe, vorzugsweise R^{d} ein Ringsystem bilden; und/oder mindestens einer der Reste R^{a}, R^{b}, R^{c}, R^{d}, R^{e} ist vorzugsweise bei jedem Auftreten gleich oder verschieden ausgewählt aus CN, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R^{e})₂, S(=O)Ar', S(=O)R¹, S(=O)₂Ar', S(=O)₂R¹, OSO₂Ar', OSO₂R', eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; dabei können zwei Reste R^{a}, R^{b}, R^{c}, R^{d}, R^{e} auch miteinander oder einer weiteren Gruppe ein Ringsystem bilden.

Vorzugsweise kann vorgesehen sein, dass mindestens einer der Reste R^{a}, bevorzugt beide Reste R^{a} ungleich H ist/sind, wobei besonders bevorzugt mindestens einer der Reste R^{a}, bevorzugt beide Reste R^{a} ungleich H, D, F, Cl, Br, I. Hierbei sind die zuvor dargelegten Ausführungen bezüglich bevorzugter Reste R^{a} zu berücksichtigen.

Bevorzugt kann weiterhin vorgesehen sein, dass mindestens einer der Reste R^{c}, bevorzugt beide Reste R^{c} ungleich H ist/sind, wobei besonders bevorzugt mindestens einer der Reste R^{c}, bevorzugt beide Reste R^{c} ungleich H, D, F, Cl, Br, I. Hierbei sind die zuvor dargelegten Ausführungen bezüglich bevorzugter Reste R^{c} zu berücksichtigen.

Besonders bevorzugt kann weiterhin vorgesehen sein, dass mindestens einer der Reste R^{a} und mindestens einer der Reste R^{c} ungleich H ist, vorzugsweise ungleich H, D, F, Cl, Br, I. Speziell bevorzugt sind beide Reste R^{a} und beide Reste R^{c} ungleich H, vorzugsweise ungleich H, D, F, Cl, Br, I Hierbei sind die zuvor dargelegten Ausführungen bezüglich bevorzugter Reste R^{a} und R^{c} zu berücksichtigen.

Bevorzugt kann des Weiteren vorgesehen sein, dass mindestens einer vorzugsweise mindestens zwei der Reste R^{a}, R^{c} eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, darstellen, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können.

In einer bevorzugten Ausgestaltung kann vorgesehen sein, dass der Rest R ein aromatisches oder heteroaromatisches Ringsystem mit 5 bis 13 aromatischen Ringatomen darstellt, das mit einem oder mehreren Resten R^{e} substituiert sein kann.

In einer weiterhin bevorzugten Ausgestaltung kann vorgesehen sein, dass zwei Reste R^{a} mit den weiteren Gruppen, an die die zwei Reste R^{a} binden, einen kondensierten Ring bilden, vorzugsweise einen aliphatischen oder heteroaliphatischen Ring mit 3 bis 20, vorzugsweise 5 bis 18 Ringatomen oder einen aromatischen oder heteroaromatischen Ring mit 5 bis 13 Ringatomen, besonders bevorzugt einen aliphatischen oder heteroaliphatischen Ring mit 3 bis 20, vorzugsweise 5 bis 18 Ringatomen, der jeweils mit einem oder mehreren Resten R¹ substituiert sein kann, wobei R¹ die zuvor genannte Bedeutung aufweist.

Ferner kann bevorzugt vorgesehen sein, dass zwei Reste R^{c} mit den weiteren Gruppen, an die die zwei Reste R^{c} binden, einen kondensierten Ring bilden, vorzugsweise einen aliphatischen oder heteroaliphatischen Ring mit 3 bis 20, vorzugsweise 5 bis 18 Ringatomen oder einen aromatischen oder heteroaromatischen Ring mit 5 bis 13 Ringatomen, besonders bevorzugt einen aliphatischen oder heteroaliphatischen Ring mit 3 bis 20, vorzugsweise 5 bis 18 Ringatomen, der jeweils mit einem oder mehreren Resten R¹ substituiert sein kann, wobei R¹ die zuvor genannte Bedeutung aufweist.

In einer speziell bevorzugten Ausführungsform umfasst der Rest R ein aromatisches oder heteroaromatisches Ringsystem mit 5 bis 13 aromatischen Ringatomen, das mit einem oder mehreren Resten R^{e} substituiert sein kann, und mindestens zwei Reste R^{a}, R^{c} bilden mit den weiteren Gruppen, an die die zwei Reste R^{a}, R^{c} binden, einen kondensierten Ring, , der jeweils mit einem oder mehreren Resten R¹ substituiert sein kann. Vorzugweise umfasst eine erfindungsgemäße Verbindung/Struktur demgemäß mindestens einen, vorzugsweise zwei kondensierte Ringe, die durch die zwei Reste R^{a} und/oder R^{c} mit den weiteren Gruppen, an die die zwei Reste R^{a}, R^{c} binden, gebildet werden und der Rest R stellt ein aromatisches oder heteroaromatisches Ringsystem mit 5 bis 13 aromatischen Ringatomen dar. Kondensierte Ringe können hierbei aliphatisch, heteroaliphatisch, aromatisch oder heteroaromatisch sein, wobei bevorzugte Ausgestaltungen zuvor und nachfolgend dargelegt sind, wobei vorzugsweise ein aliphatischer oder heteroaliphatischer Ring mit 3 bis 20, vorzugsweise 5 bis 18 Ringatomen oder ein aromatischen oder heteroaromatischen Ring mit 5 bis 13 Ringatomen gebildet wird, besonders bevorzugt ein aliphatischen oder heteroaliphatischen Ring mit 3 bis 20, vorzugsweise 5 bis 18 Ringatomen, der jeweils mit einem oder mehreren Resten R¹ substituiert sein kann.

Falls X für eine Gruppe C-Y-R^{y} steht, kann bevorzugt vorgesehen sein, dass gilt:
- R^{y}: ist bei jedem Auftreten gleich oder verschieden C(Ar')₃, C(R¹)₃, Si(Ar')₃, Si(R¹)₃, eine geradkettige Alkylgruppe mit 1 bis 40 C-Atomen oder eine Alkenyl- oder Alkinylgruppe mit 2 bis 40 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, wobei eine mit dem Rest Y verbundene CH₂-Gruppe nicht durch die genannten Gruppen ersetzt sein kann, oder ein aromatisches oder heteroaromatisches Ringsystem mit 5 bis 60 aromatischen Ringatomen, das jeweils durch einen oder mehrere Reste R¹ substituiert sein kann, dabei können zwei Reste R^{y} auch miteinander oder ein Rest R^{y} mit einem Rest R^{a}, R^{b}, R^{c}, R^{d}, R^{e} ein Ringsystem bilden, vorzugsweise einem Rest R^{a}.

Weiterhin kann bevorzugt vorgesehen sein, dass der an die Gruppe Y gebundene Rest R^{y} und der zur Gruppe benachbarte Rest R^{a} mit den weiteren Gruppen, an die die zwei Reste R^{y}, R^{a} binden, einen kondensierten Ring bilden, vorzugsweise einen aliphatischen oder heteroaliphatischen Ring mit 3 bis 20, vorzugsweise 5 bis 18 Ringatomen oder einen aromatischen oder heteroaromatischen Ring mit 5 bis 13 Ringatomen, besonders bevorzugt einen aliphatischen oder heteroaliphatischen Ring mit 3 bis 20, vorzugsweise 5 bis 18 Ringatomen, der mit einem oder mehreren Resten R¹ substituiert sein kann, wobei R¹ die zuvor genannte Bedeutung aufweist. Ferner kann auch ein Rest R^{b} und ein Rest R^{y} mit den weiteren Gruppen, an die die zwei Reste R^{y}, R^{a} binden, einen kondensierten Ring bilden, wie dies zuvor für den Rest R^{a} dargelegt ist, wobei eine Ringbildung über einen Rest R^{a} bevorzugt ist.

Weiterhin kann besonders bevorzugt vorgesehen sein, dass der an die Gruppe Y gebundene Rest R^{y} in Nachbarschaft zur Gruppe Y kein azides Proton aufweist, vorzugsweise eine Keto-Enol-Tautomerie im Falle Y gleich C=O ausgeschlossen ist. Ein azides Proton in diesem Sinne ist ein Proton, welches einen hohen pks-Wert aufweist, wobei der pks-Wert eine Protons bevorzugt mindestens 21, vorzugsweise mindestens 22 und besonders bevorzugt mindestens 25 beträgt. Vorzugsweise bilden ein an die Gruppe Y gebundene Rest R^{y} und der zur Gruppe benachbarte Rest R^{a} oder R^{b} mit den weiteren Gruppen, an die die zwei Reste R^{y}, R^{a} binden, einen kondensierten, verbrückten Ring, der die Bredtsche Regel erfüllt, wobei das Brückenatom direkt an die Gruppe Y bindet.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Eine elektronenarme Heteroarylgruppe im Sinne der vorliegenden Erfindung ist eine Heteroarylgruppe, die mindestens einen heteroaromatischen Sechsring mit mindestens einem Stickstoffatom aufweist. An diesen Sechsring können noch weitere aromatische oder heteroaromatische Fünfringe oder Sechsringe ankondensiert sein. Beispiele für elektronenarme Heteroarylgruppen sind Pyridin, Pyrimidin, Pyrazin, Pyridazin, Triazin, Chinolin, Chinazolin oder Chinoxalin.

Ein aromatisches Ringsystem im Sinne dieser Erfindung enthält 6 bis 60 C-Atome im Ringsystem, vorzugsweise 6 bis 40 C-Atome im Ringsystem. Ein heteroaromatisches Ringsystem im Sinne dieser Erfindung enthält 2 bis 60 C-Atome, vorzugsweise 3 bis 40 C-Atome, und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugt ist das aromatische Ringsystem gewählt aus Fluoren, 9,9'-Spirobifluoren, 9,9-Diarylamin oder Gruppen, in denen zwei oder mehr Aryl- und/oder Heteroarylgruppen durch Einfachbindungen miteinander verknüpft sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 bzw. 5 bis 40 aromatischen Ringatomen, welches noch jeweils mit den oben genannten Resten substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombinationen dieser Systeme.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

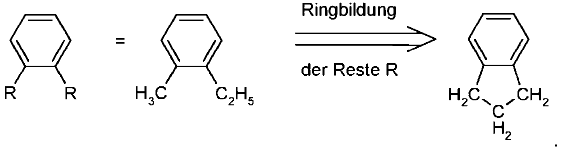

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

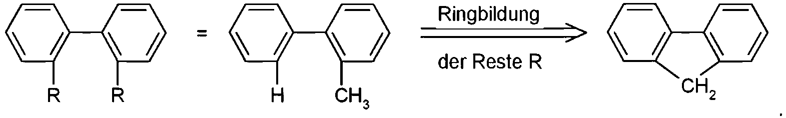

In einer bevorzugten Ausgestaltung können die erfindungsgemäßen Verbindungen eine Struktur der Formeln (I-1) bis (1-81) umfassen, besonders bevorzugt können die erfindungsgemäßen Verbindungen ausgewählt sein aus den Verbindungen der Formeln (I-1) bis (I-83), 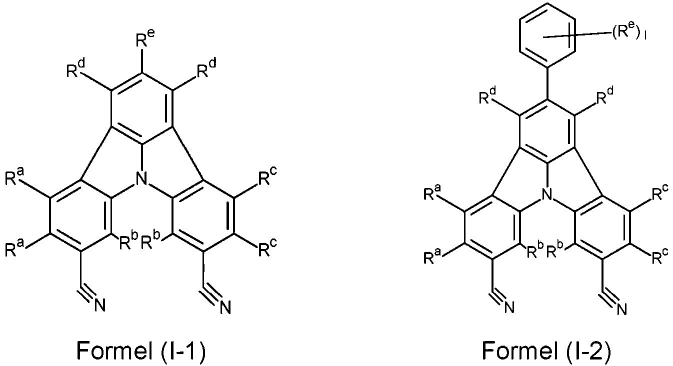 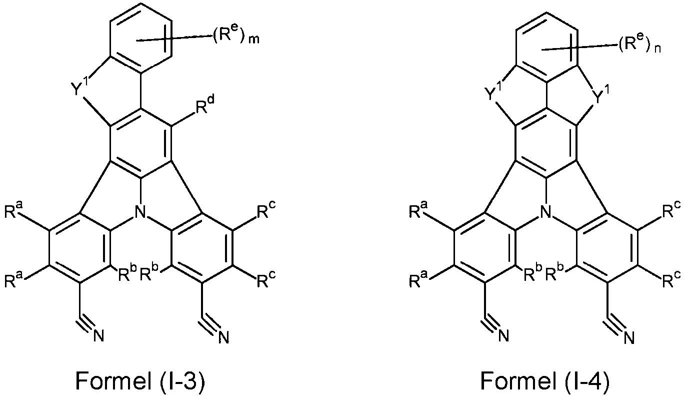 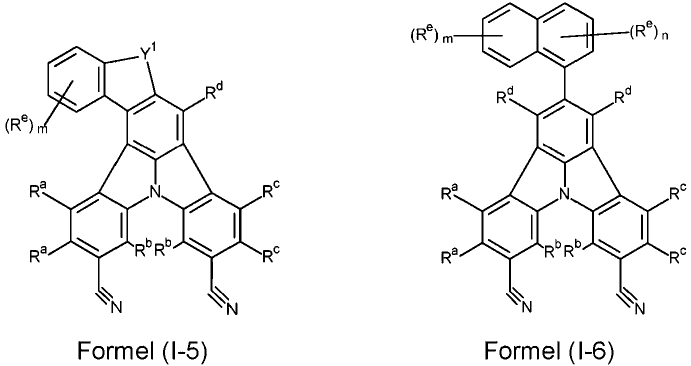 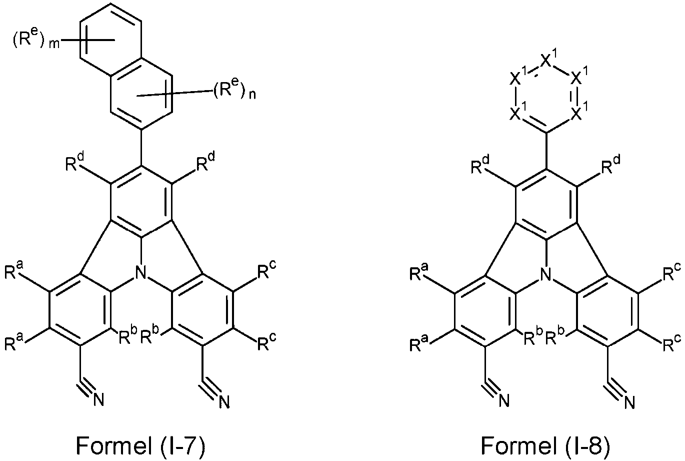 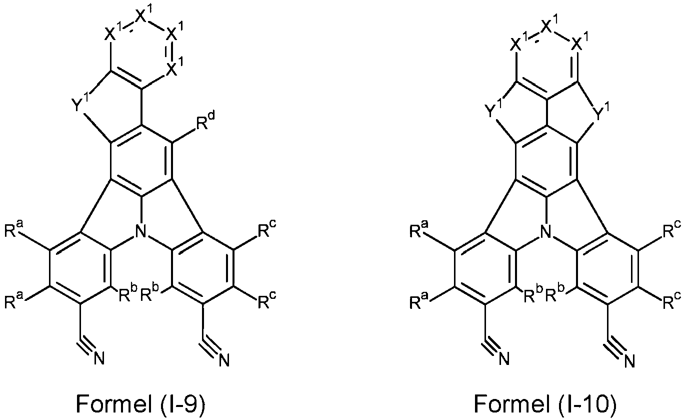 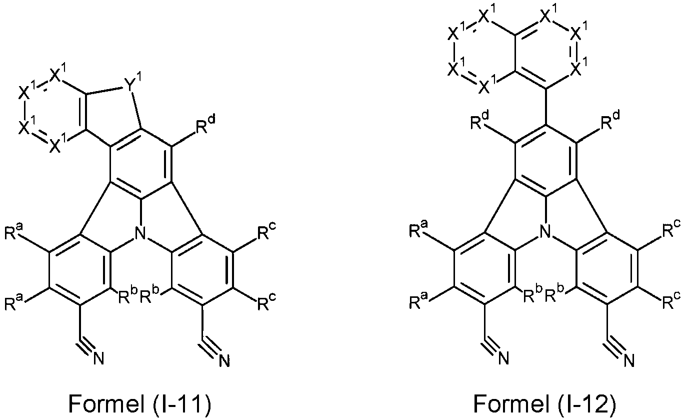 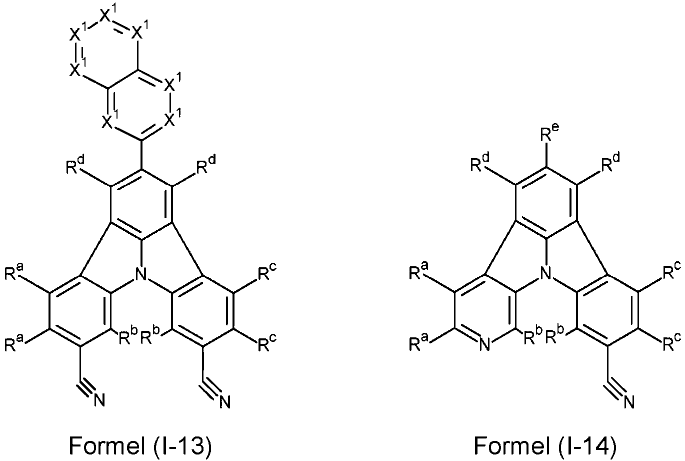 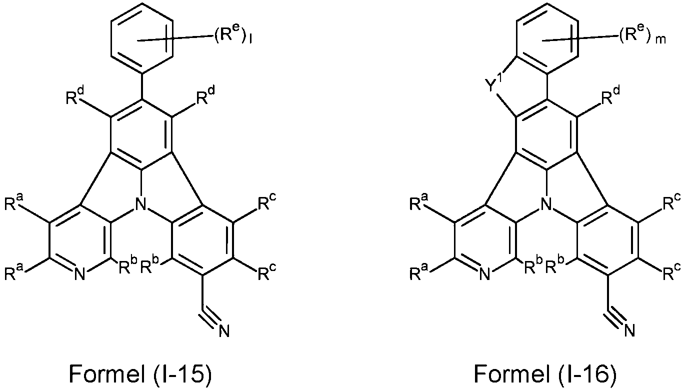 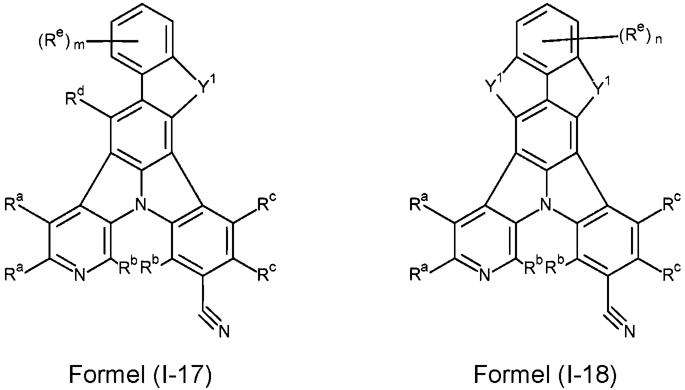 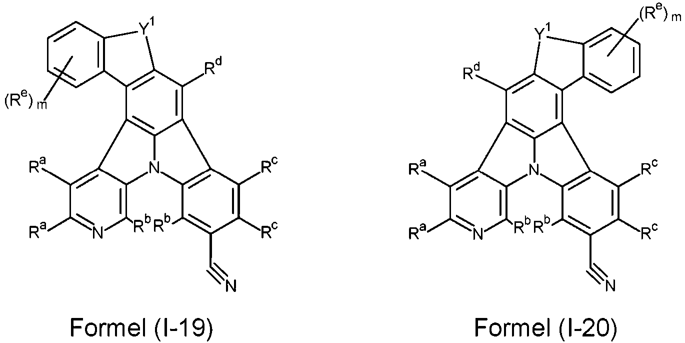 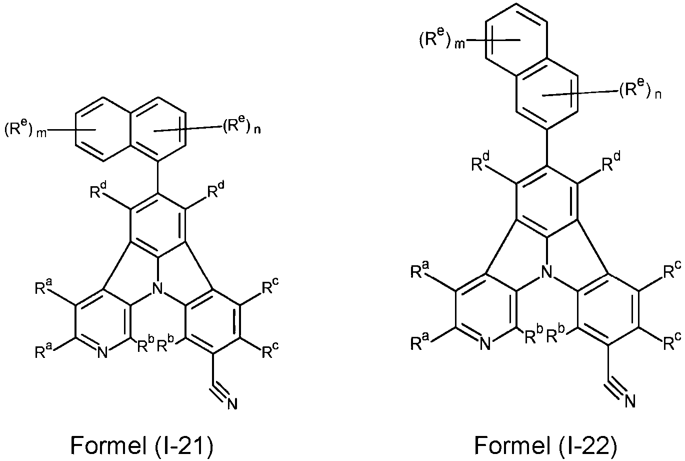 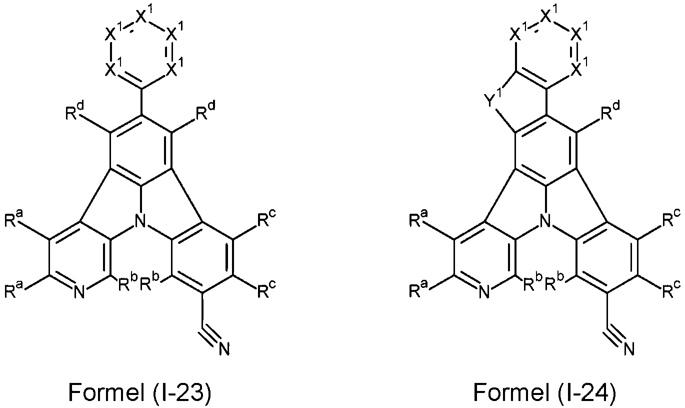 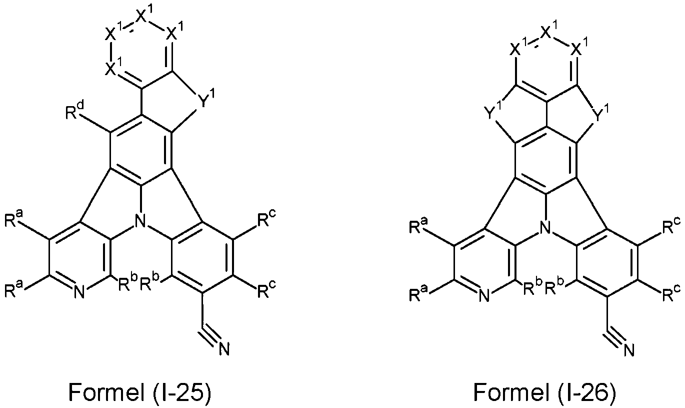 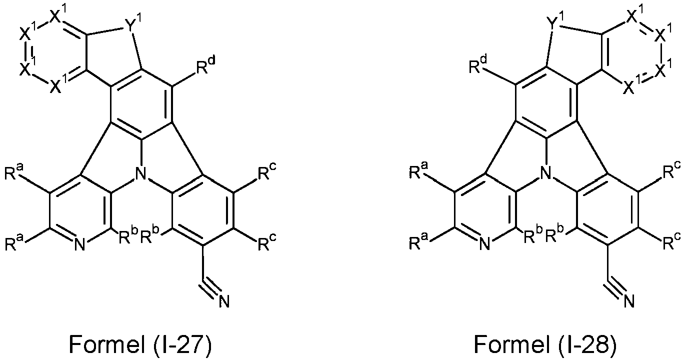 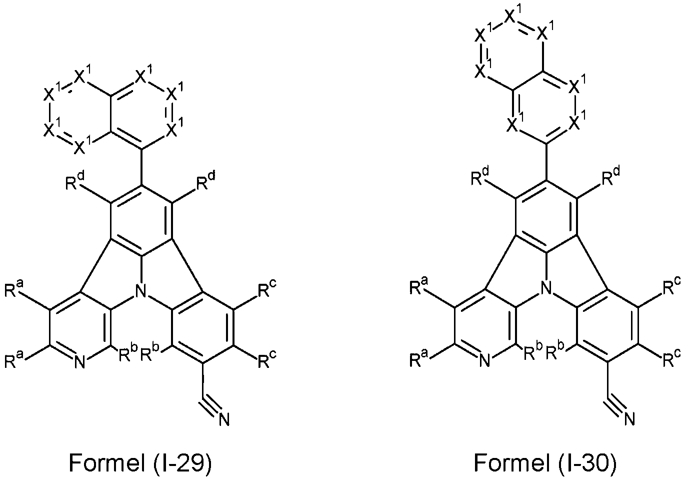 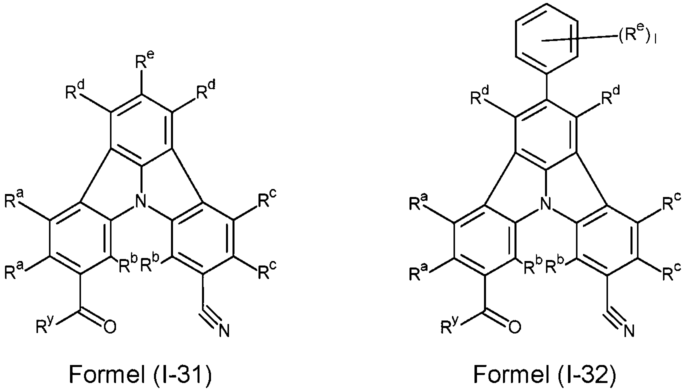 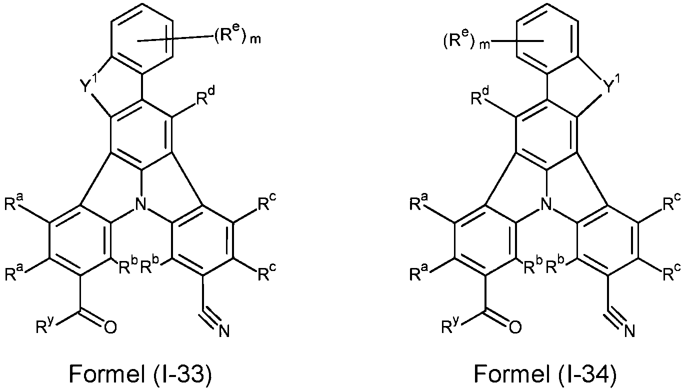 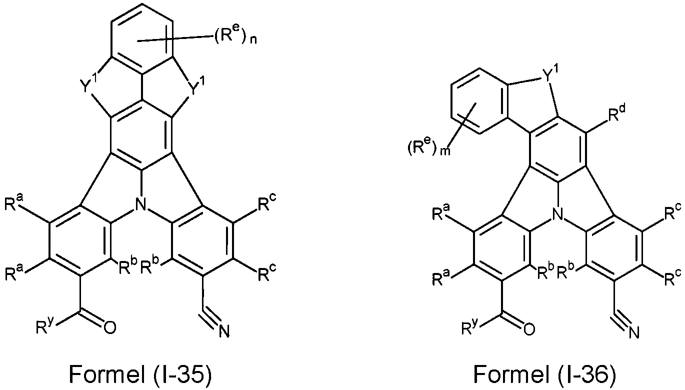 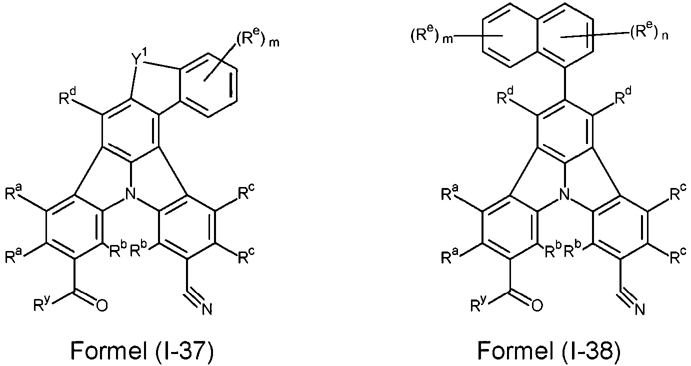 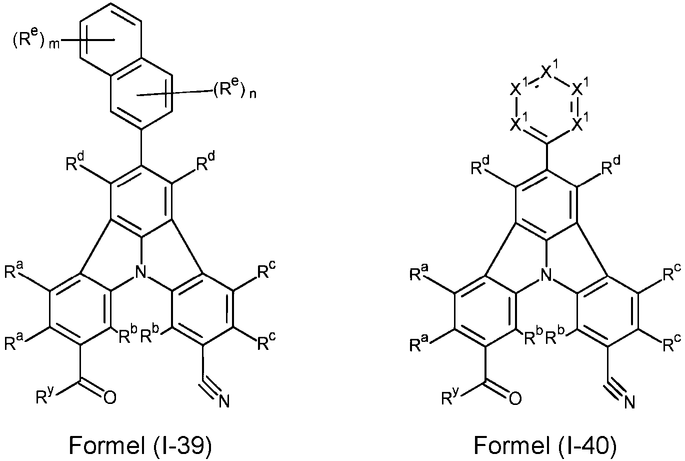 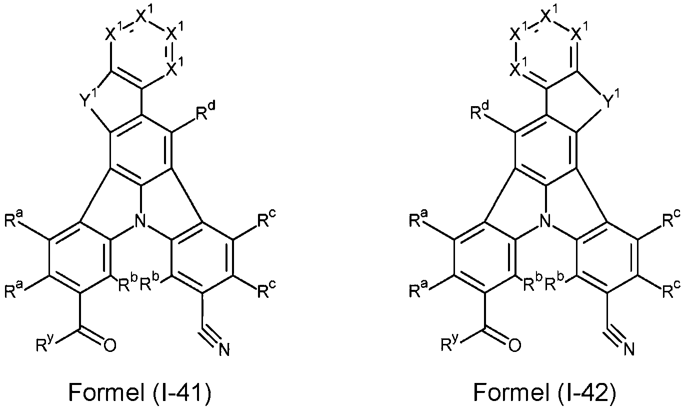 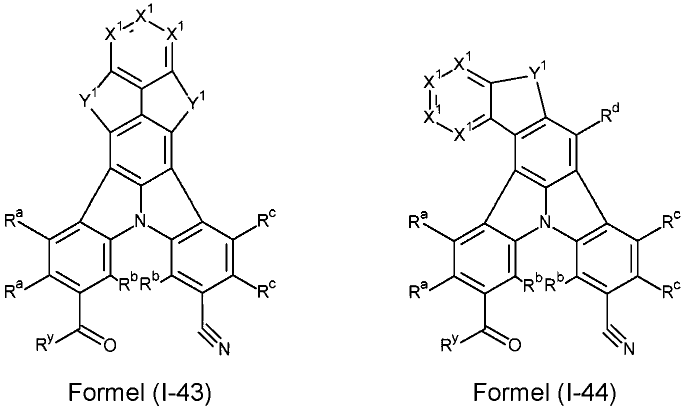 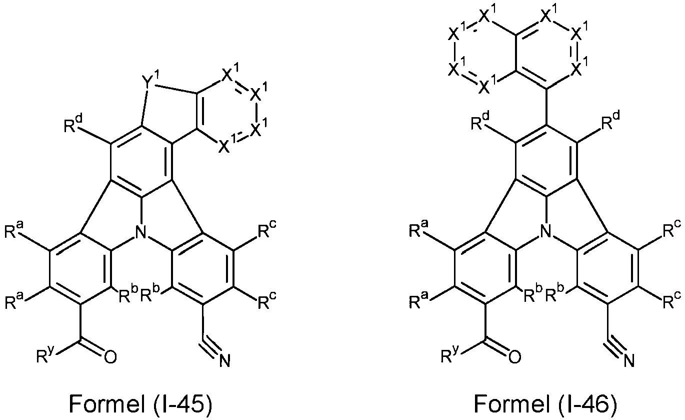 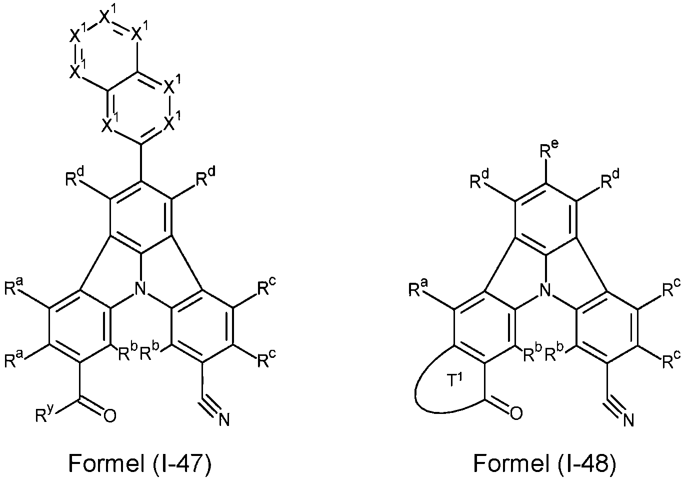 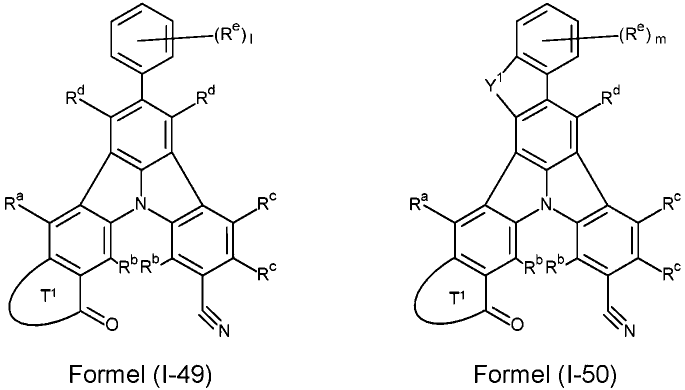 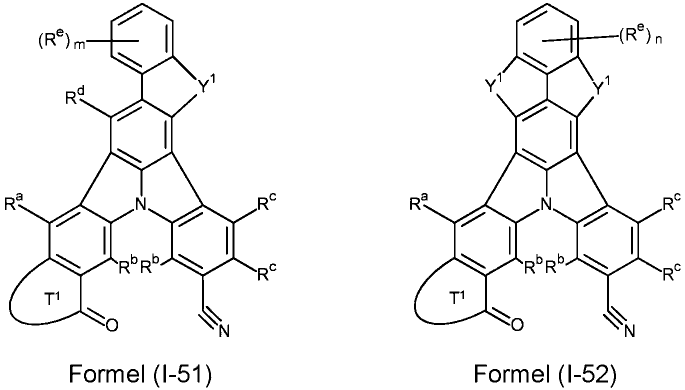 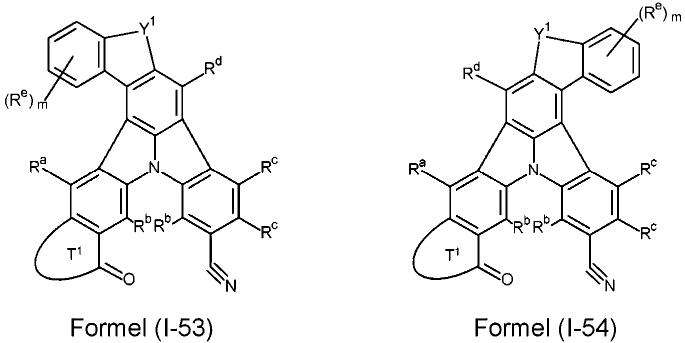 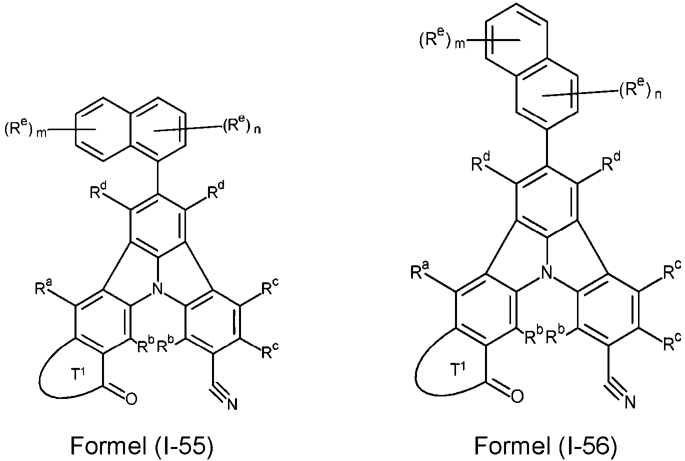 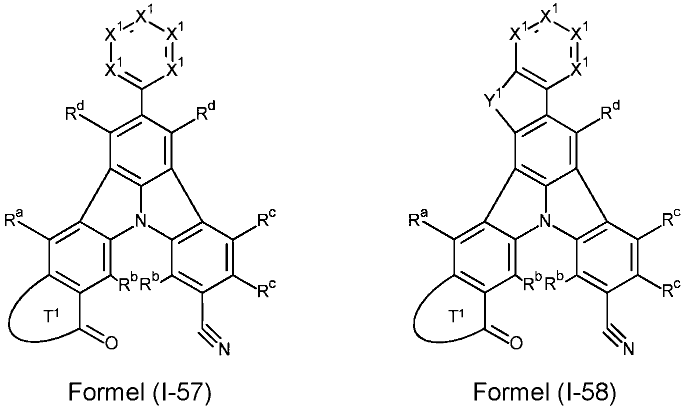 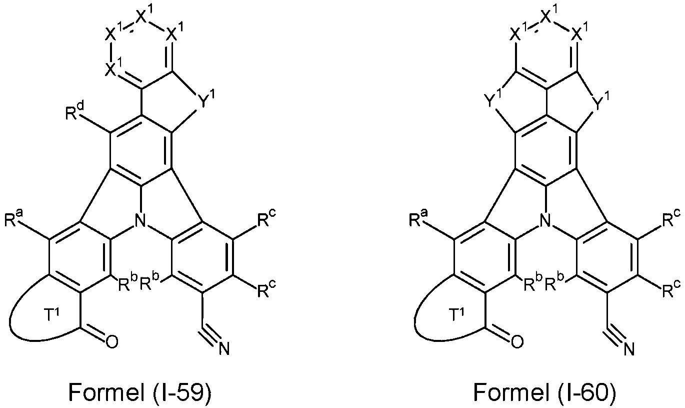 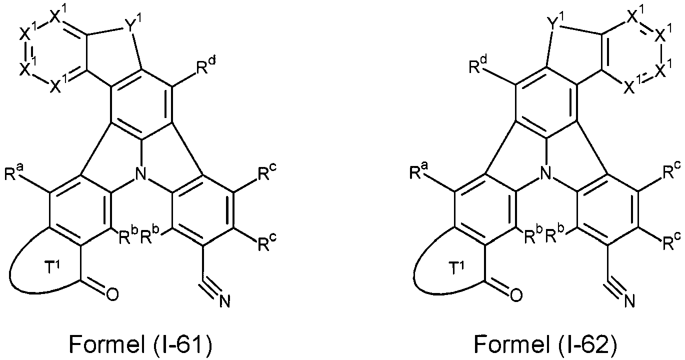 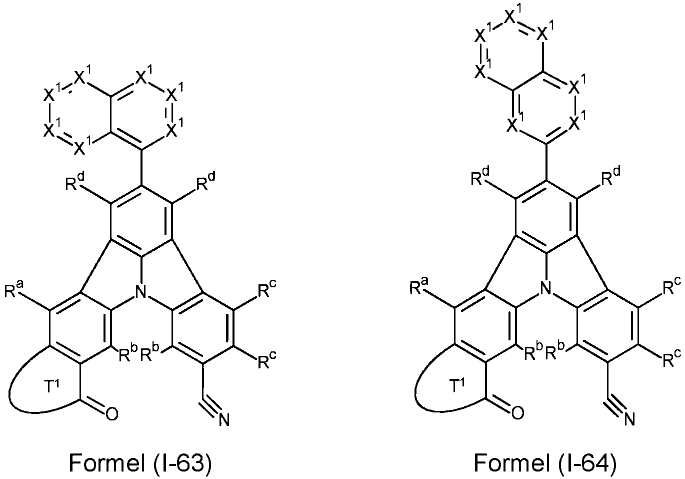 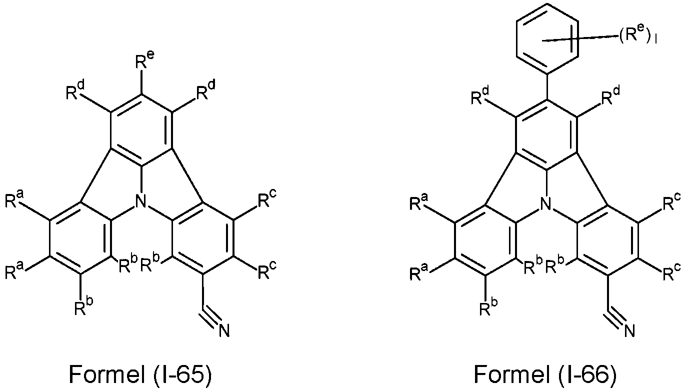 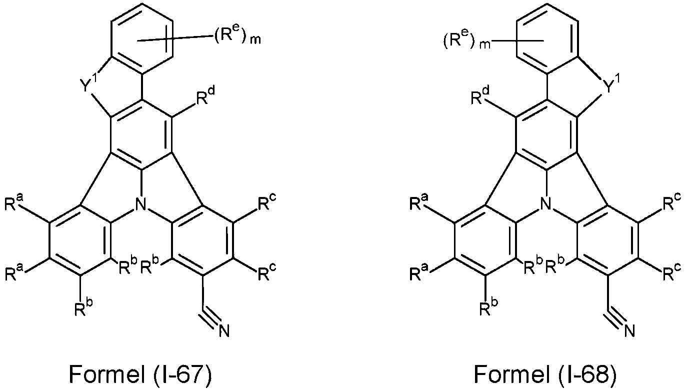 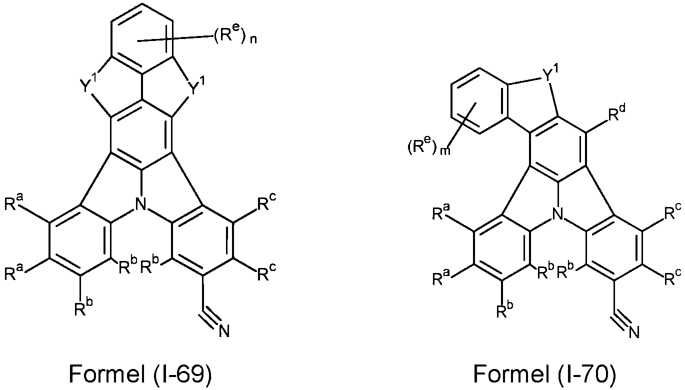 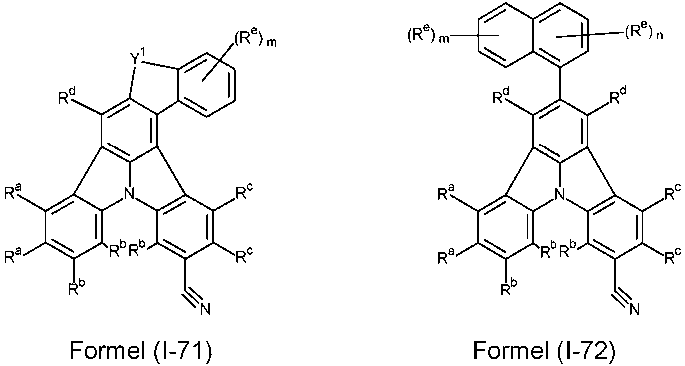 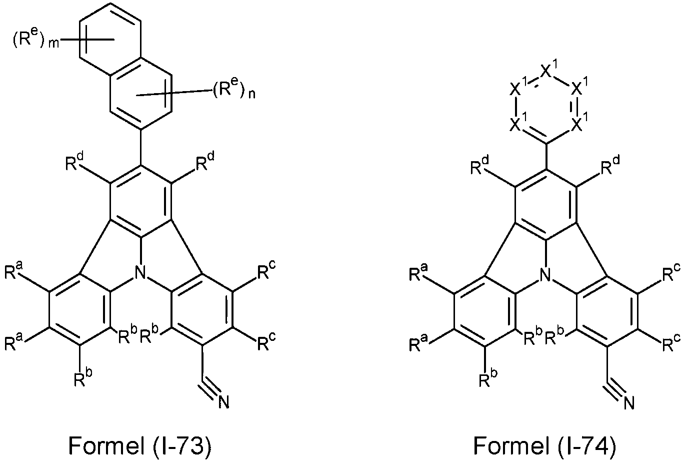 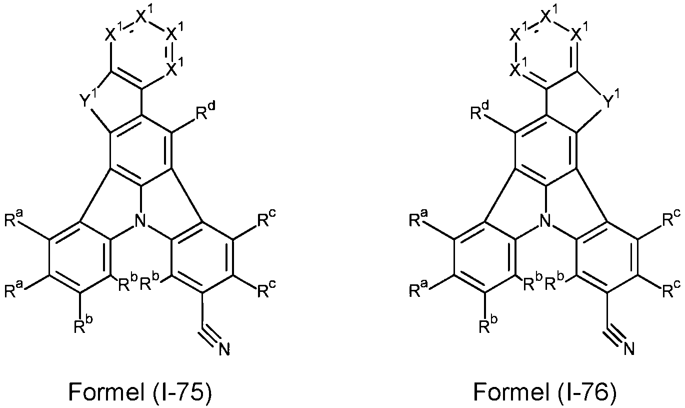 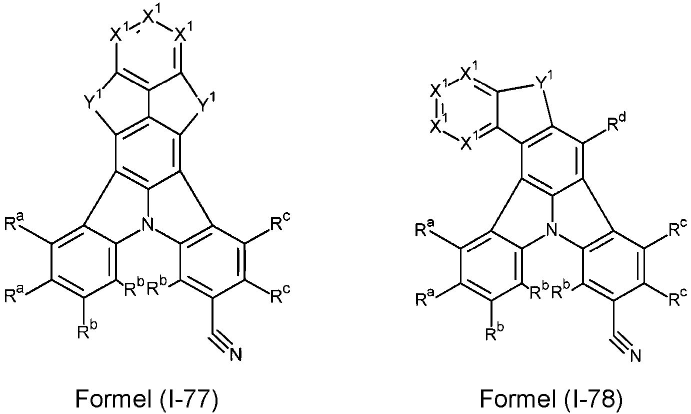 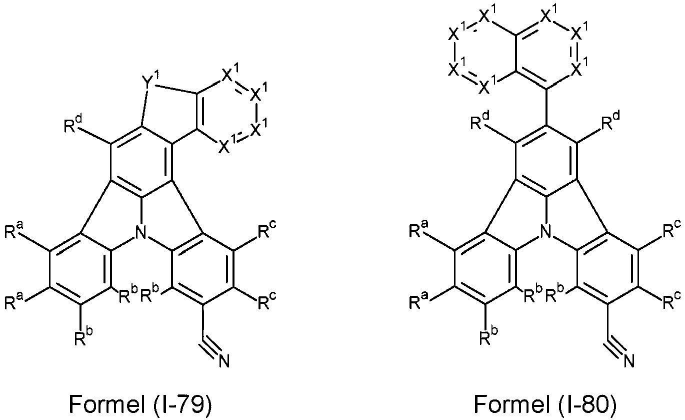 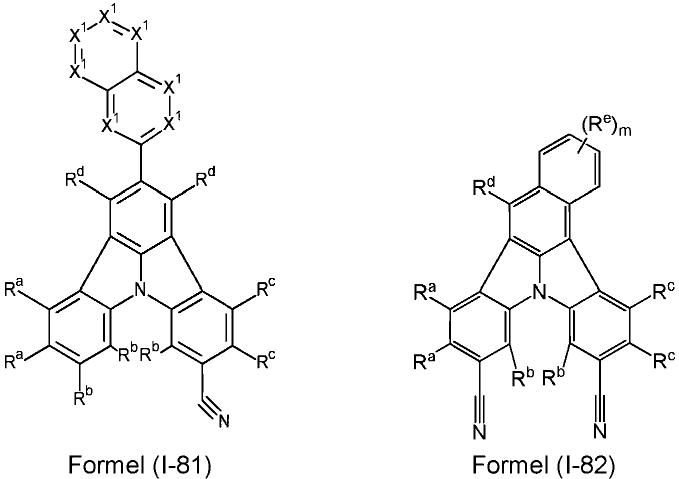 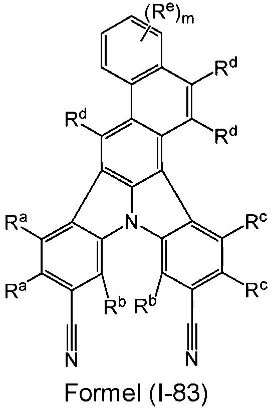 wobei die Symbole R^{a}, R^{b}, R^{c}, R^{d}, R^{e} und R^{y} die zuvor, insbesondere für Formel (I) genannten Bedeutungen aufweisen und die weiteren für die verwendeten Symbole und Indices gilt:
- X¹: steht bei jedem Auftreten gleich oder verschieden für N oder CR^{e}, vorzugsweise für CR^{e} mit der Maßgabe, dass nicht mehr als zwei der Gruppen X¹ in einem Cyclus für N stehen;
- Y¹: ist bei jedem Auftreten gleich oder verschieden C(R^{e})₂, (R^{e})₂C-C(R^{e})₂, (R^{e})C=C(R^{e}), NR^{e}, NAr', O, S, SO, SO₂, Se, P(O)R^{e}, BR^{e} oder Si(R^{e})2, vorzugsweise C(Rf^{e})₂, (R^{e})_{2C}-C(R^{e})₂, (R^{e})C=C(R^{e}), O oder S, besonders bevorzugt C(R^{e})₂;
- T¹: steht bei jedem Auftreten gleich oder verschieden für einen kondensierten Ring, vorzugsweise für einen aliphatischen oder heteroaliphatischen Ring mit 3 bis 20, vorzugsweise 5 bis 18 Ringatomen oder für einen aromatischen oder heteroaromatischen Ring mit 5 bis 13 Ringatomen, besonders bevorzugt für einen aliphatischen oder heteroaliphatischen Ring mit 3 bis 20, vorzugsweise 5 bis 18 Ringatomen, der mit einem oder mehreren Resten R¹ substituiert sein kann, wobei R¹ die zuvor, insbesondere für Formel (I) genannte Bedeutung aufweist;
- n: ist 0, 1, 2 oder 3, vorzugsweise 0, 1 oder 2;
- m: ist 0, 1, 2, 3 oder 4, vorzugsweise 0, 1 oder 2.

Überraschend zeigen Verbindungen/Strukturen, bei denen das Symbol X in Formel (I) für C-CN steht unerwartete Vorteile hinsichtlich der Leistungsfähigkeit, insbesondere in Bezug auf die Farbreinheit, so dass Verbindungen mit zwei Cyano-Substituenten an den aromatischen Ringen deutlich schmalere Emissionsspektren zeigen. Überraschend sind die Strukturen/Verbindungen der Formeln (I-1) bis (I-13) bevorzugt und Strukturen/Verbindungen der Formeln (I-1) bis (I-7) besonders bevorzugt.

In einer bevorzugten Ausgestaltung der vorliegenden Erfindung kann vorgesehen sein, dass mindestens zwei Reste R, R^{a}, R^{b}, R^{c}, R^{d}, R^{e}, R^{y} mit den weiteren Gruppen, an die die zwei Reste R, R^{a}, R^{b}, R^{c}, R^{d}, R^{e}, R^{y} binden, einen kondensierten Ring bilden, wobei die zwei Reste R, R^{a}, R^{b}, R^{c}, R^{d}, R^{e}, R^{y} mindestens eine Struktur der folgenden Formeln (Cy-1) bis (Cy-10) formen 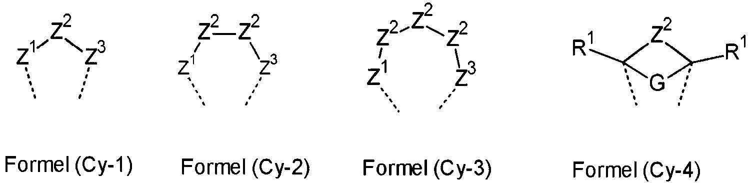 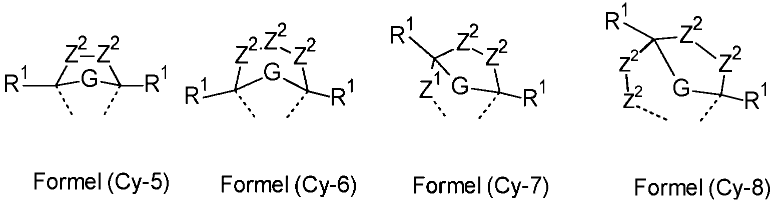 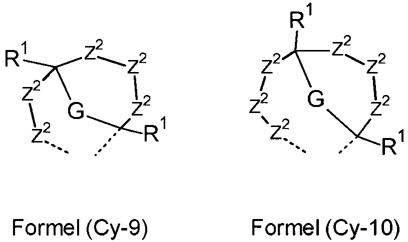 wobei R¹ und R² die zuvor dargelegte Bedeutungen aufweisen, die gestrichelten Bindungen die Anbindungsstellen an die Atome der Gruppen, an die die zwei Reste R, R^{a}, R^{b}, R^{c}, R^{d}, R^{e}, R^{y} binden, darstellen und weiterhin gilt:
- Z¹, Z³: ist gleich oder verschieden bei jedem Auftreten C(R³)₂, Si(R³)₂, O, S, NR³ oder C(=O);
- Z²: ist C(R¹)2, Si(R¹)₂, O, S, NR¹ oder C(=O), wobei zwei benachbarte Gruppen Z² für -CR¹=CR¹- oder eine ortho-verknüpfte Arylen- oder Heteroarylengruppe mit 5 bis 14 aromatischen Ringatomen, welche durch einen oder mehrere Reste R¹ substituiert sein kann, stehen können;
- G: ist eine Alkylengruppe mit 1, 2 oder 3 C-Atomen, welche mit einem oder mehreren Resten R¹ substituiert sein kann, -CR¹=CR¹- oder eine ortho-verknüpfte Arylen- oder Heteroarylengruppe mit 5 bis 14 aromatischen Ringatomen, welche durch einen oder mehrere Reste R¹ substituiert sein kann;
- R³: ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch -R²C=CR²-, -C=C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem vorzugsweise benachbarten Rest R, R^{a}, R^{c}, R^{d}, R^{e} oder R¹ ein Ringsystem, ein vorzugsweise aliphatisches Ringsystem bilden,
mit der Maßgabe, dass in diesen Gruppen nicht zwei Heteroatome direkt aneinander gebunden sind und nicht zwei Gruppen C=O direkt aneinander gebunden sind.

In einer bevorzugten Ausführungsform der Erfindung ist R³ ungleich H und/oder D.

Wenn benachbarte Reste in den erfindungsgemäßen Strukturen ein aliphatisches Ringsystem bilden, dann ist es bevorzugt, wenn dieses keine aziden benzylischen Protonen aufweist. Unter benzylischen Protonen werden Protonen verstanden, die an ein Alkyl-Kohlenstoffatom binden, welches direkt an eine Aryl- oder Heteroarylgruppe gebunden sind. Dies kann dadurch erreicht werden, dass die Kohlenstoffatome des aliphatischen Ringsystems, die direkt an eine Aryl- oder Heteroarylgruppe binden, vollständig substituiert sind und keine Wasserstoffatome gebunden enthalten. So wird die Abwesenheit von aziden benzylischen Protonen in den Formeln (Cy-1) bis (Cy-3) dadurch erreicht, dass Z¹ und Z³, wenn diese für C(R³)₂ stehen, so definiert sind, dass R³ ungleich Wasserstoff ist. Dies kann weiterhin auch dadurch erreicht werden, dass die Kohlenstoffatome des aliphatischen Ringsystems, die direkt an eine Aryl- oder Heteroarylgruppe binden, die Brückenköpfe einer bi- oder polycyclischen Struktur sind. Die an Brückenkopfkohlenstoffatome gebundenen Protonen sind aufgrund der räumlichen Struktur des Bi- oder Polycyclus wesentlich weniger azide als benzylische Protonen an Kohlenstoffatomen, die nicht in einer bi- oder polycyclischen Struktur gebunden sind, und werden im Sinne der vorliegenden Erfindung als nicht-azide Protonen angesehen. So wird die Abwesenheit von aziden benzylischen Protonen ist in Formeln (Cy-4) bis (Cy-10) dadurch erreicht, dass es sich dabei um eine bicyclische Struktur handelt, wodurch R¹, wenn es für H steht, deutlich weniger azide als benzylische Protonen, da das korrespondierende Anion der bicyclischen Struktur nicht mesomeriestabilisiert ist. Auch wenn R¹ in Formeln (Cy-4) bis (Cy-10) für H steht, handelt es sich dabei daher um ein nicht-azides Proton im Sinne der vorliegenden Anmeldung.

Vorzugsweise kann vorgesehen sein, dass insbesondere in Formeln (Cy-1) bis (Cy-3) gilt:
- R³: ist bei jedem Auftreten gleich oder verschieden F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch -R²C=CR²-, -C≡C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem vorzugsweise benachbarten Rest R, R^{a}, R^{c}, R^{d}, R^{e}, R¹ oder mit einer weiteren Gruppe ein Ringsystem, vorzugsweise ein aliphatisches Ringsystem bilden.

Vorzugsweise kann vorgesehen sein, dass insbesondere in Formeln (Cy-1) bis (Cy-3) gilt:
- R³: ist bei jedem Auftreten gleich oder verschieden F, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C≡C, Si(R²)₂, C=O, C=S, C=Se, C=NR², -C(=O)O-, -C(=O)NR²-, NR², P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann; dabei können zwei Reste R³ auch miteinander oder ein Rest R³ mit einem Rest R, R^{a}, R^{c}, R^{d}, R^{e}, R¹ oder mit einer weiteren Gruppe ein Ringsystem, vorzugsweise ein aliphatisches Ringsystem bilden.

In einer bevorzugten Ausführungsform der Struktur gemäß Formel (Cy-1) bis (Cy-10) steht maximal eine der Gruppen Z¹, Z² und Z³ für ein Heteroatom, insbesondere für O oder NR³, beziehungsweise für O oder NR¹, und die anderen Gruppen stehen für C(R³)₂ bzw. C(R¹)₂ oder Z¹ und Z³ stehen gleich oder verschieden bei jedem Auftreten für O oder NR³ und Z² steht für C(R¹)₂. In einer besonders bevorzugten Ausführungsform der Erfindung stehen Z¹ und Z³ gleich oder verschieden bei jedem Auftreten für C(R³)₂ und Z² steht für C(R¹)₂ und besonders bevorzugt für C(R³)₂ oder CH₂.

In einer bevorzugten Ausführungsform der Erfindung ist der Rest R¹, der an das Brückenkopfatom, vorzugsweise an das Brückenkopfatom gemäß Formeln (Cy-4) bis (Cy-10) gebunden ist, gleich oder verschieden bei jedem Auftreten gewählt aus der Gruppe bestehend aus H, D, F, einer geradkettigen Alkylgruppe mit 1 bis 10 C-Atomen, die mit einem oder mehreren Resten R² substituiert sein kann, bevorzugt aber unsubstituiert ist, einer verzweigten oder cyclischen Alkylgruppe mit 3 bis 10 C-Atomen, die mit einem oder mehreren Resten R² substituiert sein kann, bevorzugt aber unsubstituiert ist, oder einem aromatischen oder heteroaromatischen Ringsystem mit 5 bis 12 aromatischen Ringatomen, das jeweils durch einen oder mehrere Reste R² substituiert sein kann. Besonders bevorzugt ist der Rest R¹, der an das Brückenkopfatom gemäß Formel (CY-4) gebunden ist, gleich oder verschieden bei jedem Auftreten gewählt aus der Gruppe bestehend aus H, F, einer geradkettigen Alkylgruppe mit 1 bis 4 C-Atomen, einer verzweigten Alkylgruppe mit 3 oder 4 C-Atomen oder einer Phenylgruppe, die durch eine Alkylgruppe mit 1 bis 4 C-Atomen substituiert sein kann, bevorzugt aber unsubstituiert ist. Ganz besonders bevorzugt ist der Rest R¹ bei jedem Auftreten gleich oder verschieden gewählt aus der Gruppe bestehend aus H, Methyl oder tert-Butyl.

In einer bevorzugten Weiterbildung der vorliegenden Erfindung kann vorgesehen sein, dass mindestens zwei Reste R, R^{a}, R^{b}, R^{c}, R^{d}, R^{e}, R^{y} mit den weiteren Gruppen, an die die zwei Reste R, R^{a}, R^{b}, R^{c}, R^{d}, R^{e}, R^{y} binden, einen kondensierten Ring bilden, wobei die zwei Reste R, R^{a}, R^{b}, R^{c}, R^{d}, R^{e}, R^{y} mindestens eine Struktur der Formeln (RA-1) bis (RA-13) formen 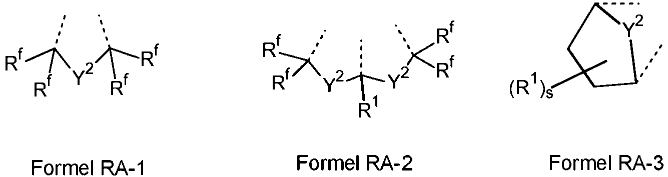 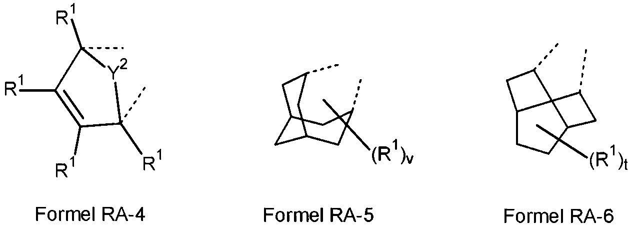 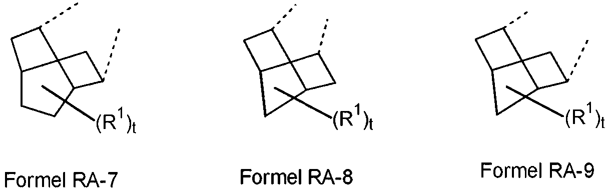 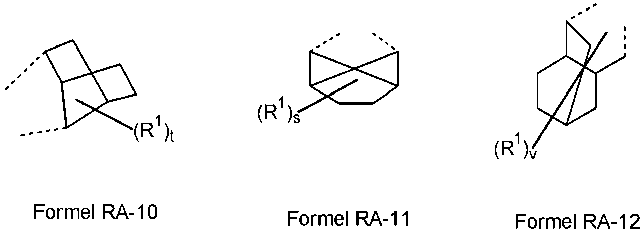 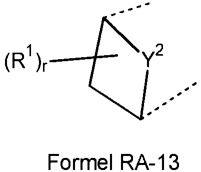 wobei R¹ die zuvor dargelegte Bedeutung hat, die gestrichelten Bindungen die Anbindungsstellen darstellen, über die die zwei Reste R, R^{a}, R^{b}, R^{c}, R^{d}, R^{e}, R^{y} binden, und die weiteren Symbole die folgende Bedeutung aufweisen:
- Y²: ist bei jedem Auftreten gleich oder verschieden C(R¹)2, (R¹)₂C-C(R¹)₂, (R¹)C=C(R¹), NR¹, NAr', O oder S, vorzugsweise C(R¹)2, (R¹)₂C-C(R¹)₂, (R¹)C=C(R¹), O oder S;
- R^{f}: ist bei jedem Auftreten gleich oder verschieden F, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C=C, Si(R²)₂, C=O, C=S, C=Se, C=NR², -C(=O)O-, -C(=O)NR²-, NR², P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann; dabei können zwei Reste R^{f} auch miteinander oder ein Rest R^{f} mit einem Rest R¹ oder mit einer weiteren Gruppe ein Ringsystem bilden;
- r: ist 0, 1, 2, 3 oder 4, vorzugsweise 0, 1, oder 2, besonders bevorzugt 0 oder 1;
- s: ist 0, 1, 2, 3, 4, 5 oder 6, vorzugsweise 0, 1, 2, 3, oder 4, besonders bevorzugt 0, 1 oder 2;
- t: ist 0, 1, 2, 3, 4, 5, 6, 7 oder 8, vorzugsweise 0, 1, 2, 3, oder 4, besonders bevorzugt 0, 1 oder 2;
- v: ist 0, 1, 2, 3, 4, 5, 6, 7, 8 oder 9, vorzugsweise 0, 1, 2, 3, oder 4, besonders bevorzugt 0, 1 oder 2.

Hierbei sind Strukturen der Formeln RA-1, RA-3, RA-4 und RA-5 bevorzugt und Strukturen der Formeln RA-4 und RA-5 besonders bevorzugt.

In einer bevorzugten Ausführungsform der Erfindung bilden mindestens zwei Reste R, R^{a}, R^{b}, R^{c}, R^{d}, R^{e}, R^{y} mit den weiteren Gruppen, an die die zwei Reste R, R^{a}, R^{b}, R^{c}, R^{d}, R^{e}, R^{y} binden, einen kondensierten Ring, wobei die zwei Reste R, R^{a}, R^{b}, R^{c}, R^{d}, R^{e}, R^{y} vorzugsweise mindestens eine der Strukturen der Formeln (RA-1a) bis (RA-4f) formen 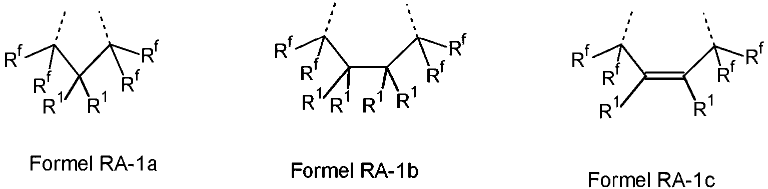 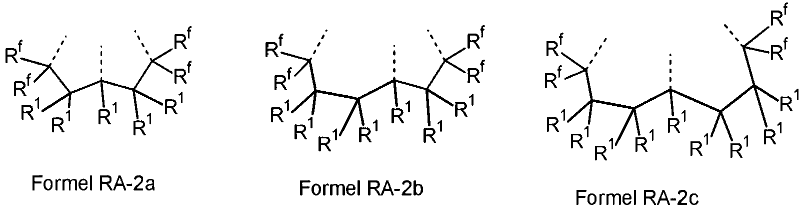 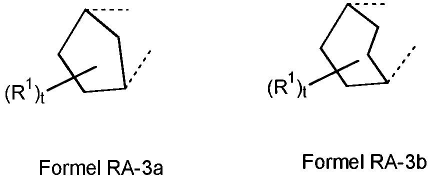 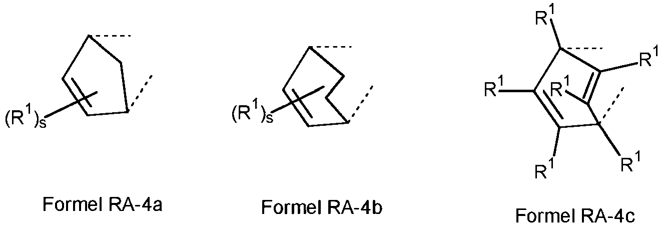 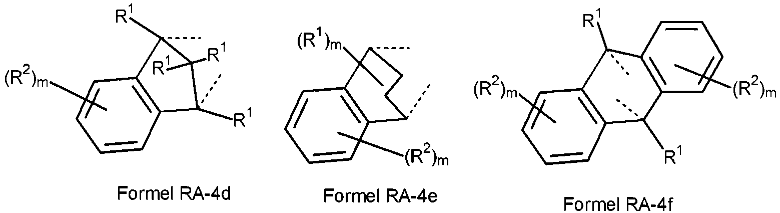 wobei die gestrichelten Bindungen die Anbindungsstellen darstellen, über die die zwei Reste R, R^{a}, R^{b}, R^{c}, R^{d}, R^{e}, R^{y} binden, der Index m 0, 1, 2, 3 oder 4, vorzugsweise 0, 1 oder 2 ist und die Symbole R¹, R², R^{f} und die Indices s und t die zuvor, insbesondere für Formel (I) und/oder Formeln (RA-1) bis (RA-13) dargelegte Bedeutung haben.

Hierbei sind Strukturen der Formeln RA-4f bevorzugt.

Ferner kann vorgesehen sein, dass zwei Reste R^{a} die Strukturen der Formeln (Cy-1) bis (Cy-10), (RA-1) bis (RA-13) und/oder (RA-1a) bis (RA-4f) formen und einen kondensierten Ring bilden.

Darüber hinaus kann vorgesehen sein, dass ein Rest R^{a} und ein Rest R^{y} Strukturen der Formeln (Cy-1) bis (Cy-10), (RA-1) bis (RA-13) und/oder (RA-1a) bis (RA-4f) formen und einen kondensierten Ring bilden, wobei der Rest R^{a} und der Rest R^{y} vorzugsweise benachbart sind. Ferner kann auch ein Rest R^{b} und ein Rest R^{y} Strukturen der Formeln (Cy-1) bis (Cy-10), (RA-1) bis (RA-13) und/oder (RA-1a) bis (RA-4f) formen und einen kondensierten Ring bilden, wobei eine Ringbildung über einen Rest R^{a} bevorzugt ist.

Weiterhin kann vorgesehen sein, dass zwei Reste R^{c} die Strukturen der Formeln (Cy-1) bis (Cy-10), (RA-1) bis (RA-13) und/oder (RA-1a) bis (RA-4f) formen und einen kondensierten Ring bilden.

In einer weiteren Ausgestaltung kann vorgesehen sein, dass zwei Reste R^{b} die Strukturen der Formeln (Cy-1) bis (Cy-10), (RA-1) bis (RA-13) und/oder (RA-1a) bis (RA-4f) formen und einen kondensierten Ring bilden, wobei die Reste R^{b} vorzugsweise benachbart sind. Des weiteren können die zwei Reste R^{b} auch von unterschiedlichen Ringen stammen, wobei die Ringe jeweils an das Stickstoffatom des Grundgerüsts binden.

Ferner kann vorgesehen sein, dass ein Rest R^{d} mit einem Rest R oder R^{e} die Strukturen der Formeln (Cy-1) bis (Cy-10), (RA-1) bis (RA-13) und/oder (RA-1a) bis (RA-4f) formen und einen kondensierten Ring bilden.

Darüber hinaus kann vorgesehen sein, dass zwei Reste R^{e} die Strukturen der Formeln (Cy-1) bis (Cy-10), (RA-1) bis (RA-13) und/oder (RA-1a) bis (RA-4f) formen und einen kondensierten Ring bilden, wobei die Reste R^{e} vorzugsweise benachbart sind.

Hierbei bilden vorzugsweise zwei Reste R^{a}, zwei Reste R^{c}, ein Rest R^{d} mit einem Rest R oder R^{e} oder zwei Reste R^{e} die Strukturen der Formeln (Cy-1) bis (Cy-10), (RA-1) bis (RA-13) und/oder (RA-1a) bis (RA-4f) und formen mindestens einen kondensierten Ring, besonders bevorzugt zwei Reste R^{a} und/oder zwei Reste R^{c}. Speziell bevorzugt bilden zwei Reste R^{a} und zwei Reste R^{c} jeweils einen kondensierten Ring.

In einer weiterhin bevorzugten Ausgestaltung bilden mindestens zwei Reste R, R^{a}, R^{b}, R^{c}, R^{d}, R^{e}, R^{y} mit den weiteren Gruppen, an die die zwei Reste R, R^{a}, R^{b}, R^{c}, R^{d}, R^{e}, R^{y} binden, einen kondensierten Ring, wobei die zwei Reste R, R^{a}, R^{b}, R^{c}, R^{d}, R^{e}, R^{y} Strukturen der Formel (RB), formen 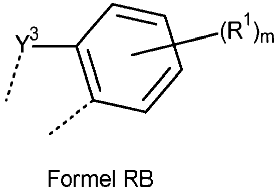 wobei R¹ die zuvor, insbesondere für Formel (I) genannte Bedeutung aufweist, die gestrichelten Bindungen die Anbindungsstellen darstellen, über die die zwei Reste R, R^{a}, R^{b}, R^{c}, R^{d}, R^{e}, R^{y} binden, der Index m 0, 1, 2, 3 oder 4, vorzugsweise 0, 1 oder 2 ist, und Y³ C(R¹)2, NR¹, NAr', BR¹, BAr', O oder S ist, vorzugsweise C(R¹)2, NAr' oder O, wobei Ar' die zuvor, insbesondere für Formel (I) genannte Bedeutung aufweist.

Hierbei kann vorgesehen sein, dass ein Rest R^{d} mit einem Rest R oder R^{e} die Strukturen der Formel (RB) formen und einen kondensierten Ring bilden. Ferner kann vorgesehen sein, dass zwei Reste R^{e} die Strukturen der Formel (RB) formen und einen kondensierten Ring bilden, wobei die Reste R^{e} vorzugsweise benachbart sind.

Insbesondere kann vorgesehen sein, dass in bevorzugten Strukturen/Verbindungen die Summe der Indices r, s, t, v, m und n vorzugsweise 0, 1, 2 oder 3, besonders bevorzugt 1 oder 2 ist.

Bevorzugt kann vorgesehen sein, dass die Verbindungen mindestens zwei kondensierte Ringe aufweisen, wobei mindestens ein kondensierter Ring durch Strukturen der Formeln (RA-1) bis (RA-13) und/oder (RA-1a) bis (RA-4f) gebildet ist und ein weiterer Ring durch Strukturen der Formeln (RA-1) bis (RA-13), (RA-1a) bis (RA-4f) oder (RB) gebildet ist.

Falls die Verbindungen mindestens zwei kondensierte Ringe aufweisen, bilden vorzugsweise zwei Reste R^{a}, zwei Reste R^{c}, ein Rest R^{d} mit einem Rest R oder R^{e} oder zwei Reste R^{e} die Strukturen der Formeln (Cy-1) bis (Cy-10), (RA-1) bis (RA-13) und/oder (RA-1a) bis (RA-4f) und formen jeweils mindestens einen kondensierten Ring, besonders bevorzugt zwei Reste R^{a} und zwei Reste R^{c}.

Weiterhin kann bevorzugt vorgesehen sein, dass der kondensierte Ring T¹, der unter anderem in Formeln (I-48) bis (I-64) dargelegt ist, ausgewählt ist aus einer Struktur der Formeln (TCY-1) bis (TRA-13) beziehungsweise (TRA-4f) 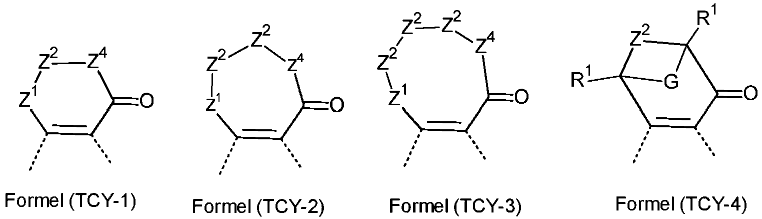 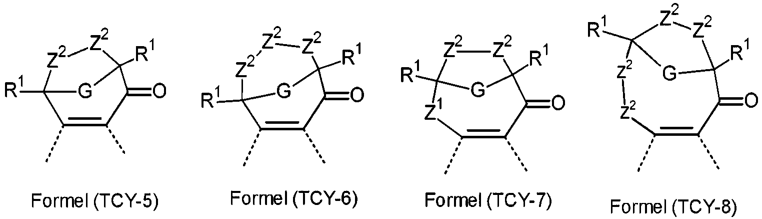 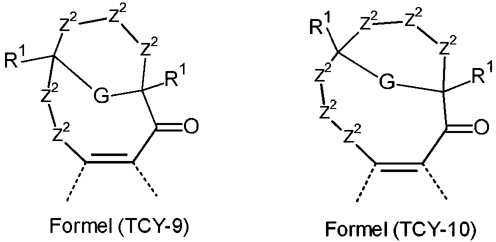 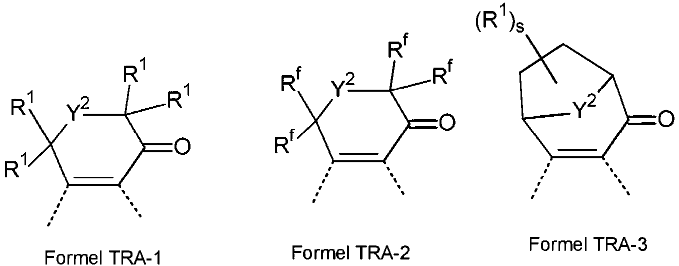 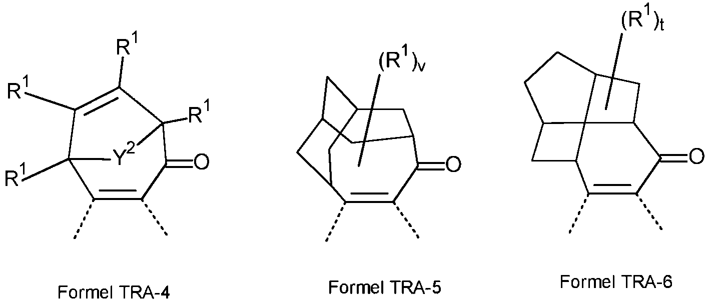 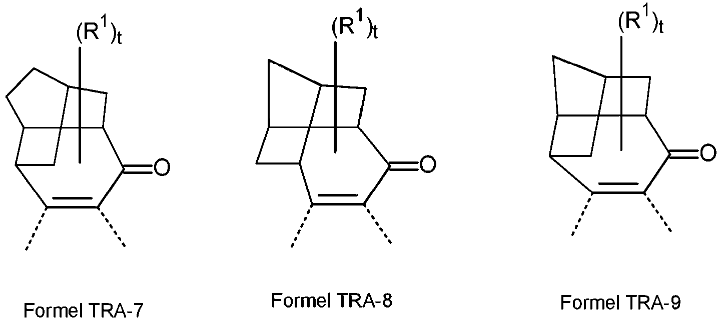 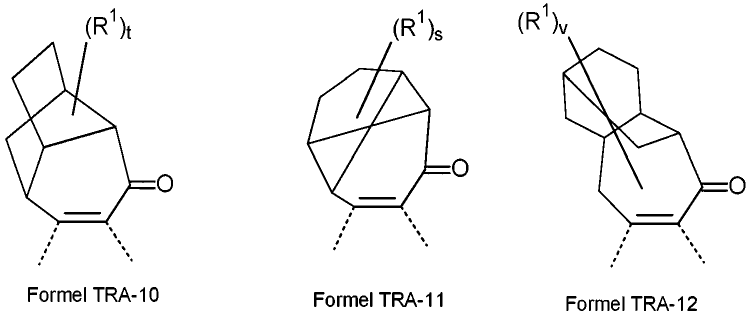 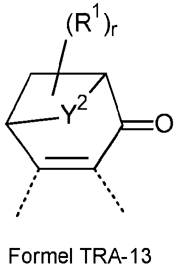 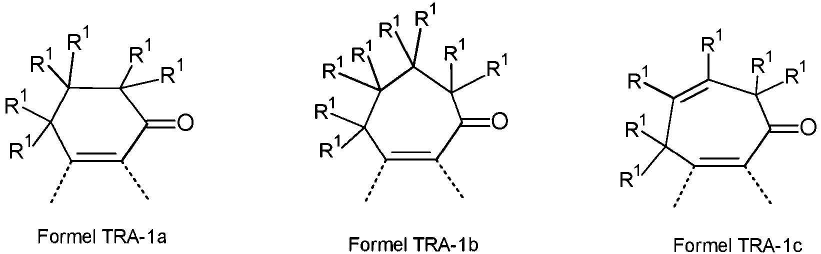 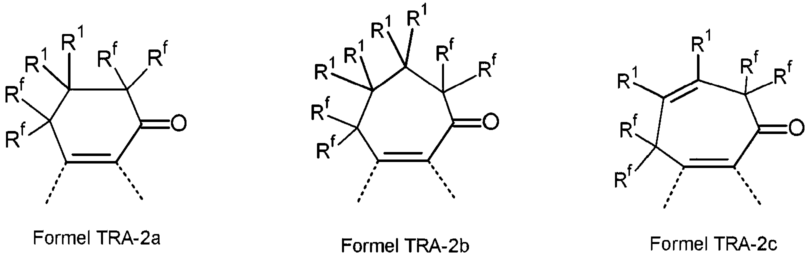 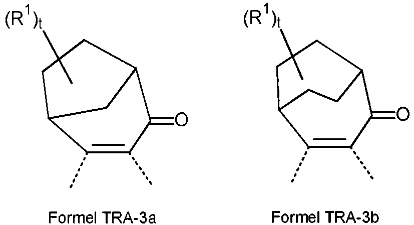 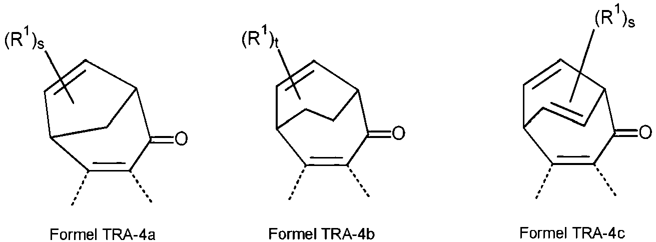 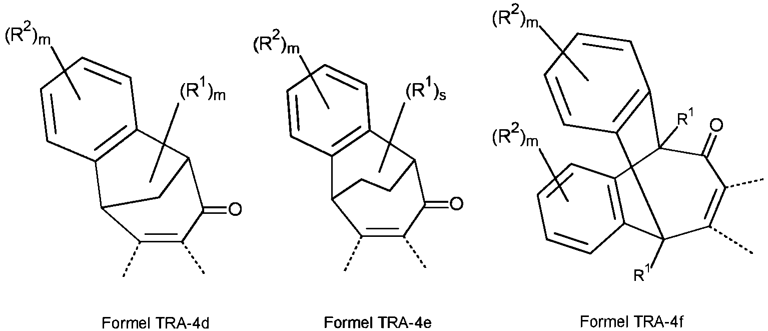 wobei die Symbole R¹ und R² die zuvor, insbesondere für Formel (I) genannten Bedeutungen aufweisen, die Symbole Z¹, Z², G und R³ die zuvor, insbesondere für Formeln (Cy-1) bis (Cy-10) genannten Bedeutungen aufweisen und Z⁴ gleich oder verschieden bei jedem Auftreten C(R³)₂, O, S oder NR³, vorzugsweise gleich oder verschieden bei jedem Auftreten C(R³)₂ ist mit der Maßgabe, dass in diesen Gruppen nicht zwei Heteroatome direkt aneinander gebunden sind und nicht zwei Gruppen C=O direkt aneinander gebunden sind, die Symbole Y² und R^{f} und die Indices r, s, t und v die zuvor, insbesondere für Formeln (RA-1) bis (RA-13) genannten Bedeutungen aufweisen, der Index m 0, 1, 2, 3 oder 4, vorzugsweise 0, 1 oder 2 ist, die gestrichelten Bindungen die Anbindungsstellen des kondensierten Rings mit den weiteren Gruppen darstellen.

Weiterhin kann vorgesehen sein, dass die Substituenten R, R^{a}, R^{b}, R^{c}, R^{d}, R^{e}, R^{f}, R^{y}, R¹ und R² gemäß obigen Formeln mit den Ringatomen des Ringsystems, an das die Substituenten R, R^{a}, R^{b}, R^{c}, R^{d}, R^{e}, R^{f}, R^{y}, R¹ und R² binden, kein kondensiertes aromatisches oder heteroaromatisches Ringsystem bilden. Dies schließt die Bildung eines kondensierten aromatischen oder heteroaromatischen Ringsystems mit möglichen Substituenten R¹ und R² ein, die an die Reste R, R^{a}, R^{b}, R^{c}, R^{d}, R^{e}, R^{f}, R^{y} und R¹ gebunden sein können.

Wenn die erfindungsgemäße Verbindung mit aromatischen oder heteroaromatischen Gruppen R, R^{a}, R^{b}, R^{c}, R^{d}, R^{e}, R^{f}, R^{y}, R¹ bzw. R² substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

Vorzugsweise kann daher vorgesehen sein, dass der Rest R keine durchgängig konjungierte Anthracen-Gruppe umfasst, bevorzugt keiner der Reste R, R^{a}, R^{b}, R^{c}, R^{d}, R^{e}, R^{f}, R^{y}, R¹, R² eine durchgängig konjungierte Anthracen-Gruppe umfasst.

Eine durchgängige Konjugation der Anthracen-Gruppe wird ausgebildet, sobald direkte Bindungen zwischen der Anthracen-Gruppe, dem erfindungsgemäßen Grundgerüst, welches in Formel (I) dargestellt ist, und einer optionalen aromatischen oder heteroaromatischen Verbindungsgruppe gebildet werden. Eine weitere Verknüpfung zwischen den zuvor genannten konjugierten Gruppen, die beispielsweise über ein S-, N- oder O-Atom oder eine Carbonylgruppe erfolgt, schadet einer Konjugation nicht. Bei einem Fluorensystem sind die beiden aromatischen Ringe unmittelbar gebunden, wobei das sp³ hybridisierte Kohlenstoffatom in Position 9 zwar eine Kondensation dieser Ringe unterbindet, jedoch eine Konjugation erfolgen kann, da dieses sp³ hybridisierte Kohlenstoffatom in Position 9 nicht zwingend zwischen den Gruppen, die über eine Verbindungsgruppe verbunden sind, liegt. Im Gegensatz hierzu kann bei einer Spirobifluorenstruktur eine durchgängige Konjugation ausgebildet werden, falls die Verbindung zwischen den Gruppen, die über die Spirobifluorengruppe verbunden sind, über die gleiche Phenylgruppe der Spirobifluorenstruktur oder über Phenylgruppen der Spirobifluorenstruktur, die unmittelbar aneinander gebunden sind und in einer Ebene liegen, erfolgt. Falls die Verbindung zwischen den Gruppen, die über eine Spirobifluorengruppe verbunden sind, über verschiedene Phenylgruppen der zweiten Spirobifluorenstruktur erfolgt, die über das sp³ hybridisierte Kohlenstoffatom in Position 9 verbunden sind, ist die Konjugation unterbrochen.

Besonders bevorzugt kann weiterhin vorgesehen sein, dass der Rest R keine Anthracen-Gruppe umfasst, bevorzugt keiner der Reste R, R^{a}, R^{b}, R^{c}, R^{d}, R^{e}, R^{f}, R^{y}, R¹, R² eine Anthracen-Gruppe umfasst.

Ganz speziell bevorzugt kann ferner vorgesehen sein, dass der Reste R kein aromatisches oder heteroaromatisches Ringsystem umfasst, welches drei linear kondensierte aromatische 6 Ringe aufweist, wobei vorzugsweise keiner der Reste R, R^{a}, R^{b}, R^{c}, R^{d}, R^{e}, R^{f}, R^{y}, R¹, R² ein aromatisches oder heteroaromatisches Ringsystem umfasst, welches drei linear kondensierte aromatische 6 Ringe aufweist.

Weiterhin kann vorgesehen sein, dass der Rest R^{y} keine Fluorenon-Gruppe umfasst oder bildet, bevorzugt keiner der Reste R, R^{a}, R^{b}, R^{c}, R^{d}, R^{e} eine Fluorenon-Gruppe umfasst oder bildet. Dies schließt Substituenten ein, die an die Reste R, R^{a}, R^{b}, R^{c}, R^{d}, R^{e}, R^{y} binden. Eine Fluorenon umfasst einen 5-Ring mit einer CO-Gruppe an den zwei aromatische 6-Ringe kondensiert sind.

Wenn zwei Reste, die insbesondere ausgewählt sein können aus R, R^{a}, R^{b}, R^{c}, R^{d}, R^{e}, R^{f}, R^{y}, R¹ und R², miteinander ein Ringsystem bilden, so kann dieses mono- oder polycyclisch, aliphatisch, heteroaliphatisch, aromatisch oder heteroaromatisch sein. Dabei können die Reste, die miteinander ein Ringsystem bilden, benachbart sein, d.h. dass diese Reste an dasselbe Kohlenstoffatom oder an Kohlenstoffatome, die direkt aneinander gebunden sind, gebunden sind, oder sie können weiter voneinander entfernt sein. Weiterhin können die mit den Substituenten R, R^{a}, R^{b}, R^{c}, R^{d}, R^{e}, R^{f}, R^{y}, R¹ und/oder R² versehenen Ringsysteme auch über eine Bindung miteinander verbunden sein, so dass hierdurch ein Ringschluss bewirkt werden kann. In diesem Fall ist jede der entsprechenden Bindungsstellen vorzugsweise mit einem Substituenten R, R^{a}, R^{b}, R^{c}, R^{d}, R^{e}, R^{f}, R^{y}, R¹ und/oder R² versehen.

Vorzugsweise kann vorgesehen sein, dass die Struktur/Verbindung in Bezug auf die Reste R^{a} und R^{c} symmetrisch ist. Weiterhin kann vorgesehen sein, dass die Struktur/Verbindung in Bezug auf die Reste R^{a}, R^{b} und R^{c} symmetrisch ist. Ferner kann vorgesehen sein, dass die Struktur/Verbindung in Bezug auf die Reste R^{a}, R^{b}, R^{c} und R^{d} symmetrisch ist.

Symmetrisch in Bezug auf die Reste R^{a} und R^{c} bedeutet insbesondere, dass die entsprechenden Reste R^{a} und R^{c} gleich sind und sich nicht unterscheiden. Hierbei bezieht sich die Gleichheit auf beide Reste R^{a} und R^{c}. Falls zwei Reste R^{a} beispielsweise einen Ring der Struktur RA-1 bilden, so bilden beide Reste R^{c} einen identischen Ring der Struktur RA-1.

Strukturen/Verbindungen, bei denen die Reste R^{a} und R^{c} symmetrisch sind, zeichnen sich durch eine überraschend hohe Farbreinheit aus, die sich insbesondere in einem engen Emissionsspektrum widerspiegelt.

In einer weiteren Ausgestaltung kann die Struktur/Verbindung in Bezug auf die Reste R^{a} und R^{c} unsymmetrisch sein.

Ferner kann vorgesehen sein, dass der Rest R mindestens eine Gruppe ausgewählt aus C(Ar)₃, C(R^{e})₃, N(Ar)₂, N(R^{e})₂, Si(Ar)₃, Si(R^{e})₃, B(R^{e})₂, vorzugsweise ausgewählt aus C(Ar)₃, C(R^{e})₃, N(Ar)₂, Si(Ar)₃, Si(R^{e})₃ umfasst, besonders bevorzugt eine Fluorengruppe, die mit einem oder mehreren Resten R^{e} substituiert sein kann, darstellt, umfasst oder mit einem Rest R^{d} bildet.

Weiterhin kann vorgesehen sein, dass der Rest R^{e} und/oder R^{d} mindestens eine Gruppe ausgewählt aus C(Ar')₃, C(R¹)₃, N(Ar')₂, N(R¹)₂, Si(Ar')₃, Si(R¹)₃, B(R¹)₂, vorzugsweise ausgewählt aus C(Ar')₃, C(R¹)₃, N(Ar')₂, Si(Ar')₃, Si(R¹)₃ umfasst, vorzugsweise eine Fluorengruppe, die mit einem oder mehreren Resten R¹ substituiert sein kann, darstellt, umfasst oder mit einem Rest R^{d} beziehungsweise R^{e} bildet.

Strukturen/Verbindungen mit einer der zuvor genannten Gruppen ausgewählt aus C(Ar')₃, C(R¹)₃, Si(Ar')₃, Si(R¹)₃, N(Ar')₂, N(R¹)₂, B(R¹)₂, besonders bevorzugt einer Fluorengruppe zeichnen sich durch eine überraschend hohe Effizienz aus.

Gemäß einer bevorzugten Ausgestaltung ist eine erfindungsgemäße Verbindung durch mindestens eine der Strukturen gemäß Formel (I) und/oder (I-1) bis (I-83) darstellbar. Vorzugsweise weisen erfindungsgemäße Verbindungen, bevorzugt umfassend Strukturen gemäß Formel (I) und/oder (I-1) bis (I-83) ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Bevorzugte aromatische bzw. heteroaromatische Ringsysteme Ar, R, R^{a}, R^{b}, R^{c}, R^{d}, R^{e}, R^{f} und/oder Ar'sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3-, 4- oder 9-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R^{e}, R¹ oder R² substituiert sein können.

Vorzugsweise kann vorgesehen sein, dass mindestens ein Substituent R, R^{a}, R^{b}, R^{c}, R^{d}, R^{e} gleich oder verschieden bei jedem Auftreten ausgewählt ist aus der Gruppe bestehend aus H, D, einer verzweigten oder cyclischen Alkyl-, Alkoxy- oder Thioalkoxygruppe mit 3 bis 20 C-Atomen oder einem aromatischen oder heteroaromatischen Ringsystem ausgewählt aus den Gruppen der folgenden Formeln Ar-1 bis Ar-75, wobei die Substituenten R, R^{a}, R^{b}, R^{c}, R^{d}, R^{e} vorzugsweise entweder einen Ring gemäß den Strukturen der Formeln (RA-1) bis (RA-13), (RA-1a) bis (RA-4f) oder (RB) bilden oder der Substituent R, R^{a}, R^{b}, R^{c}, R^{d}, R^{e} gleich oder verschieden bei jedem Auftreten ausgewählt ist aus der Gruppe bestehend aus H, D oder einem aromatischen oder heteroaromatischen Ringsystem ausgewählt aus den Gruppen der folgenden Formeln Ar-1 bis Ar-75, und/oder die Gruppe Ar' gleich oder verschieden bei jedem Auftreten ausgewählt ist aus den Gruppen der folgenden Formeln Ar-1 bis Ar-75, 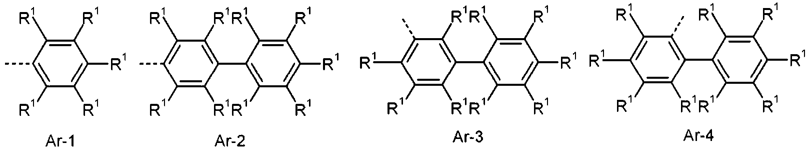 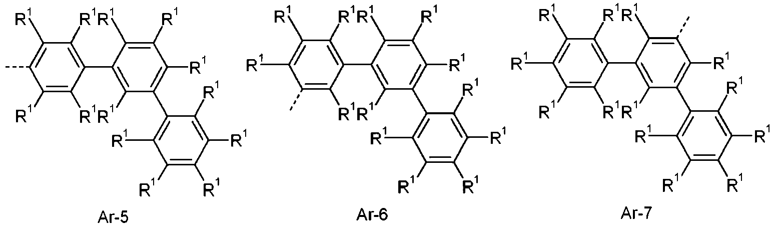 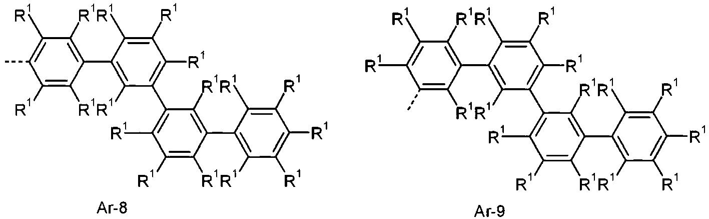 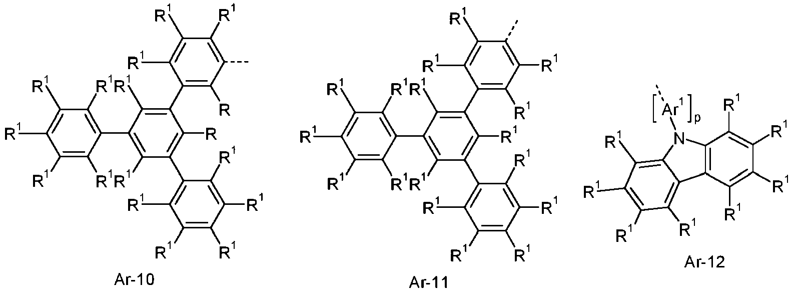 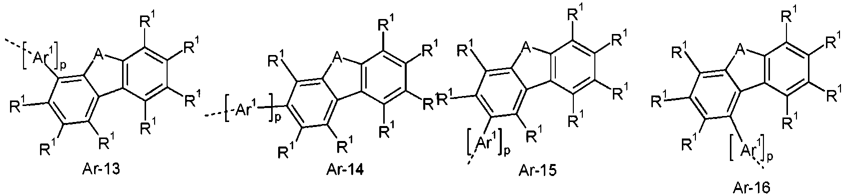 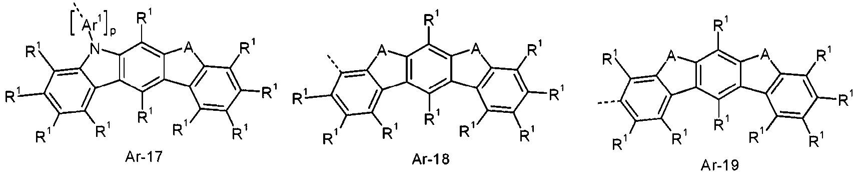 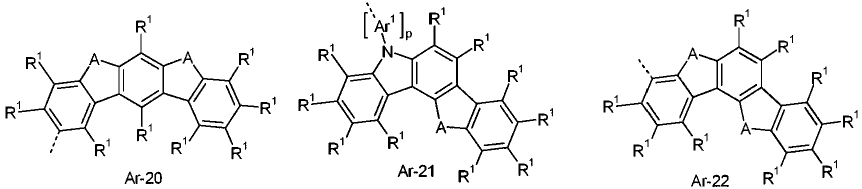 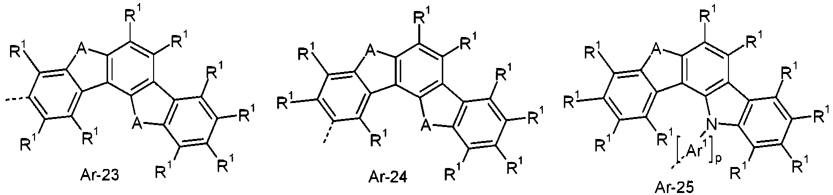 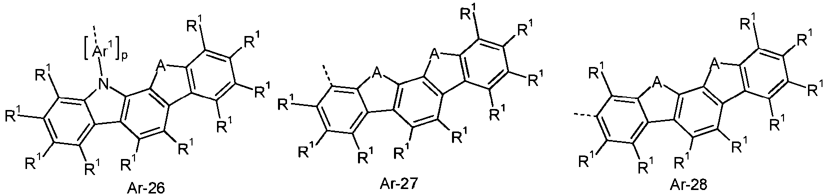 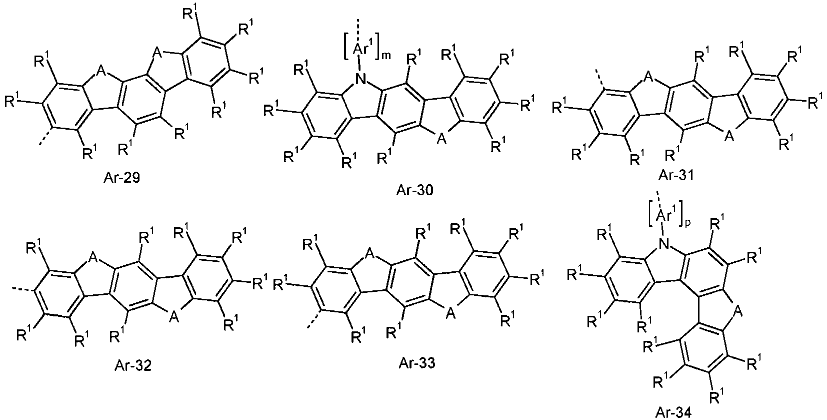 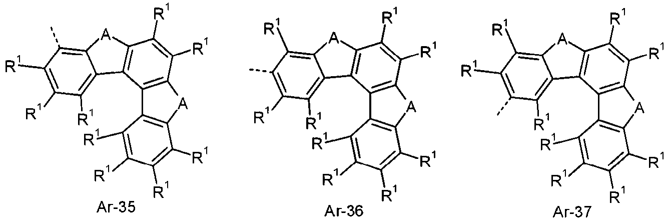 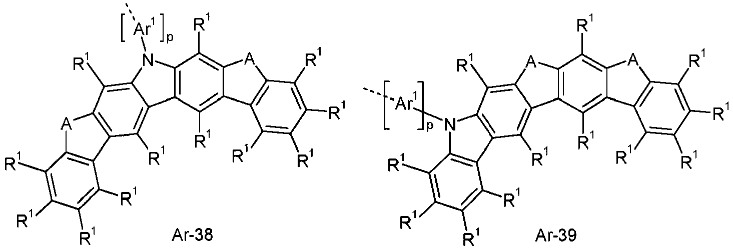 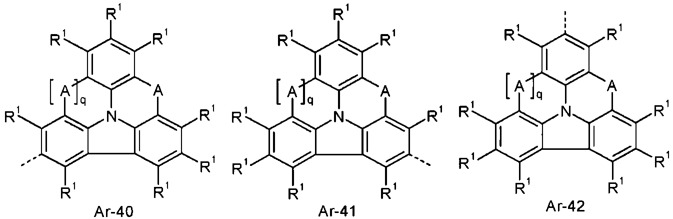 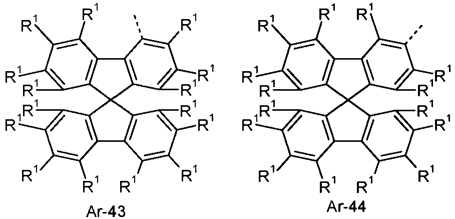 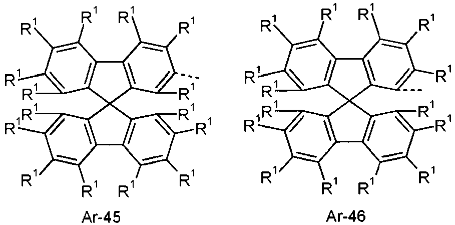 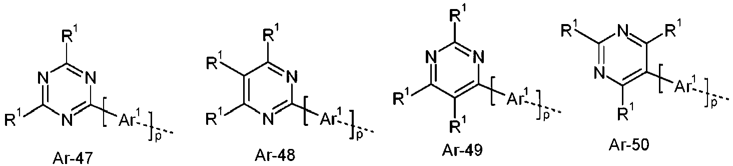 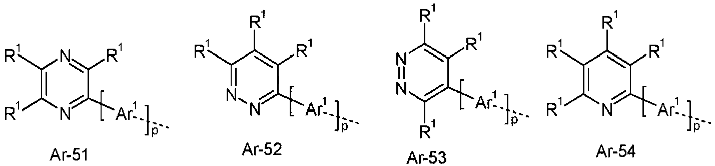 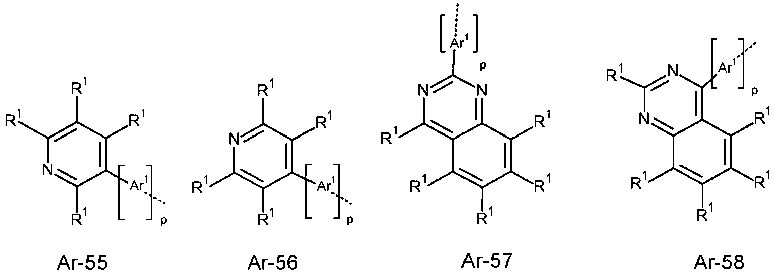 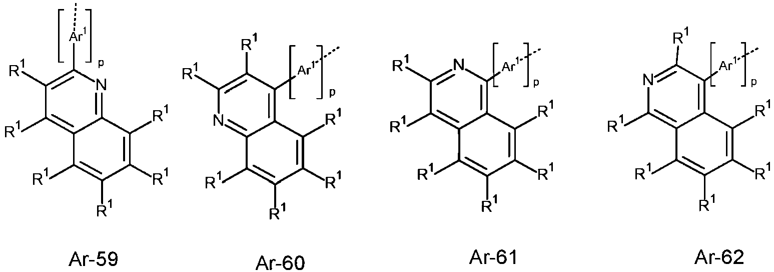 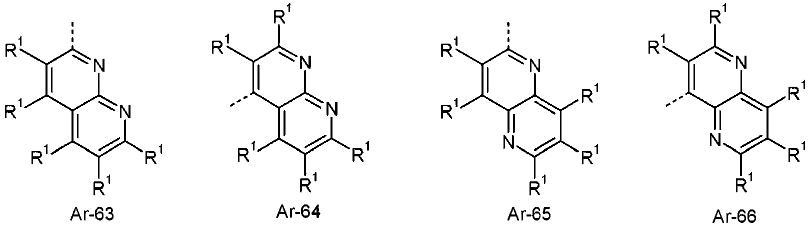 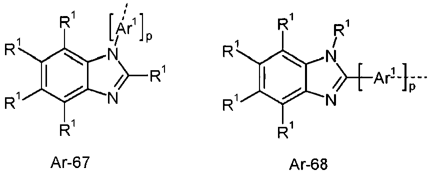 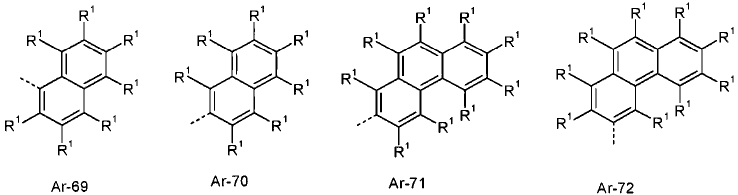 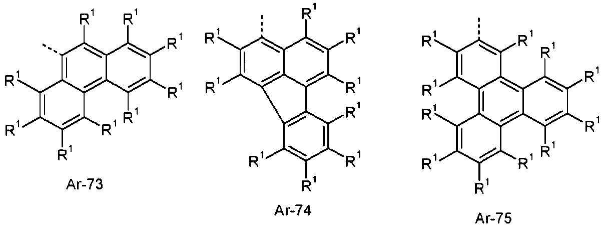 wobei R¹ die oben genannten Bedeutungen aufweist, die gestrichelte Bindung die Anbindungstelle an die entsprechende Gruppe darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)₂, NR¹, O oder S;
- p: ist 0 oder 1, wobei p = 0 bedeutet, dass die Gruppe Ar¹ nicht vorhanden ist und dass die entsprechende aromatische bzw. heteroaromatische Gruppe direkt den entsprechenden Rest gebunden ist;
- q: ist 0 oder 1, wobei q = 0 bedeutet, dass an dieser Position keine Gruppe A gebunden ist und an die entsprechenden Kohlenstoffatome statt dessen Reste R¹ gebunden sind.

Hierbei sind die Strukturen der Formeln (Ar-1), (Ar-2), (Ar-3), (Ar-12), (Ar-13), (Ar-14), (Ar-15), (Ar-16), (Ar-40), (Ar-41), (Ar-42), (Ar-43), (Ar-44), (Ar-45), (Ar-46), (Ar-69), (Ar-70), (Ar-75), bevorzugt und Strukturen der Formeln (Ar-1), (Ar-2), (Ar-3), (Ar-12), (Ar-13), (Ar-14), (Ar-15), (Ar-16) besonders bevorzugt.

Wenn die oben genannten Gruppen für Ar mehrere Gruppen A aufweisen, so kommen hierfür alle Kombinationen aus der Definition von A in Frage. Bevorzugte Ausführungsformen sind dann solche, in denen eine Gruppe A für NR¹ und die andere Gruppe A für C(R¹)₂ steht oder in denen beide Gruppen A für NR¹ stehen oder in denen beide Gruppen A für O stehen.

Wenn A für NR¹ steht, steht der Substituent R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen, welches keine kondensierten Arylgruppen aufweist und welches keine kondensierten Heteroarylgruppen, in denen zwei oder mehr aromatische bzw. heteroaromatische 6-RingGruppen direkt aneinander ankondensiert sind, aufweist, und welches jeweils auch durch einen oder mehrere Reste R² substituiert sein kann. Bevorzugt sind Phenyl, Biphenyl, Terphenyl und Quaterphenyl mit Verknüpfungsmustern, wie vorne für Ar-1 bis Ar-11 aufgeführt, wobei diese Strukturen statt durch R¹ durch einen oder mehrere Reste R² substituiert sein können, bevorzugt aber unsubstituiert sind. Bevorzugt sind weiterhin Triazin, Pyrimidin und Chinazolin, wie vorne für Ar-47 bis Ar-50, Ar-57 und Ar-58 aufgeführt, wobei diese Strukturen statt durch R¹ durch einen oder mehrere Reste R² substituiert sein können.

Im Folgenden werden bevorzugte Substituenten R, R^{a}, R^{b}, R^{c}, R^{d}, R^{e} und R^{f} beschrieben.

In einer bevorzugten Ausführungsform der Erfindung ist R, R^{a}, R^{b}, R^{c}, R^{d}, R^{e} gleich oder verschieden bei jedem Auftreten ausgewählt aus der Gruppe bestehend aus H, D, F, CN, NO₂, Si(R¹)₃, B(OR¹)₂, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R¹ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann.

In einer weiterhin bevorzugten Ausführungsform der Erfindung ist Substituent R, R^{a}, R^{b}, R^{c}, R^{d}, R^{e} gleich oder verschieden bei jedem Auftreten ausgewählt aus der Gruppe bestehend aus H, D, F, eine geradkettige Alkylgruppe mit 1 bis 20 C-Atomen oder eine verzweigte oder cyclische Alkylgruppe mit 3 bis 20 C-Atomen, wobei die Alkylgruppe jeweils mit einem oder mehreren Resten R¹ substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann.

Ferner kann vorgesehen sein, dass mindestens ein Substituent R, R^{a}, R^{b}, R^{c}, R^{d}, R^{e} gleich oder verschieden bei jedem Auftreten ausgewählt ist aus der Gruppe bestehend aus H, D, einem aromatischen oder heteroaromatischen Ringsystem mit 6 bis 30 aromatischen Ringatomen, welches mit einem oder mehreren Resten R¹ substituiert sein kann, oder einer Gruppe N(Ar')₂. In einer weiterhin bevorzugten Ausführungsform der Erfindung bilden die Substituenten R, R^{a}, R^{b}, R^{c}, R^{d}, R^{e} entweder einen Ring gemäß den Strukturen der Formeln (RA-1) bis (RA-13), (RA-1a) bis (RA-4f) oder (RB) oder R, R^{a}, R^{b}, R^{c}, R^{d}, R^{e} ist gleich oder verschieden bei jedem Auftreten ausgewählt aus der Gruppe bestehend aus H, D, einem aromatischen oder heteroaromatischen Ringsystem mit 6 bis 30 aromatischen Ringatomen, welches mit einem oder mehreren Resten R¹ substituiert sein kann, oder einer Gruppe N(Ar')₂. Besonders bevorzugt ist Substituent R, R^{a}, R^{b}, R^{c}, R^{d}, R^{e} gleich oder verschieden bei jedem Auftreten ausgewählt aus der Gruppe bestehend aus H oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R¹ substituiert sein kann.

In einer bevorzugten Ausführungsform der Erfindung ist R^{f} gleich oder verschieden bei jedem Auftreten ausgewählt aus der Gruppe bestehend aus einer geradkettigen Alkylgruppe mit 1 bis 20 C-Atomen oder einer verzweigten oder cyclischen Alkylgruppe mit 3 bis 20 C-Atomen, wobei die Alkylgruppe jeweils mit einem oder mehreren Resten R² substituiert sein kann, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R² substituiert sein kann.

In einer weiterhin bevorzugten Ausführungsform der Erfindung ist R^{f} gleich oder verschieden bei jedem Auftreten ausgewählt aus der Gruppe bestehend aus einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R² substituiert sein kann, einem aromatischen oder heteroaromatischen Ringsystem mit 6 bis 30 aromatischen Ringatomen, welches mit einem oder mehreren Resten R² substituiert sein kann. Besonders bevorzugt ist R^{a} gleich oder verschieden bei jedem Auftreten ausgewählt aus der Gruppe bestehend aus einer geradkettigen Alkylgruppe mit 1 bis 5 C-Atomen oder einer verzweigten oder cyclischen Alkylgruppe mit 3 bis 5 C-Atomen, wobei die Alkylgruppe jeweils mit einem oder mehreren Resten R² substituiert sein kann oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren Resten R² substituiert sein kann.

In einer bevorzugten Ausführungsform der Erfindung ist R^{f} bei jedem Auftreten gleich oder verschieden ausgewählt aus der Gruppe bestehend aus einer geradkettigen Alkylgruppe mit 1 bis 6 C-Atomen oder einer cyclischen Alkylgruppe mit 3 bis 6 C-Atomen, wobei die Alkylgruppe jeweils mit einem oder mehreren Resten R² substituiert sein kann, oder einem aromatischen oder heteroaromatischen Ringsystem mit 6 bis 24 aromatischen Ringatomen, das jeweils durch einen oder mehrere Reste R² substituiert sein kann; dabei können zwei Reste R^{f} auch miteinander ein Ringsystem bilden. Besonders bevorzugt ist R^{f} bei jedem Auftreten gleich oder verschieden ausgewählt aus der Gruppe bestehend aus einer geradkettigen Alkylgruppe mit 1, 2, 3 oder 4 C-Atomen oder einer verzweigten oder cyclischen Alkylgruppe mit 3 bis 6 C-Atomen, wobei die Alkylgruppe jeweils mit einem oder mehreren Resten R² substituiert sein kann, bevorzugt aber unsubstituiert ist, oder einem aromatischen Ringsystem mit 6 bis 12 aromatischen Ringatomen, insbesondere mit 6 aromatischen Ringatomen, das jeweils durch einen oder mehrere, bevorzugt nicht-aromatische Reste R² substituiert sein kann, bevorzugt aber unsubstituiert ist; dabei können zwei Reste R^{f} miteinander ein Ringsystem bilden. Ganz besonders bevorzugt ist R^{f} bei jedem Auftreten gleich oder verschieden ausgewählt aus der Gruppe bestehend aus einer geradkettigen Alkylgruppe mit 1, 2, 3 oder 4 C-Atomen, oder einer verzweigten Alkylgruppe mit 3 bis 6 C-Atomen. Ganz besonders bevorzugt steht R^{f} für eine Methylgruppe oder für eine Phenylgruppe, wobei zwei Phenylgruppen zusammen ein Ringsystem bilden können, wobei eine Methylgruppe gegenüber einer Phenylgruppe bevorzugt ist.

Bevorzugte aromatische bzw. heteroaromatische Ringsysteme für die die Substituenten R, R^{a}, R^{b}, R^{c}, R^{d}, R^{e}, R^{f} beziehungsweise Ar, Ar' oder Ar" stehen, sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R^{e}, R¹ beziehungsweise R² substituiert sein können. Besonders bevorzugt sind die oben aufgeführten Strukturen Ar-1 bis Ar-75, wobei Strukturen der Formeln (Ar-1), (Ar-2), (Ar-3), (Ar-12), (Ar-13), (Ar-14), (Ar-15), (Ar-16), (Ar-40), (Ar-41), (Ar-42), (Ar-43), (Ar-44), (Ar-45), (Ar-46), (Ar-69), (Ar-70), (Ar-75), bevorzugt und Strukturen der Formeln (Ar-1), (Ar-2), (Ar-3), (Ar-12), (Ar-13), (Ar-14), (Ar-15), (Ar-16) besonders bevorzugt sind. Hinsichtlich der Strukturen Ar-1 bis Ar-75 ist festzuhalten, dass diese mit einem Substituenten R¹ dargestellt sind. Im Falle des Ringsystems Ar sind diese Substituenten R¹ durch R^{e} und im Falle Ar", R^{f} sind diese Substituenten R¹ durch R² zu ersetzen.

Weitere geeignete Gruppen R, R^{a}, R^{b}, R^{c}, R^{d}, R^{e} sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Dabei beträgt die Gesamtzahl der aromatischen Ringatome von Ar², Ar³ und Ar⁴ maximal 60 und bevorzugt maximal 40.

Dabei können Ar⁴ und Ar² miteinander und/oder Ar² und Ar³ miteinander auch durch eine Gruppe ausgewählt aus C(R¹)₂, NR¹, O oder S verbunden sein. Bevorzugt erfolgt die Verknüpfung von Ar⁴ und Ar² miteinander bzw. von Ar² und Ar³ miteinander jeweils ortho zur Position der Verknüpfung mit dem Stickstoffatom. In einer weiteren Ausführungsform der Erfindung sind keine der Gruppen Ar², Ar³ bzw. Ar⁴ miteinander verbunden.

Bevorzugt ist Ar⁴ ein aromatisches oder heteroaromatisches Ringsystem mit 6 bis 24 aromatischen Ringatomen, bevorzugt mit 6 bis 12 aromatischen Ringatomen, das jeweils mit einem oder mehreren Resten R¹ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁴ eine unsubstituierte Phenylengruppe.

Bevorzugt sind Ar² und Ar³ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar² bzw. Ar³ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren oder Triphenylen, welche jeweils mit einem oder mehreren Resten R¹ substituiert sein können. Ganz besonders bevorzugt sind Ar² und Ar³ gleich oder verschieden bei jedem Auftreten ausgewählt aus der Gruppe bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

In einer weiteren bevorzugten Ausführungsform der Erfindung ist R¹ gleich oder verschieden bei jedem Auftreten ausgewählt aus der Gruppe bestehend aus H, D, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppe jeweils mit einem oder mehreren Resten R² substituiert sein kann, oder einem aromatischen oder heteroaromatischen Ringsystem mit 6 bis 24 aromatischen Ringatomen, das jeweils durch einen oder mehrere Reste R² substituiert sein kann. In einer besonders bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R² substituiert sein kann, bevorzugt aber unsubstituiert ist, oder einem aromatischen oder heteroaromatischen Ringsystem mit 6 bis 13 aromatischen Ringatomen, das jeweils durch einen oder mehrere Reste R² substituiert sein kann, bevorzugt aber unsubstituiert ist.

In einer weiteren bevorzugten Ausführungsform der Erfindung ist R² gleich oder verschieden bei jedem Auftreten H, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Dabei haben in erfindungsgemäßen Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Ferner kann vorgesehen sein, dass die Verbindung genau zwei oder genau drei Strukturen gemäß Formel (I) und/oder (I-1) bis (I-83) umfasst, wobei vorzugsweise eines der aromatischen oder heteroaromatischen Ringsysteme, die durch die mindestens einer der Gruppen R, R^{d}, R^{e} darstellbar ist oder an das die Gruppen Gruppen R, R^{d}, R^{e} binden, von beiden Strukturen geteilt wird.

In einer bevorzugten Ausgestaltung sind die Verbindungen ausgewählt aus Verbindungen gemäß der Formel (D-1), (D2) oder (D-3), 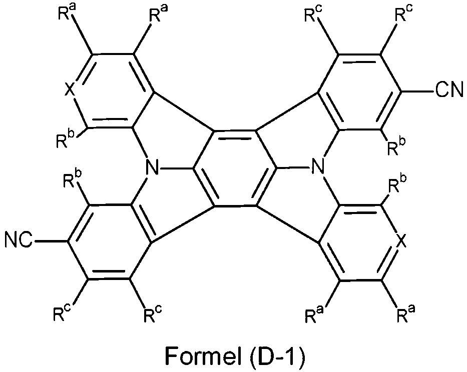 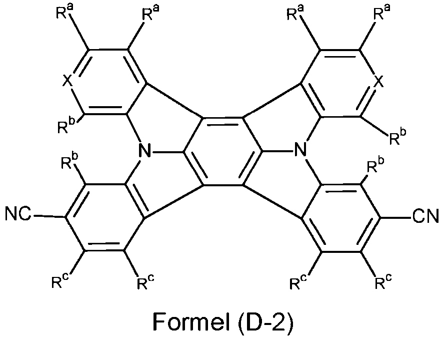 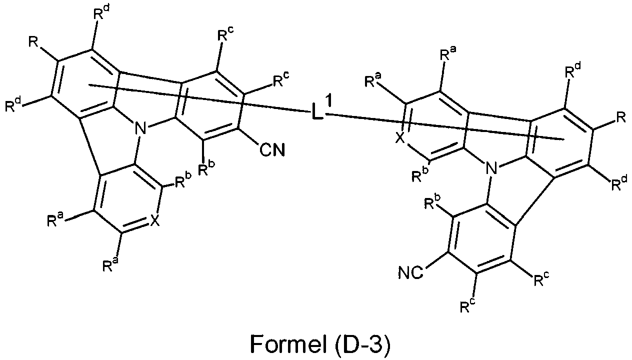 wobei die Gruppe L¹ eine Verbindungsgruppe, vorzugsweise eine Bindung oder ein aromatisches oder heteroaromatisches Ringsystem mit 5 bis 40, bevorzugt 5 bis 30 aromatischen Ringatomen darstellt, welches durch einen oder mehrere Reste R¹ substituiert sein kann, und R¹ sowie die weiteren verwendeten Symbole die zuvor, insbesondere für Formel (I) genannten Bedeutungen aufweisen.

In einer weiteren bevorzugten Ausführungsform der Erfindung steht L¹ für eine Bindung oder für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste R¹ substituiert sein kann, bevorzugt aber unsubstituiert ist, wobei R¹ die zuvor, insbesondere für Formel (I) genannte Bedeutung aufweisen kann. Besonders bevorzugt steht L¹ für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste R² substituiert sein kann, bevorzugt aber unsubstituiert ist, wobei R² die zuvor, insbesondere für Formel (I) genannte Bedeutung aufweisen kann.

Weiterhin bevorzugt steht das unter anderem in Formel (D3) dargelegte Symbol L¹ gleich oder verschieden bei jedem Auftreten für eine Bindung oder einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die in Formel (D3) dargelegte Gruppe L¹ ein aromatisches Ringsystem mit höchstens zwei kondensierten aromatischen und/oder heteroaromatischen 6-Ringen, vorzugsweise kein kondensiertes aromatisches oder heteroaromatisches Ringsystem umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme L¹ sind ausgewählt aus der Gruppe bestehend aus ortho-, meta- oder para-Phenylen, ortho-, meta- oder para-Biphenylen, Terphenylen, insbesondere verzweigtes Terphenylen, Quaterphenylen, insbesondere verzweigtes Quaterphenylen, Fluorenylen, Spirobifluorenylen, Dibenzofuranylen, Dibenzothienylen und Carbazolylen, die jeweils durch einen oder mehrere Reste R¹ substituiert sein können, bevorzugt aber unsubstituiert sind.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

In einer weiteren Ausgestaltung der vorliegenden Erfindung sind Verbindungen umfassend eine Struktur gemäß Formel (I), vorzugsweise Verbindungen gemäß Formel (I) bevorzugt, bei denen die Reste R^{a} zusammen einen Ring bilden, wobei diese Verbindungen die folgenden Eigenschaften aufweisen:

| Formel des Ringes, die Reste R^{a} bilden | Z¹ | Z² | Z³ |
|---|---|---|---|
| Cy-1 | C(R³)₂ | C(R¹)₂ | C(R³)₂ |
| Cy-2 | C(R³)₂ | C(R¹)₂ | C(R³)₂ |
| Cy-3 | C(R³)₂ | C(R¹)₂ | C(R³)₂ |
| Cy-1 | Si(R³)₂ | C(R¹)₂ | Si(R³)₂ |
| Cy-2 | Si(R³)₂ | C(R¹)₂ | Si(R³)₂ |
| Cy-3 | Si(R³)₂ | C(R¹)₂ | Si(R³)₂ |

In einer weiteren Ausgestaltung der vorliegenden Erfindung sind Verbindungen umfassend eine Struktur gemäß Formel (I), vorzugsweise Verbindungen gemäß Formel (I) bevorzugt, bei denen die Reste R^{a} zusammen einen Ring bilden, wobei diese Verbindungen die folgenden Eigenschaften aufweisen:

| Formel des Ringes, die Reste R^{a} bilden | G | R¹ | Z² |
|---|---|---|---|
| Cy-4 | Alkylengruppe mit 1, 2 oder 3 C-Atomen | H, Methyl oder Ar-1 bis Ar-75, vorzugsweise H | C(R¹ )₂ |
| Cy-5 | Alkylengruppe mit 1, 2 oder 3 C-Atomen | H, Methyl oder Ar-1 bis Ar-75, vorzugsweise H | C(R¹)₂ |
| Cy-6 | Alkylengruppe mit 1, 2 oder 3 C-Atomen | H, Methyl oder Ar-1 bis Ar-75, vorzugsweise H | C(R¹)₂ |
| Cy-7 | Alkylengruppe mit 1, 2 oder 3 C-Atomen | H, Methyl oder Ar-1 bis Ar-75, vorzugsweise H | C(R¹)₂ |
| Cy-8 | Alkylengruppe mit 1, 2 oder 3 C-Atomen | H oder Ar-1 bis H, Methyl oder Ar-1 bis Ar-75, vorzugsweise H | C(R¹)₂ |
| Cy-9 | Alkylengruppe mit 1, 2 oder 3 C-Atomen | H, Methyl oder Ar-1 bis Ar-75, vorzugsweise H | C(R¹)₂ |
| Cy-10 | Alkylengruppe mit 1, 2 oder 3 C-Atomen | H, Methyl oder Ar-1 bis Ar-75, vorzugsweise H | C(R¹)₂ |
| Cy-4 | -CR¹=CR¹- | H, Methyl oder Ar-1 bis Ar-75, vorzugsweise H | C(R¹)₂ |
| Cy-5 | -CR¹=CR¹- | H, Methyl oder Ar-1 bis Ar-75, vorzugsweise H | C(R¹)₂ |
| Cy-6 | -CR¹=CR¹- | H, Methyl oder Ar-1 bis Ar-75, vorzugsweise H | C(R¹)₂ |
| Cy-7 | -CR¹=CR¹- | H, Methyl oder Ar-1 bis Ar-75, vorzugsweise H | C(R¹)₂ |
| Cy-8 | -CR¹=CR¹- | H oder Ar-1 bis H, Methyl oder Ar-1 bis Ar-75, vorzugsweise H | C(R¹)₂ |
| Cy-9 | -CR¹=CR¹- | H, Methyl oder Ar-1 bis Ar-75, vorzugsweise H | C(R¹)₂ |
| Cy-10 | -CR¹=CR¹- | H, Methyl oder Ar-1 bis Ar-75, vorzugsweise H | C(R¹)₂ |
| Cy-4 | Arylen- oder Heteroarylengruppe mit 5 bis 14 aromatischen Ringatomen | H, Methyl oder Ar-1 bis Ar-75, vorzugsweise H | C(R¹)₂ |
| Cy-5 | Arylen- oder Heteroarylengruppe mit 5 bis 14 aromatischen Ringatomen | H, Methyl oder Ar-1 bis Ar-75, vorzugsweise H | C(R¹)₂ |
| Cy-6 | Arylen- oder Heteroarylengruppe mit 5 bis 14 aromatischen Ringatomen | H, Methyl oder Ar-1 bis Ar-75, vorzugsweise H | C(R¹)₂ |
| Cy-7 | Arylen- oder Heteroarylengruppe mit 5 bis 14 aromatischen Ringatomen | H, Methyl oder Ar-1 bis Ar-75, vorzugsweise H | C(R¹)₂ |
| Cy-8 | Arylen- oder Heteroarylengruppe mit 5 bis 14 aromatischen Ringatomen | H oder Ar-1 bis H, Methyl oder Ar-1 bis Ar-75, vorzugsweise H | C(R¹)₂ |
| Cy-9 | Arylen- oder Heteroarylengruppe mit 5 bis 14 aromatischen Ringatomen | H, Methyl oder Ar-1 bis Ar-75, vorzugsweise H | C(R¹)₂ |
| Cy-10 | Arylen- oder Heteroarylengruppe mit 5 bis 14 aromatischen Ringatomen | H, Methyl oder Ar-1 bis Ar-75, vorzugsweise H | C(R¹)₂ |

In einer weiteren Ausgestaltung gelten die zuvor dargelegten Bevorzugungen hinsichtlich einer Ringbildung zweier Reste R^{a} zu Strukturen der Formeln (Cy-1) bis (Cy-10) für zwei Reste R^{c}.

In einer weiteren Ausgestaltung gelten die zuvor dargelegten Bevorzugungen hinsichtlich einer Ringbildung zweier Reste R^{a} zu Strukturen der Formeln (Cy-1) bis (Cy-10) für zwei Reste R^{e}.

In einer weiteren Ausgestaltung der vorliegenden Erfindung sind Verbindungen umfassend eine Struktur gemäß Formel (I-1), vorzugsweise Verbindungen gemäß Formel (I-1) bevorzugt, wobei die beiden Reste R^{a} einen Ring bilden, die beiden Reste R^{c} einen Ring bilden und bei denen die Reste R^{a}, R^{b}, R^{c}, R^{d} und R^{e} folgenden Bedeutungen aufweisen:

| R^{a} | R^{b} | R^{c} | R^{d} | R^{e} |
|---|---|---|---|---|
| RA-5 | H, D, Alkyl | RA-5 | H, D, Alkyl | H, D, Alkyl |
| RA-4 | H, D, Alkyl | RA-4 | H, D, Alkyl | H, D, Alkyl |
| RA-4f | H, D, Alkyl | RA-4f | H, D, Alkyl | H, D, Alkyl |
| RA-3 | H, D, Alkyl | RA-3 | H, D, Alkyl | H, D, Alkyl |
| RB | H, D, Alkyl | RB | H, D, Alkyl | H, D, Alkyl |
| RA-5 | H, D, Alkyl | RA-5 | H, D, Alkyl und Phenyl-Ringbildung mit R^{e} | Phenyl-Ringbildung mit R^{d} |
| RA-5 | H, D, Alkyl | RA-5 | Aryl-, Heteroaryl und Phenyl-Ringbildung mit R^{e} | Phenyl-Ringbildung mit R^{d} |
| RA-4 | H, D, Alkyl | RA-4 | H, D, Alkyl und Phenyl-Ringbildung mit R^{e} | Phenyl-Ringbildung mit R^{d} |
| RA-4 | H, D, Alkyl | RA-4 | Aryl-, Heteroaryl und Phenyl-Ringbildung mit R^{e} | Phenyl-Ringbildung mit R^{d} |
| RA-4f | H, D, Alkyl | RA-4f | H, D, Alkyl und Phenyl-Ringbildung mit R^{e} | Phenyl-Ringbildung mit R^{d} |
| RA-4f | H, D, Alkyl | RA-4f | Aryl-, Heteroaryl und Phenyl-Ringbildung mit R^{e} | Phenyl-Ringbildung mit R^{d} |
| RA-3 | H, D, Alkyl | RA-3 | H, D, Alkyl und Phenyl-Ringbildung mit R^{e} | Phenyl-Ringbildung mit R^{d} |
| RA-3 | H, D, Alkyl | RA-3 | Aryl-, Heteroaryl und Phenyl-Ringbildung mit R^{e} | Phenyl-Ringbildung mit R^{d} |
| RB | H, D, Alkyl | RB | H, D, Alkyl und Phenyl-Ringbildung mit R^{e} | Phenyl-Ringbildung mit R^{d} |
| RB | H, D, Alkyl | RB | Aryl-, Heteroaryl und Phenyl-Ringbildung mit R^{e} | Phenyl-Ringbildung mit R^{d} |
| RA-5 | H, D, Alkyl | RA-5 | H, D, Alkyl und Heteroaryl-Ringbildung mit R^{e} | Heteroaryl-Ringbildung mit R^{d} |
| RA-5 | H, D, Alkyl | RA-5 | Aryl-, Heteroaryl und Heteroaryl-Ringbildung mit R^{e} | Heteroaryl-Ringbildung mit R^{d} |
| RA-4 | H, D, Alkyl | RA-4 | H, D, Alkyl und Heteroaryl-Ringbildung mit R^{e} | Heteroaryl-Ringbildung mit R^{d} |
| RA-4 | H, D, Alkyl | RA-4 | Aryl-, Heteroaryl und Heteroaryl-Ringbildung mit R^{e} | **Heteroaryl-**Ringbildung mit R^{d} |
| RA-4f | H, D, Alkyl | RA-4f | H, D, Alkyl und Heteroaryl-Ringbildung mit R^{e} | Heteroaryl-Ringbildung mit R^{d} |
| RA-4f | H, D, Alkyl | RA-4f | Aryl-, Heteroaryl und Heteroaryl-Ringbildung mit R^{e} | Heteroaryl-Ringbildung mit R^{d} |
| RA-3 | H, D, Alkyl | RA-3 | H, D, Alkyl und Heteroaryl-Ringbildung mit R^{e} | Heteroaryl-Ringbildung mit R^{d} |
| RA-3 | H, D, Alkyl | RA-3 | Aryl-, Heteroaryl und Heteroaryl-Ringbildung mit R^{e} | Heteroaryl-Ringbildung mit R^{d} |
| RB | H, D, Alkyl | RB | H, D, Alkyl und Heteroaryl-Ringbildung mit R^{e} | Heteroaryl-Ringbildung mit R^{d} |
| RB | H, D, Alkyl | RB | Aryl-, Heteroaryl und Heteroaryl-Ringbildung mit R^{e} | Heteroaryl-Ringbildung mit R^{d} |
| RA-5 | H, D, Alkyl | RA-5 | H, D, Alkyl | C(Ar')₃, Si(Ar')₃ |
| RA-4 | H, D, Alkyl | RA-4 | H, D, Alkyl | C(Ar')₃, Si(Ar')₃ |
| RA-4f | H, D, Alkyl | RA-4f | H, D, Alkyl | C(Ar')₃, Si(Ar')₃ |
| RA-3 | H, D, Alkyl | RA-3 | H, D, Alkyl | C(Ar')₃, Si(Ar')₃ |
| RB | H, D, Alkyl | RB | H, D, Alkyl | C(Ar')₃, Si(Ar')₃ |
| RA-5 | H, D, Alkyl | RA-5 | H, D, Alkyl und Ringbildung mit R^{e} | C(Ar')₃, Si(Ar')₃ |
| RA-4 | H, D, Alkyl | RA-4 | H, D, Alkyl und Ringbildung mit R^{e} | C(Ar')₃, Si(Ar')₃ |
| RA-4f | H, D, Alkyl | RA-4f | H, D, Alkyl und Ringbildung mit R^{e} | C(Ar')₃, Si(Ar')₃ |
| RA-3 | H, D, Alkyl | RA-3 | H, D, Alkyl und Ringbildung mit R^{e} | C(Ar')₃, Si(Ar')₃ |
| RB | H, D, Alkyl | RB | H, D, Alkyl und Ringbildung mit R^{e} | C(Ar')₃, Si(Ar')₃ |
| RA-5 | H, D, Alkyl | RA-5 | H, D, Alkyl | N(Ar')₃, N(R¹)₃ |
| RA-4 | H, D, Alkyl | RA-4 | H, D, Alkyl | N(Ar')₃, N(R¹)₃ |
| RA-4f | H, D, Alkyl | RA-4f | H, D, Alkyl | N(Ar')₃, N(R¹)₃ |
| RA-3 | H, D, Alkyl | RA-3 | H, D, Alkyl | N(Ar')₃, N(R¹)₃ |
| RB | H, D, Alkyl | RB | H, D, Alkyl | N(Ar')₃, N(R¹)₃ |
| RA-5 | H, D, Alkyl | RA-5 | H, D, Alkyl und Ringbildung mit R^{e} | N(Ar')₃, N(R¹)₃ |
| RA-4 | H, D, Alkyl | RA-4 | H, D, Alkyl und Ringbildung mit R^{e} | N(Ar')₃, N(R¹)₃ |
| RA-4f | H, D, Alkyl | RA-4f | H, D, Alkyl und Ringbildung mit R^{e} | N(Ar')₃, N(R¹)₃ |
| RA-3 | H, D, Alkyl | RA-3 | H, D, Alkyl und Ringbildung mit R^{e} | N(Ar')₃, N(R¹)₃ |
| RB | H, D, Alkyl | RB | H, D, Alkyl und Ringbildung mit R^{e} | N(Ar')₃, N(R¹)₃ |
| RA-5 | H, D, Alkyl | RA-5 | H, D, Alkyl und RA-5-Ringbildung mit R^{e} | RA-5-Ringbildung mit R^{d} |
| RA-4 | H, D, Alkyl | RA-4 | H, D, Alkyl und RA-4-Ringbildung mit R^{e} | RA-4-Ringbildung mit R^{d} |
| RA-4f | H, D, Alkyl | RA-4f | H, D, Alkyl und RA-4f-Ringbildung mit R^{e} | RA-4f-Ringbildung mit R^{d} |
| RA-3 | H, D, Alkyl | RA-3 | H, D, Alkyl und RA-3-Ringbildung mit R^{e} | RA-3-Ringbildung mit R^{d} |
| RB | H, D, Alkyl | RB | H, D, Alkyl und RB -Ringbildung mit R^{e} | RB-Ringbildung mit R^{d} |
| RA-5 | H, D, Alkyl | RA-5 | H, D, Alkyl | Ar-1 bis Ar-75 |
| RA-4 | H, D, Alkyl | RA-4 | H, D, Alkyl | Ar-1 bis Ar-75 |
| RA-4f | H, D, Alkyl | RA-4f | H, D, Alkyl | Ar-1 bis Ar-75 |
| RA-3 | H, D, Alkyl | RA-3 | H, D, Alkyl | Ar-1 bis Ar-75 |
| RB | H, D, Alkyl | RB | H, D, Alkyl | Ar-1 bis Ar-75 |
| RA-5 | H, D, Alkyl | RA-5 | RA-2, RA-2c | RA-2, RA-2c |
| RA-4 | H, D, Alkyl | RA-4 | RA-2, RA-2c | RA-2, RA-2c |
| RA-4f | H, D, Alkyl | RA-4f | RA-2, RA-2c | RA-2, RA-2c |
| RA-3 | H, D, Alkyl | RA-3 | RA-2, RA-2c | RA-2, RA-2c |
| RB | H, D, Alkyl | RB | RA-2, RA-2c | RA-2, RA-2c |

In einer weiteren Ausgestaltung der vorliegenden Erfindung sind Verbindungen umfassend eine Struktur gemäß Formel (I-14), vorzugsweise Verbindungen gemäß Formel (I-14) bevorzugt, wobei die beiden Reste R^{a} einen Ring bilden, die beiden Reste R^{c} einen Ring bilden und bei denen die Reste R^{a}, R^{b}, R^{c}, R^{d} und R^{e} folgenden Bedeutungen aufweisen:

| R^{a} | alle R^{b} | R^{c} | R^{d} | R^{e} |
|---|---|---|---|---|
| RA-5 | H, D, Alkyl | RA-5 | H, D, Alkyl | H, D, Alkyl |
| RA-4 | H, D, Alkyl | RA-4 | H, D, Alkyl | H, D, Alkyl |
| RA-4f | H, D, Alkyl | RA-4f | H, D, Alkyl | H, D, Alkyl |
| RA-3 | H, D, Alkyl | RA-3 | H, D, Alkyl | H, D, Alkyl |
| RB | H, D, Alkyl | RB | H, D, Alkyl | H, D, Alkyl |
| RA-5 | H, D, Alkyl | RA-5 | H, D, Alkyl und Phenyl-Ringbildung mit R^{e} | Phenyl-Ringbildung mit R^{d} |
| RA-5 | H, D, Alkyl | RA-5 | Aryl-, Heteroaryl und Phenyl-Ringbildung mit R^{e} | Phenyl-Ringbildung mit R^{d} |
| RA-4 | H, D, Alkyl | RA-4 | H, D, Alkyl und Phenyl-Ringbildung mit R^{e} | Phenyl-Ringbildung mit R^{d} |
| RA-4 | H, D, Alkyl | RA-4 | Aryl-, Heteroaryl und Phenyl-Ringbildung mit R^{e} | Phenyl-Ringbildung mit R^{d} |
| RA-4f | H, D, Alkyl | RA-4f | H, D, Alkyl und Phenyl-Ringbildung mit R^{e} | Phenyl-Ringbildung mit R^{d} |
| RA-4f | H, D, Alkyl | RA-4f | Aryl-, Heteroaryl und Phenyl-Ringbildung mit R^{e} | Phenyl-Ringbildung mit R^{d} |
| RA-3 | H, D, Alkyl | RA-3 | H, D, Alkyl und Phenyl-Ringbildung mit R^{e} | Phenyl-Ringbildung mit R^{d} |
| RA-3 | H, D, Alkyl | RA-3 | Aryl-, Heteroaryl und Phenyl-Ringbildung mit R^{e} | Phenyl-Ringbildung mit R^{d} |
| RB | H, D, Alkyl | RB | H, D, Alkyl und Phenyl-Ringbildung mit R^{e} | Phenyl-Ringbildung mit R^{d} |
| RB | H, D, Alkyl | RB | Aryl-, Heteroaryl und Phenyl-Ringbildung mit R^{e} | Phenyl-Ringbildung mit R^{d} |
| RA-5 | H, D, Alkyl | RA-5 | H, D, Alkyl und Heteroaryl-Ringbildung mit R^{e} | Heteroaryl-Ringbildung mit R^{d} |
| RA-5 | H, D, Alkyl | RA-5 | Aryl-, Heteroaryl und Heteroaryl-Ringbildung mit R^{e} | Heteroaryl-Ringbildung mit R^{d} |
| RA-4 | H, D, Alkyl | RA-4 | H, D, Alkyl und Heteroaryl-Ringbildung mit R^{e} | Heteroaryl-Ringbildung mit R^{d} |
| RA-4 | H, D, Alkyl | RA-4 | Aryl-, Heteroaryl und Heteroaryl-Ringbildung mit R^{e} | Heteroaryl-Ringbildung mit R^{d} |
| RA-4f | H, D, Alkyl | RA-4f | H, D, Alkyl und Heteroaryl-Ringbildung mit R^{e} | Heteroaryl-Ringbildung mit R^{d} |
| RA-4f | H, D, Alkyl | RA-4f | Aryl-, Heteroaryl und Heteroaryl-Ringbildung mit R^{e} | Heteroaryl-Ringbildung mit R^{d} |
| RA-3 | H, D, Alkyl | RA-3 | H, D, Alkyl und Heteroaryl-Ringbildung mit R^{e} | Heteroaryl-Ringbildung mit R^{d} |
| RA-3 | H, D, Alkyl | RA-3 | Aryl-, Heteroaryl und Heteroaryl-Ringbildung mit R^{e} | Heteroaryl-Ringbildung mit R^{d} |
| RB | H, D, Alkyl | RB | H, D, Alkyl und Heteroaryl-Ringbildung mit R^{e} | Heteroaryl-Ringbildung mit R^{d} |
| RB | H, D, Alkyl | RB | Aryl-, Heteroaryl und Heteroaryl-Ringbildung mit R^{e} | Heteroaryl-Ringbildung mit R^{d} |
| RA-5 | H, D, Alkyl | RA-5 | H, D, Alkyl | C(Ar')₃, Si(Ar')₃ |
| RA-4 | H, D, Alkyl | RA-4 | H, D, Alkyl | C(Ar')₃, Si(Ar')₃ |
| RA-4f | H, D, Alkyl | RA-4f | H, D, Alkyl | C(Ar')₃, Si(Ar')₃ |
| RA-3 | H, D, Alkyl | RA-3 | H, D, Alkyl | C(Ar')₃, Si(Ar')₃ |
| RB | H, D, Alkyl | RB | H, D, Alkyl | C(Ar')₃, Si(Ar')₃ |
| RA-5 | H, D, Alkyl | RA-5 | H, D, Alkyl und Ringbildung mit R^{e} | C(Ar')₃, Si(Ar')₃ |
| RA-4 | H, D, Alkyl | RA-4 | H, D, Alkyl und Ringbildung mit R^{e} | C(Ar')₃, Si(Ar')₃ |
| RA-4f | H, D, Alkyl | RA-4f | H, D, Alkyl und Ringbildung mit R^{e} | C(Ar')₃, Si(Ar')₃ |
| RA-3 | H, D, Alkyl | RA-3 | H, D, Alkyl und Ringbildung mit R^{e} | C(Ar')₃, Si(Ar')₃ |
| RB | H, D, Alkyl | RB | H, D, Alkyl und Ringbildung mit R^{e} | C(Ar')₃, Si(Ar')₃ |
| RA-5 | H, D, Alkyl | RA-5 | H, D, Alkyl | N(Ar')₃, N(R¹)₃ |
| RA-4 | H, D, Alkyl | RA-4 | H, D, Alkyl | N(Ar')₃, N(R¹)₃ |
| RA-4f | H, D, Alkyl | RA-4f | H, D, Alkyl | N(Ar')₃, N(R¹)₃ |
| RA-3 | H, D, Alkyl | RA-3 | H, D, Alkyl | N(Ar')₃, N(R¹)₃ |
| RB | H, D, Alkyl | RB | H, D, Alkyl | N(Ar')₃, N(R¹)₃ |
| RA-5 | H, D, Alkyl | RA-5 | H, D, Alkyl und Ringbildung mit R^{e} | N(Ar')₃, N(R¹)₃ |
| RA-4 | H, D, Alkyl | RA-4 | H, D, Alkyl und Ringbildung mit R^{e} | N(Ar')₃, N(R¹)₃ |
| RA-4f | H, D, Alkyl | RA-4f | H, D, Alkyl und Ringbildung mit R^{e} | N(Ar')₃, N(R¹)₃ |
| RA-3 | H, D, Alkyl | RA-3 | H, D, Alkyl und Ringbildung mit R^{e} | N(Ar')₃, N(R¹)₃ |
| RB | H, D, Alkyl | RB | H, D, Alkyl und Ringbildung mit R^{e} | N(Ar')₃, N(R¹)₃ |
| RA-5 | H, D, Alkyl | RA-5 | H, D, Alkyl und RA-5-Ringbildung mit R^{e} | RA-5-Ringbildung mit R^{d} |
| RA-4 | H, D, Alkyl | RA-4 | H, D, Alkyl und RA-4-Ringbildung mit R^{e} | RA-4-Ringbildung mit R^{d} |
| RA-4f | H, D, Alkyl | RA-4f | H, D, Alkyl und RA-4f-Ringbildung mit R^{e} | RA-4f-Ringbildung mit R^{d} |
| RA-3 | H, D, Alkyl | RA-3 | H, D, Alkyl und RA-3-Ringbildung mit R^{e} | RA-3-Ringbildung mit R^{d} |
| RB | H, D, Alkyl | RB | H, D, Alkyl und RB -Ringbildung mit R^{e} | RB-Ringbildung mit R^{d} |
| RA-5 | H, D, Alkyl | RA-5 | H, D, Alkyl | Ar-1 bis Ar-75 |
| RA-4 | H, D, Alkyl | RA-4 | H, D, Alkyl | Ar-1 bis Ar-75 |
| RA-4f | H, D, Alkyl | RA-4f | H, D, Alkyl | Ar-1 bis Ar-75 |
| RA-3 | H, D, Alkyl | RA-3 | H, D, Alkyl | Ar-1 bis Ar-75 |
| RB | H, D, Alkyl | RB | H, D, Alkyl | Ar-1 bis Ar-75 |
| RA-5 | H, D, Alkyl | RA-5 | RA-2, RA-2c | RA-2, RA-2c |
| RA-4 | H, D, Alkyl | RA-4 | RA-2, RA-2c | RA-2, RA-2c |
| RA-4f | H, D, Alkyl | RA-4f | RA-2, RA-2c | RA-2, RA-2c |
| RA-3 | H, D, Alkyl | RA-3 | RA-2, RA-2c | RA-2, RA-2c |
| RB | H, D, Alkyl | RB | RA-2, RA-2c | RA-2, RA-2c |

In einer weiteren Ausgestaltung der vorliegenden Erfindung sind Verbindungen umfassend eine Struktur gemäß Formel (I-2), vorzugsweise Verbindungen gemäß Formel (I-2) bevorzugt, wobei die beiden Reste R^{a} einen Ring bilden, die beiden Reste R^{c} einen Ring bilden, wobei der Index I vorzugsweise kleiner oder gleich 3, besonders bevorzugt 0, 1 oder 2 und speziell bevorzugt 0 oder 1 ist, und bei denen die Reste R^{a}, R^{b}, R^{c}, R^{d} und R^{e} folgenden Bedeutungen aufweisen:

| R^{a} | R^{b} | R^{c} | R^{d} | R^{e} (nur falls I ungleich 0 ist mindestens ein Rest R^{e}, andernfalls sind alle R^{e} H) |
|---|---|---|---|---|
| RA-5 | H, D, Alkyl | RA-5 | H, D, Alkyl | D, Alkyl |
| RA-4 | H, D, Alkyl | RA-4 | H, D, Alkyl | D, Alkyl |
| RA-4f | H, D, Alkyl | RA-4f | H, D, Alkyl | D, Alkyl |
| RA-3 | H, D, Alkyl | RA-3 | H, D, Alkyl | D, Alkyl |
| RB | H, D, Alkyl | RB | H, D, Alkyl | D, Alkyl |
| RA-5 | H, D, Alkyl | RA-5 | H, D, Alkyl | Ar-1 bis Ar-75 |
| RA-4 | H, D, Alkyl | RA-4 | H, D, Alkyl | Ar-1 bis Ar-75 |
| RA-4f | H, D, Alkyl | RA-4f | H, D, Alkyl | Ar-1 bis Ar-75 |
| RA-3 | H, D, Alkyl | RA-3 | H, D, Alkyl | Ar-1 bis Ar-75 |
| RB | H, D, Alkyl | RB | H, D, Alkyl | Ar-1 bis Ar-75 |
| RA-5 | H, D, Alkyl | RA-5 | Ar-1 bis Ar-75 | D, Alkyl |
| RA-4 | H, D, Alkyl | RA-4 | Ar-1 bis Ar-75 | D, Alkyl |
| RA-4f | H, D, Alkyl | RA-4f | Ar-1 bis Ar-75 | D, Alkyl |
| RA-3 | H, D, Alkyl | RA-3 | Ar-1 bis Ar-75 | D, Alkyl |
| RB | H, D, Alkyl | RB | Ar-1 bis Ar-75 | D, Alkyl |
| RA-5 | H, D, Alkyl | RA-5 | Ar-1 bis Ar-75 | Ar-1 bis Ar-75 |
| RA-4 | H, D, Alkyl | RA-4 | Ar-1 bis Ar-75 | Ar-1 bis Ar-75 |
| RA-4f | H, D, Alkyl | RA-4f | Ar-1 bis Ar-75 | Ar-1 bis Ar-75 |
| RA-3 | H, D, Alkyl | RA-3 | Ar-1 bis Ar-75 | Ar-1 bis Ar-75 |
| RB | H, D, Alkyl | RB | Ar-1 bis Ar-75 | Ar-1 bis Ar-75 |
| RA-5 | H, D, Alkyl | RA-5 | H, D, Alkyl und Ar-1 bis Ar-75 | D, Alkyl |
| RA-4 | H, D, Alkyl | RA-4 | H, D, Alkyl und Ar-1 bis Ar-75 | D, Alkyl |
| RA-4f | H, D, Alkyl | RA-4f | H, D, Alkyl und Ar-1 bis Ar-75 | D, Alkyl |
| RA-3 | H, D, Alkyl | RA-3 | H, D, Alkyl und Ar-1 bis Ar-75 | D, Alkyl |
| RB | H, D, Alkyl | RB | H, D, Alkyl und Ar-1 bis Ar-75 | D, Alkyl |
| RA-5 | H, D, Alkyl | RA-5 | H, D, Alkyl und Ar-1 bis Ar-75 | Ar-1 bis Ar-75 |
| RA-4 | H, D, Alkyl | RA-4 | H, D, Alkyl und Ar-1 bis Ar-75 | Ar-1 bis Ar-75 |
| RA-4f | H, D, Alkyl | RA-4f | H, D, Alkyl und Ar-1 bis Ar-75 | Ar-1 bis Ar-75 |
| RA-3 | H, D, Alkyl | RA-3 | H, D, Alkyl und Ar-1 bis Ar-75 | Ar-1 bis Ar-75 |
| RB | H, D, Alkyl | RB | H, D, Alkyl und Ar-1 bis Ar-75 | Ar-1 bis Ar-75 |
| RA-5 | H, D, Alkyl | RA-5 | H, D, Alkyl | C(Ar')₃, Si(Ar')₃ |
| RA-4 | H, D, Alkyl | RA-4 | H, D, Alkyl | C(Ar')₃, Si(Ar')₃ |
| RA-4f | H, D, Alkyl | RA-4f | H, D, Alkyl | C(Ar')₃, Si(Ar')₃ |
| RA-3 | H, D, Alkyl | RA-3 | H, D, Alkyl | C(Ar')₃, Si(Ar')₃ |
| RB | H, D, Alkyl | RB | H, D, Alkyl | C(Ar')₃, Si(Ar')₃ |
| RA-5 | H, D, Alkyl | RA-5 | Ar-1 bis Ar-75 | C(Ar')₃, Si(Ar')₃ |
| RA-4 | H, D, Alkyl | RA-4 | Ar-1 bis Ar-75 | C(Ar')₃, Si(Ar')₃ |
| RA-4f | H, D, Alkyl | RA-4f | Ar-1 bis Ar-75 | C(Ar')₃, Si(Ar')₃ |
| RA-3 | H, D, Alkyl | RA-3 | Ar-1 bis Ar-75 | C(Ar')₃, Si(Ar')₃ |
| RB | H, D, Alkyl | RB | Ar-1 bis Ar-75 | C(Ar')₃, Si(Ar')₃ |
| RA-5 | H, D, Alkyl | RA-5 | H, D, Alkyl und Ar-1 bis Ar-75 | C(Ar')₃, Si(Ar')₃ |
| RA-4 | H, D, Alkyl | RA-4 | H, D, Alkyl und Ar-1 bis Ar-75 | C(Ar')₃, Si(Ar')₃ |
| RA-4f | H, D, Alkyl | RA-4f | H, D, Alkyl und Ar-1 bis Ar-75 | C(Ar')₃, Si(Ar')₃ |
| RA-3 | H, D, Alkyl | RA-3 | H, D, Alkyl und Ar-1 bis Ar-75 | C(Ar')₃, Si(Ar')₃ |
| RB | H, D, Alkyl | RB | H, D, Alkyl und Ar-1 bis Ar-75 | C(Ar')₃, Si(Ar')₃ |
| RA-5 | H, D, Alkyl | RA-5 | H, D, Alkyl | N(Ar')₃, N(R¹)₃ |
| RA-4 | H, D, Alkyl | RA-4 | H, D, Alkyl | N(Ar')₃, N(R¹)₃ |
| RA-4f | H, D, Alkyl | RA-4f | H, D, Alkyl | N(Ar')₃, N(R¹)₃ |
| RA-3 | H, D, Alkyl | RA-3 | H, D, Alkyl | N(Ar')₃, N(R¹)₃ |
| RB | H, D, Alkyl | RB | H, D, Alkyl | N(Ar')₃, N(R¹)₃ |
| RA-5 | H, D, Alkyl | RA-5 | Ar-1 bis Ar-75 | N(Ar')₃, N(R¹)₃ |
| RA-4 | H, D, Alkyl | RA-4 | Ar-1 bis Ar-75 | N(Ar')₃, N(R¹)₃ |
| RA-4f | H, D, Alkyl | RA-4f | Ar-1 bis Ar-75 | N(Ar')₃, N(R¹)₃ |
| RA-3 | H, D, Alkyl | RA-3 | Ar-1 bis Ar-75 | N(Ar')₃, N(R¹)₃ |
| RB | H, D, Alkyl | RB | Ar-1 bis Ar-75 | N(Ar')₃, N(R¹)₃ |
| RA-5 | H, D, Alkyl | RA-5 | H, D, Alkyl und Ar-1 bis Ar-75 | N(Ar')₃, N(R¹)₃ |
| RA-4 | H, D, Alkyl | RA-4 | H, D, Alkyl und Ar-1 bis Ar-75 | N(Ar')₃, N(R¹)₃ |
| RA-4f | H, D, Alkyl | RA-4f | H, D, Alkyl und Ar-1 bis Ar-75 | N(Ar')₃, N(R¹)₃ |
| RA-3 | H, D, Alkyl | RA-3 | H, D, Alkyl und Ar-1 bis Ar-75 | N(Ar')₃, N(R¹)₃ |
| RB | H, D, Alkyl | RB | H, D, Alkyl und Ar-1 bis Ar-75 | N(Ar')₃, N(R¹)₃ |

In einer weiteren Ausgestaltung der vorliegenden Erfindung sind Verbindungen umfassend eine Struktur gemäß Formel (I-3), vorzugsweise Verbindungen gemäß Formel (I-3) bevorzugt, wobei die beiden Reste R^{a} einen Ring bilden, die beiden Reste R^{c} einen Ring bilden, wobei der Index m vorzugsweise 0, 1 oder 2 und besonders bevorzugt 0 oder 1 ist, und bei denen Rb H, D, Alkyl ist und die Reste R^{a}, R^{c}, R^{d}, R^{e} und Y¹ folgenden Bedeutungen aufweisen:

| R^{a} | R^{c} | R^{d} | R^{e} am Phenylring (nur falls m ungleich 0 ist mindestens ein Rest R^{e}, andernfalls sind alle R^{e} H) | Y¹ |
|---|---|---|---|---|
| RA-5 | RA-5 | H, D, Alkyl | D, Alkyl | C(R^{e})₂ |
| RA-4 | RA-4 | H, D, Alkyl | D, Alkyl | C(R^{e})₂ |
| RA-4f | RA-4f | H, D, Alkyl | D, Alkyl | C(R^{e})₂ |
| RA-3 | RA-3 | H, D, Alkyl | D, Alkyl | C(R^{e})₂ |
| RB | RB | H, D, Alkyl | D, Alkyl | C(R^{e})₂ |
| RA-5 | RA-5 | H, D, Alkyl | Ar-1 bis Ar-75 | C(R^{e})₂ |
| RA-4 | RA-4 | H, D, Alkyl | Ar-1 bis Ar-75 | C(R^{e})₂ |
| RA-4f | RA-4f | H, D, Alkyl | Ar-1 bis Ar-75 | C(R^{e})₂ |
| RA-3 | RA-3 | H, D, Alkyl | Ar-1 bis Ar-75 | C(R^{e})₂ |
| RB | RB | H, D, Alkyl | Ar-1 bis Ar-75 | C(R^{e})₂ |
| RA-5 | RA-5 | Ar-1 bis Ar-75 | D, Alkyl | C(R^{e})₂ |
| RA-4 | RA-4 | Ar-1 bis Ar-75 | D, Alkyl | C(R^{e})₂ |
| RA-4f | RA-4f | Ar-1 bis Ar-75 | D, Alkyl | C(R^{e})₂ |
| RA-3 | RA-3 | Ar-1 bis Ar-75 | D, Alkyl | C(R^{e})₂ |
| RB | RB | Ar-1 bis Ar-75 | D, Alkyl | C(R^{e})₂ |
| RA-5 | RA-5 | Ar-1 bis Ar-75 | Ar-1 bis Ar-75 | C(R^{e})₂ |
| RA-4 | RA-4 | Ar-1 bis Ar-75 | Ar-1 bis Ar-75 | C(R^{e})₂ |
| RA-4f | RA-4f | Ar-1 bis Ar-75 | Ar-1 bis Ar-75 | C(R^{e})₂ |
| RA-3 | RA-3 | Ar-1 bis Ar-75 | Ar-1 bis Ar-75 | C(R^{e})₂ |
| RB | RB | Ar-1 bis Ar-75 | Ar-1 bis Ar-75 | C(R^{e})₂ |
| RA-5 | RA-5 | H, D, Alkyl | Phenyl-Ringbildung von 2 R^{e} | C(R^{e})₂ |
| RA-4 | RA-4 | H, D, Alkyl | Phenyl-Ringbildung von 2 R^{e} | C(R^{e})₂ |
| RA-4f | RA-4f | H, D, Alkyl | Phenyl-Ringbildung von 2 R^{e} | C(R^{e})₂ |
| RA-3 | RA-3 | H, D, Alkyl | Phenyl-Ringbildung von 2 R^{e} | C(R^{e})₂ |
| RB | RB | H, D, Alkyl | Phenyl-Ringbildung von 2 R^{e} | C(R^{e})₂ |
| RA-5 | RA-5 | Ar-1 bis Ar-75 | Phenyl-Ringbildung von 2 R^{e} | C(R^{e})₂ |
| RA-4 | RA-4 | Ar-1 bis Ar-75 | Phenyl-Ringbildung von 2 R^{e} | C(R^{e})₂ |
| RA-4f | RA-4f | Ar-1 bis Ar-75 | Phenyl-Ringbildung von 2 R^{e} | C(R^{e})₂ |
| RA-3 | RA-3 | Ar-1 bis Ar-75 | Phenyl-Ringbildung von 2 R^{e} | C(R^{e})₂ |
| RB | RB | Ar-1 bis Ar-75 | Phenyl-Ringbildung von 2 R^{e} | C(R^{e})₂ |
| RA-5 | RA-5 | H, D, Alkyl | C(Ar')₃, Si(Ar')₃ | C(R^{e})₂ |
| RA-4 | RA-4 | H, D, Alkyl | C(Ar')₃, Si(Ar')₃ | C(R^{e})₂ |
| RA-4f | RA-4f | H, D, Alkyl | C(Ar')₃, Si(Ar')₃ | C(R^{e})₂ |
| RA-3 | RA-3 | H, D, Alkyl | C(Ar')₃, Si(Ar')₃ | C(R^{e})₂ |
| RB | RB | H, D, Alkyl | C(Ar')₃, Si(Ar')₃ | C(R^{e})₂ |
| RA-5 | RA-5 | Ar-1 bis Ar-75 | C(Ar')₃, Si(Ar')₃ | C(R^{e})₂ |
| RA-4 | RA-4 | Ar-1 bis Ar-75 | C(Ar')₃, Si(Ar')₃ | C(R^{e})₂ |
| RA-4f | RA-4f | Ar-1 bis Ar-75 | C(Ar')₃, Si(Ar')₃ | C(R^{e})₂ |
| RA-3 | RA-3 | Ar-1 bis Ar-75 | C(Ar')₃, Si(Ar')₃ | C(R^{e})₂ |
| RB | RB | Ar-1 bis Ar-75 | C(Ar')₃, Si(Ar')₃ | C(R^{e})₂ |
| RA-5 | RA-5 | H, D, Alkyl | N(Ar')₃, N(R¹)₃ | C(R^{e})₂ |
| RA-4 | RA-4 | H, D, Alkyl | N(Ar')₃, N(R¹)₃ | C(R^{e})₂ |
| RA-4f | RA-4f | H, D, Alkyl | N(Ar')₃, N(R¹)₃ | C(R^{e})₂ |
| RA-3 | RA-3 | H, D, Alkyl | N(Ar')₃, N(R¹)₃ | C(R^{e})₂ |
| RB | RB | H, D, Alkyl | N(Ar')₃, N(R¹)₃ | C(R^{e})₂ |
| RA-5 | RA-5 | Ar-1 bis Ar-75 | N(Ar')₃, N(R¹)₃ | C(R^{e})₂ |
| RA-4 | RA-4 | Ar-1 bis Ar-75 | N(Ar')₃, N(R¹)₃ | C(R^{e})₂ |
| RA-4f | RA-4f | Ar-1 bis Ar-75 | N(Ar')₃, N(R¹)₃ | C(R^{e})₂ |
| RA-3 | RA-3 | Ar-1 bis Ar-75 | N(Ar')₃, N(R¹)₃ | C(R^{e})₂ |
| RB | RB | Ar-1 bis Ar-75 | N(Ar')₃, N(R¹)₃ | C(R^{e})₂ |
| RA-5 | RA-5 | H, D, Alkyl | D, Alkyl | O, S |
| RA-4 | RA-4 | H, D, Alkyl | D, Alkyl | O, S |
| RA-4f | RA-4f | H, D, Alkyl | D, Alkyl | O, S |
| RA-3 | RA-3 | H, D, Alkyl | D, Alkyl | O, S |
| RB | RB | H, D, Alkyl | D, Alkyl | O, S |
| RA-5 | RA-5 | H, D, Alkyl | Ar-1 bis Ar-75 | O, S |
| RA-4 | RA-4 | H, D, Alkyl | Ar-1 bis Ar-75 | O, S |
| RA-4f | RA-4f | H, D, Alkyl | Ar-1 bis Ar-75 | O, S |
| RA-3 | RA-3 | H, D, Alkyl | Ar-1 bis Ar-75 | O, S |
| RB | RB | H, D, Alkyl | Ar-1 bis Ar-75 | O, S |
| RA-5 | RA-5 | Ar-1 bis Ar-75 | D, Alkyl | O, S |
| RA-4 | RA-4 | Ar-1 bis Ar-75 | D, Alkyl | O, S |
| RA-4f | RA-4f | Ar-1 bis Ar-75 | D, Alkyl | O, S |
| RA-3 | RA-3 | Ar-1 bis Ar-75 | D, Alkyl | O, S |
| RB | RB | Ar-1 bis Ar-75 | D, Alkyl | O, S |
| RA-5 | RA-5 | Ar-1 bis Ar-75 | Ar-1 bis Ar-75 | O, S |
| RA-4 | RA-4 | Ar-1 bis Ar-75 | Ar-1 bis Ar-75 | O, S |
| RA-4f | RA-4f | Ar-1 bis Ar-75 | Ar-1 bis Ar-75 | O, S |
| RA-3 | RA-3 | Ar-1 bis Ar-75 | Ar-1 bis Ar-75 | O, S |
| RB | RB | Ar-1 bis Ar-75 | Ar-1 bis Ar-75 | O, S |
| RA-5 | RA-5 | H, D, Alkyl | Phenyl-Ringbildung von 2 R^{e} | O, S |
| RA-4 | RA-4 | H, D, Alkyl | Phenyl-Ringbildung von 2 R^{e} | O, S |
| RA-4f | RA-4f | H, D, Alkyl | Phenyl-Ringbildung von 2 R^{e} | O, S |
| RA-3 | RA-3 | H, D, Alkyl | Phenyl-Ringbildung von 2 R^{e} | O, S |
| RB | RB | H, D, Alkyl | Phenyl-Ringbildung von 2 R^{e} | O, S |
| RA-5 | RA-5 | Ar-1 bis Ar-75 | Phenyl-Ringbildung von 2 R^{e} | O, S |
| RA-4 | RA-4 | Ar-1 bis Ar-75 | Phenyl-Ringbildung von 2 R^{e} | O, S |
| RA-4f | RA-4f | Ar-1 bis Ar-75 | Phenyl-Ringbildung von 2 R^{e} | O, S |
| RA-3 | RA-3 | Ar-1 bis Ar-75 | Phenyl-Ringbildung von 2 R^{e} | O, S |
| RB | RB | Ar-1 bis Ar-75 | Phenyl-Ringbildung von 2 R^{e} | O, S |
| RA-5 | RA-5 | H, D, Alkyl | C(Ar')₃, Si(Ar')₃ | O, S |
| RA-4 | RA-4 | H, D, Alkyl | C(Ar')₃, Si(Ar')₃ | O, S |
| RA-4f | RA-4f | H, D, Alkyl | C(Ar')₃, Si(Ar')₃ | O, S |
| RA-3 | RA-3 | H, D, Alkyl | C(Ar')₃, Si(Ar')₃ | O, S |
| RB | RB | H, D, Alkyl | C(Ar')₃, Si(Ar')₃ | O, S |
| RA-5 | RA-5 | Ar-1 bis Ar-75 | C(Ar')₃, Si(Ar')₃ | O, S |
| RA-4 | RA-4 | Ar-1 bis Ar-75 | C(Ar')₃, Si(Ar')₃ | O, S |
| RA-4f | RA-4f | Ar-1 bis Ar-75 | C(Ar')₃, Si(Ar')₃ | O, S |
| RA-3 | RA-3 | Ar-1 bis Ar-75 | C(Ar')₃, Si(Ar')₃ | O, S |
| RB | RB | Ar-1 bis Ar-75 | C(Ar')₃, Si(Ar')₃ | O, S |
| RA-5 | RA-5 | H, D, Alkyl | N(Ar')₃, N(R¹)₃ | O, S |
| RA-4 | RA-4 | H, D, Alkyl | N(Ar')₃, N(R¹)₃ | O, S |
| RA-4f | RA-4f | H, D, Alkyl | N(Ar')₃, N(R¹)₃ | O, S |
| RA-3 | RA-3 | H, D, Alkyl | N(Ar')₃, N(R¹)₃ | O, S |
| RB | RB | H, D, Alkyl | N(Ar')₃, N(R¹)₃ | O, S |
| RA-5 | RA-5 | Ar-1 bis Ar-75 | N(Ar')₃, N(R¹)₃ | O, S |
| RA-4 | RA-4 | Ar-1 bis Ar-75 | N(Ar')₃, N(R¹)₃ | O, S |
| RA-4f | RA-4f | Ar-1 bis Ar-75 | N(Ar')₃, N(R¹)₃ | O, S |
| RA-3 | RA-3 | Ar-1 bis Ar-75 | N(Ar')₃, N(R¹)₃ | O, S |
| RB | RB | Ar-1 bis Ar-75 | N(Ar')₃, N(R¹)₃ | O, S |
| RA-5 | RA-5 | H, D, Alkyl | D, Alkyl | N(Ar') |
| RA-4 | RA-4 | H, D, Alkyl | D, Alkyl | N(Ar') |
| RA-4f | RA-4f | H, D, Alkyl | D, Alkyl | N(Ar') |
| RA-3 | RA-3 | H, D, Alkyl | D, Alkyl | N(Ar') |
| RB | RB | H, D, Alkyl | D, Alkyl | N(Ar') |
| RA-5 | RA-5 | H, D, Alkyl | Ar-1 bis Ar-75 | N(Ar') |
| RA-4 | RA-4 | H, D, Alkyl | Ar-1 bis Ar-75 | N(Ar') |
| RA-4f | RA-4f | H, D, Alkyl | Ar-1 bis Ar-75 | N(Ar') |
| RA-3 | RA-3 | H, D, Alkyl | Ar-1 bis Ar-75 | N(Ar') |
| RB | RB | H, D, Alkyl | Ar-1 bis Ar-75 | N(Ar') |
| RA-5 | RA-5 | Ar-1 bis Ar-75 | D, Alkyl | N(Ar') |
| RA-4 | RA-4 | Ar-1 bis Ar-75 | D, Alkyl | N(Ar') |
| RA-4f | RA-4f | Ar-1 bis Ar-75 | D, Alkyl | N(Ar') |
| RA-3 | RA-3 | Ar-1 bis Ar-75 | D, Alkyl | N(Ar') |
| RB | RB | Ar-1 bis Ar-75 | D, Alkyl | N(Ar') |
| RA-5 | RA-5 | Ar-1 bis Ar-75 | Ar-1 bis Ar-75 | N(Ar') |
| RA-4 | RA-4 | Ar-1 bis Ar-75 | Ar-1 bis Ar-75 | N(Ar') |
| RA-4f | RA-4f | Ar-1 bis Ar-75 | Ar-1 bis Ar-75 | N(Ar') |
| RA-3 | RA-3 | Ar-1 bis Ar-75 | Ar-1 bis Ar-75 | N(Ar') |
| RB | RB | Ar-1 bis Ar-75 | Ar-1 bis Ar-75 | N(Ar') |
| RA-5 | RA-5 | H, D, Alkyl | Phenyl-Ringbildung von 2 R^{e} | N(Ar') |
| RA-4 | RA-4 | H, D, Alkyl | Phenyl-Ringbildung von 2 R^{e} | N(Ar') |
| RA-4f | RA-4f | H, D, Alkyl | Phenyl-Ringbildung von 2 R^{e} | N(Ar') |
| RA-3 | RA-3 | H, D, Alkyl | Phenyl-Ringbildung von 2 R^{e} | N(Ar') |
| RB | RB | H, D, Alkyl | Phenyl-Ringbildung von 2 R^{e} | N(Ar') |
| RA-5 | RA-5 | Ar-1 bis Ar-75 | Phenyl-Ringbildung von 2 R^{e} | N(Ar') |
| RA-4 | RA-4 | Ar-1 bis Ar-75 | Phenyl-Ringbildung von 2 R^{e} | N(Ar') |
| RA-4f | RA-4f | Ar-1 bis Ar-75 | Phenyl-Ringbildung von 2 R^{e} | N(Ar') |
| RA-3 | RA-3 | Ar-1 bis Ar-75 | Phenyl-Ringbildung von 2 R^{e} | N(Ar') |
| RB | RB | Ar-1 bis Ar-75 | Phenyl-Ringbildung von 2 R^{e} | N(Ar') |
| RA-5 | RA-5 | H, D, Alkyl | C(Ar')₃, Si(Ar')₃ | N(Ar') |
| RA-4 | RA-4 | H, D, Alkyl | C(Ar')₃, Si(Ar')₃ | N(Ar') |
| RA-4f | RA-4f | H, D, Alkyl | C(Ar')₃, Si(Ar')₃ | N(Ar') |
| RA-3 | RA-3 | H, D, Alkyl | C(Ar')₃, Si(Ar')₃ | N(Ar') |
| RB | RB | H, D, Alkyl | C(Ar')₃, Si(Ar')₃ | N(Ar') |
| RA-5 | RA-5 | Ar-1 bis Ar-75 | C(Ar')₃, Si(Ar')₃ | N(Ar') |
| RA-4 | RA-4 | Ar-1 bis Ar-75 | C(Ar')₃, Si(Ar')₃ | N(Ar') |
| RA-4f | RA-4f | Ar-1 bis Ar-75 | C(Ar')₃, Si(Ar')₃ | N(Ar') |
| RA-3 | RA-3 | Ar-1 bis Ar-75 | C(Ar')₃, Si(Ar')₃ | N(Ar') |
| RB | RB | Ar-1 bis Ar-75 | C(Ar')₃, Si(Ar')₃ | N(Ar') |
| RA-5 | RA-5 | H, D, Alkyl | N(Ar')₃, N(R¹)₃ | N(Ar') |
| RA-4 | RA-4 | H, D, Alkyl | N(Ar')₃, N(R¹)₃ | N(Ar') |
| RA-4f | RA-4f | H, D, Alkyl | N(Ar')₃, N(R¹)₃ | N(Ar') |
| RA-3 | RA-3 | H, D, Alkyl | N(Ar')₃, N(R¹)₃ | N(Ar') |
| RB | RB | H, D, Alkyl | N(Ar')₃, N(R¹)₃ | N(Ar') |
| RA-5 | RA-5 | Ar-1 bis Ar-75 | N(Ar')₃, N(R¹)₃ | N(Ar') |
| RA-4 | RA-4 | Ar-1 bis Ar-75 | N(Ar')₃, N(R¹)₃ | N(Ar') |
| RA-4f | RA-4f | Ar-1 bis Ar-75 | N(Ar')₃, N(R¹)₃ | N(Ar') |
| RA-3 | RA-3 | Ar-1 bis Ar-75 | N(Ar')₃, N(R¹)₃ | N(Ar') |
| RB | RB | Ar-1 bis Ar-75 | N(Ar')₃, N(R¹)₃ | N(Ar') |
| RA-5 | RA-5 | H, D, Alkyl | D, Alkyl | (R^{e})₂C-C(R^{e})₂, (R^{e})C=C(R^{e}) |
| RA-4 | RA-4 | H, D, Alkyl | D, Alkyl | (R^{e})₂C-C(R^{e})₂, (R^{e})C=C(R^{e}) |
| RA-4f | RA-4f | H, D, Alkyl | D, Alkyl | (R^{e})₂C-C(R^{e})₂, (R^{e})C=C(R^{e}) |
| RA-3 | RA-3 | H, D, Alkyl | D, Alkyl | (R^{e})₂C-C(R^{e})₂, (R^{e})C=C(R^{e}) |
| RB | RB | H, D, Alkyl | D, Alkyl | (R^{e})₂C-C(R^{e})₂, (R^{e})C=C(R^{e}) |
| RA-5 | RA-5 | H, D, Alkyl | Ar-1 bis Ar-75 | (R^{e})₂C-C(R^{e})₂, (R^{e})C=C(R^{e}) |
| RA-4 | RA-4 | H, D, Alkyl | Ar-1 bis Ar-75 | (R^{e})₂C-C(R^{e})₂, (R^{e})C=C(R^{e}) |
| RA-4f | RA-4f | H, D, Alkyl | Ar-1 bis Ar-75 | (R^{e})₂C-C(R^{e})₂, (R^{e})C=C(R^{e}) |
| RA-3 | RA-3 | H, D, Alkyl | Ar-1 bis Ar-75 | (R^{e})₂C-C(R^{e})₂, (R^{e})C=C(R^{e}) |
| RB | RB | H, D, Alkyl | Ar-1 bis Ar-75 | (R^{e})₂C-C(R^{e})₂, (R^{e})C=C(R^{e}) |
| RA-5 | RA-5 | Ar-1 bis Ar-75 | D, Alkyl | (R^{e})₂C-C(R^{e})₂, (R^{e})C=C(R^{e}) |
| RA-4 | RA-4 | Ar-1 bis Ar-75 | D, Alkyl | (R^{e})₂C=C(R^{e})₂, (R^{e})C=C(R^{e}) |
| RA-4f | RA-4f | Ar-1 bis Ar-75 | D, Alkyl | (R^{e})₂C-C(R^{e})₂, (R^{e})C=C(R^{e}) |
| RA-3 | RA-3 | Ar-1 bis Ar-75 | D, Alkyl | (R^{e})₂C-C(R^{e})₂, (R^{e})C=C(R^{e}) |
| RB | RB | Ar-1 bis Ar-75 | D, Alkyl | (R^{e})₂C-C(R^{e})₂, (R^{e})C=C(R^{e}) |
| RA-5 | RA-5 | Ar-1 bis Ar-75 | Ar-1 bis Ar-75 | (R^{e})₂C-C(R^{e})₂, (R^{e})C=C(R^{e}) |
| RA-4 | RA-4 | Ar-1 bis Ar-75 | Ar-1 bis Ar-75 | (R^{e})₂C-C(R^{e})₂, (R^{e})C=C(R^{e}) |
| RA-4f | RA-4f | Ar-1 bis Ar-75 | Ar-1 bis Ar-75 | (R^{e})₂C-C(R^{e})₂, (R^{e})C=C(R^{e}) |
| RA-3 | RA-3 | Ar-1 bis Ar-75 | Ar-1 bis Ar-75 | (R^{e})₂C-C(R^{e})₂, (R^{e})C=C(R^{e}) |
| RB | RB | Ar-1 bis Ar-75 | Ar-1 bis Ar-75 | (R^{e})₂C-C(R^{e})₂, (R^{e})C=C(R^{e}) |
| RA-5 | RA-5 | H, D, Alkyl | Phenyl-Ringbildung von 2 R^{e} | (R^{e})₂C-C(R^{e})₂, (R^{e})C=C(R^{e}) |
| RA-4 | RA-4 | H, D, Alkyl | Phenyl-Ringbildung von 2 R^{e} | (R^{e})₂C-C(R^{e})₂, (R^{e})C=C(R^{e}) |
| RA-4f | RA-4f | H, D, Alkyl | Phenyl-Ringbildung von 2 R^{e} | (R^{e})₂C-C(R^{e})₂, (R^{e})C=C(R^{e}) |
| RA-3 | RA-3 | H, D, Alkyl | Phenyl-Ringbildung von 2 R^{e} | (R^{e})₂C-C(R^{e})₂, (R^{e})C=C(R^{e}) |
| RB | RB | H, D, Alkyl | Phenyl-Ringbildung von 2 R^{e} | (R^{e})₂C-C(R^{e})₂, (R^{e})C=C(R^{e}) |
| RA-5 | RA-5 | Ar-1 bis Ar-75 | Phenyl-Ringbildung von 2 R^{e} | (R^{e})₂C-C(R^{e})₂, (R^{e})C=C(R^{e}) |
| RA-4 | RA-4 | Ar-1 bis Ar-75 | Phenyl-Ringbildung von 2 R^{e} | (R^{e})₂C-C(R^{e})₂, (R^{e})C=C(R^{e}) |
| RA-4f | RA-4f | Ar-1 bis Ar-75 | Phenyl-Ringbildung von 2 R^{e} | (R^{e})₂C-C(R^{e})₂, (R^{e})C=C(R^{e}) |
| RA-3 | RA-3 | Ar-1 bis Ar-75 | Phenyl-Ringbildung von 2 R^{e} | (R^{e})₂C-C(R^{e})₂, (R^{e})C=C(R^{e}) |
| RB | RB | Ar-1 bis Ar-75 | Phenyl-Ringbildung von 2 R^{e} | (R^{e})₂C-C(R^{e})₂, (R^{e})C=C(R^{e}) |
| RA-5 | RA-5 | H, D, Alkyl | C(Ar')₃, Si(Ar')₃ | (R^{e})₂C-C(R^{e})₂, (R^{e})C=C(R^{e}) |
| RA-4 | RA-4 | H, D, Alkyl | C(Ar')₃, Si(Ar')₃ | (R^{e})₂C-C(R^{e})₂, (R^{e})C=C(R^{e}) |
| RA-4f | RA-4f | H, D, Alkyl | C(Ar')₃, Si(Ar')₃ | (R^{e})₂C-C(R^{e})₂, (R^{e})C=C(R^{e}) |
| RA-3 | RA-3 | H, D, Alkyl | C(Ar')₃, Si(Ar')₃ | (R^{e})₂C-C(R^{e})₂, (R^{e})C=C(R^{e}) |
| RB | RB | H, D, Alkyl | C(Ar')₃, Si(Ar')₃ | (R^{e})₂C-C(R^{e})₂, (R^{e})C=C(R^{e}) |
| RA-5 | RA-5 | Ar-1 bis Ar-75 | C(Ar')₃, Si(Ar')₃ | (R^{e})₂C-C(R^{e})₂, (R^{e})C=C(R^{e}) |
| RA-4 | RA-4 | Ar-1 bis Ar-75 | C(Ar')₃, Si(Ar')₃ | (R^{e})₂C-C(R^{e})₂, (R^{e})C=C(R^{e}) |
| RA-4f | RA-4f | Ar-1 bis Ar-75 | C(Ar')₃, Si(Ar')₃ | (R^{e})₂C-C(R^{e})₂, (R^{e})C=C(R^{e}) |
| RA-3 | RA-3 | Ar-1 bis Ar-75 | C(Ar')₃, Si(Ar')₃ | (R^{e})₂C-C(R^{e})₂, (R^{e})C=C(R^{e}) |
| RB | RB | Ar-1 bis Ar-75 | C(Ar')₃, Si(Ar')₃ | (R^{e})₂C-C(R^{e})₂, (R^{e})C=C(R^{e}) |
| RA-5 | RA-5 | H, D, Alkyl | N(Ar')₃, N(R¹)₃ | (R^{e})₂C-C(R^{e})₂, (R^{e})C=C(R^{e}) |
| RA-4 | RA-4 | H, D, Alkyl | N(Ar')₃, N(R¹)₃ | (R^{e})₂C-C(R^{e})₂, (R^{e})C=C(R^{e}) |
| RA-4f | RA-4f | H, D, Alkyl | N(Ar')₃, N(R¹)₃ | (R^{e})₂C-C(R^{e})₂, (R^{e})C=C(R^{e}) |
| RA-3 | RA-3 | H, D, Alkyl | N(Ar')₃, N(R¹)₃ | (R^{e})₂C-C(R^{e})₂, (R^{e})C=C(R^{e}) |
| RB | RB | H, D, Alkyl | N(Ar')₃, N(R¹)₃ | (R^{e})₂C-C(R^{e})₂, (R^{e})C=C(R^{e}) |
| RA-5 | RA-5 | Ar-1 bis Ar-75 | N(Ar')₃, N(R¹)₃ | (R^{e})₂C-C(R^{e})₂, (R^{e})C=C(R^{e}) |
| RA-4 | RA-4 | Ar-1 bis Ar-75 | N(Ar')₃, N(R¹)₃ | (R^{e})₂C-C(R^{e})₂, (R^{e})C=C(R^{e}) |
| RA-4f | RA-4f | Ar-1 bis Ar-75 | N(Ar')₃, N(R¹)₃ | (R^{e})₂C-C(R^{e})₂, (R^{e})C=C(R^{e}) |
| RA-3 | RA-3 | Ar-1 bis Ar-75 | N(Ar')₃, N(R¹)₃ | (R^{e})₂C-C(R^{e})₂, (R^{e})C=C(R^{e}) |
| RB | RB | Ar-1 bis Ar-75 | N(Ar')₃, N(R¹)₃ | (R^{e})₂C-C(R^{e})₂, (R^{e})C=C(R^{e}) |

In obigen Tabellen stehen die in der Spalte unter der Gruppe R^{e} genannten Reste für die Substituenten am Phenylring des Grundgerüsts, der ebenfalls durch die genannten Rest R^{d} substituiert ist (siehe beispielsweise Formel (I-1)), oder für die Substituenten am Phenylring, der an den Phenylring des Grundgerüsts, der ebenfalls durch die genannten Rest R^{d} substituiert ist, bindet (siehe beispielsweise Formel (I-2) und (I-3)). In der Gruppe C(R^{e})₂ steht der Rest R^{e} insbesondere für die zuvor dargelegten Gruppen, wobei R^{e} in der Gruppe C(R^{e})₂ vorzugsweise gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24, vorzugsweise mit 5 bis 13 aromatischen Ringatomen, welches auch durch einen oder mehrere Reste R¹ substituiert sein kann. Ganz besonders bevorzugt steht R^{e} für eine Methylgruppe oder für eine Phenylgruppe. Dabei können die Reste R^{e} auch miteinander ein Ringsystem bilden, was zu einem Spirosystem führt. In den Gruppen (R^{e})₂C-C(R^{e})₂, (R^{e})C=C(R^{e}) steht der Rest R^{e} insbesondere für die zuvor dargelegten Gruppen, wobei R^{e} in Gruppen (R^{e})₂C-C(R^{e})₂, (R^{e})C=C(R^{e}) vorzugsweise für H, C₁ bis C₄-Alkyl, oder für eine Aryl- oder Heteroarylgruppe mit 5 bis 13 C-Atomen steht, wobei die Aryl- oder Heteroarylgruppe verknüpft sein kann. Hierbei können zwei Gruppen R^{e} in der Gruppe (R^{e})₂C-C(R^{e})₂ oder (R^{e})C=C(R^{e}) ein kondensiertes Ringsystem bilden.

In einer weiteren Ausgestaltung der vorliegenden Erfindung sind Verbindungen umfassend eine Struktur gemäß Formel (I-4), vorzugsweise Verbindungen gemäß Formel (I-4) bevorzugt, wobei die beiden Reste R^{a} einen Ring bilden, die beiden Reste R^{c} einen Ring bilden, wobei der Index n vorzugsweise 0, 1 oder 2 und besonders bevorzugt 0 oder 1 ist, und bei denen die Reste R^{a}, R^{b}, R^{c}, R^{e} und Y¹ folgenden Bedeutungen aufweisen:

| R^{a} | R^{b} | R^{c} | R^{e} am Phenylring (nur falls m ungleich 0 ist mindestens ein Rest R^{e}, andernfalls sind alle R^{e} H) | Y¹ |
|---|---|---|---|---|
| RA-5 | H, D, Alkyl | RA-5 | D, Alkyl | C(R^{e})₂ |
| RA-4 | H, D, Alkyl | RA-4 | D, Alkyl | C(R^{e})₂ |
| RA-4f | H, D, Alkyl | RA-4f | D, Alkyl | C(R^{e})₂ |
| RA-3 | H, D, Alkyl | RA-3 | D, Alkyl | C(R^{e})₂ |
| RB | H, D, Alkyl | RB | D, Alkyl | C(R^{e})₂ |
| RA-5 | H, D, Alkyl | RA-5 | Ar-1 bis Ar-75 | C(R^{e})₂ |
| RA-4 | H, D, Alkyl | RA-4 | Ar-1 bis Ar-75 | C(R^{e})₂ |
| RA-4f | H, D, Alkyl | RA-4f | Ar-1 bis Ar-75 | C(R^{e})₂ |
| RA-3 | H, D, Alkyl | RA-3 | Ar-1 bis Ar-75 | C(R^{e})₂ |
| RB | H, D, Alkyl | RB | Ar-1 bis Ar-75 | C(R^{e})₂ |
| RA-5 | H, D, Alkyl | RA-5 | Phenyl-Ringbildung von 2 R^{e} | C(R^{e})₂ |
| RA-4 | H, D, Alkyl | RA-4 | Phenyl-Ringbildung von 2 R^{e} | C(R^{e})₂ |
| RA-4f | H, D, Alkyl | RA-4f | Phenyl-Ringbildung von 2 R^{e} | C(R^{e})₂ |
| RA-3 | H, D, Alkyl | RA-3 | Phenyl-Ringbildung von 2 R^{e} | C(R^{e})₂ |
| RB | H, D, Alkyl | RB | Phenyl-Ringbildung von 2 R^{e} | C(R^{e})₂ |
| RA-5 | H, D, Alkyl | RA-5 | C(Ar')₃, Si(Ar')₃ | C(R^{e})₂ |
| RA-4 | H, D, Alkyl | RA-4 | C(Ar')₃, Si(Ar')₃ | C(R^{e})₂ |
| RA-4f | H, D, Alkyl | RA-4f | C(Ar')₃, Si(Ar')₃ | C(R^{e})₂ |
| RA-3 | H, D, Alkyl | RA-3 | C(Ar')₃, Si(Ar')₃ | C(R^{e})₂ |
| RB | H, D, Alkyl | RB | C(Ar')₃, Si(Ar')₃ | C(R^{e})₂ |
| RA-5 | H, D, Alkyl | RA-5 | N(Ar')₃, N(R¹)₃ | C(R^{e})₂ |
| RA-4 | H, D, Alkyl | RA-4 | N(Ar')₃, N(R¹)₃ | C(R^{e})₂ |
| RA-4f | H, D, Alkyl | RA-4f | N(Ar')₃, N(R¹)₃ | C(R^{e})₂ |
| RA-3 | H, D, Alkyl | RA-3 | N(Ar')₃, N(R¹)₃ | C(R^{e})₂ |
| RB | H, D, Alkyl | RB | N(Ar')₃, N(R¹)₃ | C(R^{e})₂ |
| RA-5 | H, D, Alkyl | RA-5 | D, Alkyl | (R^{e})₂C-C(R^{e})₂, (R^{e})C=C(R^{e}) |
| RA-4 | H, D, Alkyl | RA-4 | D, Alkyl | (R^{e})₂C-C(R^{e})₂, (R^{e})C=C(R^{e}) |
| RA-4f | H, D, Alkyl | RA-4f | D, Alkyl | (R^{e})₂C-C(R^{e})₂, (R^{e})C=C(R^{e}) |
| RA-3 | H, D, Alkyl | RA-3 | D, Alkyl | (R^{e})₂C-C(R^{e})₂, (R^{e})C=C(R^{e}) |
| RB | H, D, Alkyl | RB | D, Alkyl | (R^{e})₂C-C(R^{e})₂, (R^{e})C=C(R^{e}) |
| RA-5 | H, D, Alkyl | RA-5 | Ar-1 bis Ar-75 | (R^{e})₂C-C(R^{e})₂, (R^{e})C=C(R^{e}) |
| RA-4 | H, D, Alkyl | RA-4 | Ar-1 bis Ar-75 | (R^{e})₂C-C(R^{e})₂, (R^{e})C=C(R^{e}) |
| RA-4f | H, D, Alkyl | RA-4f | Ar-1 bis Ar-75 | (R^{e})₂C-C(R^{e})₂, (R^{e})C=C(R^{e}) |
| RA-3 | H, D, Alkyl | RA-3 | Ar-1 bis Ar-75 | (R^{e})₂C-C(R^{e})₂, (R^{e})C=C(R^{e}) |
| RB | H, D, Alkyl | RB | Ar-1 bis Ar-75 | (R^{e})₂C-C(R^{e})₂, (R^{e})C=C(R^{e}) |
| RA-5 | H, D, Alkyl | RA-5 | Phenyl-Ringbildung von 2 R^{e} | (R^{e})₂C-C(R^{e})₂, (R^{e})C=C(R^{e}) |
| RA-4 | H, D, Alkyl | RA-4 | Phenyl-Ringbildung von 2 R^{e} | (R^{e})₂C-C(R^{e})₂, (R^{e})C=C(R^{e}) |
| RA-4f | H, D, Alkyl | RA-4f | Phenyl-Ringbildung von 2 R^{e} | (R^{e})₂C-C(R^{e})₂, (R^{e})C=C(R^{e}) |
| RA-3 | H, D, Alkyl | RA-3 | Phenyl-Ringbildung von 2 R^{e} | (R^{e})₂C-C(R^{e})₂, (R^{e})C=C(R^{e}) |
| RB | H, D, Alkyl | RB | Phenyl-Ringbildung von 2 R^{e} | (R^{e})₂C-C(R^{e})₂, (R^{e})C=C(R^{e}) |
| RA-5 | H, D, Alkyl | RA-5 | C(Ar')₃, Si(Ar')₃ | (R^{e})₂C-C(R^{e})₂, (R^{e})C=C(R^{e}) |
| RA-4 | H, D, Alkyl | RA-4 | C(Ar')₃, Si(Ar')₃ | (R^{e})₂C-C(R^{e})₂, (R^{e})C=C(R^{e}) |
| RA-4f | H, D, Alkyl | RA-4f | C(Ar')₃, Si(Ar')₃ | (R^{e})₂C-C(R^{e})₂, (R^{e})C=C(R^{e}) |
| RA-3 | H, D, Alkyl | RA-3 | C(Ar')₃, Si(Ar')₃ | (R^{e})₂C-C(R^{e})₂, (R^{e})C=C(R^{e}) |
| RB | H, D, Alkyl | RB | C(Ar')₃, Si(Ar')₃ | (R^{e})₂C-C(R^{e})₂, (R^{e})C=C(R^{e}) |
| RA-5 | H, D, Alkyl | RA-5 | N(Ar')₃, N(R¹)₃ | (R^{e})₂C-C(R^{e})₂, (R^{e})C=C(R^{e}) |
| RA-4 | H, D, Alkyl | RA-4 | N(Ar')₃, N(R¹)₃ | (R^{e})₂C-C(R^{e})₂, (R^{e})C=C(R^{e}) |
| RA-4f | H, D, Alkyl | RA-4f | N(Ar')₃, N(R¹)₃ | (R^{e})₂C-C(R^{e})₂, (R^{e})C=C(R^{e}) |
| RA-3 | H, D, Alkyl | RA-3 | N(Ar')₃, N(R¹)₃ | (R^{e})₂C-C(R^{e})₂, (R^{e})C=C(R^{e}) |
| RB | H, D, Alkyl | RB | N(Ar')₃, N(R¹)₃ | (R^{e})₂C-C(R^{e})₂, (R^{e})C=C(R^{e}) |
| RA-5 | H, D, Alkyl | RA-5 | D, Alkyl | O, S |
| RA-4 | H, D, Alkyl | RA-4 | D, Alkyl | O, S |
| RA-4f | H, D, Alkyl | RA-4f | D, Alkyl | O, S |
| RA-3 | H, D, Alkyl | RA-3 | D, Alkyl | O, S |
| RB | H, D, Alkyl | RB | D, Alkyl | O, S |
| RA-5 | H, D, Alkyl | RA-5 | Ar-1 bis Ar-75 | O, S |
| RA-4 | H, D, Alkyl | RA-4 | Ar-1 bis Ar-75 | O, S |
| RA-4f | H, D, Alkyl | RA-4f | Ar-1 bis Ar-75 | O, S |
| RA-3 | H, D, Alkyl | RA-3 | Ar-1 bis Ar-75 | O, S |
| RB | H, D, Alkyl | RB | Ar-1 bis Ar-75 | O, S |
| RA-5 | H, D, Alkyl | RA-5 | Phenyl-Ringbildung von 2 R^{e} | O, S |
| RA-4 | H, D, Alkyl | RA-4 | Phenyl-Ringbildung von 2 R^{e} | O, S |
| RA-4f | H, D, Alkyl | RA-4f | Phenyl-Ringbildung von 2 R^{e} | O, S |
| RA-3 | H, D, Alkyl | RA-3 | Phenyl-Ringbildung von 2 R^{e} | O, S |
| RB | H, D, Alkyl | RB | Phenyl-Ringbildung von 2 R^{e} | O, S |
| RA-5 | H, D, Alkyl | RA-5 | C(Ar')₃, Si(Ar')₃ | O, S |
| RA-4 | H, D, Alkyl | RA-4 | C(Ar')₃, Si(Ar')₃ | O, S |
| RA-4f | H, D, Alkyl | RA-4f | C(Ar')₃, Si(Ar')₃ | O, S |
| RA-3 | H, D, Alkyl | RA-3 | C(Ar')₃, Si(Ar')₃ | O, S |
| RB | H, D, Alkyl | RB | C(Ar')₃, Si(Ar')₃ | O, S |
| RA-5 | H, D, Alkyl | RA-5 | N(Ar')₃, N(R¹)₃ | O, S |
| RA-4 | H, D, Alkyl | RA-4 | N(Ar')₃, N(R¹)₃ | O, S |
| RA-4f | H, D, Alkyl | RA-4f | N(Ar')₃, N(R¹)₃ | O, S |
| RA-3 | H, D, Alkyl | RA-3 | N(Ar')₃, N(R¹)₃ | O, S |
| RB | H, D, Alkyl | RB | N(Ar')₃, N(R¹)₃ | O, S |
| RA-5 | H, D, Alkyl | RA-5 | D, Alkyl | O, S und C(R^{e})₂ |
| RA-4 | H, D, Alkyl | RA-4 | D, Alkyl | O, S und C(R^{e})₂ |
| RA-4f | H, D, Alkyl | RA-4f | D, Alkyl | O, S und C(R^{e})₂ |
| RA-3 | H, D, Alkyl | RA-3 | D, Alkyl | O, S und C(R^{e})₂ |
| RB | H, D, Alkyl | RB | D, Alkyl | O, S und C(R^{e})₂ |
| RA-5 | H, D, Alkyl | RA-5 | Ar-1 bis Ar-75 | O, S und C(R^{e})₂ |
| RA-4 | H, D, Alkyl | RA-4 | Ar-1 bis Ar-75 | O, S und C(R^{e})₂ |
| RA-4f | H, D, Alkyl | RA-4f | Ar-1 bis Ar-75 | O, S und C(R^{e})₂ |
| RA-3 | H, D, Alkyl | RA-3 | Ar-1 bis Ar-75 | O, S und C(R^{e})₂ |
| RB | H, D, Alkyl | RB | Ar-1 bis Ar-75 | O, S und C(R^{e})₂ |
| RA-5 | H, D, Alkyl | RA-5 | Phenyl-Ringbildung von 2 R^{e} | O, S und C(R^{e})₂ |
| RA-4 | H, D, Alkyl | RA-4 | Phenyl-Ringbildung von 2 R^{e} | O, S und C(R^{e})₂ |
| RA-4f | H, D, Alkyl | RA-4f | Phenyl-Ringbildung von 2 R^{e} | O, S und C(R^{e})₂ |
| RA-3 | H, D, Alkyl | RA-3 | Phenyl-Ringbildung von 2 R^{e} | O, S und C(R^{e})₂ |
| RB | H, D, Alkyl | RB | Phenyl-Ringbildung von 2 R^{e} | O, S und C(R^{e})₂ |
| RA-5 | H, D, Alkyl | RA-5 | C(Ar')₃, Si(Ar')₃ | O, S und C(R^{e})₂ |
| RA-4 | H, D, Alkyl | RA-4 | C(Ar')₃, Si(Ar')₃ | O, S und C(R^{e})₂ |
| RA-4f | H, D, Alkyl | RA-4f | C(Ar')₃, Si(Ar')₃ | O, S und C(R^{e})₂ |
| RA-3 | H, D, Alkyl | RA-3 | C(Ar')₃, Si(Ar')₃ | O, S und C(R^{e})₂ |
| RB | H, D, Alkyl | RB | C(Ar')₃, Si(Ar')₃ | O, S und C(R^{e})₂ |
| RA-5 | H, D, Alkyl | RA-5 | N(Ar')₃, N(R¹)₃ | O, S und C(R^{e})₂ |
| RA-4 | H, D, Alkyl | RA-4 | N(Ar')₃, N(R¹)₃ | O, S und C(R^{e})₂ |
| RA-4f | H, D, Alkyl | RA-4f | N(Ar')₃, N(R¹)₃ | O, S und C(R^{e})₂ |
| RA-3 | H, D, Alkyl | RA-3 | N(Ar')₃, N(R¹)₃ | O, S und C(R^{e})₂ |
| RB | H, D, Alkyl | RB | N(Ar')₃, N(R¹)₃ | O, S und C(R^{e})₂ |
| RA-5 | H, D, Alkyl | RA-5 | D, Alkyl | (R^{e})₂C-C(R^{e})₂, (R^{e})C=C(R^{e}) C(R^{e})₂ |
| RA-4 | H, D, Alkyl | RA-4 | D, Alkyl | (R^{e})₂C-C(R^{e})₂, (R^{e})C=C(R^{e}) und C(R^{e})₂ |
| RA-4f | H, D, Alkyl | RA-4f | D, Alkyl | (R^{e})₂C-C(R^{e})₂, (R^{e})C=C(R^{e}) und C(R^{e})₂ |
| RA-3 | H, D, Alkyl | RA-3 | D, Alkyl | (R^{e})₂C-C(R^{e})₂, (R^{e})C=C(R^{e}) und C(R^{e})₂ |
| RB | H, D, Alkyl | RB | D, Alkyl | (R^{e})₂C-C(R^{e})₂, (R^{e})C=C(R^{e}) und C(R^{e})₂ |
| RA-5 | H, D, Alkyl | RA-5 | Ar-1 bis Ar-75 | (R^{e})₂C-C(R^{e})₂, (R^{e})C=C(R^{e}) und C(R^{e})₂ |
| RA-4 | H, D, Alkyl | RA-4 | Ar-1 bis Ar-75 | (R^{e})₂C-C(R^{e})₂, (R^{e})C=C(R^{e}) und C(R^{e})₂ |
| RA-4f | H, D, Alkyl | RA-4f | Ar-1 bis Ar-75 | (R^{e})₂C-C(R^{e})₂, (R^{e})C=C(R^{e}) und C(R^{e})₂ |
| RA-3 | H, D, Alkyl | RA-3 | Ar-1 bis Ar-75 | (R^{e})₂C-C(R^{e})₂, (R^{e})C=C(R^{e}) und C(R^{e})₂ |
| RB | H, D, Alkyl | RB | Ar-1 bis Ar-75 | (R^{e})₂C-C(R^{e})₂, (R^{e})C=C(R^{e}) und C(R^{e})₂ |
| RA-5 | H, D, Alkyl | RA-5 | Phenyl-Ringbildung von 2 R^{e} | (R^{e})₂C-C(R^{e})₂, (R^{e})C=C(R^{e}) und C(R^{e})₂ |
| RA-4 | H, D, Alkyl | RA-4 | Phenyl-Ringbildung von 2 R^{e} | (R^{e})₂C-C(R^{e})₂, (R^{e})C=C(R^{e}) und C(R^{e})₂ |
| RA-4f | H, D, Alkyl | RA-4f | Phenyl-Ringbildung von 2 R^{e} | (R^{e})₂C-C(R^{e})₂, (R^{e})C=C(R^{e}) und C(R^{e})₂ |
| RA-3 | H, D, Alkyl | RA-3 | Phenyl-Ringbildung von 2 R^{e} | (R^{e})₂C-C(R^{e})₂, (R^{e})C=C(R^{e}) und C(R^{e})₂ |
| RB | H, D, Alkyl | RB | Phenyl-Ringbildung von 2 R^{e} | (R^{e})₂C-C(R^{e})₂, (R^{e})C=C(R^{e}) und C(R^{e})₂ |
| RA-5 | H, D, Alkyl | RA-5 | C(Ar')₃, Si(Ar')₃ | (R^{e})₂C-C(R^{e})₂, (R^{e})C=C(R^{e}) und C(R^{e})₂ |
| RA-4 | H, D, Alkyl | RA-4 | C(Ar')₃, Si(Ar')₃ | (R^{e})₂C-C(R^{e})₂, (R^{e})C=C(R^{e}) und C(R^{e})₂ |
| RA-4f | H, D, Alkyl | RA-4f | C(Ar')₃, Si(Ar')₃ | (R^{e})₂C-C(R^{e})₂, (R^{e})C=C(R^{e}) und C(R^{e})₂ |
| RA-3 | H, D, Alkyl | RA-3 | C(Ar')₃, Si(Ar')₃ | (R^{e})₂C-C(R^{e})₂, (R^{e})C=C(R^{e}) und C(R^{e})₂ |
| RB | H, D, Alkyl | RB | C(Ar')₃, Si(Ar')₃ | (R^{e})₂C-C(R^{e})₂, (R^{e})C=C(R^{e}) und C(R^{e})₂ |
| RA-5 | H, D, Alkyl | RA-5 | N(Ar')₃, N(R¹)₃ | (R^{e})₂C-C(R^{e})₂, (R^{e})C=C(R^{e}) und C(R^{e})₂ |
| RA-4 | H, D, Alkyl | RA-4 | N(Ar')₃, N(R¹)₃ | (R^{e})₂C-C(R^{e})₂, (R^{e})C=C(R^{e}) und C(R^{e})₂ |
| RA-4f | H, D, Alkyl | RA-4f | N(Ar')₃, N(R¹)₃ | (R^{e})₂C-C(R^{e})₂, (R^{e})C=C(R^{e}) und C(R^{e})₂ |
| RA-3 | H, D, Alkyl | RA-3 | N(Ar')₃, N(R¹)₃ | (R^{e})₂C-C(R^{e})₂, (R^{e})C=C(R^{e}) und C(R^{e})₂ |
| RB | H, D, Alkyl | RB | N(Ar')₃, N(R¹)₃ | (R^{e})₂C-C(R^{e})₂, (R^{e})C=C(R^{e}) und C(R^{e})₂ |
| RA-5 | H, D, Alkyl | RA-5 | D, Alkyl | O, S und (R^{e})₂C-C(R^{e})₂, (R^{e})C=C(R^{e}) |
| RA-4 | H, D, Alkyl | RA-4 | D, Alkyl | O, S und (R^{e})₂C-C(R^{e})₂, (R^{e})C=C(R^{e}) |
| RA-4f | H, D, Alkyl | RA-4f | D, Alkyl | O, S und (R^{e})₂C-C(R^{e})₂, (R^{e})C=C(R^{e}) |
| RA-3 | H, D, Alkyl | RA-3 | D, Alkyl | O, S und (R^{e})₂C-C(R^{e})₂, (R^{e})C=C(R^{e}) |
| RB | H, D, Alkyl | RB | D, Alkyl | O, S und (R^{e})₂C-C(R^{e})₂, (R^{e})C=C(R^{e}) |
| RA-5 | H, D, Alkyl | RA-5 | Ar-1 bis Ar-75 | O, S und (R^{e})₂C-C(R^{e})₂, (R^{e})C=C(R^{e}) |
| RA-4 | H, D, Alkyl | RA-4 | Ar-1 bis Ar-75 | O, S und (R^{e})₂C-C(R^{e})₂, (R^{e})C=C(R^{e}) |
| RA-4f | H, D, Alkyl | RA-4f | Ar-1 bis Ar-75 | O, S und (R^{e})₂C-C(R^{e})₂, (R^{e})C=C(R^{e}) |
| RA-3 | H, D, Alkyl | RA-3 | Ar-1 bis Ar-75 | O, S und (R^{e})₂C-C(R^{e})₂, (R^{e})C=C(R^{e}) |
| RB | H, D, Alkyl | RB | Ar-1 bis Ar-75 | O, S und (R^{e})₂C-C(R^{e})₂, (R^{e})C=C(R^{e}) |
| RA-5 | H, D, Alkyl | RA-5 | Phenyl-Ringbildung von 2 R^{e} | O, S und (R^{e})C=C(R^{e}) |
| RA-4 | H, D, Alkyl | RA-4 | Phenyl-Ringbildung von 2 R^{e} | O, S und (R^{e})C=C(R^{e}) |
| RA-4f | H, D, Alkyl | RA-4f | Phenyl-Ringbildung von 2 R^{e} | O, S und (R^{e})₂C-C(R^{e})₂, (R^{e})C=C(R^{e}) |
| RA-3 | H, D, Alkyl | RA-3 | Phenyl-Ringbildung von 2 R^{e} | O, S und (R^{e})₂C-C(R^{e})₂, (R^{e})C=C(R^{e}) |
| RB | H, D, Alkyl | RB | Phenyl-Ringbildung von 2 R^{e} | O, S und (R^{e})₂C-C(R^{e})₂, (R^{e})C=C(R^{e}) |
| RA-5 | H, D, Alkyl | RA-5 | C(Ar')₃, Si(Ar')₃ | O, S und (R^{e})₂C-C(R^{e})₂, (R^{e})C=C(R^{e}) |
| RA-4 | H, D, Alkyl | RA-4 | C(Ar')₃, Si(Ar')₃ | O, S und (R^{e})₂C-C(R^{e})₂, (R^{e})C=C(R^{e}) |
| RA-4f | H, D, Alkyl | RA-4f | C(Ar')₃, Si(Ar')₃ | O, S und (R^{e})₂C-C(R^{e})₂, (R^{e})C=C(R^{e}) |
| RA-3 | H, D, Alkyl | RA-3 | C(Ar')₃, Si(Ar')₃ | O, S und (R^{e})₂C-C(R^{e})₂, (R^{e})C=C(R^{e}) |
| RB | H, D, Alkyl | RB | C(Ar')₃, Si(Ar')₃ | O, S und (R^{e})₂C-C(R^{e})₂, (R^{e})C=C(R^{e}) |
| RA-5 | H, D, Alkyl | RA-5 | N(Ar')₃, N(R¹)₃ | O, S und (R^{e})₂C-C(R^{e})₂, (R^{e})C=C(R^{e}) |
| RA-4 | H, D, Alkyl | RA-4 | N(Ar')₃, N(R¹)₃ | O, S und (R^{e})₂C-C(R^{e})₂, (R^{e})C=C(R^{e}) |
| RA-4f | H, D, Alkyl | RA-4f | N(Ar')₃, N(R¹)₃ | O, S und (R^{e})₂C-C(R^{e})₂, (R^{e})C=C(R^{e}) |
| RA-3 | H, D, Alkyl | RA-3 | N(Ar')₃, N(R¹)₃ | O, S und (R^{e})₂C-C(R^{e})₂, (R^{e})C=C(R^{e}) |
| RB | H, D, Alkyl | RB | N(Ar')₃, N(R¹)₃ | O, S und (R^{e})₂C-C(R^{e})₂, (R^{e})C=C(R^{e}) |

In obiger Tabelle stehen die in der Spalte unter der Gruppe R^{e} genannten Reste für die Substituenten am Phenylring der mit der Gruppe Y¹ verbunden ist. In der Gruppe C(R^{e})₂ steht der Rest R^{e} insbesondere für die zuvor dargelegten Gruppen, wobei R^{e} in der Gruppe C(R^{e})₂ vorzugsweise gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24, vorzugsweise mit 5 bis 13 aromatischen Ringatomen, welches auch durch einen oder mehrere Reste R¹ substituiert sein kann. Ganz besonders bevorzugt steht R^{e} für eine Methylgruppe oder für eine Phenylgruppe. Dabei können die Reste R^{e} auch miteinander ein Ringsystem bilden, was zu einem Spirosystem führt. In den Gruppen (R^{e})₂C-C(R^{e})₂, (R^{e})C=C(R^{e}) steht der Rest R^{e} insbesondere für die zuvor dargelegten Gruppen, wobei R^{e} in Gruppen (R^{e})₂C-C(R^{e})₂, (R^{e})C=C(R^{e}) vorzugsweise für H, C₁ bis C₄-Alkyl, oder für eine Aryl- oder Heteroarylgruppe mit 5 bis 13 C-Atomen steht, wobei die Aryl- oder Heteroarylgruppe verknüpft sein kann. Hierbei können zwei Gruppen R^{e} in der Gruppe (R^{e})₂C-C(R^{e})₂ oder (R^{e})C=C(R^{e}) ein kondensiertes Ringsystem bilden.

In einer weiteren Ausgestaltung der vorliegenden Erfindung sind Verbindungen umfassend eine Struktur gemäß Formel (I-5), vorzugsweise Verbindungen gemäß Formel (I-5) bevorzugt, wobei die beiden Reste R^{a} einen Ring bilden, die beiden Reste R^{c} einen Ring bilden, wobei der Index m vorzugsweise 0, 1 oder 2 und besonders bevorzugt 0 oder 1 ist, und bei denen R^{b} H, D, Alkyl ist und die Reste R^{a}, R^{c}, R^{d}, R^{e} und Y¹ folgenden Bedeutungen aufweisen:

| R^{a} | R^{c} | R^{d} | R^{e} am Phenylring (nur falls I ungleich 0 ist mindestens ein Rest R^{e}, andernfalls sind alle R^{e} H) | Y¹ |
|---|---|---|---|---|
| RA-5 | RA-5 | H, D, Alkyl | D, Alkyl | C(R^{e})₂ |
| RA-4 | RA-4 | H, D, Alkyl | D, Alkyl | C(R^{e})₂ |
| RA-4f | RA-4f | H, D, Alkyl | D, Alkyl | C(R^{e})₂ |
| RA-3 | RA-3 | H, D, Alkyl | D, Alkyl | C(R^{e})₂ |
| RB | RB | H, D, Alkyl | D, Alkyl | C(R^{e})₂ |
| RA-5 | RA-5 | H, D, Alkyl | Ar-1 bis Ar-75 | C(R^{e})₂ |
| RA-4 | RA-4 | H, D, Alkyl | Ar-1 bis Ar-75 | C(R^{e})₂ |
| RA-4f | RA-4f | H, D, Alkyl | Ar-1 bis Ar-75 | C(R^{e})₂ |
| RA-3 | RA-3 | H, D, Alkyl | Ar-1 bis Ar-75 | C(R^{e})₂ |
| RB | RB | H, D, Alkyl | Ar-1 bis Ar-75 | C(R^{e})₂ |
| RA-5 | RA-5 | Ar-1 bis Ar-75 | D, Alkyl | C(R^{e})₂ |
| RA-4 | RA-4 | Ar-1 bis Ar-75 | D, Alkyl | C(R^{e})₂ |
| RA-4f | RA-4f | Ar-1 bis Ar-75 | D, Alkyl | C(R^{e})₂ |
| RA-3 | RA-3 | Ar-1 bis Ar-75 | D, Alkyl | C(R^{e})₂ |
| RB | RB | Ar-1 bis Ar-75 | D, Alkyl | C(R^{e})₂ |
| RA-5 | RA-5 | Ar-1 bis Ar-75 | Ar-1 bis Ar-75 | C(R^{e})₂ |
| RA-4 | RA-4 | Ar-1 bis Ar-75 | Ar-1 bis Ar-75 | C(R^{e})₂ |
| RA-4f | RA-4f | Ar-1 bis Ar-75 | Ar-1 bis Ar-75 | C(R^{e})₂ |
| RA-3 | RA-3 | Ar-1 bis Ar-75 | Ar-1 bis Ar-75 | C(R^{e})₂ |
| RB | RB | Ar-1 bis Ar-75 | Ar-1 bis Ar-75 | C(R^{e})₂ |
| RA-5 | RA-5 | H, D, Alkyl | Phenyl-Ringbildung von 2 R^{e} | C(R^{e})₂ |
| RA-4 | RA-4 | H, D, Alkyl | Phenyl-Ringbildung von 2 R^{e} | C(R^{e})₂ |
| RA-4f | RA-4f | H, D, Alkyl | Phenyl-Ringbildung von 2 R^{e} | C(R^{e})₂ |
| RA-3 | RA-3 | H, D, Alkyl | Phenyl-Ringbildung von 2 R^{e} | C(R^{e})₂ |
| RB | RB | H, D, Alkyl | Phenyl-Ringbildung von 2 R^{e} | C(R^{e})₂ |
| RA-5 | RA-5 | Ar-1 bis Ar-75 | Phenyl-Ringbildung von 2 R^{e} | C(R^{e})₂ |
| RA-4 | RA-4 | Ar-1 bis Ar-75 | Phenyl-Ringbildung von 2 R^{e} | C(R^{e})₂ |
| RA-4f | RA-4f | Ar-1 bis Ar-75 | Phenyl-Ringbildung von 2 R^{e} | C(R^{e})₂ |
| RA-3 | RA-3 | Ar-1 bis Ar-75 | Phenyl-Ringbildung von 2 R^{e} | C(R^{e})₂ |
| RB | RB | Ar-1 bis Ar-75 | Phenyl-Ringbildung von 2 R^{e} | C(R^{e})₂ |
| RA-5 | RA-5 | H, D, Alkyl | C(Ar')₃, Si(Ar')₃ | C(R^{e})₂ |
| RA-4 | RA-4 | H, D, Alkyl | C(Ar')₃, Si(Ar')₃ | C(R^{e})₂ |
| RA-4f | RA-4f | H, D, Alkyl | C(Ar')₃, Si(Ar')₃ | C(R^{e})₂ |
| RA-3 | RA-3 | H, D, Alkyl | C(Ar')₃, Si(Ar')₃ | C(R^{e})₂ |
| RB | RB | H, D, Alkyl | C(Ar')₃, Si(Ar')₃ | C(R^{e})₂ |
| RA-5 | RA-5 | Ar-1 bis Ar-75 | C(Ar')₃, Si(Ar')₃ | C(R^{e})₂ |
| RA-4 | RA-4 | Ar-1 bis Ar-75 | C(Ar')₃, Si(Ar')₃ | C(R^{e})₂ |
| RA-4f | RA-4f | Ar-1 bis Ar-75 | C(Ar')₃, Si(Ar')₃ | C(R^{e})₂ |
| RA-3 | RA-3 | Ar-1 bis Ar-75 | C(Ar')₃, Si(Ar')₃ | C(R^{e})₂ |
| RB | RB | Ar-1 bis Ar-75 | C(Ar')₃, Si(Ar')₃ | C(R^{e})₂ |
| RA-5 | RA-5 | H, D, Alkyl | N(Ar')₃, N(R¹)₃ | C(R^{e})₂ |
| RA-4 | RA-4 | H, D, Alkyl | N(Ar')₃, N(R¹)₃ | C(R^{e})₂ |
| RA-4f | RA-4f | H, D, Alkyl | N(Ar')₃, N(R¹)₃ | C(R^{e})₂ |
| RA-3 | RA-3 | H, D, Alkyl | N(Ar')₃, N(R¹)₃ | C(R^{e})₂ |
| RB | RB | H, D, Alkyl | N(Ar')₃, N(R¹)₃ | C(R^{e})₂ |
| RA-5 | RA-5 | Ar-1 bis Ar-75 | N(Ar')₃, N(R¹)₃ | C(R^{e})₂ |
| RA-4 | RA-4 | Ar-1 bis Ar-75 | N(Ar')₃, N(R¹)₃ | C(R^{e})₂ |
| RA-4f | RA-4f | Ar-1 bis Ar-75 | N(Ar')₃, N(R¹)₃ | C(R^{e})₂ |
| RA-3 | RA-3 | Ar-1 bis Ar-75 | N(Ar')₃, N(R¹)₃ | C(R^{e})₂ |
| RB | RB | Ar-1 bis Ar-75 | N(Ar')₃, N(R¹)₃ | C(R^{e})₂ |
| RA-5 | RA-5 | H, D, Alkyl | D, Alkyl | O, S |
| RA-4 | RA-4 | H, D, Alkyl | D, Alkyl | O, S |
| RA-4f | RA-4f | H, D, Alkyl | D, Alkyl | O, S |
| RA-3 | RA-3 | H, D, Alkyl | D, Alkyl | O, S |
| RB | RB | H, D, Alkyl | D, Alkyl | O, S |
| RA-5 | RA-5 | H, D, Alkyl | Ar-1 bis Ar-75 | O, S |
| RA-4 | RA-4 | H, D, Alkyl | Ar-1 bis Ar-75 | O, S |
| RA-4f | RA-4f | H, D, Alkyl | Ar-1 bis Ar-75 | O, S |
| RA-3 | RA-3 | H, D, Alkyl | Ar-1 bis Ar-75 | O, S |
| RB | RB | H, D, Alkyl | Ar-1 bis Ar-75 | O, S |
| RA-5 | RA-5 | Ar-1 bis Ar-75 | D, Alkyl | O, S |
| RA-4 | RA-4 | Ar-1 bis Ar-75 | D, Alkyl | O, S |
| RA-4f | RA-4f | Ar-1 bis Ar-75 | D, Alkyl | O, S |
| RA-3 | RA-3 | Ar-1 bis Ar-75 | D, Alkyl | O, S |
| RB | RB | Ar-1 bis Ar-75 | D, Alkyl | O, S |
| RA-5 | RA-5 | Ar-1 bis Ar-75 | Ar-1 bis Ar-75 | O, S |
| RA-4 | RA-4 | Ar-1 bis Ar-75 | Ar-1 bis Ar-75 | O, S |
| RA-4f | RA-4f | Ar-1 bis Ar-75 | Ar-1 bis Ar-75 | O, S |
| RA-3 | RA-3 | Ar-1 bis Ar-75 | Ar-1 bis Ar-75 | O, S |
| RB | RB | Ar-1 bis Ar-75 | Ar-1 bis Ar-75 | O, S |
| RA-5 | RA-5 | H, D, Alkyl | Phenyl-Ringbildung von 2 R^{e} | O, S |
| RA-4 | RA-4 | H, D, Alkyl | Phenyl-Ringbildung von 2 R^{e} | O, S |
| RA-4f | RA-4f | H, D, Alkyl | Phenyl-Ringbildung von 2 R^{e} | O, S |
| RA-3 | RA-3 | H, D, Alkyl | Phenyl-Ringbildung von 2 R^{e} | O, S |
| RB | RB | H, D, Alkyl | Phenyl-Ringbildung von 2 R^{e} | O, S |
| RA-5 | RA-5 | Ar-1 bis Ar-75 | Phenyl-Ringbildung von 2 R^{e} | O, S |
| RA-4 | RA-4 | Ar-1 bis Ar-75 | Phenyl-Ringbildung von 2 R^{e} | O, S |
| RA-4f | RA-4f | Ar-1 bis Ar-75 | Phenyl-Ringbildung von 2 R^{e} | O, S |
| RA-3 | RA-3 | Ar-1 bis Ar-75 | Phenyl-Ringbildung von 2 R^{e} | O, S |
| RB | RB | Ar-1 bis Ar-75 | Phenyl-Ringbildung von 2 R^{e} | O, S |
| RA-5 | RA-5 | H, D, Alkyl | C(Ar')₃, Si(Ar')₃ | O, S |
| RA-4 | RA-4 | H, D, Alkyl | C(Ar')₃, Si(Ar')₃ | O, S |
| RA-4f | RA-4f | H, D, Alkyl | C(Ar')₃, Si(Ar')₃ | O, S |
| RA-3 | RA-3 | H, D, Alkyl | C(Ar')₃, Si(Ar')₃ | O, S |
| RB | RB | H, D, Alkyl | C(Ar')₃, Si(Ar')₃ | O, S |
| RA-5 | RA-5 | Ar-1 bis Ar-75 | C(Ar')₃, | O, S |
| RA-4 | RA-4 | Ar-1 bis Ar-75 | C(Ar')₃, Si(Ar')₃ | O, S |
| RA-4f | RA-4f | Ar-1 bis Ar-75 | C(Ar')₃, Si(Ar')₃ | O, S |
| RA-3 | RA-3 | Ar-1 bis Ar-75 | C(Ar')₃, Si(Ar')₃ | O, S |
| RB | RB | Ar-1 bis Ar-75 | C(Ar')₃, Si(Ar')₃ | O, S |
| RA-5 | RA-5 | H, D, Alkyl | N(Ar')₃, N(R¹)₃ | O, S |
| RA-4 | RA-4 | H, D, Alkyl | N(Ar')₃, N(R¹)₃ | O, S |
| RA-4f | RA-4f | H, D, Alkyl | N(Ar')₃, N(R¹)₃ | O, S |
| RA-3 | RA-3 | H, D, Alkyl | N(Ar')₃, N(R¹)₃ | O, S |
| RB | RB | H, D, Alkyl | N(Ar')₃, N(R¹)₃ | O, S |
| RA-5 | RA-5 | Ar-1 bis Ar-75 | N(Ar')₃, N(R¹)₃ | O, S |
| RA-4 | RA-4 | Ar-1 bis Ar-75 | N(Ar')₃, N(R¹)₃ | O, S |
| RA-4f | RA-4f | Ar-1 bis Ar-75 | N(Ar')₃, N(R¹)₃ | O, S |
| RA-3 | RA-3 | Ar-1 bis Ar-75 | N(Ar')₃, N(R¹)₃ | O, S |
| RB | RB | Ar-1 bis Ar-75 | N(Ar')₃, N(R¹)₃ | O, S |
| RA-5 | RA-5 | H, D, Alkyl | D, Alkyl | N(Ar') |
| RA-4 | RA-4 | H, D, Alkyl | D, Alkyl | N(Ar') |
| RA-4f | RA-4f | H, D, Alkyl | D, Alkyl | N(Ar') |
| RA-3 | RA-3 | H, D, Alkyl | D, Alkyl | N(Ar') |
| RB | RB | H, D, Alkyl | D, Alkyl | N(Ar') |
| RA-5 | RA-5 | H, D, Alkyl | Ar-1 bis Ar-75 | N(Ar') |
| RA-4 | RA-4 | H, D, Alkyl | Ar-1 bis Ar-75 | N(Ar') |
| RA-4f | RA-4f | H, D, Alkyl | Ar-1 bis Ar-75 | N(Ar') |
| RA-3 | RA-3 | H, D, Alkyl | Ar-1 bis Ar-75 | N(Ar') |
| RB | RB | H, D, Alkyl | Ar-1 bis Ar-75 | N(Ar') |
| RA-5 | RA-5 | Ar-1 bis Ar-75 | D, Alkyl | N(Ar') |
| RA-4 | RA-4 | Ar-1 bis Ar-75 | D, Alkyl | N(Ar') |
| RA-4f | RA-4f | Ar-1 bis Ar-75 | D, Alkyl | N(Ar') |
| RA-3 | RA-3 | Ar-1 bis Ar-75 | D, Alkyl | N(Ar') |
| RB | RB | Ar-1 bis Ar-75 | D, Alkyl | N(Ar') |
| RA-5 | RA-5 | Ar-1 bis Ar-75 | Ar-1 bis Ar-75 | N(Ar') |
| RA-4 | RA-4 | Ar-1 bis Ar-75 | Ar-1 bis Ar-75 | N(Ar') |
| RA-4f | RA-4f | Ar-1 bis Ar-75 | Ar-1 bis Ar-75 | N(Ar') |
| RA-3 | RA-3 | Ar-1 bis Ar-75 | Ar-1 bis Ar-75 | N(Ar') |
| RB | RB | Ar-1 bis Ar-75 | Ar-1 bis Ar-75 | N(Ar') |
| RA-5 | RA-5 | H, D, Alkyl | Phenyl-Ringbildung von 2 R^{e} | N(Ar') |
| RA-4 | RA-4 | H, D, Alkyl | Phenyl-Ringbildung von 2 R^{e} | N(Ar') |
| RA-4f | RA-4f | H, D, Alkyl | Phenyl-Ringbildung von 2 R^{e} | N(Ar') |
| RA-3 | RA-3 | H, D, Alkyl | Phenyl-Ringbildung von 2 R^{e} | N(Ar') |
| RB | RB | H, D, Alkyl | Phenyl-Ringbildung von 2 R^{e} | N(Ar') |
| RA-5 | RA-5 | Ar-1 bis Ar-75 | Phenyl-Ringbildung von 2 R^{e} | N(Ar') |
| RA-4 | RA-4 | Ar-1 bis Ar-75 | Phenyl-Ringbildung von 2 R^{e} | N(Ar') |
| RA-4f | RA-4f | Ar-1 bis Ar-75 | Phenyl-Ringbildung von 2 R^{e} | N(Ar') |
| RA-3 | RA-3 | Ar-1 bis Ar-75 | Phenyl-Ringbildung von 2 R^{e} | N(Ar') |
| RB | RB | Ar-1 bis Ar-75 | Phenyl-Ringbildung von 2 R^{e} | N(Ar') |
| RA-5 | RA-5 | H, D, Alkyl | C(Ar')₃, Si(Ar')₃ | N(Ar') |
| RA-4 | RA-4 | H, D, Alkyl | C(Ar')₃, Si(Ar')₃ | N(Ar') |
| RA-4f | RA-4f | H, D, Alkyl | C(Ar')₃, Si(Ar')₃ | N(Ar') |
| RA-3 | RA-3 | H, D, Alkyl | C(Ar')₃, Si(Ar')₃ | N(Ar') |
| RB | RB | H, D, Alkyl | C(Ar')₃, Si(Ar')₃ | N(Ar') |
| RA-5 | RA-5 | Ar-1 bis Ar-75 | C(Ar')₃, Si(Ar')₃ | N(Ar') |
| RA-4 | RA-4 | Ar-1 bis Ar-75 | C(Ar')₃, Si(Ar')₃ | N(Ar') |
| RA-4f | RA-4f | Ar-1 bis Ar-75 | C(Ar')₃, Si(Ar')₃ | N(Ar') |
| RA-3 | RA-3 | Ar-1 bis Ar-75 | C(Ar')₃, Si(Ar')₃ | N(Ar') |
| RB | RB | Ar-1 bis Ar-75 | C(Ar')₃, Si(Ar')₃ | N(Ar') |
| RA-5 | RA-5 | H, D, Alkyl | N(Ar')₃, N(R¹)₃ | N(Ar') |
| RA-4 | RA-4 | H, D, Alkyl | N(Ar')₃, N(R¹)₃ | N(Ar') |
| RA-4f | RA-4f | H, D, Alkyl | N(Ar')₃, N(R¹)₃ | N(Ar') |
| RA-3 | RA-3 | H, D, Alkyl | N(Ar')₃, N(R¹)₃ | N(Ar') |
| RB | RB | H, D, Alkyl | N(Ar')₃, N(R¹)₃ | N(Ar') |
| RA-5 | RA-5 | Ar-1 bis Ar-75 | N(Ar')₃, N(R¹)₃ | N(Ar') |
| RA-4 | RA-4 | Ar-1 bis Ar-75 | N(Ar')₃, N(R¹)₃ | N(Ar') |
| RA-4f | RA-4f | Ar-1 bis Ar-75 | N(Ar')₃, N(R¹)₃ | N(Ar') |
| RA-3 | RA-3 | Ar-1 bis Ar-75 | N(Ar')₃, N(R¹)₃ | N(Ar') |
| RB | RB | Ar-1 bis Ar-75 | N(Ar')₃, N(R¹)₃ | N(Ar') |
| RA-5 | RA-5 | H, D, Alkyl | D, Alkyl | (R^{e})₂C-C(R^{e})₂, (R^{e})C=C(R^{e}) |
| RA-4 | RA-4 | H, D, Alkyl | D, Alkyl | (R^{e})₂C-C(R^{e})₂, (R^{e})C=C(R^{e}) |
| RA-4f | RA-4f | H, D, Alkyl | D, Alkyl | (R^{e})₂C-C(R^{e})₂, (R^{e})C=C(R^{e}) |
| RA-3 | RA-3 | H, D, Alkyl | D, Alkyl | (R^{e})₂C-C(R^{e})₂, (R^{e})C=C(R^{e}) |
| RB | RB | H, D, Alkyl | D, Alkyl | (R^{e})₂C-C(R^{e})₂, (R^{e})C=C(R^{e}) |
| RA-5 | RA-5 | H, D, Alkyl | Ar-1 bis Ar-75 | (R^{e})₂C-C(R^{e})₂, (R^{e})C=C(R^{e}) |
| RA-4 | RA-4 | H, D, Alkyl | Ar-1 bis Ar-75 | (R^{e})₂C-C(R^{e})₂, (R^{e})C=C(R^{e}) |
| RA-4f | RA-4f | H, D, Alkyl | Ar-1 bis Ar-75 | (R^{e})₂C-C(R^{e})₂, (R^{e})C=C(R^{e}) |
| RA-3 | RA-3 | H, D, Alkyl | Ar-1 bis Ar-75 | (R^{e})₂C-C(R^{e})₂, (R^{e})C=C(R^{e}) |
| RB | RB | H, D, Alkyl | Ar-1 bis Ar-75 | (R^{e})₂C-C(R^{e})₂, (R^{e})C=C(R^{e}) |
| RA-5 | RA-5 | Ar-1 bis Ar-75 | D, Alkyl | (R^{e})₂C-C(R^{e})₂, (R^{e})C=C(R^{e}) |
| RA-4 | RA-4 | Ar-1 bis Ar-75 | D, Alkyl | (R^{e})₂C-C(R^{e})₂, (R^{e})C=C(R^{e}) |
| RA-4f | RA-4f | Ar-1 bis Ar-75 | D, Alkyl | (R^{e})₂C-C(R^{e})₂, (R^{e})C=C(R^{e}) |
| RA-3 | RA-3 | Ar-1 bis Ar-75 | D, Alkyl | (R^{e})₂C-C(R^{e})₂, (R^{e})C=C(R^{e}) |
| RB | RB | Ar-1 bis Ar-75 | D, Alkyl | (R^{e})₂C-C(R^{e})₂, (R^{e})C=C(R^{e}) |
| RA-5 | RA-5 | Ar-1 bis Ar-75 | Ar-1 bis Ar-75 | (R^{e})₂C-C(R^{e})₂, (R^{e})C=C(R^{e}) |
| RA-4 | RA-4 | Ar-1 bis Ar-75 | Ar-1 bis Ar-75 | (R^{e})₂C-C(R^{e})₂, (R^{e})C=C(R^{e}) |
| RA-4f | RA-4f | Ar-1 bis Ar-75 | Ar-1 bis Ar-75 | (R^{e})₂C-C(R^{e})₂, (R^{e})C=C(R^{e}) |
| RA-3 | RA-3 | Ar-1 bis Ar-75 | Ar-1 bis Ar-75 | (R^{e})₂C-C(R^{e})₂, (R^{e})C=C(R^{e}) |
| RB | RB | Ar-1 bis Ar-75 | Ar-1 bis Ar-75 | (R^{e})₂C-C(R^{e})₂, (R^{e})C=C(R^{e}) |
| RA-5 | RA-5 | H, D, Alkyl | Phenyl-Ringbildung von 2 R^{e} | (R^{e})₂C-C(R^{e})₂, (R^{e})C=C(R^{e}) |
| RA-4 | RA-4 | H, D, Alkyl | Phenyl-Ringbildung von 2 R^{e} | (R^{e})₂C-C(R^{e})₂, (R^{e})C=C(R^{e}) |
| RA-4f | RA-4f | H, D, Alkyl | Phenyl-Ringbildung von 2 R^{e} | (R^{e})₂C-C(R^{e})₂, (R^{e})C=C(R^{e}) |
| RA-3 | RA-3 | H, D, Alkyl | Phenyl-Ringbildung von 2 R^{e} | (R^{e})₂C-C(R^{e})₂, (R^{e})C=C(R^{e}) |
| RB | RB | H, D, Alkyl | Phenyl-Ringbildung von 2 R^{e} | (R^{e})₂C-C(R^{e})₂, (R^{e})C=C(R^{e}) |
| RA-5 | RA-5 | Ar-1 bis Ar-75 | Phenyl-Ringbildung von 2 R^{e} | (R^{e})₂C-C(R^{e})₂, (R^{e})C=C(R^{e}) |
| RA-4 | RA-4 | Ar-1 bis Ar-75 | Phenyl-Ringbildung von 2 R^{e} | (R^{e})₂C-C(R^{e})₂, (R^{e})C=C(R^{e}) |
| RA-4f | RA-4f | Ar-1 bis Ar-75 | Phenyl-Ringbildung von 2 R^{e} | (R^{e})₂C-C(R^{e})₂, (R^{e})C=C(R^{e}) |
| RA-3 | RA-3 | Ar-1 bis Ar-75 | Phenyl-Ringbildung von 2 R^{e} | (R^{e})₂C-C(R^{e})₂, (R^{e})C=C(R^{e}) |
| RB | RB | Ar-1 bis Ar-75 | Phenyl-Ringbildung von 2 R^{e} | (R^{e})₂C-C(R^{e})₂, (R^{e})C=C(R^{e}) |
| RA-5 | RA-5 | H, D, Alkyl | C(Ar')₃, Si(Ar')₃ | (R^{e})₂C-C(R^{e})₂, (R^{e})C=C(R^{e}) |
| RA-4 | RA-4 | H, D, Alkyl | C(Ar')₃, Si(Ar')₃ | (R^{e})₂C-C(R^{e})₂, (R^{e})C=C(R^{e}) |
| RA-4f | RA-4f | H, D, Alkyl | C(Ar')₃, Si(Ar')₃ | (R^{e})₂C-C(R^{e})₂, (R^{e})C=C(R^{e}) |
| RA-3 | RA-3 | H, D, Alkyl | C(Ar')₃, Si(Ar')₃ | (R^{e})₂C-C(R^{e})₂, (R^{e})C=C(R^{e}) |
| RB | RB | H, D, Alkyl | C(Ar')₃, Si(Ar')₃ | (R^{e})₂C-C(R^{e})₂, (R^{e})C=C(R^{e}) |
| RA-5 | RA-5 | Ar-1 bis Ar-75 | C(Ar')₃, Si(Ar')₃ | (R^{e})₂C-C(R^{e})₂, (R^{e})C=C(R^{e}) |
| RA-4 | RA-4 | Ar-1 bis Ar-75 | C(Ar')₃, Si(Ar')₃ | (R^{e})₂C-C(R^{e})₂, (R^{e})C=C(R^{e}) |
| RA-4f | RA-4f | Ar-1 bis Ar-75 | C(Ar')₃, Si(Ar')₃ | (R^{e})₂C-C(R^{e})₂, (R^{e})C=C(R^{e}) |
| RA-3 | RA-3 | Ar-1 bis Ar-75 | C(Ar')₃, Si(Ar')₃ | (R^{e})₂C-C(R^{e})₂, (R^{e})C=C(R^{e}) |
| RB | RB | Ar-1 bis Ar-75 | C(Ar')₃, Si(Ar')₃ | (R^{e})₂C-C(R^{e})₂, (R^{e})C=C(R^{e}) |
| RA-5 | RA-5 | H, D, Alkyl | N(Ar')₃, N(R¹)₃ | (R^{e})₂C-C(R^{e})₂, (R^{e})C=C(R^{e}) |
| RA-4 | RA-4 | H, D, Alkyl | N(Ar')₃, N(R¹)₃ | (R^{e})₂C-C(R^{e})₂, (R^{e})C=C(R^{e}) |
| RA-4f | RA-4f | H, D, Alkyl | N(Ar')₃, N(R¹)₃ | (R^{e})₂C-C(R^{e})₂, (R^{e})C=C(R^{e}) |
| RA-3 | RA-3 | H, D, Alkyl | N(Ar')₃, N(R¹)₃ | (R^{e})₂C-C(R^{e})₂, (R^{e})C=C(R^{e}) |
| RB | RB | H, D, Alkyl | N(Ar')₃, N(R¹)₃ | (R^{e})₂C-C(R^{e})₂, (R^{e})C=C(R^{e}) |
| RA-5 | RA-5 | Ar-1 bis Ar-75 | N(Ar')₃, N(R¹)₃ | (R^{e})₂C-C(R^{e})₂, (R^{e})C=C(R^{e}) |
| RA-4 | RA-4 | Ar-1 bis Ar-75 | N(Ar')₃, N(R¹)₃ | (R^{e})₂C-C(R^{e})₂, (R^{e})C=C(R^{e}) |
| RA-4f | RA-4f | Ar-1 bis Ar-75 | N(Ar')₃, N(R¹)₃ | (R^{e})₂C-C(R^{e})₂, (R^{e})C=C(R^{e}) |
| RA-3 | RA-3 | Ar-1 bis Ar-75 | N(Ar')₃, N(R¹)₃ | (R^{e})₂C-C(R^{e})₂, (R^{e})C=C(R^{e}) |
| RB | RB | Ar-1 bis Ar-75 | N(Ar')₃, N(R¹)₃ | (R^{e})₂C-C(R^{e})₂, (R^{e})C=C(R^{e}) |

In obiger Tabelle stehen die in der Spalte unter der Gruppe R^{e} genannten Reste für die Substituenten am Phenylring der mit der Gruppe Y¹ verbunden ist. In der Gruppe C(R^{e})₂ steht der Rest R^{e} insbesondere für die zuvor dargelegten Gruppen, wobei R^{e} in der Gruppe C(R^{e})₂ vorzugsweise gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24, vorzugsweise mit 5 bis 13 aromatischen Ringatomen, welches auch durch einen oder mehrere Reste R¹ substituiert sein kann. Ganz besonders bevorzugt steht R^{e} für eine Methylgruppe oder für eine Phenylgruppe. Dabei können die Reste R^{e} auch miteinander ein Ringsystem bilden, was zu einem Spirosystem führt.

In den Gruppen (R^{e})₂C-C(R^{e})₂, (R^{e})C=C(R^{e}) steht der Rest R^{e} insbesondere für die zuvor dargelegten Gruppen, wobei R^{e} in Gruppen (R^{e})₂C-C(R^{e})₂, (R^{e})C=C(R^{e}) vorzugsweise für H, C₁ bis C₄-Alkyl, oder für eine Aryl- oder Heteroarylgruppe mit 5 bis 13 C-Atomen steht, wobei die Aryl- oder Heteroarylgruppe verknüpft sein kann. Hierbei können zwei Gruppen R^{e} in der Gruppe (R^{e})₂C-C(R^{e})₂ oder (R^{e})C=C(R^{e}) ein kondensiertes Ringsystem bilden.

In einer weiteren Ausgestaltung der vorliegenden Erfindung sind Verbindungen umfassend eine Struktur gemäß Formel (I-6), vorzugsweise Verbindungen gemäß Formel (I-6) bevorzugt, wobei die beiden Reste R^{a} einen Ring bilden, die beiden Reste R^{c} einen Ring bilden, wobei die Summe der Indices m und n vorzugsweise kleiner oder gleich 4, besonders bevorzugt 0, 1 oder 2 und speziell bevorzugt 0 oder 1 ist, und bei denen die Reste R^{a}, R^{b}, R^{c}, R^{d} und R^{e} folgenden Bedeutungen aufweisen:

| R^{a} | R^{b} | R^{c} | R^{d} | R^{e} (nur falls n oder m ungleich 0 ist mindestens ein Rest R^{e}, andernfalls sind alle R^{e} H) |
|---|---|---|---|---|
| RA-5 | H, D, Alkyl | RA-5 | H, D, Alkyl | D, Alkyl |
| RA-4 | H, D, Alkyl | RA-4 | H, D, Alkyl | D, Alkyl |
| RA-4f | H, D, Alkyl | RA-4f | H, D, Alkyl | D, Alkyl |
| RA-3 | H, D, Alkyl | RA-3 | H, D, Alkyl | D, Alkyl |
| RB | H, D, Alkyl | RB | H, D, Alkyl | D, Alkyl |
| RA-5 | H, D, Alkyl | RA-5 | H, D, Alkyl | Ar-1 bis Ar-75 |
| RA-4 | H, D, Alkyl | RA-4 | H, D, Alkyl | Ar-1 bis Ar-75 |
| RA-4f | H, D, Alkyl | RA-4f | H, D, Alkyl | Ar-1 bis Ar-75 |
| RA-3 | H, D, Alkyl | RA-3 | H, D, Alkyl | Ar-1 bis Ar-75 |
| RB | H, D, Alkyl | RB | H, D, Alkyl | Ar-1 bis Ar-75 |
| RA-5 | H, D, Alkyl | RA-5 | Ar-1 bis Ar-75 | D, Alkyl |
| RA-4 | H, D, Alkyl | RA-4 | Ar-1 bis Ar-75 | D, Alkyl |
| RA-4f | H, D, Alkyl | RA-4f | Ar-1 bis Ar-75 | D, Alkyl |
| RA-3 | H, D, Alkyl | RA-3 | Ar-1 bis Ar-75 | D, Alkyl |
| RB | H, D, Alkyl | RB | Ar-1 bis Ar-75 | D, Alkyl |
| RA-5 | H, D, Alkyl | RA-5 | Ar-1 bis Ar-75 | Ar-1 bis Ar-75 |
| RA-4 | H, D, Alkyl | RA-4 | Ar-1 bis Ar-75 | Ar-1 bis Ar-75 |
| RA-4f | H, D, Alkyl | RA-4f | Ar-1 bis Ar-75 | Ar-1 bis Ar-75 |
| RA-3 | H, D, Alkyl | RA-3 | Ar-1 bis Ar-75 | Ar-1 bis Ar-75 |
| RB | H, D, Alkyl | RB | Ar-1 bis Ar-75 | Ar-1 bis Ar-75 |
| RA-5 | H, D, Alkyl | RA-5 | H, D, Alkyl und Ar-1 bis Ar-75 | D, Alkyl |
| RA-4 | H, D, Alkyl | RA-4 | H, D, Alkyl und Ar-1 bis Ar-75 | D, Alkyl |
| RA-4f | H, D, Alkyl | RA-4f | H, D, Alkyl und Ar-1 bis Ar-75 | D, Alkyl |
| RA-3 | H, D, Alkyl | RA-3 | H, D, Alkyl und Ar-1 bis Ar-75 | D, Alkyl |
| RB | H, D, Alkyl | RB | H, D, Alkyl und Ar-1 bis Ar-75 | D, Alkyl |
| RA-5 | H, D, Alkyl | RA-5 | H, D, Alkyl und Ar-1 bis Ar-75 | Ar-1 bis Ar-75 |
| RA-4 | H, D, Alkyl | RA-4 | H, D, Alkyl und Ar-1 bis Ar-75 | Ar-1 bis Ar-75 |
| RA-4f | H, D, Alkyl | RA-4f | H, D, Alkyl und Ar-1 bis Ar-75 | Ar-1 bis Ar-75 |
| RA-3 | H, D, Alkyl | RA-3 | H, D, Alkyl und Ar-1 bis Ar-75 | Ar-1 bis Ar-75 |
| RB | H, D, Alkyl | RB | H, D, Alkyl und Ar-1 bis Ar-75 | Ar-1 bis Ar-75 |
| RA-5 | H, D, Alkyl | RA-5 | H, D, Alkyl | C(Ar')₃, Si(Ar')₃ |
| RA-4 | H, D, Alkyl | RA-4 | H, D, Alkyl | C(Ar')₃, Si(Ar')₃ |
| RA-4f | H, D, Alkyl | RA-4f | H, D, Alkyl | C(Ar')₃, Si(Ar')₃ |
| RA-3 | H, D, Alkyl | RA-3 | H, D, Alkyl | C(Ar')₃, Si(Ar')₃ |
| RB | H, D, Alkyl | RB | H, D, Alkyl | C(Ar')₃, Si(Ar')₃ |
| RA-5 | H, D, Alkyl | RA-5 | Ar-1 bis Ar-75 | C(Ar')₃, Si(Ar')₃ |
| RA-4 | H, D, Alkyl | RA-4 | Ar-1 bis Ar-75 | C(Ar')₃, Si(Ar')₃ |
| RA-4f | H, D, Alkyl | RA-4f | Ar-1 bis Ar-75 | C(Ar')₃, Si(Ar')₃ |
| RA-3 | H, D, Alkyl | RA-3 | Ar-1 bis Ar-75 | C(Ar')₃, Si(Ar')₃ |
| RB | H, D, Alkyl | RB | Ar-1 bis Ar-75 | C(Ar')₃, Si(Ar')₃ |
| RA-5 | H, D, Alkyl | RA-5 | H, D, Alkyl und Ar-1 bis Ar-75 | C(Ar')₃, Si(Ar')₃ |
| RA-4 | H, D, Alkyl | RA-4 | H, D, Alkyl und Ar-1 bis Ar-75 | C(Ar')₃, Si(Ar')₃ |
| RA-4f | H, D, Alkyl | RA-4f | H, D, Alkyl und Ar-1 bis Ar-75 | C(Ar')₃, Si(Ar')₃ |
| RA-3 | H, D, Alkyl | RA-3 | H, D, Alkyl und Ar-1 bis Ar-75 | C(Ar')₃, Si(Ar')₃ |
| RB | H, D, Alkyl | RB | H, D, Alkyl und Ar-1 bis Ar-75 | C(Ar')₃, Si(Ar')₃ |
| RA-5 | H, D, Alkyl | RA-5 | H, D, Alkyl | N(Ar')₃, N(R¹)₃ |
| RA-4 | H, D, Alkyl | RA-4 | H, D, Alkyl | N(Ar')₃, N(R¹)₃ |
| RA-4f | H, D, Alkyl | RA-4f | H, D, Alkyl | N(Ar')₃, N(R¹)₃ |
| RA-3 | H, D, Alkyl | RA-3 | H, D, Alkyl | N(Ar')₃, N(R¹)₃ |
| RB | H, D, Alkyl | RB | H, D, Alkyl | N(Ar')₃, N(R¹)₃ |
| RA-5 | H, D, Alkyl | RA-5 | Ar-1 bis Ar-75 | N(Ar')₃, N(R¹)₃ |
| RA-4 | H, D, Alkyl | RA-4 | Ar-1 bis Ar-75 | N(Ar')₃, N(R¹)₃ |
| RA-4f | H, D, Alkyl | RA-4f | Ar-1 bis Ar-75 | N(Ar')₃, N(R¹)₃ |
| RA-3 | H, D, Alkyl | RA-3 | Ar-1 bis Ar-75 | N(Ar')₃, N(R¹)₃ |
| RB | H, D, Alkyl | RB | Ar-1 bis Ar-75 | N(Ar')₃, N(R¹)₃ |
| RA-5 | H, D, Alkyl | RA-5 | H, D, Alkyl und Ar-1 bis Ar-75 | N(Ar')₃, N(R¹)₃ |
| RA-4 | H, D, Alkyl | RA-4 | H, D, Alkyl und Ar-1 bis Ar-75 | N(Ar')₃, N(R¹)₃ |
| RA-4f | H, D, Alkyl | RA-4f | H, D, Alkyl und Ar-1 bis Ar-75 | N(Ar')₃, N(R¹)₃ |
| RA-3 | H, D, Alkyl | RA-3 | H, D, Alkyl und Ar-1 bis Ar-75 | N(Ar')₃, N(R¹)₃ |
| RB | H, D, Alkyl | RB | H, D, Alkyl und Ar-1 bis Ar-75 | N(Ar')₃, N(R¹)₃ |

Der Begriff "Alkyl" in obigen Tabellen umfasst insbesondere geradkettige Alkylgruppen oder verzweigte oder cyclische Alkylgruppen gemäß der zuvor dargelegten Definition für die jeweilige Gruppe.

Der Begriff "Aryl-, Heteroaryl" in obigen Tabellen umfasst insbesondere Aryl- oder Heteroarylgruppen mit 5 bis 40 aromatischen Ringatomen gemäß der zuvor dargelegten Definition für die jeweilige Gruppe, wobei die Arylgruppen vorzugsweise 6 bis 12, besonders bevorzugt 6 Ringatome und die Heteroarylgruppen vorzugsweise 5 bis 13, besonders bevorzugt 5 Ringatome aufweisen. Besonders bevorzugt umfassen Heteroarylgruppen ein oder zwei Heteroatome, vorzugsweise N, O oder S.

Die Bezeichnungen "RA-3", RA-4"," RA-4f", "RA-5", "Ar-1", "Ar-75" beziehen sich auf die zuvor und nachfolgend dargelegten Strukturformeln.

Phenyl-Ringbildung mit einer Gruppe bedeutet, dass die beiden Gruppen zusammen eine Phenylgruppe bilden, die jeweils gemäß der zuvor dargelegten Definition für die jeweilige Gruppe mit Resten R¹ substituiert sein kann. Üblich bilden sich hierdurch mit der an das Stickstoffatom gebundenen Phenylgruppe, die durch die Reste R^{d} und R beziehungsweise R^{e} substituiert ist, eine Naphthylgruppe. Für die weiteren Ringbildungsdefinitionen gilt entsprechendes.

Der Begriff "und" insbesondere bei der Beschreibung bevorzugter Gruppen R^{d} bedeutet, dass die beiden Reste unterschiedlich sind, wobei einer der Reste R^{d} einer ersten und der zweite Rest R^{d} einer zweiten Definition entspricht. Der Ausdruck "Aryl-, Heteroaryl und Phenyl-Ringbildung mit R^{e}" bedeutet, dass einer der Reste R^{d} für eine Aryl-, Heteroarylgruppe steht und der zweite Rest R^{d} mit R^{e}" einen Phenylring bildet. Falls ein Feld keinen Ausdruck "und" umfasst, so stellen alle Reste eine entsprechende Gruppe dar. Der Ausdruck "Ar-1 bis Ar-75" für die Gruppe R^{d} bedeutet, dass beide Reste R^{d} einen Aryl- oder Heteroarylrest gemäß obigen oder nachfolgenden Formeln Ar-1 bis Ar-75 darstellen. Für die weitere Verwendung des Begriffs "und" in obigen Tabellen gilt entsprechendes.

Die für die Formeln (I-1), (I-2), (I-3), (I-4), (I-5), (I-6) dargelegten Bevorzugungen hinsichtlich der verschiedenen Substituenten R^{a}, R^{b}, R^{c}, R^{d} und R^{e} sowie gegebenenfalls Y¹ gelten selbstverständlich auch für die weiteren der zuvor dargelegten Formeln (I-7), (I-8), (I-9), (I-10), (1-11), (I-12) und (I-13).

Ferner ist festzuhalten, dass diese für die Formeln (I-1), (I-2), (I-3), (I-4), (I-5), (I-6) dargelegten Bevorzugungen hinsichtlich der verschiedenen Substituenten R^{a}, R^{b}, R^{c}, R^{d} und R^{e} sowie gegebenenfalls Y¹ gelten des Weiteren für Verbindungen mit X gleich N, C-Y-R^{y}, CR^{b} gemäß Formeln (I-15) bis (I-47) und (I-65) bis (I-83).

Für den Fall, dass X gleich C-Y-R^{y} ist und die Gruppe R^{y} mit einem benachbarten Rest R^{a} ein Ring gemäß Formel (T¹) gebildet wird, gelten die für die Formeln (I-1), (I-2), (I-3), (I-4), (I-5), (I-6) dargelegten Bevorzugungen hinsichtlich der verschiedenen Substituenten R^{a}, R^{b}, R^{c}, R^{d} und R^{e} sowie gegebenenfalls Y¹ entsprechend, wobei vorzugsweise ein Ring zwischen den Gruppen R^{c} gemäß obigen Definitionen und ein Ring T¹ gemäß den entsprechenden Formeln (TCY-1) bis (TRA-13) beziehungsweise (TRA-4f) gebildet wird, wobei beispielsweise ein Ring zwischen zwei R^{c} gemäß der Formel (CY-1) einem Ring T¹ gemäß Formel (TCY-1) entspricht.

Darüber hinaus gelten die zuvor dargelegten für die Formeln (I-1), (I-2), (I-3), (I-4), (I-5), (I-6) dargelegten Bevorzugungen insbesondere hinsichtlich der verschiedenen Substituenten R^{b}, R^{d} und R^{e} sowie gegebenenfalls Y¹ für den Fall, dass die jeweils zwei Substituenten R^{a}, R^{c} keinen Ring bilden oder einen Ring der Formeln (Cy-1) bis (Cy-10), (RA-1) bis (RA-13), (RA-1a) bis (RA-4f) der in den Tabellen nicht genannt ist. Ferner gelten diese Bevorzugungen für den Fall, dass die beiden jeweils zwei Substituenten R^{a}, R^{c}, unterschiedliche Ringe der Formeln (Cy-1) bis (Cy-10), (RA-1) bis (RA-13), (RA-1a) bis (RA-4f) bilden.

Falls die jeweils zwei Substituenten R^{a}, R^{c} keinen Ring bilden, sind diese Substituenten R^{a}, R^{c} vorzugsweise ausgewählt aus H, D, Alkyl, Aryl-, Heteroaryl gemäß der zuvor dargelegten Definition für die Gruppen R^{a}, R^{c}.

Die zuvor insbesondere für die Formeln (I-1), (I-2), (I-3), (I-4), (I-5), (I-6) dargelegten Bevorzugungen hinsichtlich der verschiedenen Substituenten R^{a}, R^{b}, R^{c}, R^{d} und R^{e} sowie gegebenenfalls Y¹ und den nachfolgend hierzu dargelegten Ausführungen bezüglich Formeln (I-7) bis (I-83) sowie dem Fall, dass die Substituenten R^{a}, R^{c} keinen Ring bilden oder einen Ring der Formeln (Cy-1) bis (Cy-10), (RA-1) bis (RA-13), (RA-1a) bis (RA-4f) der in den Tabellen nicht genannt ist, gelten weiterhin für Verbindungen mit genau zwei oder drei Strukturen gemäß Formel (I) und/oder (I-1) bis (I-83) entsprechend.

Beispiele für bevorzugte Verbindungen gemäß den oben aufgeführten Ausführungsformen sind die in der folgenden Tabelle aufgeführten Verbindungen:

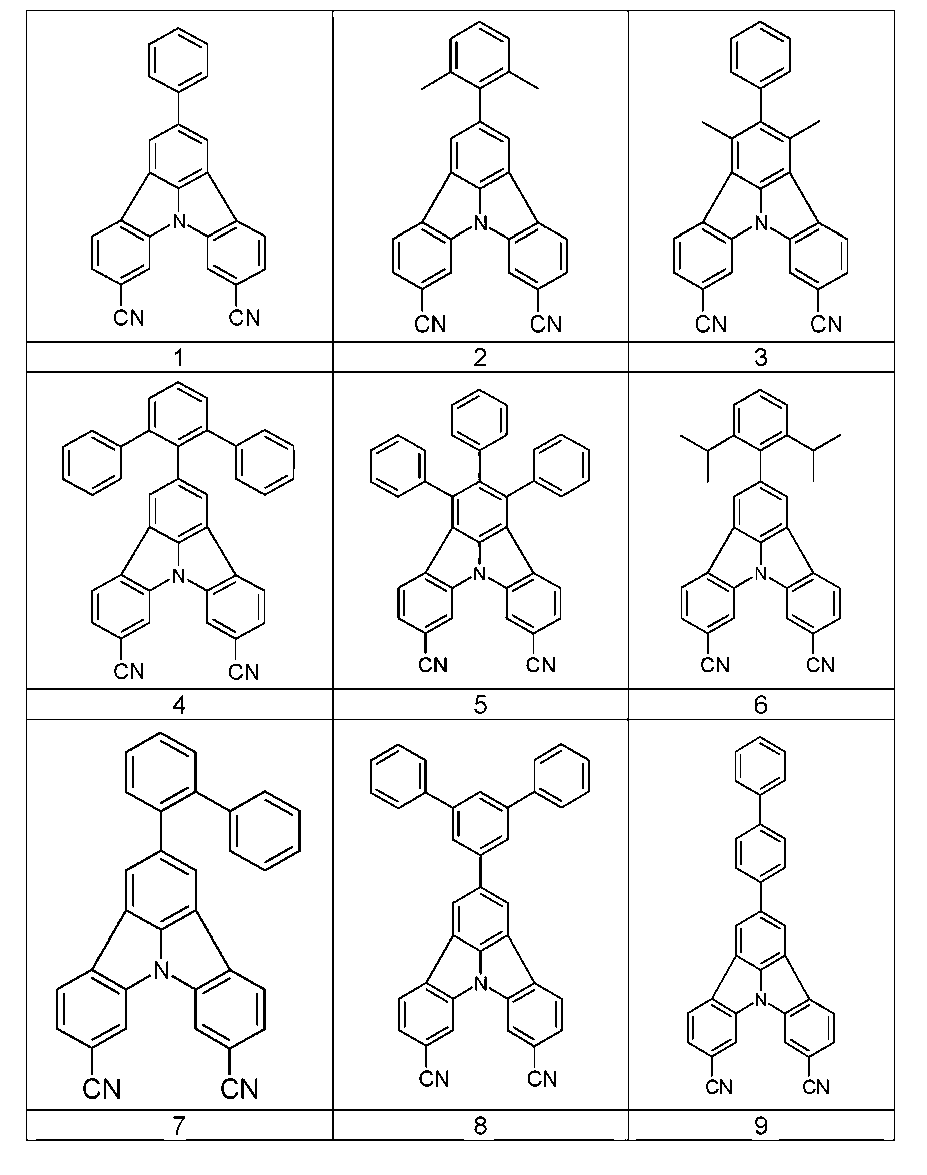

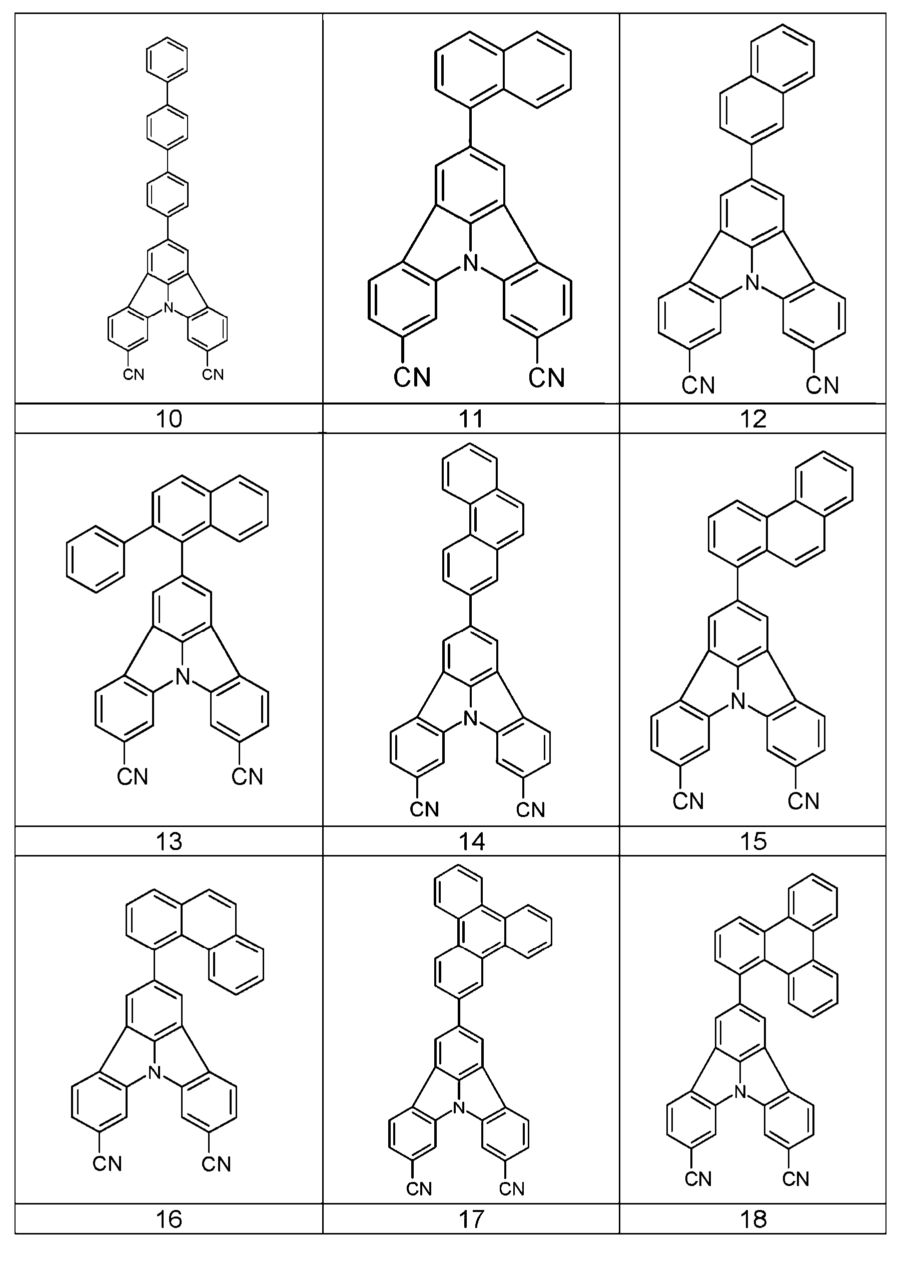

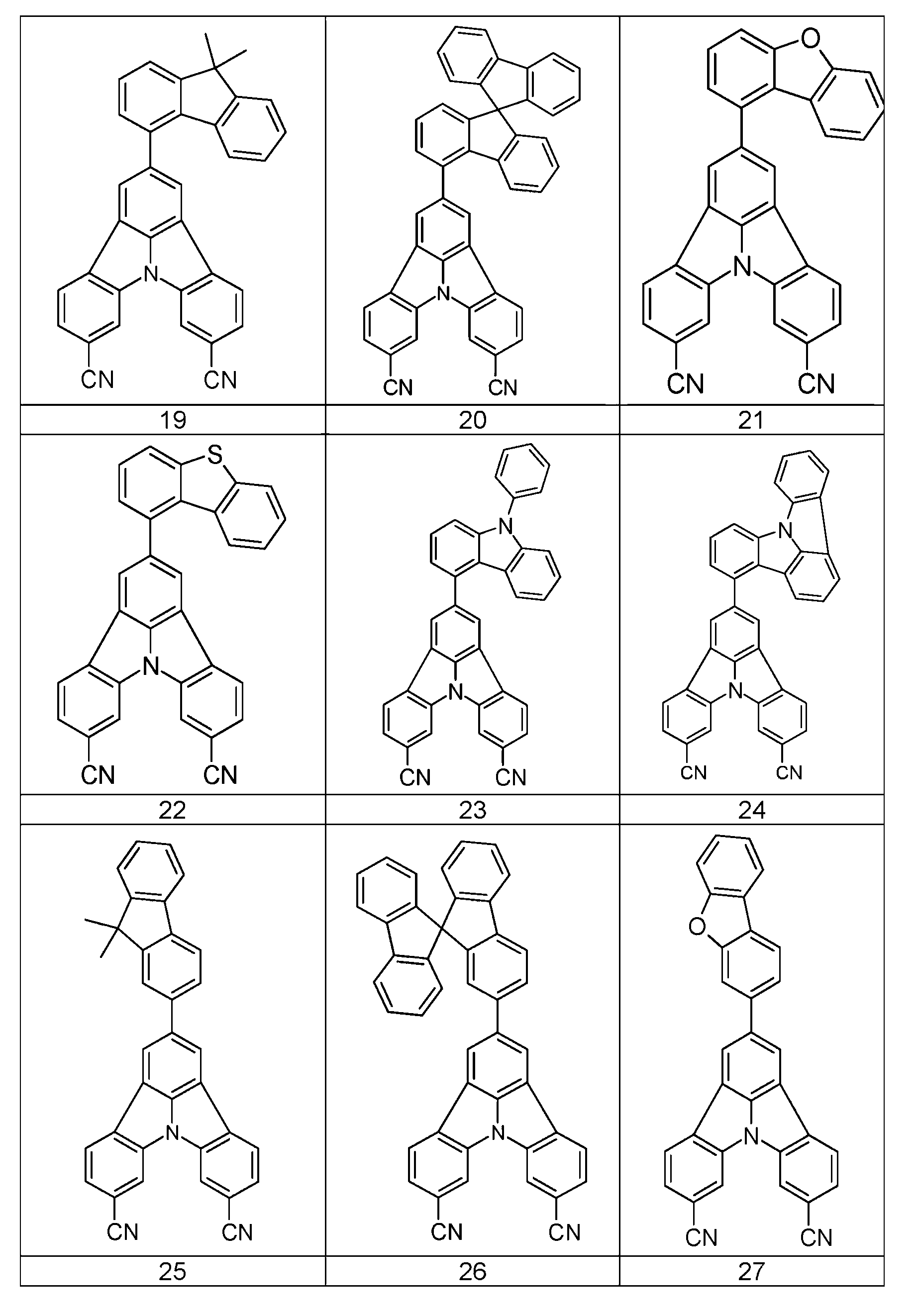

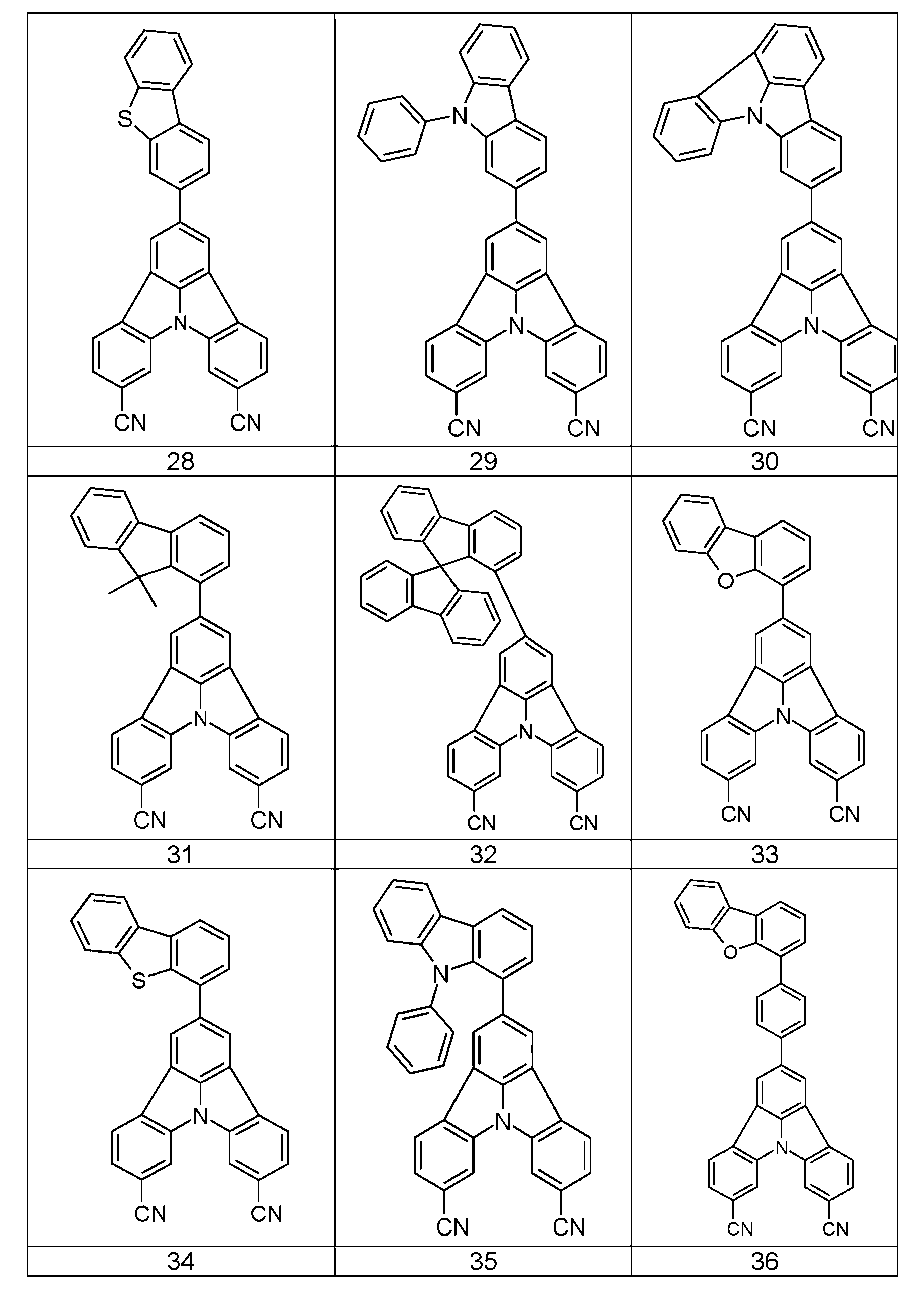

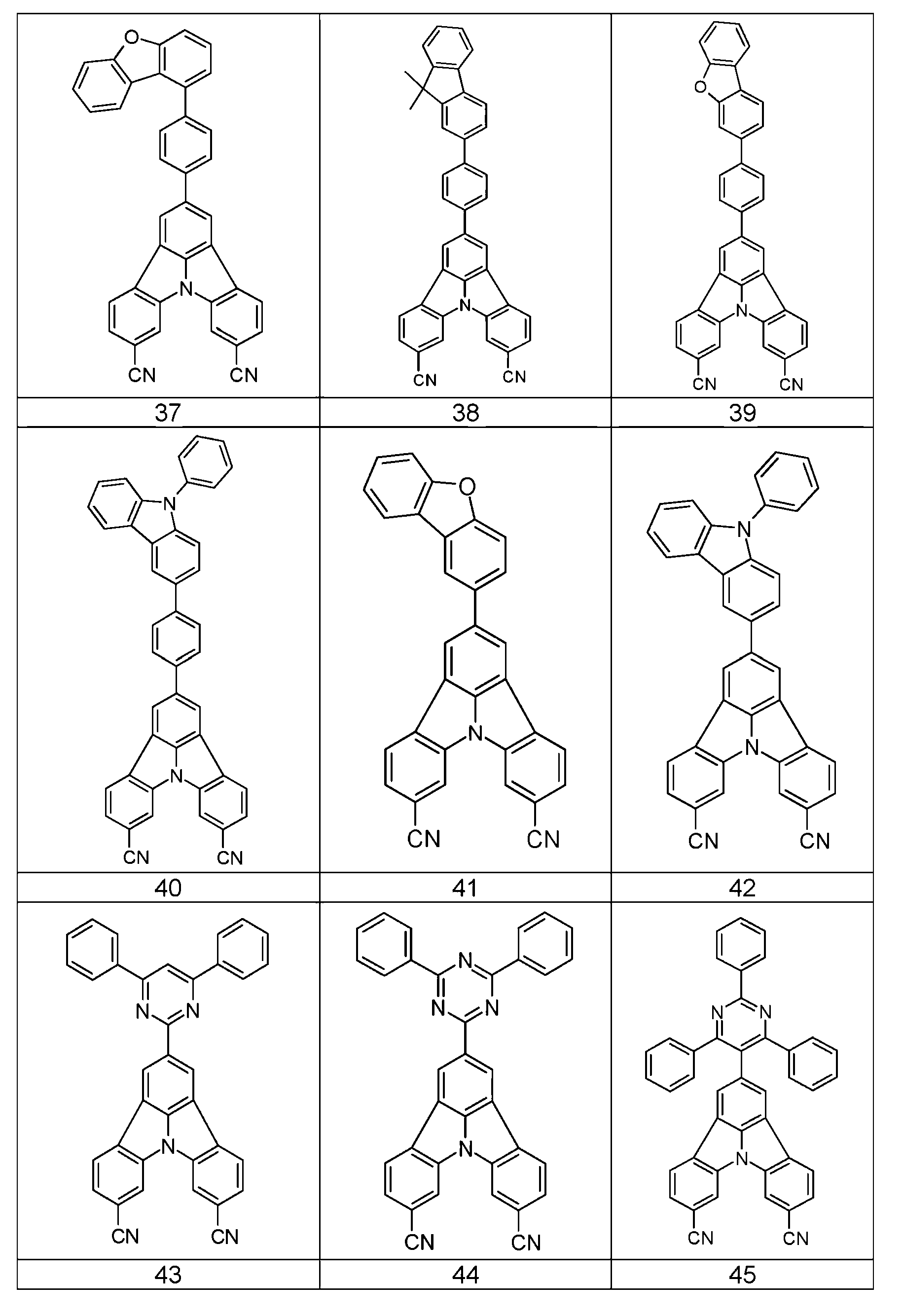

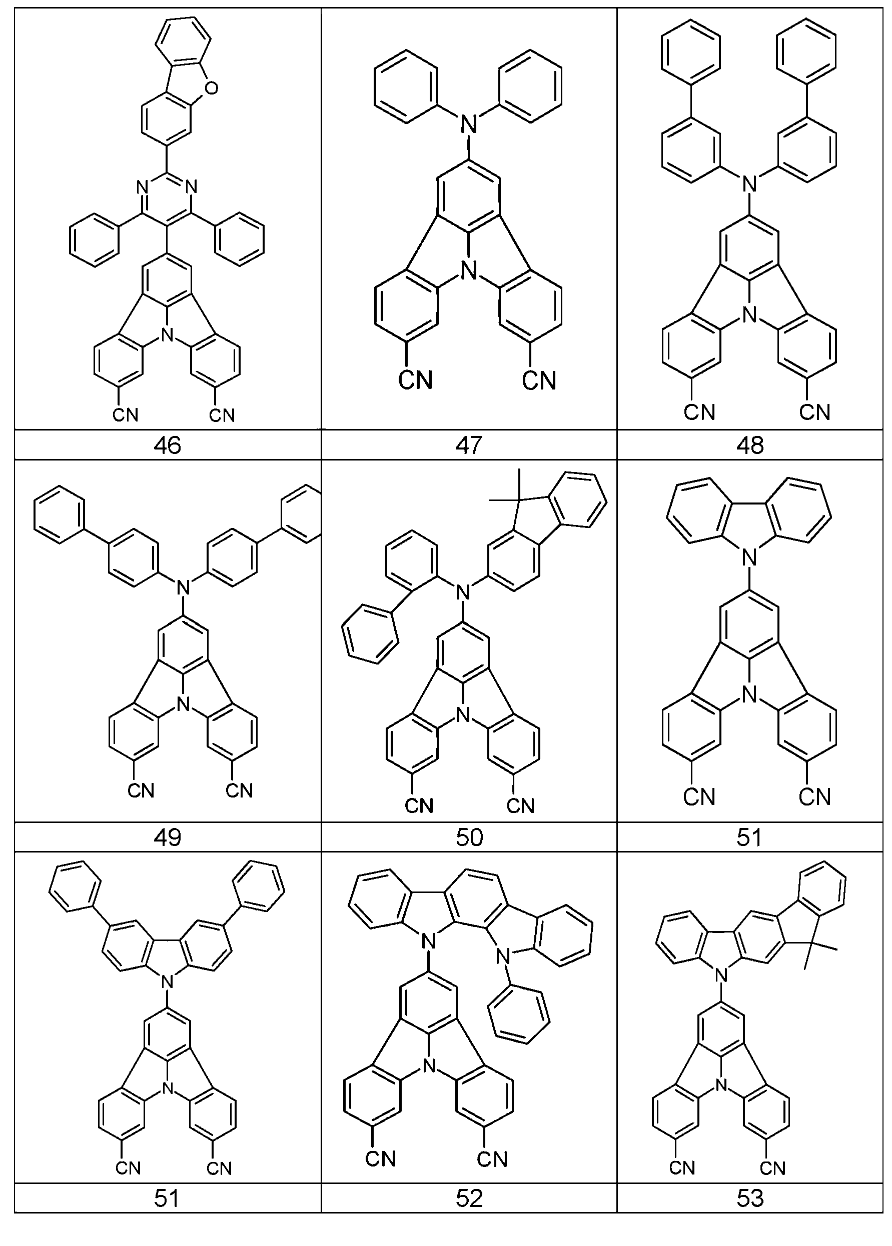

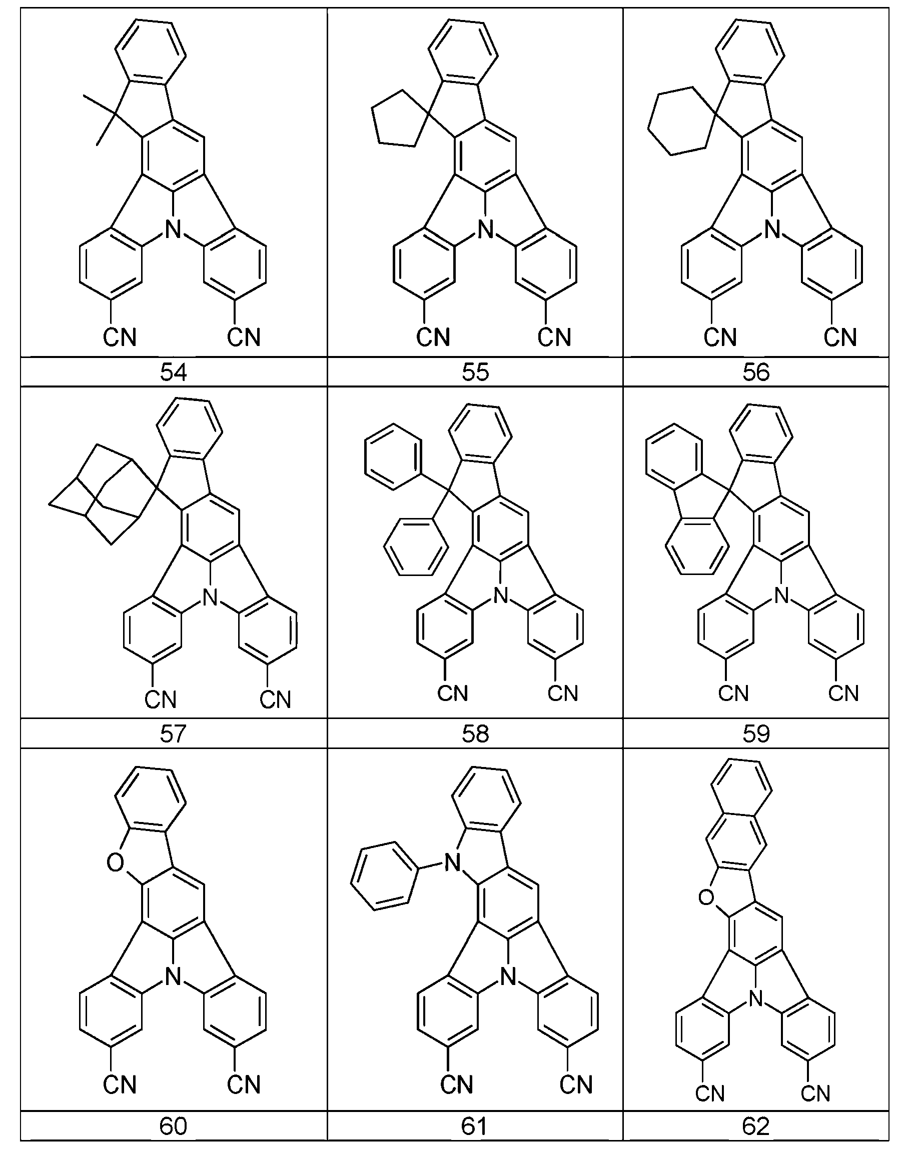

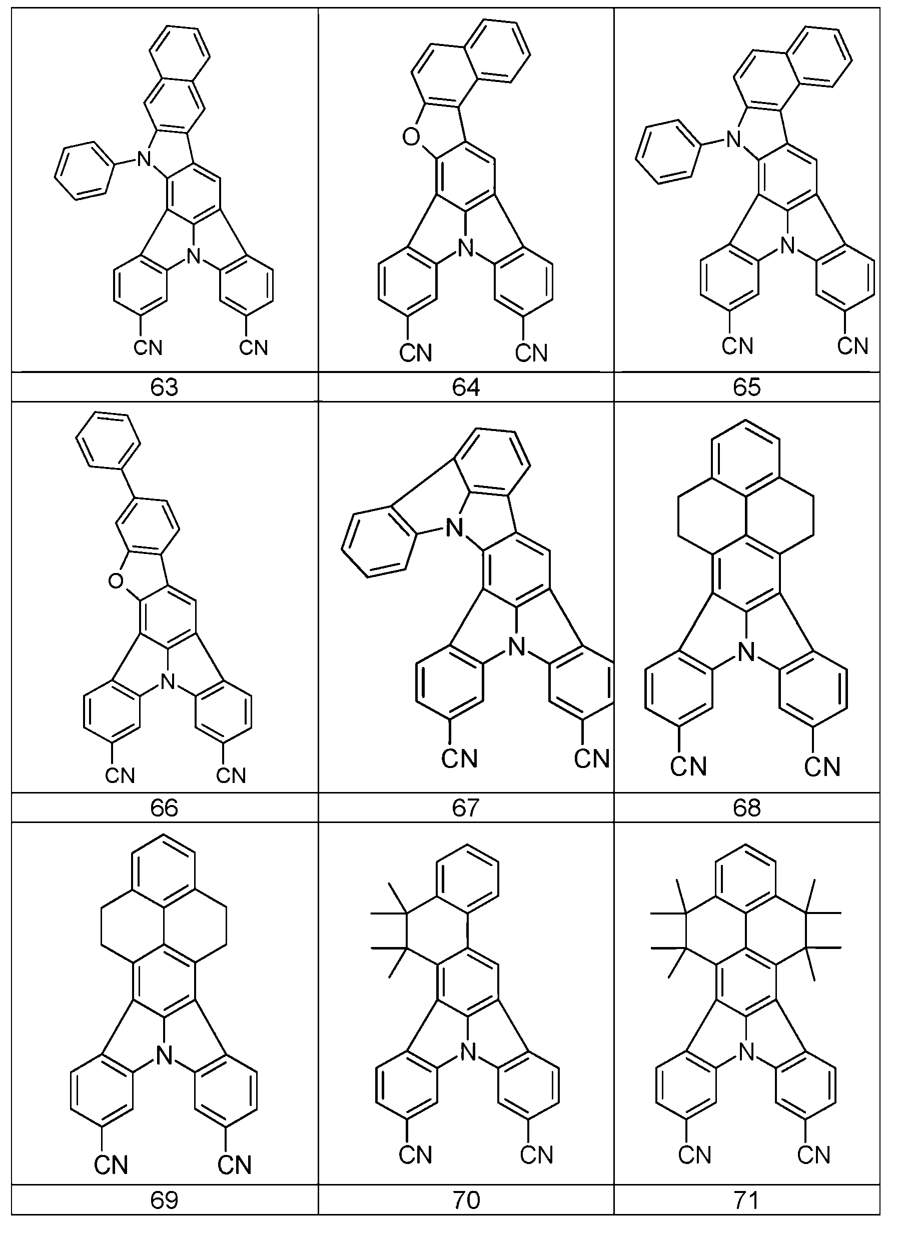

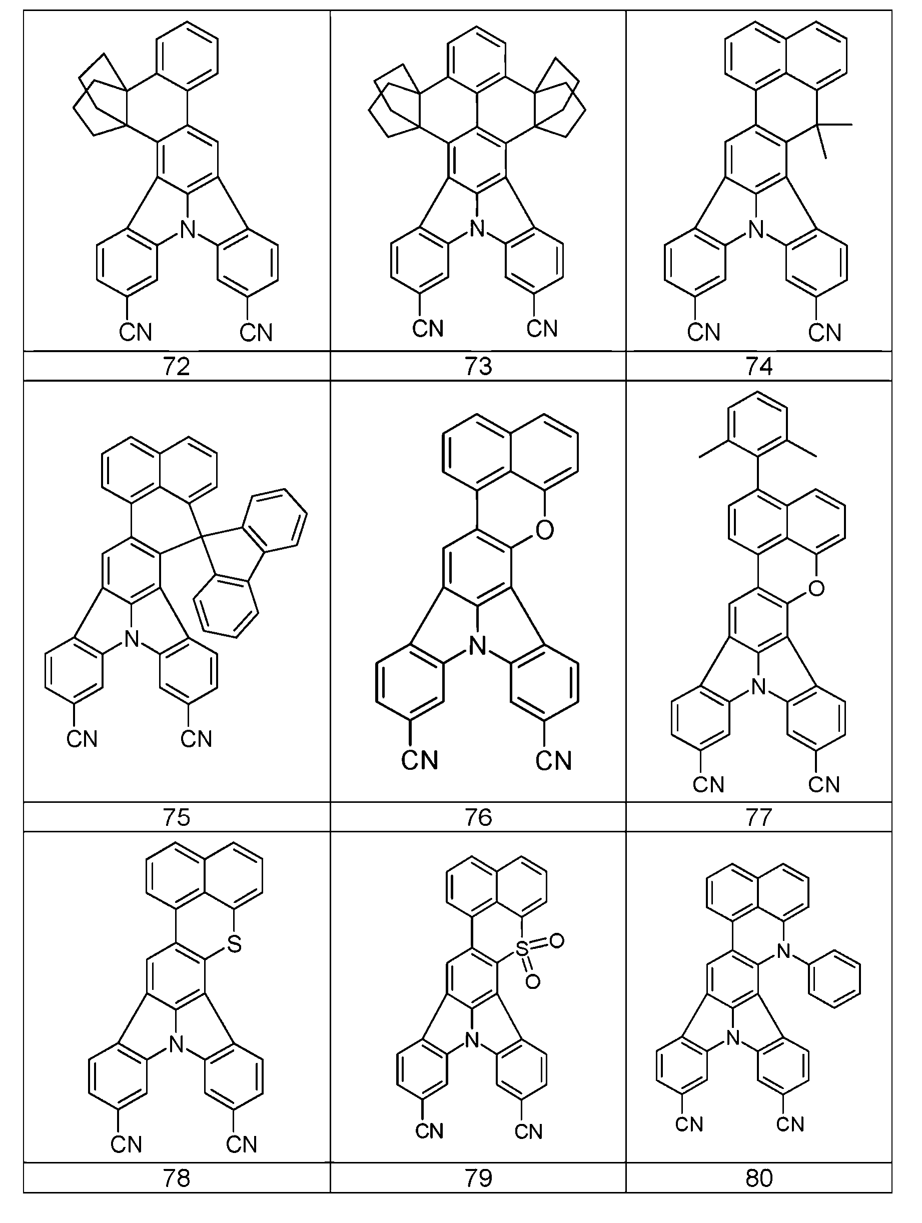

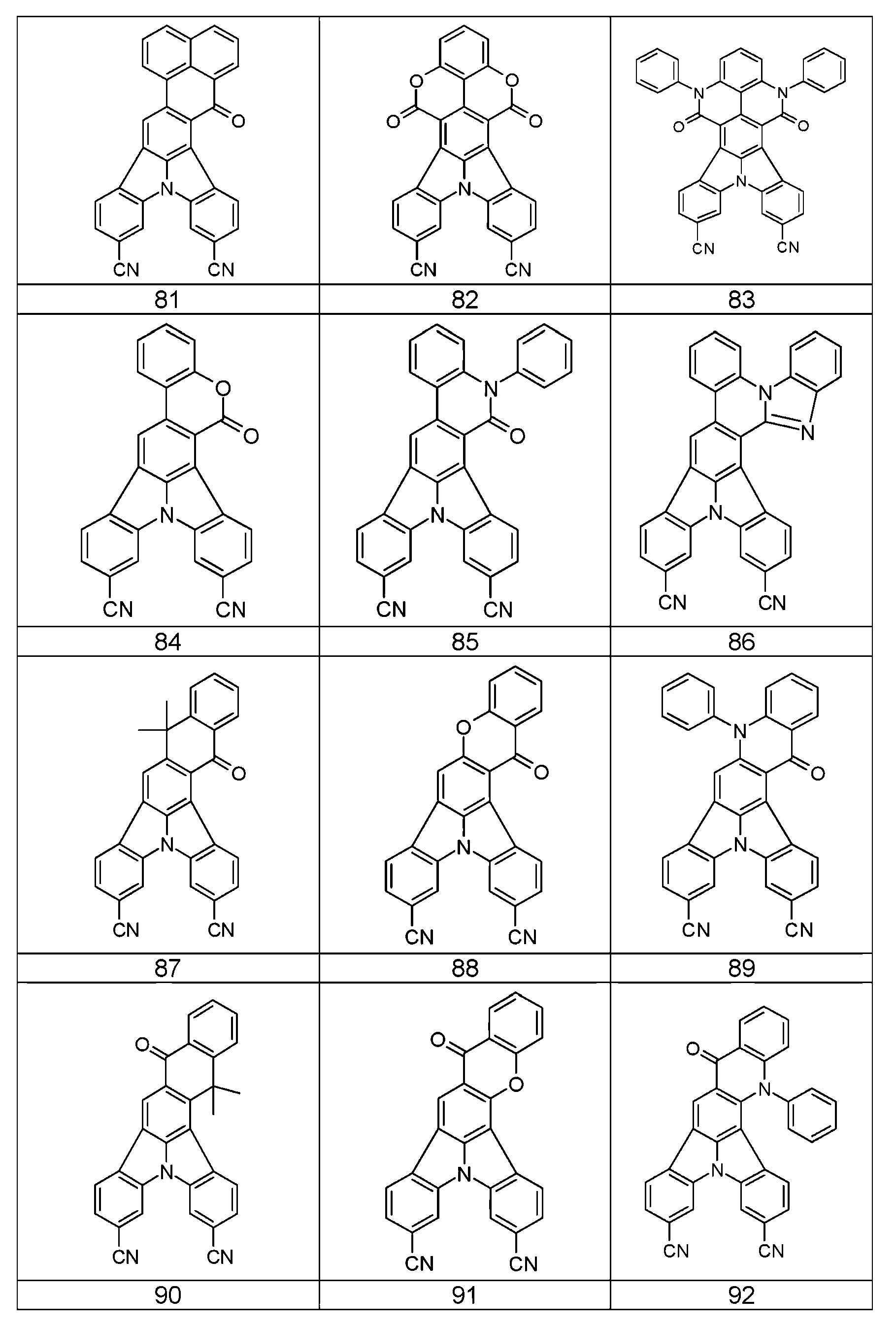

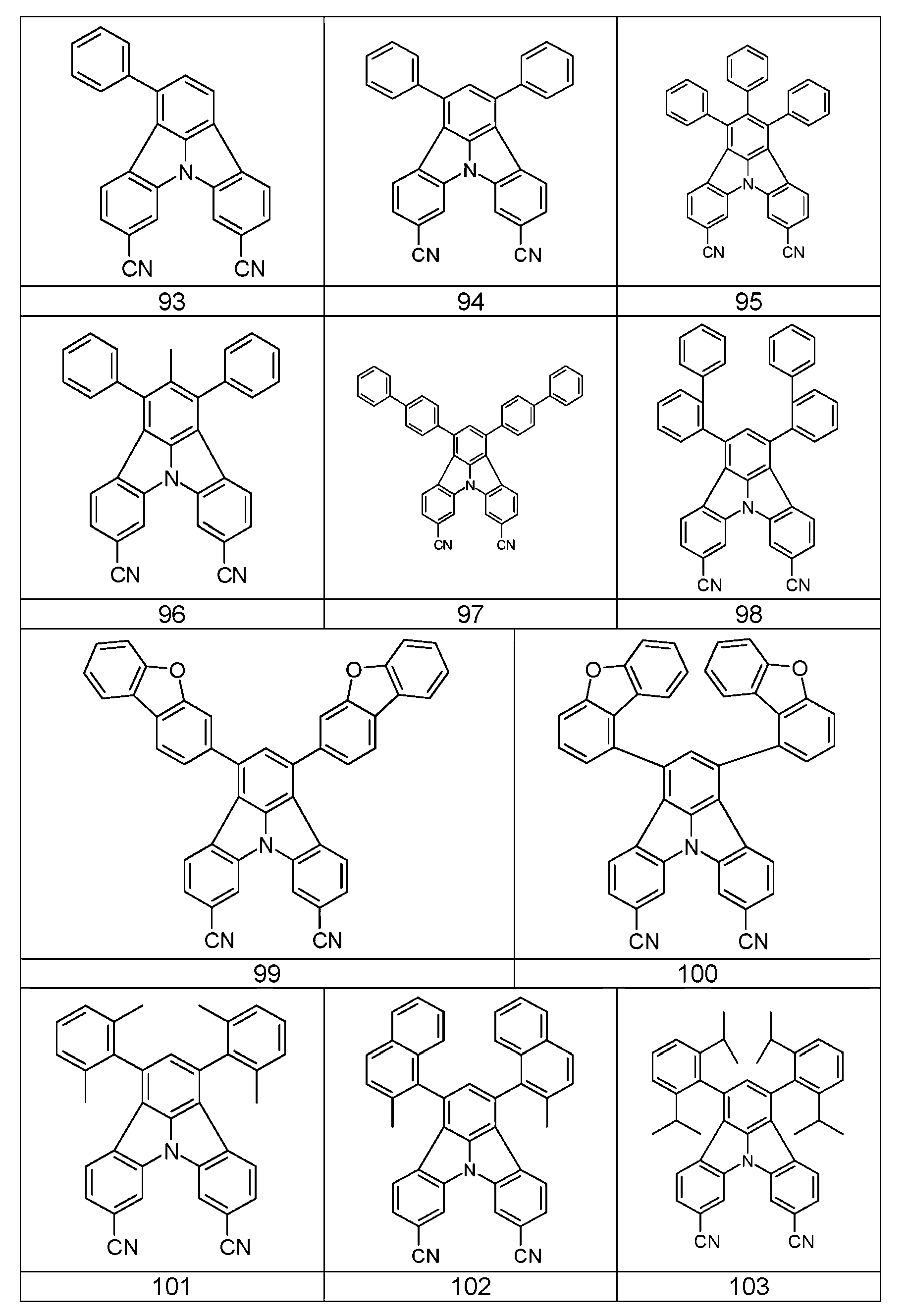

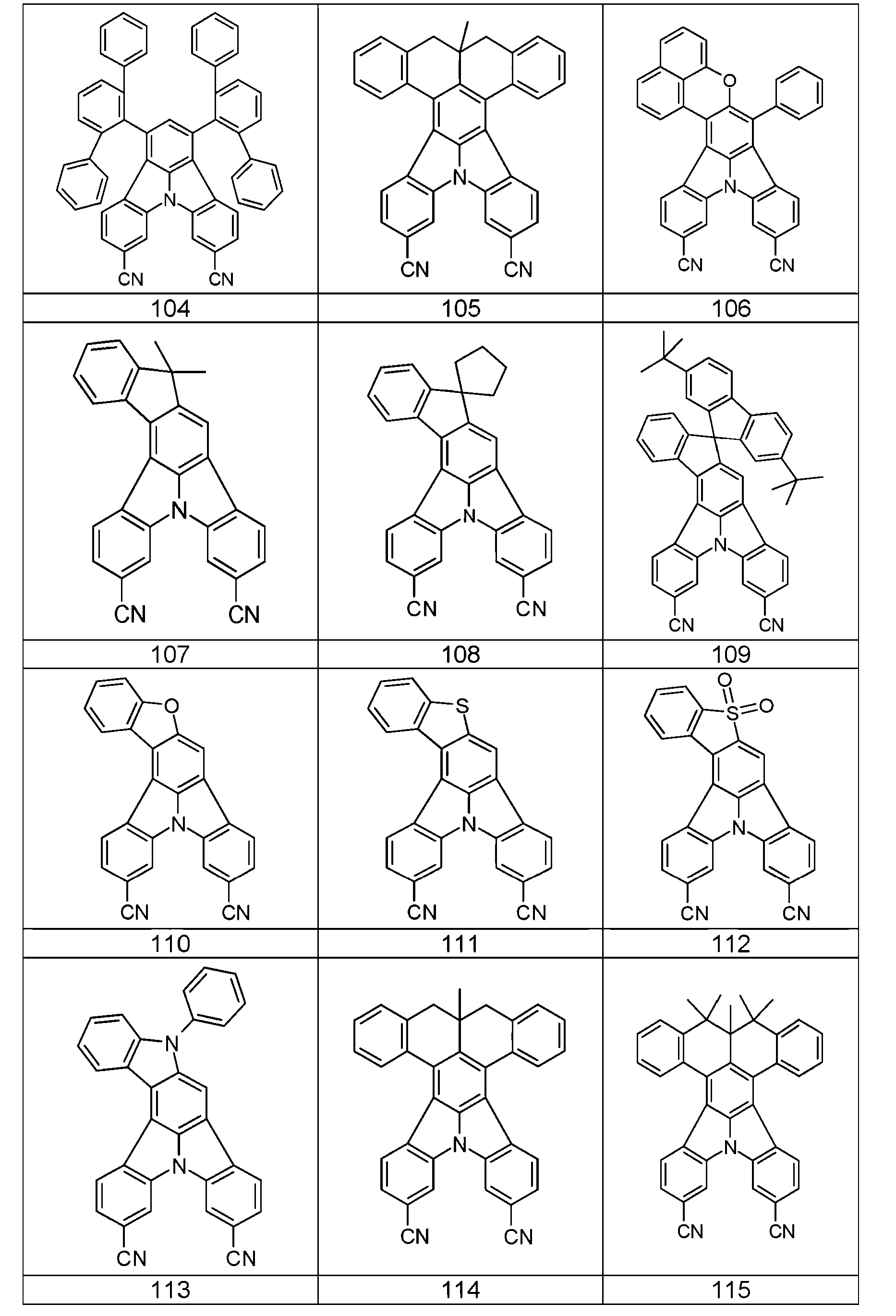

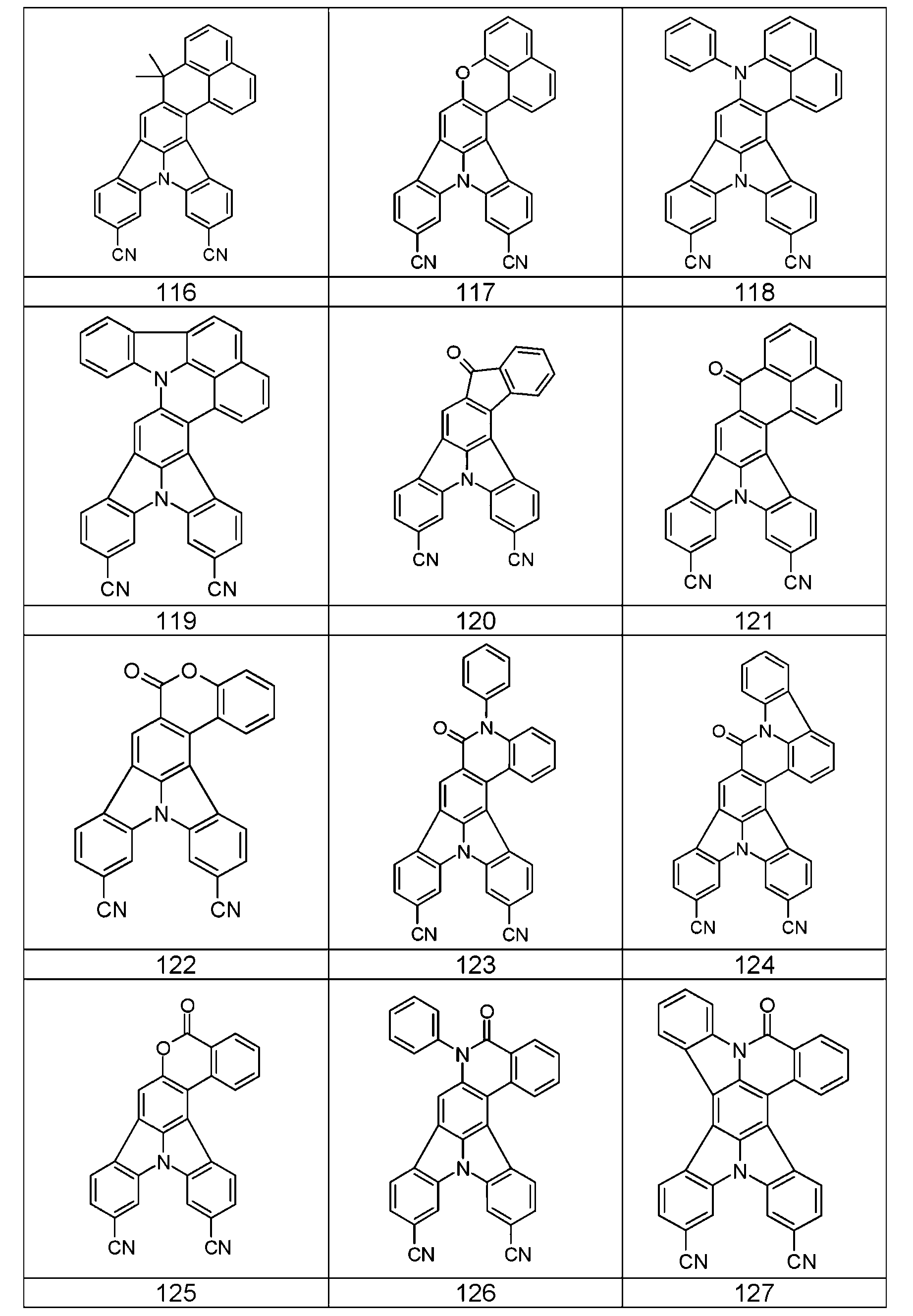

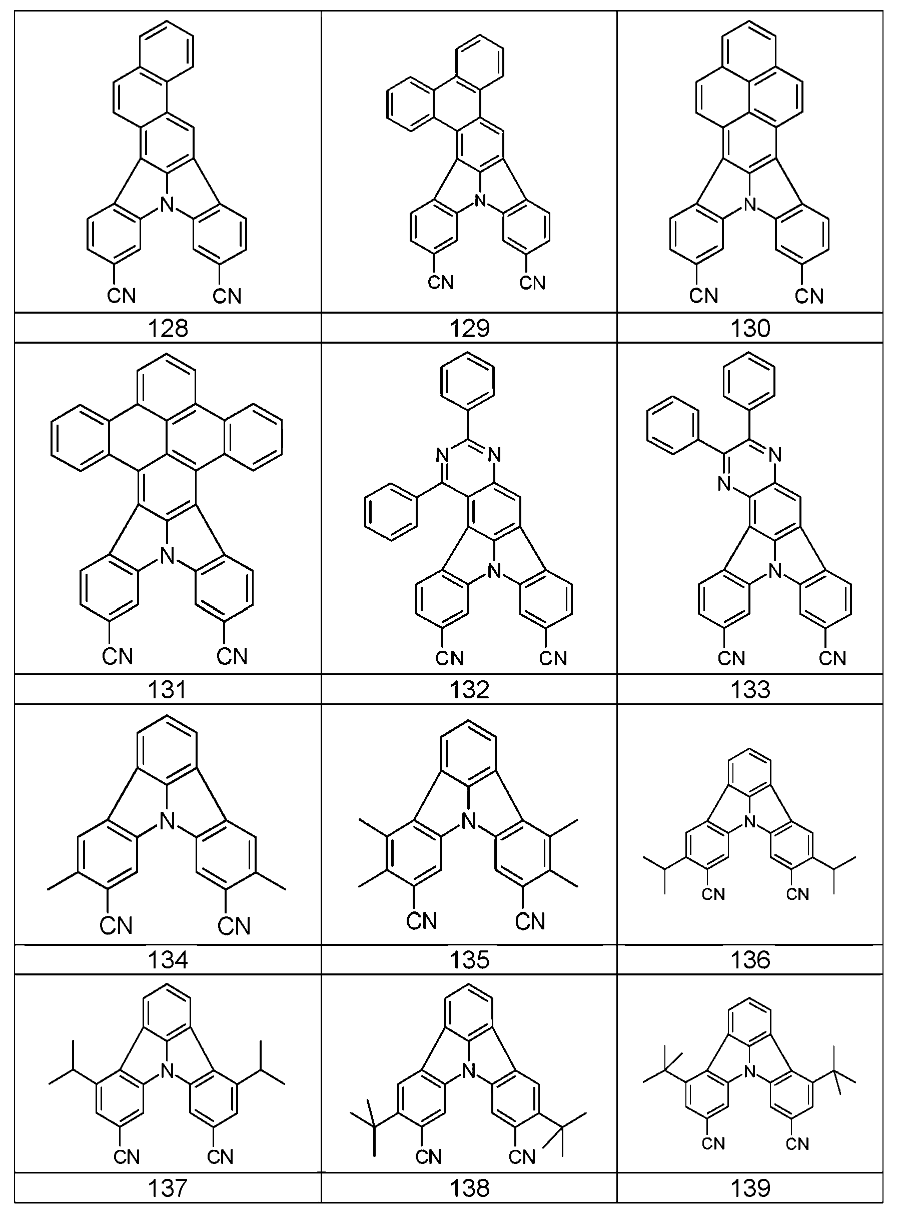

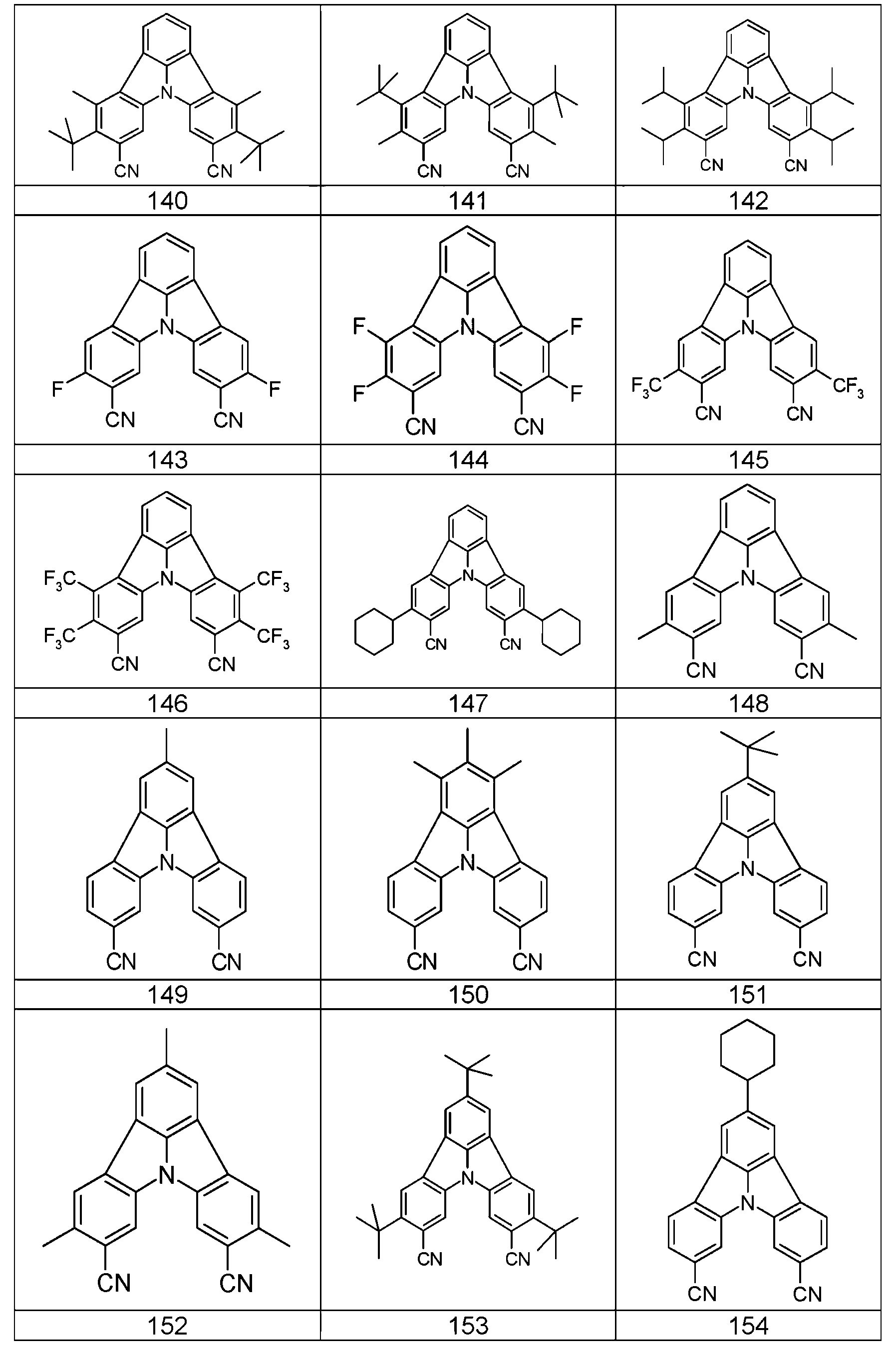

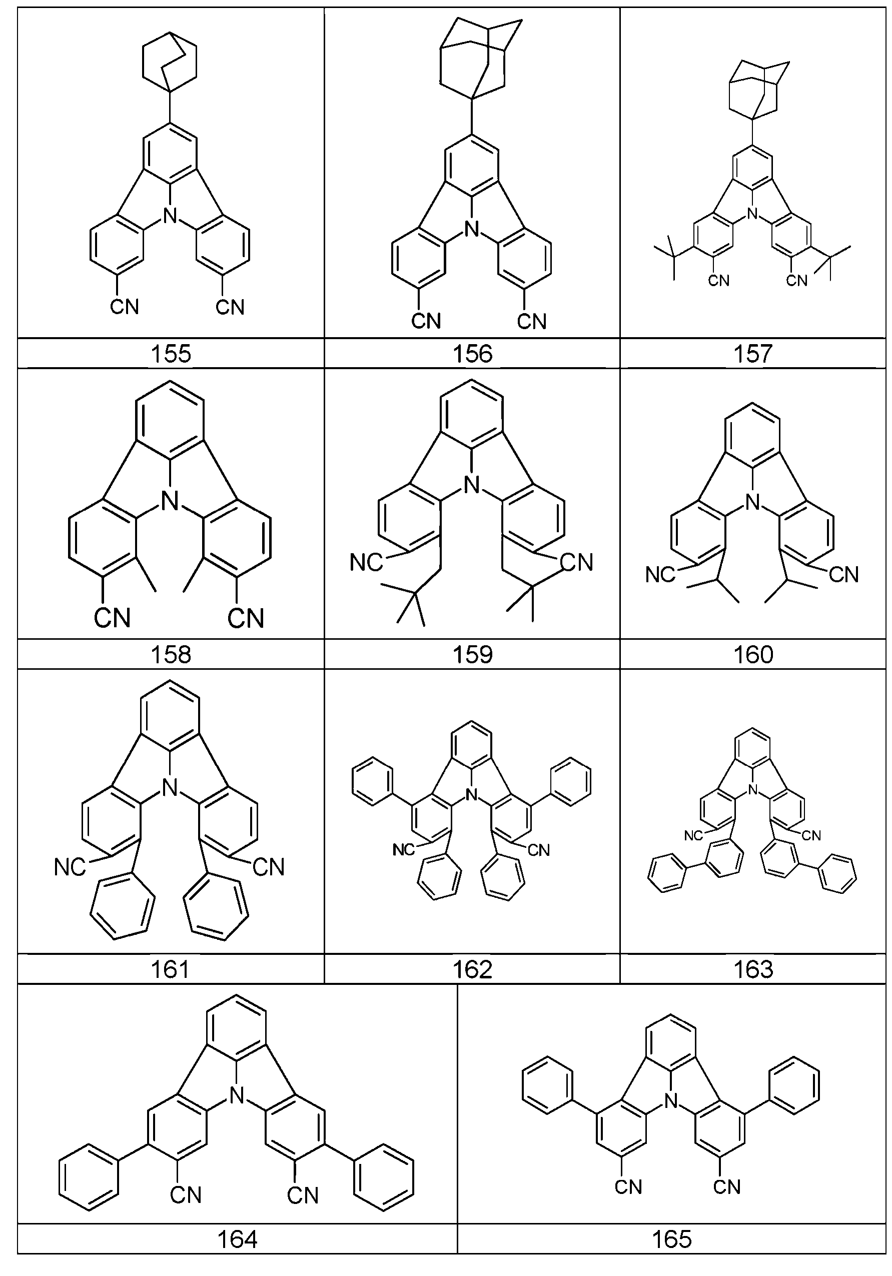

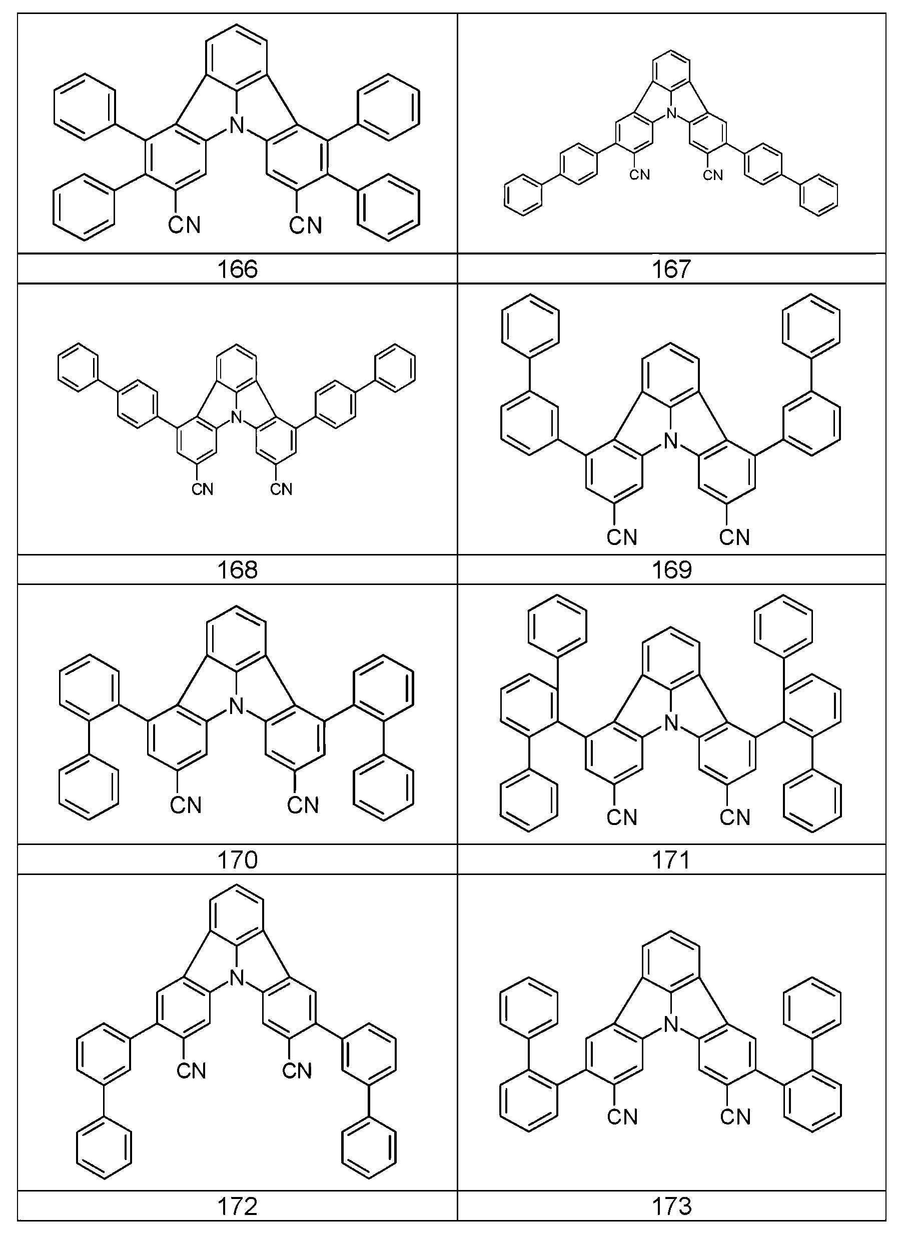

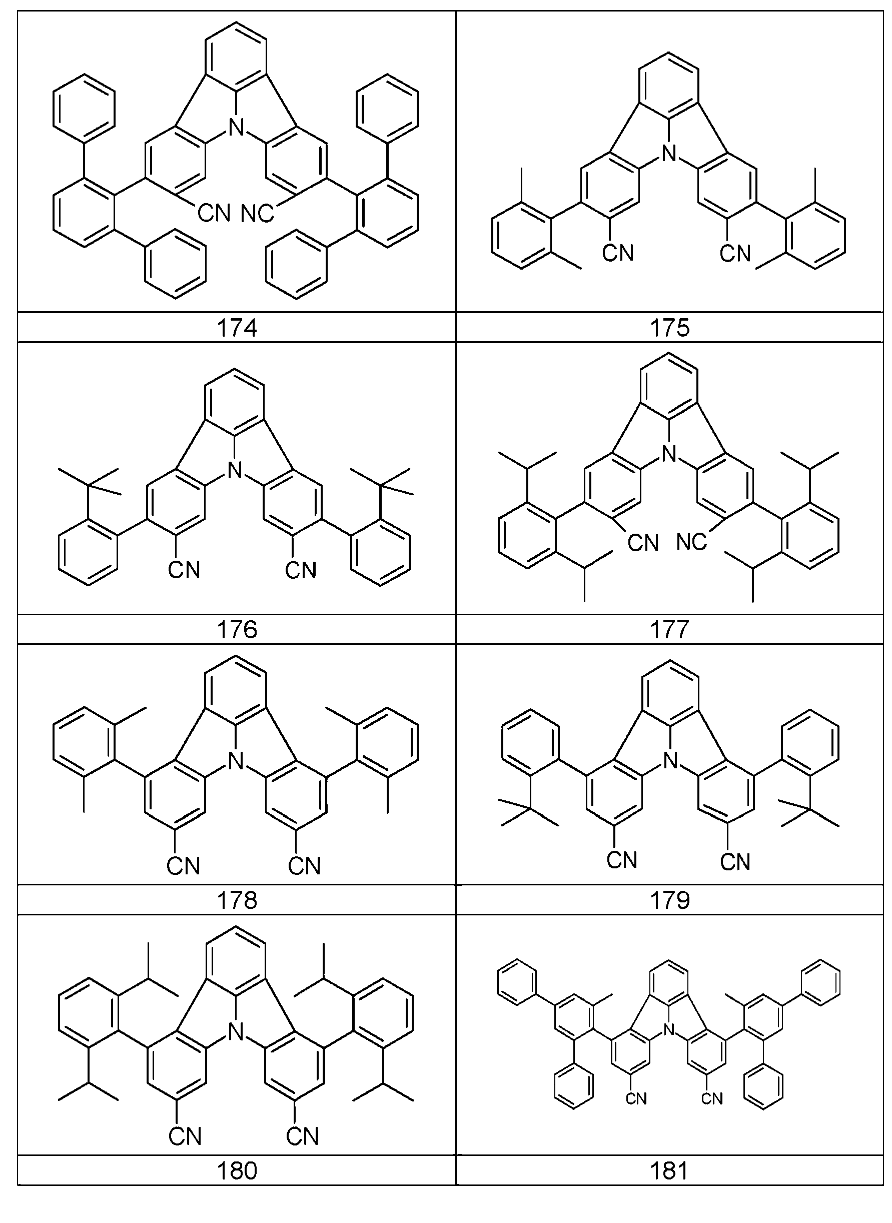

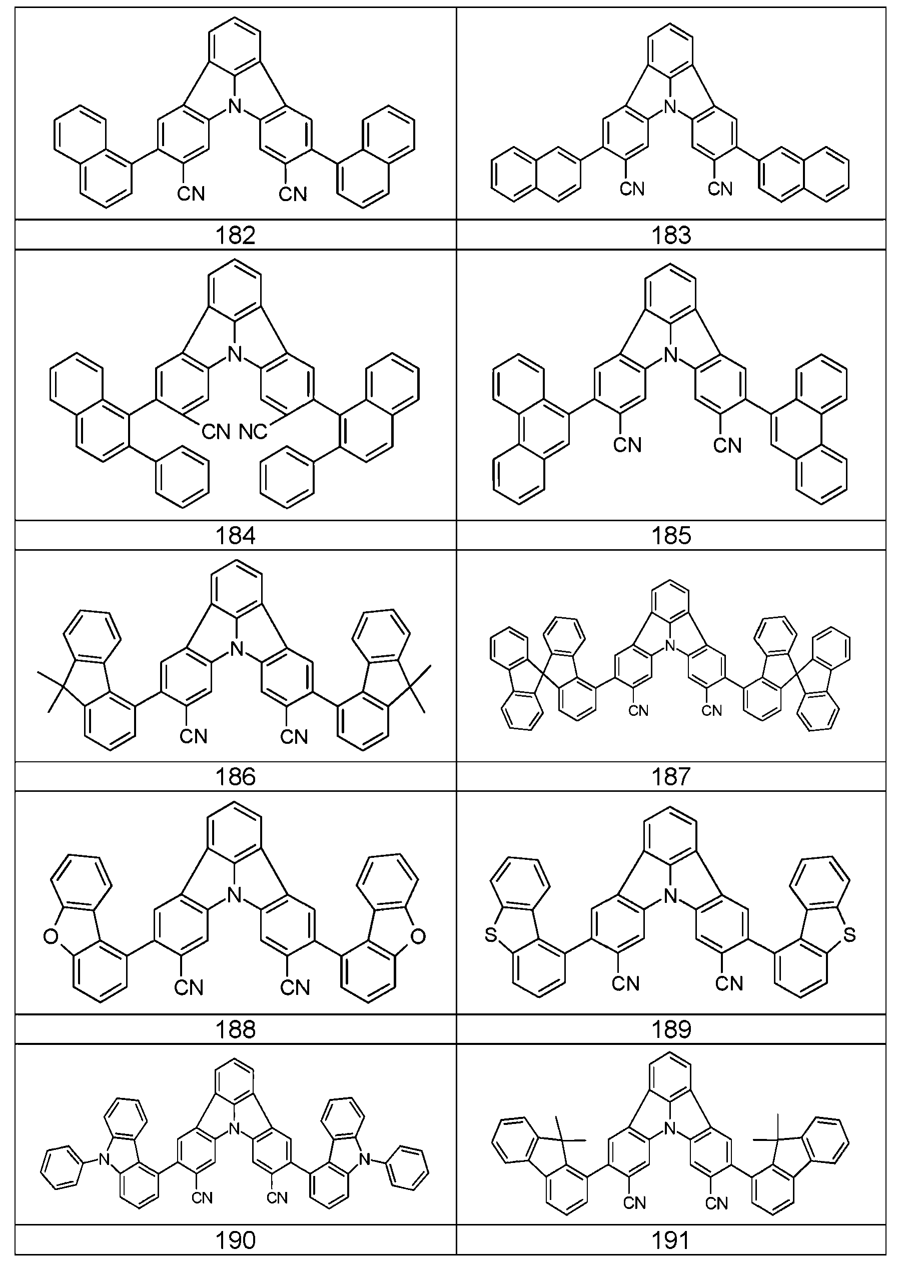

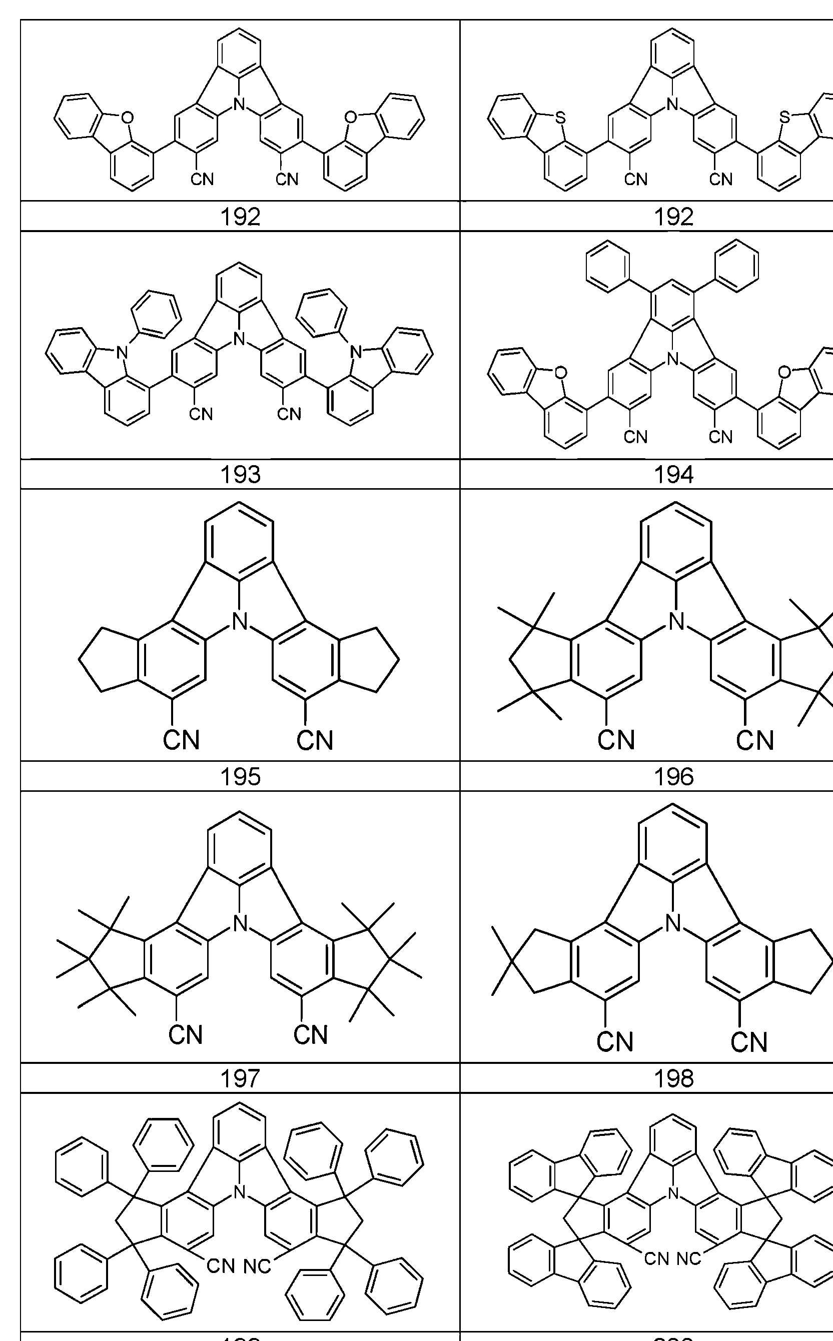

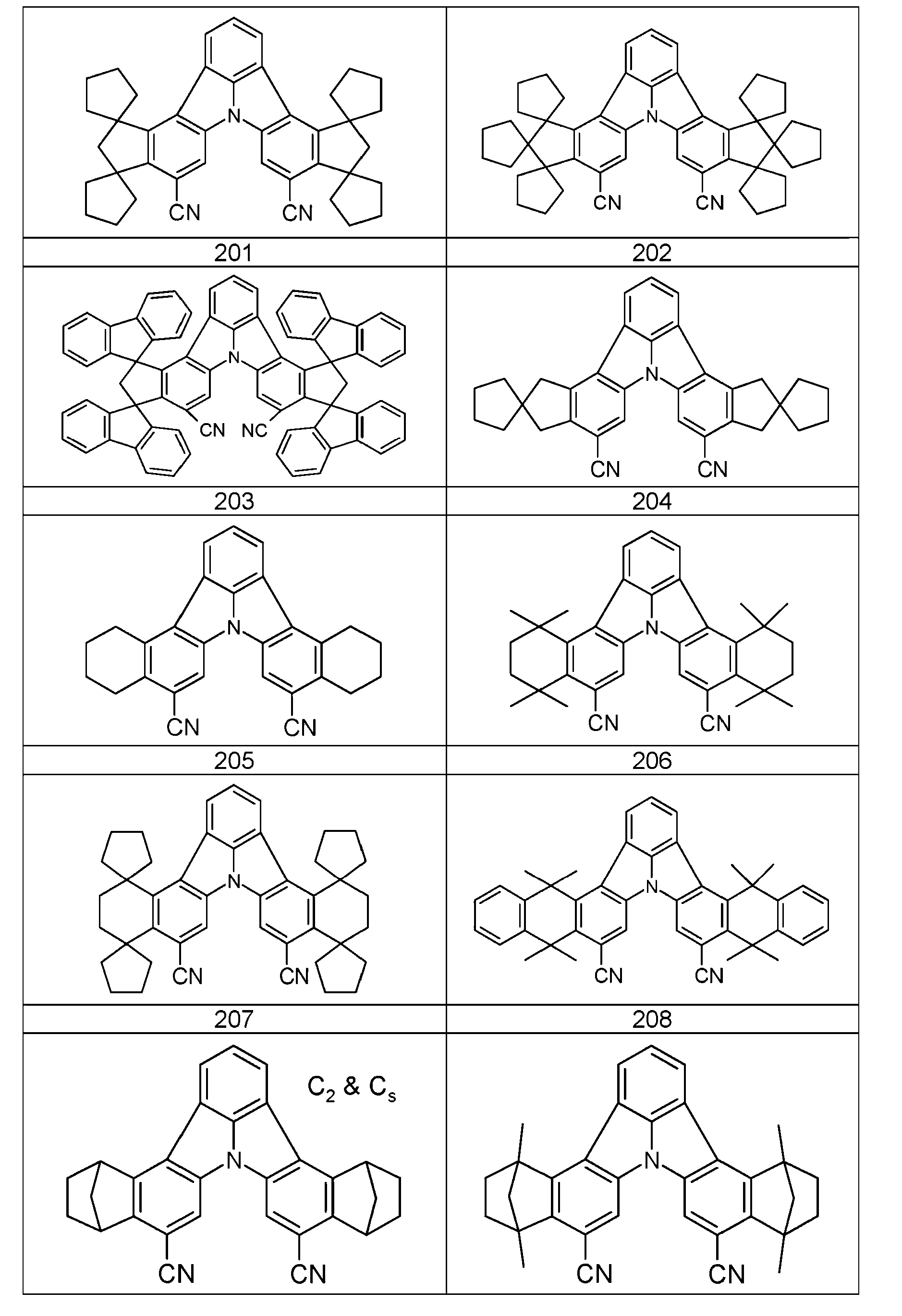

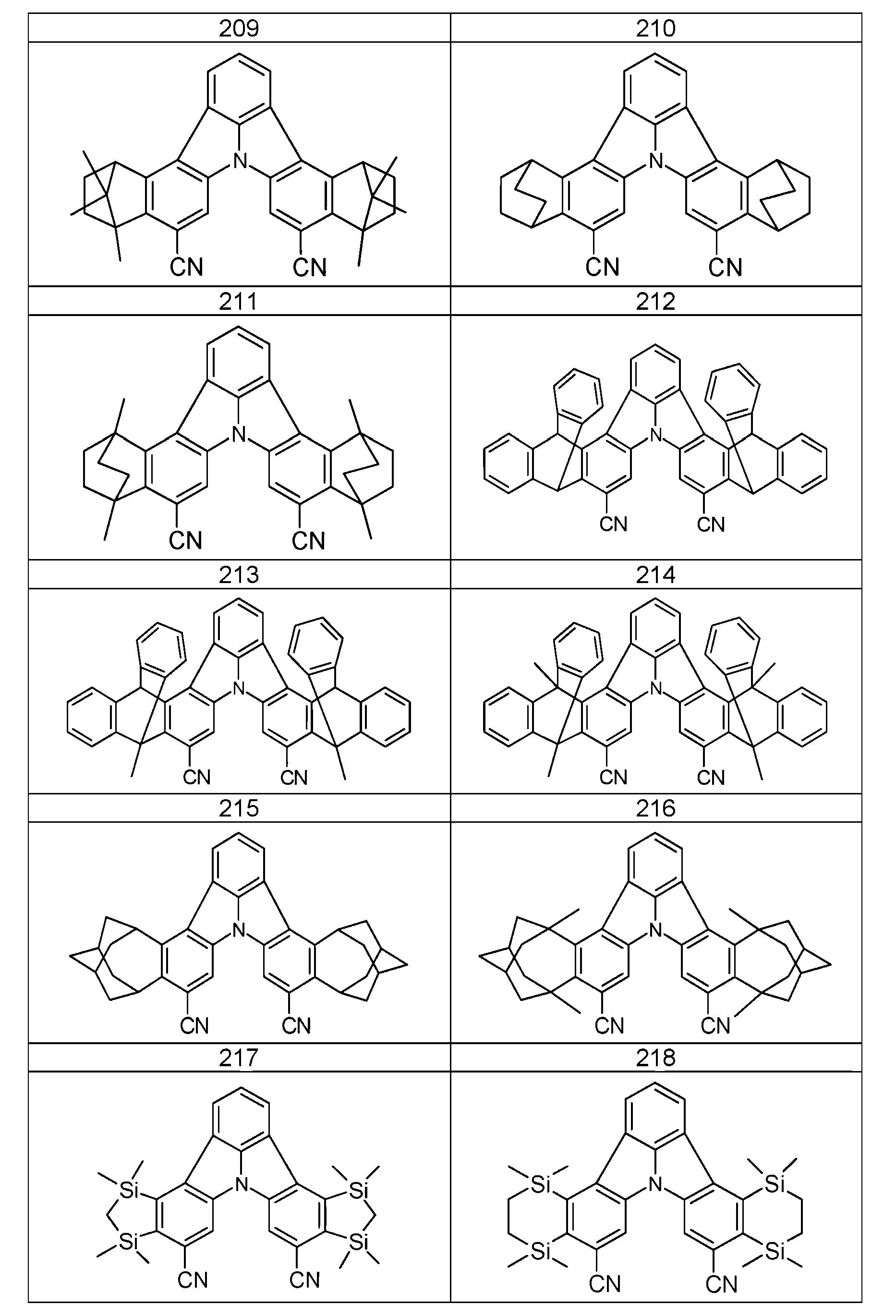

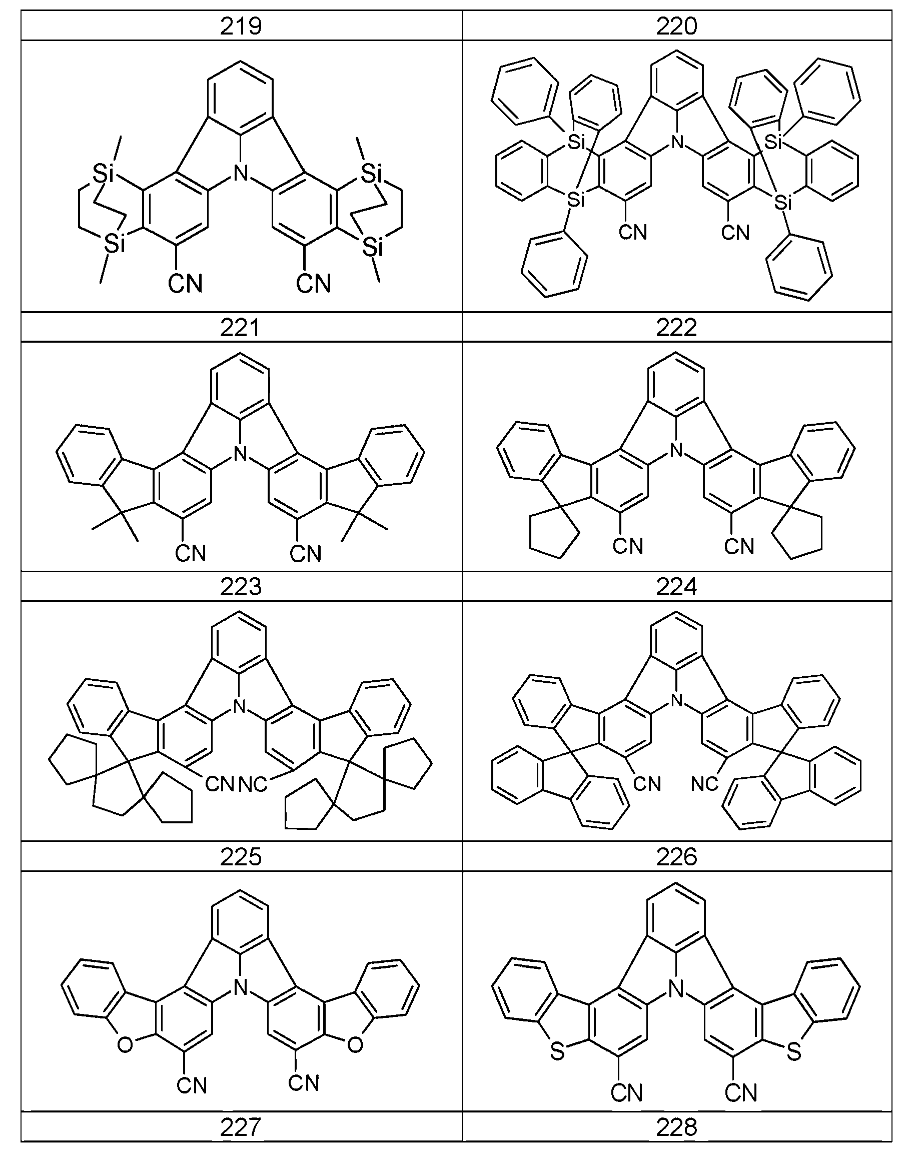

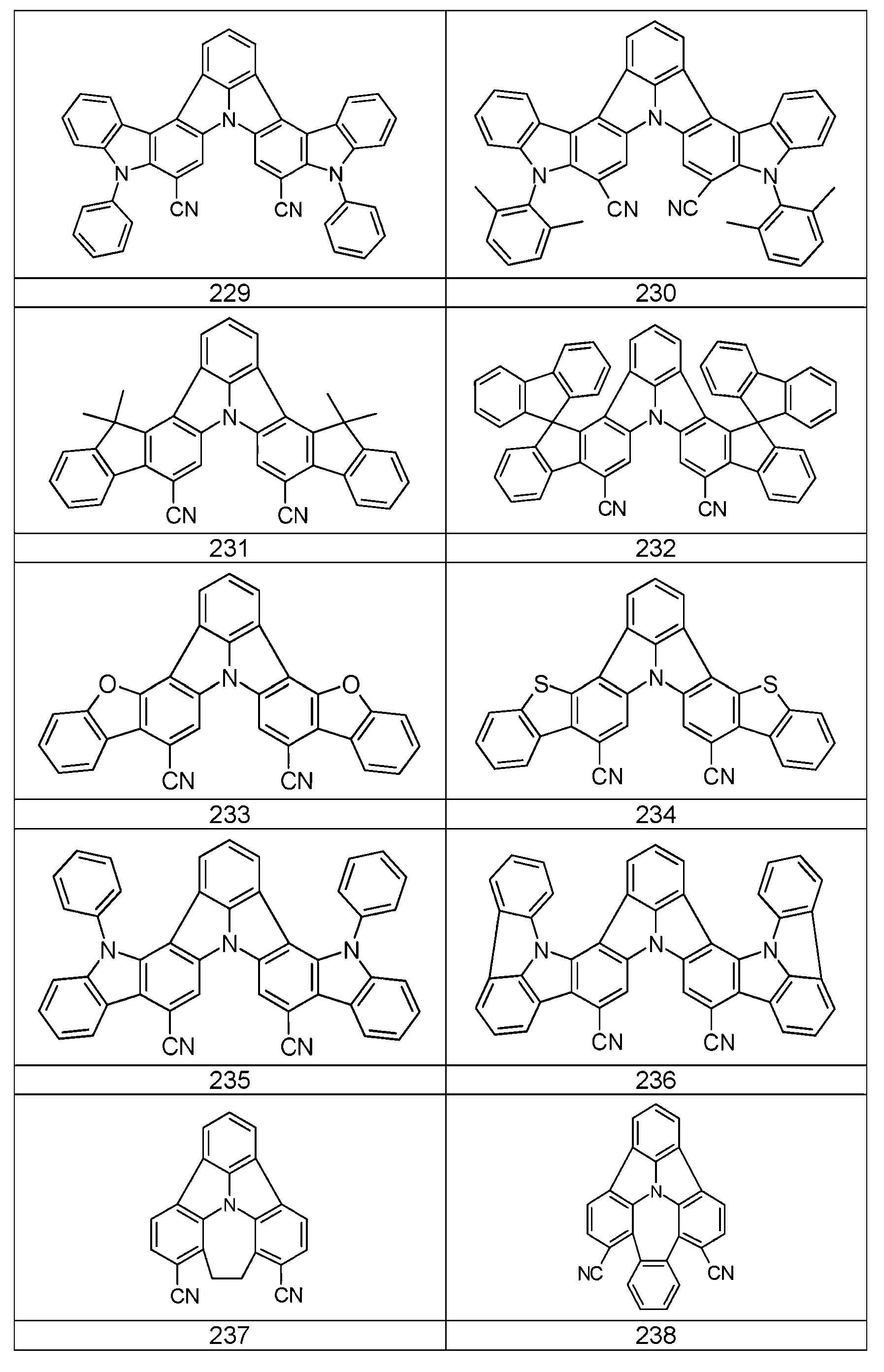

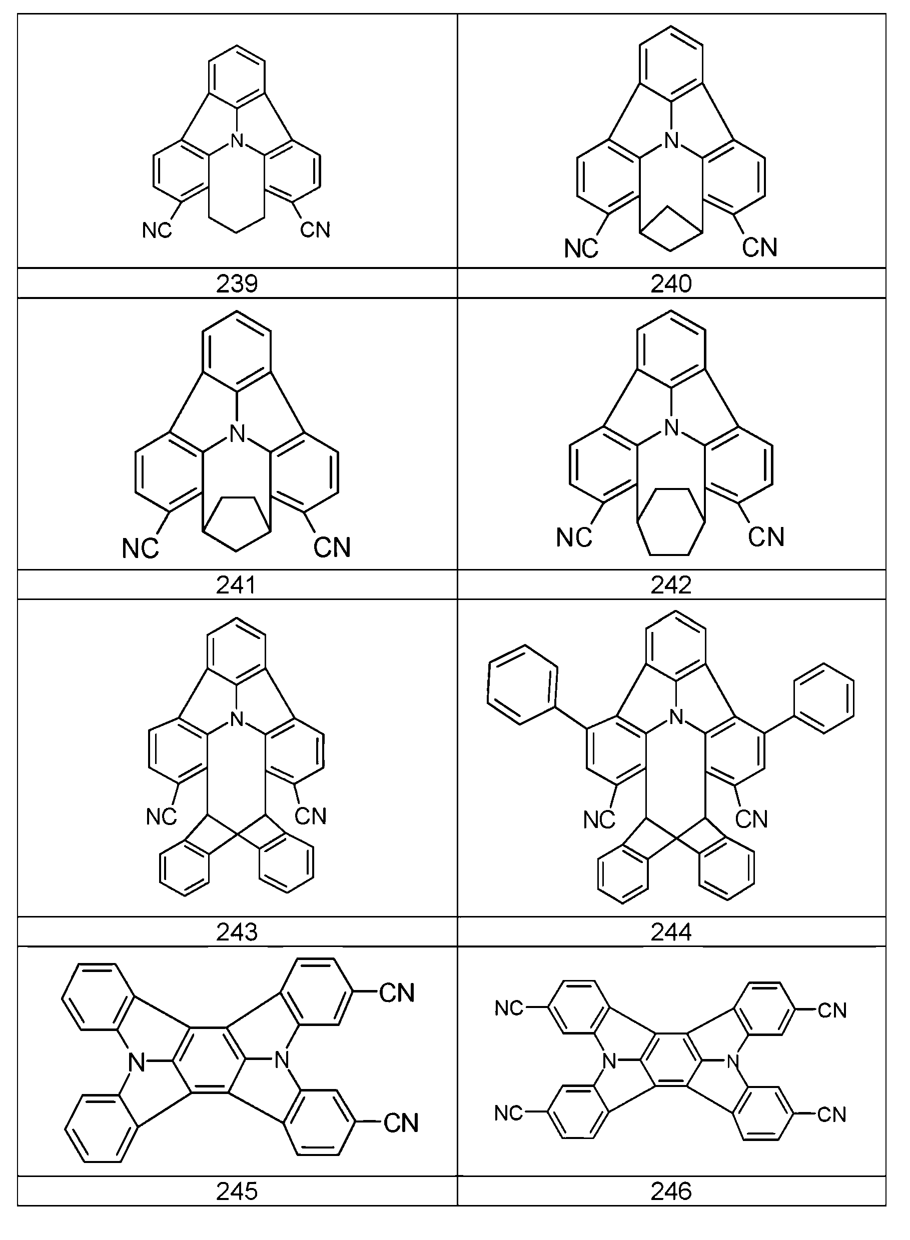

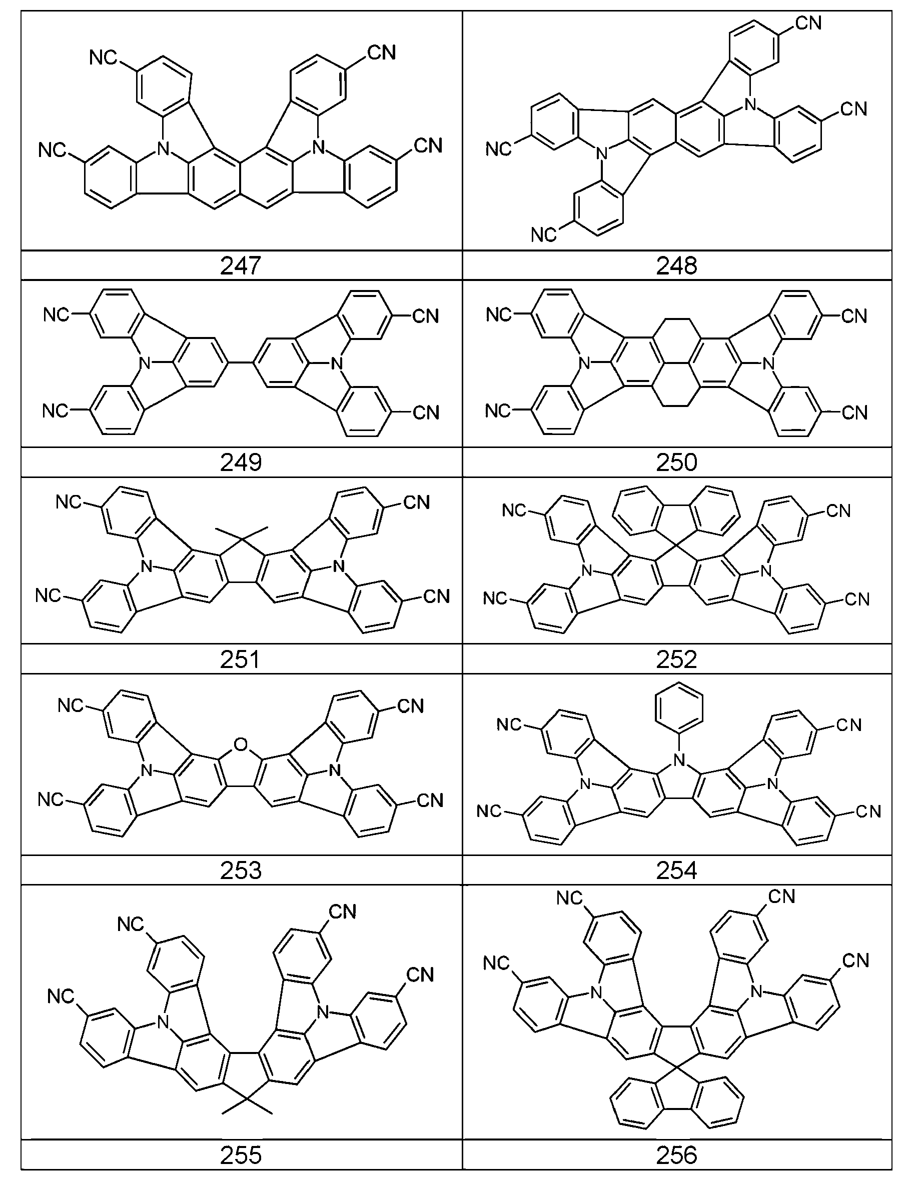

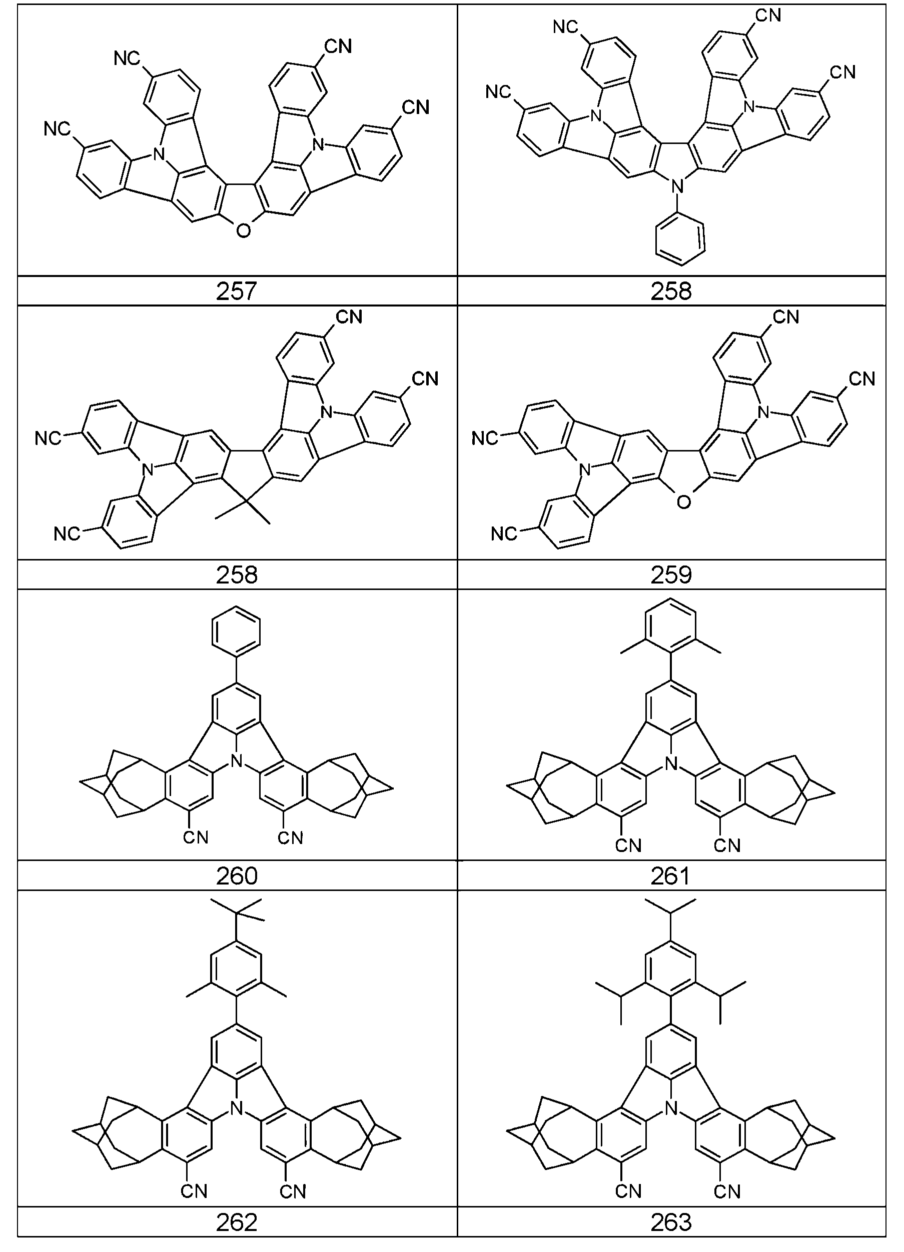

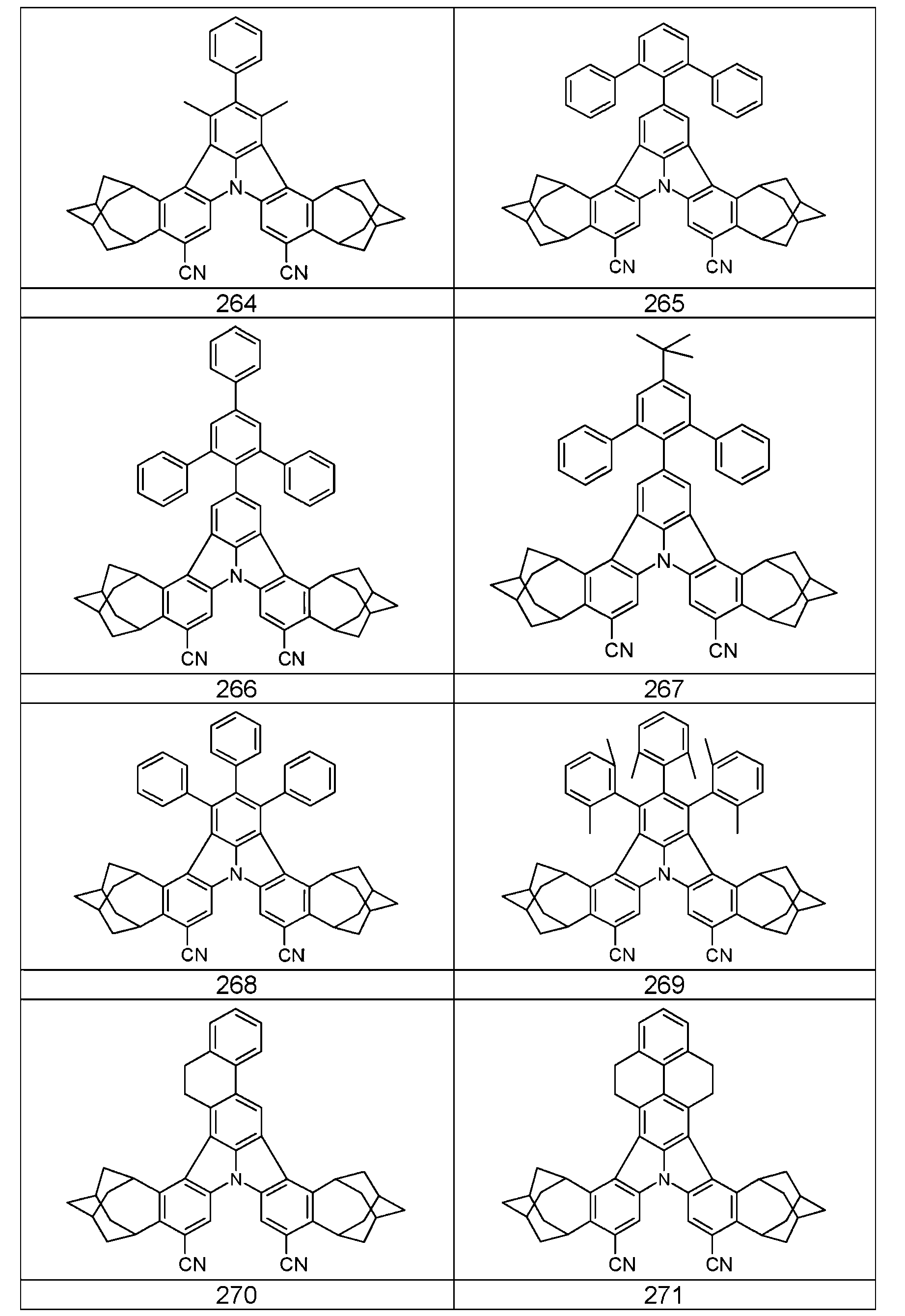

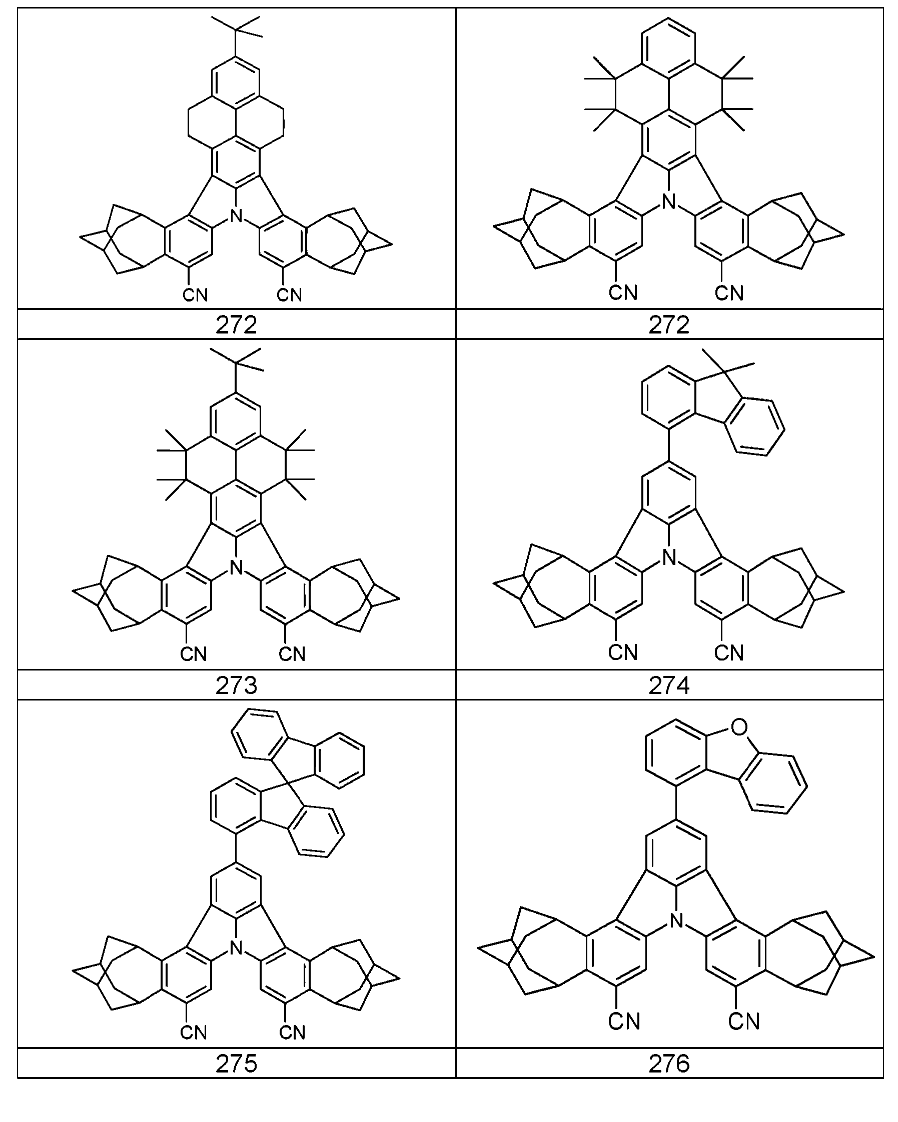

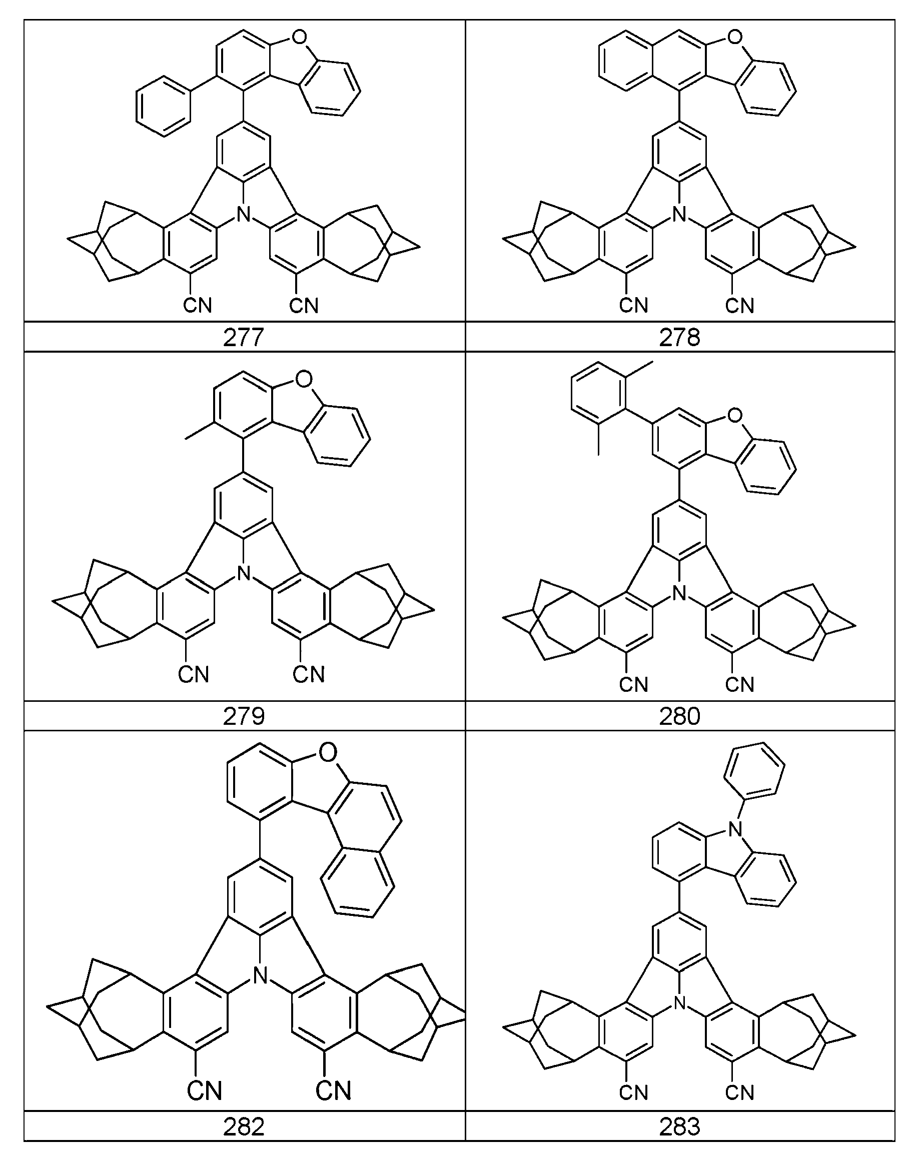

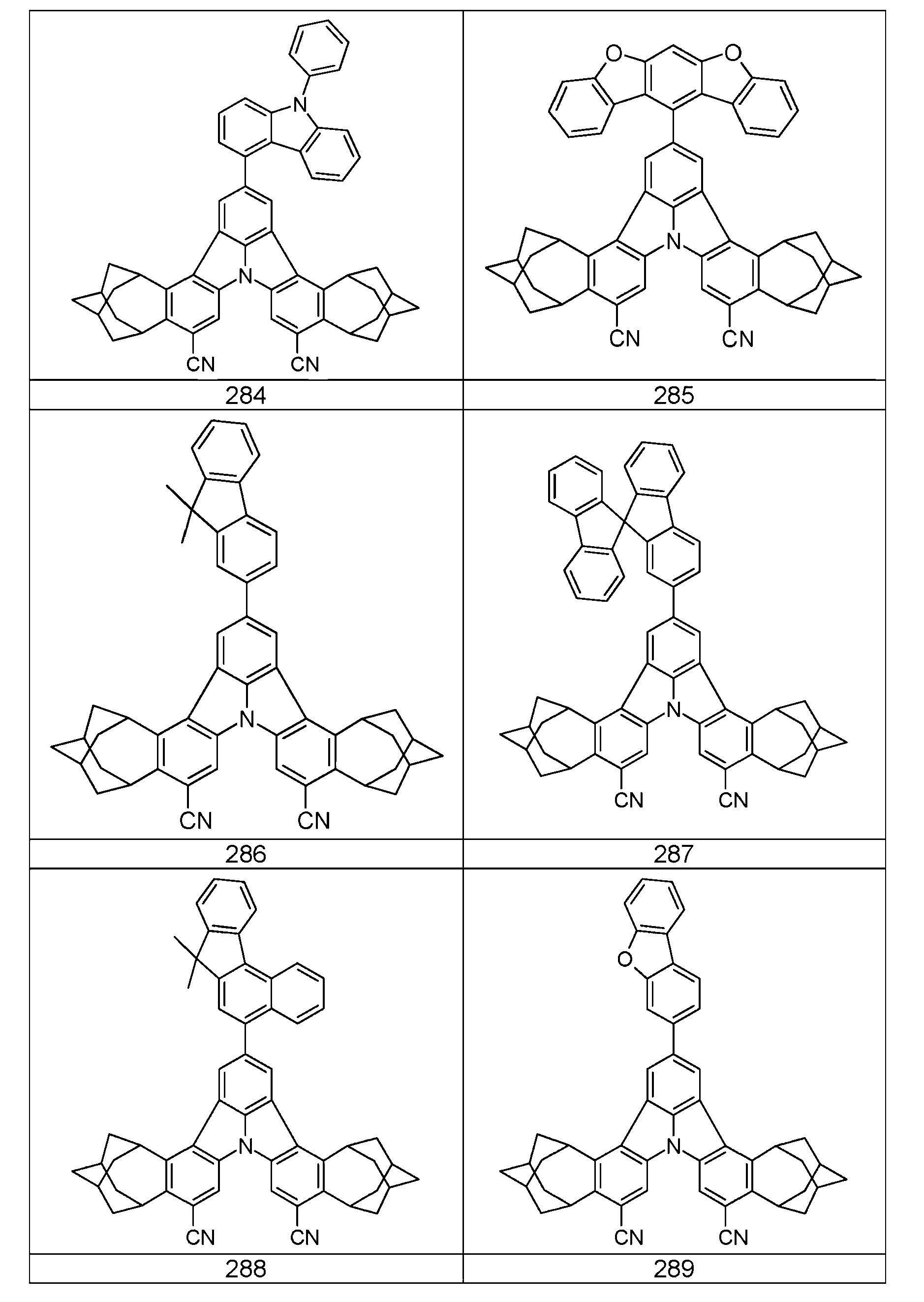

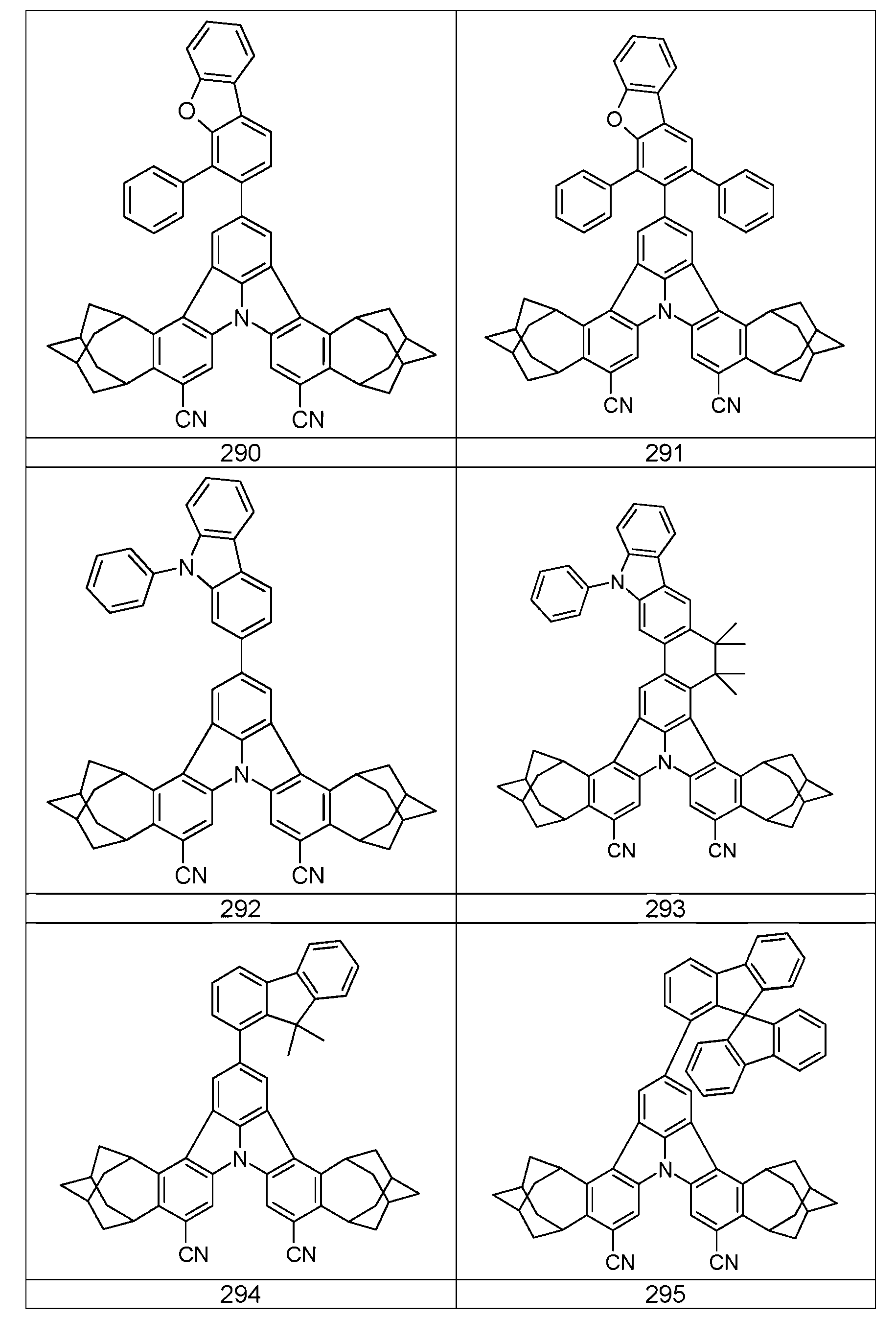

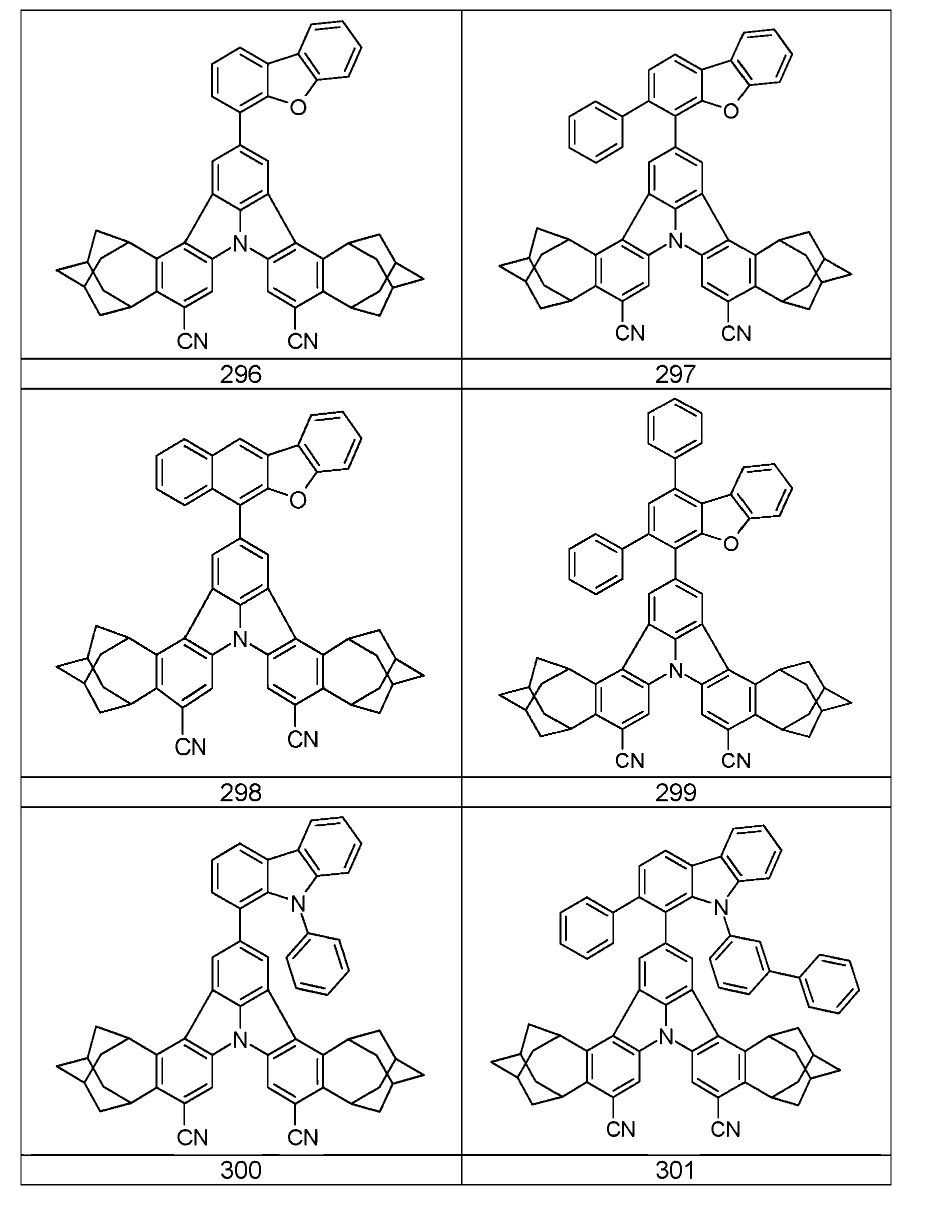

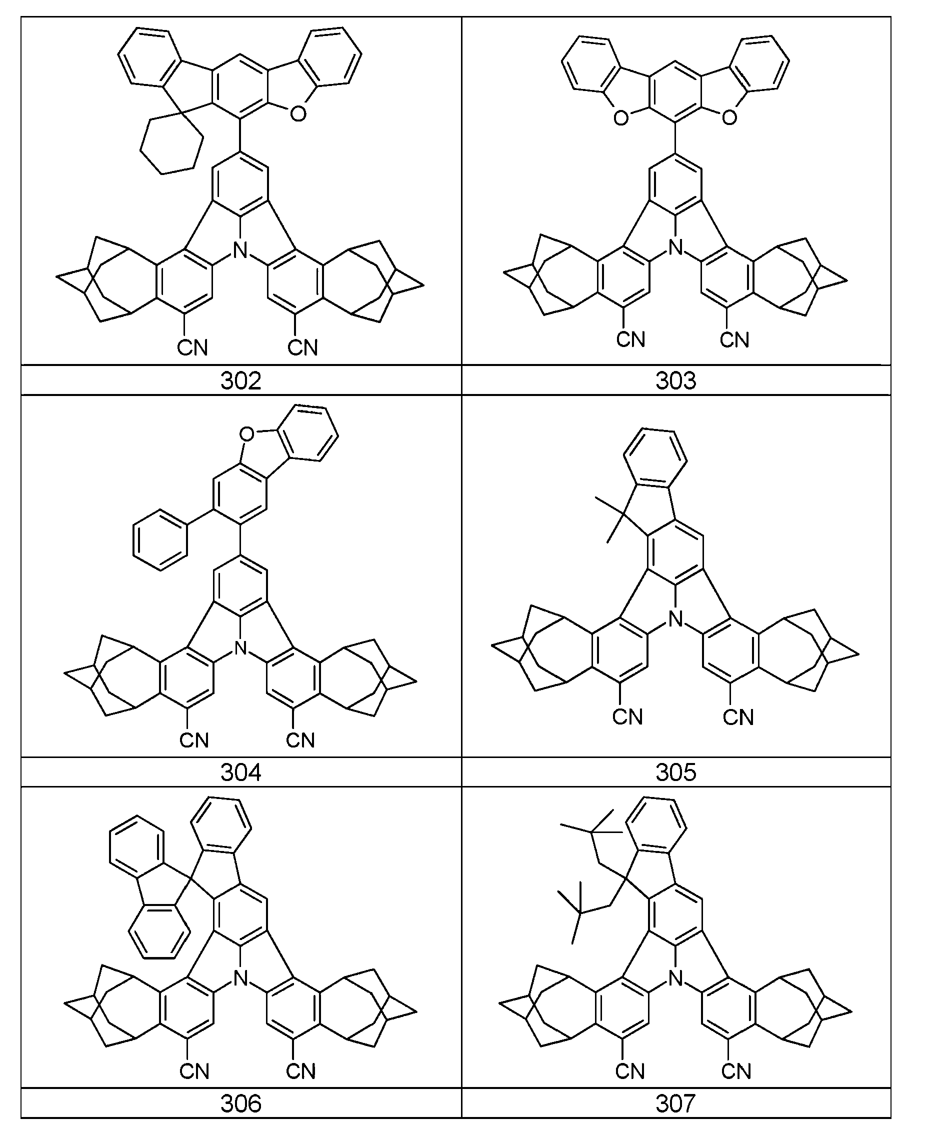

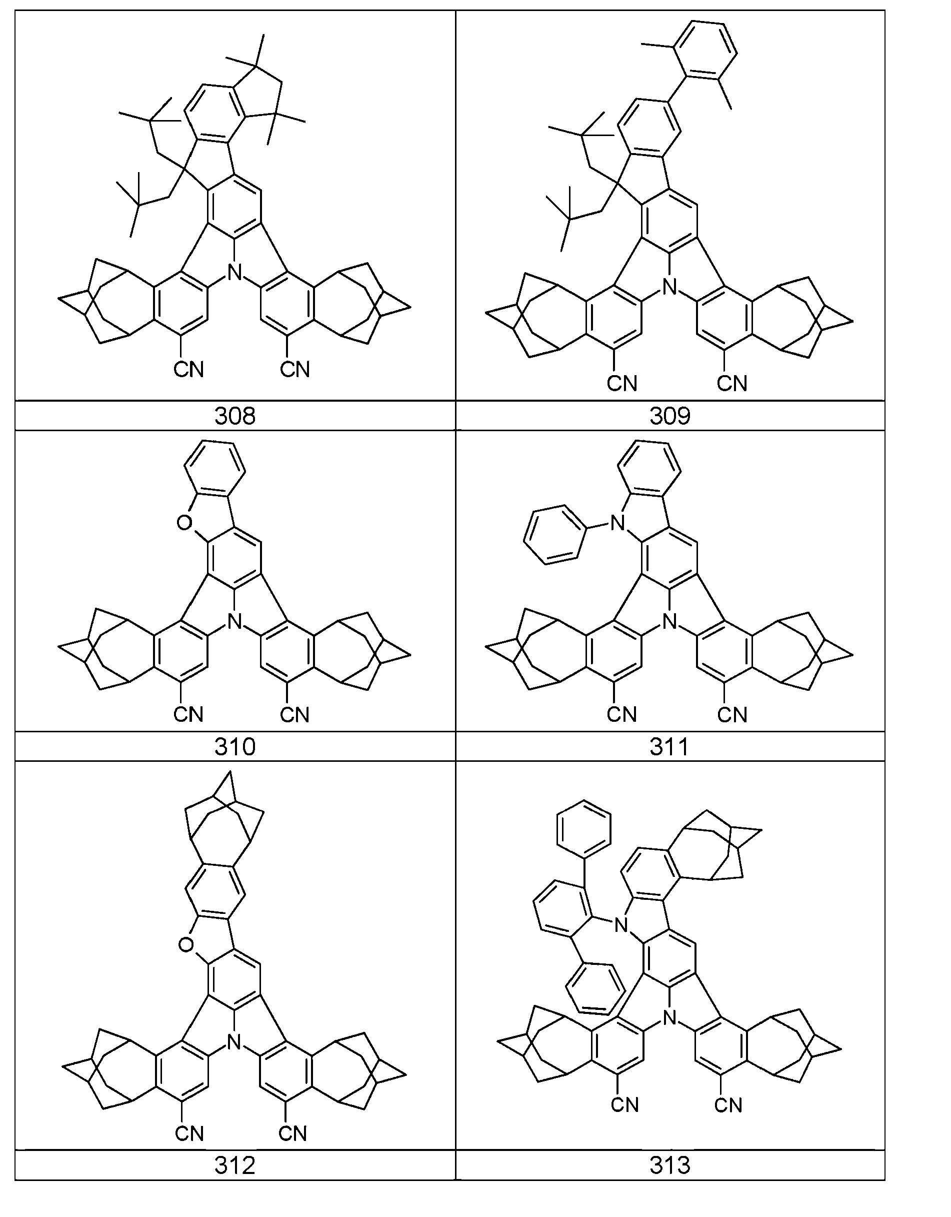

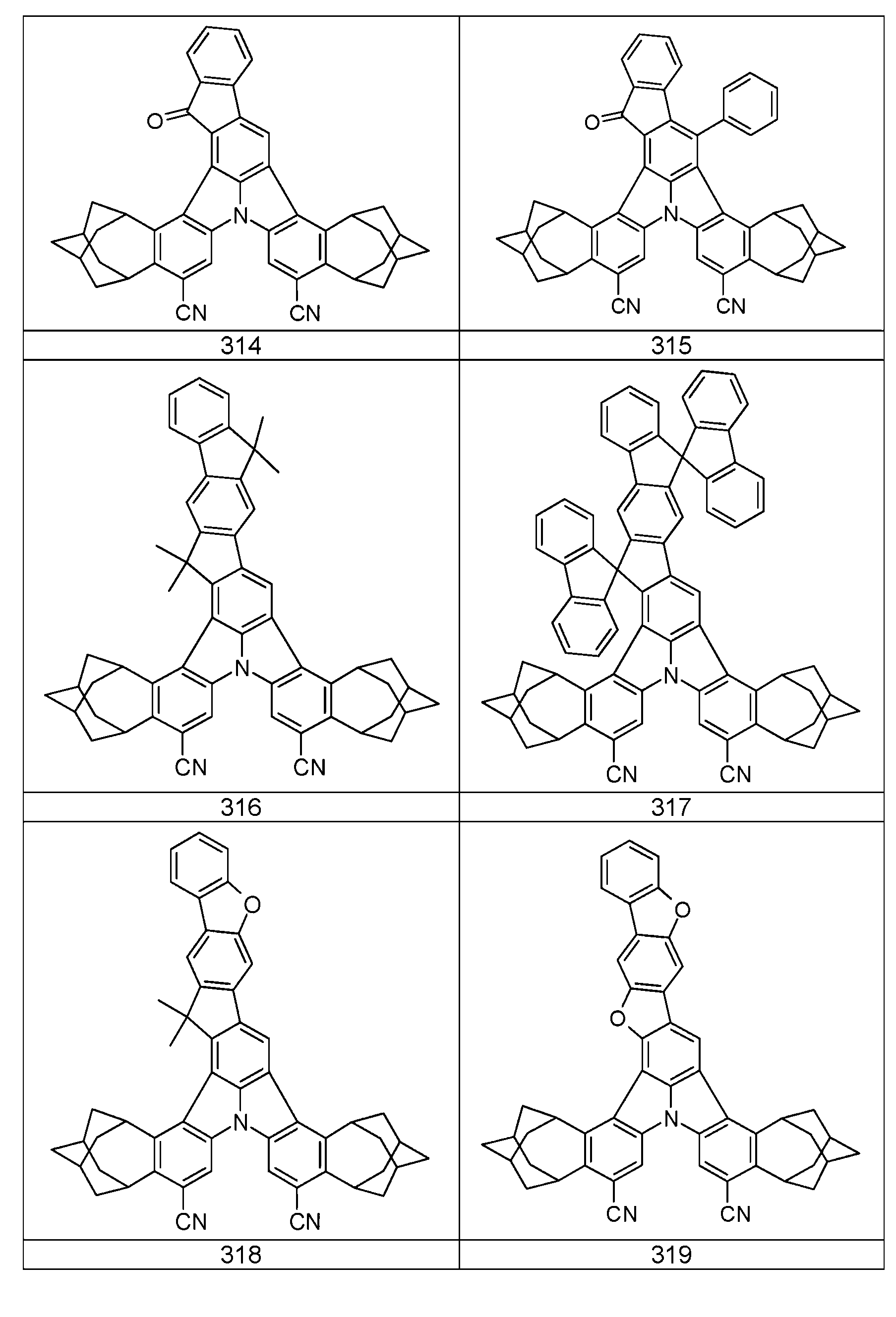

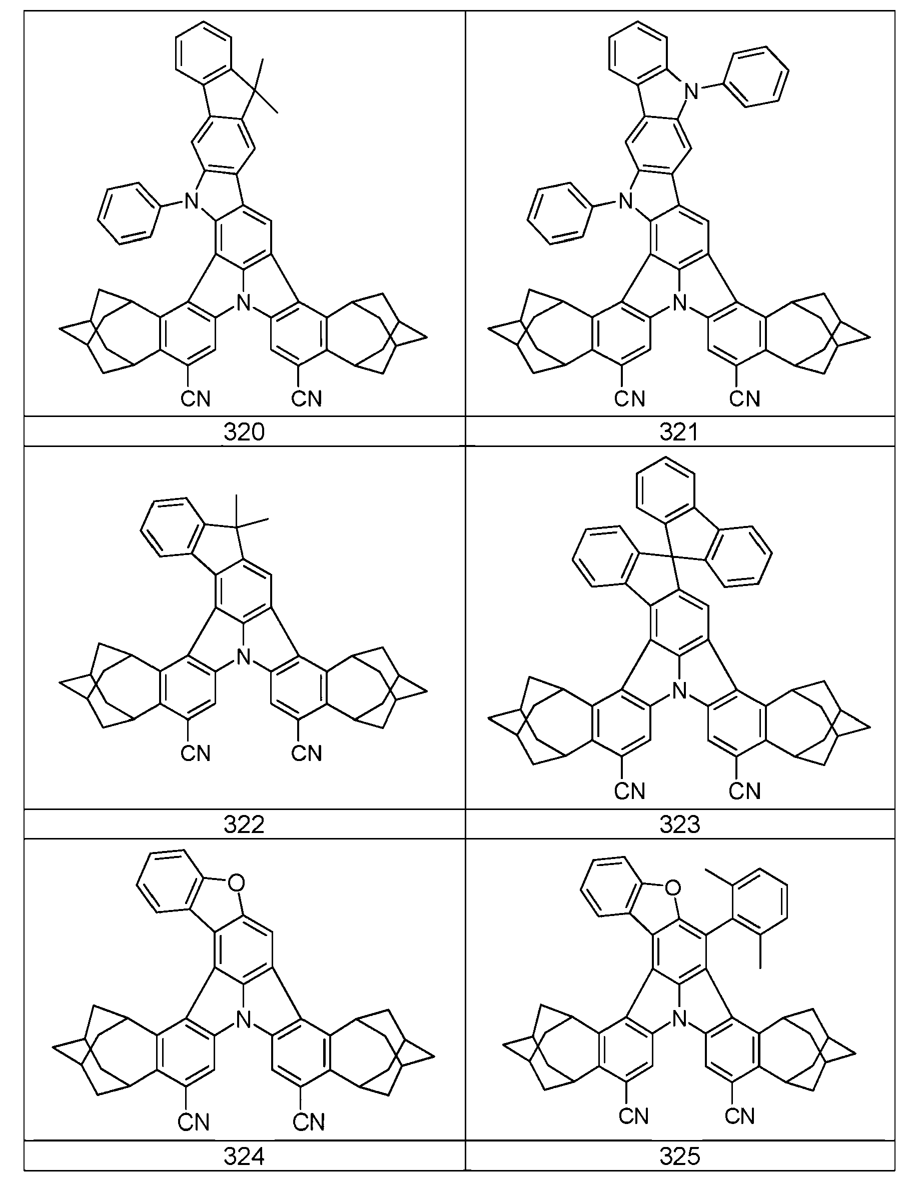

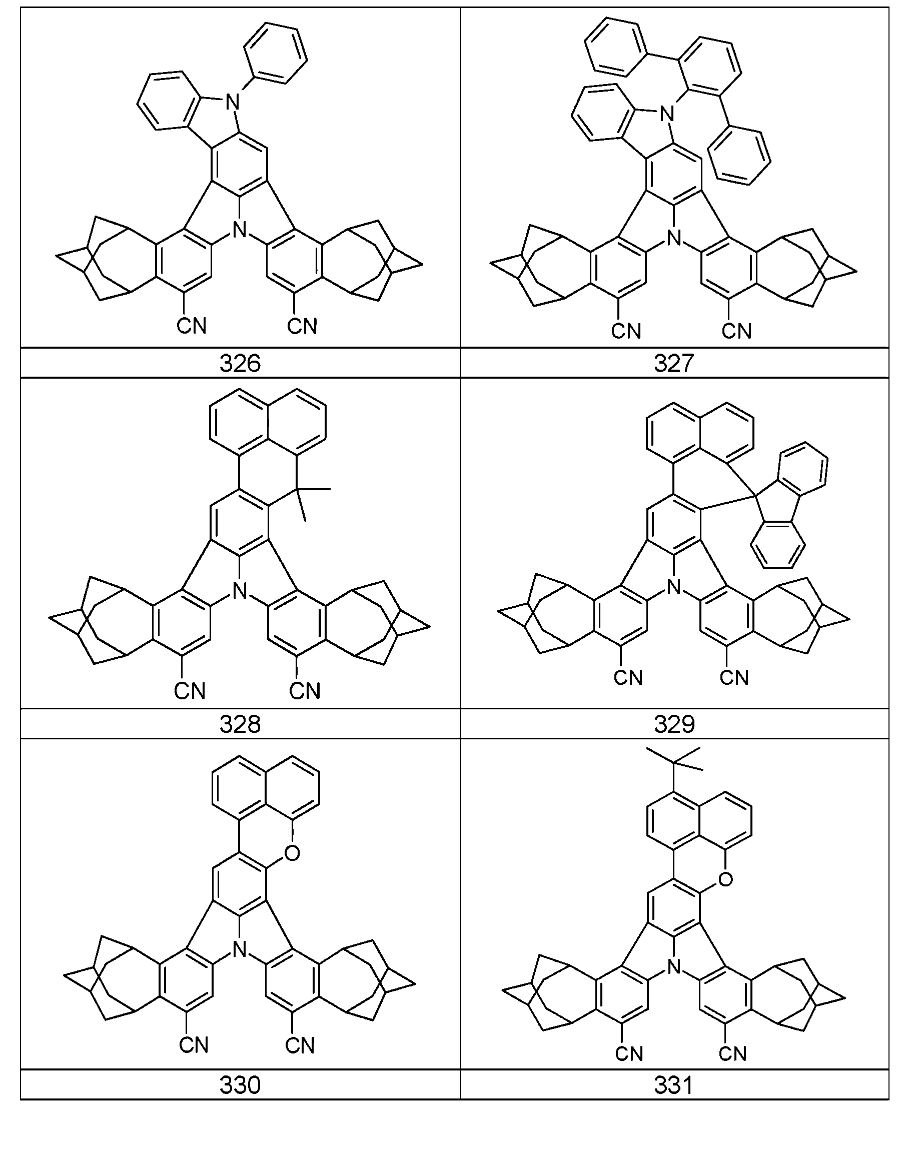

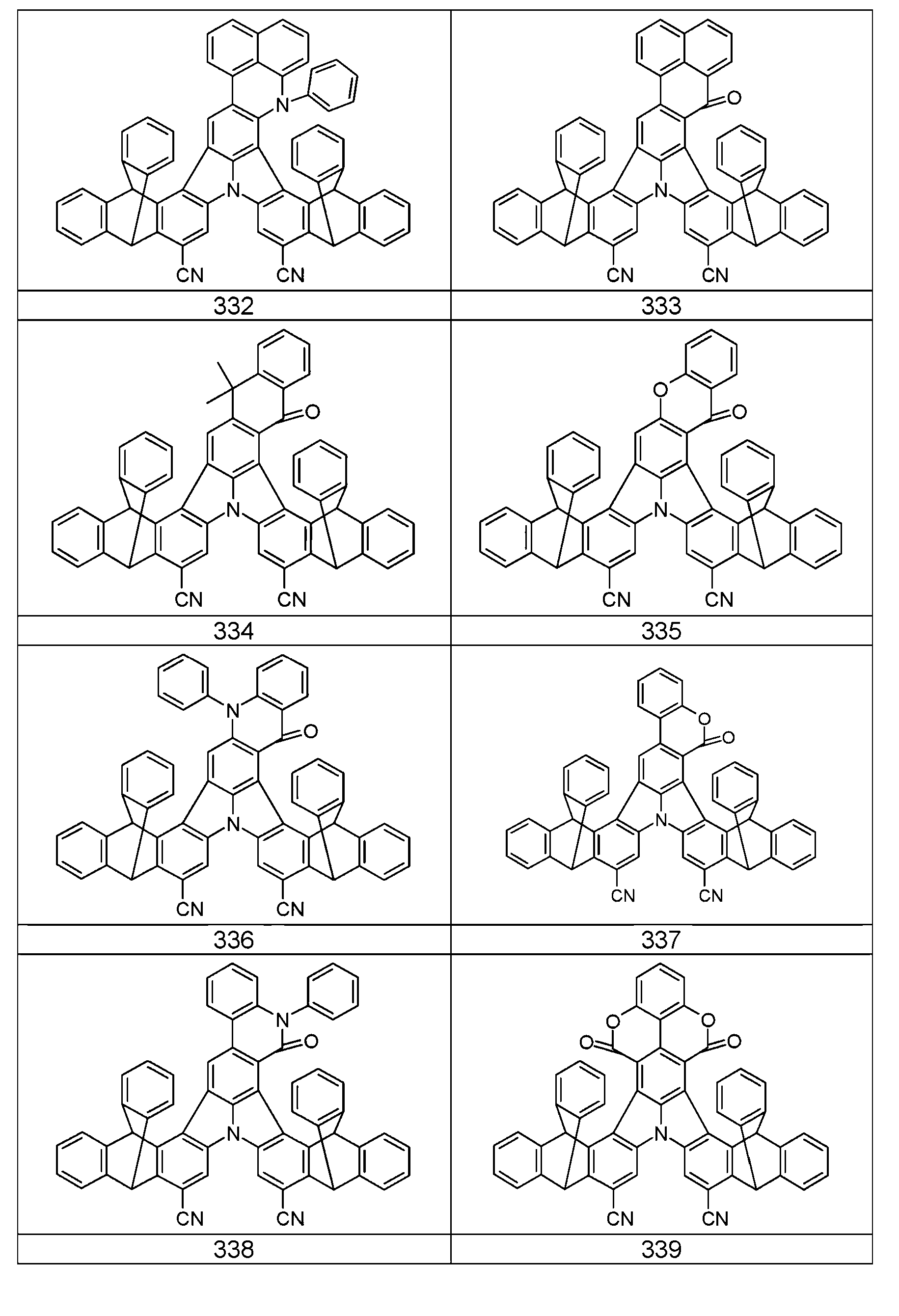

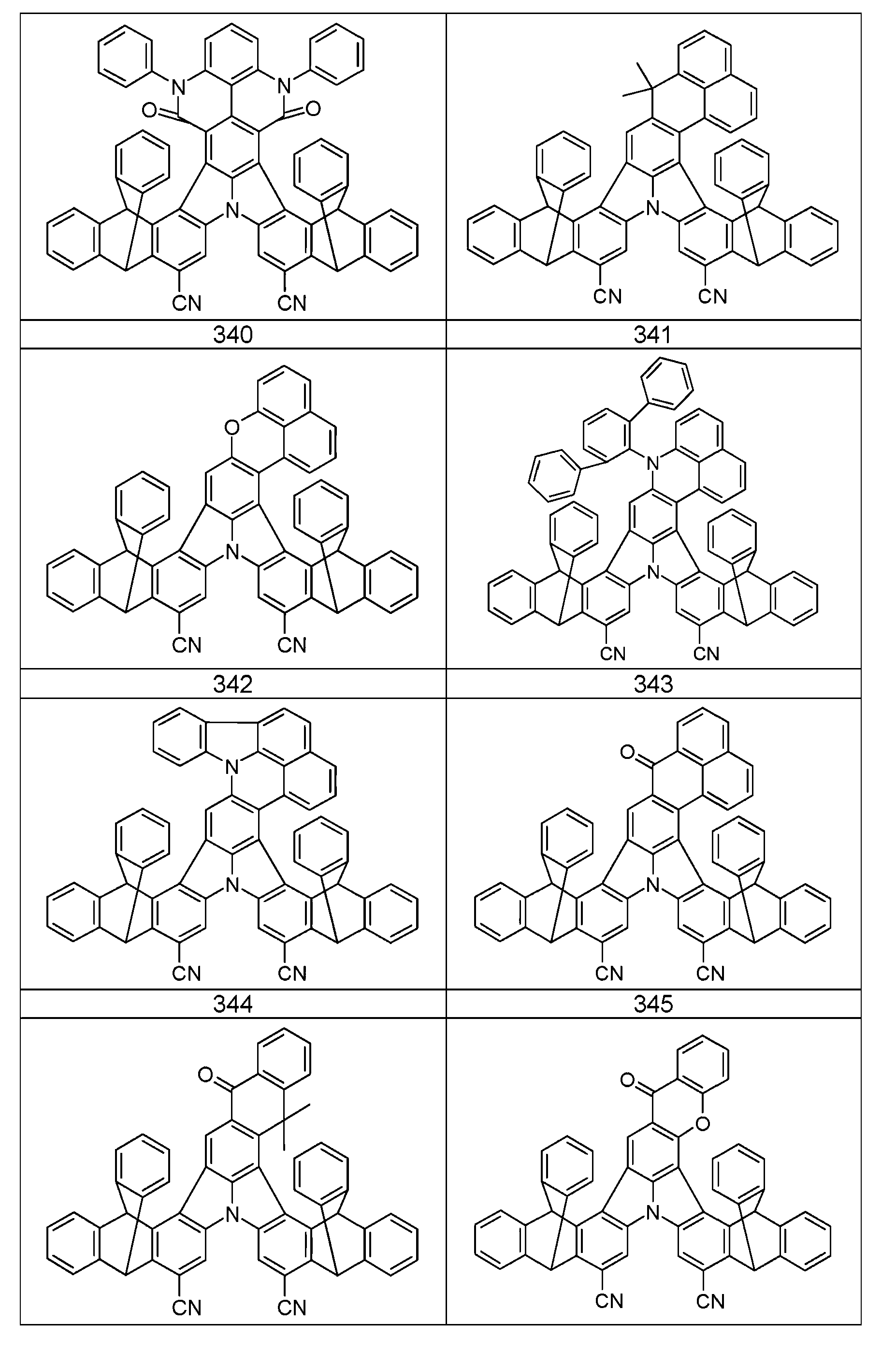

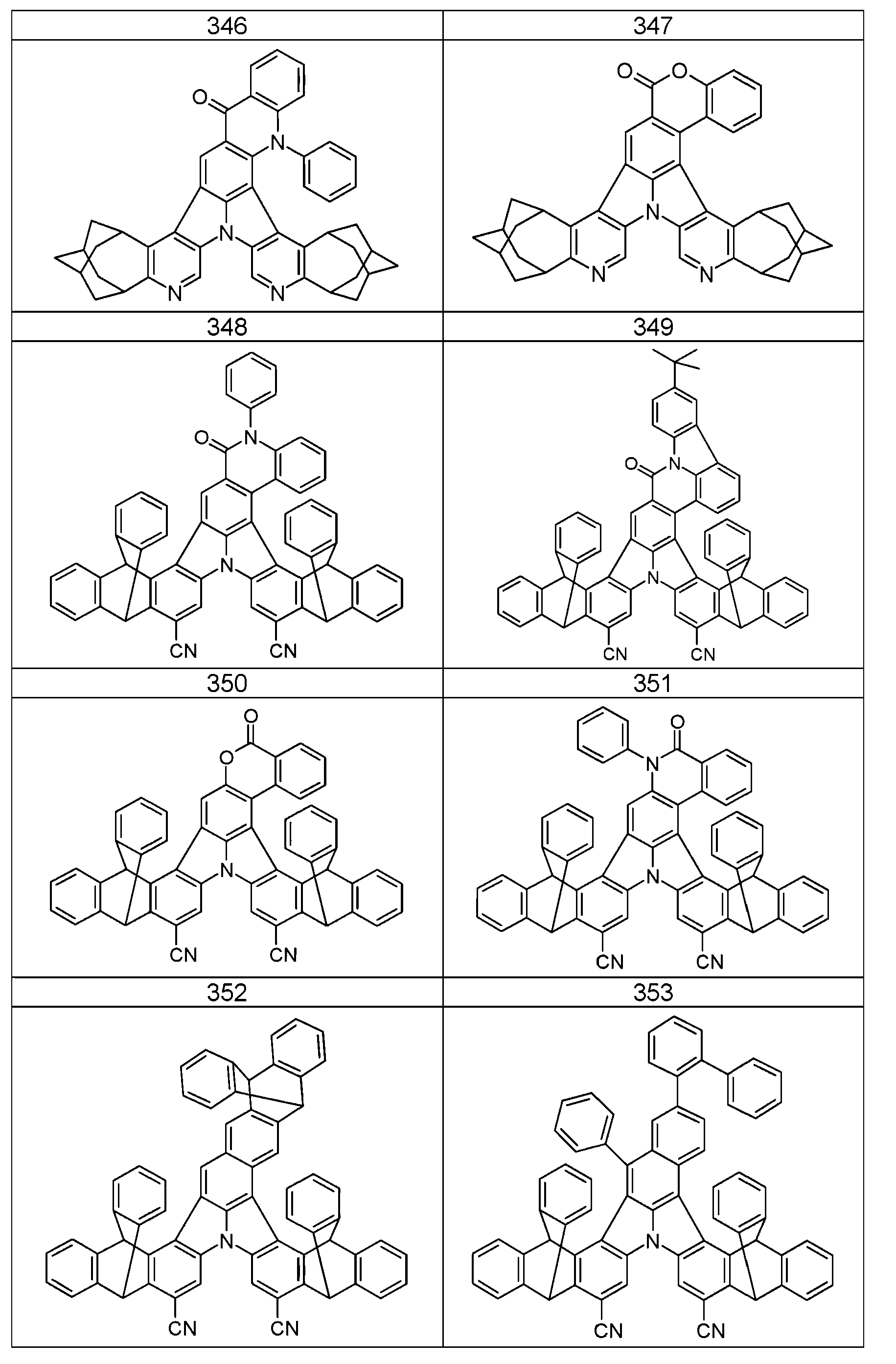

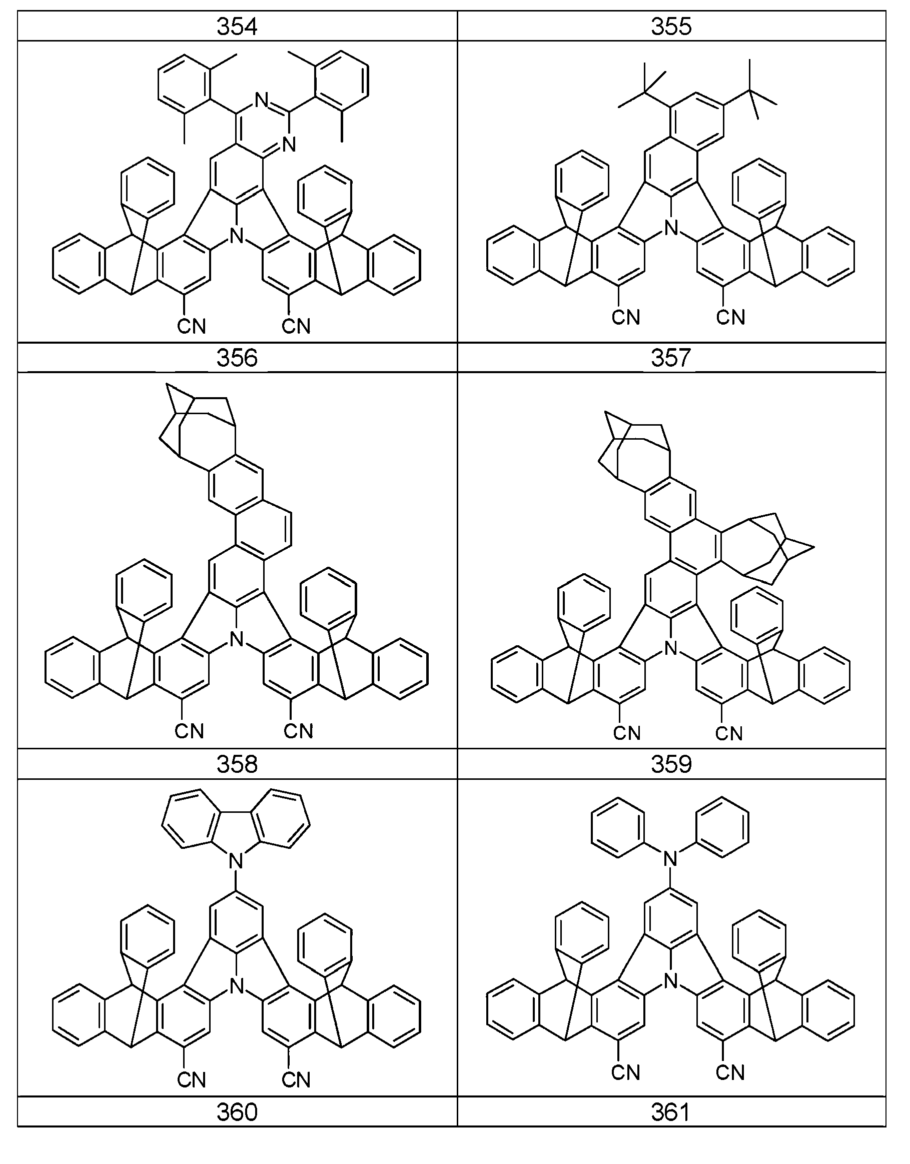

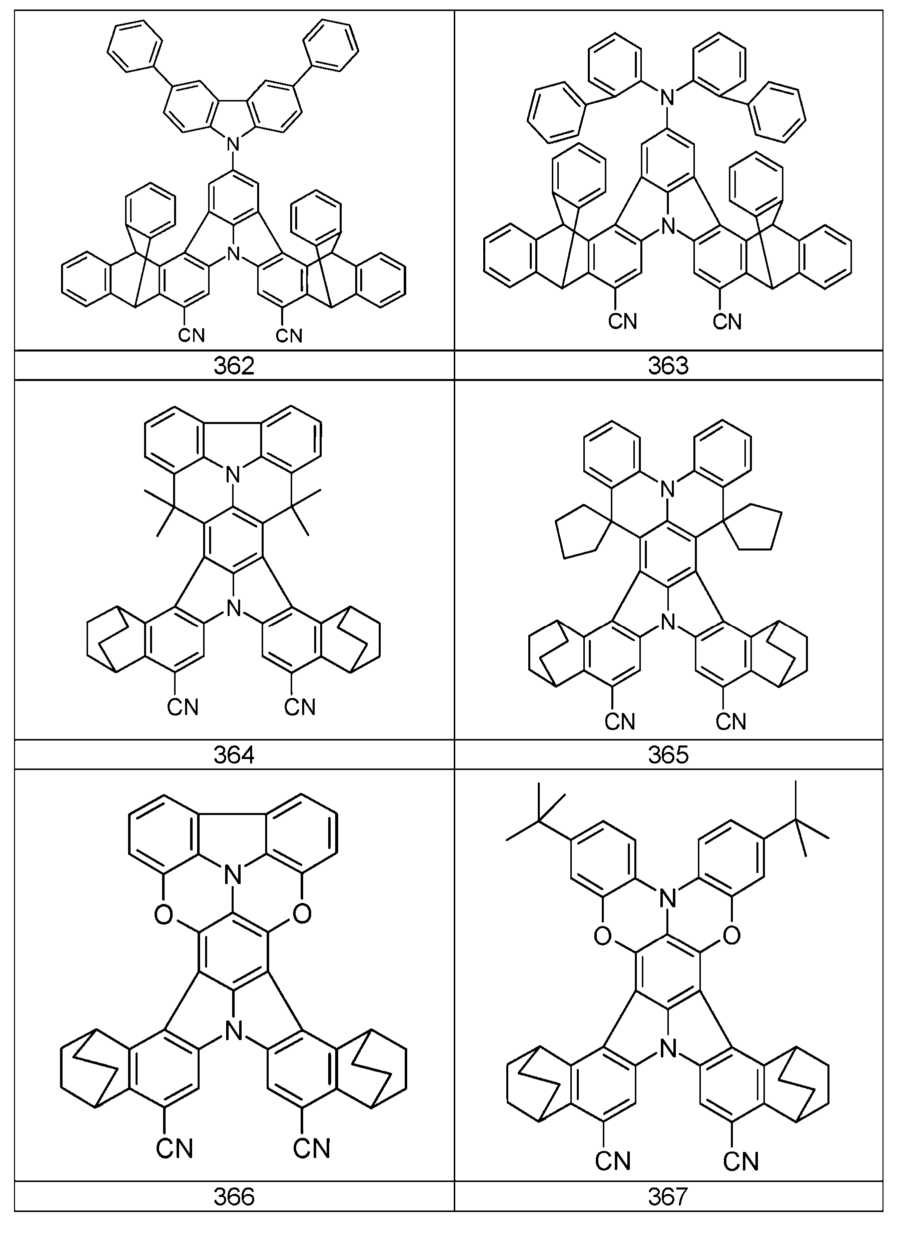

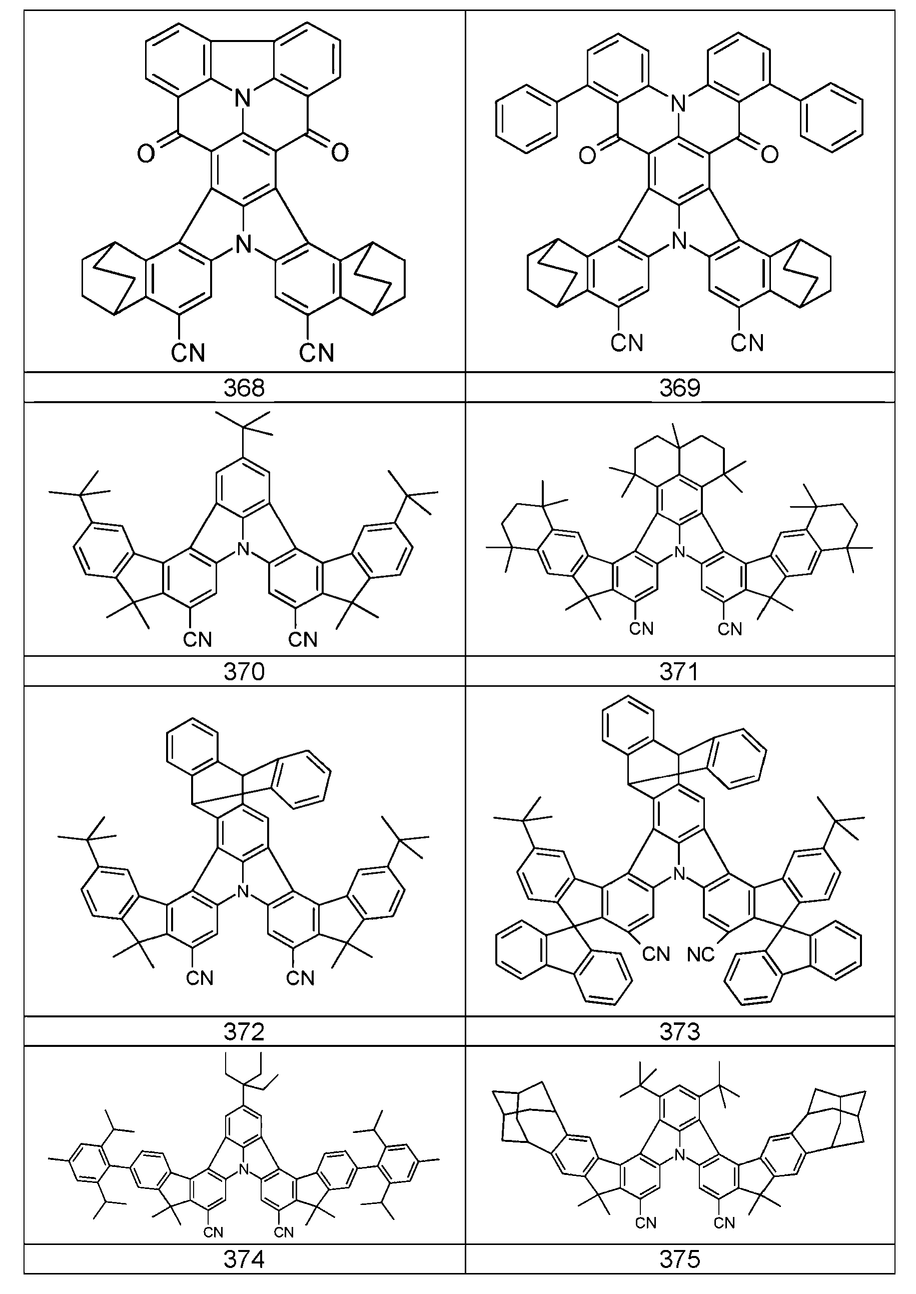

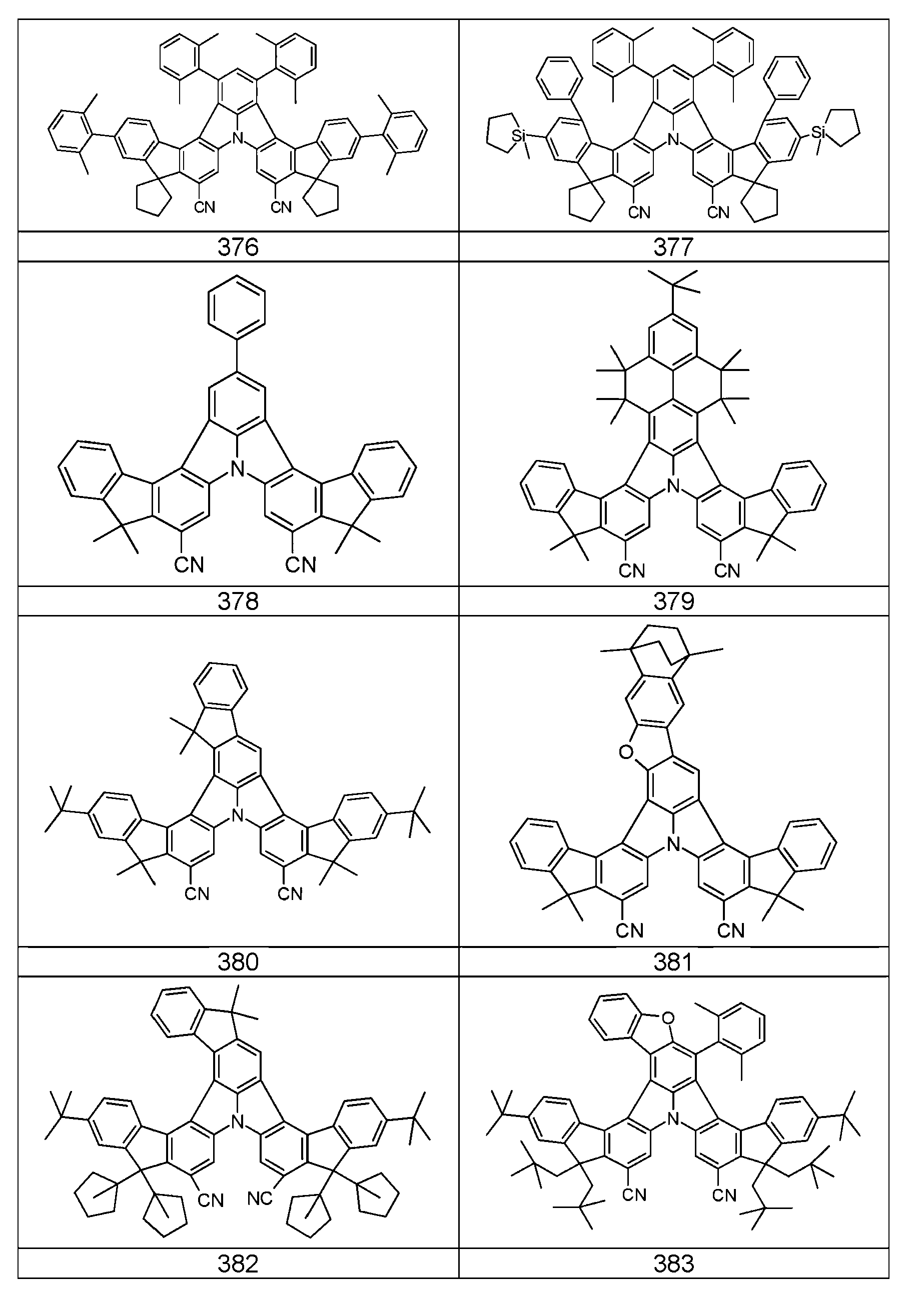

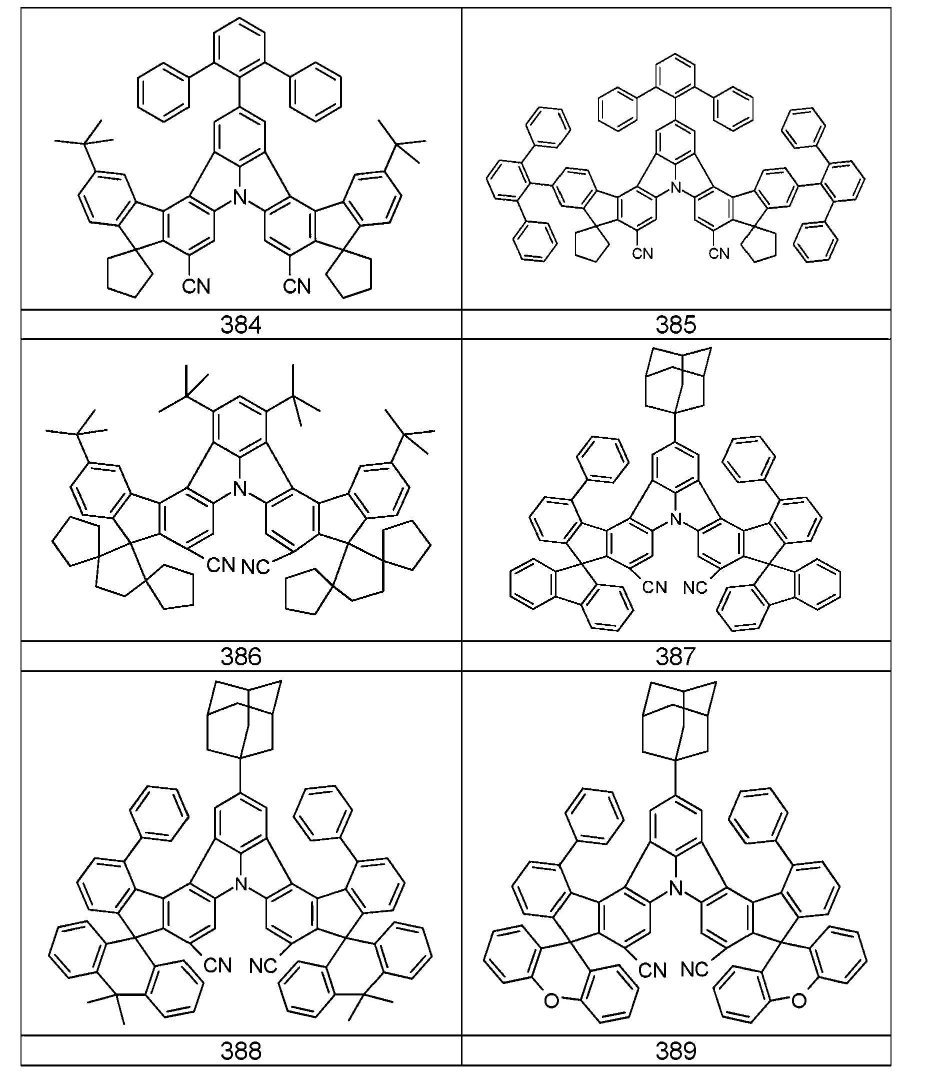

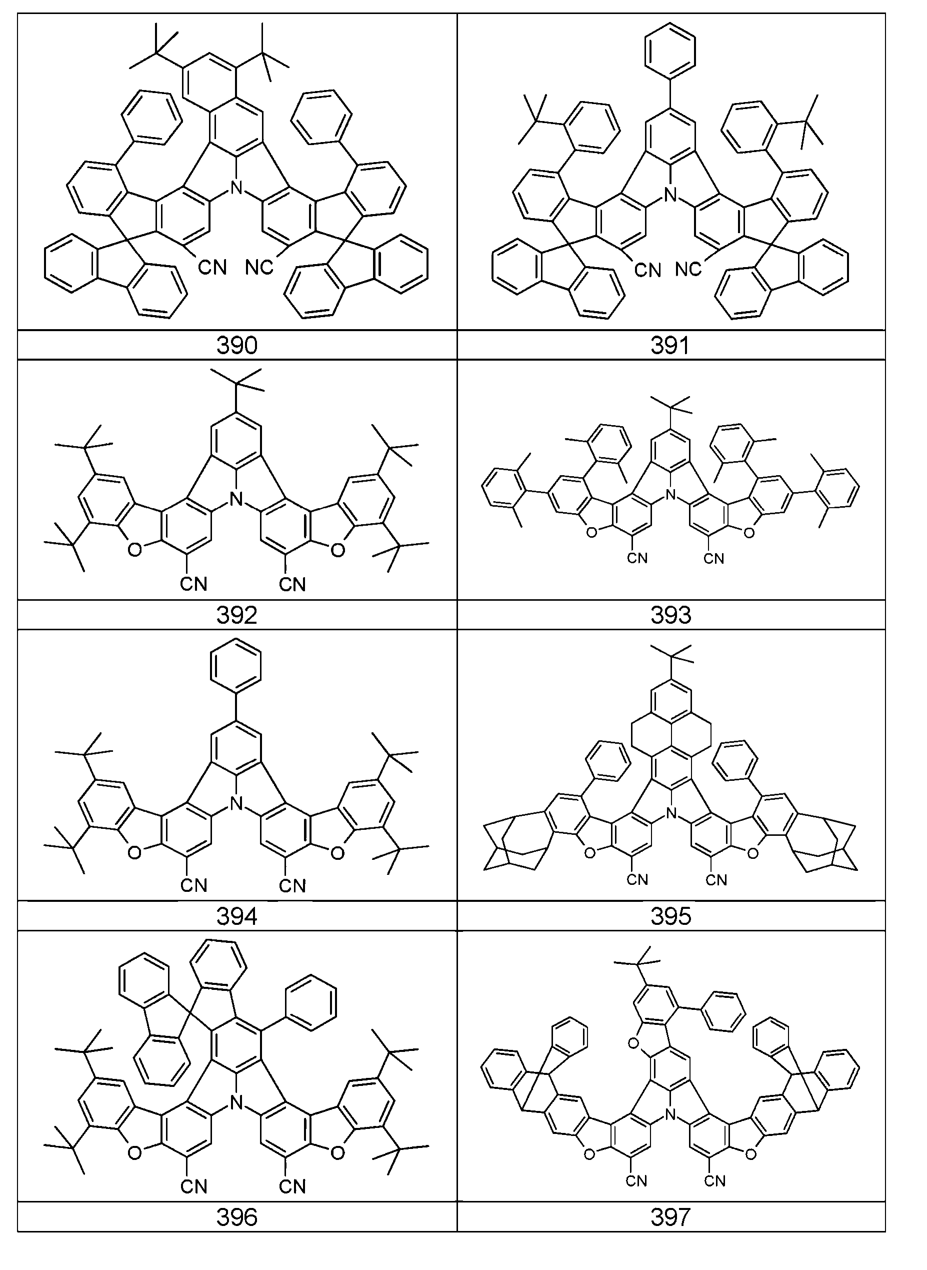

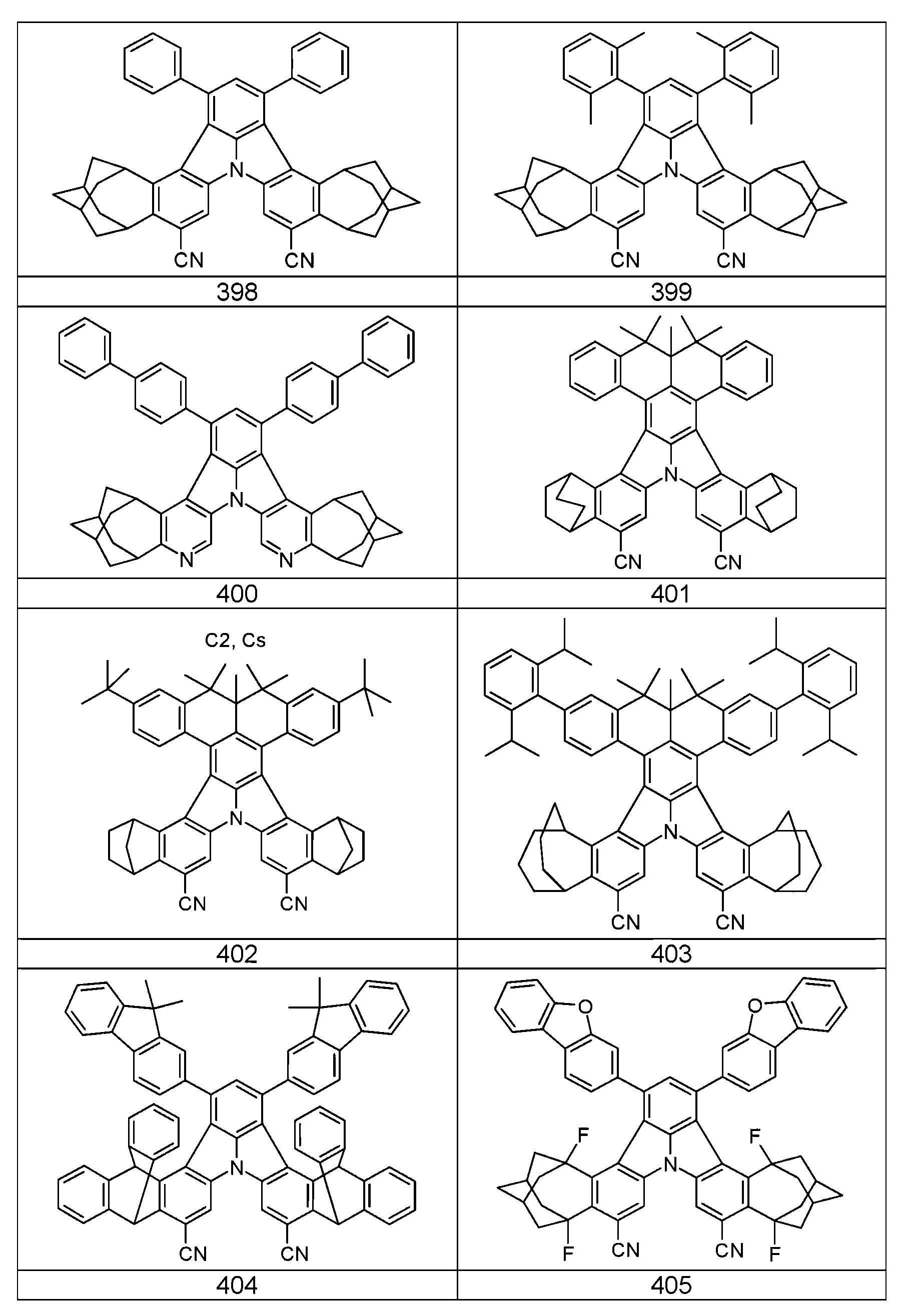

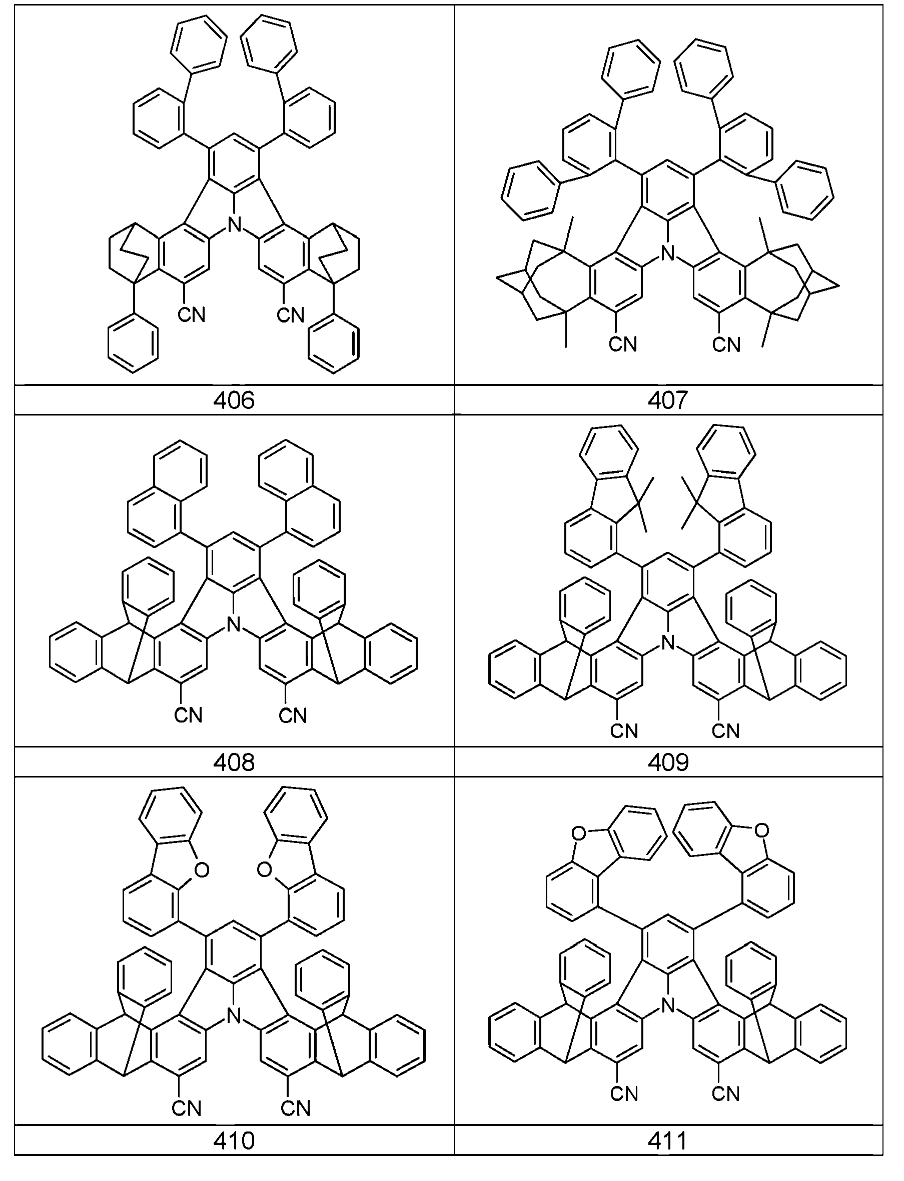

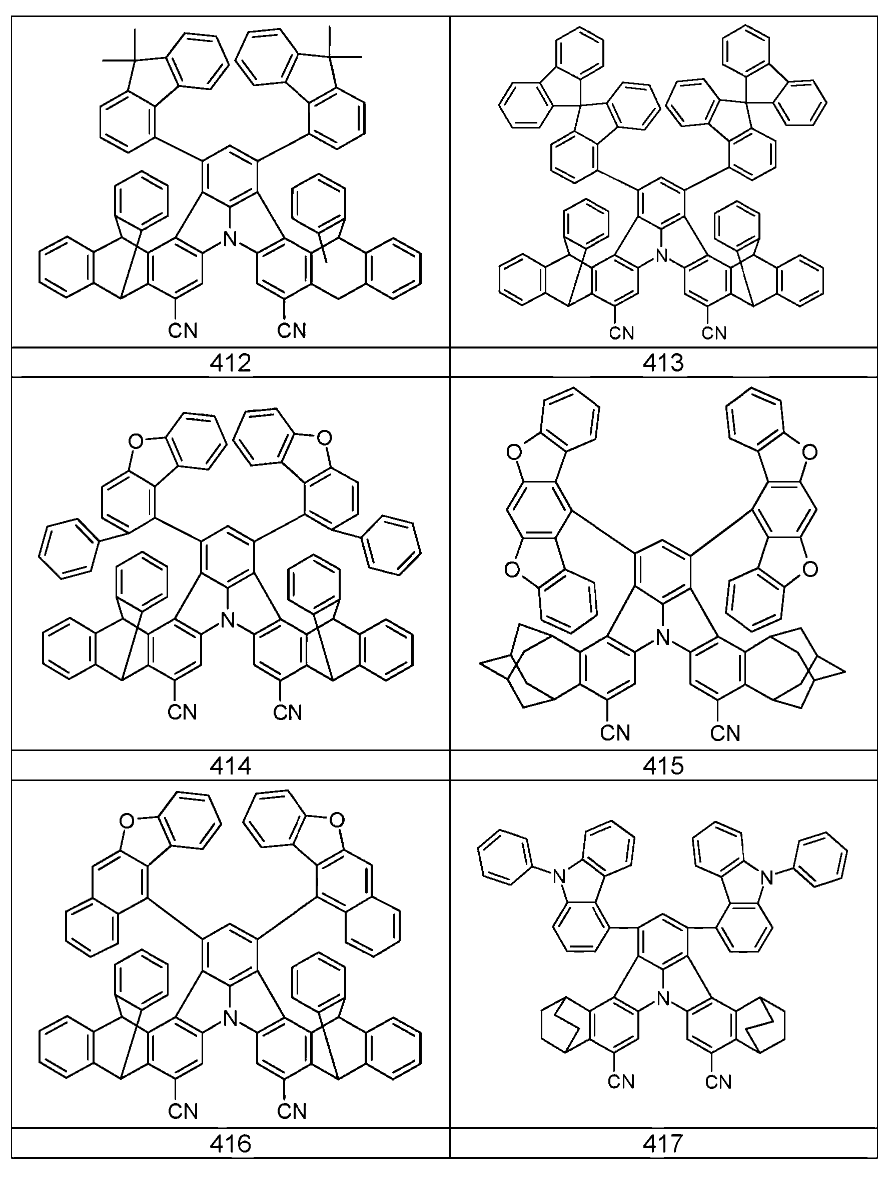

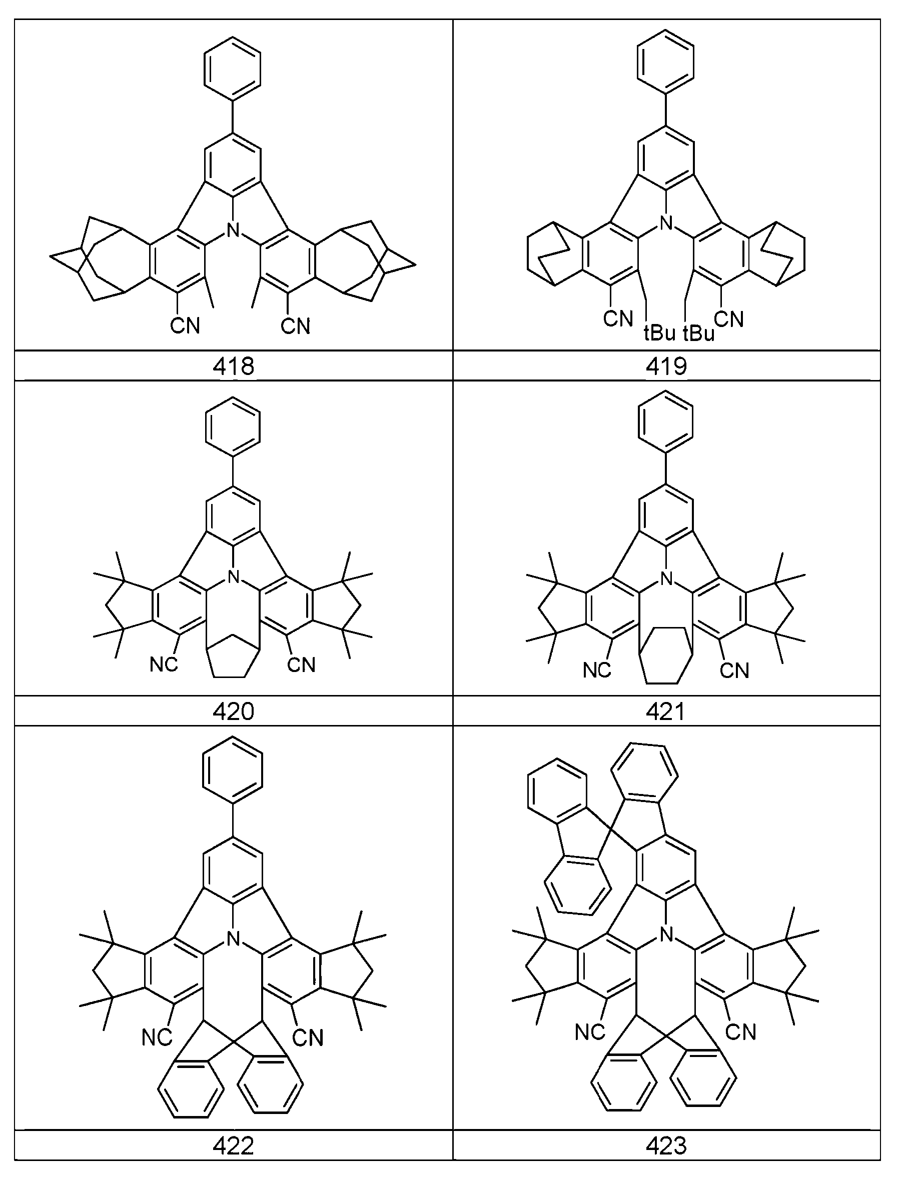

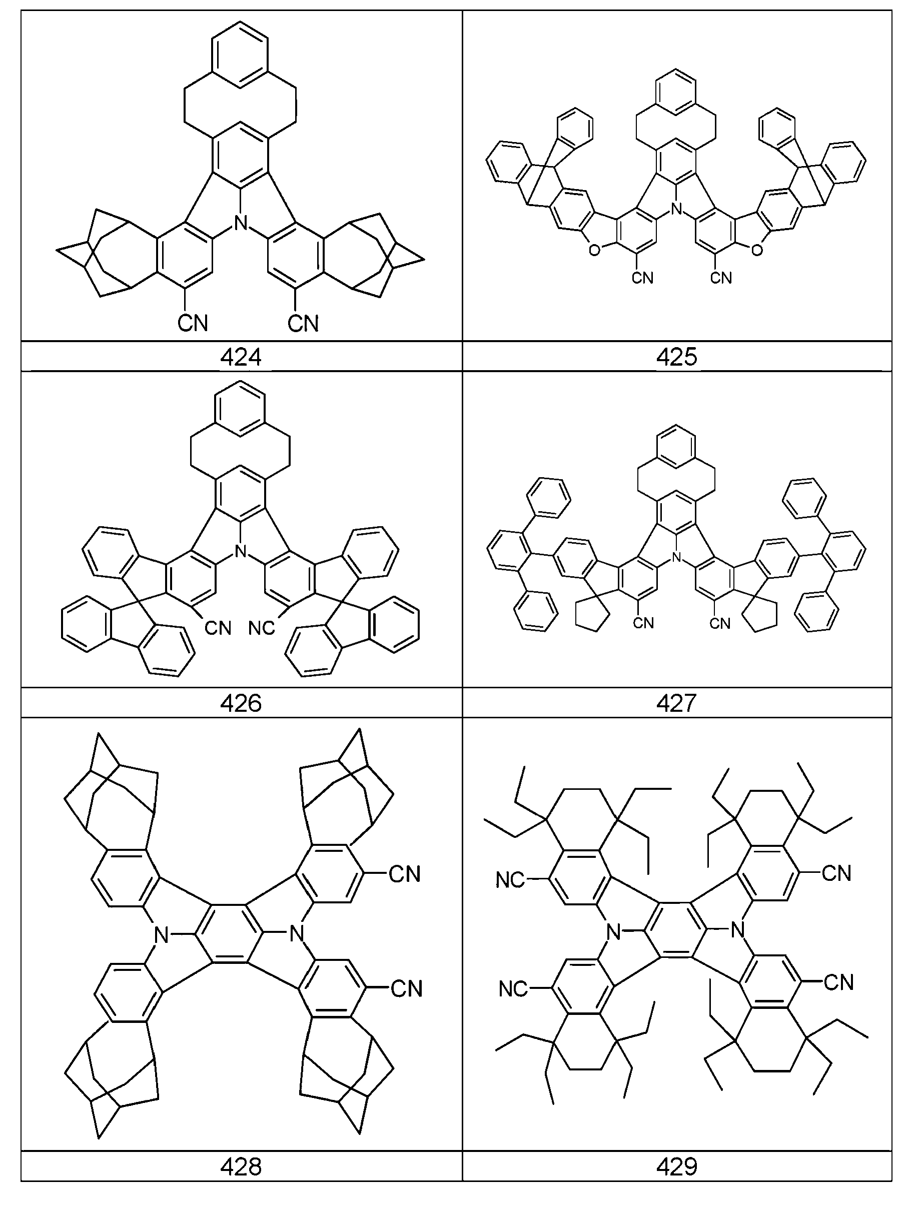

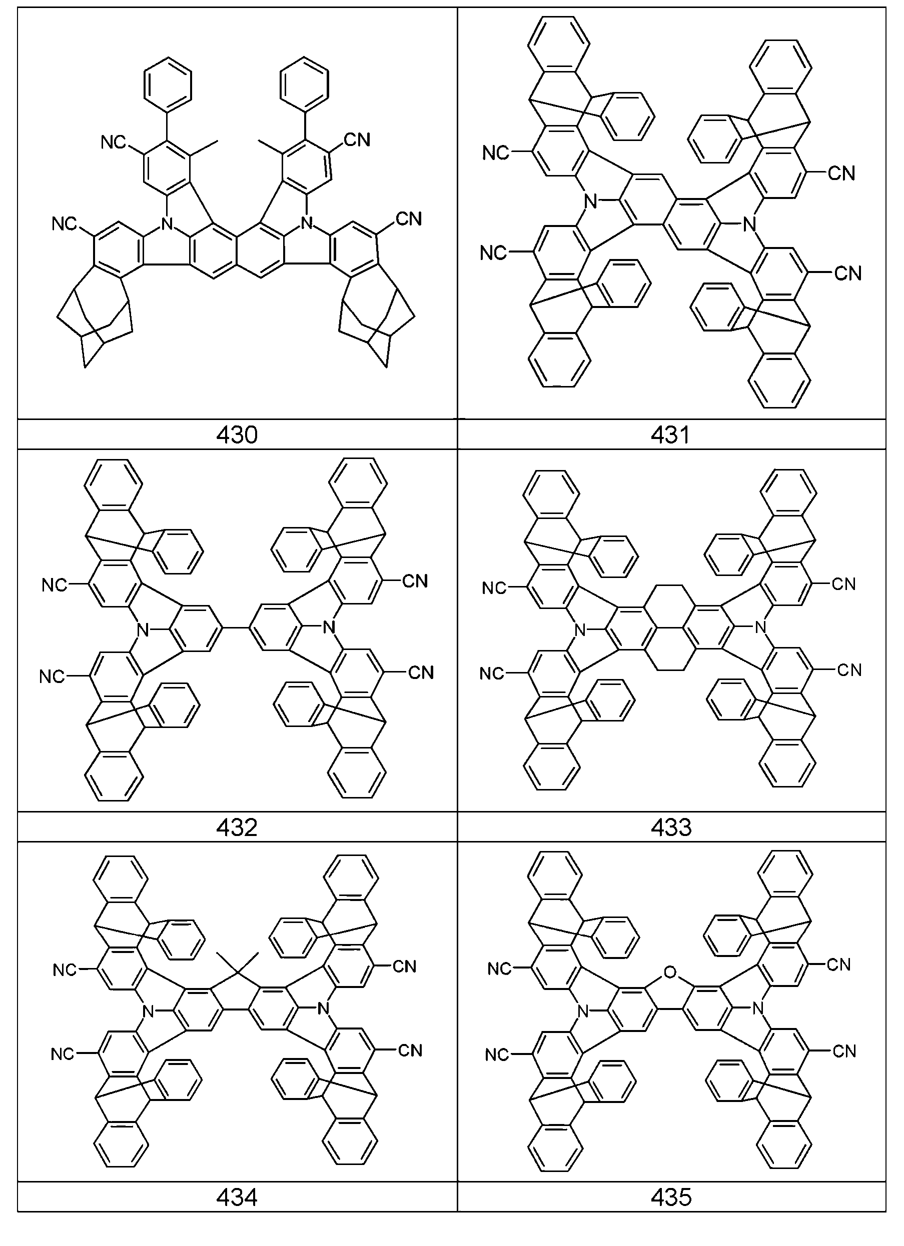

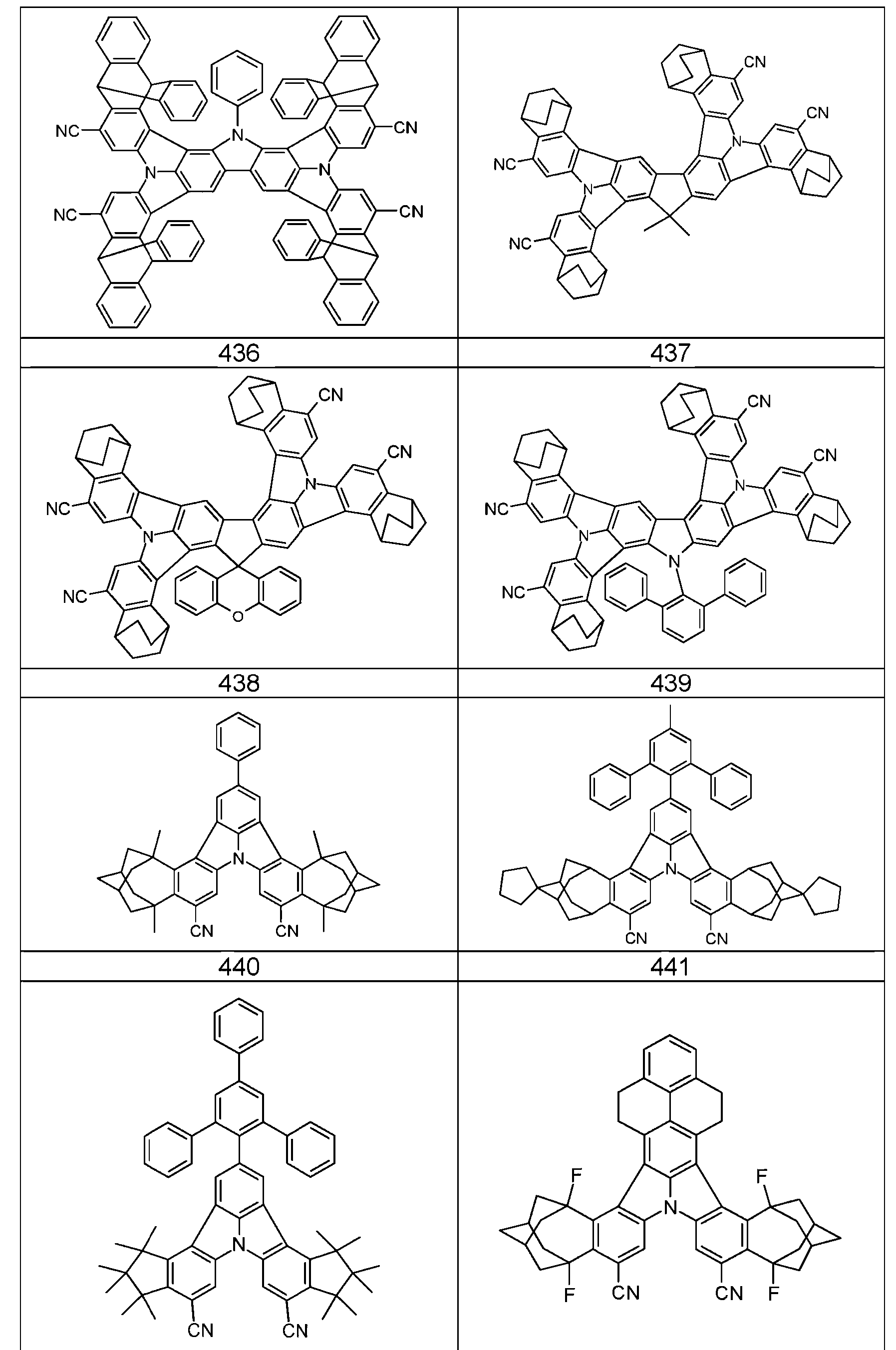

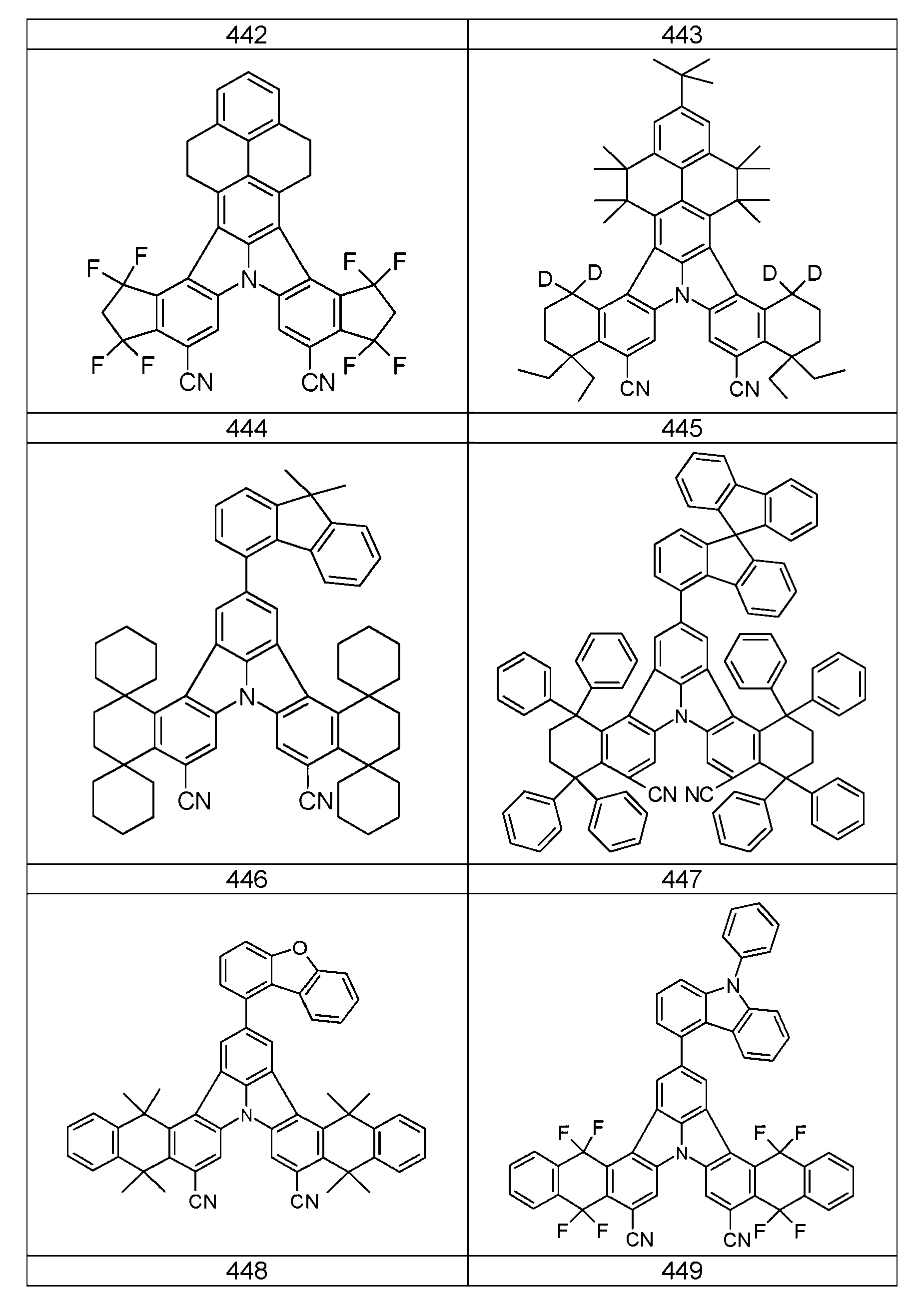

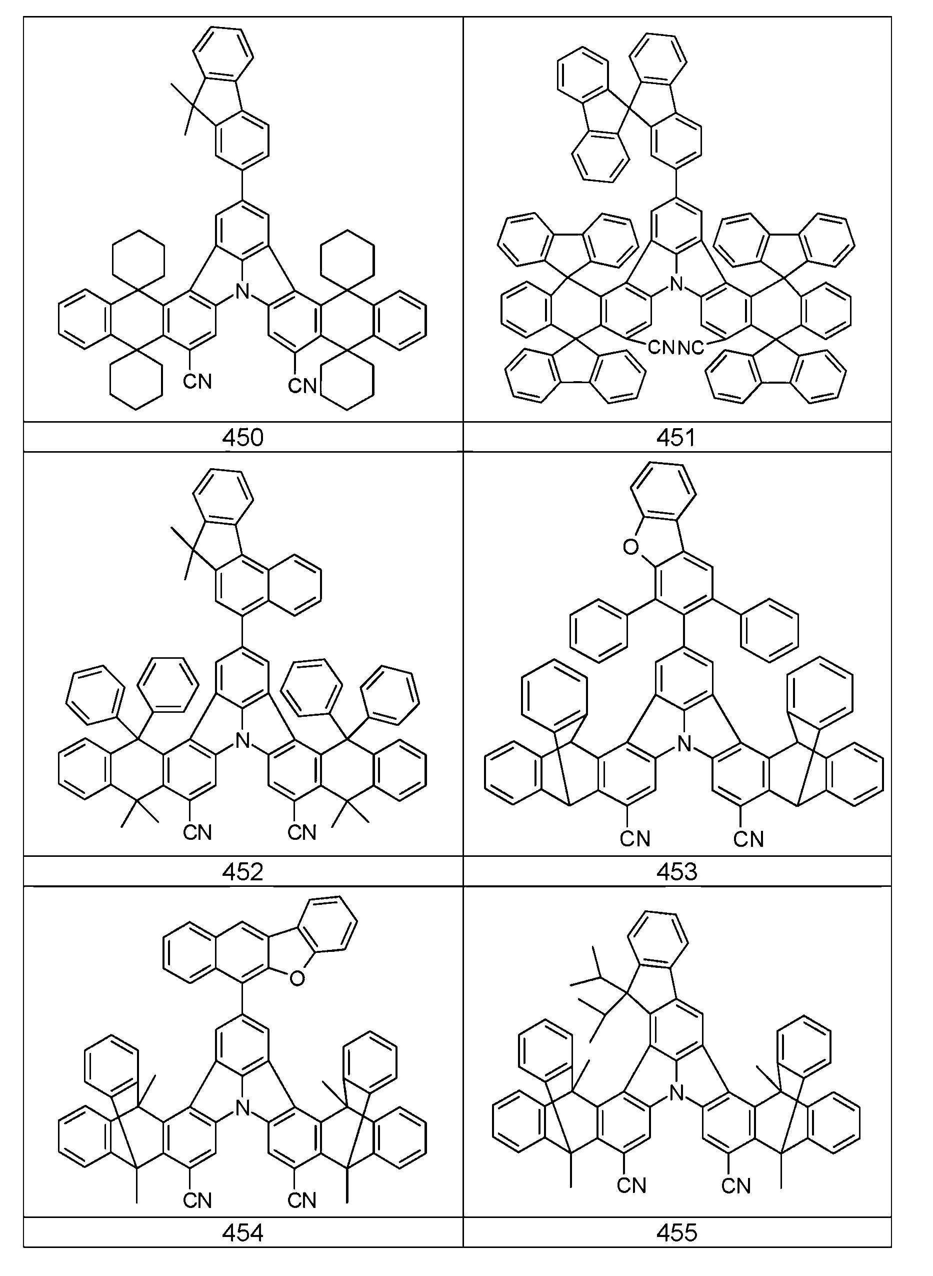

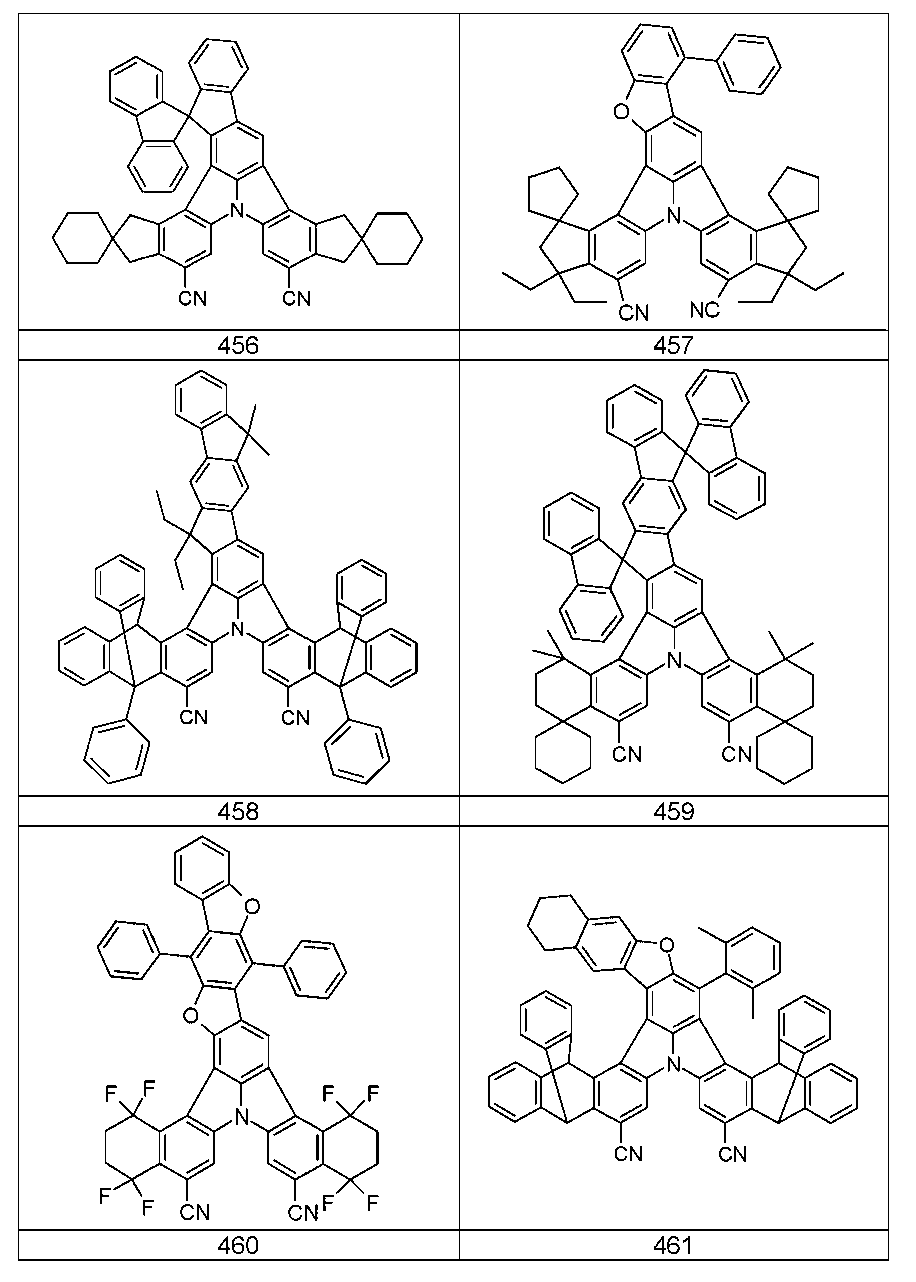

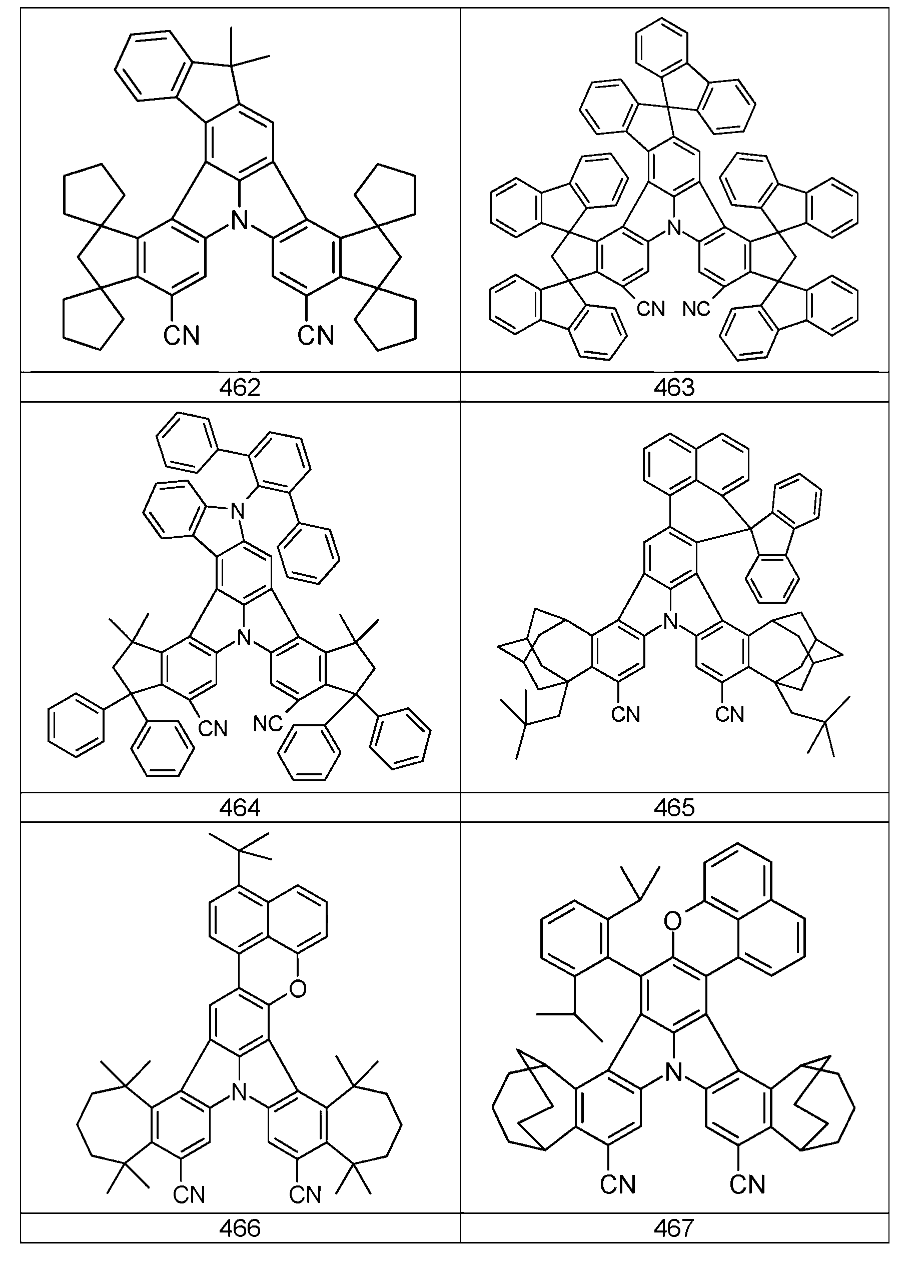

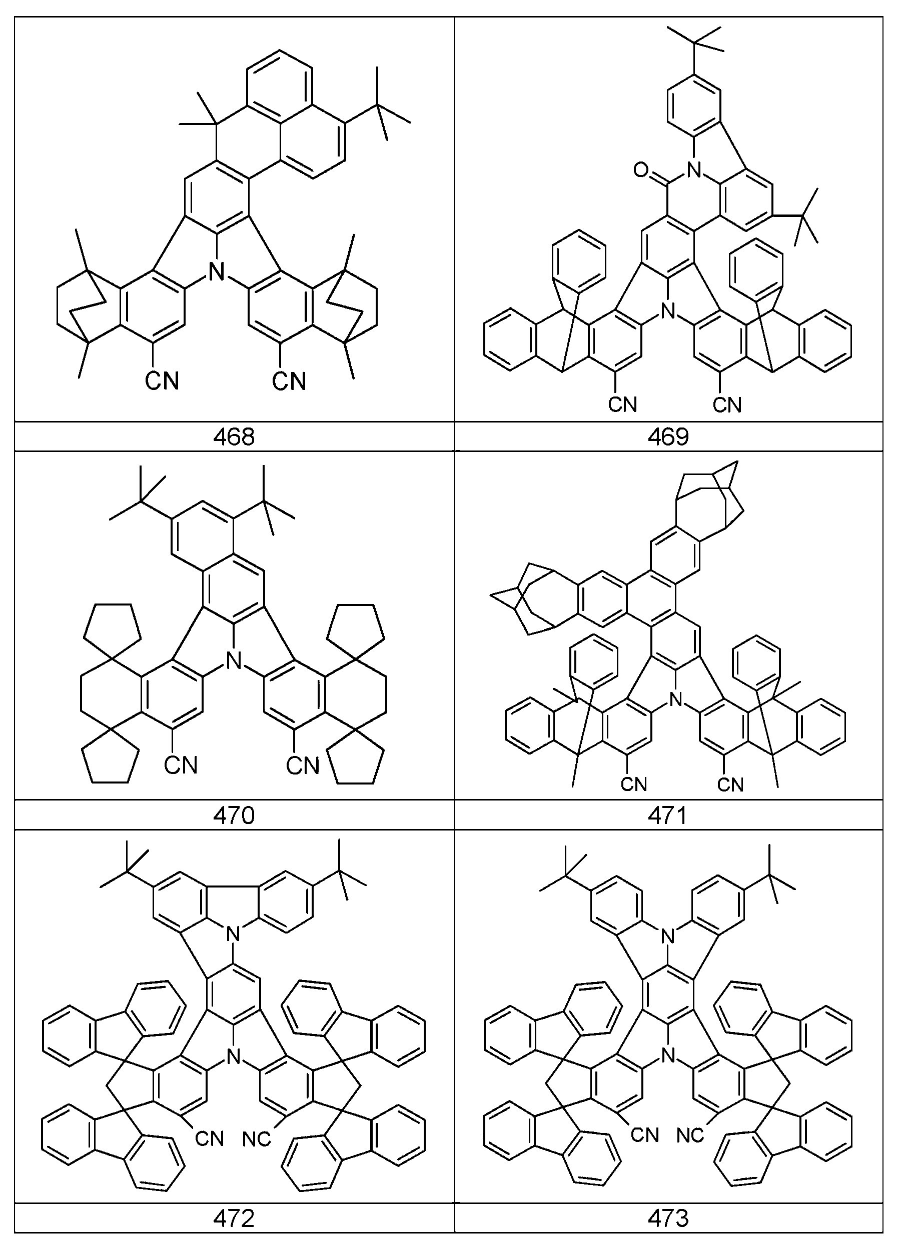

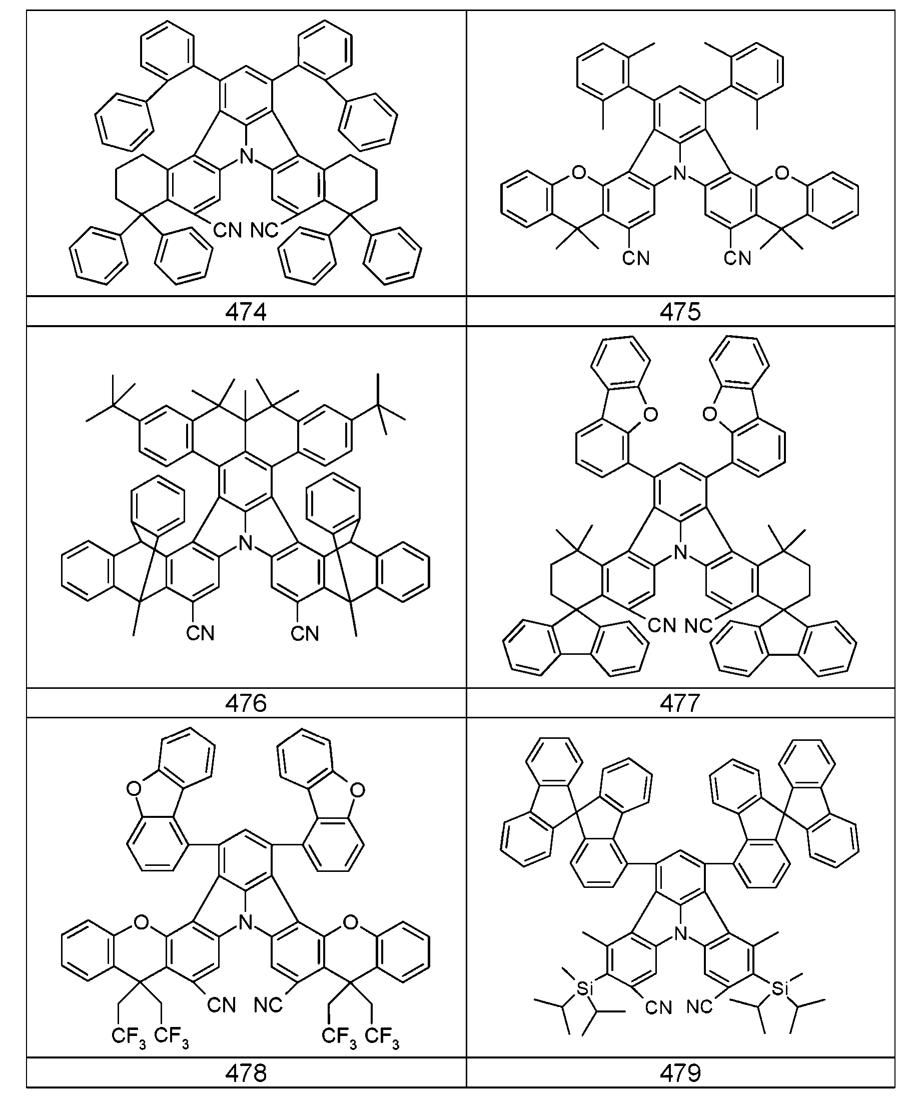

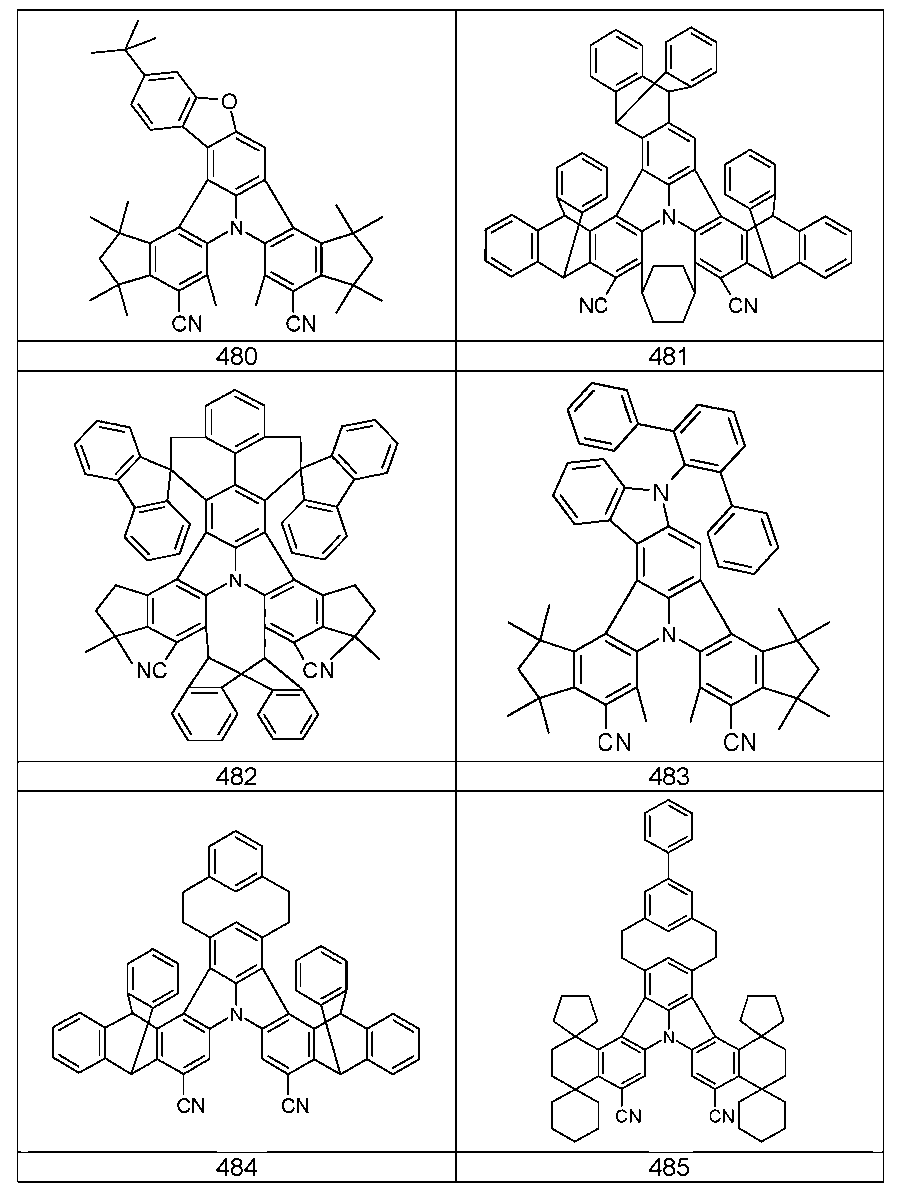

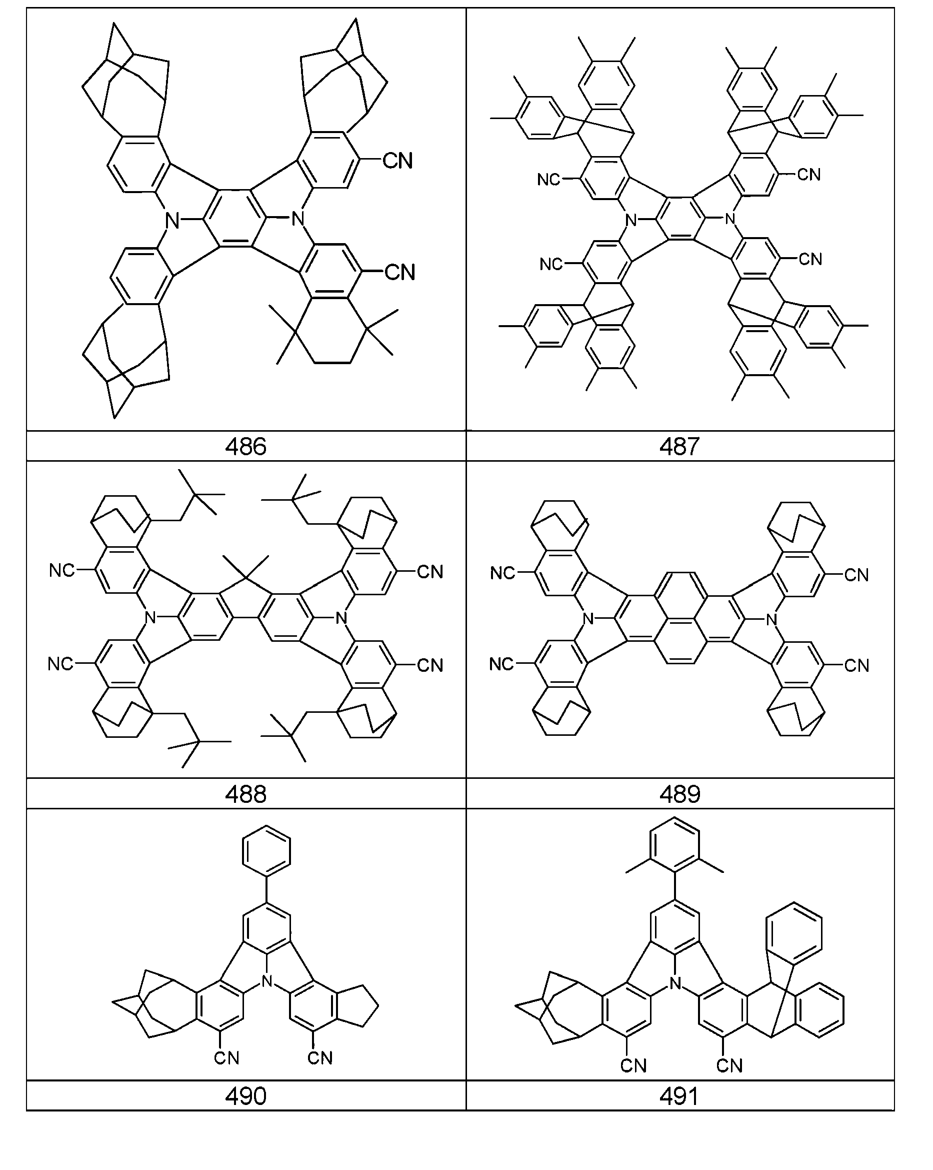

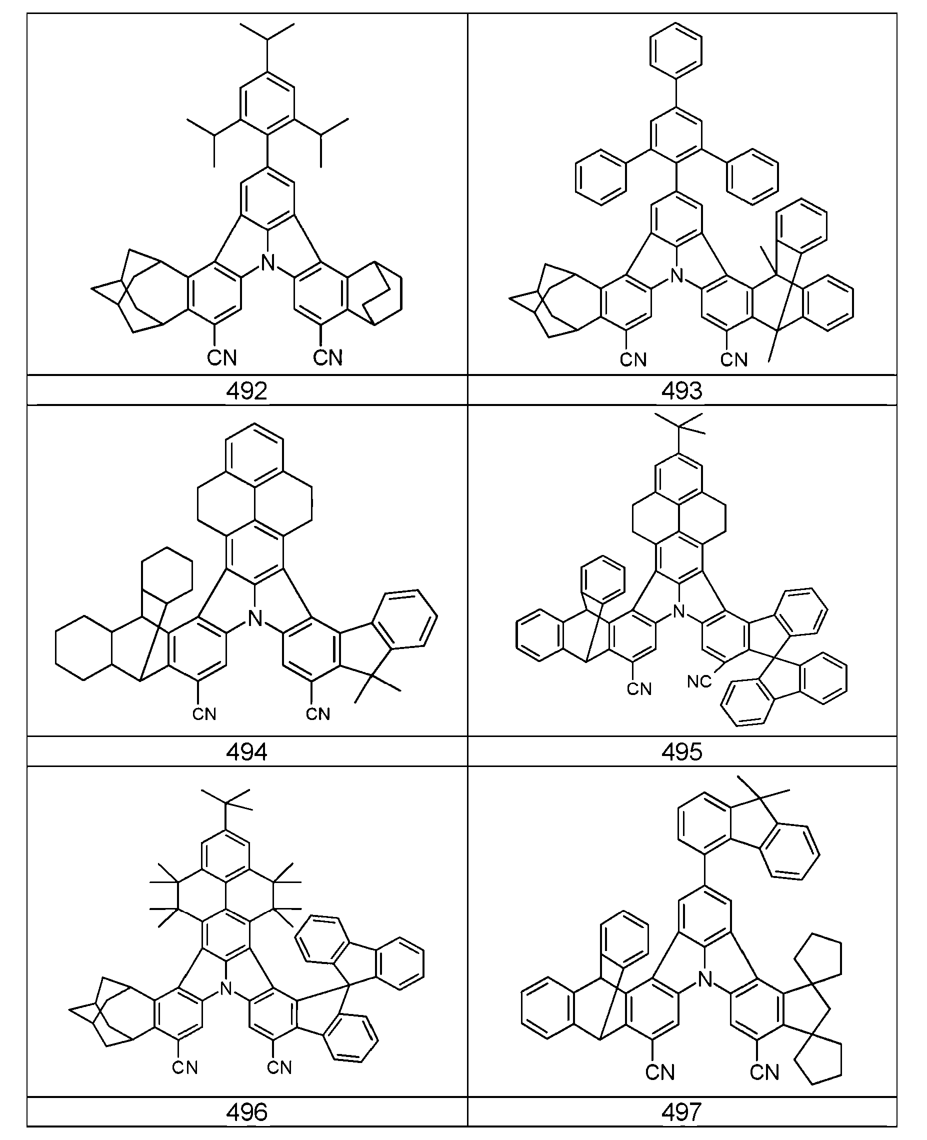

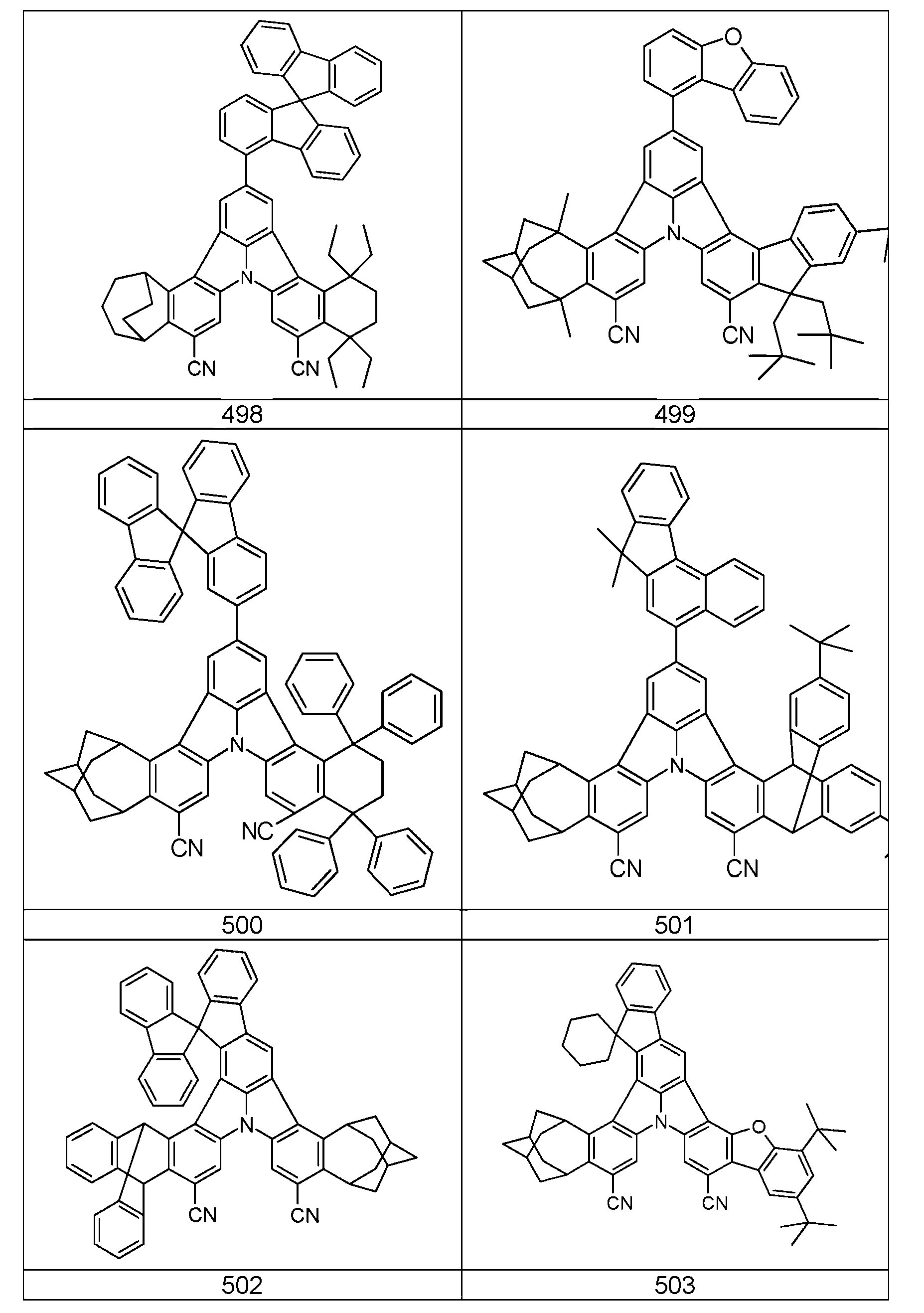

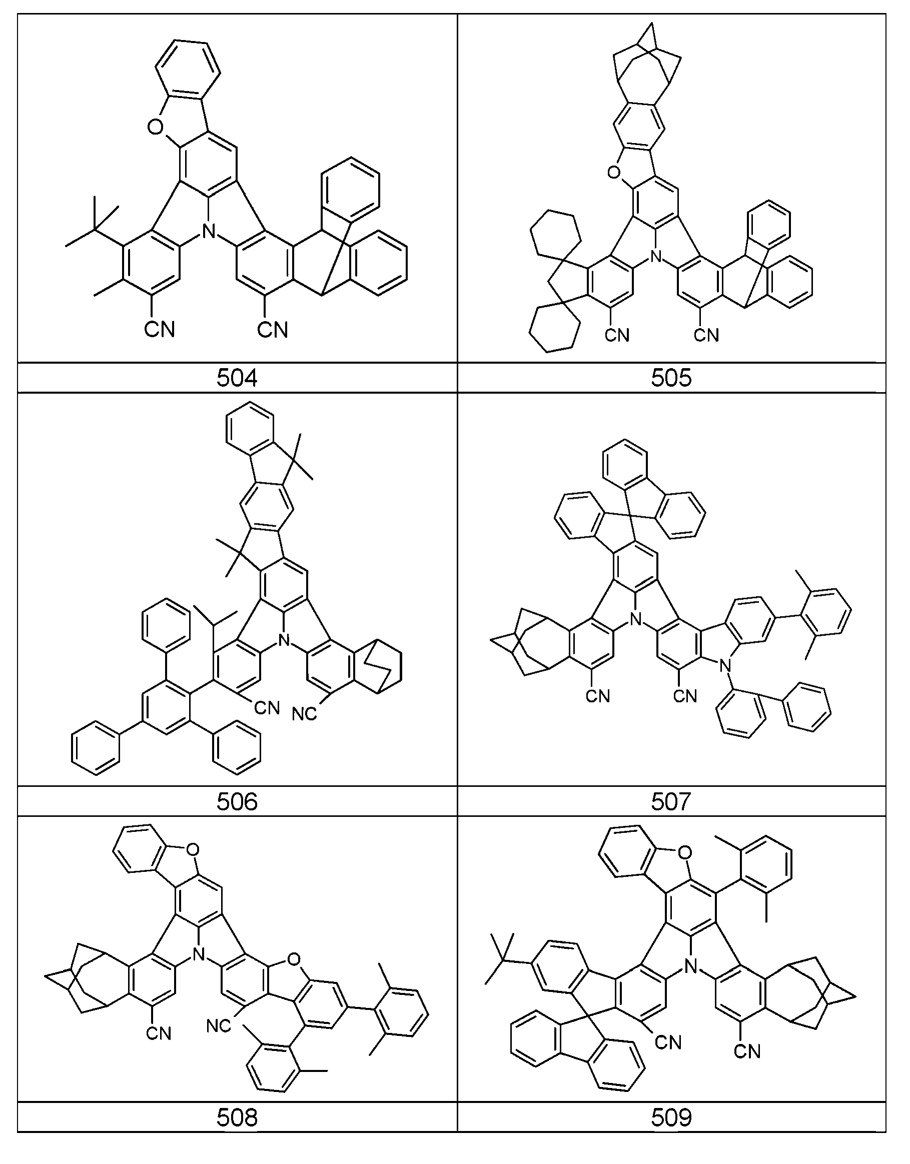

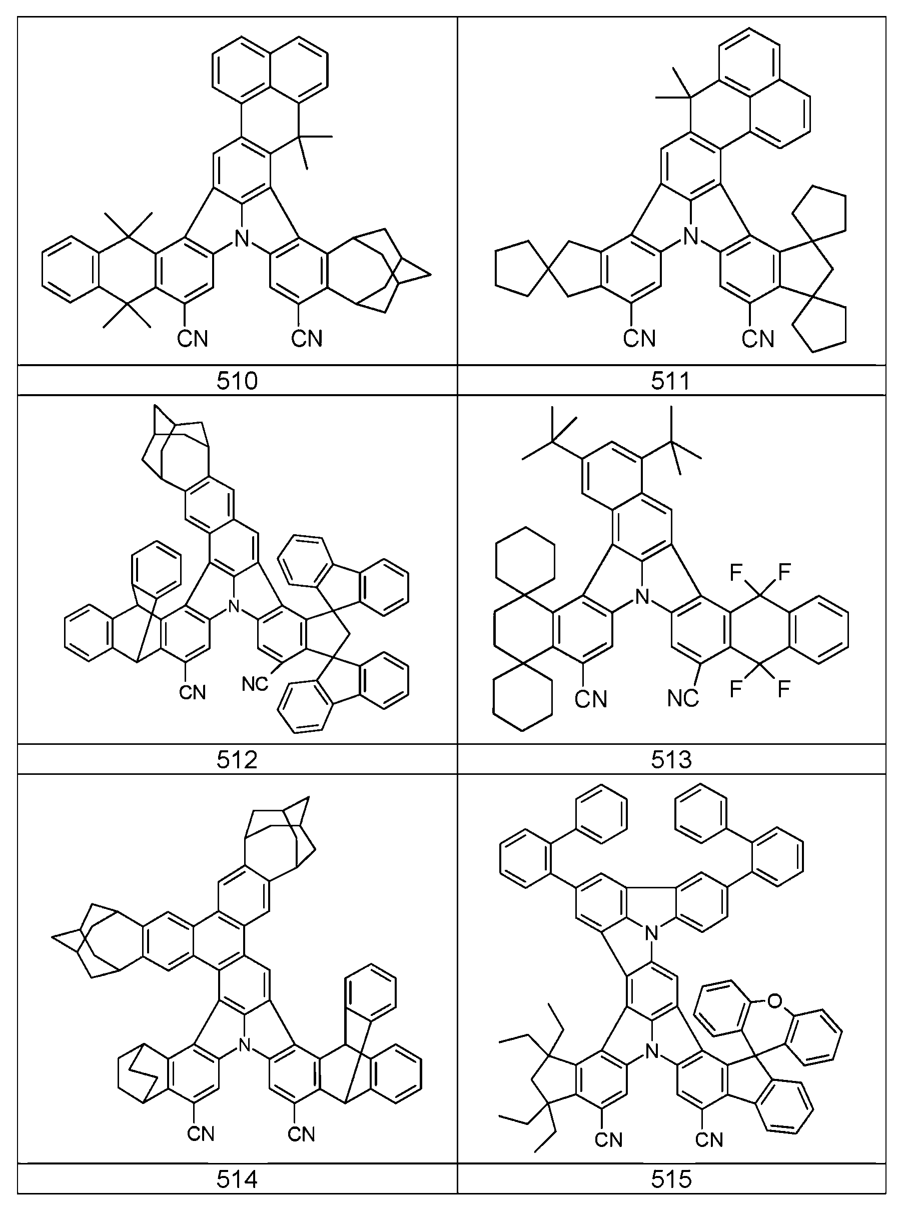

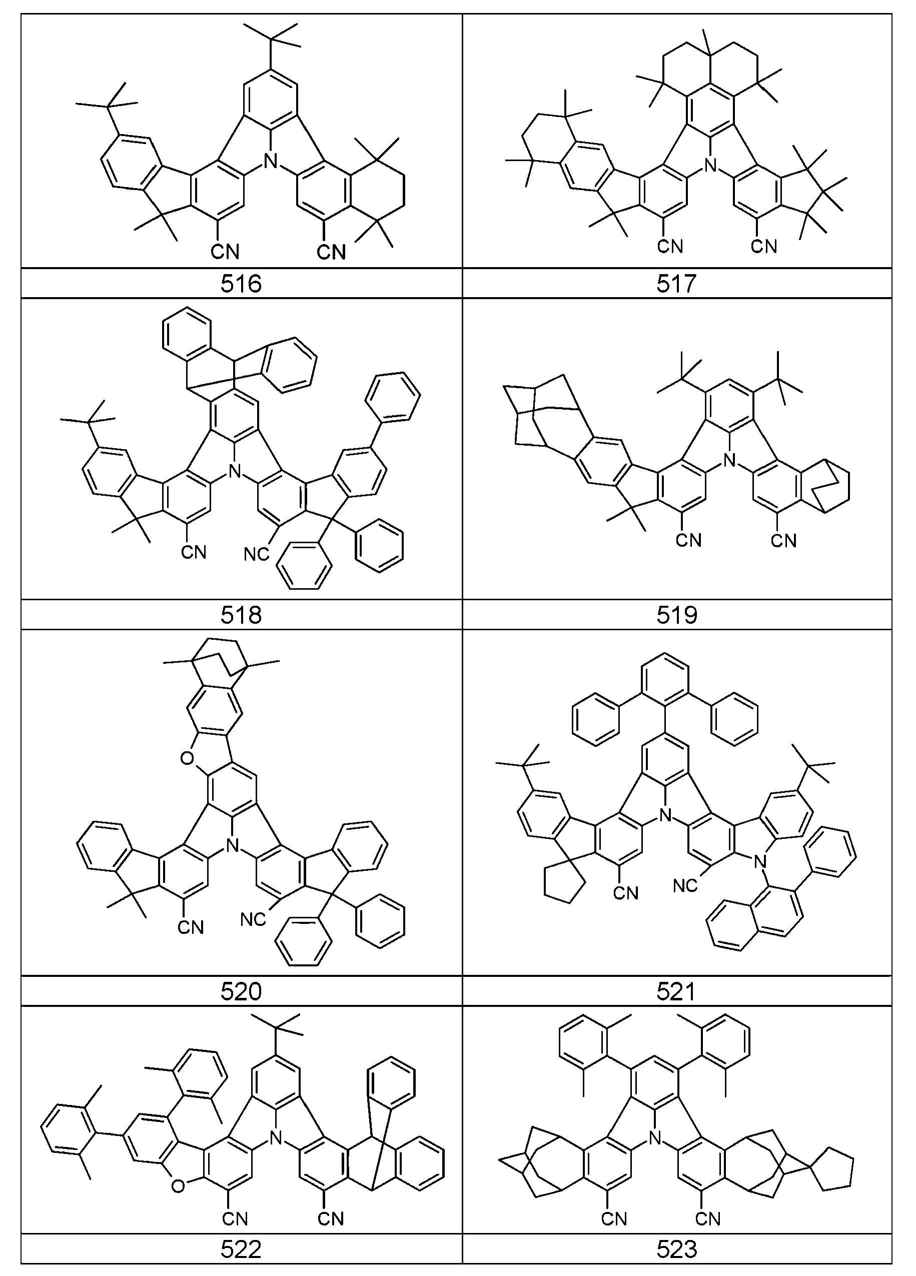

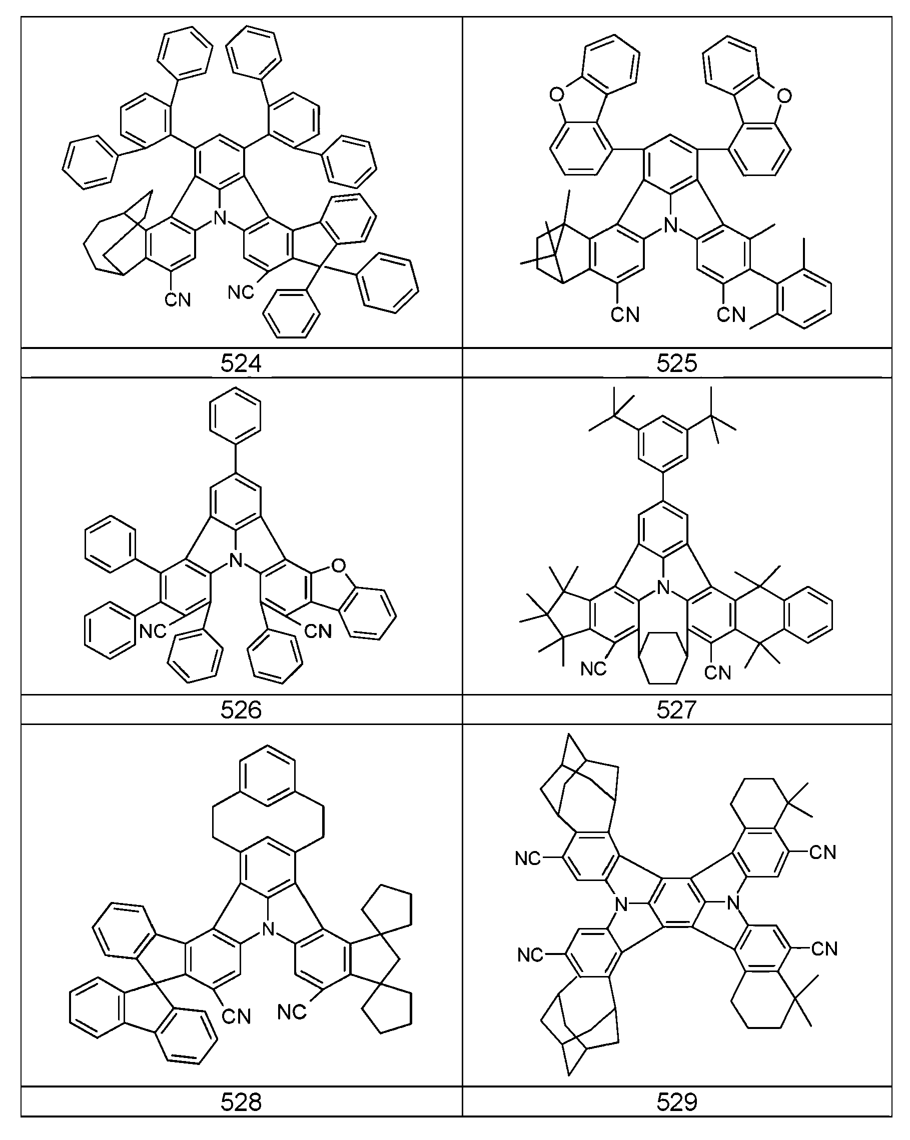

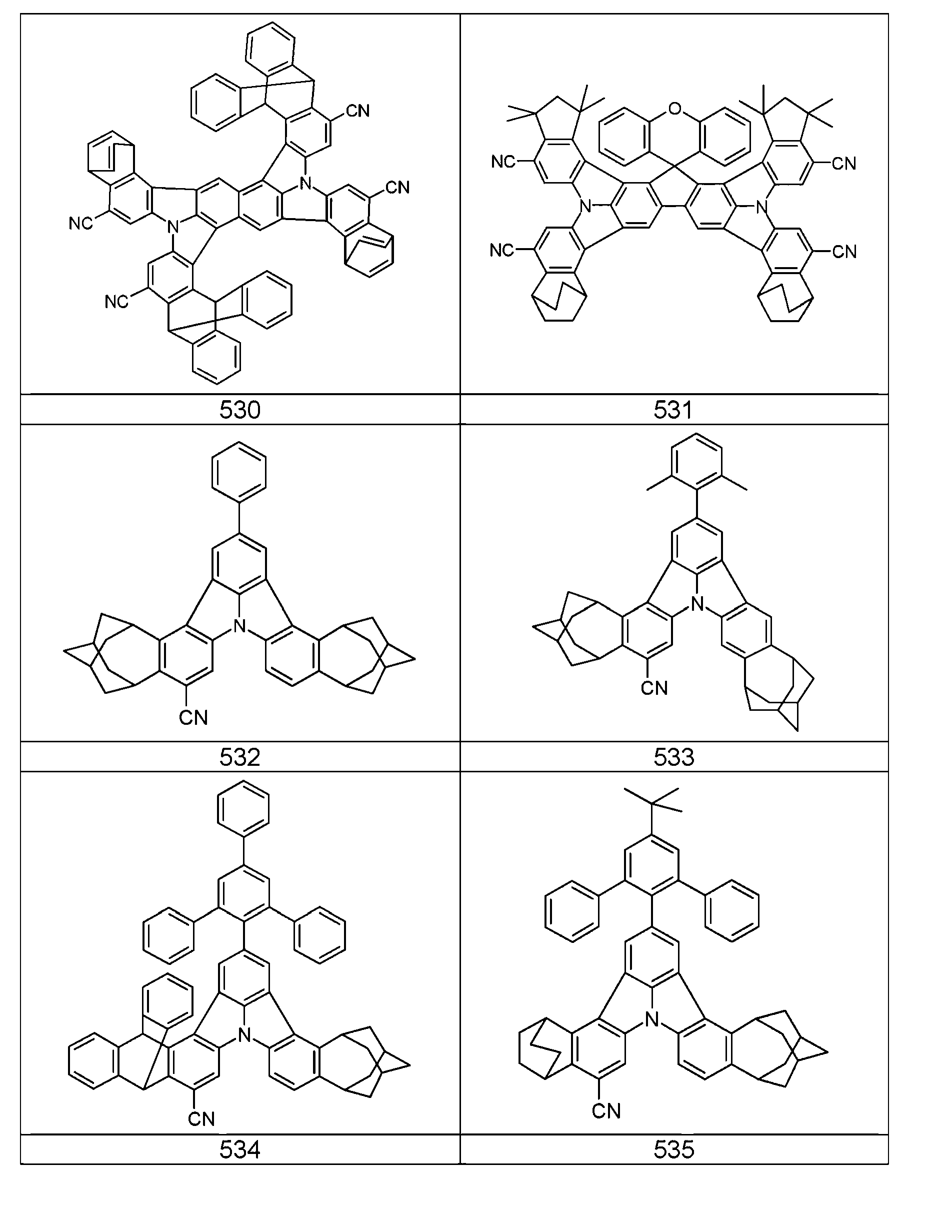

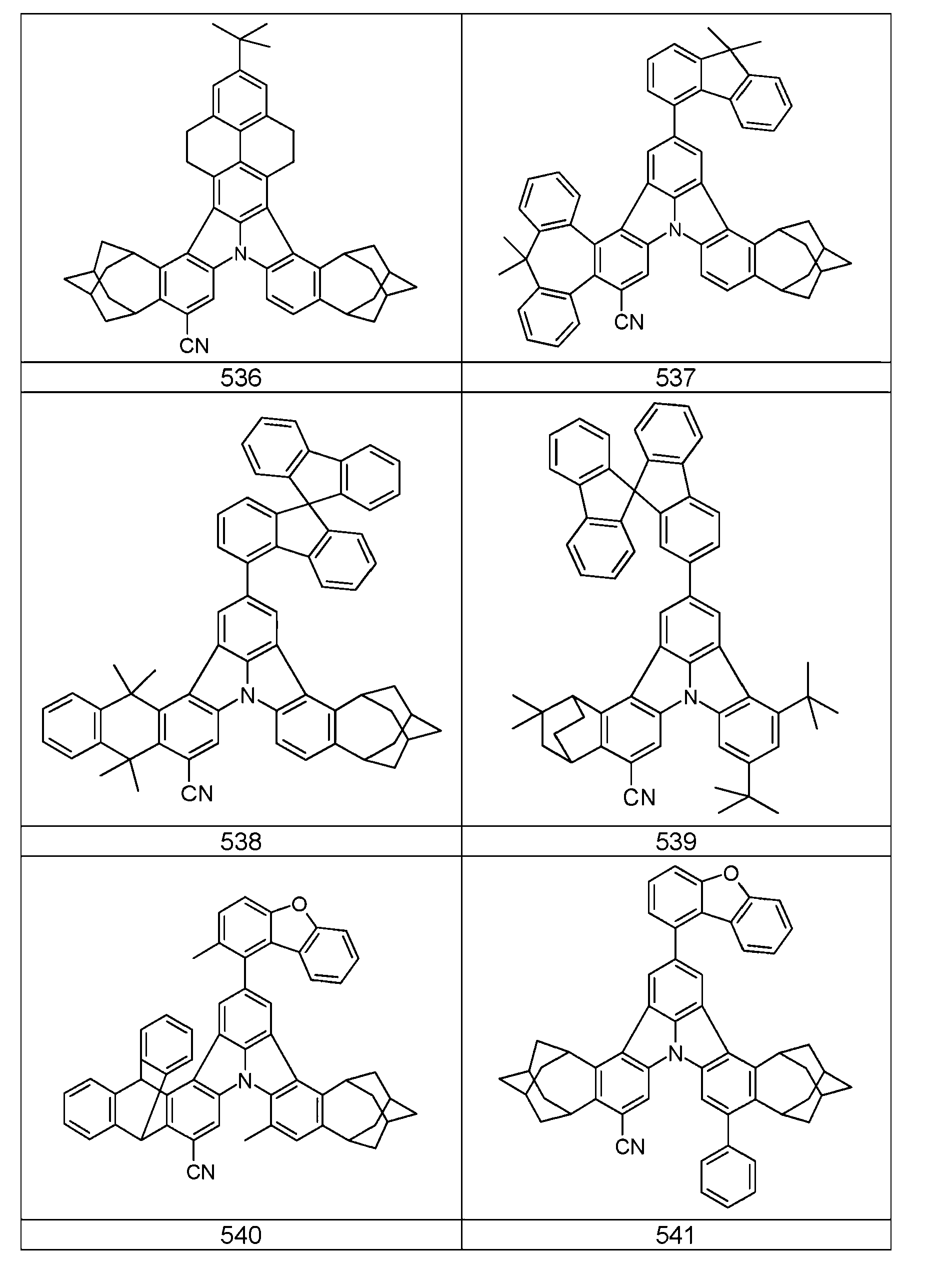

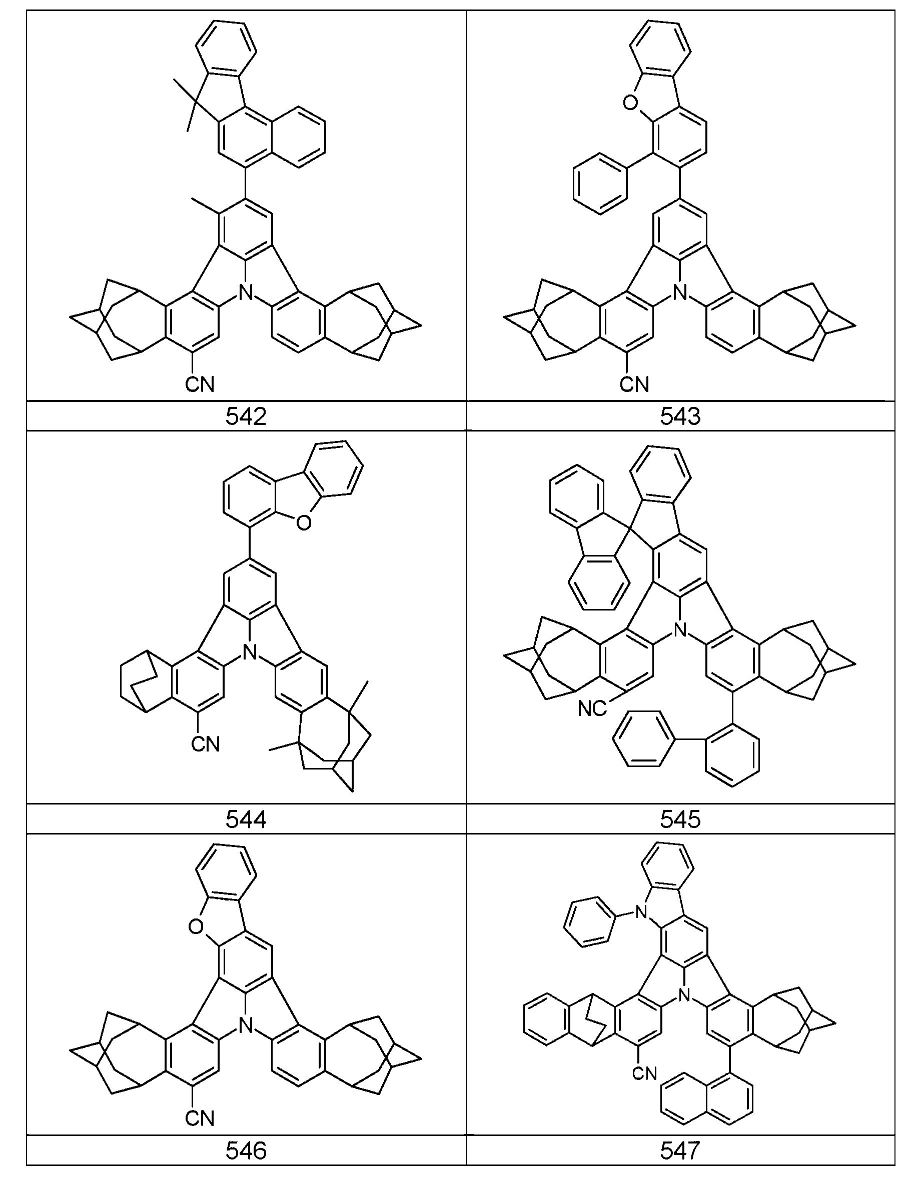

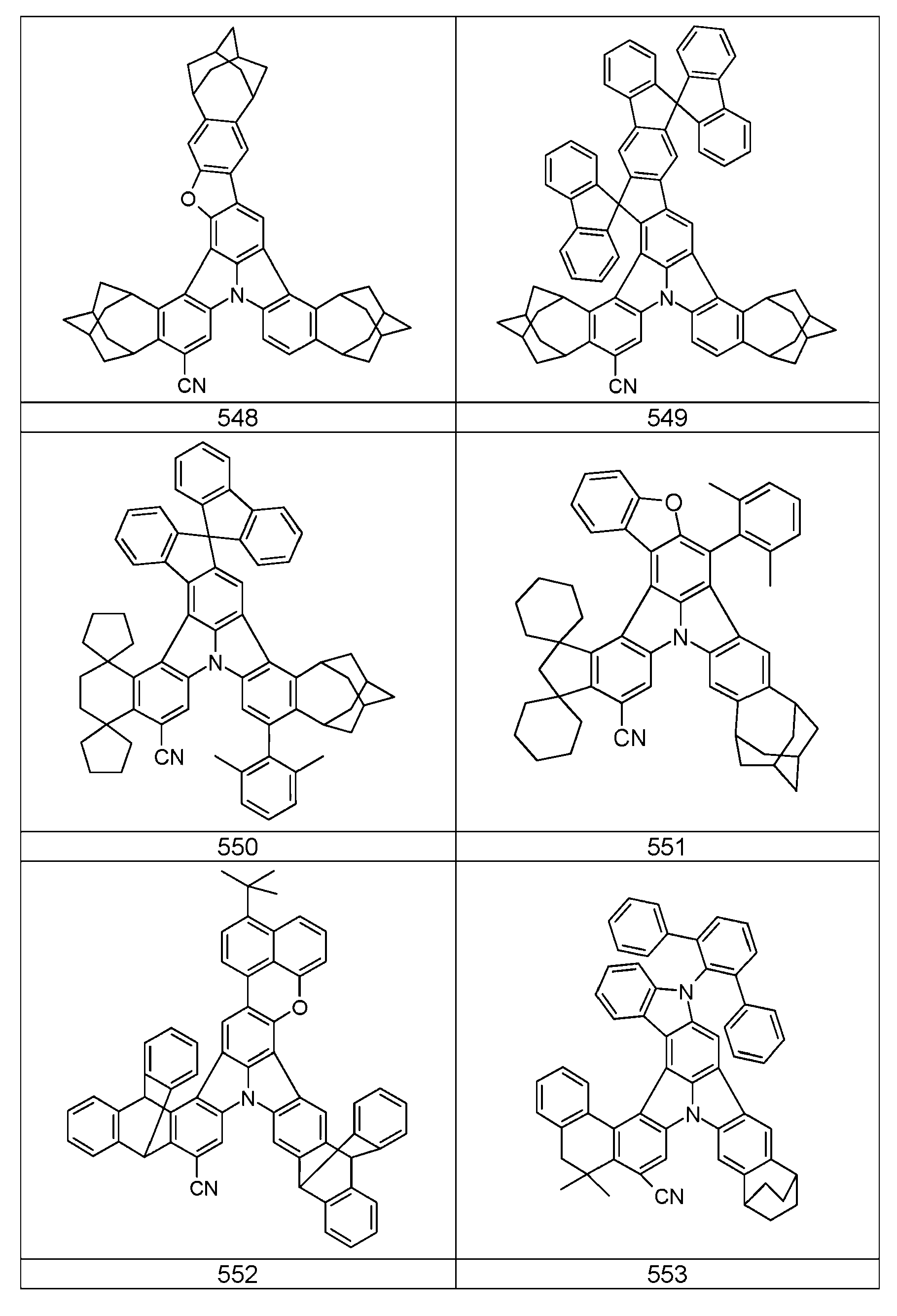

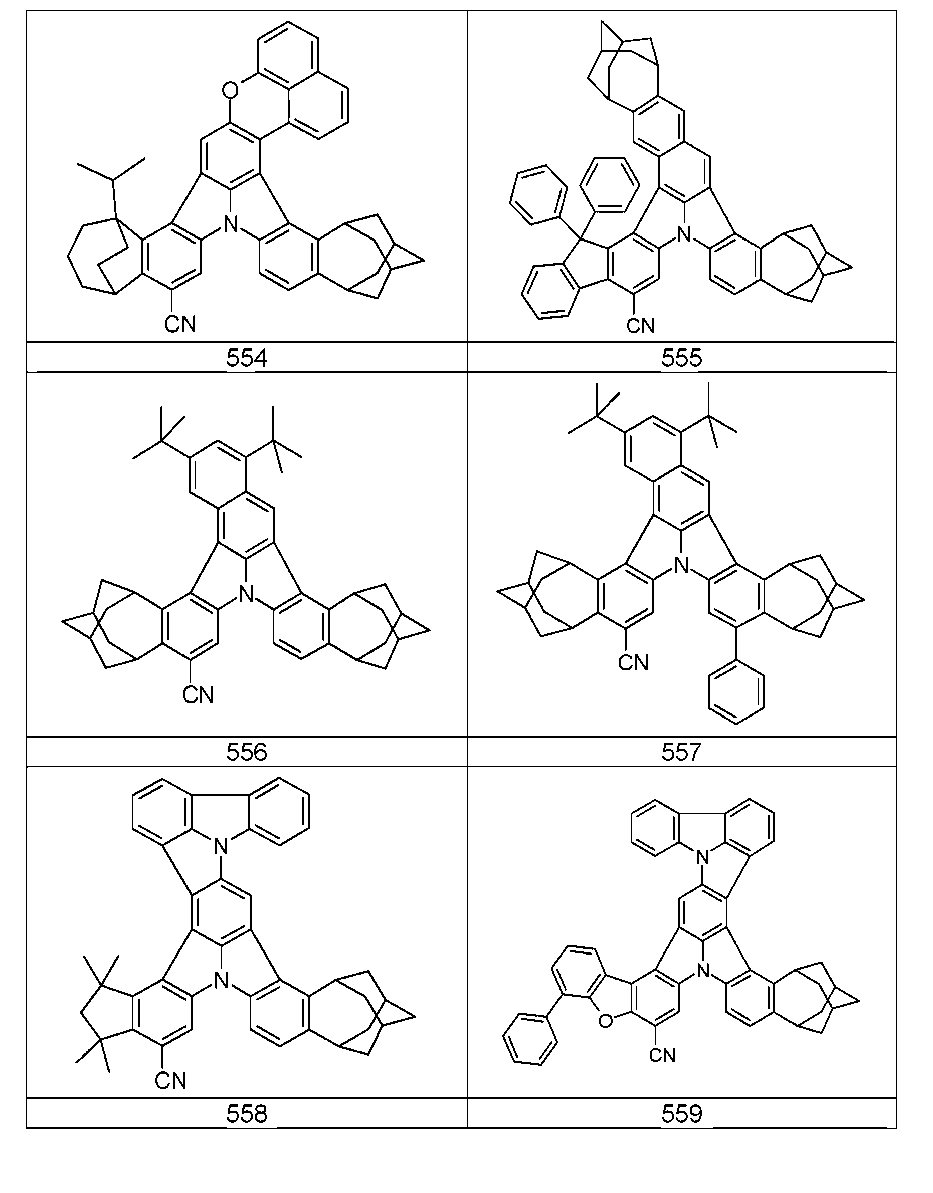

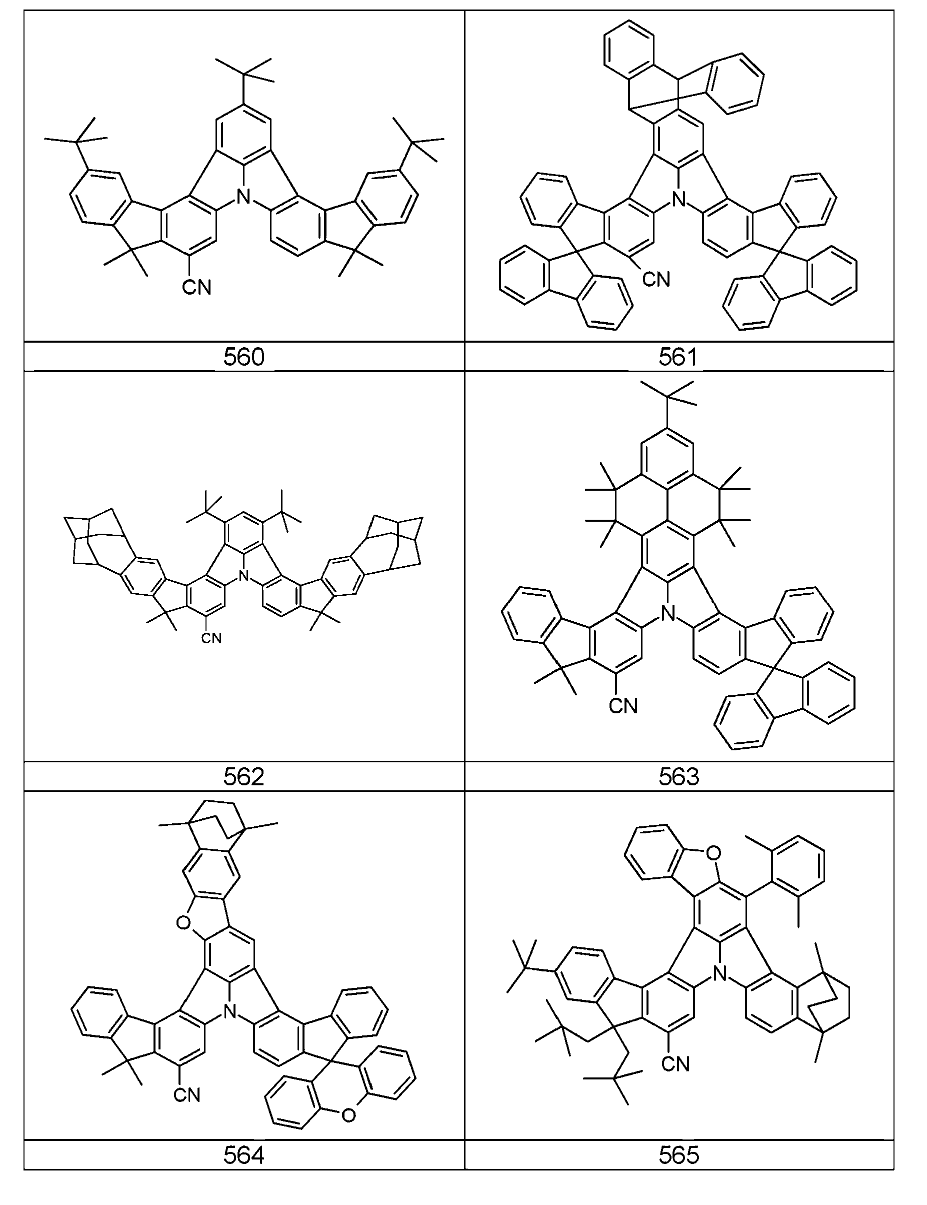

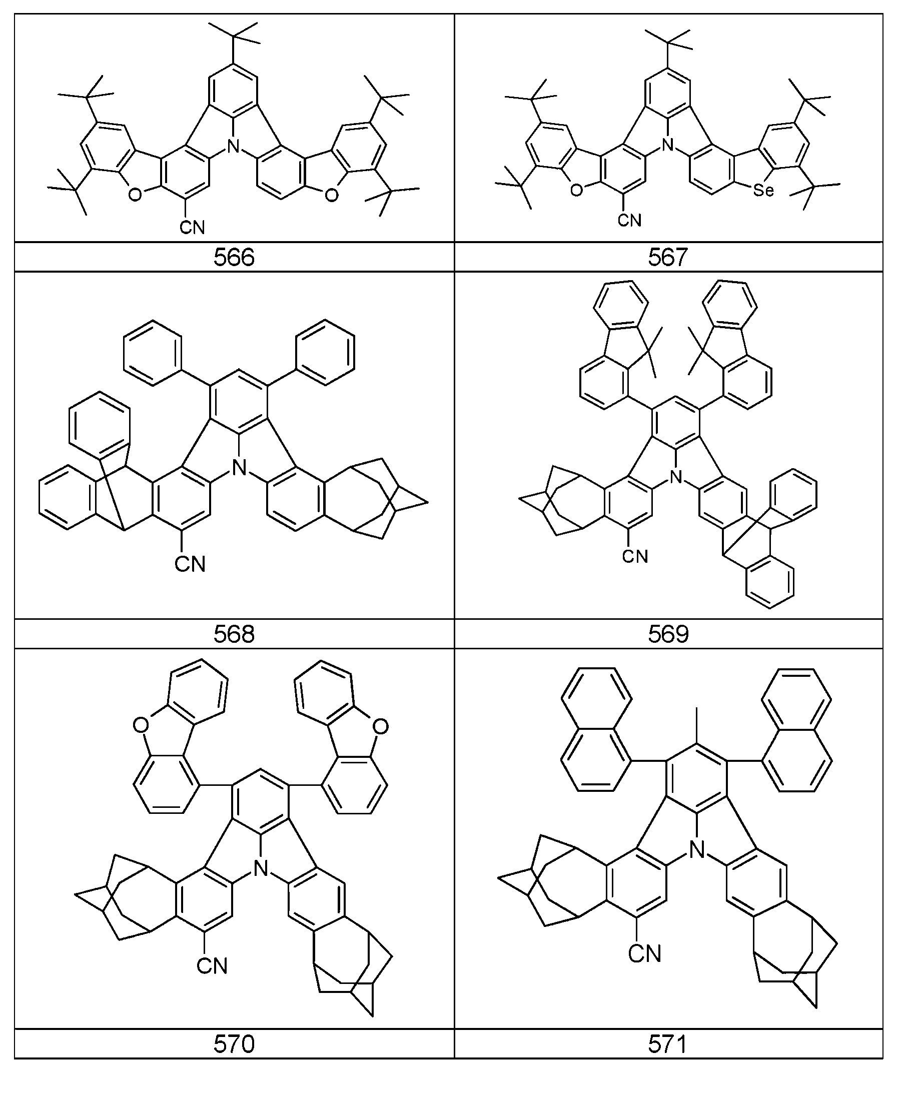

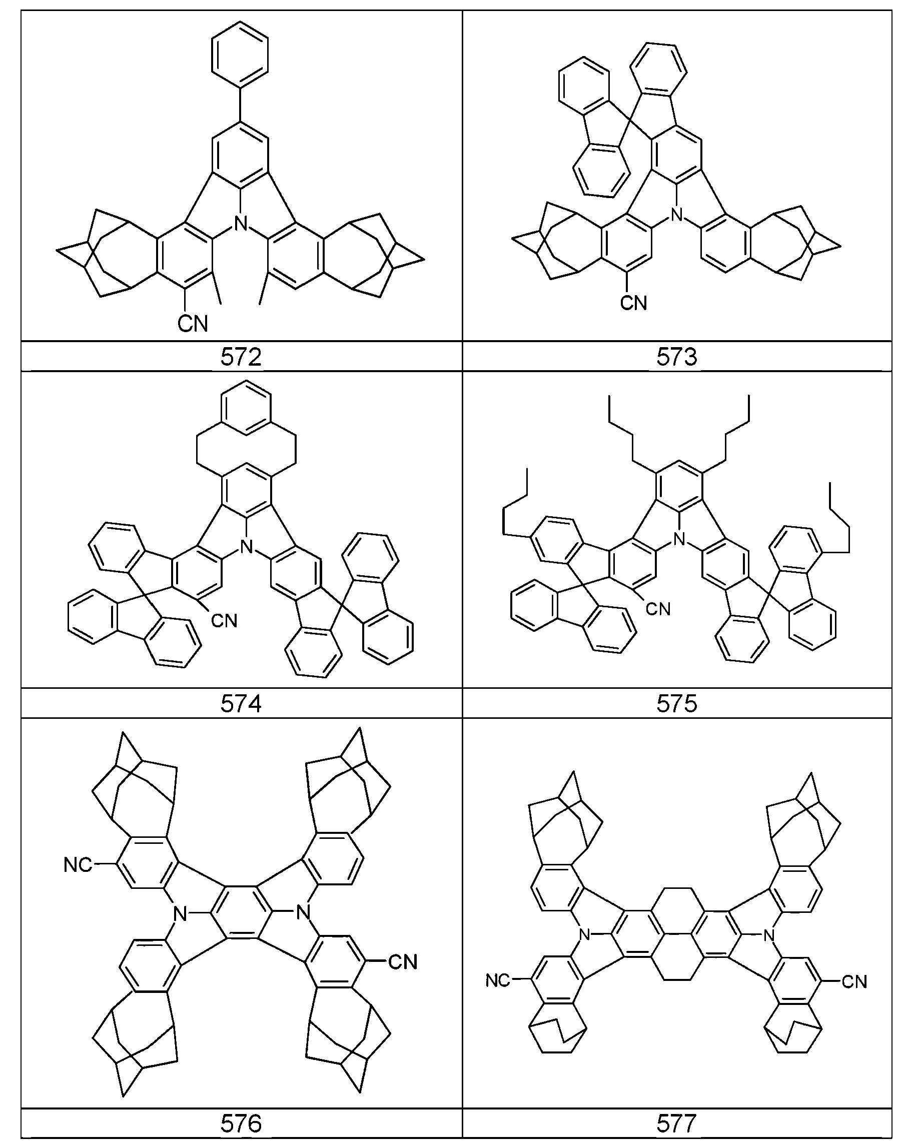

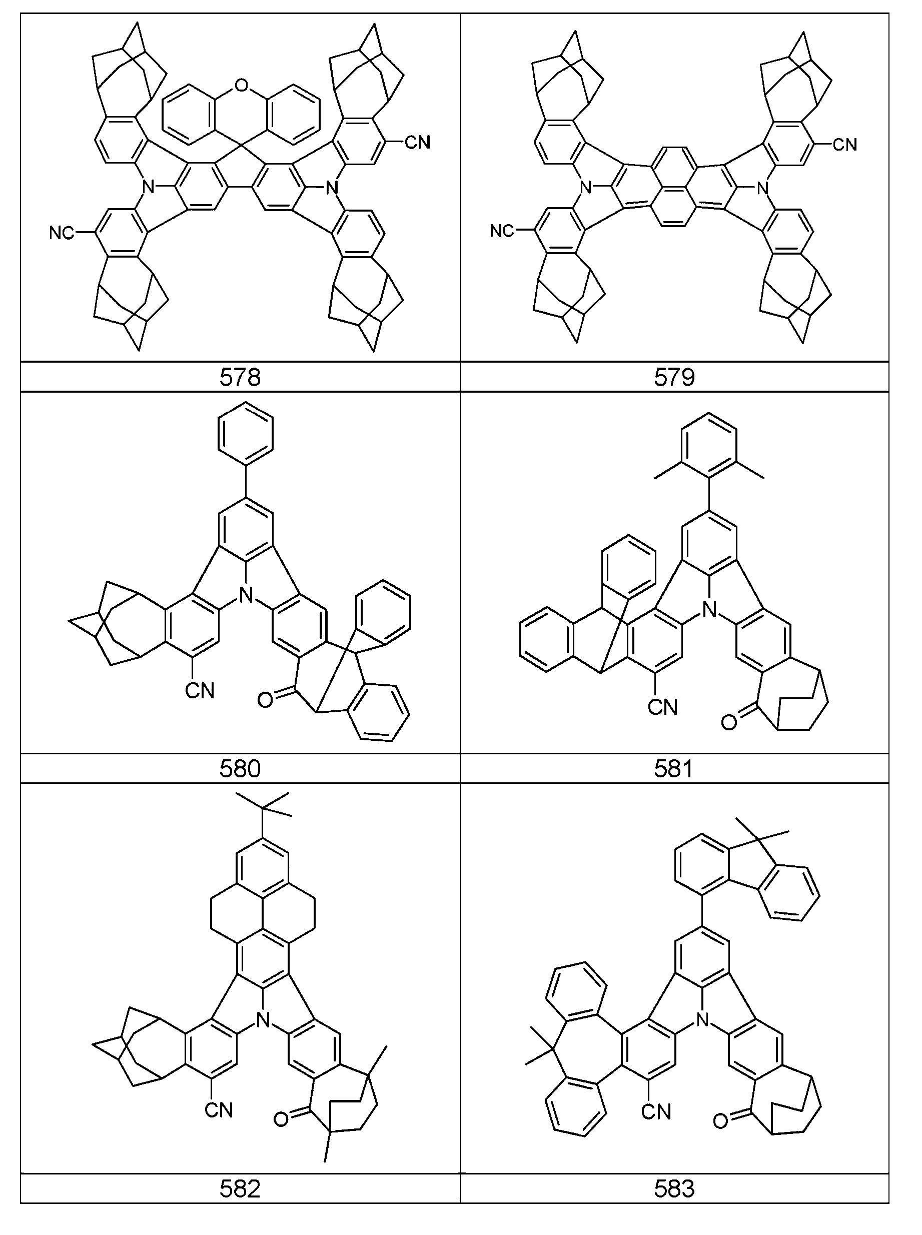

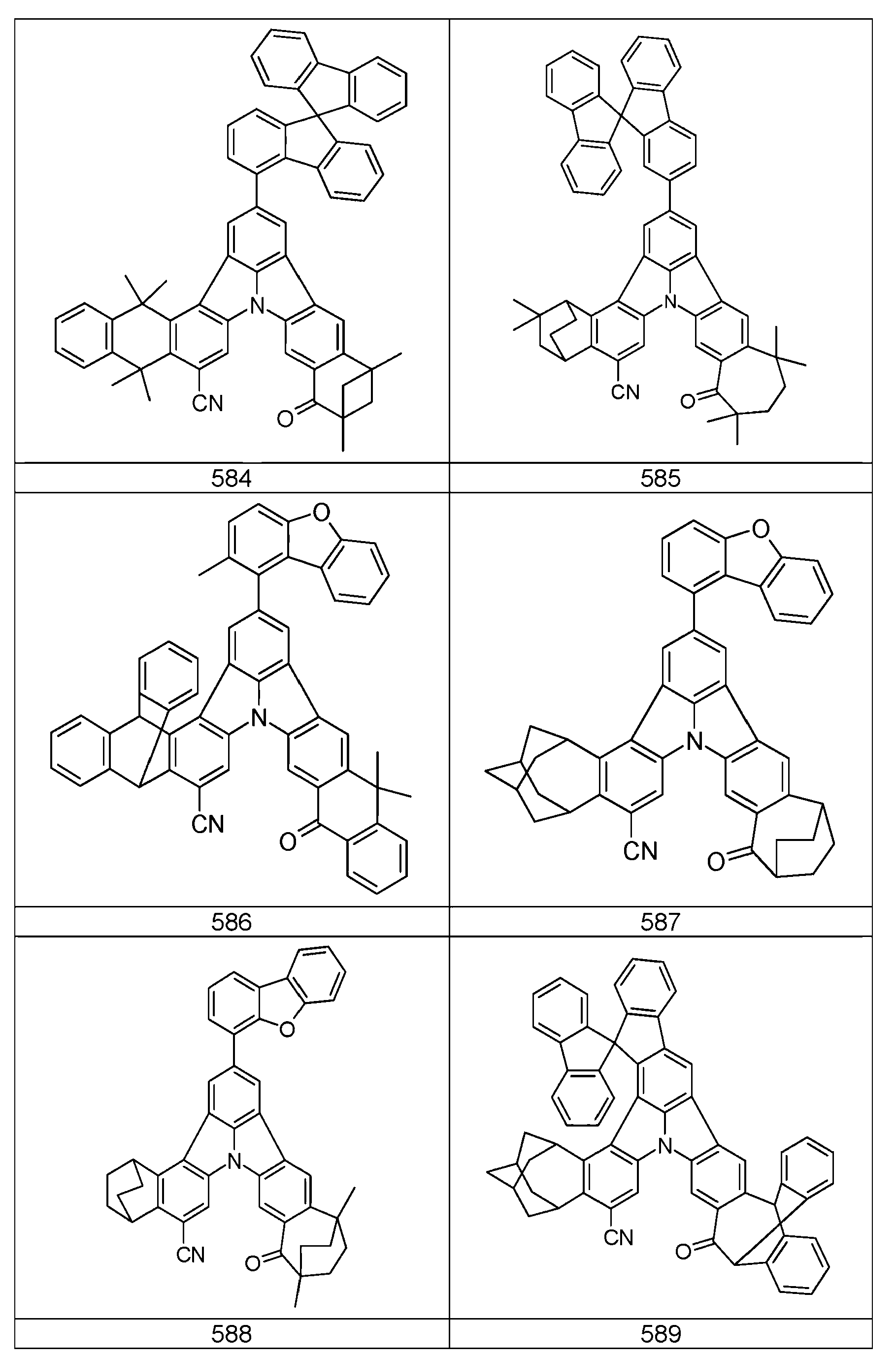

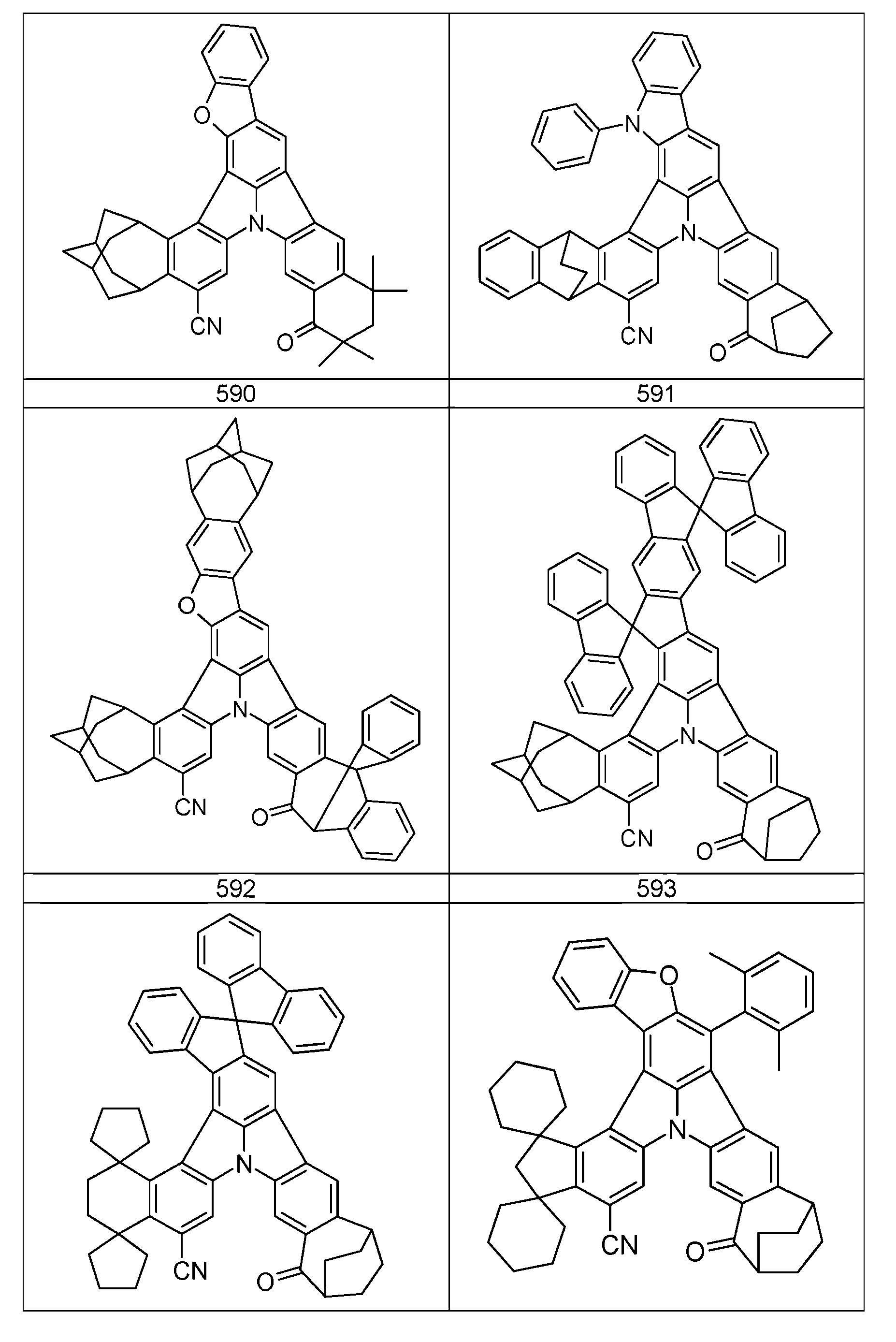

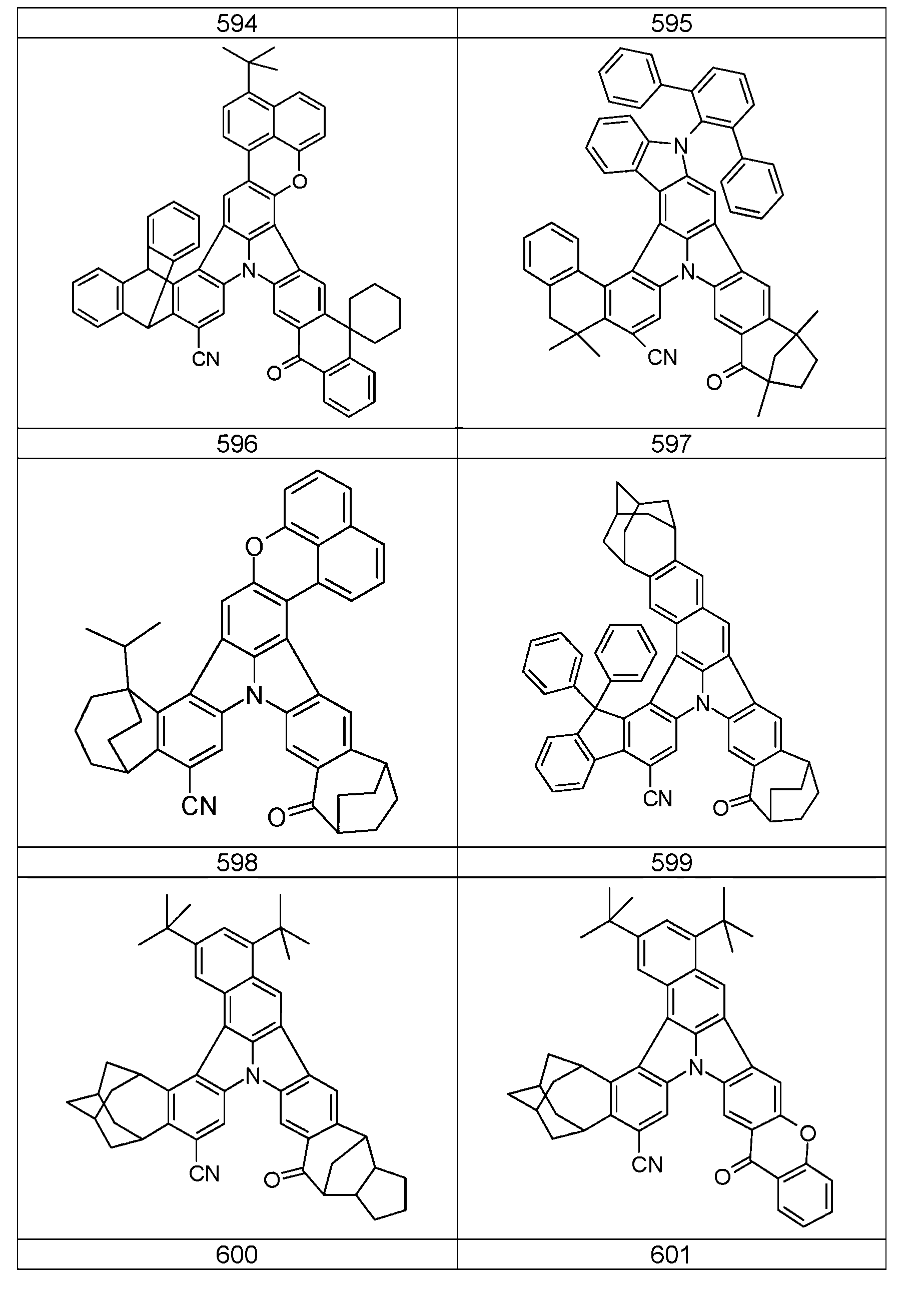

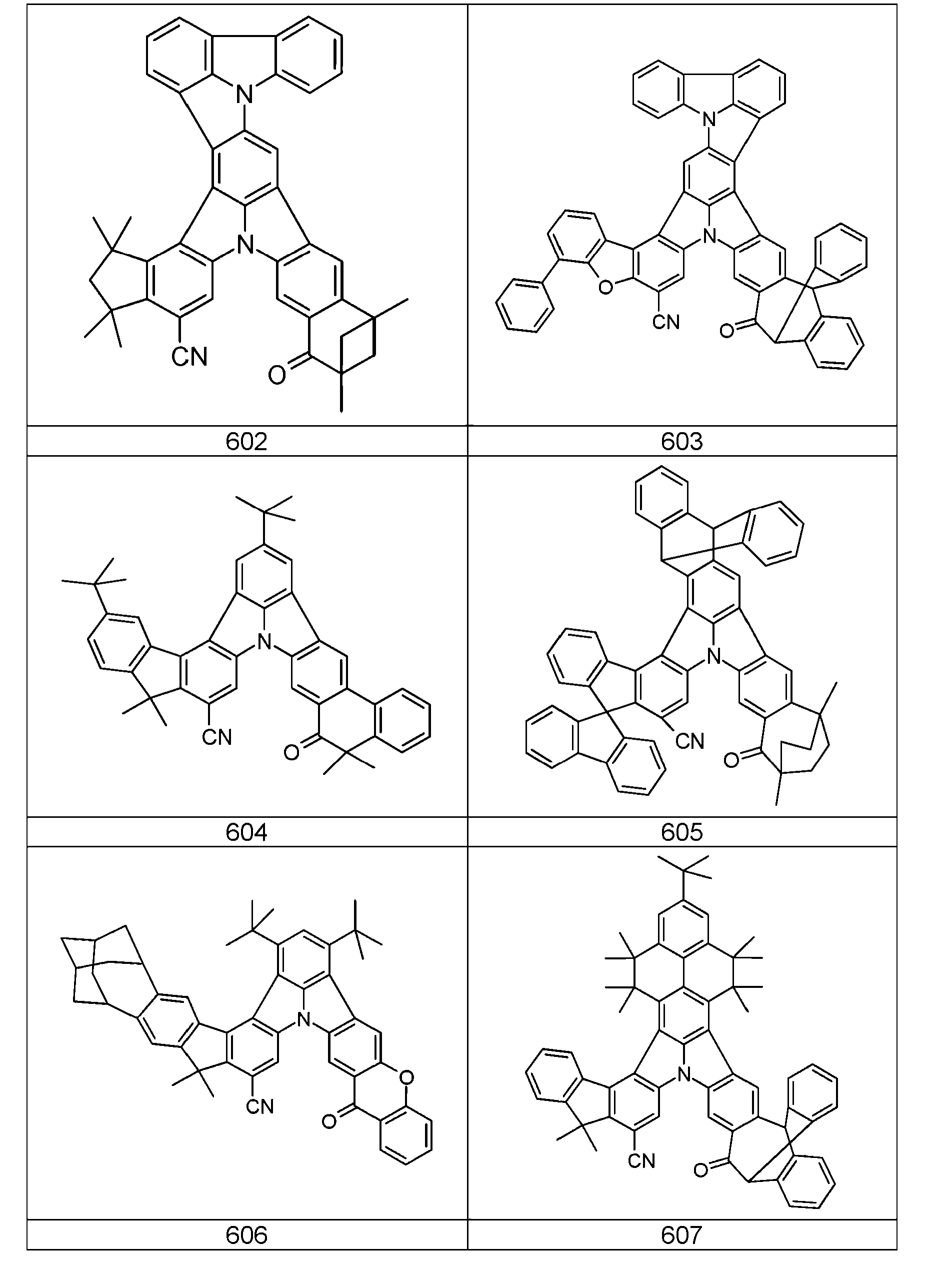

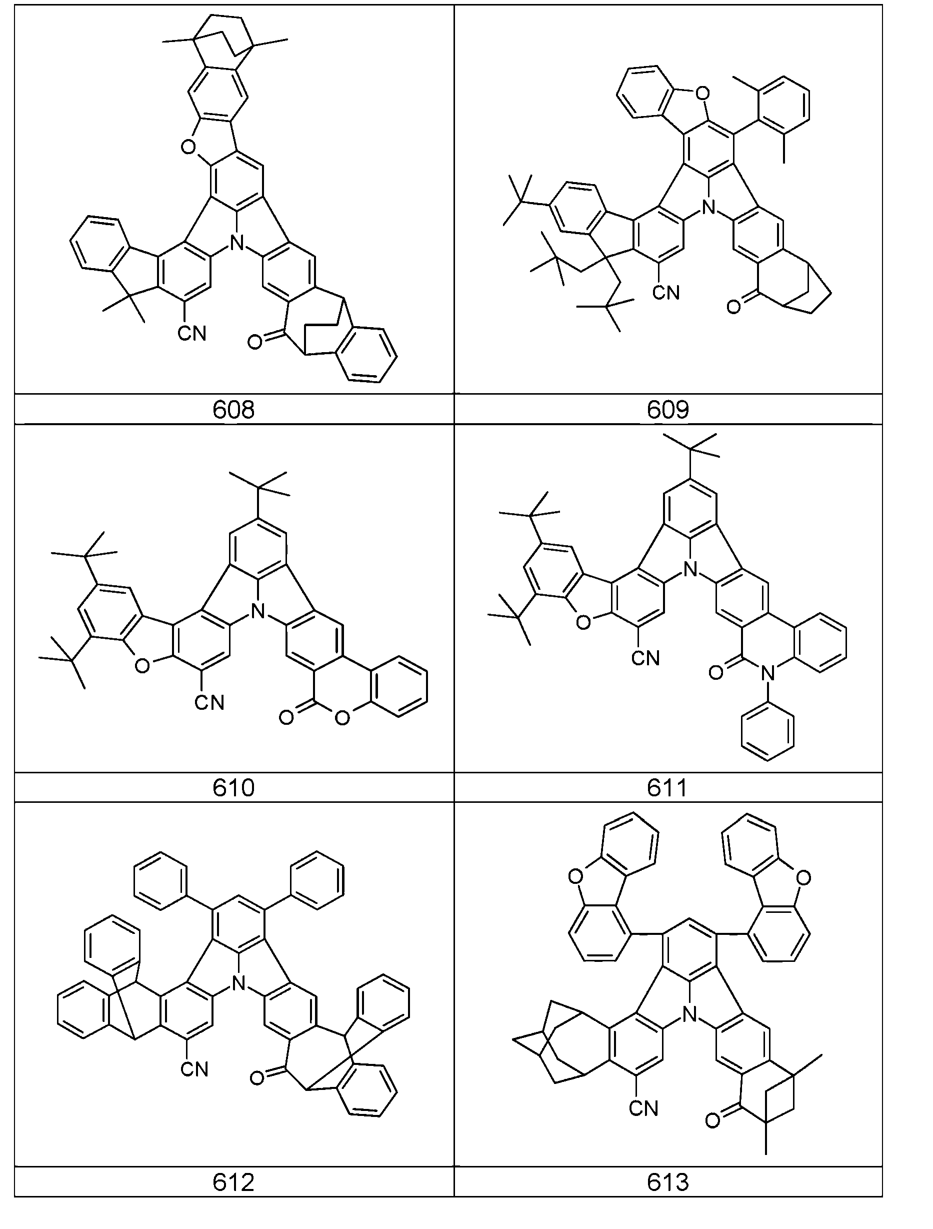

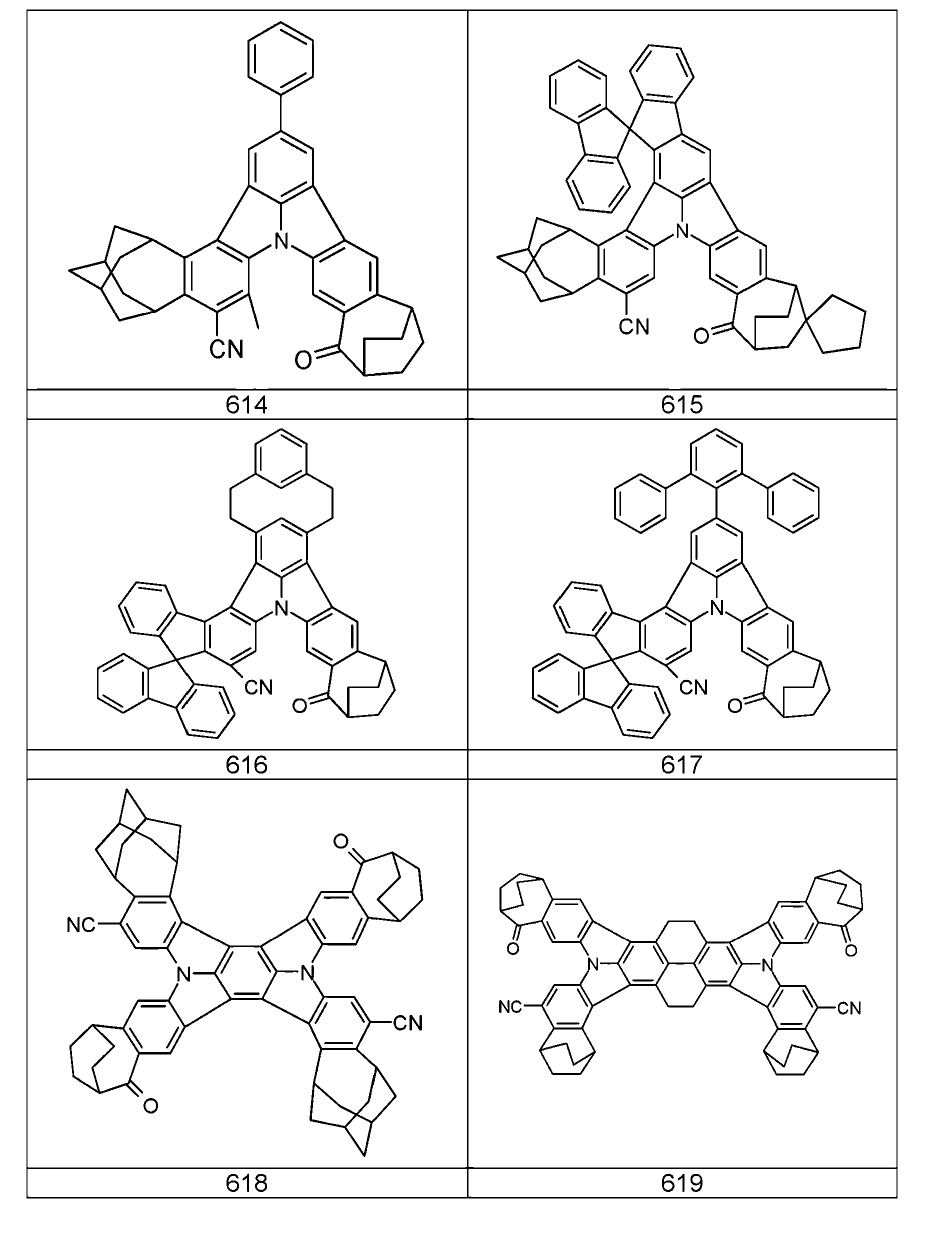

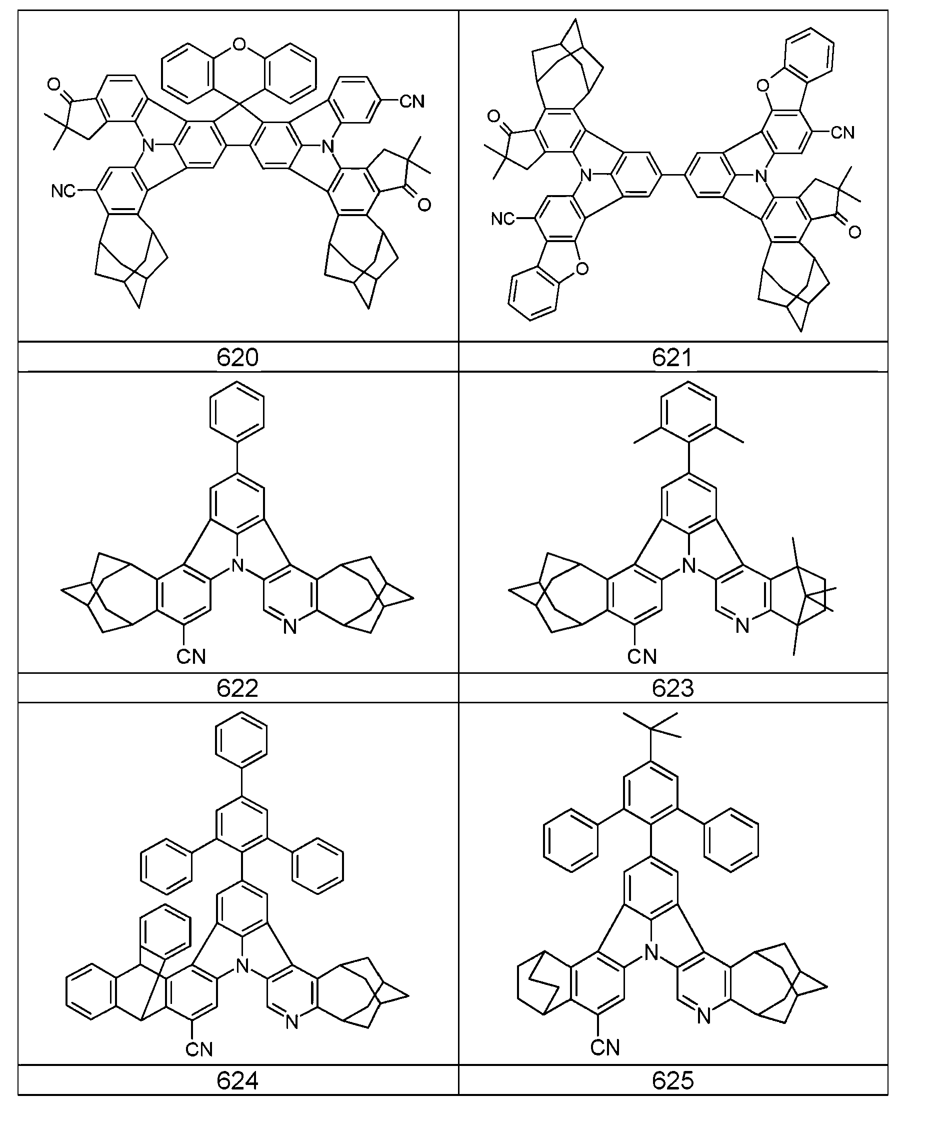

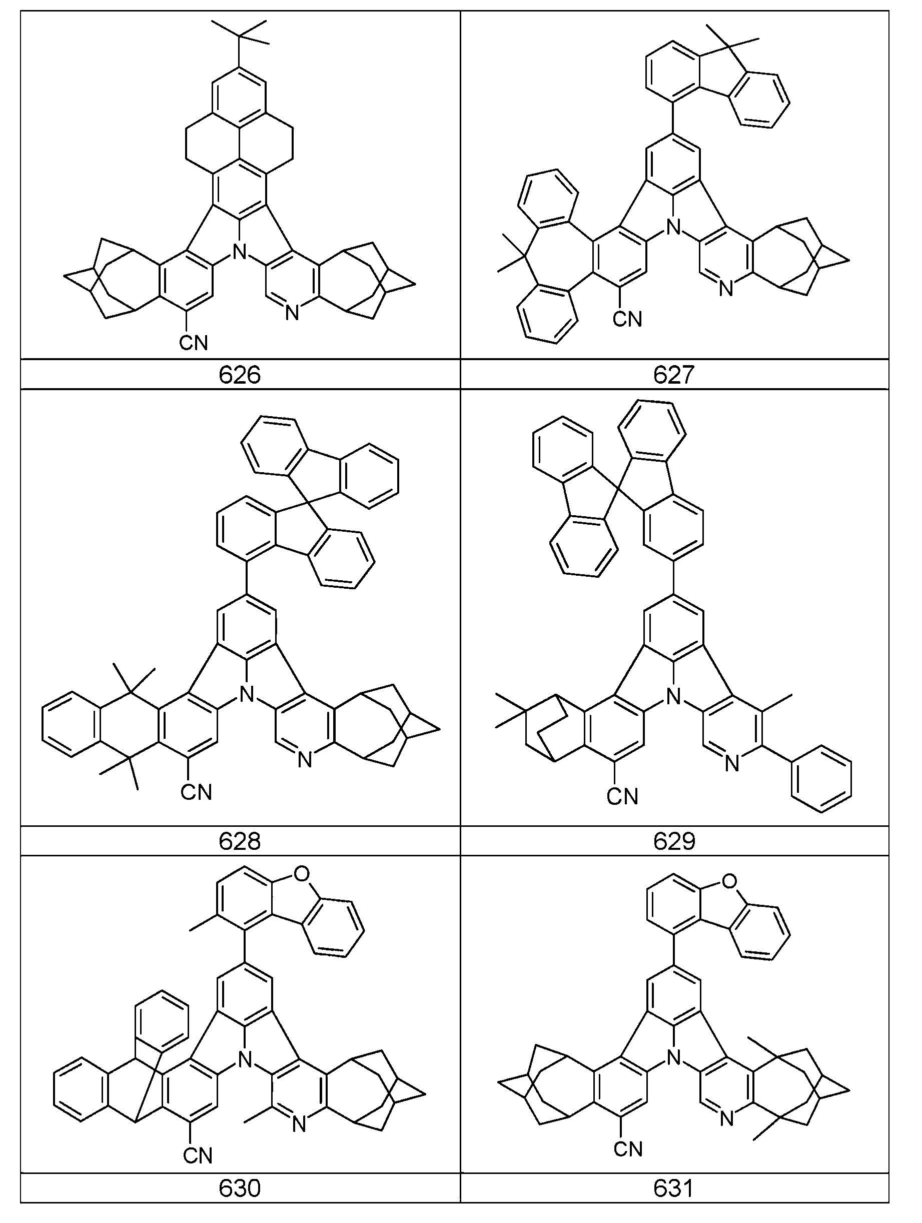

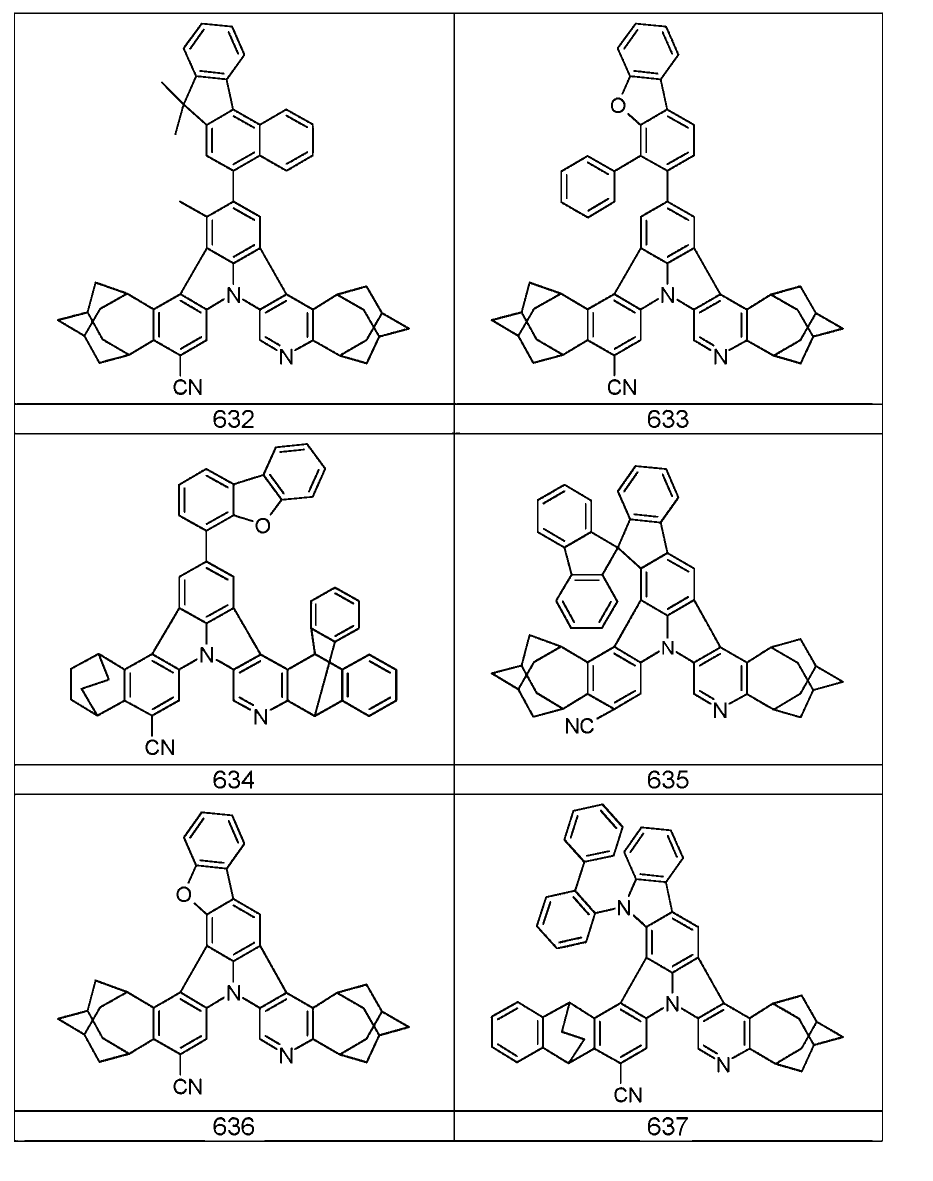

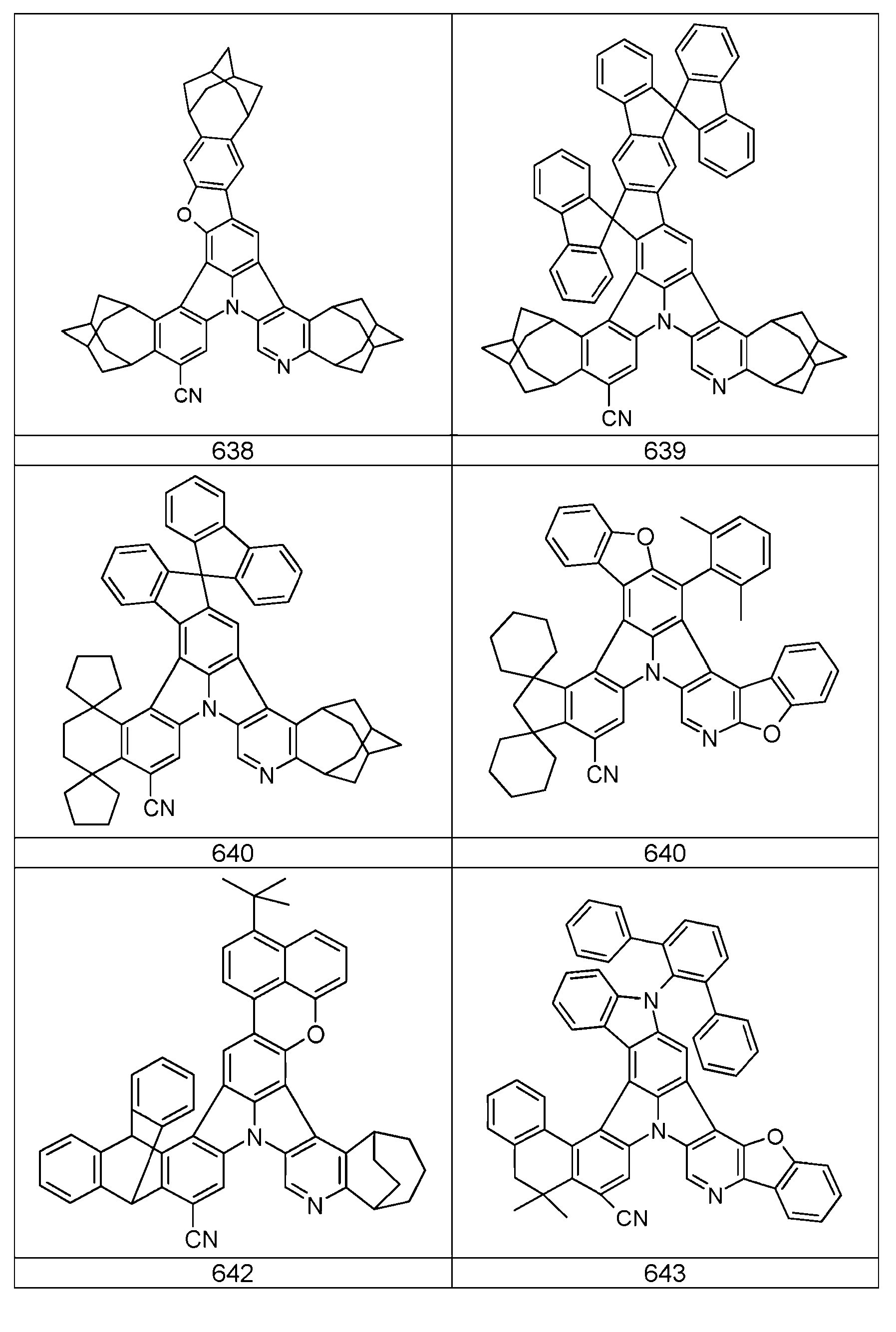

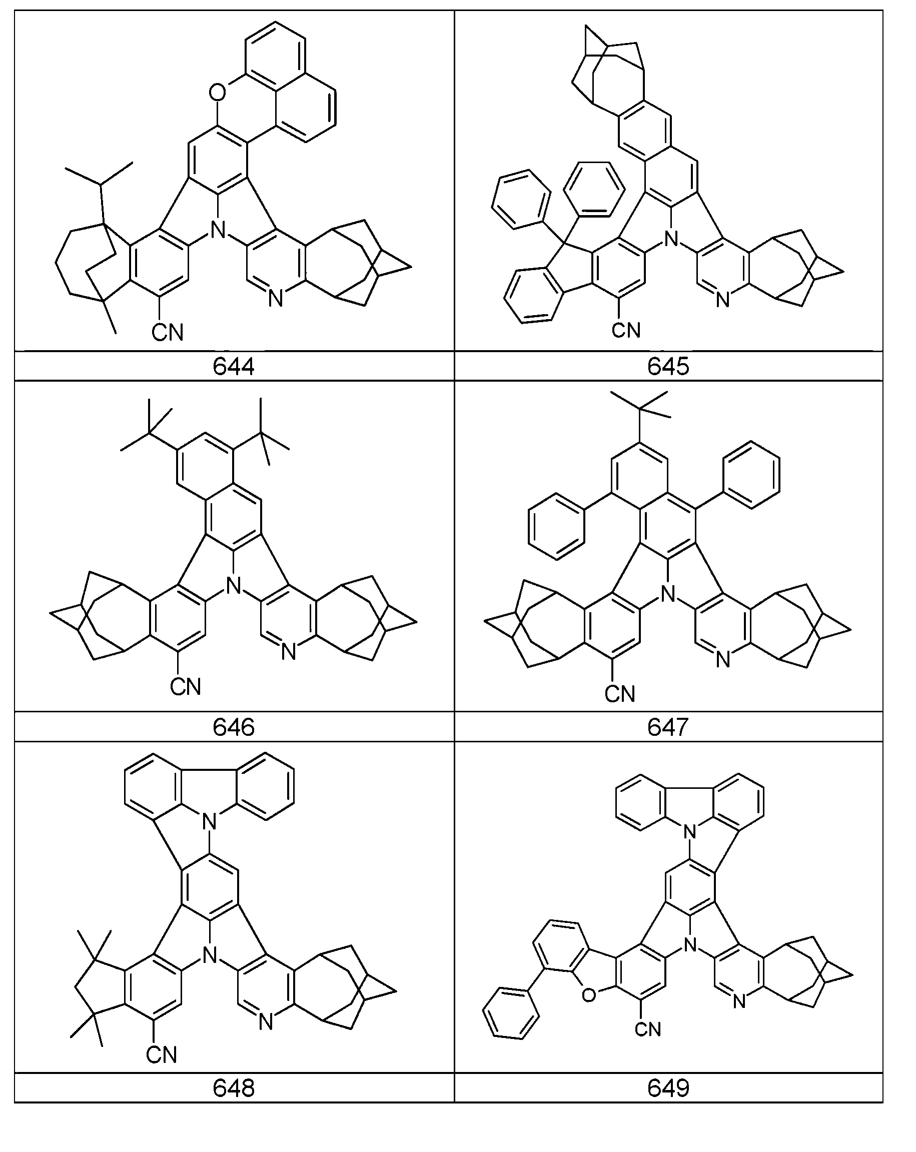

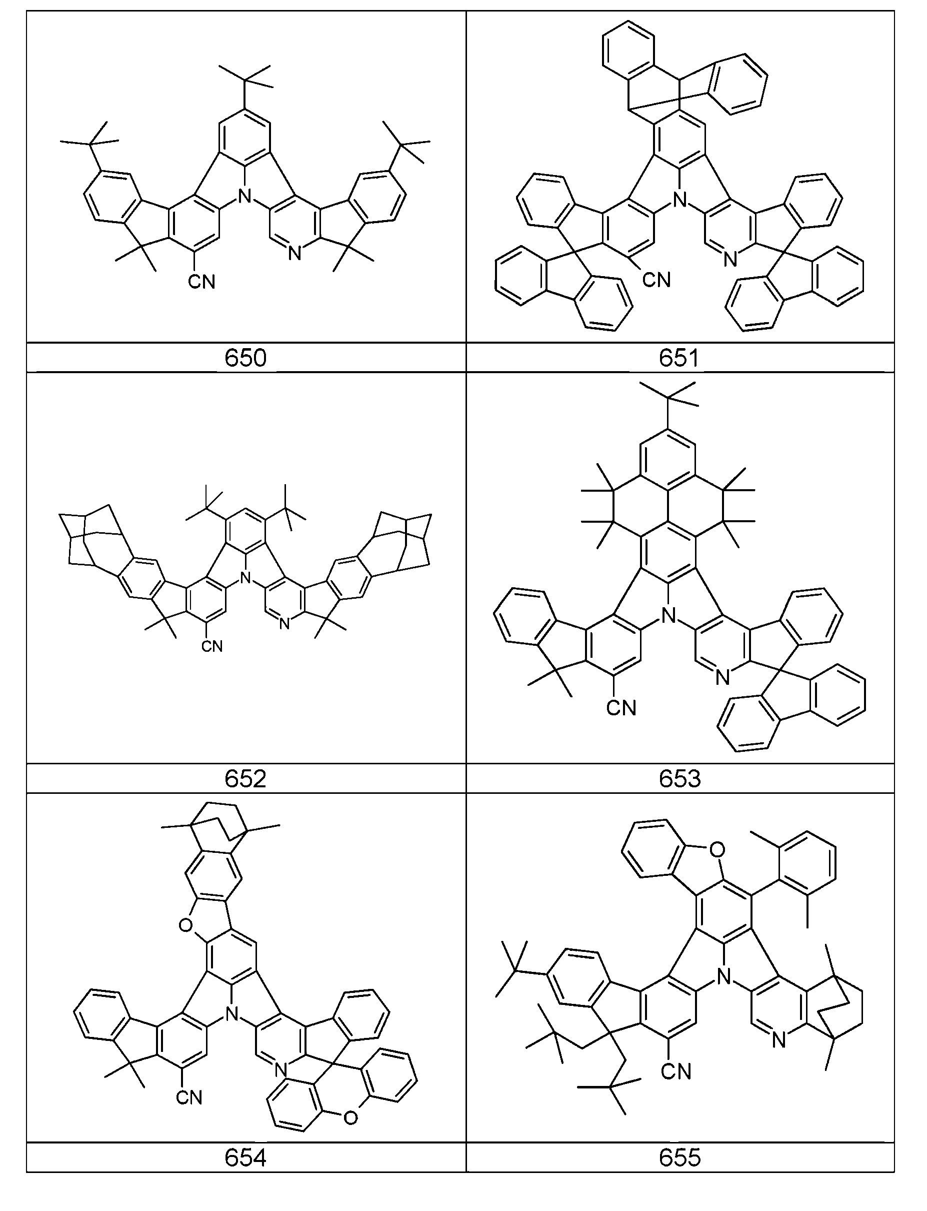

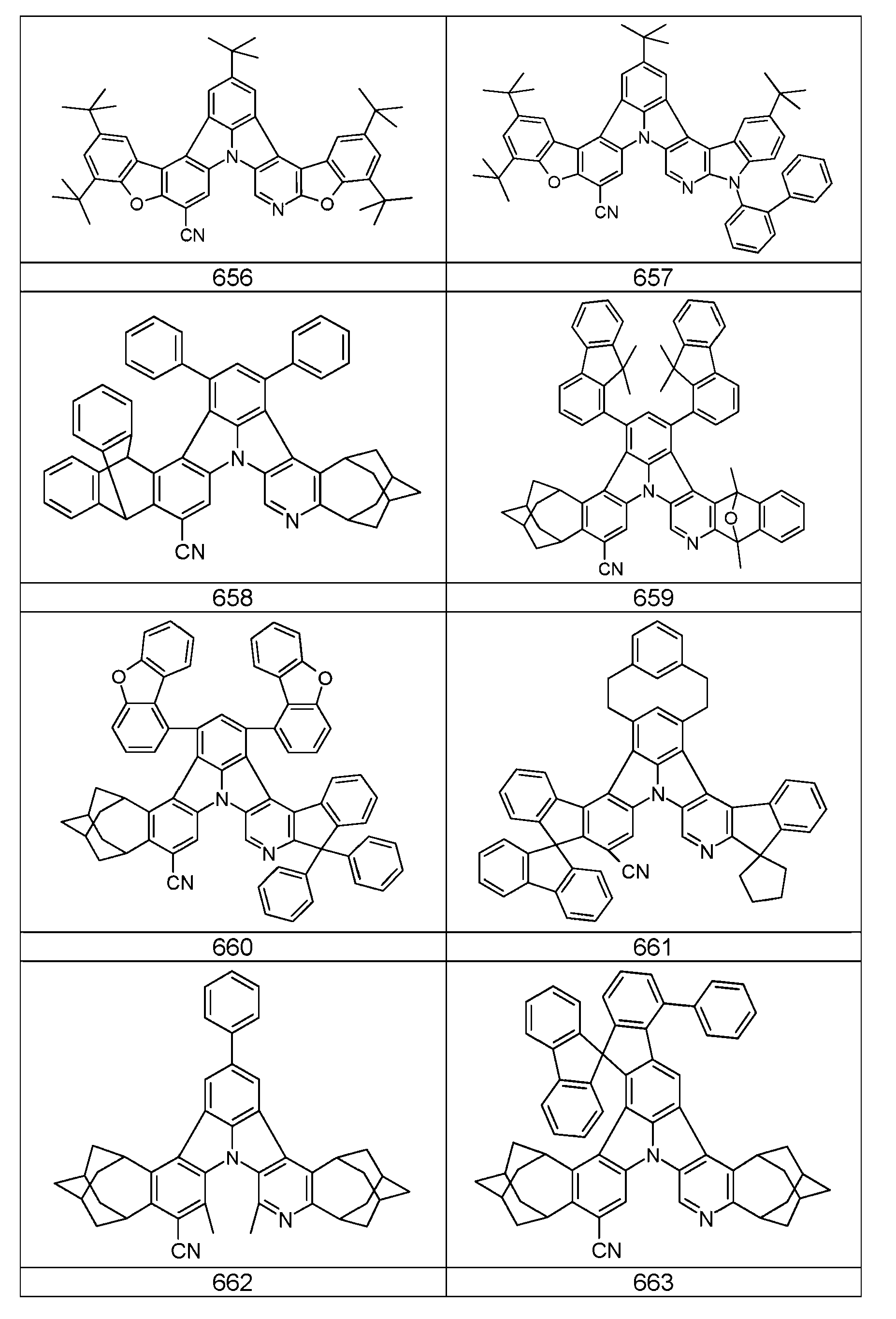

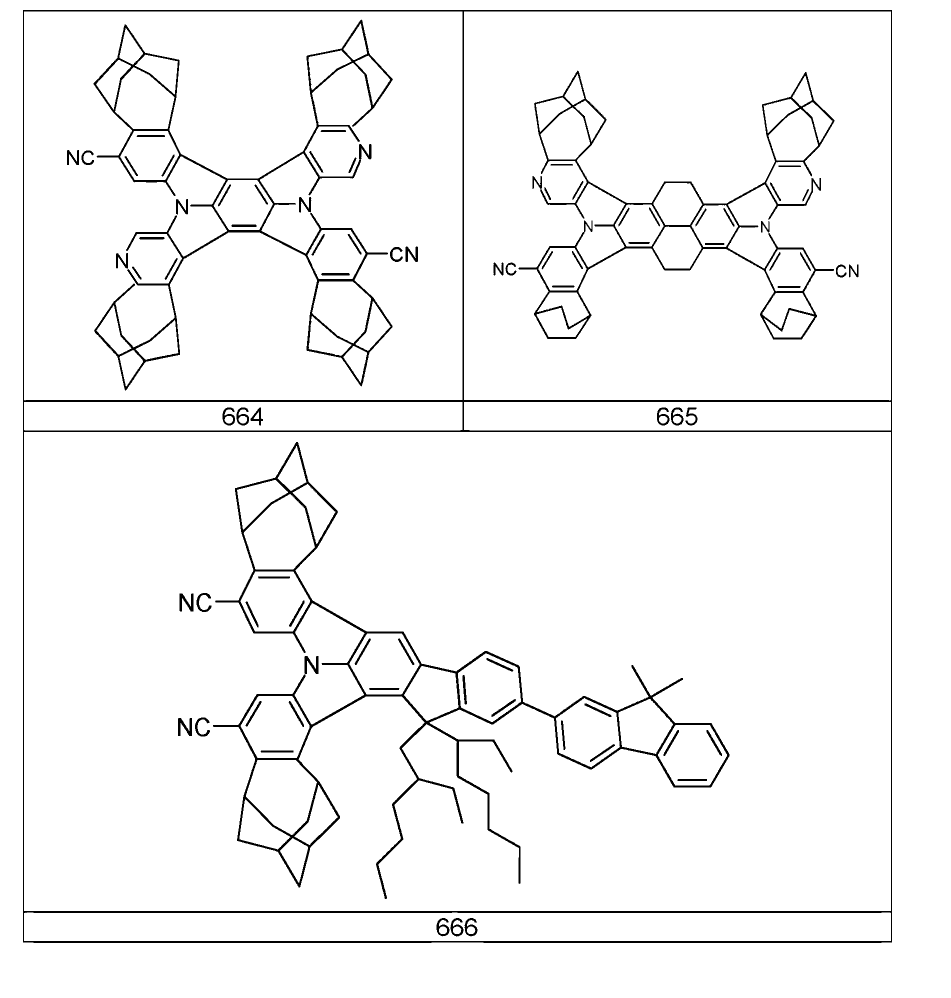

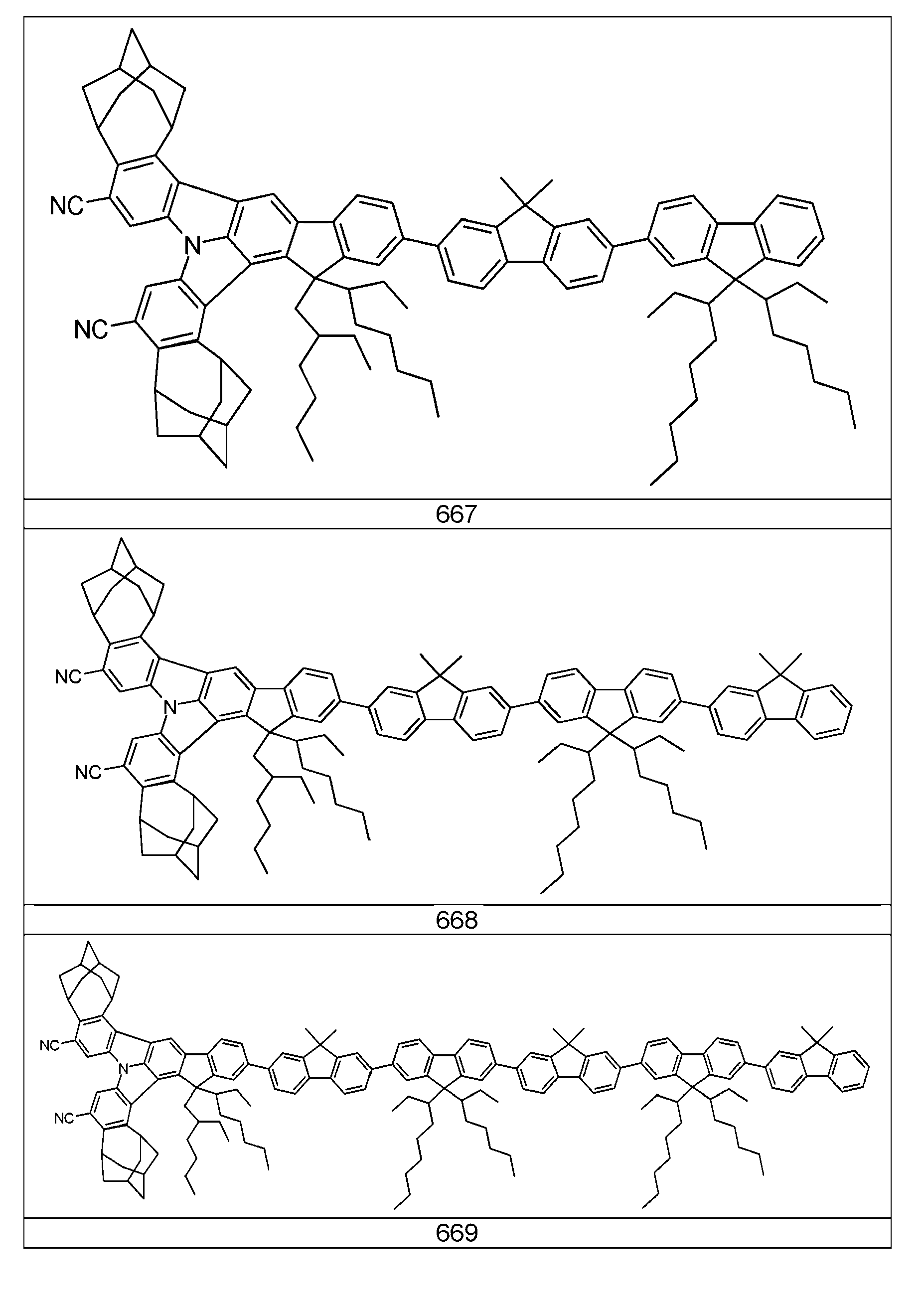

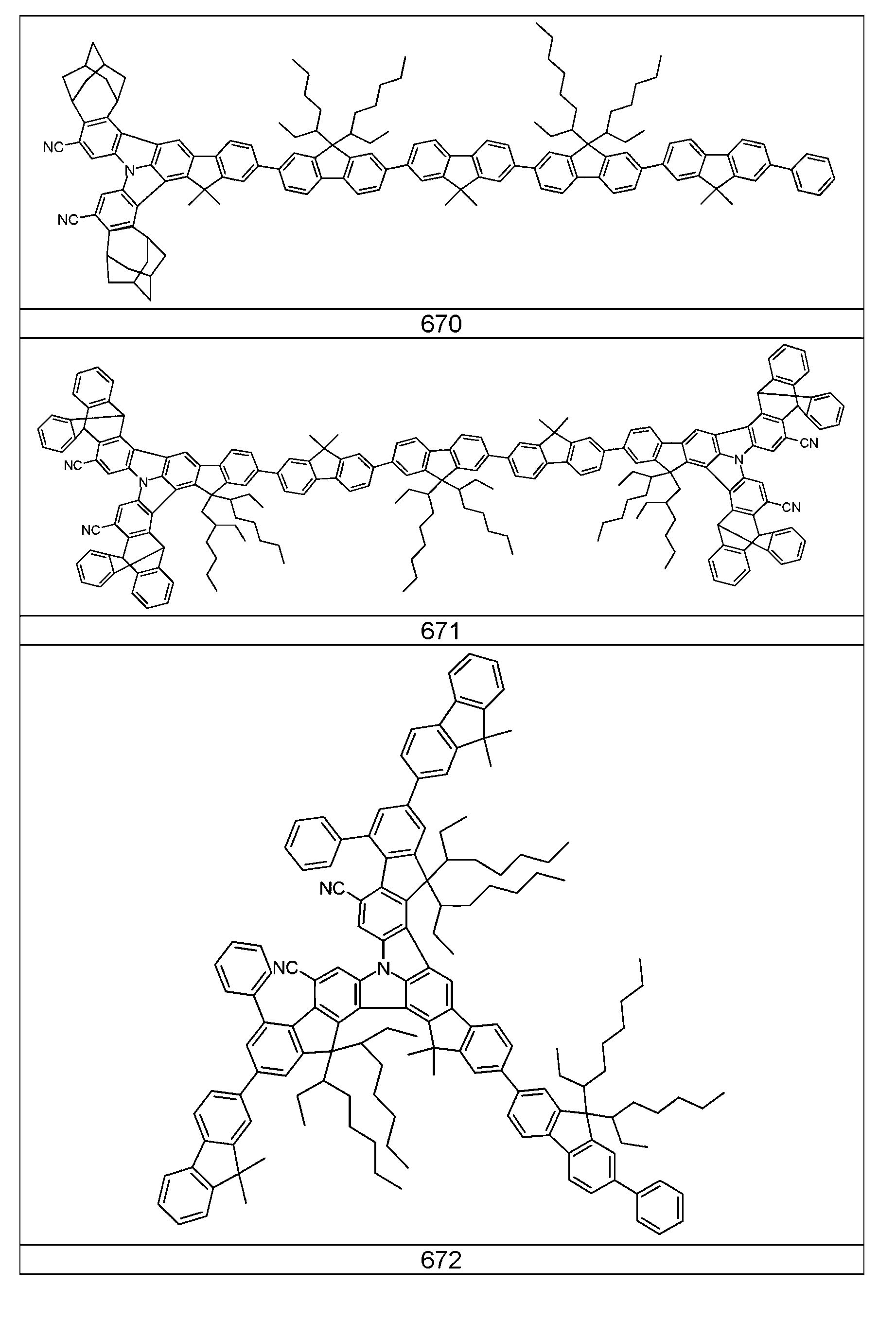

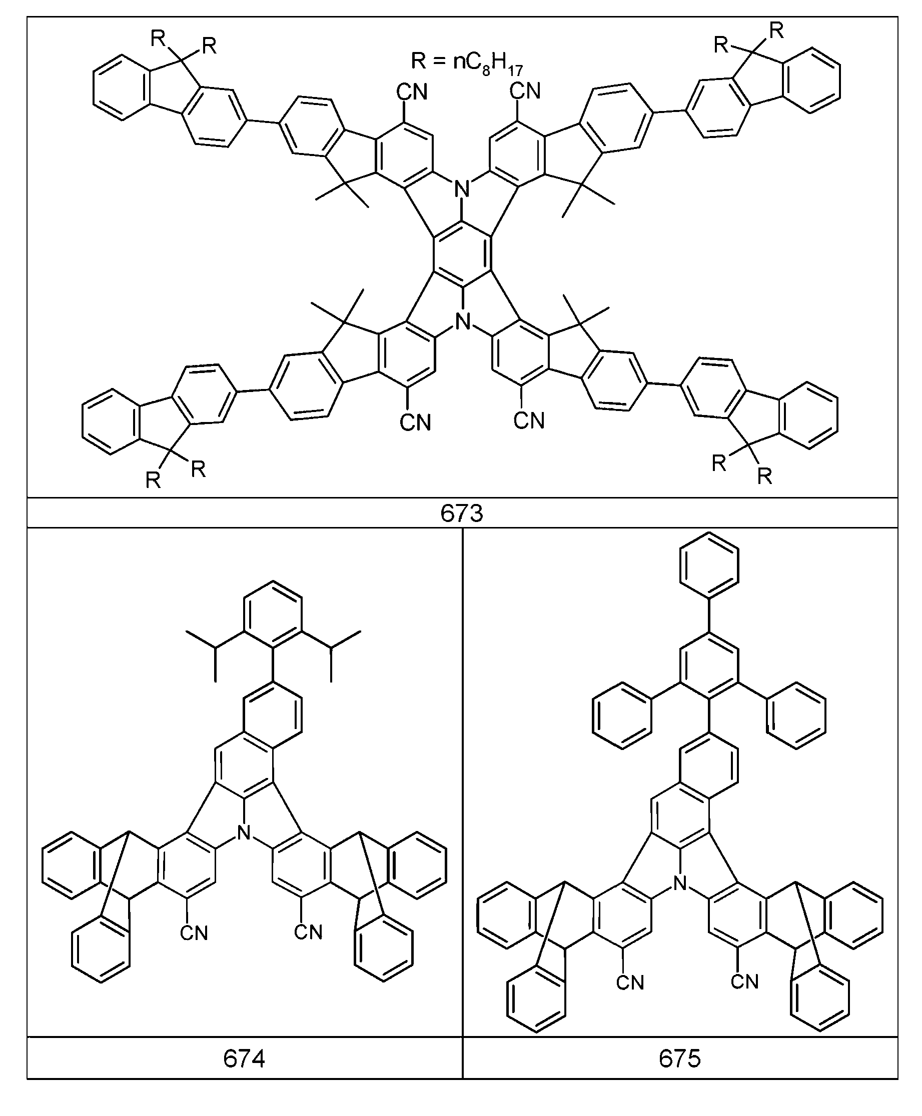

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der erfindungsgemäßen Verbindungen, bei dem ein Grundgerüst mit einer aromatischen Aminogruppe synthetisiert wird und mindestens ein aromatischer oder heteroaromatischer Rest eingeführt wird, vorzugsweise mittels einer nukleophilen aromatischen Substitutionsreaktion oder einer Kupplungsreaktion.

Geeignete Verbindungen, umfassend ein Grundgerüst mit einer aromatischen Aminogruppe können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Verbindungen umgesetzt werden, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen in hoher Reinheit, bevorzugt mehr als 99 % (bestimmt mittels ¹H-NMR und/oder HPLC) erhalten.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, lod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formel (I) und bevorzugten Ausführungsformen dieser Formel oder erfindungsgemäße Verbindungen, wobei ein oder mehrere Bindungen der erfindungsgemäßen Verbindungen oder der Strukturen der Formel (I) und bevorzugten Ausführungsformen dieser Formel zum Polymer, Oligomer oder Dendrimer vorhanden sind. Je nach Verknüpfung der Strukturen der Formel (I) und bevorzugten Ausführungsformen dieser Formel bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (I) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen bevorzugt, umfassend Strukturen gemäß den Formel (I) bzw. die zuvor und nachfolgend ausgeführten bevorzugten Ausführungsformen bevorzugt, die eine Glasübergangstemperatur von mindestens 70 °C, besonders bevorzugt von mindestens 110 °C, ganz besonders bevorzugt von mindestens 125 °C und insbesondere bevorzugt von mindestens 150 °C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung bzw. eine Zusammensetzung, enthaltend mindestens eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Falls die weitere Verbindung ein Lösungsmittel umfasst, so wird diese Mischung hierin als Formulierung bezeichnet. Die weitere Verbindung kann aber auch mindestens eine weitere organische oder anorganische Verbindung sein, die ebenfalls in der elektronischen Vorrichtung eingesetzt wird, beispielsweise ein Emitter und/oder ein Matrixmaterial, wobei sich diese Verbindungen von den erfindungsgemäßen Verbindungen unterscheiden. Geeignete Emitter und Matrixmaterialien sind hinten im Zusammenhang mit der organischen Elektrolumineszenzvorrichtung aufgeführt. Die weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Emittern, die TADF (thermally activated delayed fluorescence) zeigen, HostMaterialien, Elektronentransportmaterialien, Elektroneninjektionsmaterialien, Lochleitermaterialien, Lochinjektionsmaterialien, Elektronenblockiermaterialien, Lochblockiermaterialien, Wide-Band-Gap-Materialien und n-Dotanden, vorzugsweise Hostmaterialien.

Ein weiterer Gegenstand der vorliegenden Erfindung ist die Verwendung einer erfindungsgemäßen Verbindung in einer elektronischen Vorrichtung, insbesondere in einer organischen Elektrolumineszenzvorrichtung, vorzugsweise als Emitter, besonders bevorzugt als grüner, roter oder blauer Emitter, speziell bevorzugt als blauer Emitter. Hierbei zeigen erfindungsgemäße Verbindungen bevorzugt fluorezierende Eigenschaften und stellen somit bevorzugt fluoreszierenden Emitter bereit.

Ein nochmals weiterer Gegenstand der vorliegenden Erfindung ist eine elektronische Vorrichtung enthaltend mindestens eine erfindungsgemäße Verbindung. Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus Besonders bevorzugt ist elektronische Vorrichtung ausgewählt aus der Gruppe bestehend aus organischen Elektrolumineszenzvorrichtungen (OLEDs, sOLED, PLEDs, LECs, etc.), vorzugsweise organische lichtemittierenden Dioden (OLEDs), organische lichtemittierenden Dioden auf Basis von kleinen Molekülen (sOLEDs), organische lichtemittierenden Dioden auf Basis von Polymeren (PLEDs), lichtemittierenden elektrochemischen Zellen (LECs), organischen Laserdioden (O-Laser), "organic plasmon emitting devices" (D. M. Koller et al., Nature Photonics 2008, 1-4); organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs) und organischen elektrischen Sensoren, bevorzugt organischen Elektrolumineszenzvorrichtungen (OLEDs, sOLED, PLEDs, LECs, etc.), besonders bevorzugt organische lichtemittierenden Dioden (OLEDs), organische lichtemittierenden Dioden auf Basis von kleiner Moleküle (sOLEDs), organische lichtemittierenden Dioden auf Basis von Polymeren (PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem- Elektrolumineszenzvorrichtung handeln, insbesondere für weiß emittierende OLEDs.

Die erfindungsgemäße Verbindung kann dabei in unterschiedlichen Schichten eingesetzt werden, je nach genauer Struktur. Bevorzugt ist eine organische Elektrolumineszenzvorrichtung, enthaltend eine Verbindung gemäß Formel (I) bzw. die oben ausgeführten bevorzugten Ausführungsformen in einer emittierenden Schicht als Emitter, vorzugsweise roter, grüner oder blauer Emitter, besonders bevorzugt als blauer Emitter.

Wenn die erfindungsgemäße Verbindung als Emitter in einer emittierenden Schicht eingesetzt wird, wird bevorzugt ein geeignetes Matrixmaterial eingesetzt, welches als solches bekannt ist.

Eine bevorzugte Mischung aus der erfindungsgemäßen Verbindung und einem Matrixmaterial enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% an Matrixmaterial bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, Dibenzofuranderivate, z. B. gemäß WO 2015/169412, WO 2016/015810, WO 2016/023608, WO 2017/148564 oder WO 2017/148565 oder Biscarbazole, z. B. gemäß JP 3139321 B2.

Weiterhin kann als Co-Host eine Verbindung verwendet werden, die nicht oder nicht in wesentlichem Umfang am Ladungstransport teilnimmt, wie beispielsweise in WO 2010/108579 beschrieben. Insbesondere eignen sich in Kombination mit der erfindungsgemäßen Verbindung als Co-Matrix-Material Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

In einer bevorzugten Ausgestaltung wird eine erfindungsgemäße Verbindung, die als Emitter verwendet wird, vorzugsweise in Kombination mit einem oder mehreren phosphoreszierenden Materialien (Triplettemitter) und/oder einer Verbindung eingesetzt, die ein TADF-Hostmaterial (thermally activated delayed fluorescence) darstellt. Hierbei wird vorzugsweise ein Hyperfluoreszenz- und/oder Hyperphosphoreszenz-System gebildet.

In WO 2015/091716 A1 und in WO 2016/193243 A1 werden OLEDs offenbart, die in der Emissionsschicht sowohl eine phosphoreszierende Verbindung als auch einen fluoreszierenden Emitter enthalten, wobei die Energie von der phosphoreszierenden Verbindung auf den fluoreszierenden Emitter übertragen wird (Hyperphosphoreszenz). Die phosphoreszierende Verbindung verhält sich in diesem Zusammenhang demnach wie ein Host-Material. Wie der Fachmann weiß, haben Hostmaterialien höhere Singulett und Triplett-Energien im Vergleich zu dem Emittern, damit die Energie des Host-Materials auch möglichst optimal auf den Emitter übertragen werden. Die im Stand der Technik offenbarten Systeme weisen genau solch eine Energierelation auf.

Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2018/011186, WO 2018/001990, WO 2018/019687, WO 2018/019688, WO 2018/041769, WO 2018/054798, WO 2018/069196, WO 2018/069197, WO 2018/069273, WO 2018/178001, WO 2018/177981, WO 2019/020538, WO 2019/115423, WO 2019/158453 und WO 2019/179909 entnommen werden. Generell eignen sich alle phosphoreszierenden Komplexe, wie sie gemäß dem Stand der Technik für phosphoreszierende Elektrolumineszenzvorrichtungen verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Eine erfindungsgemäße Verbindung kann vorzugsweise in Kombination mit einem TADF-Hostmaterial und/oder einem TADF-Emitter eingesetzt werden, wie dies zuvor dargelegt ist.

Der als thermisch aktivierte verzögerte Fluoreszenz (TADF = "thermally activated delayed fluorescence") bezeichnete Vorgang wird beispielsweise von B. H. Uoyama et al., Nature 2012, Vol. 492, 234 beschrieben. Um diesen Prozess zu ermöglichen, ist im Emitter ein vergleichsweise kleiner Singulett-Triplett-Abstand ΔE(S₁ - T₁) von zum Beispiel weniger als etwa 2000 cm⁻¹ nötig. Um den an sich spin-verbotenen Übergang T₁ → S₁ zu öffnen, kann neben dem Emitter eine weitere Verbindung in der Matrix vorgesehen werden, die eine starke Spin-Bahn-Kopplung aufweist, sodass über die räumliche Nähe und die damit mögliche Wechselwirkung zwischen den Molekülen ein Inter-System-Crossing ermöglicht wird, oder die Spin-Bahn-Kopplung wird über ein im Emitter enthaltenes Metallatom erzeugt.

Weitere wertvolle Informationen zu Hyperfluoreszenz-Systemen sind unter anderem in WO2012/133188 (Idemitsu), WO2015/022974 (Kyushu Univ.), WO2015/098975 (Idemitsu), WO2020/053150 (Merck) und DE202019005189 (Merck) dargelegt.

Weitere wertvolle Informationen zu Hyperphosphoreszenz-Systemen sind unter anderem in WO2015/091716 A1, WO2016/193243 A1 (BASF), WO01/08230 A1 (Princeton Univ. (Mark Thompson)), US2005/0214575A1 (Fuji), WO2012/079673 (Merck), WO2020/053314 (Merck) und WO2020/053315 (Merck) dargelegt.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (I) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Formulierungen zum Auftragen einer Verbindung gemäß Formel (I) oder deren oder deren zuvor dargelegten bevorzugten Ausführungsformen sind neu Ein weiterer Gegenstand der vorliegenden Erfindung ist daher eine Formulierungen, enthaltend mindestens ein Lösungsmittel und eine Verbindung gemäß Formel (I) oder deren zuvor dargelegten bevorzugten Ausführungsformen.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich gegenüber dem Stand der Technik insbesondere durch eine verbesserte Lebensdauer und eine höhere Farbreinheit aus. Dabei bleiben die weiteren elektronischen Eigenschaften der Elektrolumineszenzvorrichtungen, wie Effizienz oder Betriebsspannung, mindestens gleich gut. In einer weiteren Varianten zeichnen sich die erfindungsgemäßen Verbindungen und die erfindungsgemäßen organischen Elektrolumineszenzvorrichtungen gegenüber dem Stand der Technik insbesondere durch eine verbesserte Effizienz und/oder Betriebsspannung und höhere Lebensdauer aus.

Die erfindungsgemäßen Verbindungen können auch zur Farbkonversion eingesetzt werden. Vorzugsweise können sie zur Farbkonvertierung lichtemittierender Vorrichtungen verwendet werden. Bevorzugte Anwendungsgebiete sind Pixel in Displays, Flächenelemente in Anzeigen (Sinage) und Beleuchtungselemente.

Die lichtemittierende Vorrichtung kann dabei aus der Vielzahl der bekannten Vorrichtungen ausgewählt werden. Zwei ausgewählte Beispiele für lichtemittierende Vorrichtungen sind LEDs und organische Elektrolumineszenzvorrichtungen.

Zu dem Zweck der Farbkonversion werden die Verbindungen in eine Komposition eingearbeitet, die dann durch bekannte Verfahren (Spin-coating, Slit-coating, Raklen, Siebdruck, Nozzle-Printing, Ink-Jet-Printing, etc.) zu Pixeln oder flächigen Schichten verarbeitet werden.

Die Kompositionen enthalten, neben einer oder mehreren erfindungsgemäßen Verbindungen, typischerweise vernetzbare Komponenten (Monomeren, Oligomeren, Polymeren), z. B. auf Basis von Acrylaten, Acrylamiden, Polyestern, Siliconen, etc. und einem oder mehreren thermisch oder photochemisch aktivierbaren Starterkomponenten. Daneben können weitere Komponenten wie org. Hilfsstoffe (Antioxidantien, Stabilisatoren, Verlaufshilfsmittel, Viskositätsmoderatoren, etc.) oder anorg. Füllstoffe (SiO₂, TiO₂, Al₂O₃, etc.) eingebracht werden. Weiterhin kann bevorzugt sein, wenn die Komposition eine oder mehrere weitere fluoreszierende Materialien enthält, die sich von den erfindungsgemäßen Verbindungen unterscheiden. In Betracht kommen dabei alle die dem Fachmann bekannten fluoreszierenden Materialien. Es können anorganische oder organische fluoreszierende Materilien verwendet werden.

Das Prinzip der Farbkonvertierung, der Farbkonversionsfilme sowie deren Herstellung und Komponenten sind dem Fachmann gut bekannt (z.B. WO 2017/054898 A1, WO2019/002239 A1, X. Bai et al, 30, SID DIGEST 2019, J. E. Kwon, J. A. Chem. Soc., 135, .30, 11239, 2013, W. H. Kim et al, Appl. Sci, 10, 2112, 2020)

Die vorliegende Erfindung betrifft daher auch eine Zusammensetzung enthaltend eine oder mehr erfindungsgemäße Verbindungen sowie eine vernetzbare Komponente. Die vernetzbare Komponente kann jede beliebige Komponenten sein, die der Fachmann zu diesem Zweck in betracht ziehen würde. Vorzugsweise handelt es sich bei der vernetzbaren Komponente um eine ein Acrylaten, Acrylamid, Polyester oder Silicon, ganz bevorzugt sind Acrylate. Ganz bevorzugt enthält die Zusammensetzung neben einer oder mehrerer erfindungsgemäßer Verbindungen sowie der vernetzbaren Komponente noch eine Starterkomponente und noch bevorzugter ist, wenn die Zusammensetzung zusätzlich noch einen oder mehrere Hilfsstoffe enthält, wobei die oben genannten Hilfsstoffe in Betracht kommen.

Ferner betrifft die vorliegende Erfindung auch einen Farbkonversionsfilm enthaltend eine oder meherer der erfindungsgemäßen Verbindungen. Durch Verwendung der Farbkonversionsfilme können effiziente und reine Emissionsfarben mit schmalen Emissionsbanden erreicht werden. Die Farbkonversionsfilme können, beispielsweise, auf eine blau emittierende organische Elektrolumineszenzvorrichtung aufgebracht werden. Die erfindungsgemäßen Verbindungen absorbieren zumindest einen Teil des von der organischen Elektrolumineszenzvorrichtung emittierten Lichts und re-emittiert Licht einer größerer Wellenlänge (Color Down-Conversion). Je nach verwendeter erfindungsgemäßer Verbindungen können auf diese Art effizente, farbreine und schmalbandige blaue, grüne, gelbe, rote oder infrarote Emissionen erhalten werden. Die erfindungsgemäße Verbindung wird in diesem Fall nicht als elektrolumineszierende, sonderen als phololumineszierende Komponente eingesetzt.

Weiterhin betrifft die vorliegende Erfindung eine lichtemittierende Vorrichtung enthaltend eine organische Elektrolumineszenzvorrichtung und einen Farbkonversionsfilm. Vorzugsweise ist der Farbkonversionsfilm im Lichtausstrittsbereich der organischen Elektrolumineszenzvorrichtung angeordnet.

Weiterhin betrifft die vorliegende Erfindung die Farbkonversion mit Hilfe der erfindungsgemäßen Verbindungen in der Agrarindustrie, um die von einer Quelle emittierte Strahlung, beispielwiese die Strahlung der Sonne oder einer künstlichen Lichtquelle, so zu ändern, dass biologisches Material, vorzugsweise Pflanzen, Algen oder Pilze, maßgeschneiderte Bedingungen erfahren. Damit können der Zustand und das Wachstum des biologischen Materials optimal eingestellt und beeinflusst werden. Zu dem Zweck werden die erfindungsgemäßen Verbindungen vorzugsweise in eine Folie eingebracht. Die Erfindungsgemäßen Verbindungen können aber auch in Dächern von Gewächshäusern eingebaut werden. Eine weitere Möglichkeit stellt die Verarbeitung der erfindungsgemäßen Verbindungen in einer Lösung oder Dispersion dar, die direkt auf das biologische Material aufgesprüht werden kann.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:
1. Elektronische Vorrichtungen, insbesondere organische Elektrolumineszenzvorrichtungen enthaltend Verbindungen gemäß Formel (I) bzw. die zuvor und nachfolgend ausgeführten bevorzugten Ausführungsformen als Emitter weisen sehr schmal Emissionsbanden mit geringen FWHM-Werten (**F**ull **W**idth **H**alf **M**aximum) auf und führen zu besonders Farb-reiner Emission, erkennbar an den kleinen CIE-y-Werten. Besonders überraschend ist hierbei, dass sowohl blaue Emitter mit geringen FWHM-Werten als auch Emitter mit geringen FWHM-Werten bereitgestellt werden, die im grünen, gelben oder roten Bereich des Farbspektrums emittieren.
2. Die Emissionsbanden weisen in der langwelligen Emissionsflanke eine Schulter oder ein Nebenmaximum auf, die jeweils weniger als 40 %, oftmals weniger als 30 %, der Intensität des Hauptmaximums aufweisen. Dies führt in Top-Emission OLED-Bauteilen zu einer günstig geringen Blickwinkelabhänigkeit des Farbeindrucks im Vergleich zu schmalbandigen Bor-enthaltenden Emittern nach Stand der Technik, die oftmals keine derartigen Schultern bzw. Nebenmaxima aufweisen und eine größere Blickwinkelabhänigkeit des Farbeindrucks zeigen.
3. Elektronische Vorrichtungen, insbesondere organische Elektrolumineszenzvorrichtungen enthaltend Verbindungen gemäß Formel (I) bzw. die zuvor und nachfolgend ausgeführten bevorzugten Ausführungsformen, insbesondere als Emitter, weisen eine sehr gute Lebensdauer auf. Hierbei bewirken diese Verbindungen insbesondere einen geringen Roll-off, d.h. einen geringen Abfall der Leistungseffizienz der Vorrichtung bei hohen Leuchtdichten.
4. Elektronische Vorrichtungen, insbesondere organische Elektrolumineszenzvorrichtungen enthaltend Verbindungen gemäß Formel (I) bzw. die zuvor und nachfolgend ausgeführten bevorzugten Ausführungsformen als Emitter weisen eine hervorragende Effizienz auf. Hierbei bewirken erfindungsgemäße Verbindungen gemäß Formel (I) bzw. die zuvor und nachfolgend ausgeführten bevorzugten Ausführungsformen eine geringe Betriebsspannung bei Verwendung in elektronischen Vorrichtungen.
5. Die erfindungsgemäßen Verbindungen gemäß Formel (I) bzw. die zuvor und nachfolgend ausgeführten bevorzugten Ausführungsformen zeigen eine sehr hohe Stabilität und Lebensdauer.
6. Mit Verbindungen gemäß Formel (I) bzw. den zuvor und nachfolgend ausgeführten bevorzugten Ausführungsformen kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.
   Excitonenenergie wird von einem Matrix oder Host in der Emissionsschicht typischerweise entweder über den sogenannten Dexter- oder über den Förstertransfer auf den Emitter übertragen. Der Försterenergietransfer (FRET) von einem Host oder einer Matrix auf den erfindungsgemäßen Emitter ist dabei besonders bevorzugt, da dieser besonders effizient ist, was zu elektronischen Vorrichtungen mit besonders guten Leistungsdaten (bspw. Effizienz, Spannung und Lebensdauer) führt. Es zeigt sich, dass der Energieübertrag von einem Host oder einer Matrix auf die erfindungsgemäßen Verbindungen vorzugsweise über den Förstertransfer erfolgt.
7. Verbindungen gemäß Formel (I) bzw. die zuvor und nachfolgend ausgeführten bevorzugten Ausführungsformen weisen eine ausgezeichnete Glasfilmbildung auf.
8. Verbindungen gemäß Formel (I) bzw. die zuvor und nachfolgend ausgeführten bevorzugten Ausführungsformen bilden aus Lösungen sehr gute Filme und zeigen eine ausgezeichnete Löslichkeit.

### Abbildung

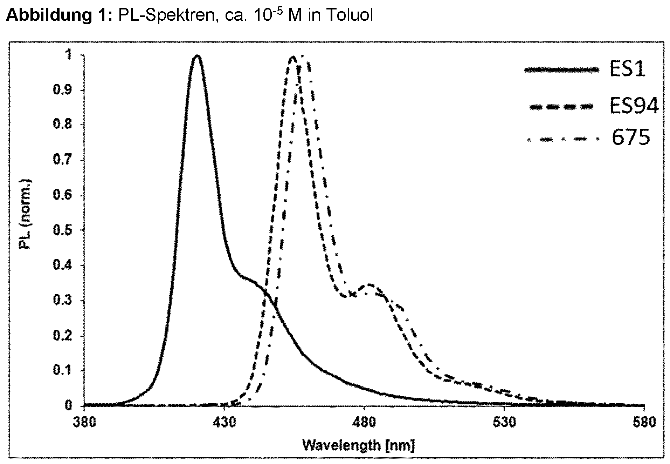

Abbildung 1 zeigt das Photolumineszenspektren (PL-Speltren) der Verbindungen ES1, ES94 und 675, gemessen mit einem PL-Spektrometer der Fa. Hitachi, F-4500 PL, in ca. 10⁻⁵ molarer, entgaster Toluol-Lösung bei Raumtemperatur (ca. 25 °C).

Diese oben genannten Vorteile gehen nicht mit einer unmäßig hohen Verschlechterung der weiteren elektronischen Eigenschaften einher.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Stufe sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen. Bei Verbindungen, die mehrere Konfigurationsisomere, Enantiomere, Diastereomere oder tautomere Formen ausweisen können, wird eine Form stellvertretend gezeigt.

### 1) Darstellung der Synthone

### 1.1) Nitrile: Beispiel S1

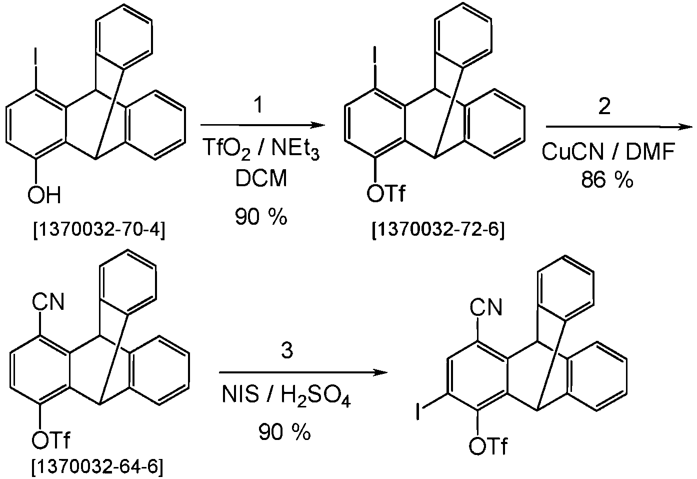

S1 kann auf der o.g. Route, gemäß der folgenden Literaturen, in 69 % Ausbeute dargestellt werden:
Stufe 1 und 2: W. S. Tan et al., J. Chin. Chem. Soc., 2012, 59, 399.
Stufe 3: J. M. Herbert et al., J. Label. Compd. Radiopharm., 2007, 50, 440.

Die Reinigung erfolgt via Flash-Chromatographie an einem Säulenautomaten (Combi-Flash Torrent, Fa. Axel Semrau).

Analog können folgende Synthone dargestellt werden.

| Bsp. | Edukt | Produkt | Ausbeute |
|---|---|---|---|
| S2 | 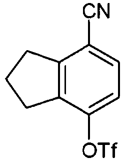 1403327-05-8, Stufe 3 | 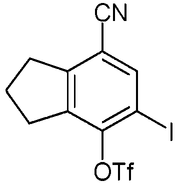 | 85 % |
| S3 | 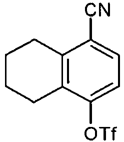 664364-61-8, Stufe 3 | 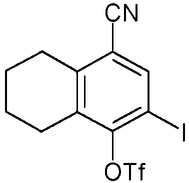 | 83 % |
| S4 | 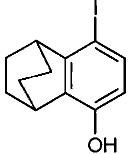 1560647-41-7, Stufe 1 - 3 | 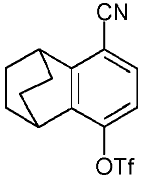 | 64 % |

### Alternative Darstellungsweise:

Alternativ kann S1 bis S 4 auf folgender Route in verbesserter Ausbeute dargestellt werden:

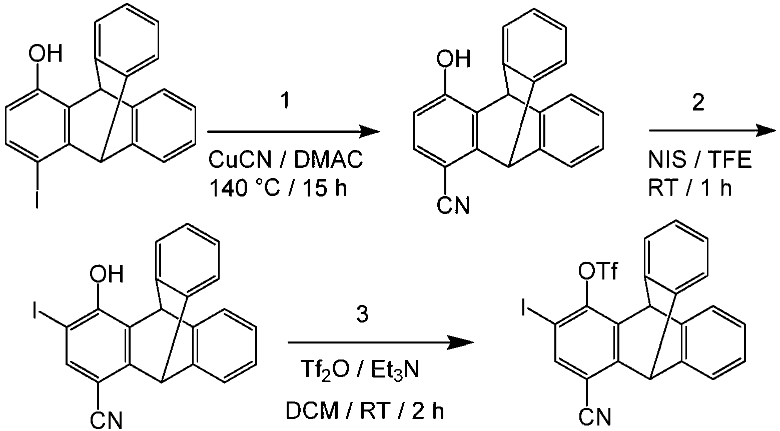

Stufe 1 und 3: Analog W. S. Tan et al., J. Chin. Chem. Soc., 2012, 59, 399. Ausbeute Stufe 1 - 95 %, Ausbeute Stufe 3 quantitativ.

Stufe 2: lodierung mit N-Iodsuccinimid in Trifluorethanol (TFE) bzw. Hexafluor-iso-propanol analog R.-J. Tang et al. J. Org. Chem., 2018, 83, 930. Ausbeute 93 %.

### Beispiel S1b:

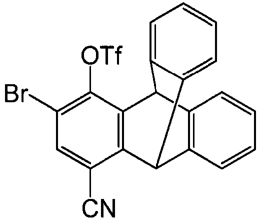

Analog können durch Einsatz von N-Bromsuccinimid die entsprechenden Brom-Triflate erhalten werden. Ausbeute über 3 Stufen 87 %.

### Beispiel S1c:

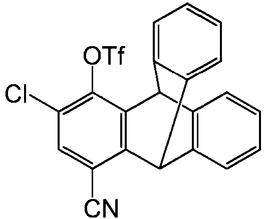

Analog können durch Einsatz von N-Chlorsuccinimid die entsprechenden Chlor-Triflate erhalten werden. Ausbeute über 3 Stufen 69 %.

### Beispiel S1d:

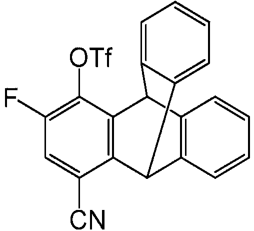

Analog können durch Fluorierung in Stufe 2 analog R. D. Chambers et al., J. Fluor. Chem., 2000, 102, 169 die entsprechenden Fluor-Triflate erhalten werden. Ausbeute über 3 Stufen 30 %.

### Beispiel S1e:

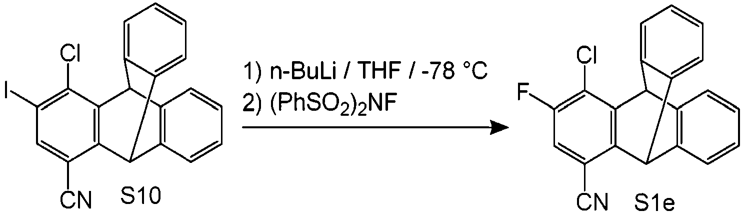

I-F-Austausch analog C. S. Hartley et al., Chem. Mater. 2004, 16, 5297. Ausbeute 28 %.

Alternativ kann S1e wie folgt dargestellt werden:

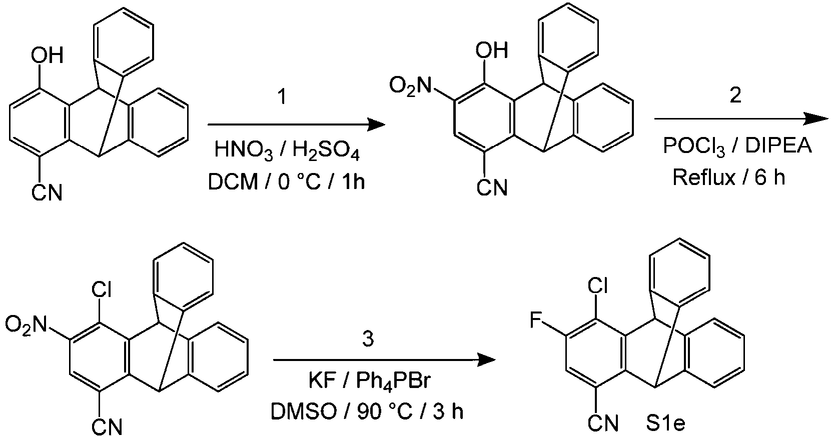

Stufe 1: Analog M. A. Zolfigol et al., Molecules 2001, 6, 614. Ausbeute: 93 %.

Stufe 2: G. Ralf et al. Journal fuer Praktische Chemie 1987, 329(6), 945. Ausbeute 89 %.

Stufe 3: Analog J. H. Clark et al., Chem. & Ind. 1991, 436. Ausbeute: 38 %.

### Optimierte Synthese von S1e:

### Stufe 1:

Eine auf 0 °C gekühlte Lösung von 29.5 g (100 mmol) 1-Cyano-4-hydroxytriptycen wird tropfenweise während 1 h mit einem Gemisch aus 19.0 g 65 Gew.-%iger Salpetersäure und 20.0 g 96 % Gew.-%iger Salpetersäure versetzt. Man rührt noch 30 min. nach und gießt dann unter sehr gutem Rühren vorsichtig (Schäumen!) auf ein Gemisch aus 37.8 g (450 mmol) Natriumhydrogencarbonat und 3 l Eiswasser. Man Trennt die org. Phase ab, extrahiert die wässrige Phase dreimal mit je 200 ml DCM, trocknet die vereinigten org. Phasen mit ges. Kochsalzlösung und stellt auf Magnesiumsulfat. Man Filtriert vom Trockenmittel ab, entfernt das DCM im Vakuum und chromatographiert den Rückstand (Kieselgel, n-Heptan/EE 5:1). Ausbeute: 31.5 g (93 mmol) 93 %; Reinheit ca. 98 % ig n. ¹H-NMR.

### Stufe 2:

Ein gut gerührtes Gemisch aus 34.0 g (100 mmol) 1-Cyano-3-nitro-4-hydroxytriptycen und 93.5ml (1 mol) Phosphorylchlorid wird mit 21.0 ml (120 mmol) Di-iso-propyl-ethylamin (DIPEA) versetzt und 4 h unter Rückfluss erhitzt. Man gießt die Reaktionsmischung unter sehr gutem Rühren langsam (exotherm, Induktionsperiode!) auf 2 l Eiswasser und rührt 30 min. nach. Man extrahiert die wässrige Phase fünf mal mit je 200 ml DCM trocknet die vereinigten org. Phasen mit ges. Kochsalzlösung und stellt auf Magnesiumsulfat. Man Filtriert vom Trockenmittel ab, entdernt das DCM im Vakuum und chromatographiert den Rückstand (Kieselgel, n-Heptan/EE 5:1). Ausbeute: 40.1 g (89 mmol) 89 %; Reinheit ca. 97 % ig n. ¹H-NMR.

### Stufe 3:

Analog J. H. Clark et al., Chem. & Ind. 1991, 436. Ausbeute: 38 %.

Alternativ kann S1e in verbesserter Ausbeute wie folgt dargestellt werden:

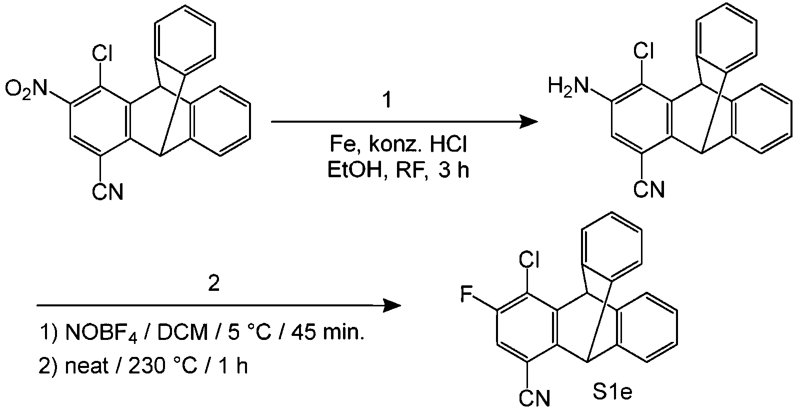

Stufe 1: Analog S. Chandrappa et al., Synlett 2010, 3019. Ausbeute: 87 %.

Stufe 2: D. J. Milner et al., Synth. Commun., 1992, 22(1), 73. Ausbeute: 77 %.

### Optimierte Synthese von S1e:

### Stufe 1:

Eine gut gerührte Suspension von 35.9 g (100 mmol) 1-Cyano-3-nitro-4-chlor-triptycen und 25.1 g (450 mmol) Eisenpulver in 700 ml EtOH wird unter Rückfluss tropfenweise während 30 min. mit 75.0 ml wässriger Salzsäure, 37 Gew.-%ig versetzt (Achtung: Wasserstoffentwicklung!). Man rührt noch 3 h am Rückfluss nach, lässt erkalten, verdünnt mit 2 l Wasser und 2 l DCM und stellt unter vorsichtiger Zugabe (Schäumen!) von festem Natriumcarbonat alkalisch (pH ~ 9). Man saugt das Gemisch über Celite ab, trennt die org. Phase des Filtrats ab, extrahiert die wässrige Phase fünf mal mit je 100 ml DCM, trocknet die vereinigten org. Phasen durch zweimaliges waschen mit je 300 ml ges. Kochsalzlösung und stellt auf Magnesiumsulfat. Man filtriert vom Trockenmittel ab, entfernt das DCM im Vakuum, zieht das Rohprodukt auf Isolute auf und chromatographiert (Kieselgel, n-Heptan/DCM 1:1 > 1:2). Gegebenenfalls wird erneut chromatographiert, bis das Produkt weis bis leicht beige anfällt. Ausbeute: 28.5 g (87 mmol) 87 %; Reinheit ca. 98 % ig n. ¹H-NMR.

### Stufe 2:

Eine gut gerührte, auf 0 °C gekühlte Lösung von 12.9 g (110 mmol) Nitrosyltetrafluoroborat [NO][BF₄] in 500 ml DCM wird während 10 min. Portionsweise mit 32.9 g (100 mmol) 1-Cyano-3-amino-4-chlor-triptycen versetzt. Man rührt noch 30 min. nach, filtriert vom Diazonium-Salz ab, wäscht dieses einmal mit 100 ml DCM/n-Heptan (1:2, vv) und einmal mit 100 ml n-Heptan und trocknet kurz bei RT im Vakuum. Ausbeute: 40.6 g (97 %).

Das'so erhaltene Diazoniumsalz wir in ein einem rotierenden Kolben im leichten Argonstrom bei 220-230 °C bis zur beendeten Stickstoffentwicklung zersetzt (ca. 1 h). Der erkaltete Rückstand wird mit 500 ml DCM extrahiert. Man filtriert von unlöslichen Anteilen ab, engt das Filtrat zur Trockene ein und chromatographiert den Rückstand (Kieselgel, n-Heptan/DCM 2:1). Ausbeute: 25.4 g (77 mmol); Reinheit: Reinheit ca. 98 % ig n. ¹H-NMR.

Analog können folgende Synthone dargestellt werden. Ausbeute über 4 bzw. 5 Stufen.

| Bsp. | Edukt | Produkt | Ausbeute |
|---|---|---|---|
| S2e | 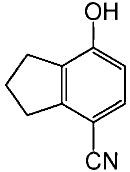 1403327-04-7 | 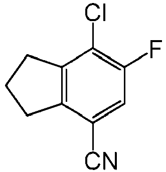 | 42 % |
| S3e | 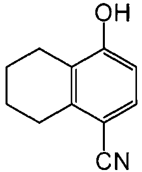 89682-91-7 | 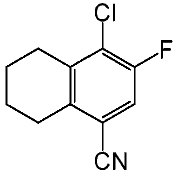 | 44 % |
| S4e | 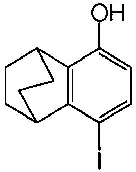 1560647-41-7 | 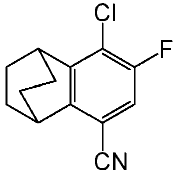 | 35 % |

### Beispiel S10:

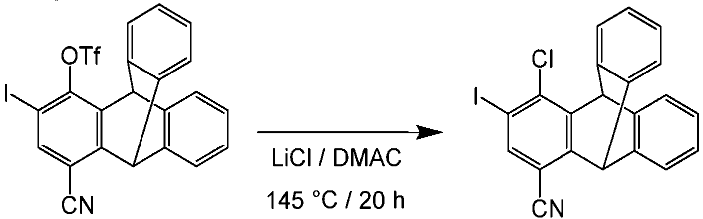

Durchführung analog W. S. Tan et al., J. Chin. Chem. Soc., 2012, 59, 399. Anstelle von DMF wird Dimethylacetamid (DMAC) verwendet, was zu verbesserten Ausbeuten führt. Ausbeute 66 %.

Analog zu S1 (alternative Darstellungsweise) und S10 können folgende Synthone dargestellt werden.

| Bsp. | Edukt | Produkt | Ausbeute |
|---|---|---|---|
| S10b | S1b | 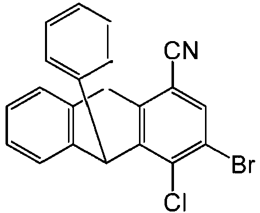 | 68 % |
| S11 | S2 | 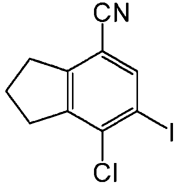 | 57 % |
| S12 | S3 | 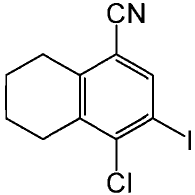 | 64 % |
| S13 | S4 | 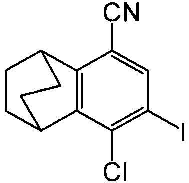 | 57 % |
| S14 | 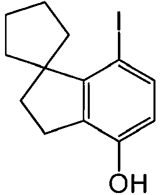 1588404-75-4 | 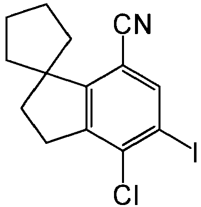 | 48 % |
| S15 | 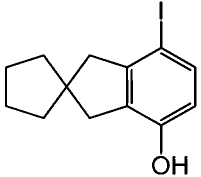 1419387-01-1 | 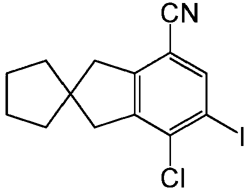 | 49 % |
| S16 | 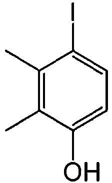 17938-69-1 | 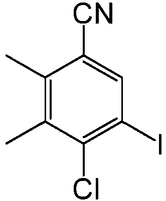 | 50 % |
| S17 | 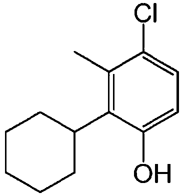 93257-53-5 | 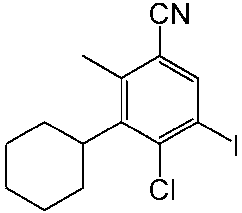 | 43 % |

### 1.2) Bicyclische Ketone:

### Beispiel S50:

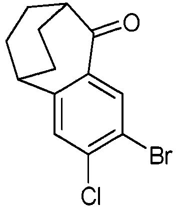

### A) Via Grignard-Route:

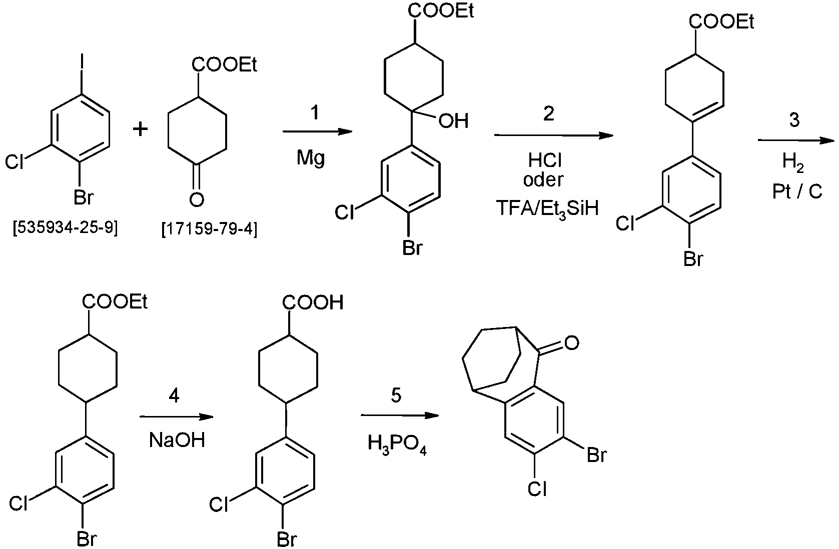

S50 kann auf der o.g. Grignard-Route ausgehend von den o.g. Edukten, gemäß der folgenden Literaturen, in 34 % Ausbeute dargestellt werden:
Stufe 1-4: B. M. Fox et al., J. Med. Chem., 2014, 52, 3464.
Stufe 5: I. Dragutan et al., Org. Prep. Proced., Int., 1975, 7, 2, 75.

Die Reinigung, insbesondere die Abtrennung von Regioisomeren der Cyclisierung in Stufe 5, erfolgt via Flash-Chromatographie an einem Säulenautomaten (Combi-Flash Torrent, Fa. Axel Semrau).

### B) Via Suzuki-Route:

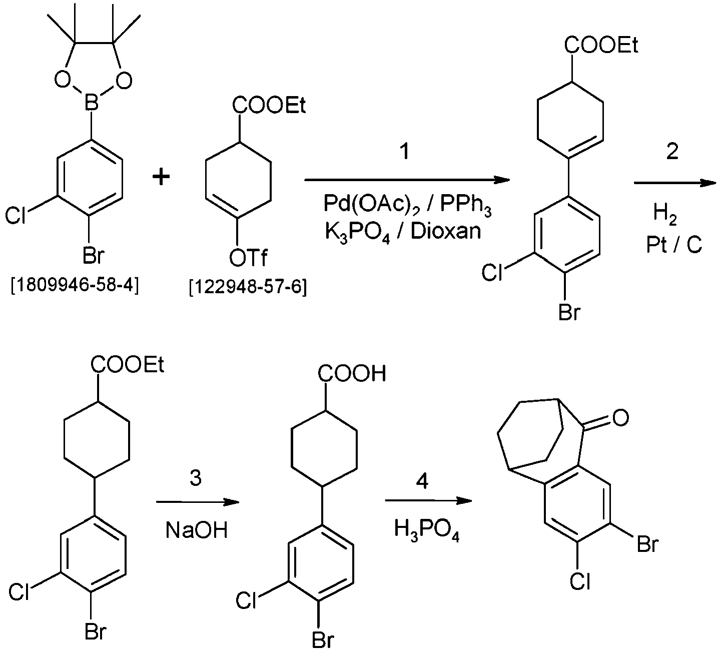

S50 kann auch auf der o.g. Suzuki-Route ausgehend von den o.g. Edukten, gemäß der folgenden Literaturen, in 41 % Ausbeute dargestellt werden:
Stufe 1 bis 3: C. Dolente et al., WO 2011/120877
Stufe 4: I. Dragutan et al., Org. Prep. Proced., Int., 1975, 7, 2, 75.

Die Reinigung, insbesondere die Abtrennung von Regioisomeren der Cyclisierung in Stufe 4, erfolgt via Flash-Chromatographie an einem Säulenautomaten (Combi Flash Torrent, Fa. Axel Semrau).

### Beispiel S51:

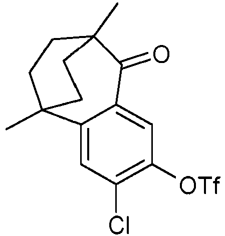

### C) Via Friedel-Crafts Alkylierung und Acylierung

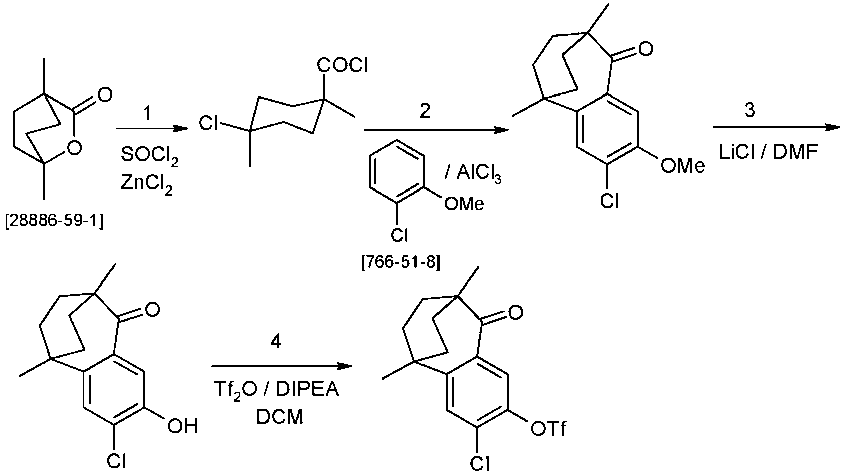

S51 kann auf der o.g. Friedel-Crafts-Route, gemäß der folgenden Literaturen, in 28 % Ausbeute dargestellt werden, wobei anstelle von Anisol 2-Chloranisol verwendet wird:
Stufe 1: Ismailov, A. G. et al., Nauch. Tr. Azerb. Un-t. Ser. Khim. N, 1979, (4),47.
Stufe 2: Ismailov, A. G. et al., Zhurnal Organicheskoi Khimii, 1978, 14(4), 811.
Stufe 3 und 4: M. L. Maddess et al., Org. Process Res. Dev. 2014, 18, 528-538.

Die Reinigung, insbesondere die Abtrennung von Regioisomeren der Cyclisierung in Stufe 2, erfolgt via Flash-Chromatographie an einem Säulenautomaten (Combi-Flash Torrent, Fa. Axel Semrau).

Analog können folgende Synthone dargestellt werden:

| Bsp. | Edukt | Produkt | Ausbeute |
|---|---|---|---|
| S52 | 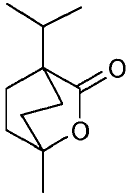 1222103-23-2 | 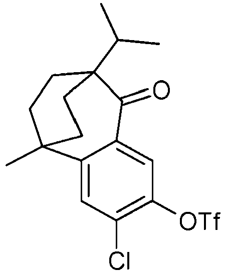 | 30 % |
| S53 | 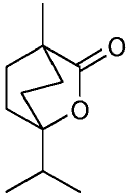 1222103-24-3 | 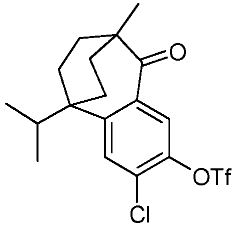 | 23 % |
| S54 | 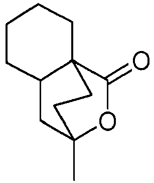 108793-30-2 | 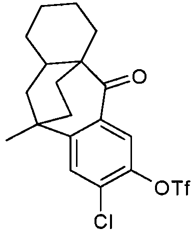 | 27 % |
| S55 | 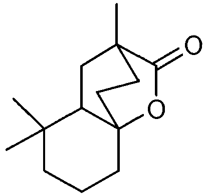 21280-29-5 | 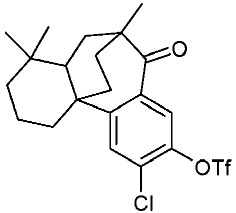 | 25 % |
| S56 | 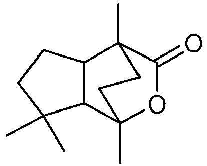 64390-25-6 | 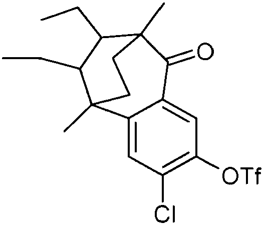 | 22 % |
| S57 | 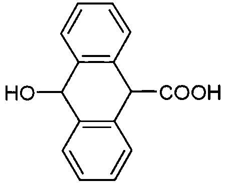 1076197-47-1 | 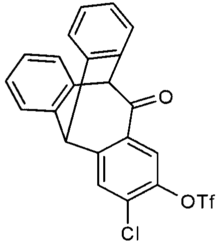 | 30 % |

### Beispiel S58:

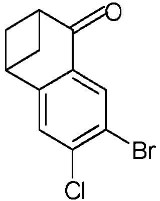

S58 kann auf der o.g. Grignard-Route A) entsprechend der o.g. Literatur oder nach der von G. M. Castanedo et al., J. Med. Chem., 2017, 60, 627 beschriebenen Grignard-Route, durch Einsatz von 1-Brom-2-chlor-4-iodbenzol anstelle von 1-Brom-2-fluor-4-iodbenzol, in 55 % Ausbeute dargestellt werden.

### 1.3) Synthese der substituieren lodo-Chloro-Pyridine

### Syntheseschema am Beispiel eines Homoadamtan-enamins:

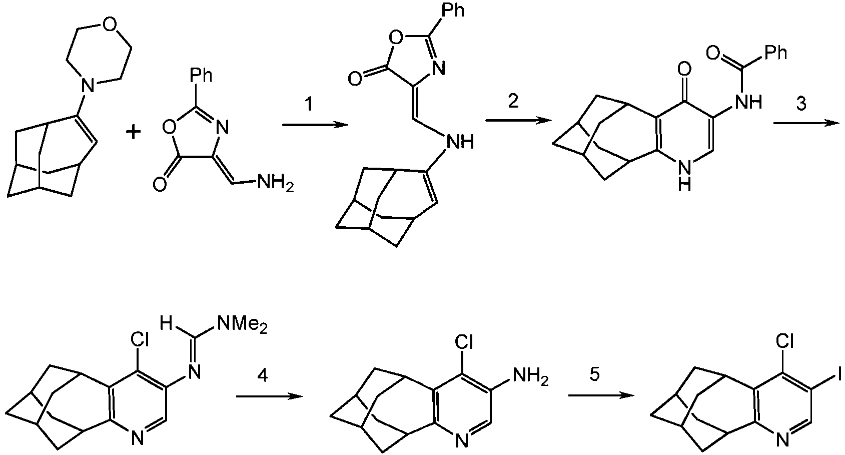

Die Stufe 1 bis 5 werden analog literaturbekannten Synthesen durchgeführt:

| | |
|---|---|
| Stufe 1 bis 4: | M. Adachi et al., Tetrahedron Letters, 37 (49), 8871, 1996; EP 0 556 008 B1. |
| Stufe 5: | J. D. Eckelbarger et al., US 8835409; E. A. Krasnokutskaya et al., Synthesis, 2007,1, 81. |

### A) Synthese der Enamine:

Die Enamine können nach dem in WO 2020/064662, Seite 108 ausgeführten Verfahren aus den gezeigten Ketonen und Morpholin in Ausbeuten von ca. 60 - 80 % dargestellt werden, bzw. sind literaturbekannt.

| Bsp. | Edukt Keton / Morpholin | Produkt Enamin |
|---|---|---|
| S100 | 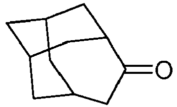 24669-56-5 | 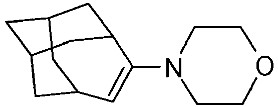 |
| S101 | 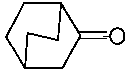 2716-23-6 | 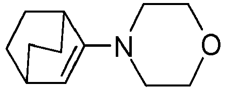 |
| S102 | 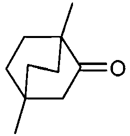 59117-09-8 | 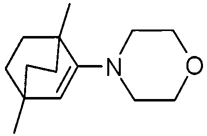 |
| S103 | 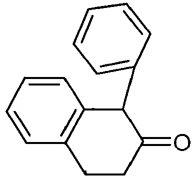 6372-63-0 | 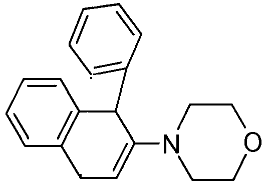 |
| S104 | 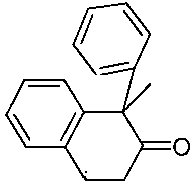 73164-06-4 | 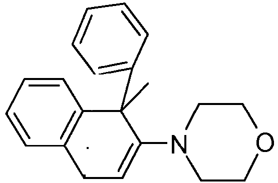 |
| S105 | 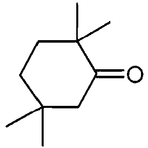 15189-14-7 | 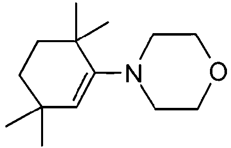 |
| S106 | 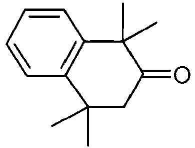 6308-02-7 | 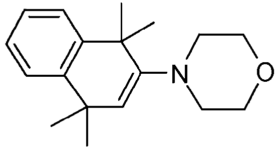 |
| S107 | 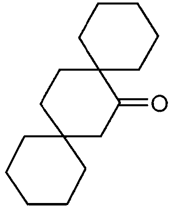 1781-82-4 | 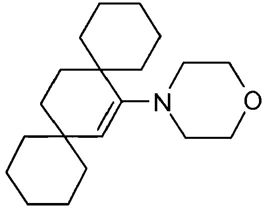 |
| S108 | 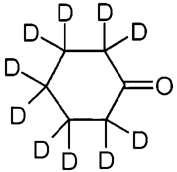 51209-49-5 | 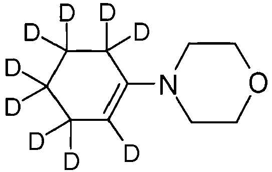 |
| S109 | 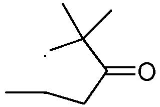 4694-115 | 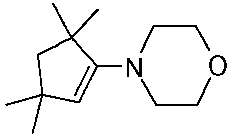 |
| S110 | 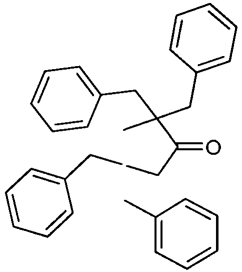 96676-35-6 | 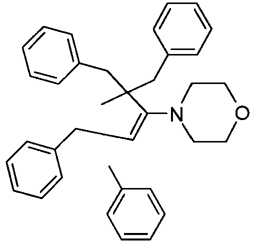 |
| S111 | 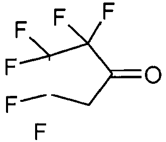 180690-80-6 | 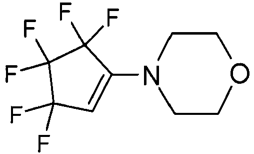 |
| S112 | --- | 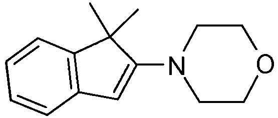 124032-58-2 |
| S113 | 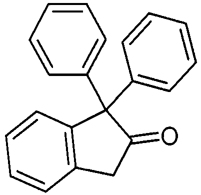 54193-73-6 | 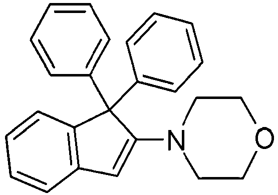 |
| S114 | 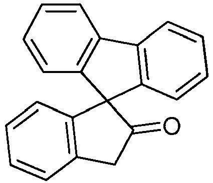 126495-32-7 | 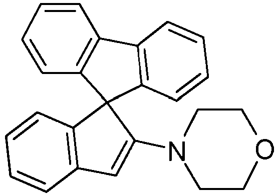 |
| S115 | --- | 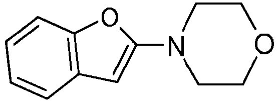 1195901-19-9 |
| S116 | --- | 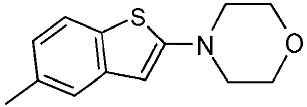 73129-56-3 |
| S117 | 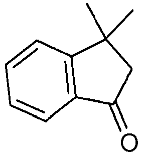 26465-81-6 | 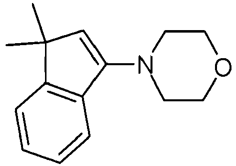 |
| S118 | 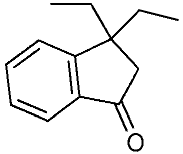 26465-81-6 | 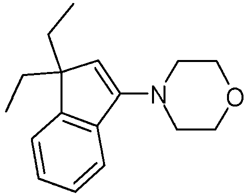 |
| S119 | 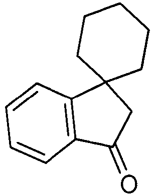 36449-72-6 | 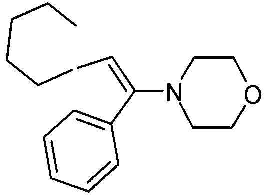 |
| S120 | 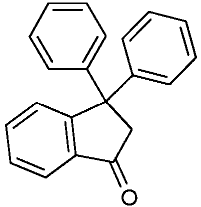 55010-17-8 | 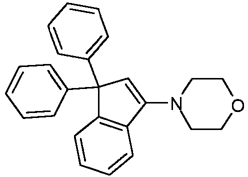 |
| S121 | 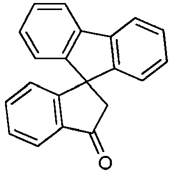 866762-72-3 | 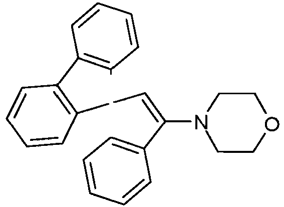 |
| S122 | --- | 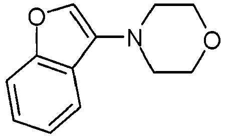 56639-83-9 |

### B) Synthese der substituierten Pyridine:

### Stufe 1: Beispiel S200

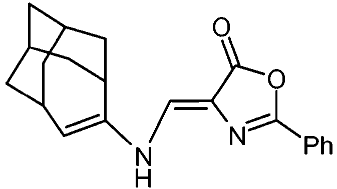

Ein Gemisch aus 23.3 g (100 mmol) S100 (analog für die anderen 6- und 7-Ring Enamine), 22.6 g (120 mmol) 4-(Aminomethylene)-2-phenyl-5(4H)-oxazolon [3674-51-9], 47.3 ml (500 mmol) Acetanhydrid [108-24-7] und 150 ml Toluol wird 4 h bei 100 °C gerührt (5-Ring-Enamine werden in o-Xylol bei 130 °C / 4 h im Autoklaven umgesetzt). Man engt im Vakuum komplett ein, versetzt das Öl mit 70 ml Methanol, rührt 3 h nach, saugt vom auskristallisierten Produkt ab, wäscht einmal mit 25 ml eiskaltem Methanol und trocknet im Vakuum. Das so erhaltene Rohprodukt wird ohne Reinigung weiter umgesetzt. Ausbeute: 26.2 g (78 mmol), 78 % E,Z-Isomerengemisch; Reinheit: ca. 95 % ig n. ¹H-NMR.

### Stufe 2: Beispiel S300

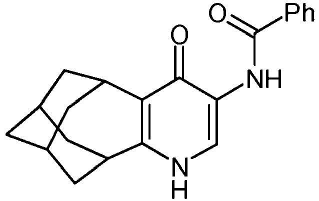

Ein Gemisch aus 33.4 g (100 mmol) S200 und 200 ml 1-Methyl-2-pyrrolidinon (NMP) wird 1.5 h bei 200 - 205 °C gerührt. Man lässt auf ca. 100 °C erkalten entfernt das NMP weitgehend im Vakuum, nimmt den glasartigen, zähen Rückstand in 100 ml warmem Acetonitril auf, rührt 12 h bei Raumtemperatur nach, saugt vom auskristallisierten Produkt ab und trocknet im Vakuum. Ausbeute: 25.1 g (75 mmol), 75 %; Reinheit: ca. 95 % ig n. ¹H-NMR.

### Stufe 3: Beispiel S400

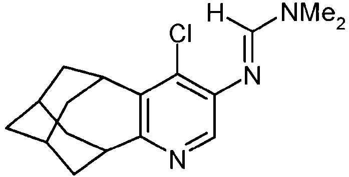

Eine Suspension von 33.4 g (100 mmol) S300 in einem Gemisch aus 150 ml N,N-Dimethylformamid (DMF) wird unter Eis-Kochsalz-Kühlung (ca. -10 °C) tropfenweise mit 14.0 ml (150 mmol) Phosphorylchlorid in 50 ml DMF versetzt und dann 16 h bei Raumtemperatur nachgerührt. Man gießt die Reaktionsmischung vorsichtig auf 1000 ml Eiswasser, rührt 10 min. nach, gibt 200 ml Dichlormethan (DCM) zu, rührt 10 min. nach und trennt die org. Phase ab. Man stellt die wässrige Phase unter vorsichtiger Zugabe von konz. wässriger Ammoniak-Lösung basisch (pH 8-9), extrahiert die wässrige Phase dreimal mit je 200 ml Ethylacetat, wäscht die vereinigten Ethylacetat-Extrakte zweimal mit je 200 ml Eiswasser, einmal mit 200 ml ges. Natriumhydrogencarbonat-Lösung und zweimal mit je 100 ml ges. Kochsalzlösung. Man trocknet über einem Gemisch aus Magnesiumsulfat und Natriumcarbonat, filtriert vom Trockenmittel ab, engt die org. Phase im Vakuum ein und kristallisiert den Rückstand einmal aus Acetonitril unter Zusatz von Ethylacetat (EE) um. Ausbeute: 24.7 g (81 mmol), 81 %; Reinheit: ca. 95 % ig n. ¹H-NMR.

### Stufe 4: Beispiel S500

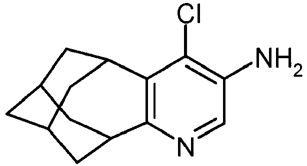

Ein Gemisch von 30.4 g (100 mmol) S400, 100 ml 3 N Schwefelsäure und 200 ml Dioxan wird 1.5 h bei 100 °C gerührt. Nach Erkalten wird die Reaktionsmischung mit 1000 ml Eis-Wasser verdünnt und dann unter Eiskühlung mit 3 N NaOH auf pH ~ 7.5 eingestellt. Man extrahiert die wässrige Phase dreimal mit je 200 ml DCM, wäscht die vereinigten org. Phasen zweimal mit je 200 ml Wasser, einmal mit 200 ml ges. Kochsalzlösung und trocknet über Magnesiumsulfat. Man filtriert von Trockenmittel ab, engt das Filtrat zur Trockene ein und kristallisiert aus Methanol um. Ausbeute: 23.1 g (93 mmol), 93 %; Reinheit: ca. 95 % ig n. ¹H-NMR.

### Stufe 5: Beispiel S600

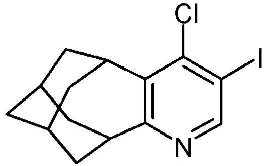

### Variante 1:

24.9 g (100 mmol) S500 werden unter gutem Rühren in 500 ml auf 3 - 5 °C gekühlte, konzentrierte, Salzsäure eingetragen. Die Suspension wird unter gutem Rühren tropfenweise während 15 min. mit einer kalten Lösung von 10.4 g (150 mmol) Natriumnitrit in 50 ml Wasser versetzt und anschließend ca. 20 min. bei 5 °C nachgerührt. Man gießt die so erhaltene Diazonium-Lösung in eine gut gerührte, auf 5 °C gekühlte Lösung von 90.0 g (600 mmol) Kaliumiodid in 5000 ml Wasser, das mit 1000 ml DCM versetzt ist (Achtung: Schäumen!). Nach beendeter Stickstoffentwicklung (ca. 25 min.) versetzt man bis zur Entfärbung mit Natriumbisulfit-Lösung und stellt mit 5 N NaOH unter sehr guter Kühlung vorsichtig auf pH ~ 7.5 ein. Man verdünnt mit weiteren 1500 ml DCM, trennt die org. Phase ab, re-extrahiert die wässrige zweimal mit je mit 500 ml DCM, wäscht die vereinigten org. Phasen zweimal mit je 500 ml Wasser und zweimal mit ja 500 ml ges. Kochsalzlösung und trocknet dann über Magnesiumsulfat. Nach Entfernen das DCM im Vakuum wird der Rückstand flashchromatographiert (Combi-Flash Torrent der Fa. A. Semrau). Ausbeute: 22.9 g (63 mmol), 63 %; Reinheit: ca. 97 % ig n. ¹H-NMR.

### Variante 2:

Eine Lösung von 24.9 g (100 mmol) S500 in 500 ml Acetonitril wird portionsweise mit 57.1 g (300 mmol) p-Toluolsulfonsäure-Monohydrat [6192-52-5] versetzt und dann im Eisbad auf 10 °C gekühlt. Die Suspension wir unter gutem Rühren und Eiskühlung portionsweise mit einer Lösung von 13.9 g (200 mmol) Natriumnitrit und 37.5 g (250 mmol) Kaliumiodid in 60 ml Wasser versetzt und 15 min. bei 10 °C gerührt. Anschließend lässt man auf Raumtemperatur erwärmen und rührt 70 min. nach. Man verdünnt dann mit 1500 ml Wasser, stelle durch Zugabe von gesättigter Natriumhydrogencarbonat-Lösung auf pH = 9.5 ein und versetzt mit 200 ml 2M Natrium-bisulfit-Lösung. Man saugt vom ausgefallenen Rohprodukt ab, wäscht dieses zweimal mit je 50 ml Wasser und saugt kurz trocken. Man löst das Rohprodukt in 500 ml DCM, trocknet die Lösung über Natriumsulfat, saugt vom Trockenmittel ab und zieht das Rohprodukt auf Isolute auf. Die Reinigung erfolgt durch Flash-chromatographie (Combi-Flash Torrent der Fa. A. Semrau). Ausbeute: 25.0 g (72 mmol), 72 %; Reinheit: ca. 97 % ig n. ¹H-NMR.

Analog zu den Stufen 1 bis 5 können folgende Pyridine erhalten werden. Ausbeute über fünf Stufe (Stufe 1-5):

| Bsp. | Enamin | Produkt | Ausbeute |
|---|---|---|---|
| S601 | S101 | 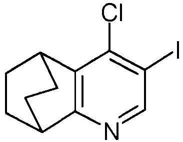 | 28 % |
| S602 | S102 | 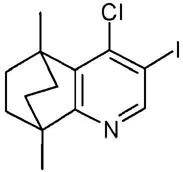 | 25 % |
| S603 | S103 | 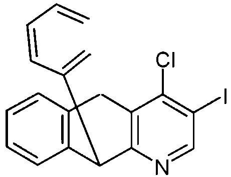 | 30 % |
| S604 | S104 | 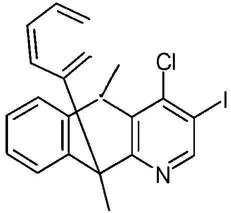 | 23 % |
| S605 | S105 | 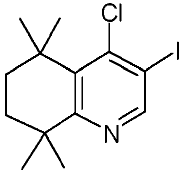 | 24 % |
| S606 | S106 | 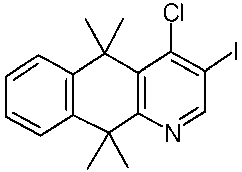 | 26 % |
| S607 | S107 | 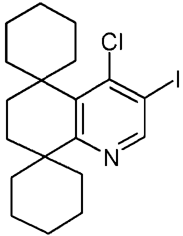 | 19 % |
| S608 | S108 | 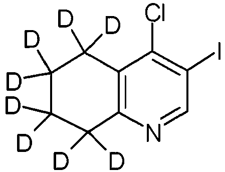 | 32 % |
| S609 | S109 | 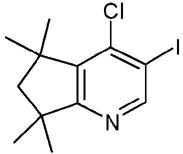 | 19 % |
| S610 | S110 | 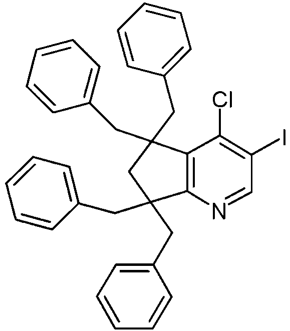 | 15 % |
| S611 | S111 | 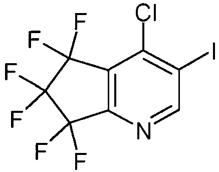 | 23 % |
| S612 | S112 | 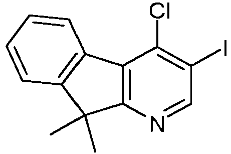 | 21 % |
| S613 | S113 | 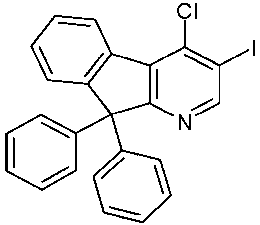 | 20 % |
| S614 | S114 | 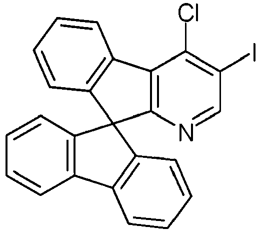 | 20 % |
| S615 | S115 | 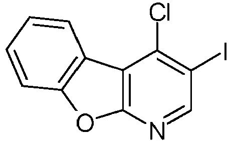 | 22 % |
| S616 | S116 | 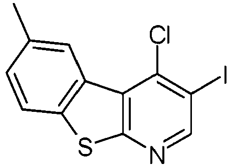 | 18 % |
| S617 | S117 | 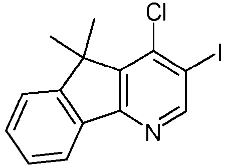 | 23 % |
| S618 | S118 | 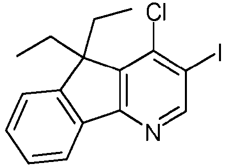 | 21 % |
| S619 | S119 | 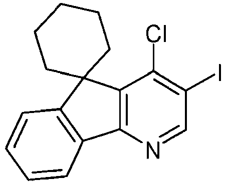 | 18 % |
| S620 | S120 | 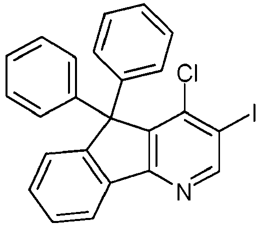 | 19 % |
| S621 | S121 | 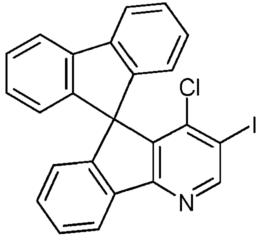 | 17 % |
| S622 | S122 | 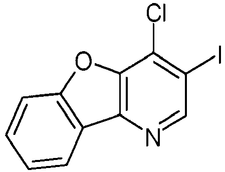 | 24 % |

### 1.4) Synthese von Anilinen

### Beispiel 700:

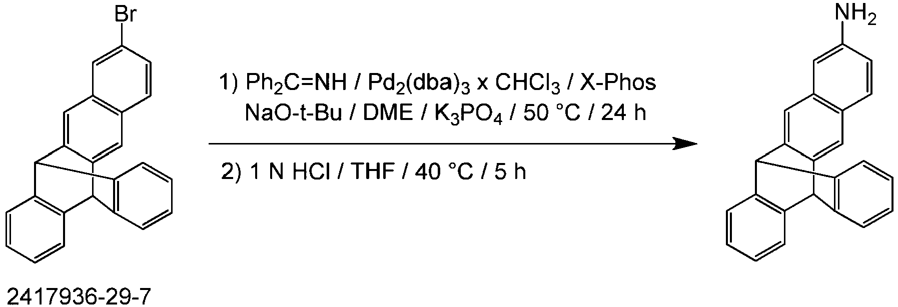

Durchführung analog S. Bhagwanth et al., Tetrahedron Letters 50 (2009) 1582. Ansatz: 38.g (100 mmol) Bromid, es werden Glaskugeln zum mechanisch gerührten Reaktionsgemisch zugesetzt. Ausbeute: 21.6 g (67 mmol) 67 %; Reinheit: ca. 97 % ig n. 1H-NMR.

| Bsp. | Edukte | Produkt | Ausbeute |
|---|---|---|---|
| S701 | 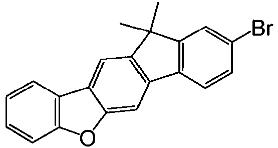 1898280-80-2 | 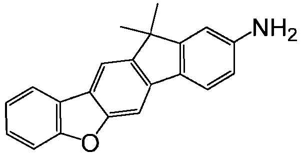 | 55 % |
| S702 | 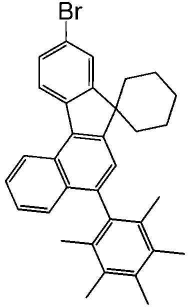 1579280-82-2 | 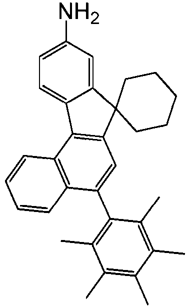 | 61 % |
| S703 | 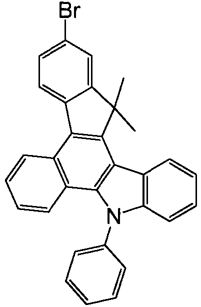 1678504-51-2 | 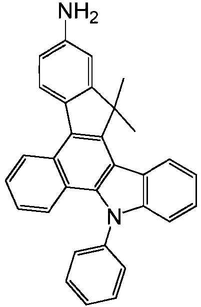 | 64 % |
| S704 | 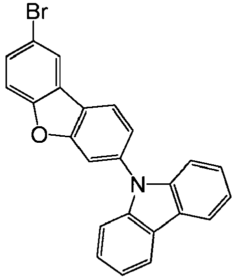 1914081-14-3 | 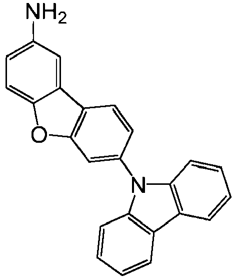 | 60 % |
| S705 | 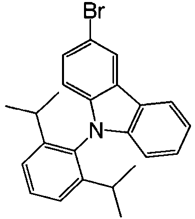 1147894-96-9 | 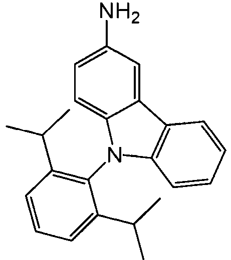 | 70 % |

### 1.5) Synthese der symmetrisch substituierten Amine

### Beispiel A1

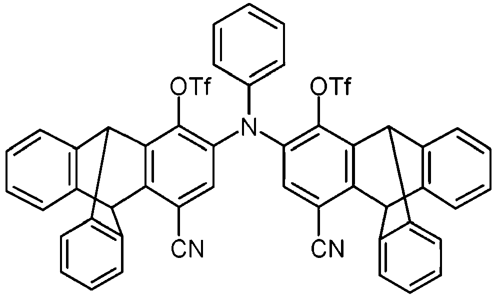

### Variante 1: Buchwald-Kupplung

Durchführung analog folgender Literaturen:
P. B. Tiruveedhula et al., Organic & Biomolecular Chemistry, 13 (43), 10705, 2015. K. Revunova et al., Polyhedron, 2013, 52, 1118.

Ein Gemisch aus 60.9 g (110 mmol) S1, 4.57 ml (50 mmol) Anilin, 65.2 g (200 mmol) Cäsiumcarbonat, 2.18 g (3.5 mmol) rac-BINAP [98327-87-8], 561 mg (2.5 mmol) Palladium(II)acetat, 500 ml Toluol und 50 g Glaskugeln (3 mm Durchmesser) wird zunächst 4 h bei 60 °C und dann 12 - 16 h bei 100 °C gerührt. Man lässt die Reaktionsmischung auf 60 °C erkalten und saugt von den Salzen über ein mit Toluol vorgeschlämmtes Celite-Bett ab. Man engt das Filtrat zur Trockene ein, kocht den Rückstand mit 200 ml Methanol aus, saugt vom Feststoff ab, wäscht diesen zweimal mit je 50 ml Methanol, trocknet im Vakuum und flash-chromatographiert (Combi-Flash Torrent der Fa. A. Semrau). Ausbeute: 31.2 g (33 mmol), 66 %; Reinheit: ca. 95 % ig n. ¹H-NMR.

Alternativ können andere Phosphine (z. B. Tri-tert-butylphosphin, Di-tert-butyl-methyl-phosphin, SPhos, XPhos, AmPhos, etc.) und Basen (s. B. Alkoholate wie Na-tert-butylat) eingesetzt werden.

### Variante 2: Jourdan-Ullmann-Kupplung

Durchführung analog folgender Literatur:
Y.-L- Tasi et al., J. Luminesc., 2007, 127, 41.

Ein Gemisch aus 60.9 g (110 mmol) S1, 4.57 ml (50 mmol) Anilin, 27.6 g (200 mmol) Kaliumcarbonat, 42.7 g (300 mmol) Natriumsulfat, 954 mg (15 mmol) Kupferpulver, 500 ml Nitrobenzol und 1000 g Glaskugeln (3 mm Durchmesser) wird 12 - 16 h bei 160 °C gerührt. Man lässt die Reaktionsmischung auf 60 °C erkalten und saugt von den Salzen über ein mit Toluol vorgeschlämmtes Celite-Bett ab. Man engt das Filtrat zur Trockene ein, kocht den Rückstand mit 200 ml Methanol aus, saugt vom Feststoff ab, wäscht diesen zweimal mit je 50 ml Methanol, trocknet im Vakuum und flash-chromatographiert (Combi-Flash Torrent der Fa. A. Semrau). Ausbeute: 27.9 g (29.5 mmol), 59 %; Reinheit: ca. 95 % ig n. ¹H-NMR.

### 1.6) Synthese der unsymmetrisch substituierten Amine

### Beispiel A500:

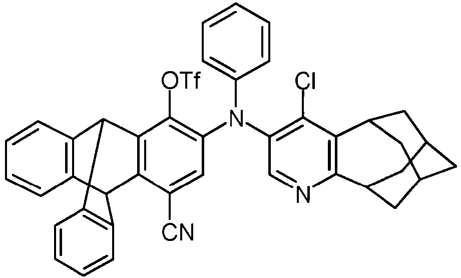

Ein Gemisch aus 27.7 g (50 mmol) S1, 4.57 ml (50 mmol) Anilin, 65.2 g (200 mmol) Cäsiumcarbonat, 2.18 g (3.5 mmol) rac-BINAP [98327-97-8], 561 mg (2.5 mmol) Palladium(II)acetat, 500 ml Toluol und 50 g Glaskugeln (3 mm Durchmesser) wird bis zum vollständigen Umsatz (DC Kontrolle, typischerweise 2-4 h) bei 60 °C gerührt. Dann fügt man 18.0 g (50 mmol) S600 zu, steigert die Temperatur auf 100 °C. Nach vollständigem Umsatz (DC Kontrolle, typischerweise 12 - 16 h) lässt die Reaktionsmischung auf 60 °C erkalten und saugt von den Salzen über ein mit Toluol vorgeschlämmtes Celite-Bett ab. Man engt das Filtrat zur Trockene ein, kocht den Rückstand mit 200 ml Methanol aus, saugt vom Feststoff ab, wäscht diesen zweimal mit je 50 ml Methanol, trocknet im Vakuum und flash-chromatographiert (Combi-Flash Torrent der Fa. A. Semrau). Ausbeute: 21.0 g (28 mmol), 56 %; Reinheit: ca. 95 % ig n. ¹H-NMR.

So erhaltene unsymmetrische Amin können wie unter 2.) beschrieben zu den erfindungsgemäßen Emitter EAS umgesetzt werden.

### 2.) Synthese der erfindungsgemäßen Emitter:

### 2.1) Synthese der symmetrisch substituierten Emitter:

### Beispiel ES1

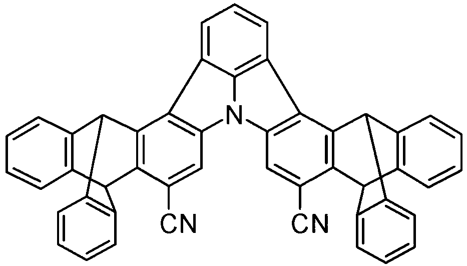

### Variante 1:

Durchführung analog folgender Literatur:
T. Kader et al., Chem. Eur. J., 2019, 25, 4412 - 4425.

Ein Gemisch aus 47.2 g (50 mmol) A1, 27.6 g (200 mmol) Kaliumcarbonat, 1.72 g (3 mmol) (NHC)Pd(allyl)Cl [478980-03-9], 50 g Glaskugeln (3 mm Durchmesser) und 500 ml N,N,-Dimethylacetamid (DMAc) wird unter gutem Rühren 16 h auf 150 °C erhitzt. Nach Erkalten auf 80 °C tropft man 1000 ml Wasser zu, saugt vom ausgefallenen Feststoff ab, wäscht diesen zweimal mit je 100 ml Wasser, zweimal mit je 50 ml Methanol und trocknet im Vakuum. Das Rohprodukt wird flash-chromatographiert (Combi-Flash Torrent der Fa. A. Semrau, DCM: 2 % MeOH), und anschließend durch wiederholte Heißextraktionskristallisation (DCM:Acetonitril 1:3 bis 2:1) und nachfolgende fraktionierte Sublimation oder durch Tempern im Hochvakuum gereinigt. Ausbeute: 14.9 g (23 mmol), 46 %; Reinheit: > 99.9 % ig n. HPLC.

Anstelle des (NHC)Pd(allyl)Cl kann auch 4 mmol [(tBu)₃PH][BF₄] und 2 mmol Pd(OAc)₂ eigesetzt werden.

### Variante 2:

Durchführung analog folgender Literatur:
A. W. Jones et al., Adv. Synth. Catal. 2015, 357, 945.

Ein Gemisch aus 47.2 g (50 mmol) A1, 3.1 g (10 mmol) Palladium(II)pivalat [106224-36-6], 27.8 g (120 mmol) Silber(I)oxid [20667-12-3], 9.6 g (120 mmol) Kupfer(II)oxid [1317-38-0], 50 g Glaskugeln (3 mm Durchmesser) und 200 ml Pivalinsäure (PivOH) wird unter gutem Rühren 24 h auf 130 °C erhitzt. Nach Erkalten auf 80 °C tropft man 1000 ml Wasser zu, saugt vom ausgefallenen Feststoff ab, wäscht diesen zweimal mit je 100 ml Wasser, zweimal mit je 50 ml Methanol und trocknet im Vakuum. Das Rohprodukt wird flash-chromatographiert (Combi-Flash Torrent der Fa. A. Semrau, DCM: 2 % MeOH), anschließend durch wiederholte Heißextraktionskristallisation (DCM:Acetonitril 1:3 bis 2:1) und nachfolgende fraktionierte Sublimation oder durch Tempern im Hochvakuum gereinigt. Ausbeute: 12.3 g (19 mmol), 38 %; Reinheit: > 99.9 % ig n. HPLC.

Analog zu den Stufen 1.4 und 2.1 können folgende Emitter ES dargestellt werden, Ausbeute über zwei Stufe:

| Bsp. | Nitril Amin | Produkt | Ausbeute |
|---|---|---|---|
| ES2 | 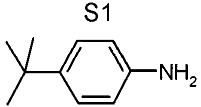 769-92-6 | 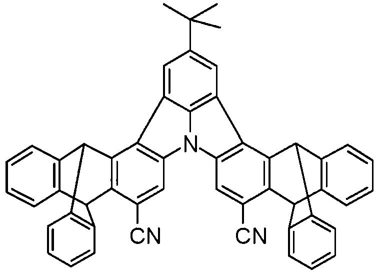 | 36 % |
| ES3 | 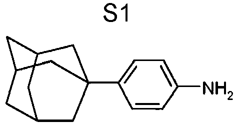 1459-48-9 | 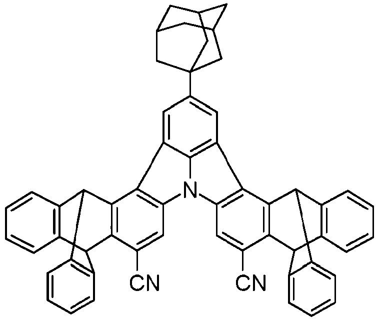 | 38 % |
| ES4 | 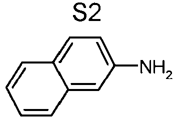 91-59-8 | 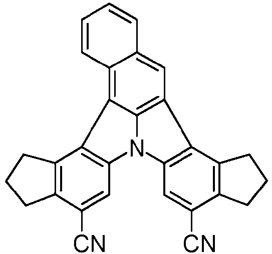 | 35 % |
| ES5 | 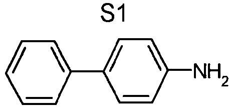 92-67-1 | 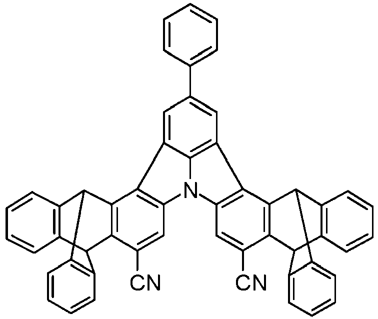 | 33 % |
| ES6 | 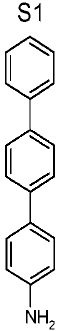 7293-45-0 | 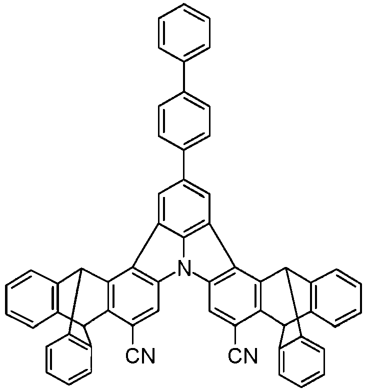 | 36 % |
| ES7 | 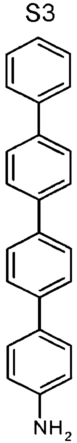 25660-12-2 | 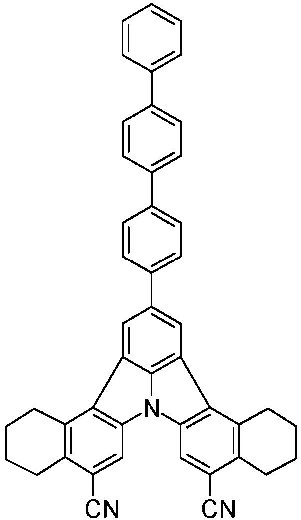 | 40 % |
| ES8 | 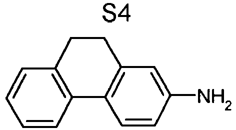 76302-58-4 | 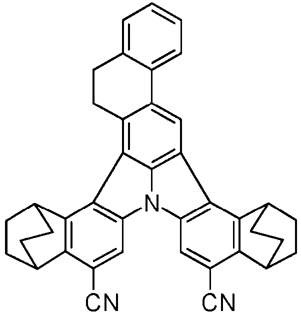 | 33 % |
| ES9 | 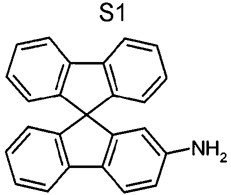 118951-68-1 | 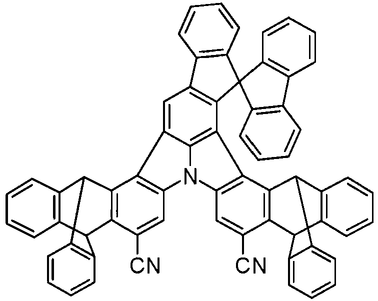 | 3% |
| ES10 | 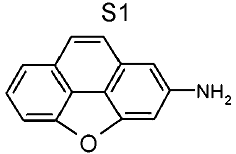 2018346-63-7 | 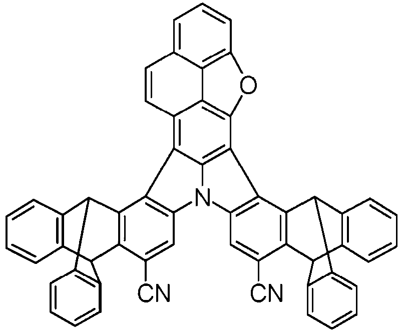 | 37 % |
| ES11 | 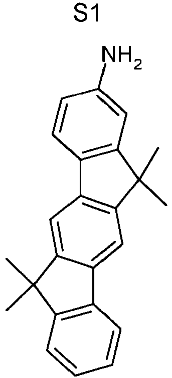 2295808-71-6 | 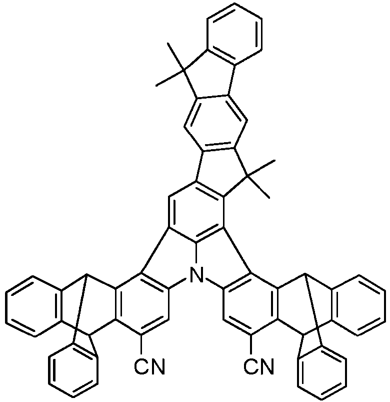 | 35 % |
| ES12 | 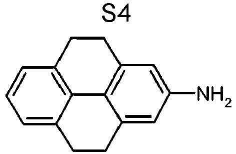 101283-00-5 | 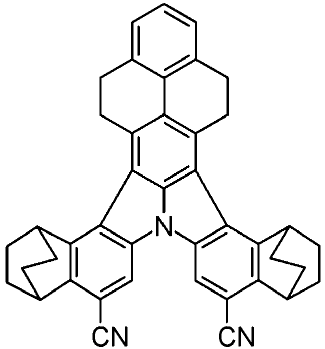 | 37 % |
| ES13 | 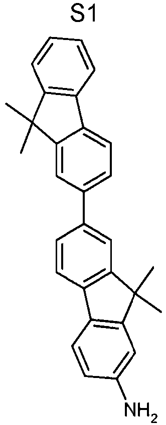 1416158-30-9 | 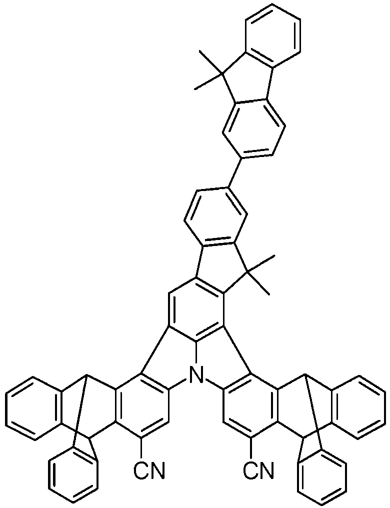 | 39 % |
| ES14 | 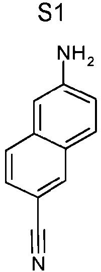 129667-70-5 | 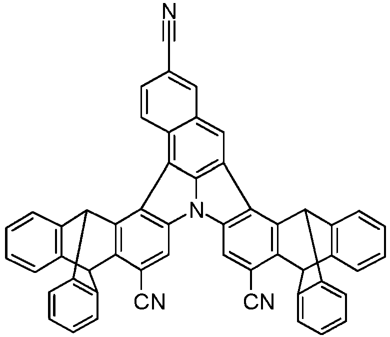 | 30 % |
| ES15 | 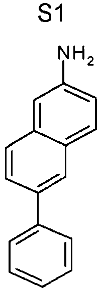 861046-41-5 | 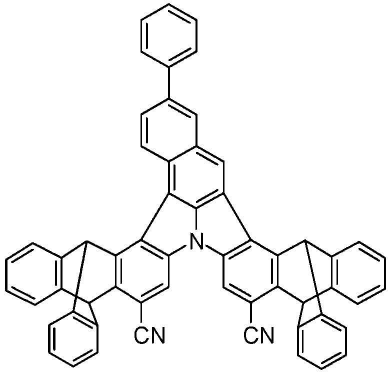 | 32 % |
| ES16 | 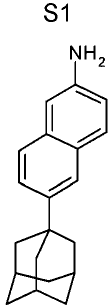 37521-66-7 | 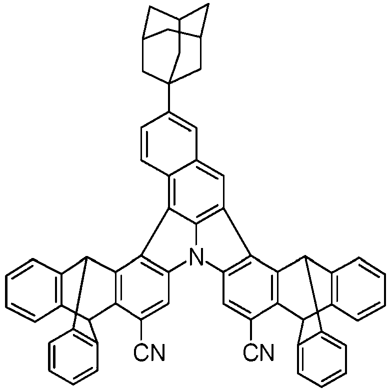 | 35 % |
| ES17 | 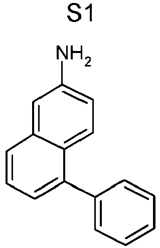 1609130-36-0 | 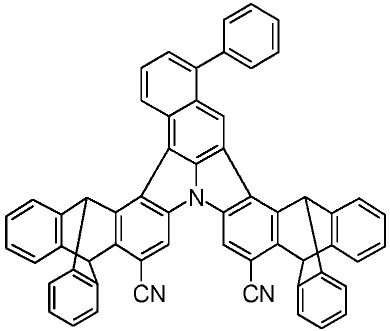 | 38 % |
| ES18 | 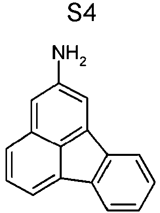 13177-26-9 | 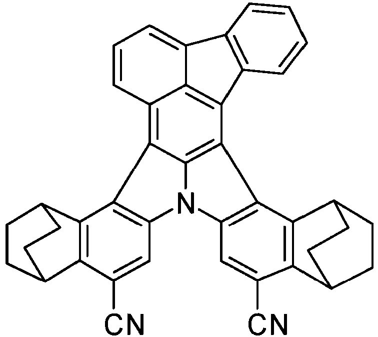 | 33 % |
| ES19 | 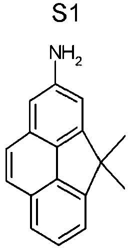 1639349-82-8 | 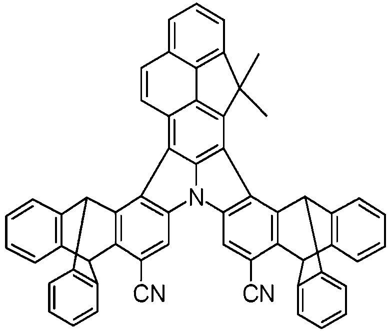 | 35 % |
| ES20 | 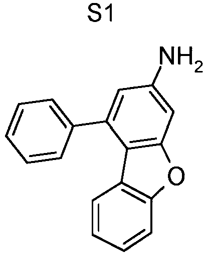 1882060-04-9 | 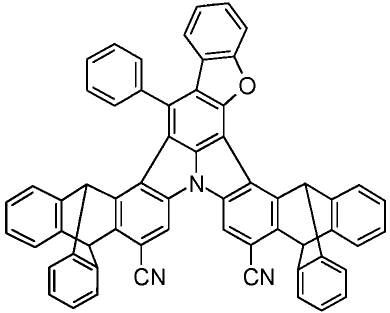 | 34 % |
| ES21 | 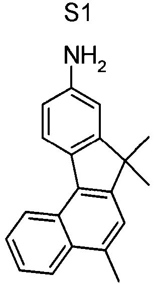 2379812-68-5 | 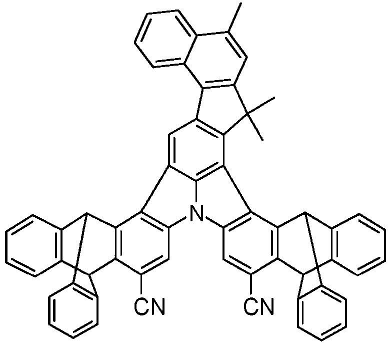 | 34 % |
| ES22 | 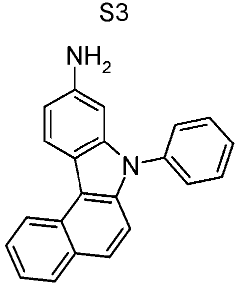 2086712-51-6 | 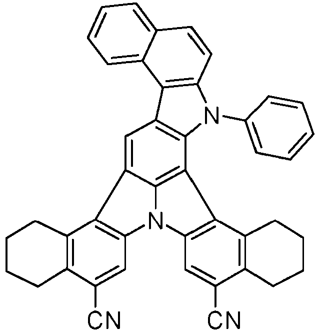 | 35 % |
| ES23 | 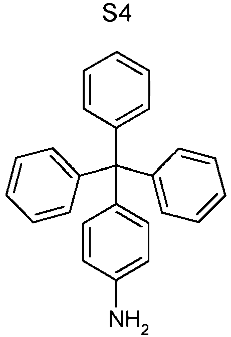 22948-06-7 | 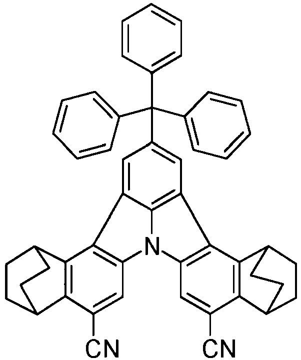 | 32 $ |
| ES24 | 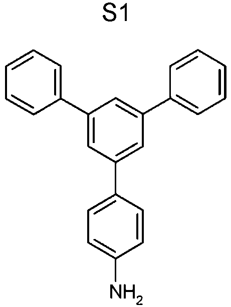 343239-58-7 | 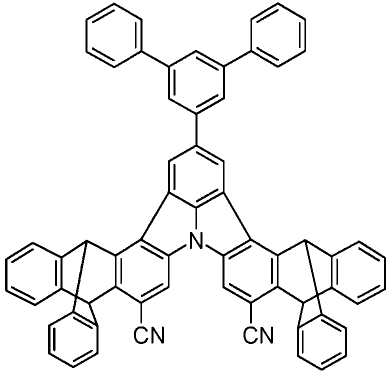 | 33 % |
| ES25 | 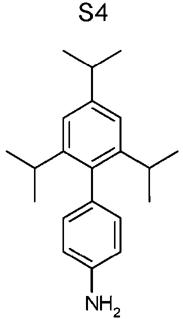 1801716-11-9 | 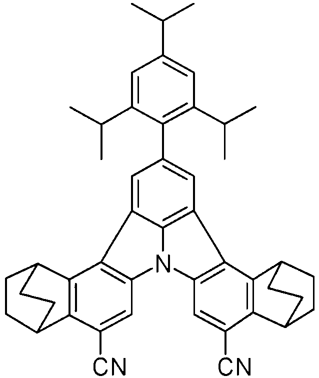 | 39 % |
| ES26 | 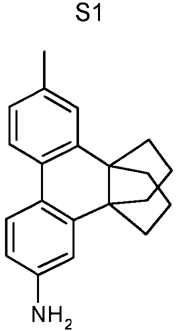 1884138-08-2 | 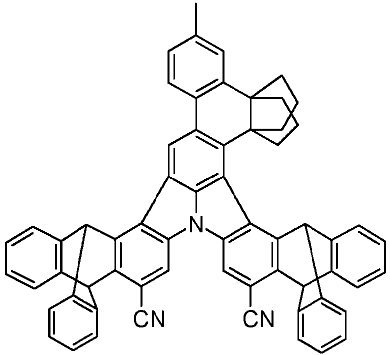 | 34 % |
| ES27 | 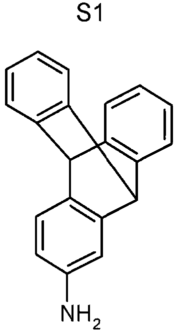 31997-11-2 | 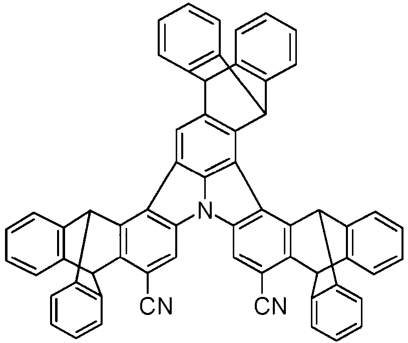 | 36 % |
| ES28 | 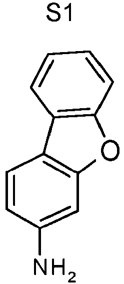 4106-66-5 | 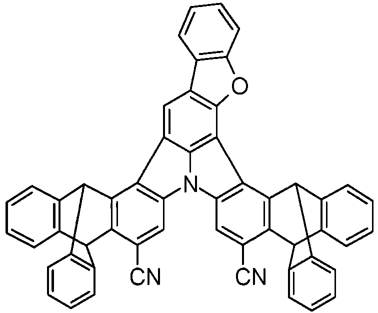 | 37 % |
| ES29 | 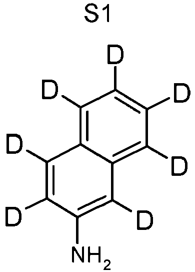 93951-94-1 | 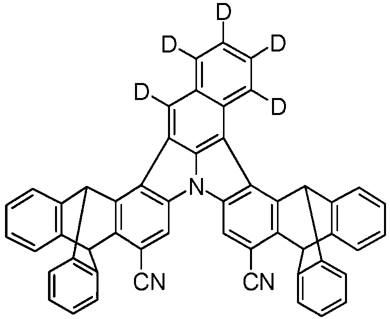 | 31 % |
| ES30 | 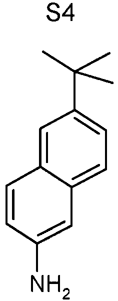 37521-64-5 | 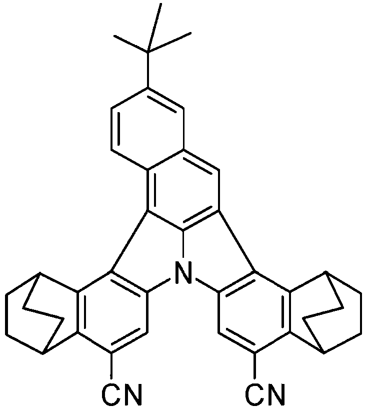 | 33 % |
| ES31 | 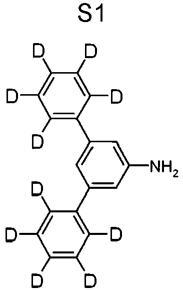 1846604-58-7 | 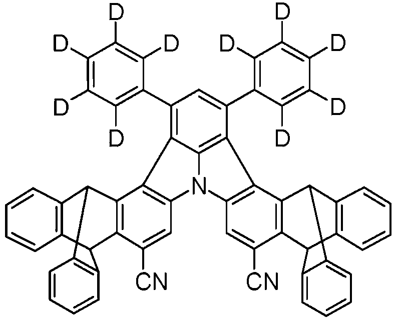 | 35 % |
| ES32 | 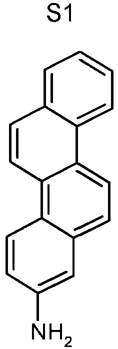 789-47-9 | 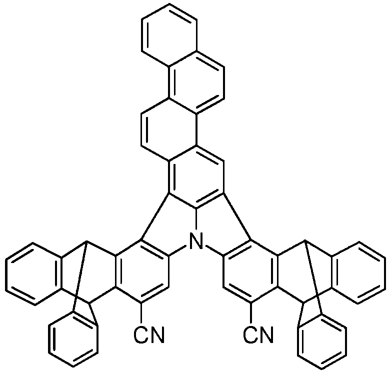 | 34 % |
| ES33 | 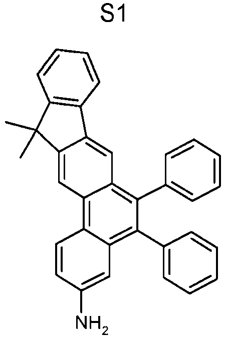 1409971-49-8 | 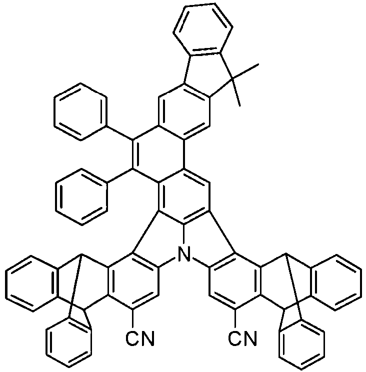 | 29 % |
| ES34 | 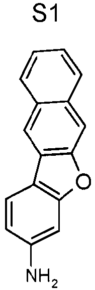 2281888-57-9 | 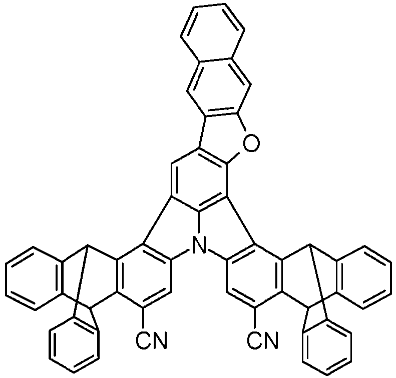 | 40 % |
| ES35 | 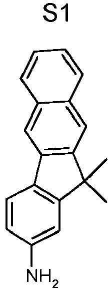 1642327-33-0 | 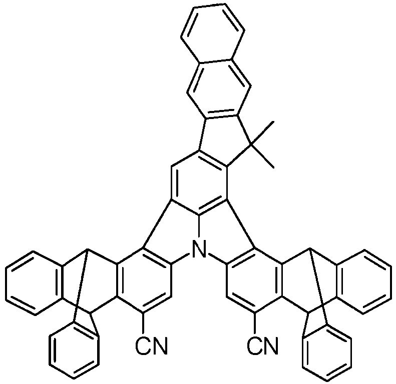 | 38 % |
| ES36 | 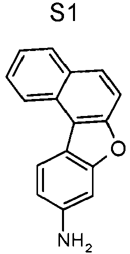 2460139-08-4 | 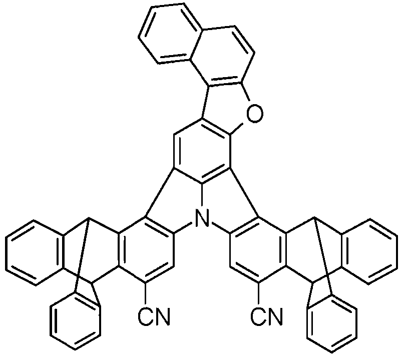 | 39 % |
| ES37 | 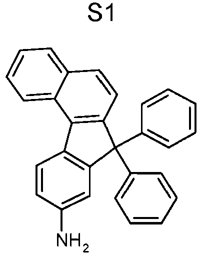 2411114-95-7 | 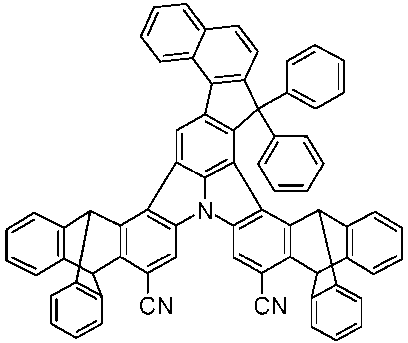 | 34 % |
| ES38 | 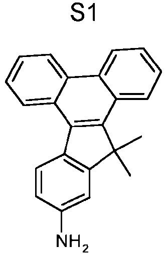 2226959-71-1 | 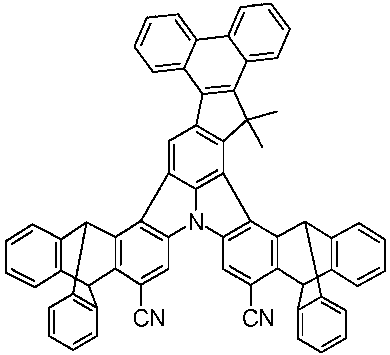 | 36 % |
| ES39 | 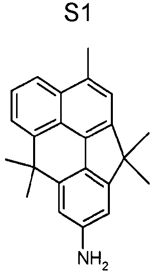 1225219-95-3 | 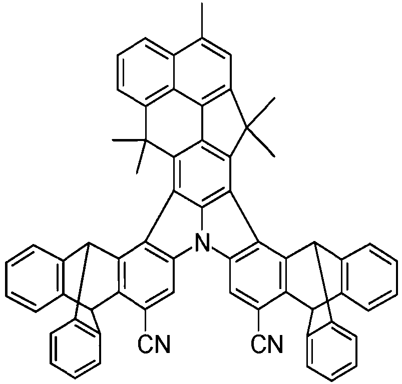 | 35 % |
| ES40 | 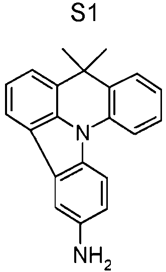 1448337-95-8 | 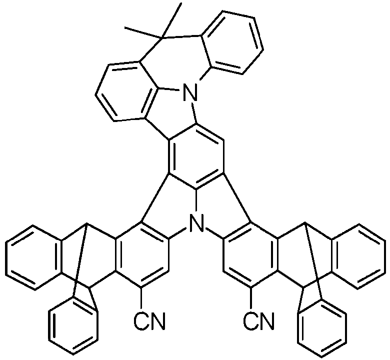 | 37 % |
| ES41 | 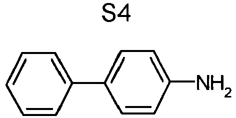 92-67-1 | 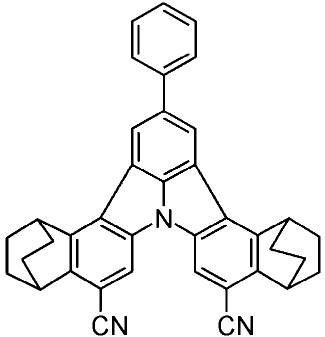 | 38 % |
| ES42 | 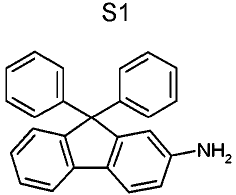 1268519-74-9 | 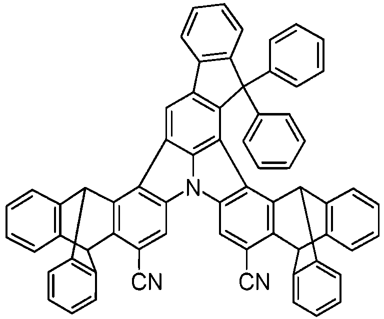 | 37 % |
| ES43 | 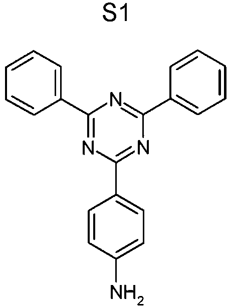 174152-47-7 | 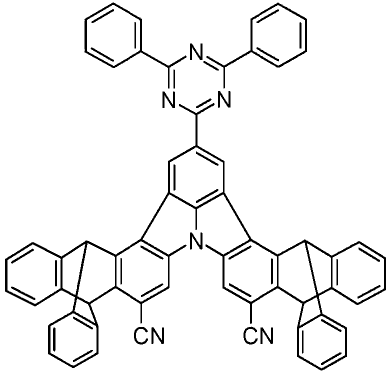 | 28 % |
| ES44 | 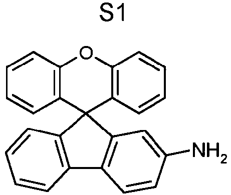 1520097-73-7 | 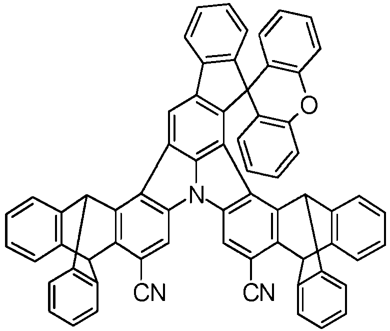 | 33 % |
| ES45 | 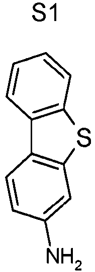 25288-76-0 | 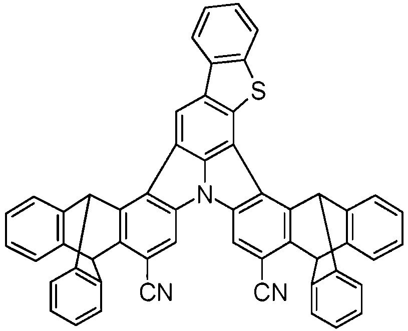 | 35 % |
| ES46 | 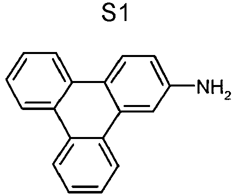 17169-81-2 | 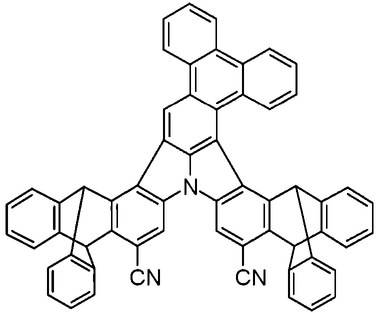 | 38 % |
| ES47 | 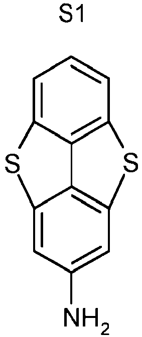 1820037-24-8 | 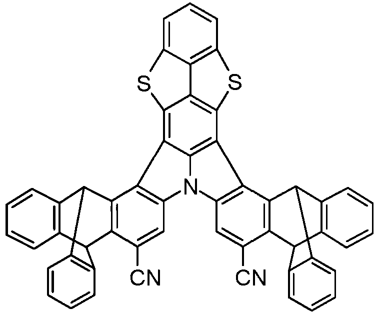 | 26 % |
| ES48 | 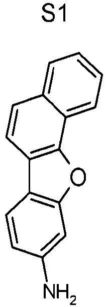 2364548-23-0 | 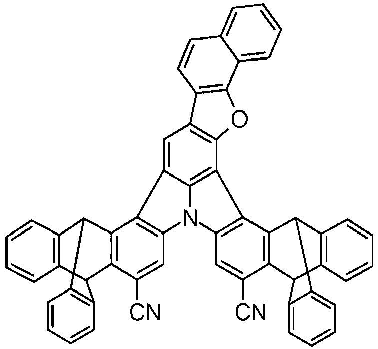 | 39 % |
| ES49 | 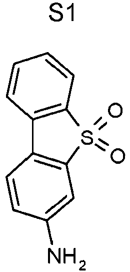 93618-98-5 | 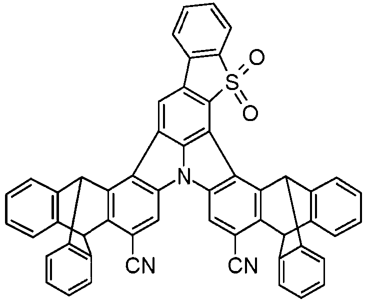 | 29 % |
| ES50 | 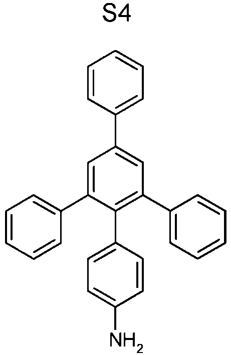 1093882-02-0 | 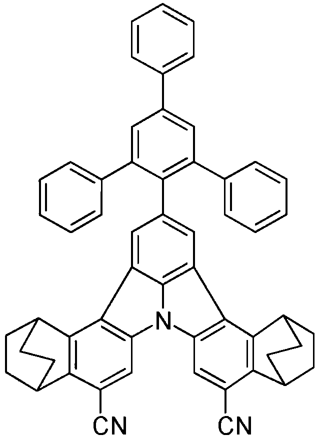 | 37 % |
| ES51 | 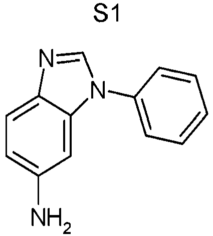 667919-05-3 | 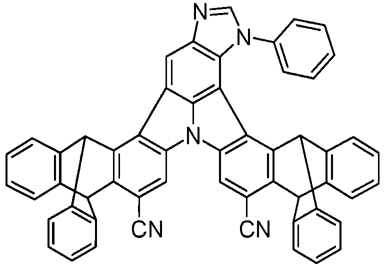 | 35 % |
| ES52 | 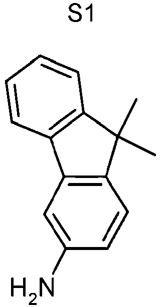 1421789-14-1 | 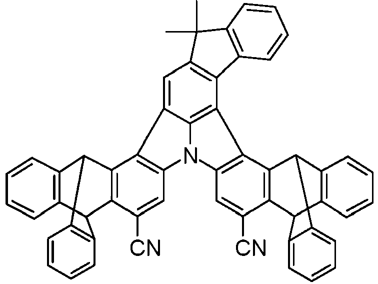 | 33 % |
| ES53 | 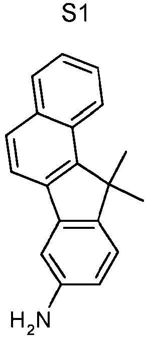 2411114-70-8 | 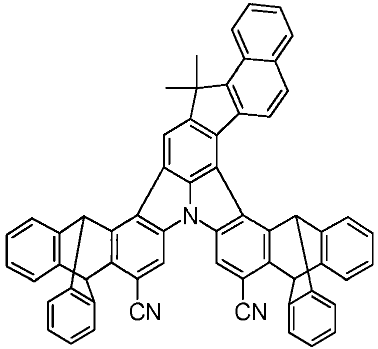 | 35 % |
| ES54 | 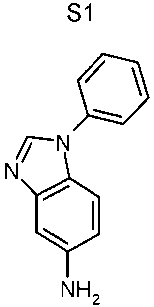 53897-95-3 | 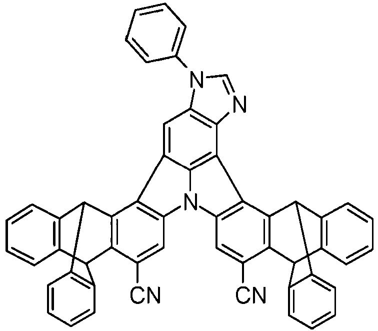 | 31 % |
| ES55 | 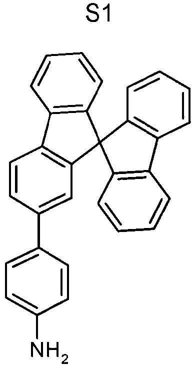 1853250-47-1 | 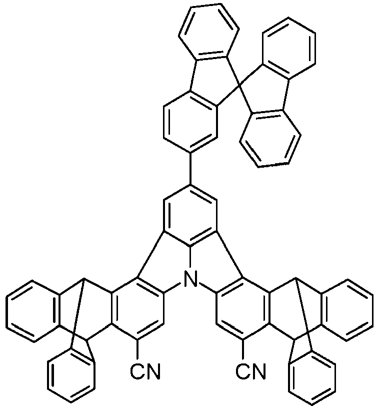 | 38 % |
| ES56 | 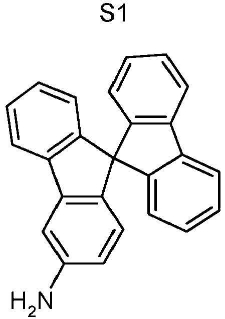 1644466-73-8 | 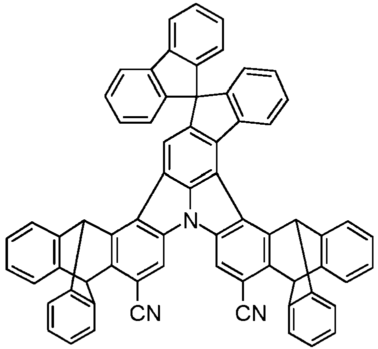 | 36 % |
| ES57 | 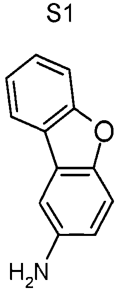 3693-22-9 | 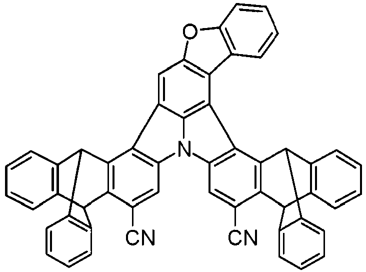 | 37 % |
| ES58 | 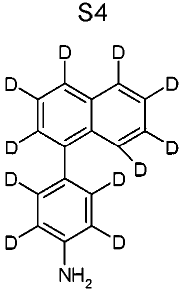 1940112-89-9 | 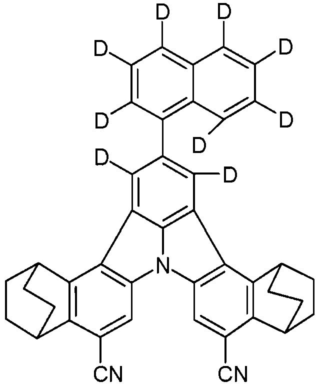 | 35 % |
| ES59 | 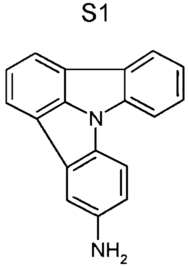 1191512-09-0 | 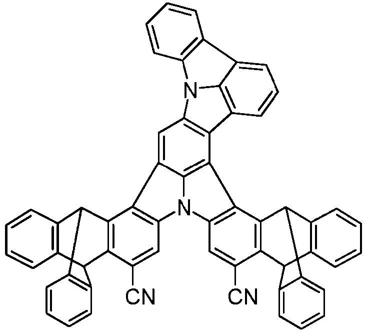 | 30 % |
| ES60 | 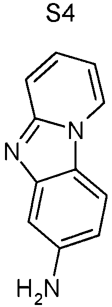 130595-01-6 | 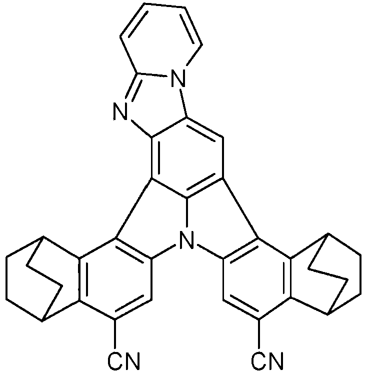 | 28 % |
| ES61 | 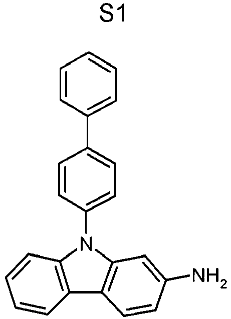 2129673-55-6 | 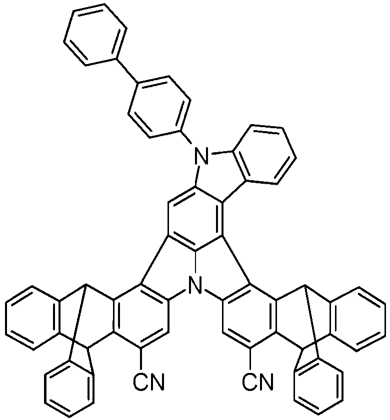 | 36 % |
| ES62 | 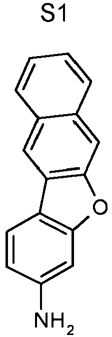 2179038-73-2 | 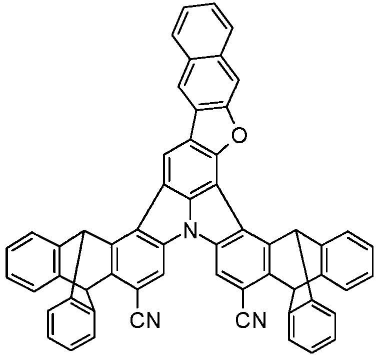 | 38 % |
| ES63 | 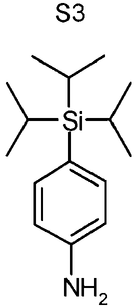 1346517-64-3 | 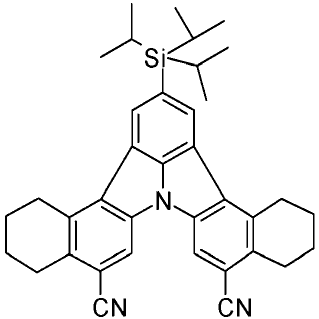 | 27 % |
| ES64 | 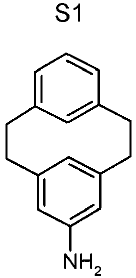 43215-86-7 | 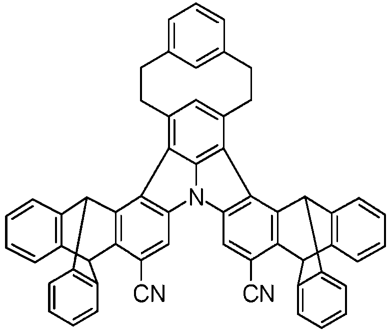 | 32 % |
| ES65 | 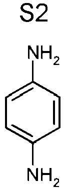 106-50-3 | 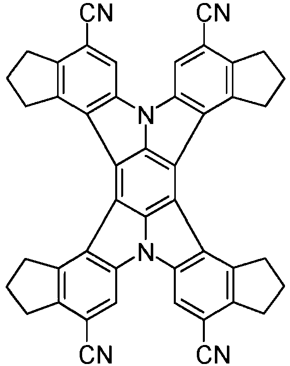 | 21 % |
| | 25 mmol | | |
| ES66 | 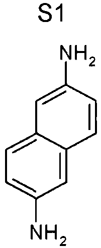 2243-67-6 | 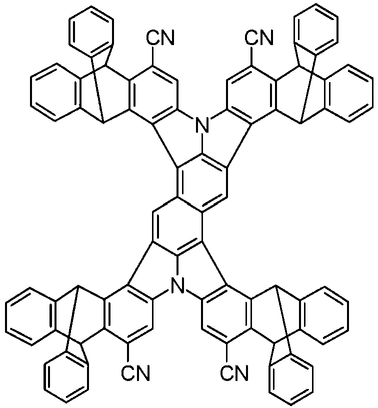 | 22 % |
| | 25 mmol | | |
| ES67 | 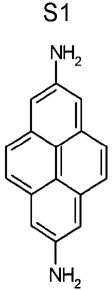 64535-41-7 | 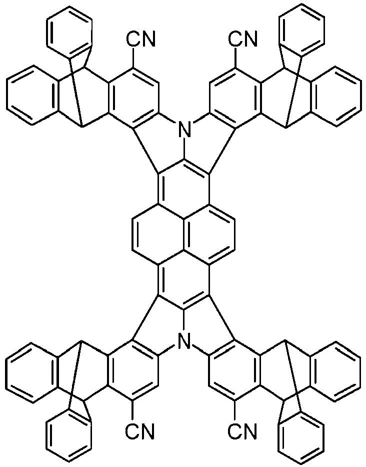 | 19 % |
| | 25 mmol | | |
| ES68 | 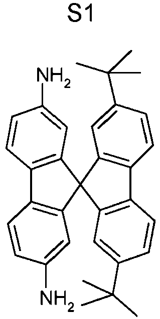 866464-33-7 | 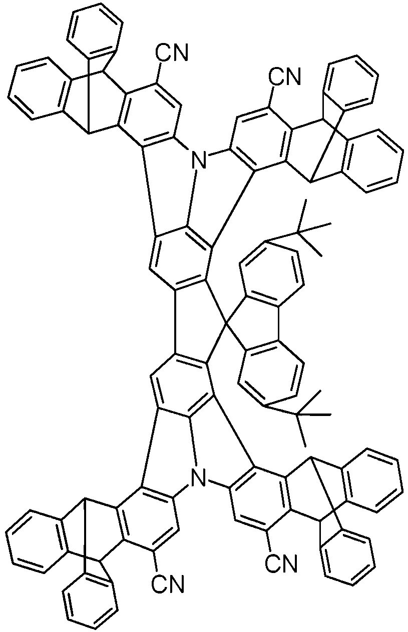 | 12 % |
| | 25 mmol | | |
| ES69 | 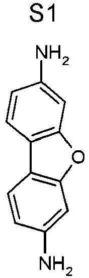 5896-30-0 | 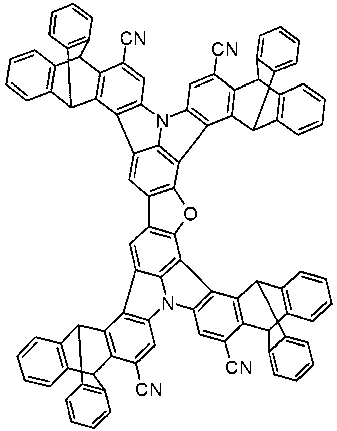 | 21 % |
| | 25 mmol | | |
| ES70 | 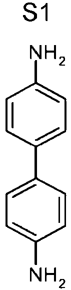 92-87-5 | 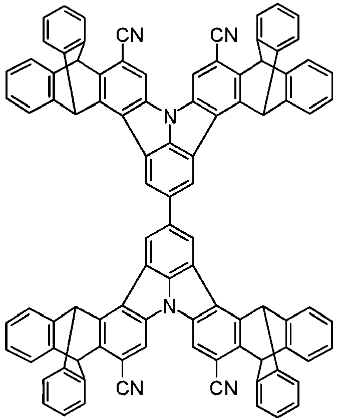 | 20 % |
| | 25 mmol | | |
| ES71 | 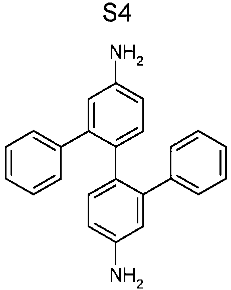 167559-51-5 | 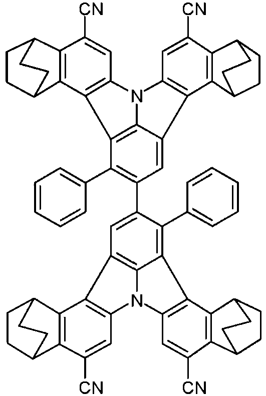 | 18 % |
| | 25 mmol | | |

### 2.2) Synthese der unsymmetrisch substituierten Emitter EAS:

### Beispiel EAS1

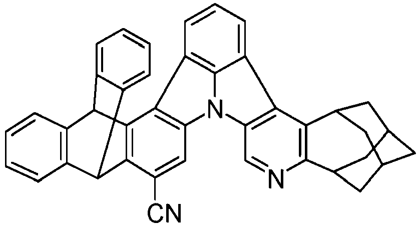

### Variante 1:

Durchführung analog folgender Literatur:
T. Kader et al., Chem. Eur. J., 2019, 25, 4412 - 4425.

Ein Gemisch aus 37.5 g (50 mmol) A500, 27.6 g (200 mmol) Kaliumcarbonat, 1.72 g (3 mmol) (NHC)Pd(allyl)CI [478980-03-9], 50 g Glaskugeln (3 mm Durchmesser) und 500 ml N,N,-Dimethylacetamid (DMAc) wird unter gutem Rühren 16 h auf 140 °C erhitzt. Nach Erkalten auf 80 °C tropft man 1000 ml Wasser zu, saugt vom ausgefallenen Feststoff ab, wäscht diesen zweimal mit je 100 ml Wasser, zweimal mit je 50 ml Methanol und trocknet im Vakuum. Das Rohprodukt wird flash-chromatographiert (Combi-Flash Torrent der Fa. A. Semrau, DCM: 2 % MeOH), wodurch auch auftretende Isomere aufgetrennt werden. Abschließend werden die so erhaltenen Emitter durch wiederholte Heißextraktionskristallisation (DCM:Acetonitril 1:3 bis 2:1) und nachfolgende fraktionierte Sublimation oder durch Tempern im Hochvakuum gereinigt. Ausbeute: 14.0 g (25 mmol), 50 %; Reinheit: > 99.9 % ig n. HPLC.

Anstelle des (NHC)Pd(allyl)Cl kann auch 4 mmol [(tBu)₃PH][BF₄] und 2 mmol Pd(OAc)₂ eigesetzt werden.

Analog zu den Stufen 1.5 und 2.2 können folgende Emitter EAS dargestellt werden, Ausbeute über zwei Stufe:

| Bsp. | Synthone S Amin | Produkt | Ausbeute |
|---|---|---|---|
| EAS2A | 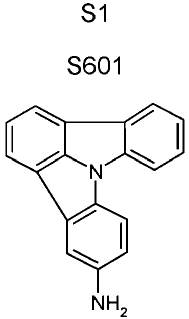 1191512-09-0 | 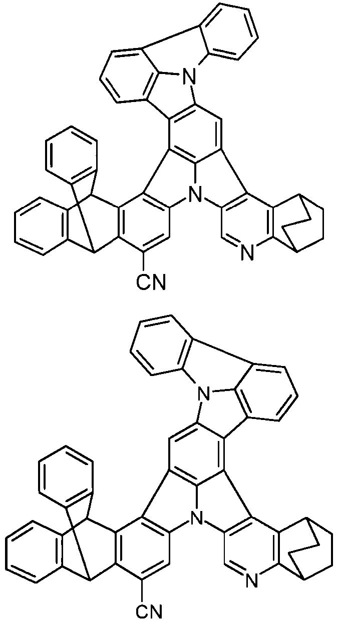 | 17 % |
| EAS2B | | | 17 % |
| EAS3A | 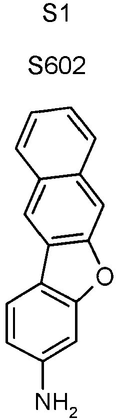 2179038-73-2 | 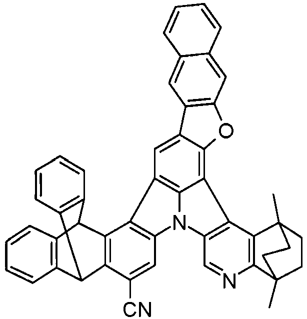 | 19 % |
| EAS3B | | | 17 % |
| | | 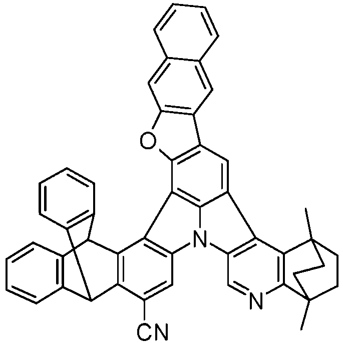 | |
| EAS4A | 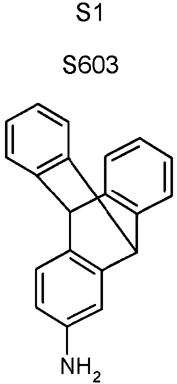 31997-11-2 | 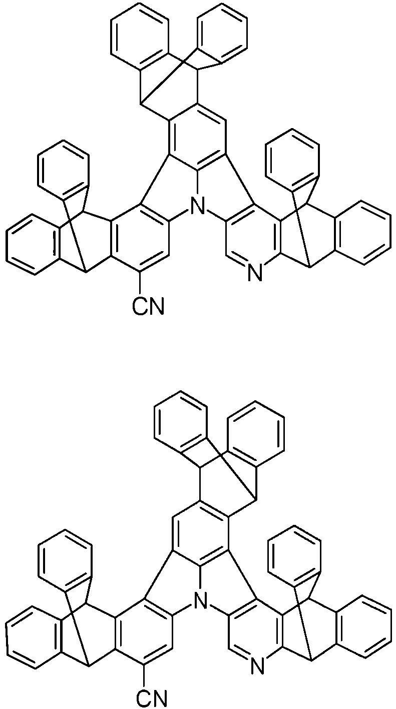 | 19 % |
| EAS4B | | | 15 % |
| EAS5 | 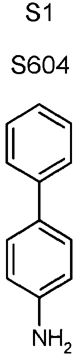 92-67-1 | 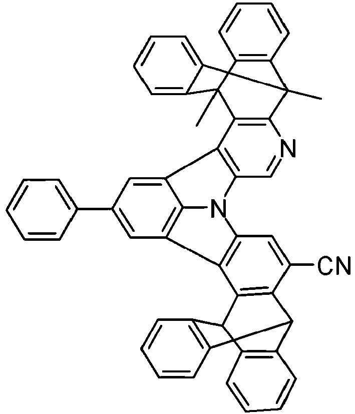 | 33 % |
| EAS6 | 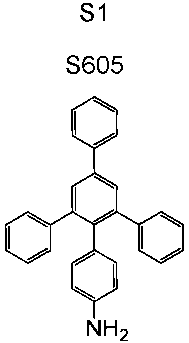 1093882-02-0 | 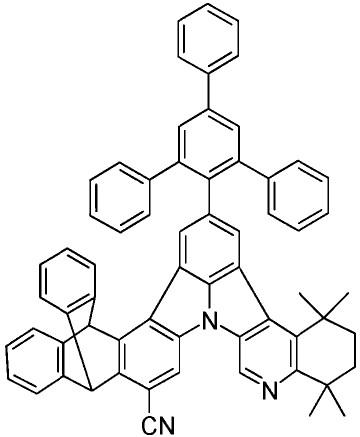 | 30 % |
| EAS7 | 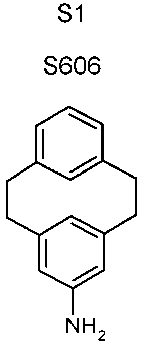 43215-86-7 | 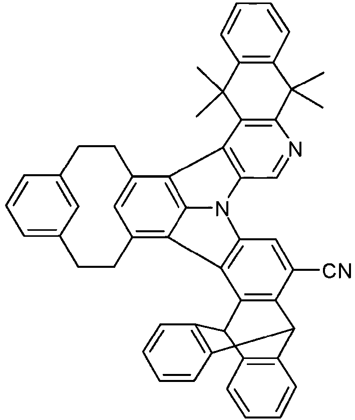 | 35 % |
| EAS8 | 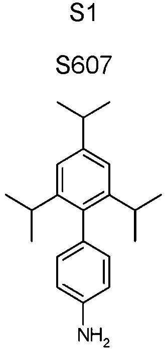 1801716-11-9 | 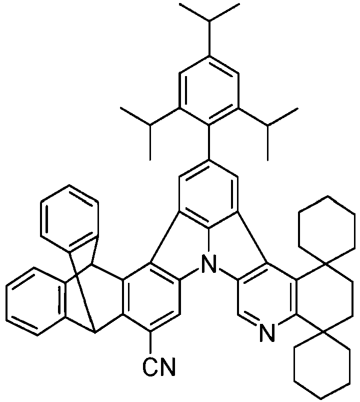 | 30 % |
| EAS9 | 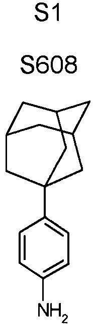 1459-48-9 | 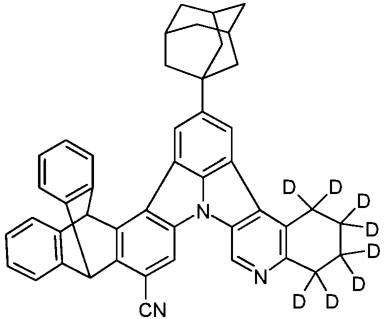 | 32 % |
| EAS10 | 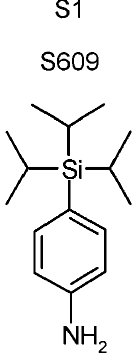 1346517-64-3 | 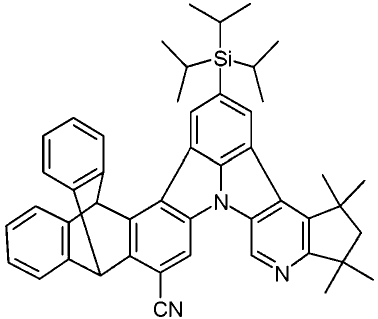 | 22 % |
| EAS11 | 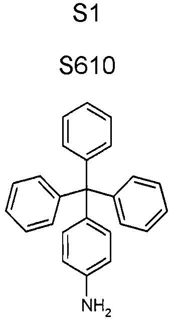 22948-06-7 | 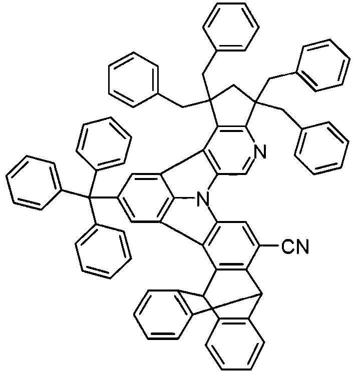 | 33 % |
| EAS12 | 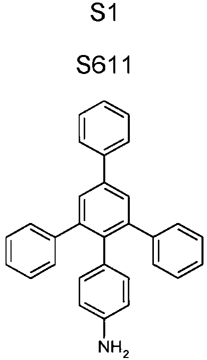 1093882-02-0 | 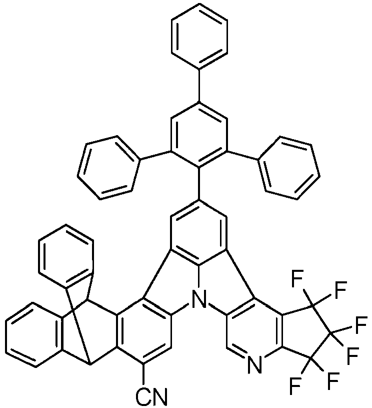 | 25 % |
| EAS13 | 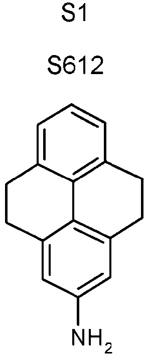 101283-00-5 | 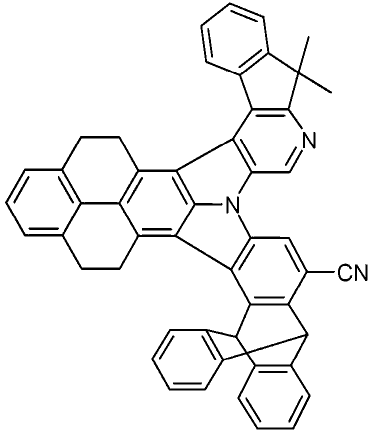 | 39 % |
| EAS14 | 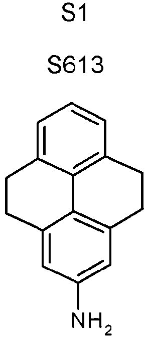 101283-00-5 | 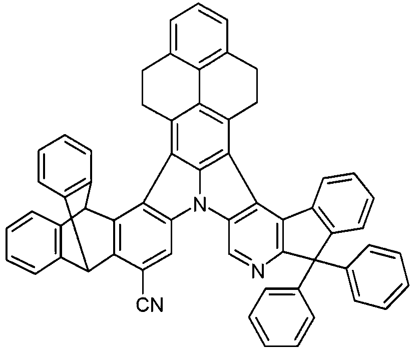 | 38 % |
| EAS 15A | 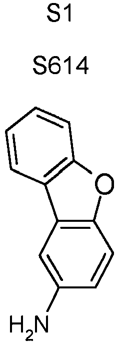 3693-22-9 | 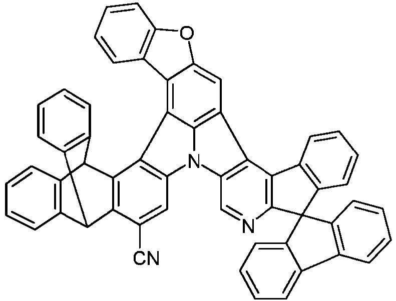 | 17 % |
| EAS15B | | 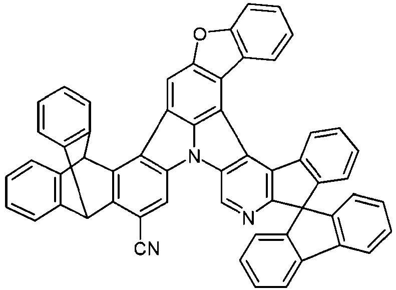 | 19 % |
| EAS16 | 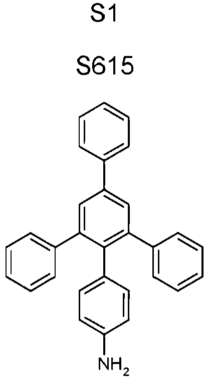 1093882-02-0 | 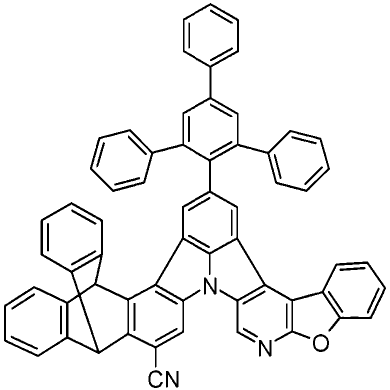 | 37 % |
| EAS17 | 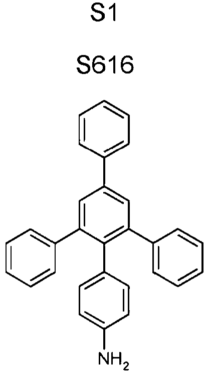 1093882-02-0 | 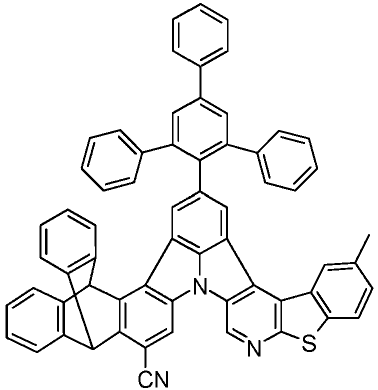 | 37 % |
| EAS18 | 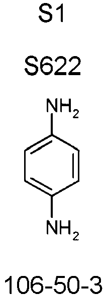 25 mmol | 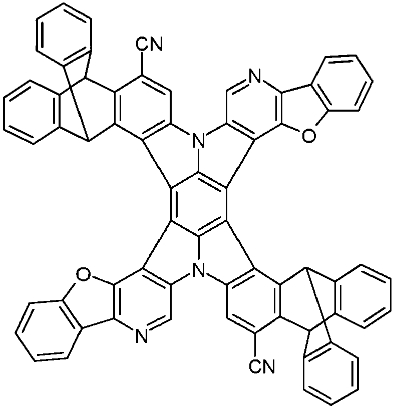 | 22 % |
| EAS19 | 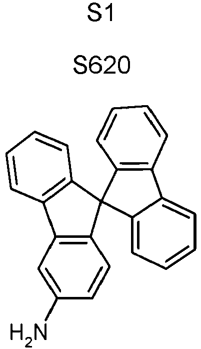 1644466-73-8 | 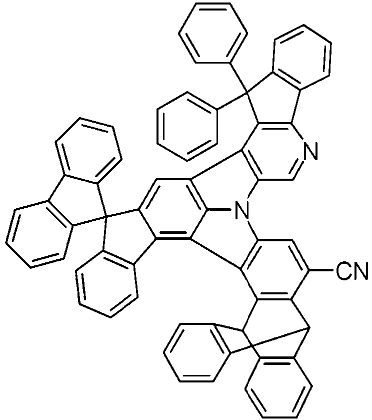 | 20 % |
| EAS20 | 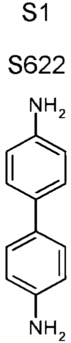 92-87-5 25 mmol | 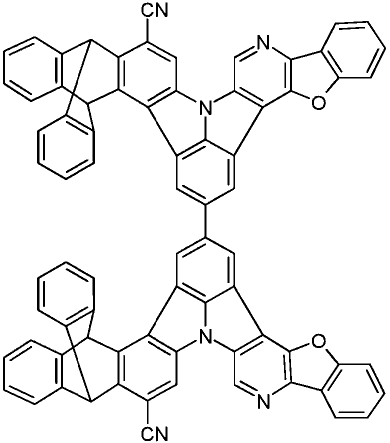 | 31 % |
| EAS21A | 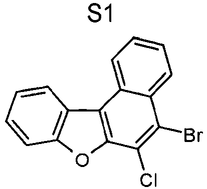 2445776-20-3 | 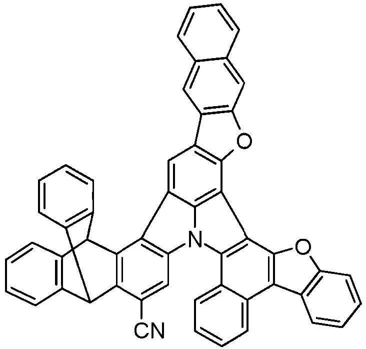 | 20 % |
| EAS21B | 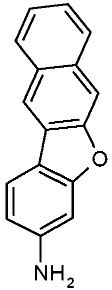 2179038-73-2 | 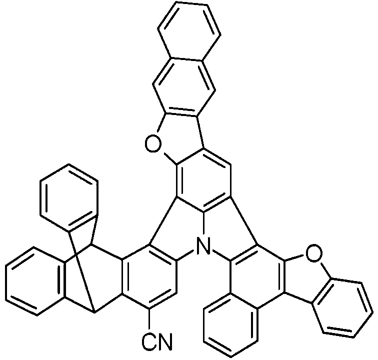 | 15 % |
| EAS22 | 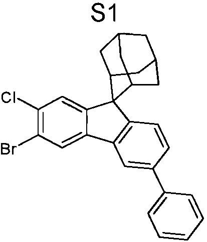 2500975-91-5 | 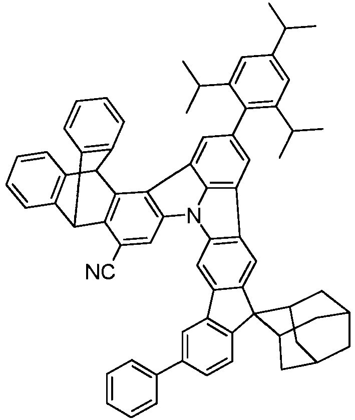 | 36 % |
| | 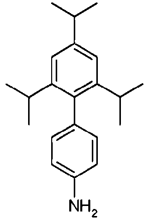 1801716-11-9 | | |
| EAS23A | 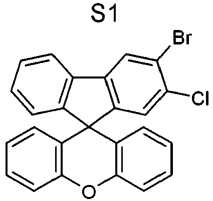 2492439-24-2 | 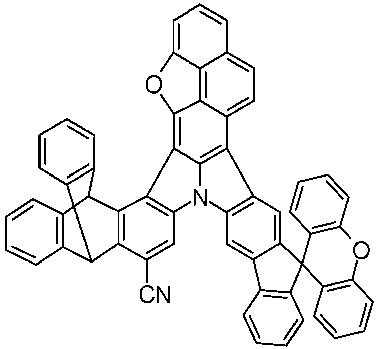 | 18% |
| EAS23B | 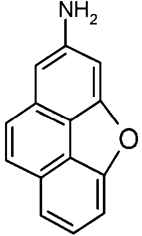 2018346-63-7 | 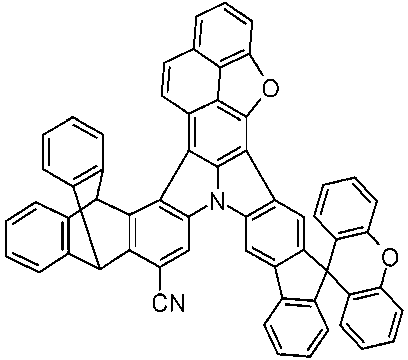 | 19 % |
| EAS24A | 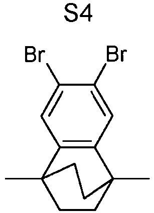 1801624-64-5 | 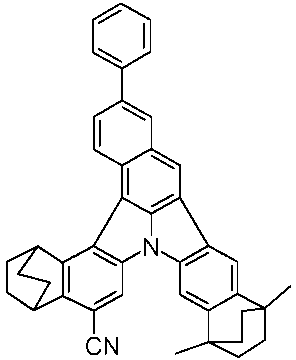 | 19 % |
| EAS24B | 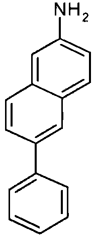 861046-41-5 | 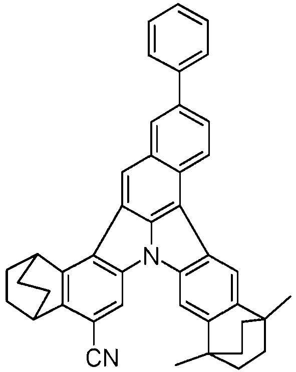 | 20% |
| EAS25 | 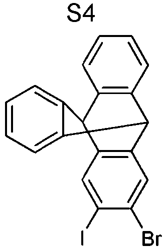 52776-04-2 | 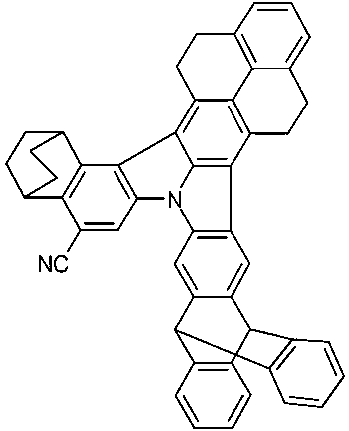 | 34 % |
| | 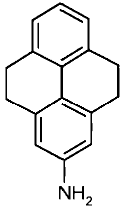 101283-00-5 | | |
| EAS26 | 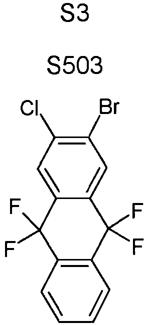 2304436-80-2 | 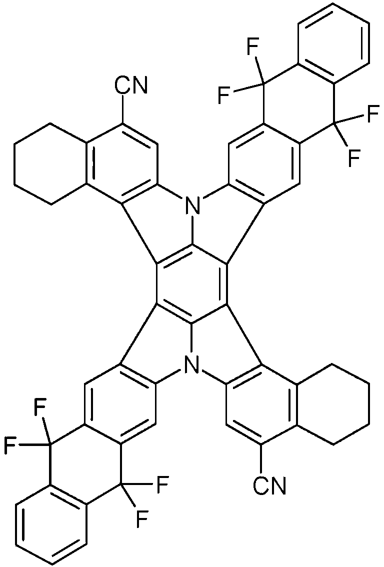 | 23 % |
| | 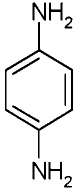 106-50-3 | | |
| | 25 mmol | | |
| EAS27 | 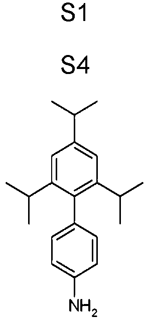 1801716-11-9 | 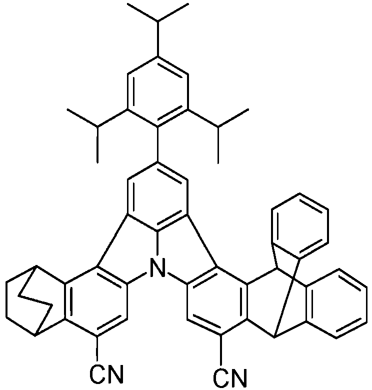 | 35 % |
| EAS28 | 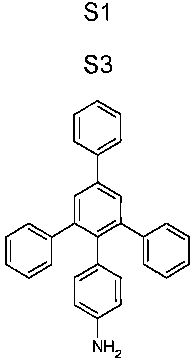 1093882-02-0 | 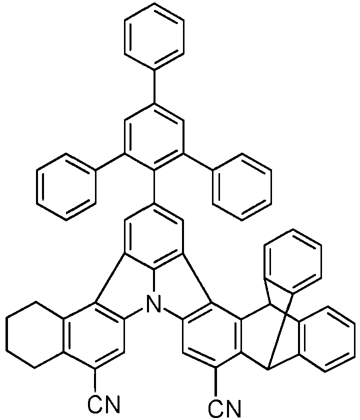 | 34 % |
| EAS29 | 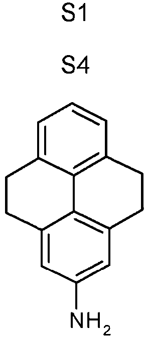 101283-00-5 | 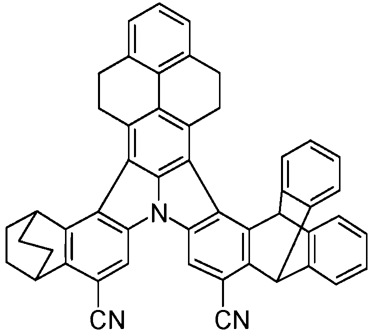 | 33 % |
| EAS30A | 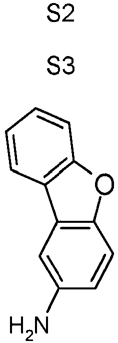 3693-22-9 | 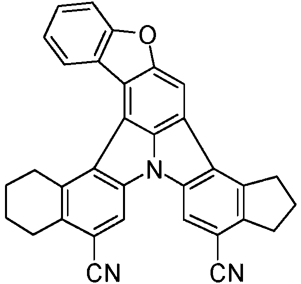 | |
| EAS30B | | 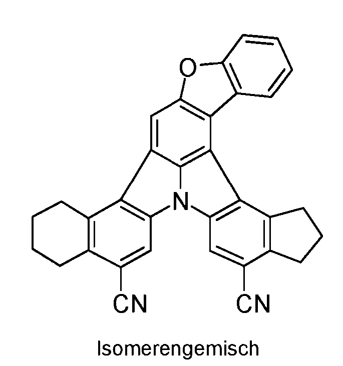 | 34 % |
| ESA31 | 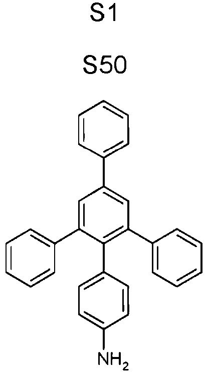 1093882-02-0 | 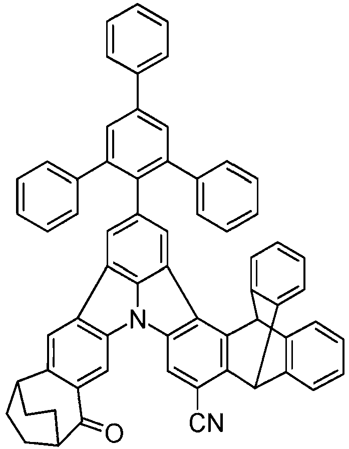 | 30 % |
| EAS32A | 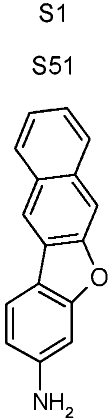 2179038-73-2 | 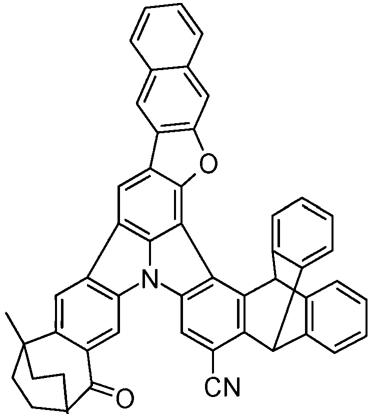 | 19 % |
| EAS32B | | 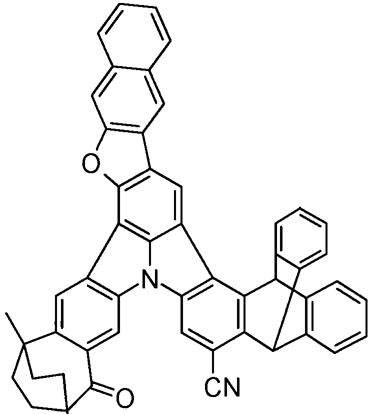 | 17 % |
| EAS33A | S1 | 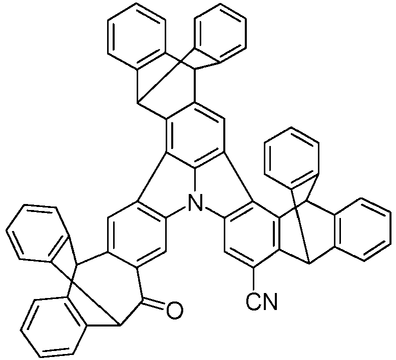 | 19 % |
| | S57 | | |
| EAS33B | 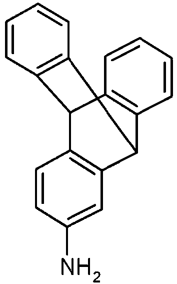 31997-11-2 | 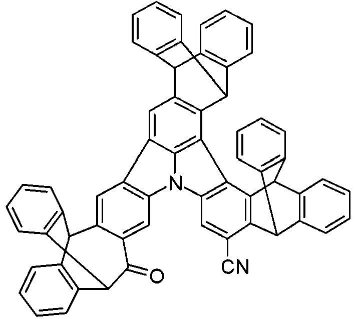 | 16 % |
| EAS34 | 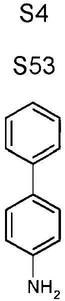 92-67-1 | 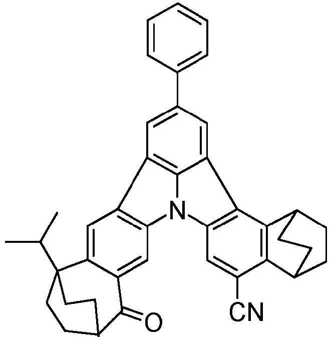 | 35 % |
| EAS35 | 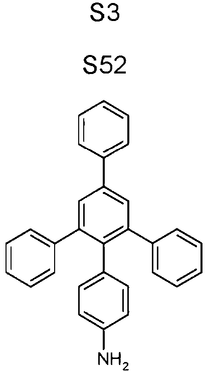 1093882-02-0 | 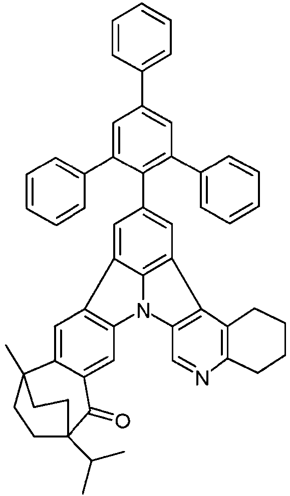 | 35% |
| EAS36 | 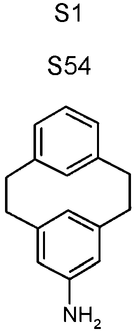 43215-86-7 | 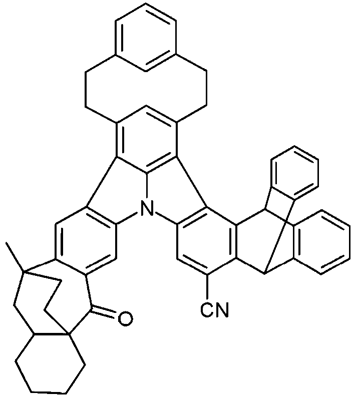 | 30 % |
| EAS37 | 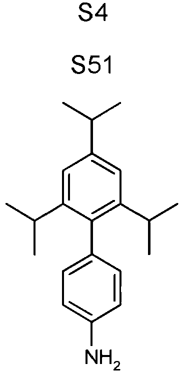 1801716-11-9 | 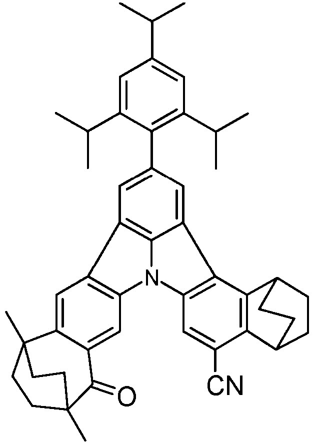 | 36 % |
| EAS38 | 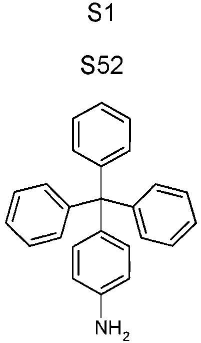 22948-06-7 | 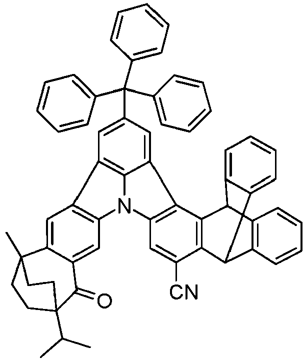 | 35 % |
| EAS39 | 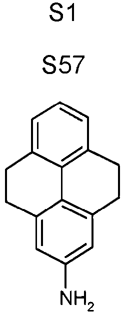 101283-00-5 | 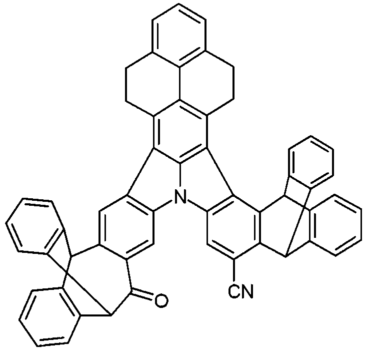 | 37 % |
| EAS40A | 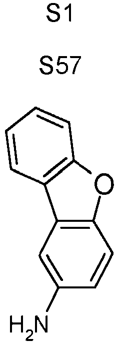 3693-22-9 | 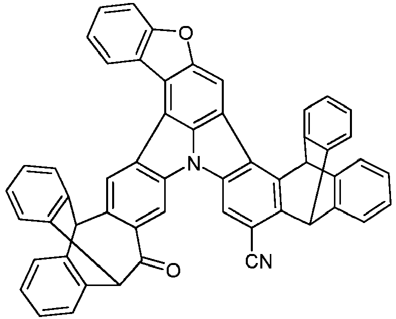 | 19 % |
| EAS40B | | 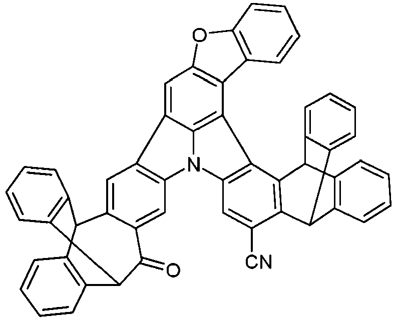 | 21 % |
| EAS41 | 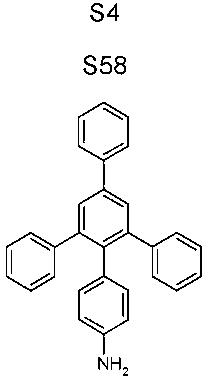 1093882-02-0 | 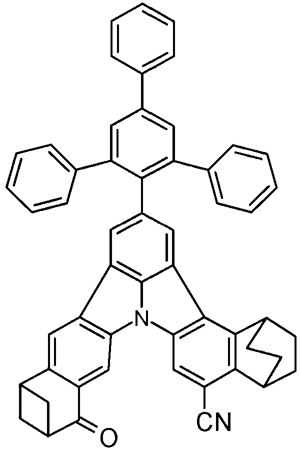 | 35 % |
| EAS42 | 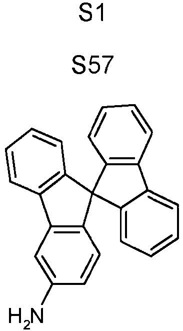 1644466-73-8 | 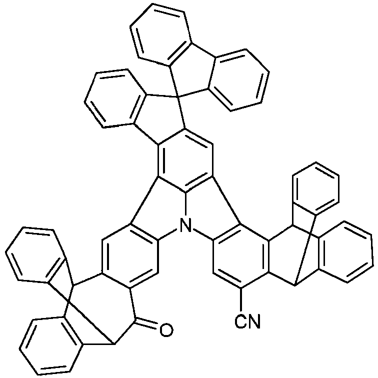 | 20 % |
| EAS43 | 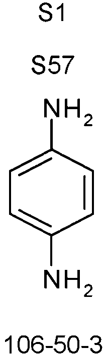 25 mmol | 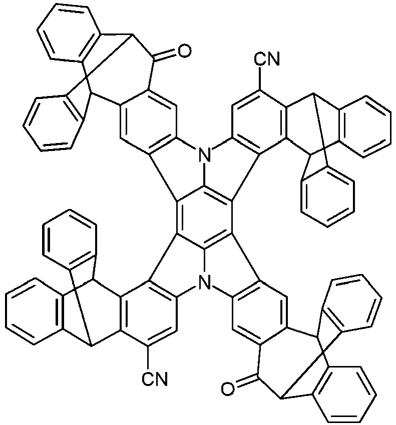 | 25 % |
| EAS44 | 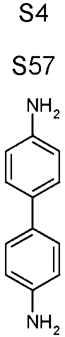 92-87-5 | 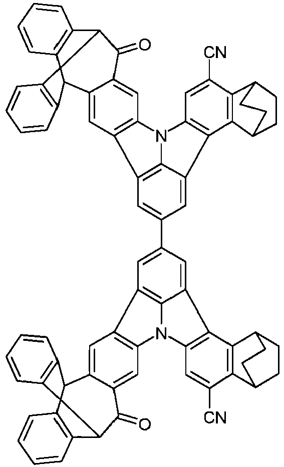 | 28 % |
| | 25 mmol | | |

### Alternative Synthesewege:

Die erfindungsgemäßen Verbindungen können, zum Teil mit verbesserten Ausbeuten, auf folgenden alternativen Synthesewegen dargestellt werden:

### 2.3) Alternatives Verfahren A:

Stufenweiser Aufbau durch zwei konsekutiver Buchwald-Kopplungen gefolgt von einer Pd-katalysierten, intramolekularen Cyclisierung am Beispiel ES1:

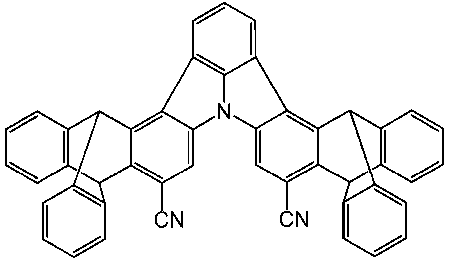

### Stufe 1): Buchwald-Kupplung 1

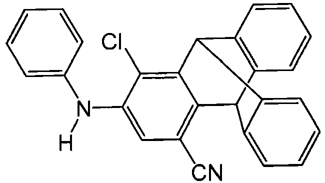

Ein Gemisch aus 43.7 g (100 mmol) S10, 9.13 ml (100 mmol) Anilin [62-53-3], 20.2 g (210 mmol) Natrium-tert-butanolat [865-48-5], 1.11 g (2 mmol) Bis-diphenylphosphino-ferrocen (dppf) [12150-46-8], 499 mg (2 mmol) Palladium(II)acetat, 500 ml Toluol und 50 g Glaskugeln (3 mm Durchmesser) wird bis zum vollständigen Umsatz (ca. 1 h) bei unter schwachem Rückfluss gerührt. Man lässt die Reaktionsmischung auf 60 °C erkalten, versetzt mit 300 ml Wasser, trennt die org. Phase ab, wäscht diese einmal mit 300 ml Wasser, einmal mit 300 ml ges. Kochsalzlösung und trocknet über Magnesiumsulfat. Man filtriert über ein mit Toluol vorgeschlämmtes Kieselgelbett ab, wäscht mit 500 ml Ethylacetat nach und engt das Filtrat zur Trockene ein. Der Rückstand wird chromatographisch gereinigt (Kieselgel, Cyclohexan/EE, Combi-Flash Torrent der Fa. A. Semrau). Ausbeute: 32.7 g (81 mmol), 81 %; Reinheit: ca. 97 % ig n. ¹H-NMR.

### Stufe 2: Buchwald-Kupplung 2

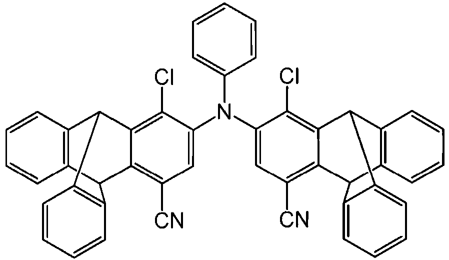

Ein Gemisch aus 40.4 g (100 mmol) Stufe 1), 43.7 g (110 mmol) S10, 20.2 g (210 mmol) Natrium-tert-butanolat [865-48-5], 725 mg (2.5 mmol) Tri-tert-butylphosphonium tetrafluoroborat [131274-22-1], 449 mg (2 mmol) Palladium(II)acetat, 500 ml Toluol und 50 g Glaskugeln (3 mm Durchmesser) wird bis zum vollständigen Umsatz (ca. 12 h) bei unter schwachem Rückfluss gerührt. Man lässt die Reaktionsmischung auf 60 °C erkalten, versetzt mit 300 ml Wasser, trennt die org. Phase ab, wäscht diese einmal mit 300 ml Wasser, einmal mit 300 ml ges. Kochsalzlösung und trocknet über Magnesiumsulfat. Man filtriert über ein mit Toluol vorgeschlämmtes Kieselgelbett ab, wäscht mit 500 ml Ethylacetat nach und engt das Filtrat zur Trockene ein. Der Rückstand wird chromatographisch gereinigt (Kieselgel, Cyclohexan/EE, Combi-Flash Torrent der Fa. A. Semrau). Ausbeute: 55.6 g (77 mmol), 77 %; Reinheit: ca. 97 % ig n. ¹H-NMR.

### Stufe 3): Cyclisierung

Ein Gemisch aus 71.7 g (100 mmol) Stufe 2), 41.5 g (300 mmol) Kaliumcarbonat, 725 mg (2.5 mmol) Tri-tert-butylphosphonium tetrafluoroborat [131274-22-1], 449 mg (2 mmol) Palladium(II)acetat, 1000 ml Dimethylacetamid und 50 g Glaskugeln (3 mm Durchmesser) wird bis zum vollständigen Umsatz (ca. 12 h) bei 150 °C gerührt. Nach Erkalten auf 80 °C tropft man 2000 ml Wasser zu, saugt vom ausgefallenen Feststoff ab, wäscht diesen zweimal mit je 200 ml Wasser, zweimal mit je 50 ml Methanol und trocknet im Vakuum. Das Rohprodukt wird flashchromatographiert (RP-Kieselgel, Acetonitril/THF, Combi-Flash Torrent der Fa. A. Semrau), anschließend durch wiederholte Heißextraktionskristallisation (DCM:Acetonitril 1:3 bis 3:1) und nachfolgende fraktionierte Sublimation oder durch Tempern im Hochvakuum gereinigt. Ausbeute: 36.5 g (56 mmol), 56 %; Reinheit: > 99.9 % ig n. HPLC.

Das alternative Verfahren A eignet sich nicht nur zum Aufbau von symmetrisch substituierten Emittern, sondern speziell auch zum Aufbau von asymmetrisch substituierten Emittern, durch Einsatz von zwei verschiedenen lod-Chlor-Benzonitrilen in Stufe 1) und Stufe 2).

Analog können folgende Verbindungen dargestellt werden:

| Bsp. | Edukte | Produkt | Ausbeute |
|---|---|---|---|
| ES72 | 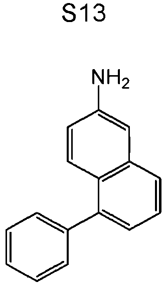 1609130-36-0 | 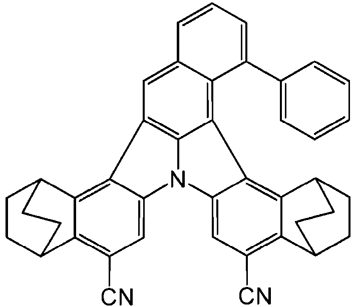 | 13 % |
| ES73 | 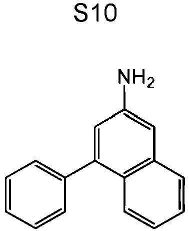 228107-17-3 | 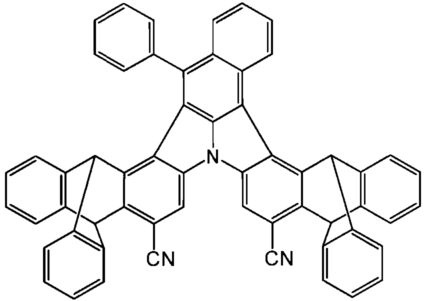 | 37 % |
| ES74 | 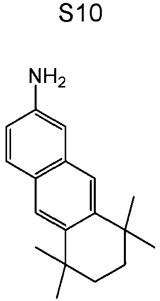 199392-14-8 | 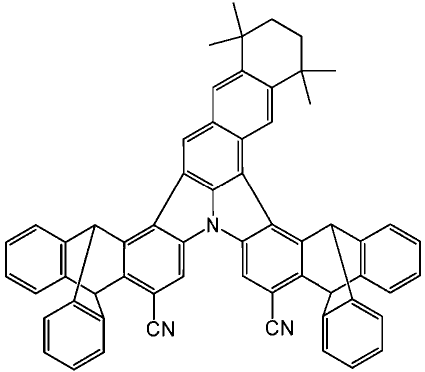 | 38 % |
| ES75 | 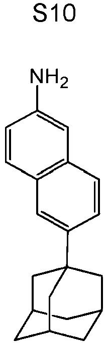 37521-66-7 | 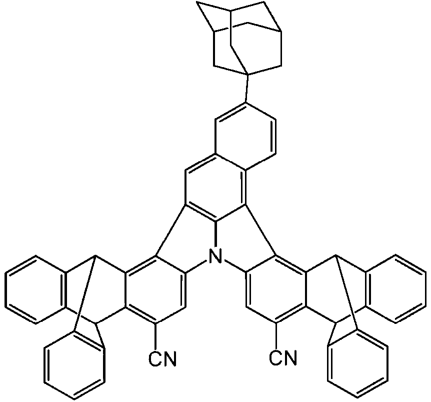 | 34 % |
| ES76 | 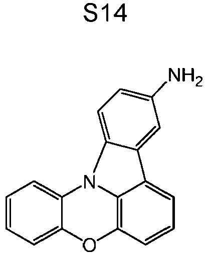 2268818-23-9 | 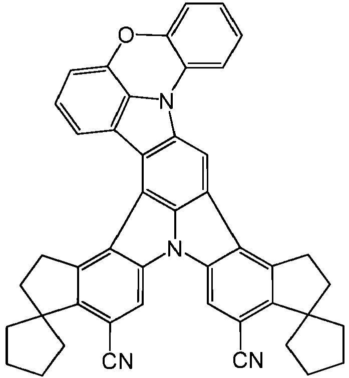 | 29 % |
| ES77 | 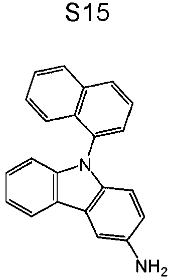 2097255-51-9 | 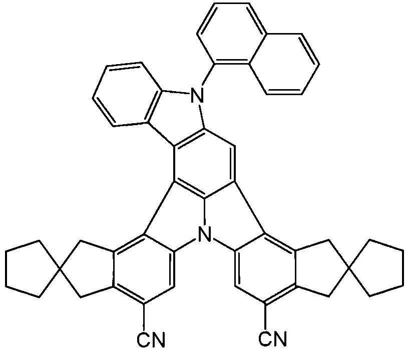 | 35 % |
| ES78 | 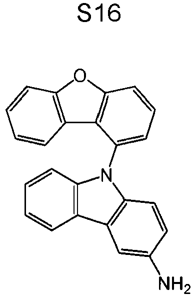 2609787-30-4 | 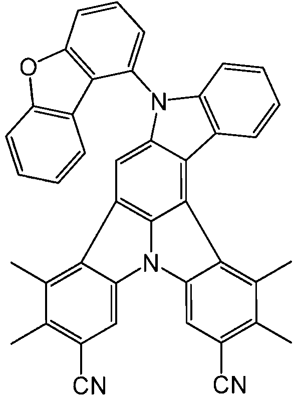 | 41 % |
| ES79 | 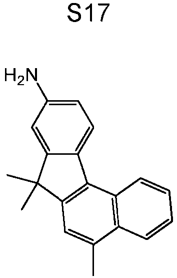 2379812-68-5 | 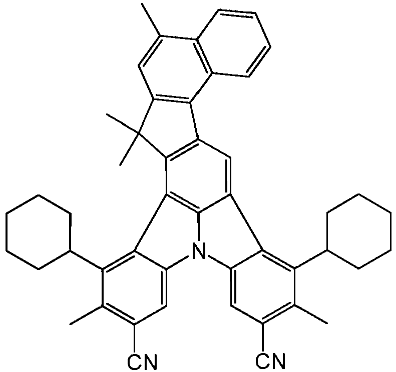 | 30 % |
| EAS45A | S10 Stufe 1 | 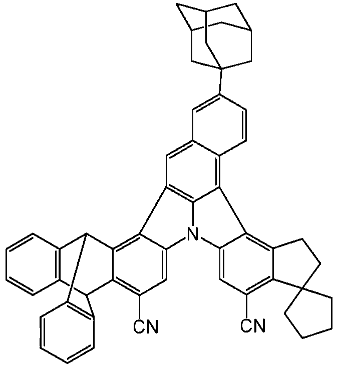 | 19 % |
| | S14 Stufe 2 | | |
| | 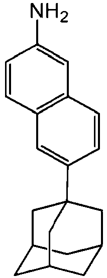 | | |
| EAS45B | 37521-66-7 | | 10 % |
| | | 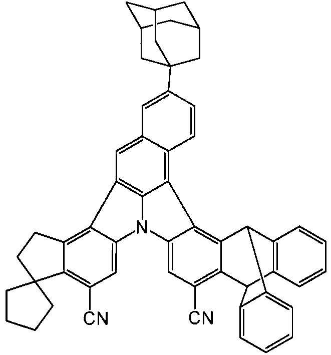 | |

### 2.4) Alternatives Verfahren B:

### Stufenweise Aminierung - Cyclisierung via eines Carbazol Intermediats:

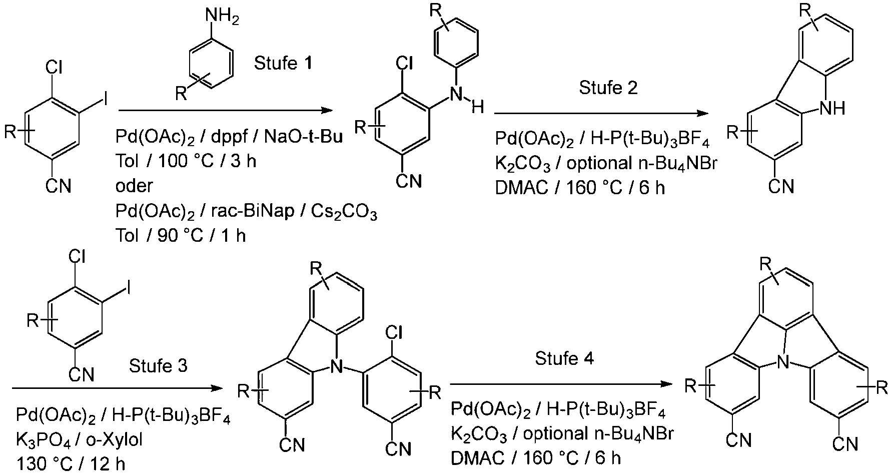

Stufe 1: Standard Buchwald-Kupplungs-Verfahren zur Darstellung von sec. Aminen aus einem Anilin und dem lod-Chlor-Benzonitril, z.B. analog U. Masanobu, et al., J. Am. Chem. Soc., 2004, 126(28), 8755 oder P. B. Tiruveedhula, et al., Org. & Biomol. Chem., 2015, 13(43), 10705. Typische Ausbeuten 70 - 95 %.

Stufe 2: Intramolekulare Cyclisierung zum Carbazol analog P. B. Tiruveedhula, et al., Org. & Biomol. Chem., 2015, 13(43), 10705 oder F. Chen et al., RSC Adv., 2015, 5, 51512. Beim Einsatz unsymmtrisch substituierter Aniline werden die regioisomeren Carbazole als Gemisch isoliert und weiter umgesetzt. Typische Ausbeuten 60 - 90 %.

Stufe 3: Standard-Buchwald-Kupplungs-Verfahren zur Darstellung von N-arylierten Carbazolen, alternativ kann eine Ullmann-Kupplung durchgeführt werden z.B. analog J. H. Cho et al., Bull. Korean Chem. Soc., (2011), 32(7), 2461. Typische Ausbeuten 40 - 80 %.

Stufe 4: Intramolekulare Cyclisierung, analog Stufe 2 z.B. analog T. Kader et al., Chem. Europ. J., 2019, 25(17), 4412 oder analog US 9,000,421 B1 unter Einsatz von Tricyclohexylphosphonium tetrafluoroborat bzw. mit NHC-Pd-Komplexen wie z.B. Allyl-[1,3-bis-(2,6-diisopropylphenyl)-imidazol-2-yliden]-chloropalladium(II). Typische Ausbeuten 50-80 %.

Alternativ können die Carbazol-Intermediate wie folgt aufgebaut werden:

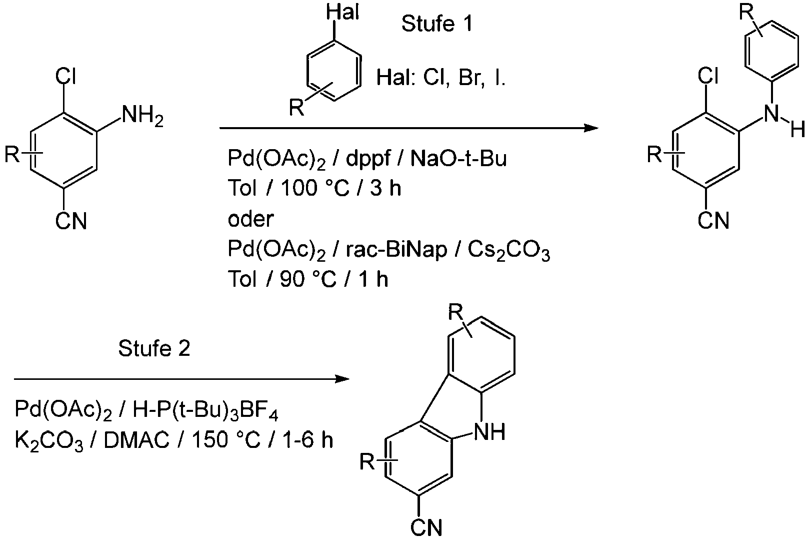

Stufe 1: Standard Buchwald-Kupplungs-Verfahren zur Darstellung von sec. Aminen aus einem Anilin und dem lod-Chlor-Benzonitril, z.B. analog U. Masanobu, et al., J. Am. Chem. Soc., 2004, 126(28), 8755 oder P. B. Tiruveedhula, et al., Org. & Biomol. Chem., 2015, 13(43), 10705. Typische Ausbeuten 70 - 95 %.

Stufe 2: s. oben.

Bevorzugt kann Stufe 3 auch mit 3-Fluor-4-Triflat- oder 3-Fluor-4-Chlor-Benzonitrilen wie folgt ausgeführt werden:

### Stufe 3

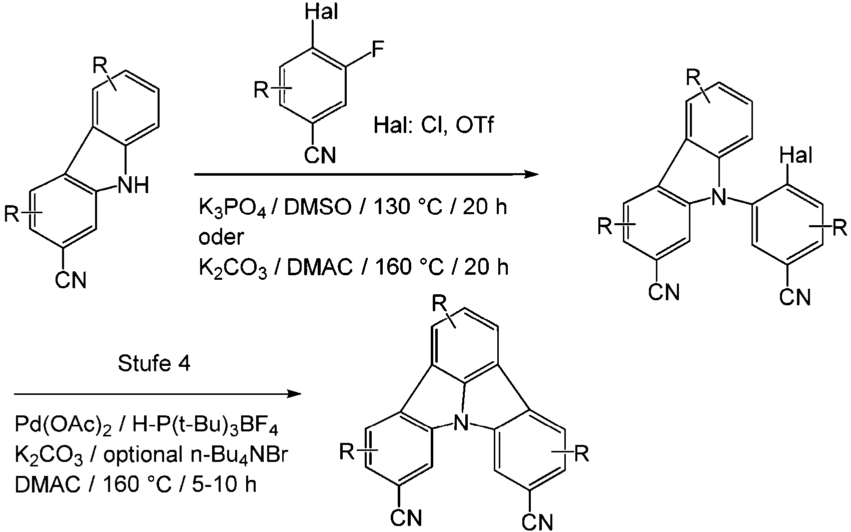

Stufe 3: analog WO2019063288. Typische Ausbeuten 60 - 80 %. Stufe 4: s. oben.

### Optimierte Synthese von ES94:

### Stufe 1:

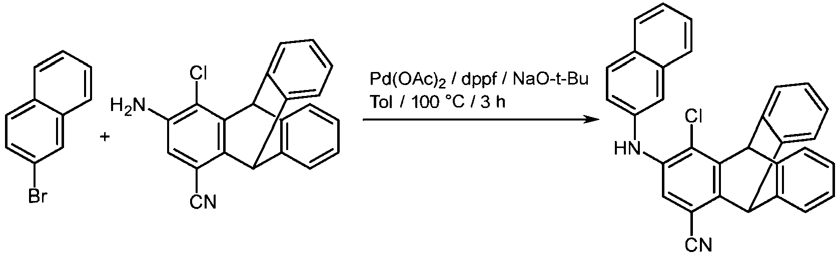

Ein gut gerührtes Gemisch aus 32.9 g (100 mmol) 1-Cyano-3-amino-4-chlor-triptycen (s. S. 228), 20.7 g (100 mmol) 2-Brom-naphthalin, 28.8 g (300 mmol) Natrium-tert-butylat, 1.11 g (2 mmol) dppf, 225 mg (1 mmol) Palladium(II)acetat in 500 ml Toluol wird 1 h unter Rückfluss erhitzt. Man lässt auf 70 °C erkalten, gibt 500 ml Wasser zu, rührt 10 min. nach, trennt die org. Phase ab, wäscht diese zweimal mit je 300 ml Wasser, einmal mit 300 ml ges. Kochsalzlösung und trocknet über Magnesiumsulfat. Man filtriert über ein mit Toluol vorgeschlämmtes Celite-Bett ab, engt das Filtrat im Vakuum ein, löst den Rückstand in 300 ml DCM und entfrent dieses im Vakuum, wobei das abdestillierte DCM durch simultane Zugabe von EtOH subsituiert wird. Man saugt von auskristallisierten Produkt ab, wäscht dieses dreimal mit je 50 ml EtOH und trocknet im Vakuum. Ausbeute: 42.3 g (93 mmol) 93 %; Reinheit: ca. 98 % ig nach ¹H-NMR.

### Stufe 2:

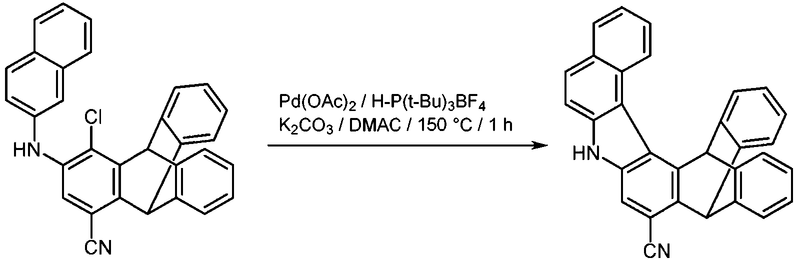

Ein gut gerührtes Gemisch aus 45.4 g (100 mmol) des Amins, 500 mmol Kaliumcarbonat, 1.16 g (4 mmol) Tri-tert-butylphosphonium-tetrafluroborat, 449 mg (2 mmol) Palladium(II)acetat, 100 g Glaskugeln (3 mm Durchmesser) und 1000 ml Dimethylacetamid (DMAC) wird 1 h bei 150 °C gerührt. Man filtriert noch heiß über ein mit DMAC vorgeschlämmtes Clite-Bett ab, engt das Flitrat zur Trockene ein, löst den Rückstand in 500 ml DCM und entfrent dieses im Vakuum, wobei das abdestillierte DCM durch simultane Zugabe von 300 ml EtOH subsituiert wird. Man saugt von auskristallisierten Produkt ab, wäscht dieses dreimal mit je 50 ml EtOH und trocknet im Vakuum. Ausbeute: 32.9 g (78 mmol) 78 %; Reinheit: ca. 98 % ig nach ¹H-NMR.

### Stufe 3 und 4: Eintopfreaktion

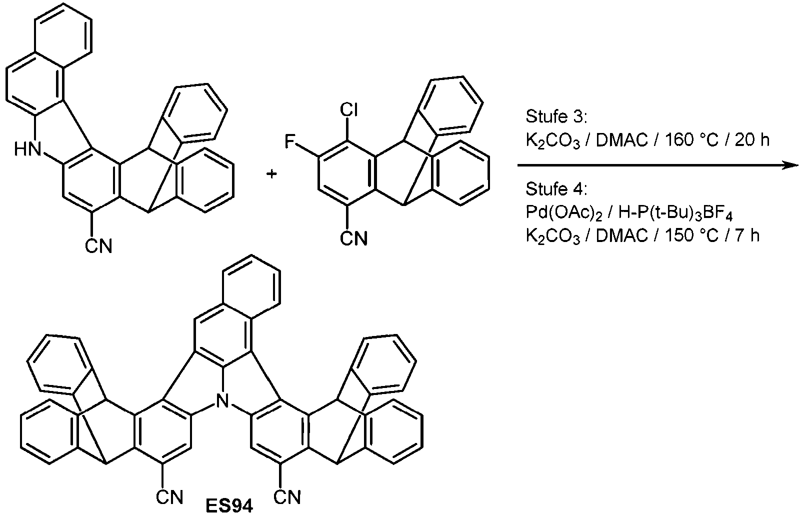

Ein gut gerührtes Gemisch aus 20.9 g (50 mmol) des Carbazols, 19.6 g (50 mmol) S1e, 34.6 g (250 mmol) Kaliumcarbonat, 100 g Glaskugeln (3 mm Durchmesser), 500 ml DMAC wird 20 h bei 150 °C gerührt. Man lässt die Reaktionsmischung auf RT abkühlen, gibt 1.16 g (4 mmol) Tri-tert-butylphosphonium-tetrafluroborat und 449 mg (2 mmol) Palladium(II)-acetat zu und rührt erneut 7 h bei 150 °C. Man filtriert noch heiß über ein mit DMAC vorgeschlämmtes Clite-Bett ab, engt das Flitrat zur Trockene ein, löst den Rückstand in 500 ml DCM und entfrent dieses im Vakuum, wobei das abdestillierte DCM durch simultane Zugabe von 300 ml EtOH subsituiert wird. Man saugt von auskristallisierten Rohprodukt ab, wäscht dieses dreimal mit je 50 ml EtOH und trocknet im Vakuum. Das Rohprodukt wird durch fünfmalige Heißextraktionskristallisation (DCM:Acetonitril 2:1) und nachfolgende fraktionierte Sublimation im Hochvakuum (T ∼ 300 °C, p ∼ 10⁻⁵ mbar) gereinigt. Ausbeute: 20.0 g (29 mmol), 58 %; Reinheit: > 99.9 % ig n. HPLC.

Das alternative Verfahren B eignet sich nicht nur zum Aufbau von symmetrisch substituierten Emittern, sondern speziell auch zum regiodirektionalen Aufbau von asymmetrisch substituierten Emittern, durch Einsatz von zwei verschiedenen lod-Chlor-Benzonitrilen in Stufe 1) und Stufe 3) bzw. 3-Fluor-4-Triflat- oder 3-Fluor-Chlor-Benzonitril in Stufe 3).

Analog können folgende Verbindungen dargestellt werden:

| Bsp. | Edukte | Produkt | Ausbeute |
|---|---|---|---|
| ES80 | S10 Stufe 1 & 3 | 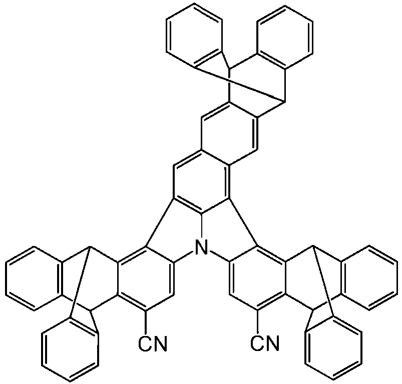 | 30 % |
| | S700 | | |
| ES80 | S10 Stufe 1 | ES80 | 37 % |
| | S1d Stufe 3 | | |
| | S700 | | |
| ES80 | S10 Stufe 1 | ES80 | 54 % |
| | S1e Stufe 3 | | |
| | S700 | | |
| ES81 | S14 Stufe 1 & 3 | 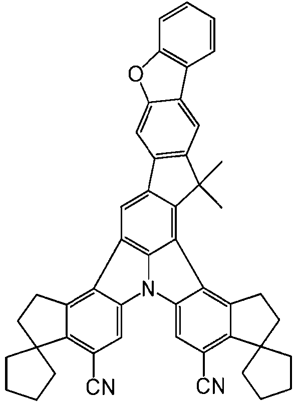 | 28 % |
| | S701 | | |
| ES82 | S10 Stufe 1 & 3 | 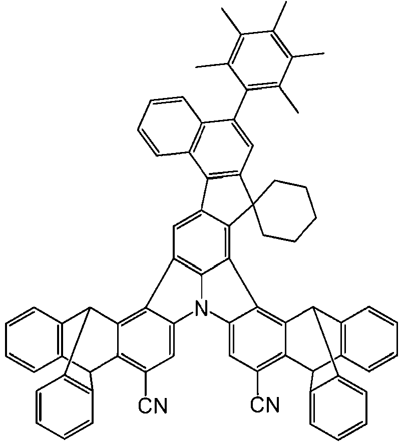 | 20 % |
| | S702 | | |
| ES83 | S10 Stufe 1 & 3 | 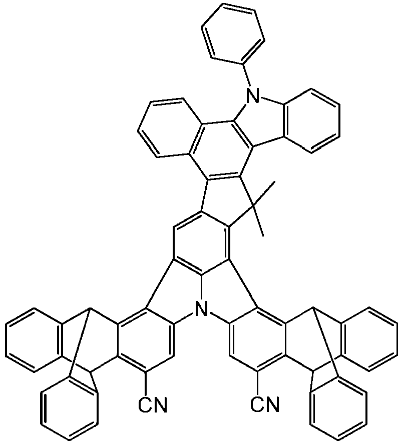 | 27 % |
| | S703 | | |
| ES83 | S10 Stufe 1 | ES83 | 35 % |
| | S1d Stufe 3 | | |
| | S703 | | |
| ES83 | S10 Stufe 1 | ES83 | 52 % |
| | S1e Stufe 3 | | |
| | S703 | | |
| ES84 | S13 Stufe 1 & 3 | 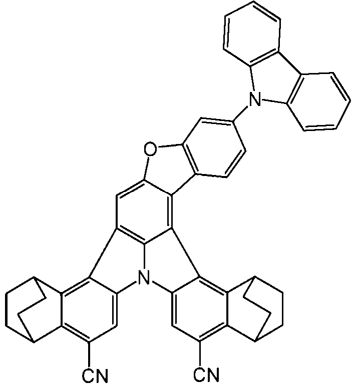 | 29 % |
| | S704 | | |
| ES84 | S13 Stufe 1 | ES84 | 49 % |
| | S4e Stufe 3 | | |
| | S704 | | |
| EAS46A | S10 Stufe 1 | 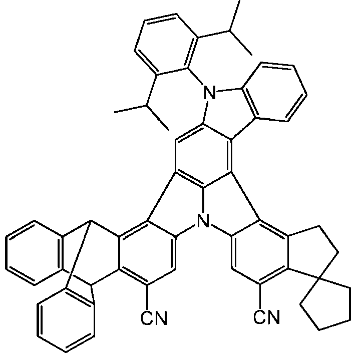 | 19 % |
| EAS46B | S14 Stufe 3 | 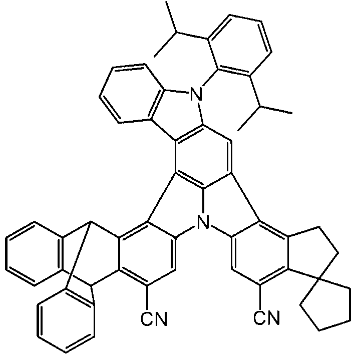 | 6 |
| | S705 | | |

### 2.5) Alternatives Verfahren C:

### Aufbau durch Suzuki-Kupplung von 2,6-Bis-boranyl-anilinen mit den Halogen-Benzonitrilen und anschließende doppelte, cyclisierende Buchwald-Aminierung:

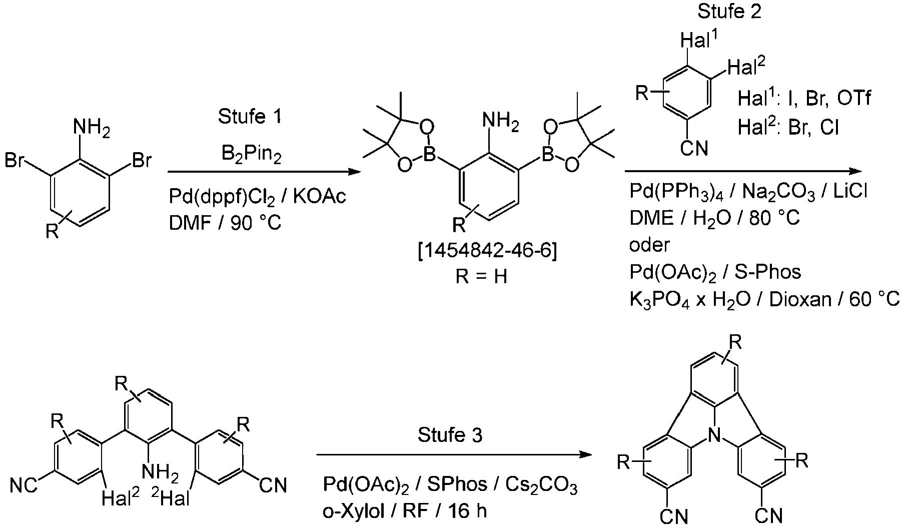

Stufe 1: Borylierung analog A. Osichow et al., Organomet. 2013, 32(18), 5239. Typische Ausbeuten 60 - 90 %.

Stufe 2: Regioselektive Suzuki-Kuppling an den Chlor-Triflaten bzw. Chlor-Bromiden/Iodiden, bevorzugt eingesetzte Hal¹/Hal² Kombinationen sind OTf/Cl oder I/CI oder Br/CI analog M. I. Dawson et al., Journal of Medicinal Chemistry, 2007, 50(11), 2622 bzw. WO2021121371. Typische Ausbeuten 50 - 80 %.

Stufe 3: Cyclisierung analog US 2017/0324045. Typische Ausbeuten 30 - 60 %.

Analog können folgende Verbindungen dargestellt werden:

| Bsp. | Edukte | Produkt | Ausbeute |
|---|---|---|---|
| ES85 | 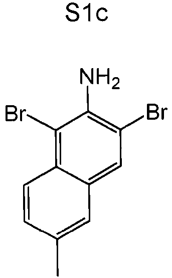 1642-18-8 | 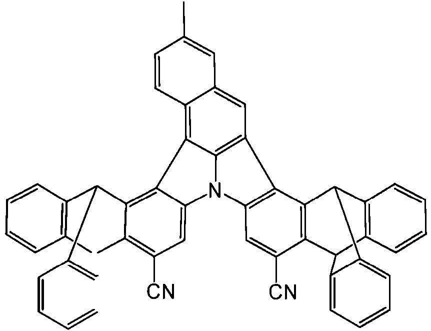 | 31 % |
| ES86 | 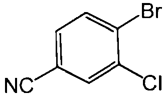 57418-97-0 | 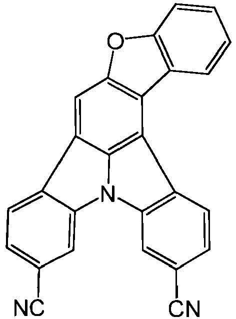 | 34 % |
| | 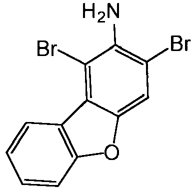 667937-52-2 | | |
| ES87 | 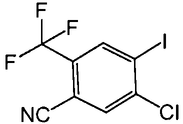 2384544-52-7 | 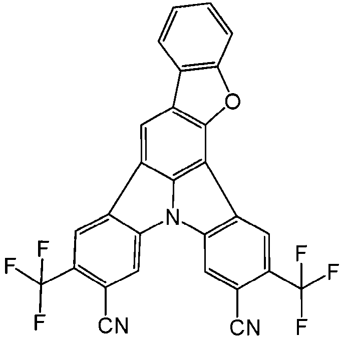 | 36 % |
| | 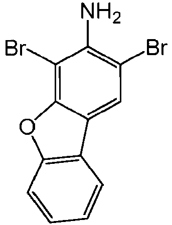 133953-35-2 | | |

### 2.6) Alternatives Verfahren D:

### Aufbau aus 3-Fluor-4-Halogen-Benzonitrilen durch Suzuki-Kupplung und intramolekulare Cyclisierung via S_{N}2Ar Reaktion:

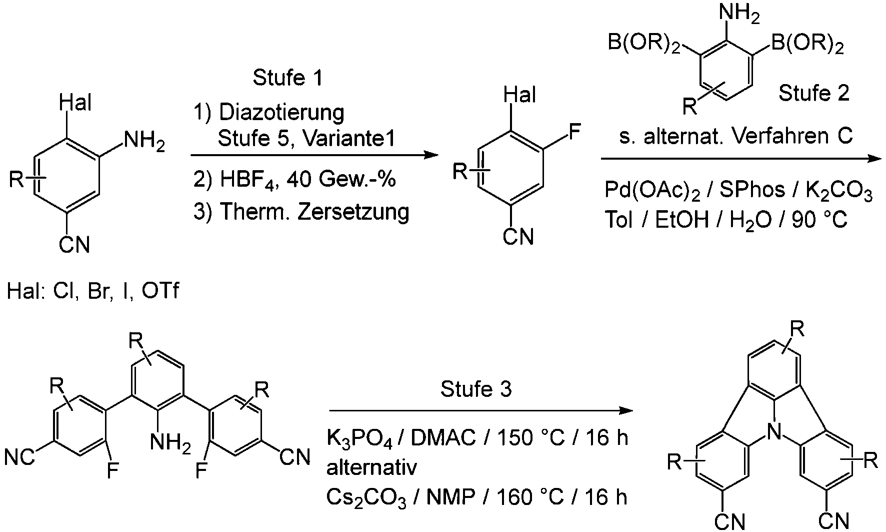

Stufe 1: Balz-Schiemann-Reaktion analog G. Balz et al., Chem. Ber., 1927, 5, 1186 oder via NOBF₄ analog D. J. Milner et al., Synth. Commun., 1992, 22, 73. Siehe auch optimierte Synthese von 1e. Typische Ausbeuten 30 - 85 %.

Stufe 2: Suzuki-Kupplung an den 3-Fluor-4-Halogen-Benzonitrilen. Typische Ausbeuten 40 - 80 %.

Stufe 3: Intramolekulare Cyclisierung via S_{N2}Ar Reaktion z.B. analog CN108727396. Typische Ausbeuten 40 - 80 %.

Analog können folgende Verbindungen dargestellt werden:

| Bsp. | Edukte | Produkt | Ausbeute |
|---|---|---|---|
| ES88 | 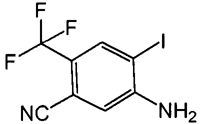 2385618-00-6 | 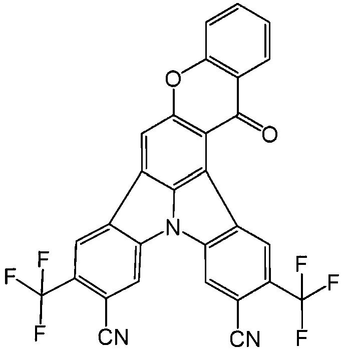 | 34 % |
| | 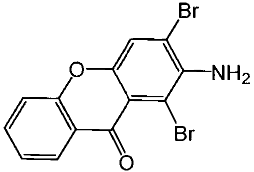 840507-06-4 | | |
| ES89 | S1e | 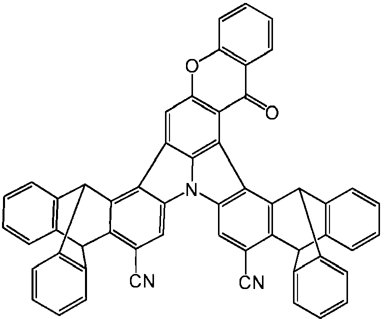 | 14 % |
| | 2385618-00-6 | | |
| | 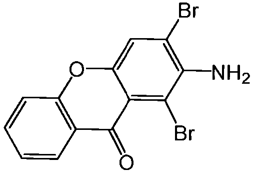 | | |
| ES90 | 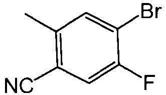 916792-15-9 | 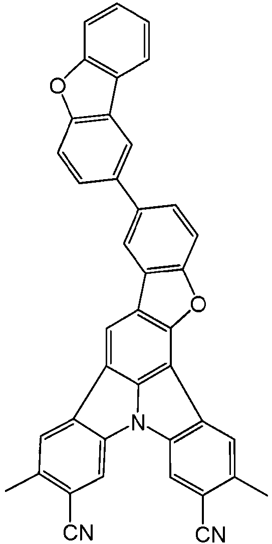 | 32 % |
| | 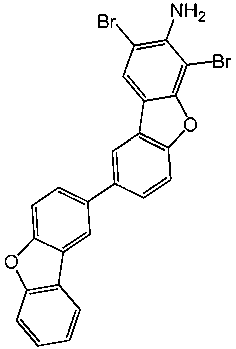 1500101-93-8 | | |

### 2.7) Alternatives Verfahren E:

### Aufbau aus 2,6-Dichloranilinen durch Buchwald-Kupplung und Pd-katalysierte intramolekulare Cyclisierung:

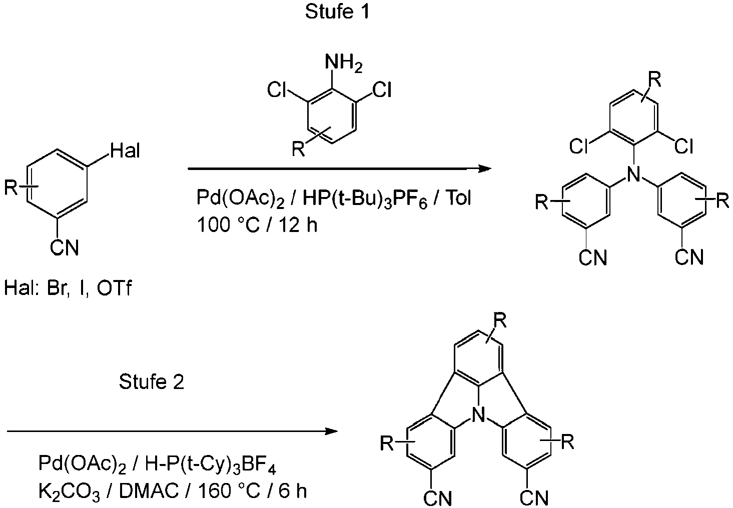

Stufe 1 und Stufe 2: z.B. analog US 2021/0005826. Typische Ausbeuten über beide Stufen 20 - 50 %.

Analog können folgende Verbindungen dargestellt werden:

| Bsp. | Edukte | Produkt | Ausbeute |
|---|---|---|---|
| ES91 | 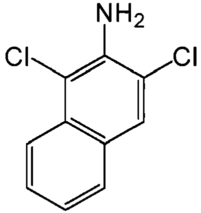 843744-23-0 | 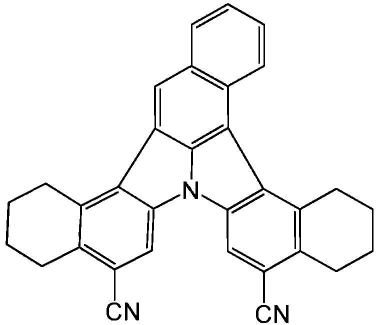 | 48 % |
| | 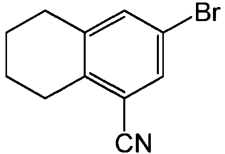 2113577-09-4 | | |
| ES92 | 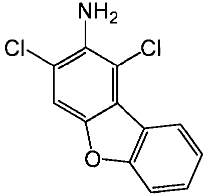 133618-04-9 | 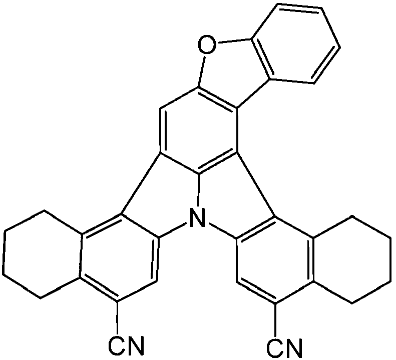 | 46 % |
| | 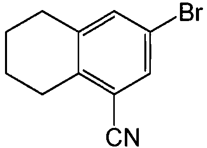 2113577-09-4 | | |
| ES93 | 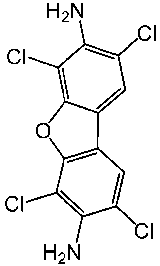 71585-31-4 | 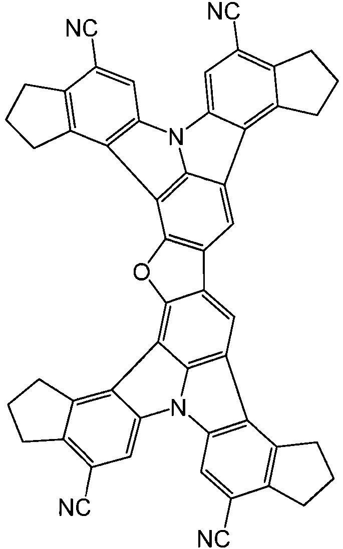 | 22 % |
| | 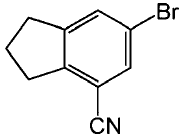 1895095-05-2 | | |

### Messung von Photolumineszenspektren (PL-Speltren):

Abbildung 1 zeigt PL-Spekren der erfindungsgemäßen Verbindungen ES1, ES94 und Verb. 675 (s.S. 204), gemessen mit einem PL-Spektrometer der Fa. Hitachi, F-4500 PL, in ca. 10⁻⁵ molarer, entgaster Toluol-Lösung bei Raumtemperatur (ca. 25 °C).

Die PL-Spektren weisen sehr schmale Emissionsbanden mit geringen FWHM Werten (< 0.18 eV) auf und führen zu besonders farbreiner Emission. Außerdem zeigen sie in der langwelligen Emissionsflanke eine Schulter bzw. ein Nebenmaximum, die jeweils weniger als 40 %,
der Intensität des Hauptmaximums aufweisen. Dies führt in Top-Emission OLED-Bauteilen zu einer günstig geringen Blickwinkelabhänigkeit des Farbeindrucks im Vergleich zu schmalbandigen Bor-enthaltenden Emittern nach Stand der Technik, die oftmals keine derartigen Schultern bzw. Nebenmaxima aufweisen und eine größere Blickwinkelabhänigkeit des Farbeindrucks zeigen.

### Herstellung von OLED-Bauteilen

### 1) Vakuum-prozessierte Bauteile

Die erfindungsgemäßen Verbindungen lassen sich unter anderem als Dotand in der Emissionsschicht in Fluoreszenz- und in Hyperphosphoreszenz-OLED-Bauteilen einsetzen.

Die Herstellung von erfindungsgemäßen OLEDs (*organic light emitting diodes*) sowie OLEDs nach dem Stand der Technik erfolgt nach einem allgemeinen Verfahren gemäß WO 2004/058911, das auf die hier beschriebenen Gegebenheiten (Schichtdickenvariation, verwendete Materialien) angepasst wird.

In den folgenden Beispielen werden die Ergebnisse verschiedener OLEDs vorgestellt. Gereinigte Glasplättchen (Reinigung in Miele Laborspülmaschine, Reiniger Merck Extran), die mit strukturiertem ITO (Indium Zinn Oxid) der Dicke 50 nm beschichtet sind werden 25 Minuten mit UV-Ozon vorbehandelt (UV-Ozon Generator PR-100, Firma UVP) und innerhalb 30min zur verbesserten Prozessierung mit 20 nm PEDOT:PSS beschichtet (Poly(3,4-ethylene-dioxythiophene)poly(styrenesulfonate), bezogen als CLEVIOS^{™} P VP AI 4083 von Heraeus Precious Metals GmbH Deutschland, aus wässriger Lösung aufgeschleudert) und anschließend bei 180°C 10 min. lang ausgeheizt. Diese beschichteten Glasplättchen bilden die Substrate, auf welche die OLEDs aufgebracht werden. Nach der Herstellung werden die OLEDs zum Schutz gegen Sauerstoff und Wasserdampf verkapselt. Der genaue Schichtaufbau der elektrolumineszierenden OLEDs ist den Beispielen zu entnehmen. Die zur Herstellung der OLEDs benötigten Materialien sind in Tabelle 10 gezeigt.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik. Die Elektrolumineszenzspektren werden bei einer Leuchtdichte von 100 bzw. 1000 cd/m² bestimmt und daraus die Emissionsfarbe und die EL-FWHM-Werte (ELectroluminescence - Full Width Half Maximum - Breite der EL-Emissionsspektren auf halber Peakhöhe in eV, zur besseren Vergleichbarkeit über den gesamten Spektralbereich) entnommen.

### Fluoreszenz-OLED- Bauteile:

Alle Materialien werden in einer Vakuumkammer thermisch aufgedampft. Dabei besteht die Emissionsschicht (EML) immer aus mindestens einem Matrixmaterial (Hostmaterial, Wirtsmaterial) SMB und einem emittierenden Dotierstoff (Dotand, Emitter) ES bzw. EAS, der dem Matrixmaterial bzw. den Matrixmaterialien durch Co-Verdampfung in einem bestimmten Volumenanteil beigemischt wird. Eine Angabe wie SMB:ES bzw. EAS (97:3%) bedeutet hierbei, dass das Material SMB in einem Volumenanteil von 97% und der Dotand ES bzw. EAS in einem Anteil von 3% in der Schicht vorliegt. Analog kann auch die Elektronentransportschicht aus einer Mischung zweier Materialien bestehen, z.B. wie hier aus ETM1 (50%) und ETM2 (50%), s. Tabelle 1. Die zur Herstellung der OLEDs verwendeten Materialien sind in Tabelle 10 gezeigt. Als Vergleich gemäß dem Stand der Technik werden die Verbindungen D-Ref.1, s. Tabelle 10 verwendet.

### Blaue Fluoreszenz-OLED- Bauteile BF:

Die OLEDs haben prinzipiell folgenden Schichtaufbau:

### Substrat

- Lochinjektionsschicht 1 (HIL1) aus HTM1 dotiert mit 5 % NDP-9 (kommerziell erhältlich von der Fa. Novaled), 20 nm
- Lochtransportschicht 1 (HTL1) aus HTM1, 160 nm
- Lochtransportschicht 2 (HTL2), s. Tabelle 1
- Emissionsschicht (EML), s. Tabelle 1
- Elektronentransportschicht (ETL2), s. Tabelle 1
- Elektronentransportschicht (ETL1) aus ETM1 (50%) und ETM2 (50%), 30 nm Elektroneninjektionsschicht (EIL) aus ETM2, 1 nm
- Kathode aus Aluminium, 100 nm

**Tabelle 1: Aufbau Blaue Fluoreszenz-OLED- Bauteile**

| **Bsp.** | **HTL2** | **EML** | **ETL2** |
|---|---|---|---|
| **Ref-BF1** | HTM2 10 nm | SMB1:Ref.-D1 (97:3%) 20 nm | ETM1 10 nm |
| **Ref-BF2** | HTM2 10 nm | SMB1:Ref.-D2 (97:3%) 20 nm | ETM1 10 nm |
| **BF1** | HTM2 10 nm | SMB1:ES44 (97:3%) 20 nm | ETM1 10 nm |
| **BF2** | HTM2 10 nm | SMB2:ES44 (95:5%) 20 nm | ETM1 10 nm |
| **BF3** | HTM2 10 nm | SMB3:ES56 (97:3%) 20 nm | ETM1 10 nm |
| **BF4** | HTM2 10 nm | SMB1:ES9 (97:3%) 20 nm | ETM1 10 nm |
| **BF5** | HTM2 10 nm | SMB1:ES10 (95:5%) 20 nm | ETM1 10 nm |
| **BF6** | HTM2 10 nm | SMB1:ES11 (97:3%) 20 nm | ETM1 10 nm |
| **BF7** | HTM2 10 nm | SMB1:ES21 (97:3%) 20 nm | ETM1 10 nm |
| **BF8** | HTM2 10 nm | SMB1:ES37 (97:3%) 20 nm | ETM1 10 nm |
| **BF9** | HTM2 10 nm | SMB1:ES40 (97:3%) 20 nm | ETM1 10 nm |
| **BF10** | HTM2 10 nm | SMB1:ES59 (97:3%) 20 nm | ETM1 10 nm |
| **BF11** | HTM2 10 nm | SMB1:61 (97:3%) 20 nm | ETM1 10 nm |
| **BF12** | HTM2 10 nm | SMB1:EAS2A (96:4%) 20 nm | ETM1 10 nm |
| **BF13** | HTM2 10 nm | SMB1:EAS2B (96:4%) 20 nm | ETM1 10 nm |
| **BF14** | HTM2 10 nm | SMB1:EAS3A (97:3%) 20 nm | ETM1 10 nm |
| **BF15** | HTM2 10 nm | SMB1:EAS15B (97:3%) 20 nm | ETM1 10 nm |
| **BF16** | HTM2 10 nm | SMB1:EAS42 (97:3%) 20 nm | ETM1 10 nm |
| **BF17** | HTM2 10 nm | SMB1:ES 73 (97:3%) 20 nm | ETM1 10 nm |
| **BF18** | HTM2 10 nm | SMB1:ES78 (95:5%) 20 nm | ETM1 10 nm |
| **BF19** | HTM2 10 nm | SMB1:EAS46A (97:3%) 20 nm | ETM1 10 nm |
| **BF20** | HTM2 10 nm | SMB1:ES94 (97:3%) 20 nm | ETM1 10 nm |

**Tabelle 2: Ergebnisse**

| **Bsp.** | **EQE (%) 1000 cd/m²** | **Spannung (V) 1000 cd/m²** | **Farbe** | **EL-FWHM [eV]** |
|---|---|---|---|---|
| **Ref-BF1** | 6.3 | 4.5 | Blau | 0.17 |
| **Ref-BF2** | 7.9 | 4.3 | Blau | 0.43 |
| **BF1** | 8.4 | 4.3 | Blau | 0.15 |
| **BF2** | 7.9 | 4.2 | Blau | 0.15 |
| **BF3** | 8.2 | 4.2 | Blau | 0.15 |
| **BF4** | 6.6 | 4.4 | Tiefblau | 0.13 |
| **BF5** | 7.9 | 4.6 | Blau | 0.15 |
| **BF6** | 7.3 | 4.4 | Tiefblau | 0.13 |
| **BF7** | 8.3 | 4.3 | Blau | 0.15 |
| **BF8** | 8.0 | 4.4 | Blau | 0.16 |
| **BF9** | 8.6 | 4.5 | Blau | 0.15 |
| **BF10** | 7.9 | 4.3 | Blau | 0.13 |
| **BF11** | 8.8 | 4.3 | Blau | 0.15 |
| **BF12** | 7.6 | 4.4 | Blau | 0.14 |
| **BF13** | 7.9 | 4.4 | Blau | 0.15 |
| **BF14** | 8.3 | 4.3 | Blau | 0.16 |
| **BF15** | 7.8 | 4.3 | Blau | 0.15 |
| **BF16** | 7.7 | 4.4 | Blau | 0.14 |
| **BF17** | 7.6 | 4.4 | Blau | 0.14 |
| **BF18** | 8.1 | 4.3 | Blau | 0.16 |
| **BF19** | 8.5 | 4.3 | Blau | 0.15 |
| **BF20** | 8.9 | 4.2 | Blau | 0.13 |

### Hyperphosphoreszenz-OLED-Bauteile:

Alle Materialien werden in einer Vakuumkammer thermisch aufgedampft. Dabei besteht die Emissionsschicht (EML) bzw. die Emissionsschichten immer aus mindestens einem Matrixmaterial (Hostmaterial, Wirtsmaterial) TMM, einem (phosphoreszierenden) Sensitizer PS und einem fluoreszierenden Emitter ES bzw. EAS. Das Matrixmaterial (Hostmaterial, Wirtsmaterial) TMM kann aus zwei Komponenten bestehen, die als Mischung verdampft werden (premixed Host, z.B. TMM2), die Komponenten und die Zusammensetzung wird ebenfalls in Tabelle 10 gezeigt. Sensitizer PS und fluoreszierender Emitter ES bzw. EAS werden dem Hostmaterial TMM durch Coverdampfung in einem bestimmten Volumenanteil beigemischt. Eine Angabe wie TMM:PS(5%):ES bzw. EAS(3%) bedeutet hierbei, dass das Material TMM in einem Volumenanteil von 92%, PS in einem Anteil von 5% und ES bzw. EAS in einem Anteil von 3% in der Schicht vorliegt.

### Blaue Hyperphosphoreszenz-OLED-Bauteile BH:

Die OLEDs haben prinzipiell folgenden Schichtaufbau:
- Substrat
- Lochinjektionsschicht 1 (HIL1) aus HTM2 dotiert mit 5 % NDP-9 (kommerziell erhältlich von der Fa. Novaled), 20 nm
- Lochtransportschicht 1 (HTL1) aus HTM2, 30 nm
- Lochtransportschicht 2 (HTL2), s. Tabelle 3
- Emissionsschicht (EML), s. Tabelle 3
- Elektronentransportschicht (ETL2), s. Tabelle 3
- Elektronentransportschicht (ETL1) aus ETM1 (50%) und ETM2 (50%), 20 nm
- Elektroneninjektionsschicht (EIL) aus ETM2, 1 nm
- Kathode aus Aluminium, 100 nm

**Tabelle 3: Aufbau Blaue Hyperphosphoreszenz-OLED-Bauteile**

| **Bsp.** | **HTL2** | **EML** | **ETL2** |
|---|---|---|---|
| **BH1** | HTM3 10 nm | TMM1:PS1 (7%):ES37(1.5%) 25 nm | ETM3 10 nm |
| **BH2** | HTM3 10 nm | TMM1:PS1(7%):ES37(2%) 25 nm | ETM3 10 nm |
| **BH3** | HTM3 10 nm | TMM1:PS1(7%):EAS19(2%) 25 nm | ETM3 10 nm |
| **BH4** | HTM3 10 nm | TMM1:PS3(7%):ES80(2%) 25 nm | ETM3 10 nm |
| **BH5** | HTM3 10 nm | TMM1:PS3(7%):EAS46(2%) 25 nm | ETM3 10 nm |
| **BH6** | HTM3 10 nm | TMM1:PS3(7%):EAS42(2%) 25 nm | ETM3 10 nm |

**Tabelle 4: Ergebnisse**

| **Bsp.** | **EQE (%) 100 cd/m²** | **Spannung (V) 100 cd/m²** | **Farbe** | **EL-FWHM [eV]** |
|---|---|---|---|---|
| **BH1** | 13.4 | 3.4 | Blau | 0.16 |
| **BH2** | 11.1 | 3.4 | Blau | 0.16 |
| **BH3** | 12.8 | 3.3 | Blau | 0.17 |
| **BH4** | 20.1 | 3.2 | Blau | 0.17 |
| **BH5** | 23.4 | 3.2 | Blau | 0.16 |
| **BH6** | 21.2 | 3.3 | Blau | 0.17 |

### Grüne Hyperphosphoreszenz-OLED-Bauteile GH:

Die OLEDs haben prinzipiell folgenden Schichtaufbau:
- Substrat
- Lochinjektionsschicht 1 (HIL1) aus HTM2 dotiert mit 5 % NDP-9 (kommerziell erhältlich von der Fa. Novaled), 20 nm
- Lochtransportschicht 1 (HTL1) aus HTM2, 30 nm
- Lochtransportschicht 2 (HTL2), s. Tabelle 5
- Emissionsschicht (EML), s. Tabelle 5
- Elektronentransportschicht (ETL2), s. Tabelle 5
- Elektronentransportschicht (ETL1) aus ETM1 (50%) und ETM2 (50%), 30 nm
- Elektroneninjektionsschicht (EIL) aus ETM2, 1 nm
- Kathode aus Aluminium, 100 nm

**Tabelle 5: Aufbau Grüne Hyperphosphoreszenz-OLED-Bauteile**

| **Bsp.** | **HTL2** | **EML** | **ETL2** |
|---|---|---|---|
| **GH1** | HTM3 10 nm | TMM1:PS1(8%):ES39(2%) 25 nm | ETM3 10 nm |
| **GH2** | HTM3 10 nm | TMM1:PS3(8%):ES39(2%) 25 nm | ETM3 10 nm |

**Tabelle 6: Ergebnisse**

| **Bsp.** | **EQE (%) 100 cd/m²** | **Spannung (V) 100 cd/m²** | **Farbe** | **EL-FWHM [eV]** |
|---|---|---|---|---|
| **GH1** | 19.3 | 3.4 | Grün | 0.16 |
| **GH2** | 22.4 | 3.3 | Grün | 0.16 |

### Orange-Rote Hyperphosphoreszenz-OLED-Bauteile RH:

Die OLEDs haben prinzipiell folgenden Schichtaufbau:
- Substrat
- Lochinjektionsschicht 1 (HIL1) aus HTM1 dotiert mit 5 % NDP-9 (kommerziell erhältlich von der Fa. Novaled), 20 nm
- Lochtransportschicht 1 (HTL1) aus HTM1, 30 nm
- Lochtransportschicht 2 (HTL2), s. Tabelle 7
- Emissionsschicht (EML), s. Tabelle 7
- Elektronentransportschicht (ETL2), s. Tabelle 7
- Elektronentransportschicht (ETL1) aus ETM1 (50%) und ETM2 (50%), 45 nm
- Elektroneninjektionsschicht (EIL) aus ETM2, 1 nm
- Kathode aus Aluminium, 100 nm

**Tabelle 7: Aufbau Organe-Rote Hyperphosphoreszenz-OLED Bauteile**

| **Bsp.** | **HTL2** | **EML** | **ETL2** |
|---|---|---|---|
| **RH1** | HTM2 10 nm | TMM2:PS2(8%):ES67(2%) 20 nm | ETM1 10 nm |

**Tabelle 8: Ergebnisse**

| **Bsp.** | **EQE (%) 100 cd/m²** | **Spannung (V) 100 cd/m²** | **Farbe** | **EL-FWHM [eV]** |
|---|---|---|---|---|
| **RH1** | 20.2 | 3.2 | Rot | 0.15 |

### 2) Lösungs-prozessierte Bauteile:

Die Herstellung lösungsbasierter OLEDs ist in der Literatur grundsätzlich beschrieben, z.B. in der WO 2004/037887 und der WO 2010/097155. Bei den folgenden Beispielen wurden beide Herstellungsverfahren (Aufbringung aus Gasphase und Lösungsprozessierung) kombiniert, so dass bis einschließlich Emissionsschicht aus Lösung prozessiert wurde und die darauffolgenden Schichten (Lochblockierschicht / Elektronentransportschicht) im Vakuum aufgedampft wurden. Die vorbeschriebenen allgemeinen Verfahren werden dafür wie folgt auf die hier beschriebenen Gegebenheiten (Schichtdickenvariation, Materialien) angepasst und kombiniert.

Der verwendete Aufbau ist damit wie folgt:
- Substrat
- ITO, 50 nm
- PEDOT, 20 nm
- Lochtransportschicht HIL-Sol, aus HTM-Sol, 20 nm
- Emissionsschicht aus SMB4(97%) und ES(3%) bzw. EAS(3%), 50 nm
- Elektronentransportschicht (ETL1) aus ETM1 (50%) und ETM2 (50%), 25 nm
- Kathode aus Aluminium, 100 nm

Als Substrat werden Glasplättchen, die mit strukturiertem ITO (Indium-Zinn-Oxid) der Dicke 50 nm beschichtet sind, verwendet. Zur besseren Prozessierung werden diese mit dem Buffer (PEDOT) Clevios P VP AI 4083 (Heraeus Clevios GmbH, Leverkusen) beschichtet oben steht PEDOT. Das Aufschleudern erfolgt an Luft aus Wasser. Die Schicht wird anschließend für 10 Minuten bei 180°C ausgeheizt. Auf die so beschichteten Glasplättchen werden die Lochtransportschicht sowie die Emissionsschicht aufgebracht. Bei der Lochtransportschicht handelt es sich um das Polymer HTM-Sol der in Tabelle 10 gezeigten Struktur, das gemäß WO2010/097155 synthetisiert wurde. Das Polymer wird in Toluol gelöst, so dass die Lösung typischerweise einen Feststoffgehalt von ca. 5 g/l besitzt, wenn, wie hier, die für ein Device typische Schichtdicke von 20 nm mittels Spincoating erzielt werden soll. Die Schichten werden in einer Inertgasatmosphäre, im vorliegenden Fall Argon, aufgeschleudert und 60 min bei 180°C ausgeheizt.

Die Emissionsschicht setzt sich immer aus mindestens einem Matrixmaterial (Hostmaterial, Wirtsmaterial) und einem emittierenden Dotierstoff (Dotand, Emitter) zusammen. Eine Angabe wie SMB4 (97%) und ES bzw. EAS (3%) bedeutet hierbei, dass das Material SMB4 in einem Gewichtsanteil von 97% und der Dotand ES bzw. EAS in einem Gewichtsanteil von 3% in der Emissionsschicht vorliegt. Die Mischung für die Emissionsschicht wird in Toluol oder Chlorbenzol gelöst. Der typische Feststoffgehalt solcher Lösungen liegt bei ca. 18 g/l, wenn, wie hier, die für ein Device typische Schichtdicke von 50 nm mittels Spincoating erzielt werden soll. Die Schichten werden in einer Inertgasatmosphäre, im vorliegenden Fall Argon, aufgeschleudert und 10 Minuten bei 140° bis 160 °C ausgeheizt. Die verwendeten Materialien sind in Tabelle 10 gezeigt.

Die Materialien für die Elektronentransportschicht sowie für die Kathode werden in einer Vakuumkammer thermisch aufgedampft. Dabei kann z.B. die Elektronentransportschicht aus mehr als einem Material bestehen, die einander durch Co-Verdampfung in einem bestimmten Volumenanteil beigemischt werden. Eine Angabe wie ETM1 (50%) und ETM2 (50%) bedeutet hierbei, dass die Materialien ETM1 und ETM2 in einem Volumenanteil von je 50% in der Schicht vorliegen. Die im vorliegenden Fall verwendeten Materialien sind in Tabelle 10 gezeigt.

**Tabelle 9: Ergebnisse der Lösungs-prozessierten OLEDs bei 1000 cd/m²**

| **Bsp.** | **Dotand** | **EQE (%)** | **Spannung (V)** | **Farbe** | **EL-FWHM [eV]** |
|---|---|---|---|---|---|
| Sol-BF1 | ES15 | 7.8 | 4.4 | Blau | 0.15 |

**Tabelle 10: Strukturformeln der verwendeten Materialien**

| | |
|---|---|
| 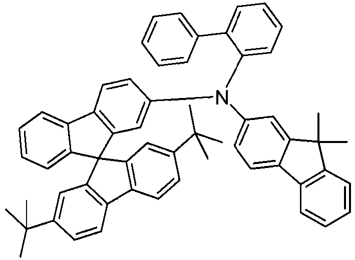 HTM1 [1365840-52-3] | 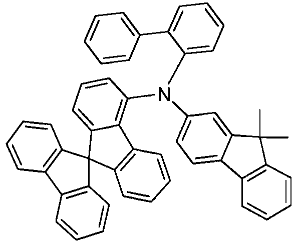 HTM2 [1450933-44-4] |
| 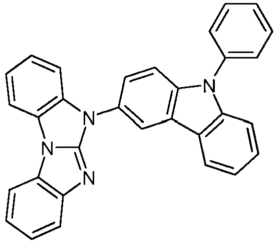 HTM3 [1401068-29-8] | 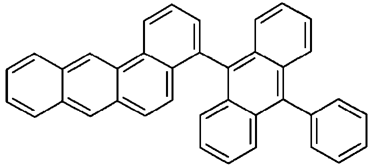 SMB1 [1087346-88-0] |
| 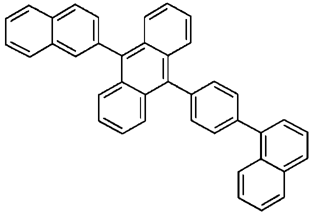 SMB2 [667940-34-3] | 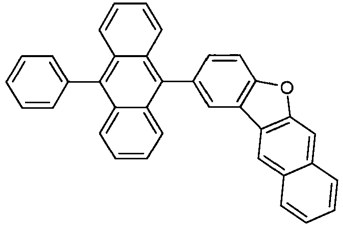 SMB3 [1627916-48-6] |
| 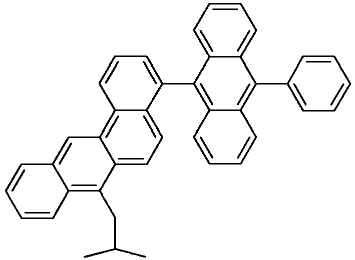 SMB4 [1818872-85-3] | 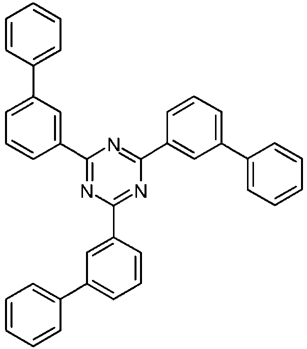 TMM1 / ETM3 [1201800-83-0] |
| 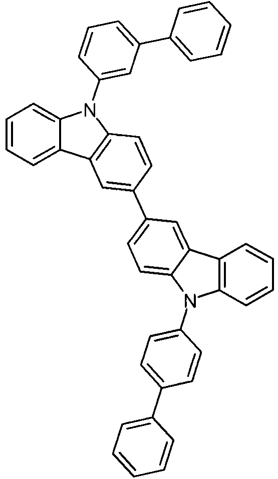 [1643476-29-2] (40%) | 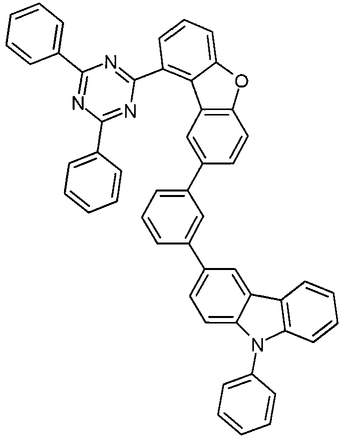 [1822310-86-0] (60%) |
| TMM2 | |
| 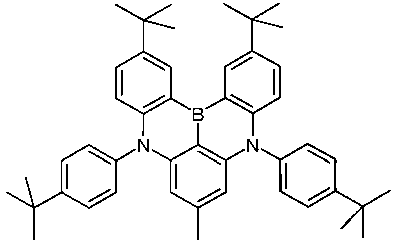 [1805802-42-9] Ref.-D1 | 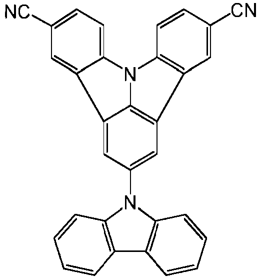 [2222555-03-3] Ref.-D2 |
| 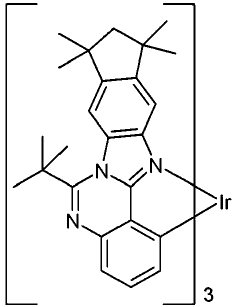 PS1 [1541114-98-0] | 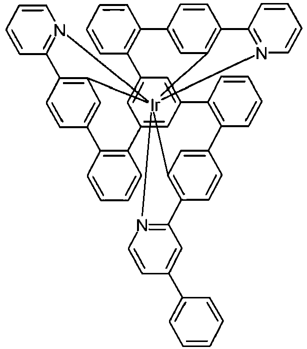 PS2 [2245865-85-2] |
| 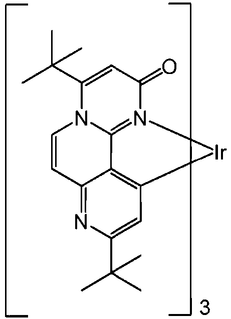 PS3 [1615218-73-9] | |
| 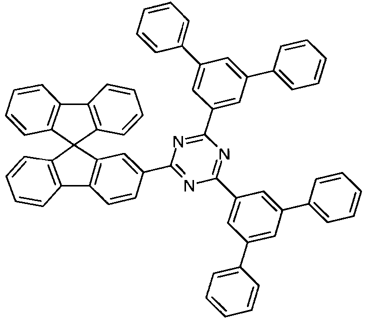 ETM1 [1233200-52-6] | 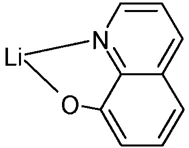 ETM2 [25387-93-3] |
| 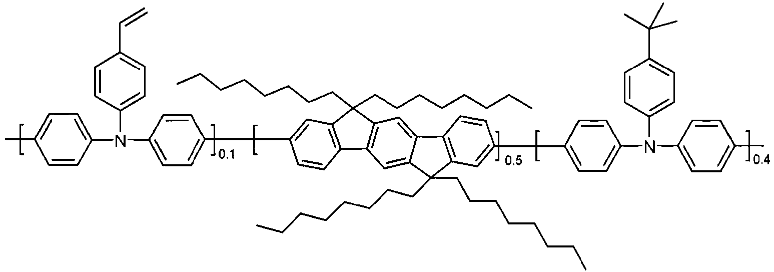 HTM-Sol | |

Die Abkürzungen der erfindungsgemäßen Verbindungen, die in den zuvor in Bezug auf die OLED-Bauteile dargelegten Tabellen verwendet werden, beziehen sich auf die in den obigen Synthesebeispielen bereitgestellten Abkürzungen.

Die erfindungsgemäßen Verbindungen zeigen im Vgl. zu den Referenzen schmalere Elektrolumineszenzspektren, erkennbar an den geringeren bzw. gleichgroßen EL-FWHM-Werten (**EL**ectroluminescence - **F**ull **W**idth **H**alf **M**aximum - Breite der EL-Emissionsspektren in eV auf halber Peakhöhe). Schmalere Elektrolumineszenzspektren führen zu deutlich verbesserter Farbreinheit (kleinere CIE y Werte). Außerdem sind die EQE-Werte (**E**xterne **Q**uanten **E**ffizienen) deutlich größer und die Betriebsspannungen geringer im Vgl. zur Referenz, was zu deutlich verbesserten Leistungseffizienzen des Devices und somit zu geringerem Stromverbrauch führt.

### Herstellung von Bauteilen zur Farbkonversion

Die erfindungsgemäßen Verbindungen können zur Farbkonversion eingesetzt werden. Dazu werden die Verbindungen in eine Komposition eingearbeitet, die dann durch bekannte Verfahren (Spin-coating, Slitcoating, Raklen, Siebdruck, Nozzle-Printing, Ink-Jet-Printing, etc.) zu Pixeln oder flächigen Schichten verarbeitet werden. Die Kompositionen bestehen typischerweise aus vernetzbaren Komponenten (Monomeren, Oligomeren, Polymeren), z. B. auf Basis von Acrylaten, Acrylamiden, Polyestern, Siliconen, etc. und einem oder mehreren thermisch oder photochemisch aktivierbaren Starterkomponenten. Daneben können weitere Komponenten wie org. Hilfsstoffe (Antioxidantien, Stabilisatoren, Verlaufshilfsmittel, Viskositätsmoderatoren, etc.) oder anorg. Füllstoffe (SiO₂, TiO₂, Al₂O₃, etc.) eingebracht werden.

### Allgemeine Herstellungsprozedur der Komposition und abgeleiteter Schichten:

0.5 g der erfindungsgemäßen Verbindung ES bzw. EAS, 0.2 g Titandioxid (TiO₂ ToyoColor, Fa. Toyo Ink Group) und 10 g OE-6550 Optical Encapsulant (Fa. Dow Corning) werden unter sehr gutem Rühren (Magnetrührer) unter Einwirkung von Ultraschall (Ultraschallbad) bei 40 °C homogenisiert. Schichten mit ca. 15 µm Schichtdicke werden durch Rakeln erzeugt und dann durch Ausheizen unter Stickstoffatmosphäre (150 °C, 1 Stunde) gehärtet.

### Spektrale Messung der Schichten:

Fluoreszenspektren und EQE-Werte (Externe Quanten Effizienz, EQE = Emittierte Photonen / Absorbierte Photonen) der Schichten werden in einem Fluoreszenspektometer (C9920, Hamamatsu photonics) mit Ulbricht-Kugel und Faseroptik (Anregungswellenlänge CWL: 420 - 440 nm für Blaue, 450 nm für Grüne, Gelbe und Rote Emitter, Referenzmessung an Luft bei Raumtemperatur) ermittelt.

### Ergebnisse

**Tabelle 11 fasst die Ergebnisse zusammen:**

| Bsp. | Material | Farbe | FWHM [eV] | EQE [%] |
|---|---|---|---|---|
| CCG1 | ES39 | Tiefgrün | 0.16 | 26.4 |
| CCG2 | ES65 | Gelb | 0.15 | 26.8 |
| CCG3 | ES66 | Gelb | 0.14 | 27.6 |
| CCG4 | ES84 | Grün | 0.15 | 29.6 |
| CCR2 | ES67 | Rot | 0.15 | 25.0 |
| CCB3 | ES5 | Tiefblau | 0.14 | 25.2 |
| CCB4 | ES13 | Blau | 0.15 | 27.4 |
| CCB5 | ES16 | Blau | 0.15 | 25.6 |
| CCB6 | ES17 | Blau | 0.15 | 27.3 |
| CCB7 | ES19 | Blau | 0.14 | 28.6 |
| CCB8 | ES20 | Tiefblau | 0.14 | 24.3 |
| CCB9 | ES22 | Tiefblau | 0.15 | 24.0 |
| CCB10 | ES26 | Tiefblau | 0.15 | 26.7 |
| CCB11 | ES29 | Blau | 0.15 | 32.1 |
| CCB12 | ES32 | Blau | 0.16 | 34.0 |
| CCB13 | ES33 | Blau | 0.15 | 29.4 |
| CCB14 | ES35 | Blau | 0.15 | 30.1 |
| CCB15 | ES36 | Tiefblau | 0.15 | 29.6 |
| CCB16 | ES45 | Tiefblau | 0.14 | 21.6 |
| CCB17 | ES46 | Blau | 0.14 | 33.5 |
| CCB18 | ES53 | Blau | 0.14 | 30.3 |
| CCB19 | ES57 | Blau | 0.15 | 31.4 |
| CCB20 | EAS5 | Tiefblau | 0.15 | 29.9 |
| CCB21 | EAS16 | Tiefblau | 0.17 | 25.8 |
| CCB22 | EAS25 | Tiefblau | 0.16 | 24.9 |
| CCB23 | EAS33A | Tiefblau | 0.15 | 26.8 |
| CCB24 | EAS39 | Tiefblau | 0.15 | 27.8 |
| CCB25 | EAS40B | Blau | 0.15 | 31.0 |
| CCB26 | EAS42 | Blau | 0.15 | 30.3 |
| CCB27 | ES72 | Blau | 0.19 | 31.4 |
| CCB28 | ES74 | Blau | 0.14 | 29.9 |
| CCB29 | ES75 | Blau | 0.14 | 30.4 |
| CCB30 | ESA45A | Blau | 0.15 | 29.1 |
| CCB31 | ES80 | Blau | 0.14 | 28.9 |
| CCB32 | ES81 | Blau | 0.18 | 28.7 |
| CCB33 | ES85 | Blau | 0.15 | 30.3 |
| CCB34 | ES89 | Tiefblau | 0.16 | 31.2 |

Bevorzugte Aspekte der Erfindung sind:
1. Verbindung umfassend mindestens eine Struktur der Formel (I), vorzugsweise Verbindung gemäß der Formel (I), 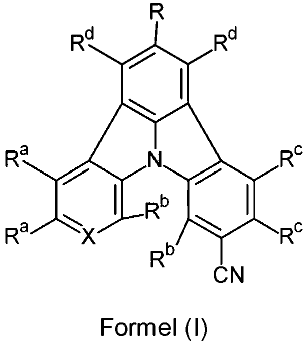 wobei für die verwendeten Symbole und Indizes gilt:
   - X: steht bei jedem Auftreten gleich oder verschieden für N, C-CN, C-Y-R^{y} oder CR^{b}, vorzugsweise für N, C-CN, oder C-Y-R^{y}, besonders bevorzugt für C-CN;
   - Y: ist bei jedem Auftreten gleich oder verschieden CO, P(=O)R^{a}, SO, SO₂, C(O)O, C(S)O, C(O)S, C(=O)NR^{a}, C(=O)NAr, vorzugsweise CO, P(=O)R^{a}, SO, SO₂, besonders bevorzugt CO;
   - R: ist bei jedem Auftreten gleich oder verschieden H, D, OH, F, Cl, Br, I, CN, NO₂, N(Ar)₂, N(R^{e})₂, C(=O)N(Ar)₂, C(=O)N(R^{e})₂, C(Ar)₃, C(R^{e})₃, Si(Ar)3, Si(R^{e})₃, B(Ar)₂, B(R^{e})₂, C(=O)Ar, C(=O)R^{e}, P(=O)(Ar)₂, P(=O)(R^{e})₂, P(Ar)₂, P(R^{e})₂, S(=O)Ar, S(=O)R^{e}, S(=O)₂Ar, S(=O)₂R^{e}, OSO₂Ar, OSO₂R^{e}, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R^{e} substituiert sein kann, wobei eine oder mehrere nicht benachbarte CH₂-Gruppen durch R^{e}C=CR^{e}, C≡C, Si(R^{e})₂, C=O, C=S, C=Se, C=NR^{e}, -C(=O)O-, -C(=O)NR^{e}-, NR^{e}, P(=O)(R^{e}), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R^{e} substituiert sein kann, oder eine Aryloxy- oder Heteroaryloxygruppe mit 5 bis 60 aromatischen Ringatomen, die durch einen oder mehrere Reste R^{e} substituiert sein kann, oder eine Arylthio- oder Heteroarylthiogruppe mit 5 bis 60 aromatischen Ringatomen, die durch einen oder mehrere Reste R^{e} substituiert sein kann, oder eine Diarylamino-, Arylheteroarylamino-, Diheteroarylaminogruppe mit 5 bis 60 aromatischen Ringatomen, die durch einen oder mehrere Reste R^{e} substituiert sein kann, oder eine Arylalkyl- oder Heteroarylalkylgruppe mit 5 bis 60 aromatischen Ringatomen und 1 bis 10 C-Atomen im Alkylrest, die durch einen oder mehrere Reste R^{e} substituiert sein kann; dabei kann ein Rest R mit einer weiteren Gruppe, vorzugsweise R^{d} ein Ringsystem bilden;
   - Ar: ist bei jedem Auftreten gleich oder verschieden ein aromatisches oder heteroaromatisches Ringsystem mit 5 bis 60 aromatischen Ringatomen, das mit einem oder mehreren Resten R^{e} substituiert sein kann, dabei können zwei Reste Ar, welche an dasselbe C-Atom, Si-Atom, N-Atom, P-Atom oder B-Atom binden, auch durch eine Einfachbindung oder eine Brücke, ausgewählt aus B(R^{e}), C(R^{e})₂, Si(R^{e})₂, C=O, C=NR^{e}, C=C(R^{e})₂, O, S, S=O, SO₂, N(R^{e}), P(R^{e}) und P(=O)R^{e}, miteinander verbrückt sein;
   - R^{a}, R^{b}, R^{c}, R^{d}, R^{e}: ist bei jedem Auftreten gleich oder verschieden H, D, OH, F, Cl, Br, I, CN, NO₂, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy- , Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; dabei können zwei Reste R^{a}, R^{b}, R^{c}, R^{d}, R^{e} auch miteinander oder einer weiteren Gruppe, vorzugsweise R oder R^{y} ein Ringsystem bilden;
   - R^{y}: ist bei jedem Auftreten gleich oder verschieden C(Ar')₃, C(R¹)₃, Si(Ar')₃, Si(R¹)₃, N(Ar')₂, N(R¹)₂, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, wobei eine mit dem Rest Y verbundene CH₂-Gruppe nicht durch C=O, C=S, C=Se, C=NR¹, -C(=O)O-, -C(=O)NR¹-, P(=O)(R¹), SO oder SO₂ ersetzt sein kann, wobei eine mit dem Rest Y verbundene CH₂-Gruppe besonders bevorzugt nicht durch Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein kann, oder ein aromatisches oder heteroaromatisches Ringsystem mit 5 bis 60 aromatischen Ringatomen, das jeweils durch einen oder mehrere Reste R¹ substituiert sein kann; dabei können zwei Reste R^{y} auch miteinander oder ein Rest R^{y} mit einem Rest R^{a}, R^{b}, R^{c}, R^{d}, R^{e} ein Ringsystem bilden, vorzugsweise einem Rest R^{a};
   - Ar': ist bei jedem Auftreten gleich oder verschieden ein aromatisches oder heteroaromatisches Ringsystem mit 5 bis 60 aromatischen Ringatomen, das mit einem oder mehreren Resten R¹ substituiert sein kann, dabei können zwei Reste Ar', welche an dasselbe C-Atom, Si-Atom, N-Atom, P-Atom oder B-Atom binden, auch durch eine Einfachbindung oder eine Brücke, ausgewählt aus B(R¹), C(R¹)₂, Si(R¹)₂, C=O, C=NR¹, C=C(R¹)₂, O, S, S=O, SO₂, N(R¹), P(R¹) und P(=O)R¹, miteinander verbrückt sein;
   - R¹: ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch -R²C=CR²-, -C=C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei oder mehrere, vorzugsweise benachbarte Reste R¹ miteinander ein Ringsystem bilden, dabei können einer oder mehrere Reste R¹ mit einem weiteren Teil der Verbindung ein Ringsystem bilden;
   - Ar": ist bei jedem Auftreten gleich oder verschieden ein aromatisches oder heteroaromatisches Ringsystem mit 5 bis 30 aromatischen Ringatomen, das mit einem oder mehreren Resten R² substituiert sein kann, dabei können zwei Reste Ar", welche an dasselbe C-Atom, Si-Atom, N-Atom, P-Atom oder B-Atom binden, auch durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verbrückt sein;
   - R²: ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann, dabei können zwei oder mehrere, vorzugsweise benachbarte Substituenten R² miteinander ein Ringsystem bilden.
2. Verbindung nach Aspekt 1, dadurch gekennzeichnet, dass mindestens ein vorzugsweise mindestens zwei der Reste R, R^{a}, R^{b}, R^{c}, R^{d}, R^{e} ungleich H sind, vorzugsweise ungleich H, D, OH, NO₂, F, Cl, Br, I.
3. Verbindung nach Aspekt 1 oder 2, dadurch gekennzeichnet, dass mindestens ein vorzugsweise mindestens zwei der Reste R^{a}, R^{c} eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, darstellen, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können.
4. Verbindung nach einem oder mehreren der Aspekte 1 bis 3, dadurch gekennzeichnet, dass der Rest R ein aromatisches oder heteroaromatisches Ringsystem mit 5 bis 13 aromatischen Ringatomen darstellt, das mit einem oder mehreren Resten R^{e} substituiert sein kann.
5. Verbindung nach einem oder mehreren der Aspekte 1 bis 4, dadurch gekennzeichnet, dass zwei Reste R^{a} mit den weiteren Gruppen, an die die zwei Reste R^{a} binden, einen kondensierten Ring bilden, vorzugsweise einen aliphatischen oder heteroaliphatischen Ring mit 3 bis 20, vorzugsweise 5 bis 18 Ringatomen oder einen aromatischen oder heteroaromatischen Ring mit 5 bis 13 Ringatomen, besonders bevorzugt einen aliphatischen oder heteroaliphatischen Ring mit 3 bis 20, vorzugsweise 5 bis 18 Ringatomen.
6. Verbindung nach einem oder mehreren der Aspekte 1 bis 5, dadurch gekennzeichnet, dass zwei Reste R^{c} mit den weiteren Gruppen, an die die zwei Reste R^{c} binden, einen kondensierten Ring bilden, vorzugsweise einen aliphatischen oder heteroaliphatischen Ring mit 3 bis 20, vorzugsweise 5 bis 18 Ringatomen oder einen aromatischen oder heteroaromatischen Ring mit 5 bis 13 Ringatomen, besonders bevorzugt einen aliphatischen oder heteroaliphatischen Ring mit 3 bis 20, vorzugsweise 5 bis 18 Ringatomen.
7. Verbindung nach einem oder mehreren der Aspekte 1 bis 6, umfassend mindestens eine Struktur der Formeln (I-1) bis (I-83), vorzugsweise Verbindung gemäß einer der Formeln (I-1) bis (I-83), 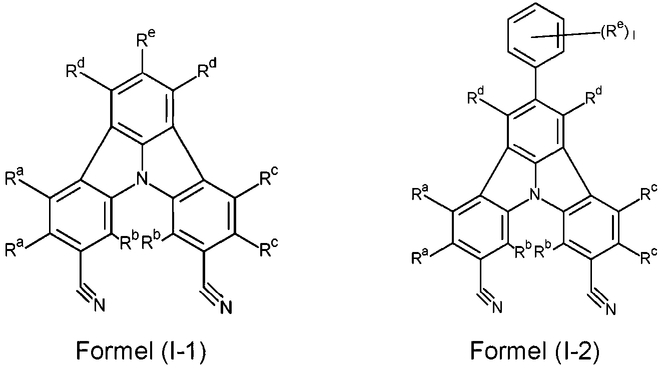 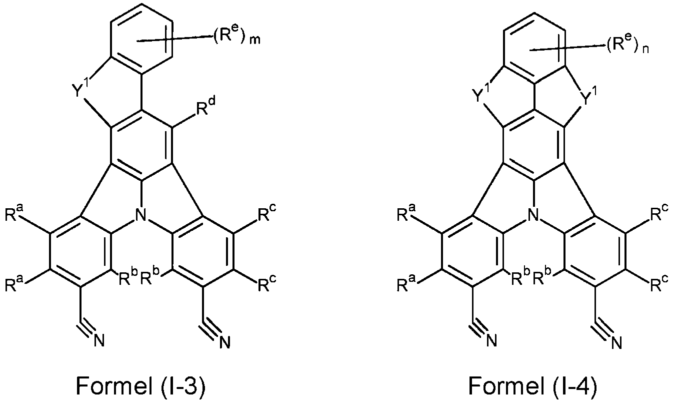 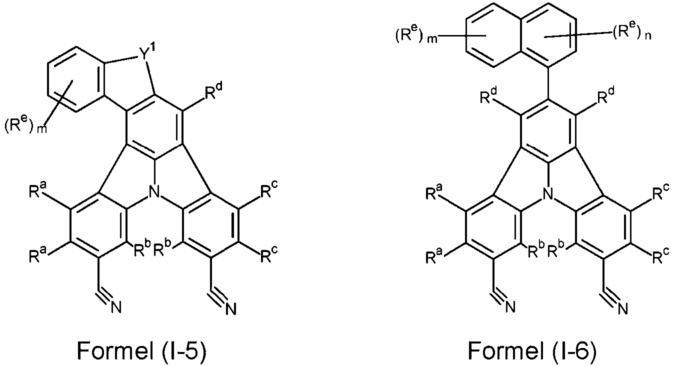 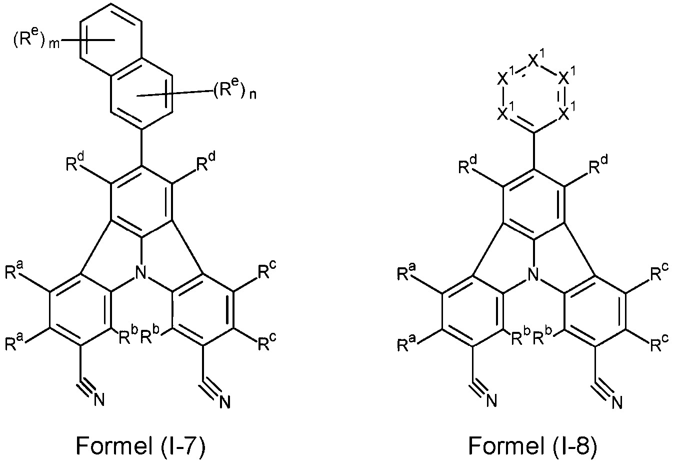 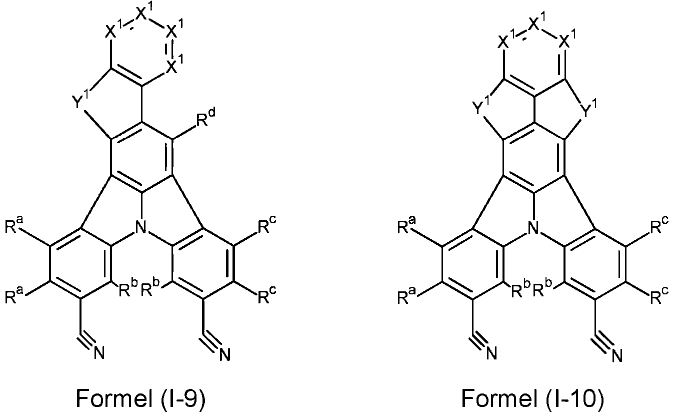 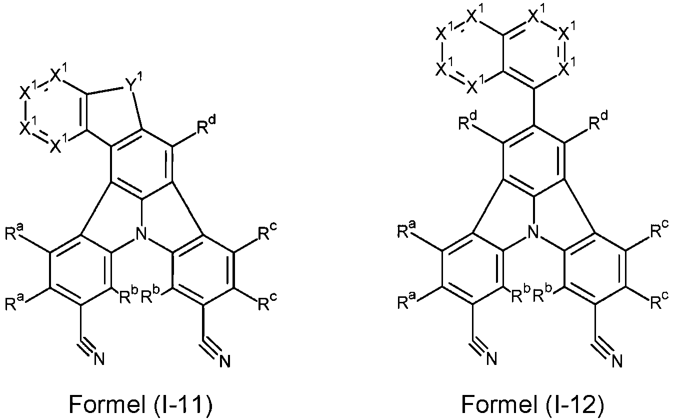 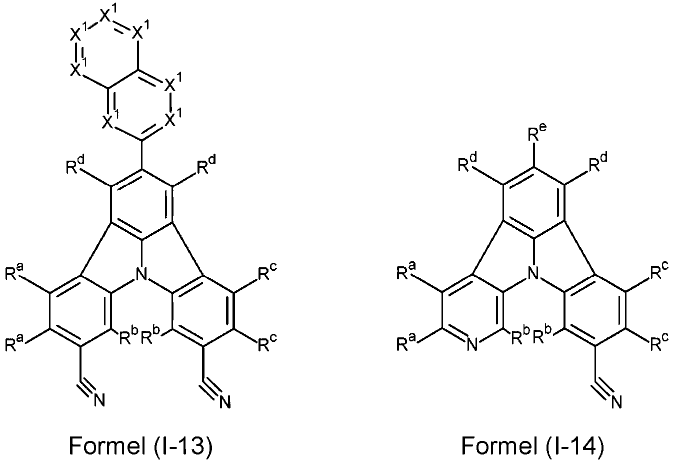 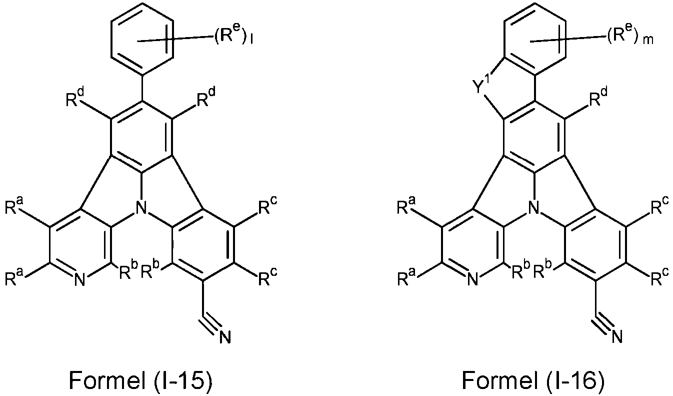 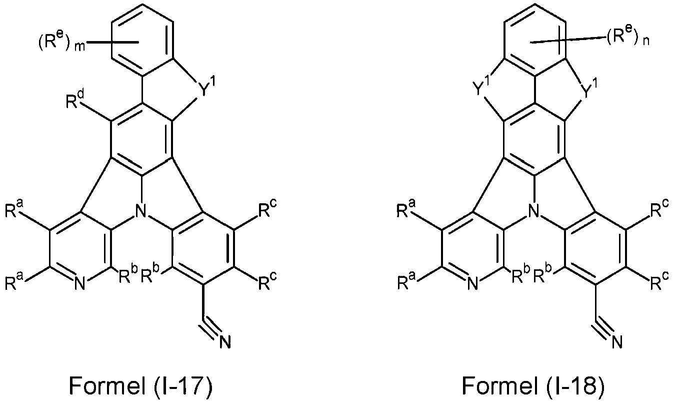 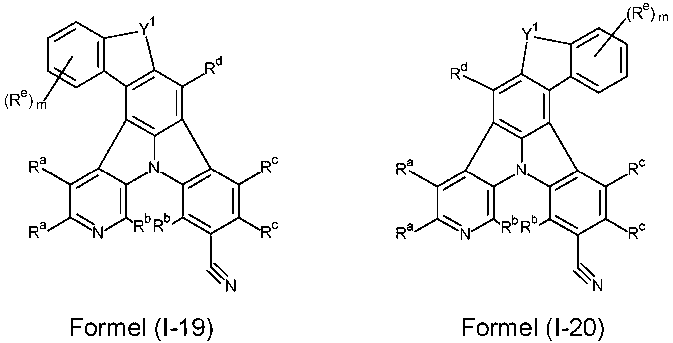 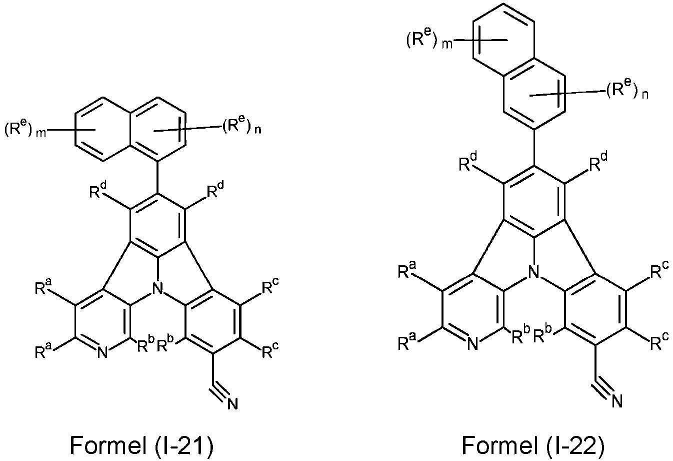 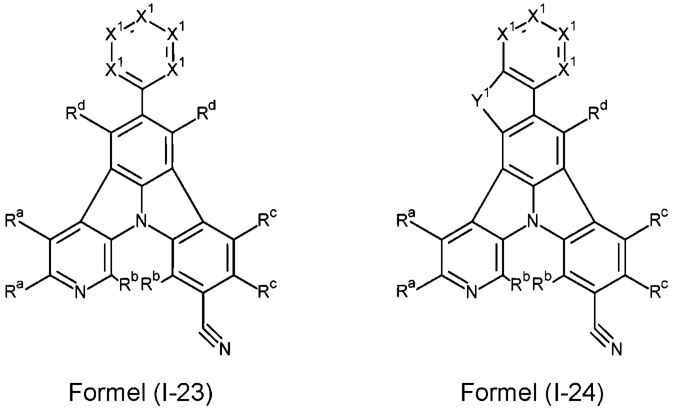 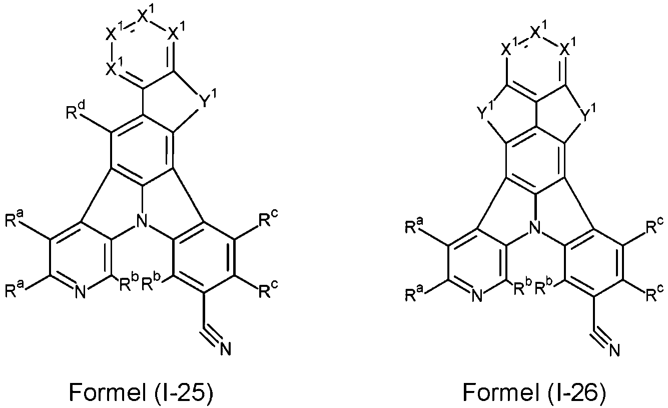 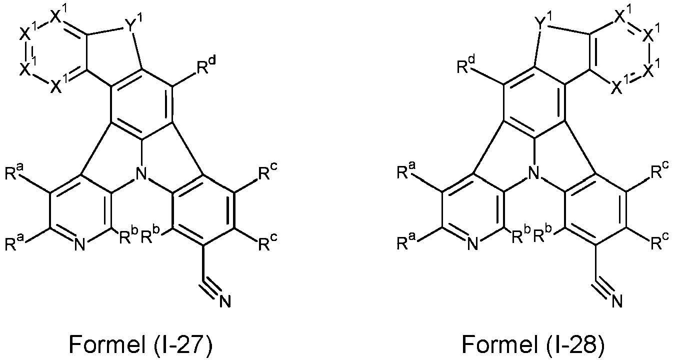 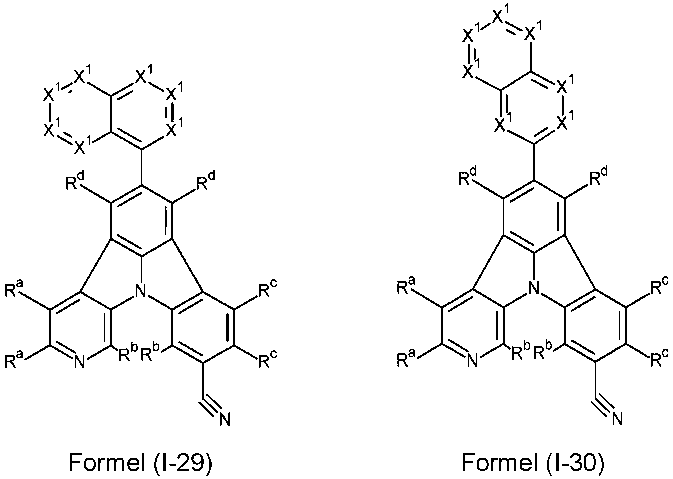 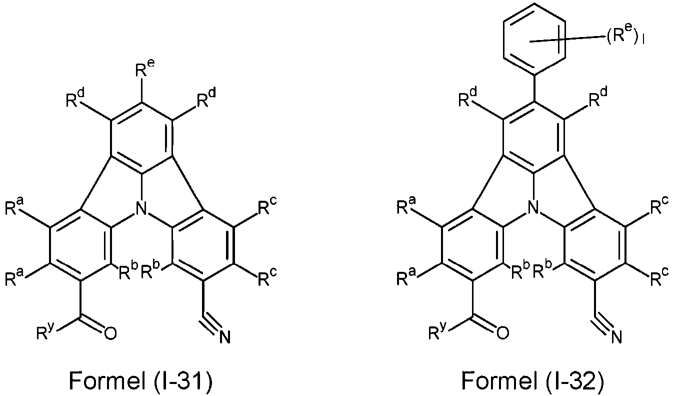 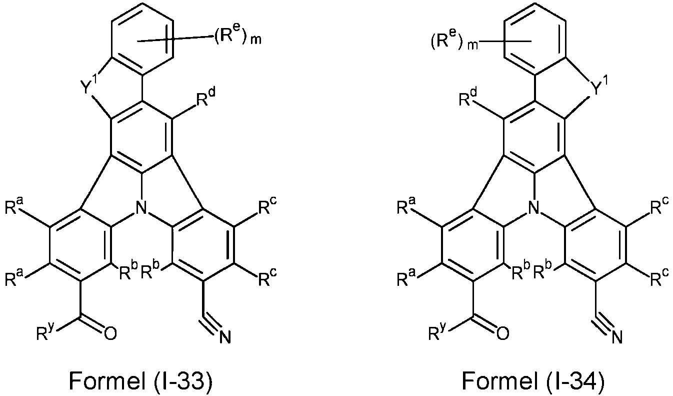 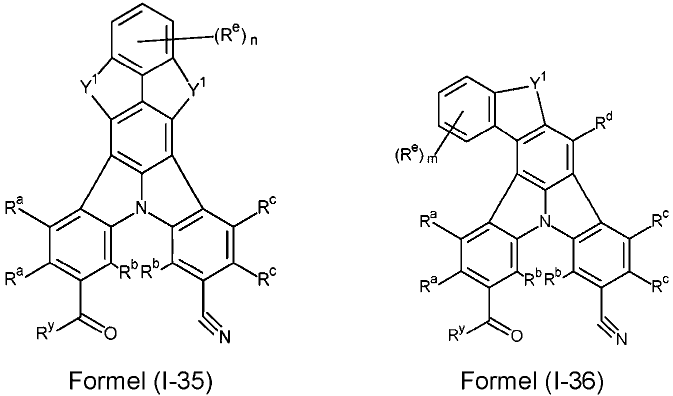 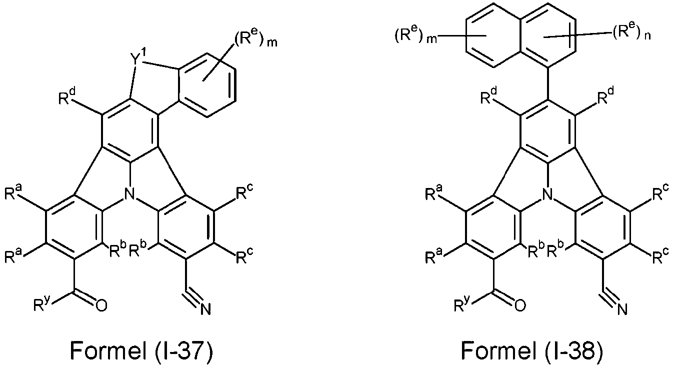 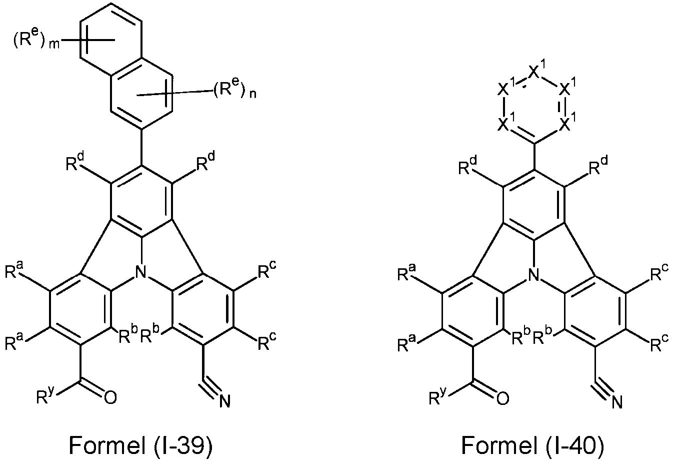 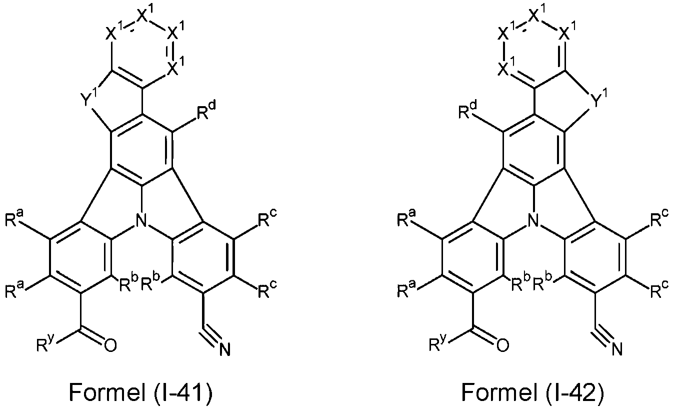 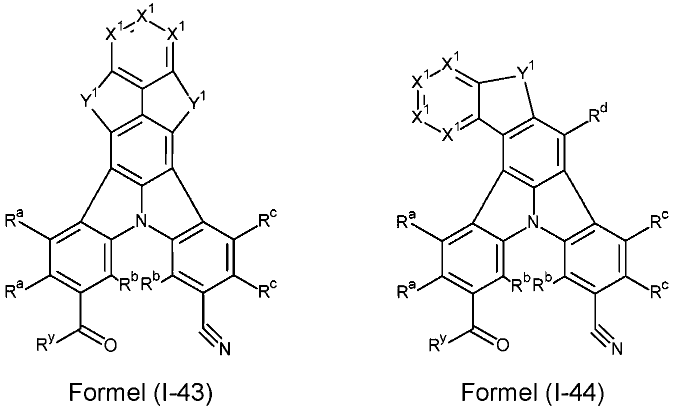 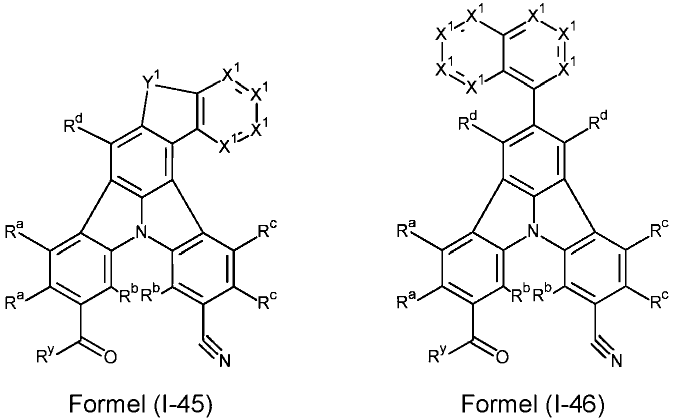 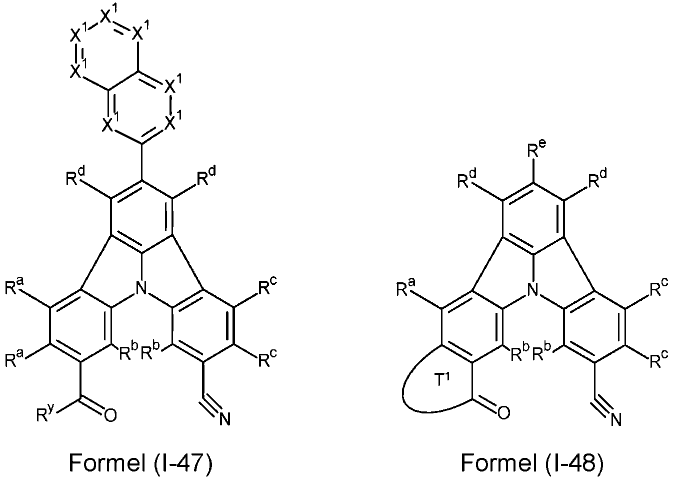 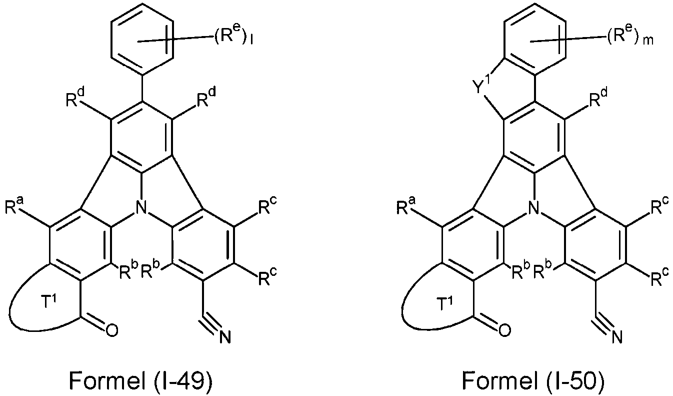 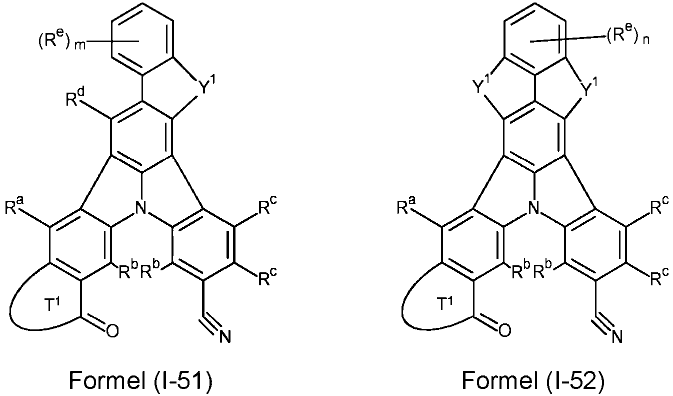 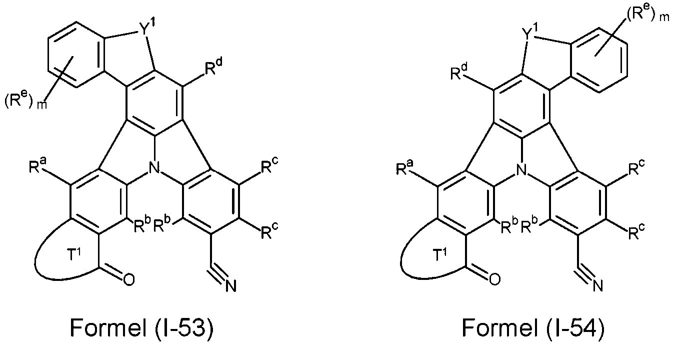 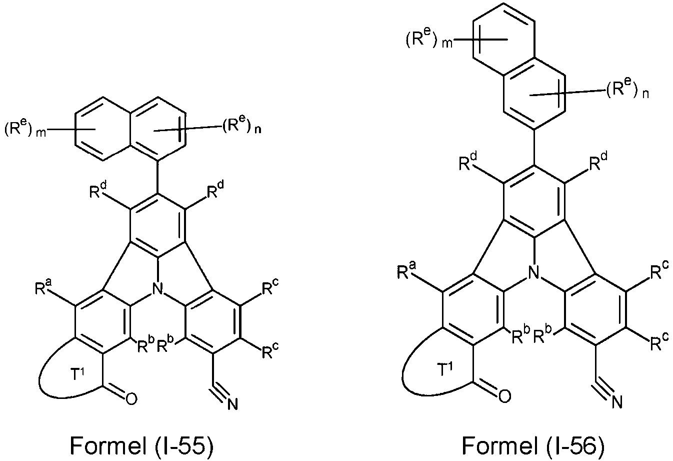 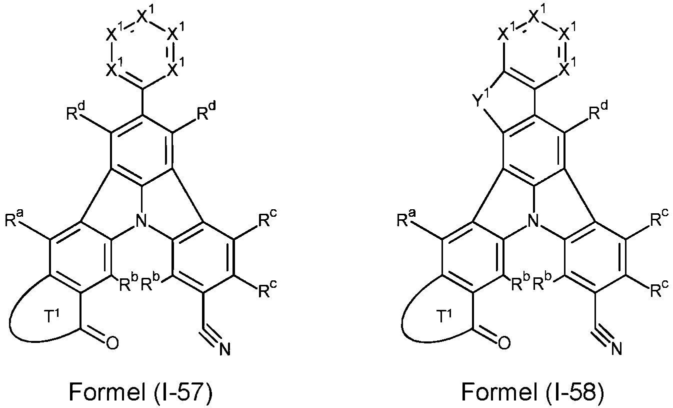 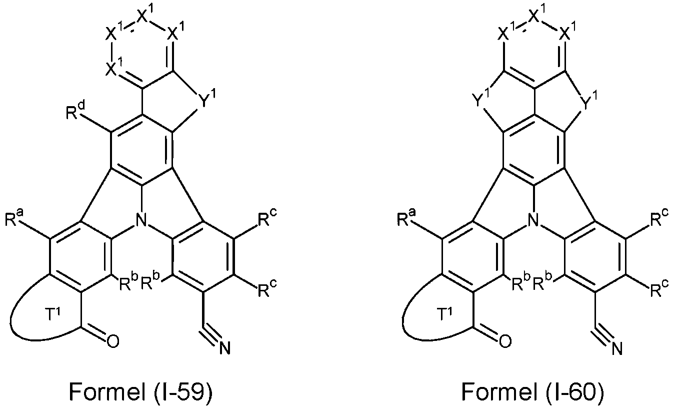 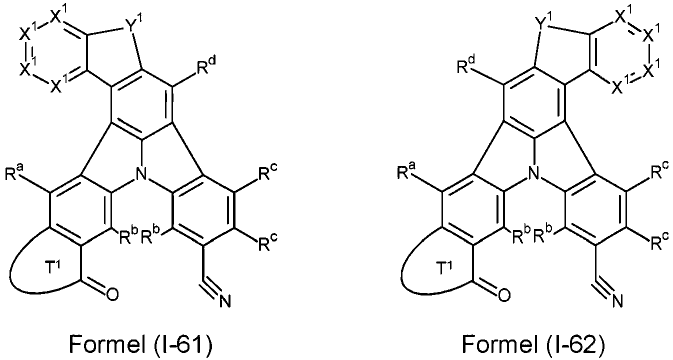 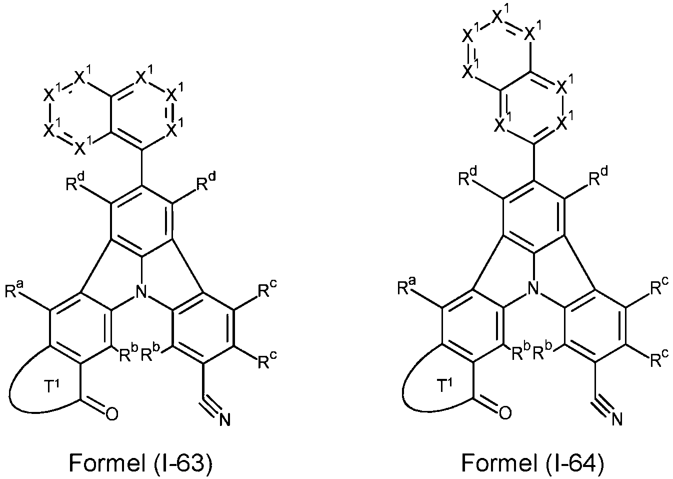 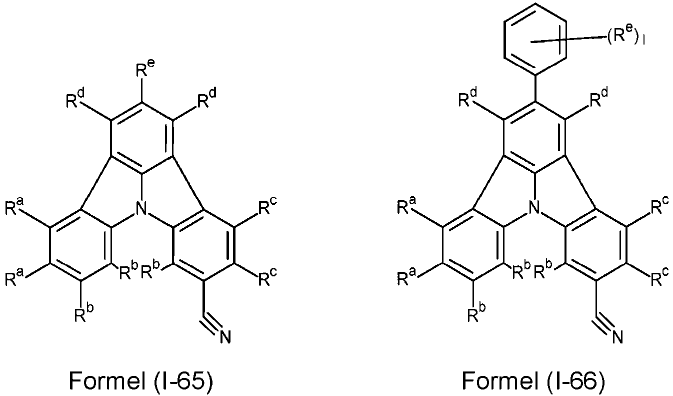 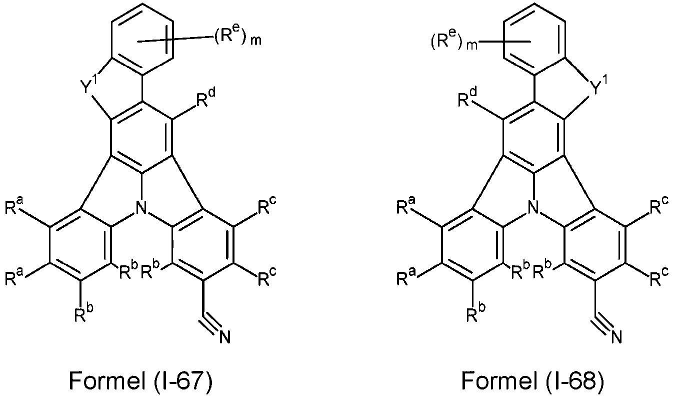 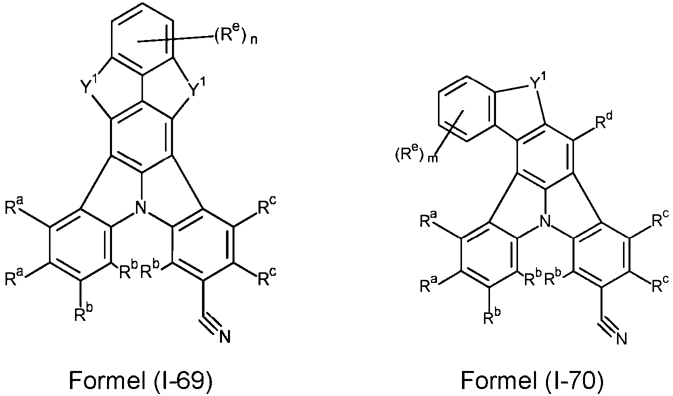 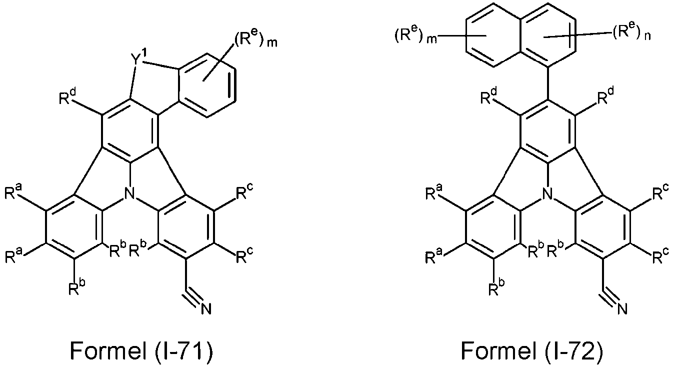 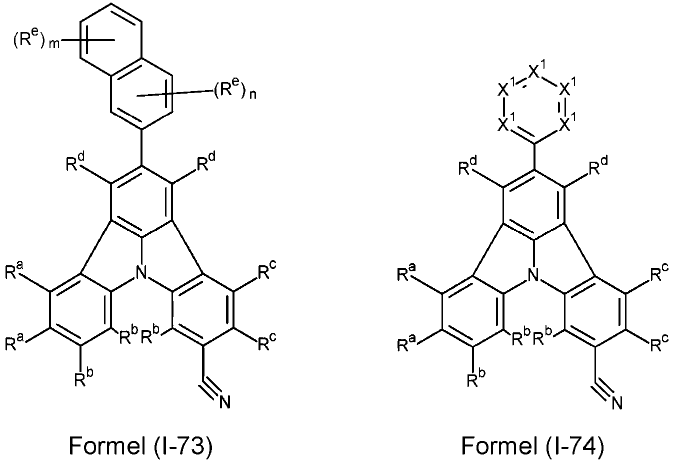 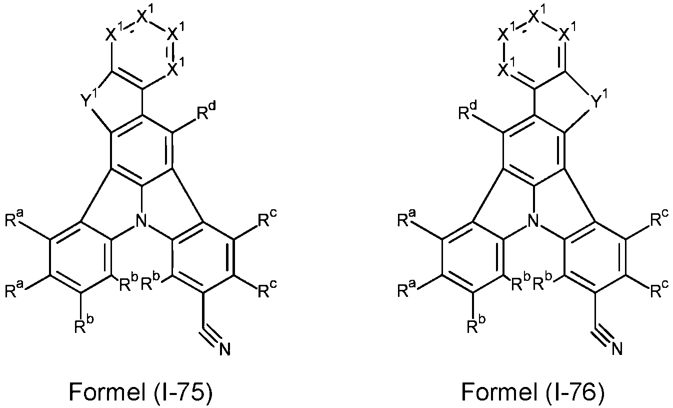 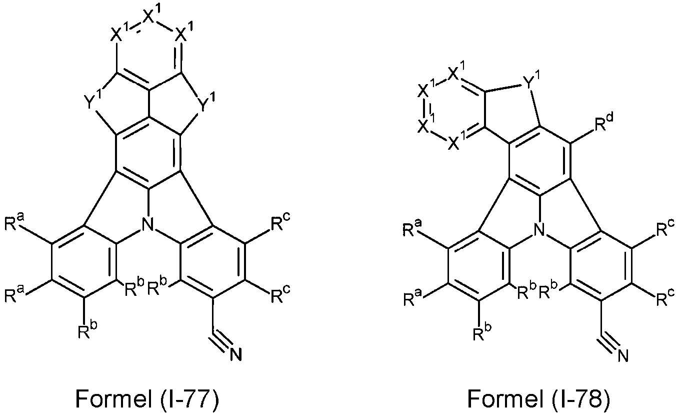 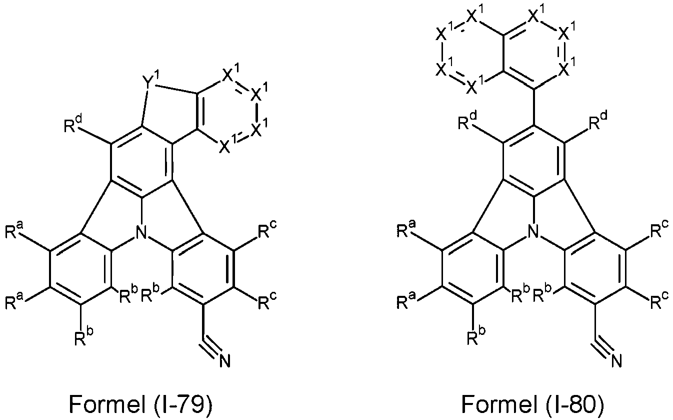 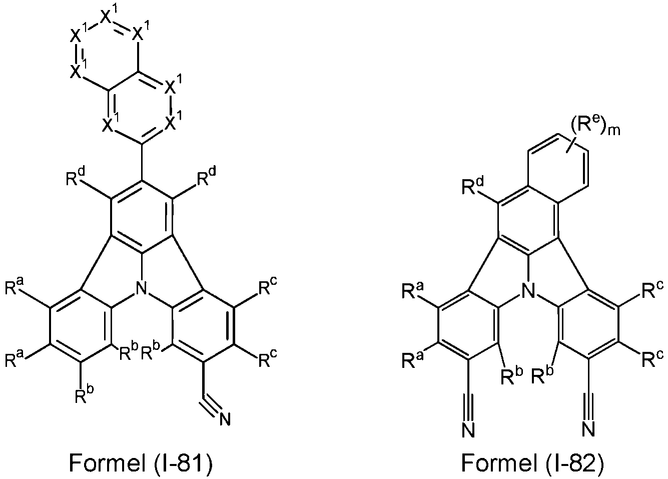 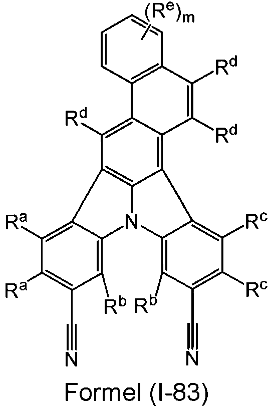 wobei die Symbole R^{a}, R^{b}, R^{c}, R^{d}, R^{e} und R^{y} die in Aspekt 1 genannten Bedeutungen aufweisen und die weiteren für die verwendeten Symbole und Indices gilt:
   - X¹: steht bei jedem Auftreten gleich oder verschieden für N oder CR^{e}, vorzugsweise für CR^{e} mit der Maßgabe, dass nicht mehr als zwei der Gruppen X¹ in einem Cyclus für N stehen;
   - Y¹: ist bei jedem Auftreten gleich oder verschieden C(R^{e})₂, (R^{e})₂C-C(R^{e})₂, (R^{e})C=C(R^{e}), NR^{e}, NAr', O, S, SO, SO₂, Se, P(O)R^{e}, BR^{e} oder Si(R^{e})₂, vorzugsweise C(R^{e})₂, (R^{e})₂C-C(R^{e})₂, (R^{e})C=C(R^{e}), O oder S, besonders bevorzugt C(R^{e})₂;
   - T¹: steht bei jedem Auftreten gleich oder verschieden für einen kondensierten Ring, vorzugsweise für einen aliphatischen oder heteroaliphatischen Ring mit 3 bis 20, vorzugsweise 5 bis 18 Ringatomen oder für einen aromatischen oder heteroaromatischen Ring mit 5 bis 13 Ringatomen, besonders bevorzugt für einen aliphatischen oder heteroaliphatischen Ring mit 3 bis 20, vorzugsweise 5 bis 18 Ringatomen, der mit einem oder mehreren Resten R¹ substituiert sein kann, wobei R¹ die in Aspekt 1 genannte Bedeutung aufweist;
   - n: ist 0, 1, 2 oder 3, vorzugsweise 0, 1 oder 2;
   - m: ist 0, 1, 2, 3 oder 4, vorzugsweise 0, 1 oder 2.
8. Verbindung nach einem oder mehreren der Aspekte 1 bis 7, dadurch gekennzeichnet, dass mindestens zwei Reste R, R^{a}, R^{b}, R^{c}, R^{d}, R^{e}, R^{y} mit den weiteren Gruppen, an die die zwei Reste R, R^{a}, R^{b}, R^{c}, R^{d}, R^{e}, R^{y} binden, einen kondensierten Ring bilden, wobei die zwei Reste R, R^{a}, R^{b}, R^{c}, R^{d}, R^{e}, R^{y} mindestens eine Struktur der folgenden Formeln (Cy-1) bis (Cy-10) formen, 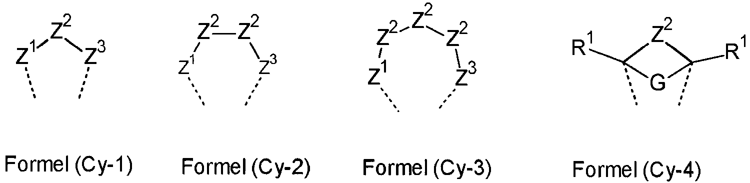 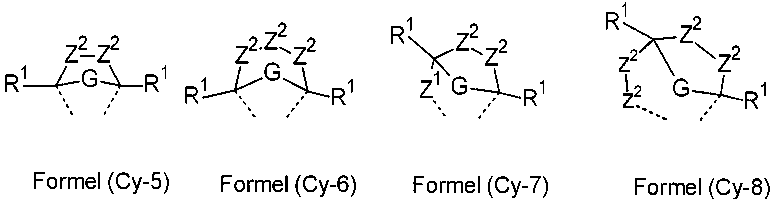 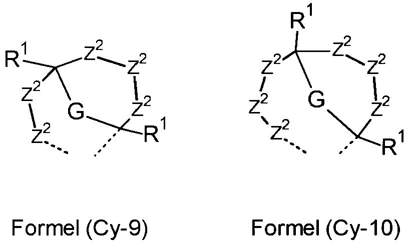 wobei R¹ und R² die in Aspekt 1 genannten Bedeutungen aufweisen, die gestrichelten Bindungen die Anbindungsstellen an die Atome der Gruppen, an die die zwei Reste R, R^{a}, R^{b}, R^{c}, R^{d}, R^{e}, R^{y} binden, darstellen und weiterhin gilt:
   - Z¹, Z³: ist gleich oder verschieden bei jedem Auftreten C(R³)₂, O, S, NR³ oder C(=O);
   - Z²: ist C(R¹)₂, O, S, NR¹ oder C(=O), wobei zwei benachbarte Gruppen Z² für -CR¹=CR¹- oder eine ortho-verknüpfte Arylen- oder Heteroarylengruppe mit 5 bis 14 aromatischen Ringatomen, welche durch einen oder mehrere Reste R¹ substituiert sein kann, stehen können;
   - G: ist eine Alkylengruppe mit 1, 2 oder 3 C-Atomen, welche mit einem oder mehreren Resten R¹ substituiert sein kann, -CR¹=CR¹- oder eine ortho-verknüpfte Arylen- oder Heteroarylengruppe mit 5 bis 14 aromatischen Ringatomen, welche durch einen oder mehrere Reste R¹ substituiert sein kann;
   - R³: ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch -R²C=CR²-, -C=C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem Rest R, R^{a}, R^{c}, R^{d}, R^{e} oder R¹ ein Ringsystem bilden;
   mit der Maßgabe, dass in diesen Gruppen nicht zwei Heteroatome direkt aneinander gebunden sind und nicht zwei Gruppen C=O direkt aneinander gebunden sind.
9. Verbindung nach einem oder mehreren der Aspekte 1 bis 8, dadurch gekennzeichnet, dass mindestens zwei Reste R, R^{a}, R^{b}, R^{c}, R^{d}, R^{e}, R^{y} mit den weiteren Gruppen, an die die zwei Reste R, R^{a}, R^{b}, R^{c}, R^{d}, R^{e}, R^{y} binden, einen kondensierten Ring bilden, wobei die zwei Reste R, R^{a}, R^{b}, R^{c}, R^{d}, R^{e}, R^{y} mindestens eine Struktur der Formeln (RA-1) bis (RA-13) formen 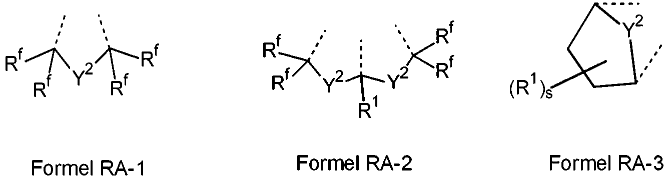 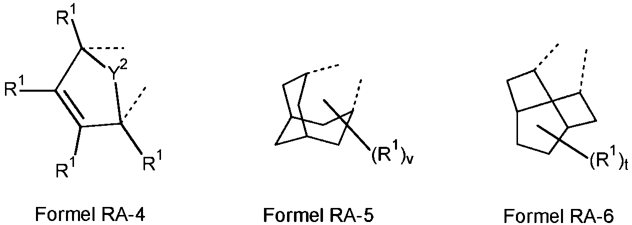 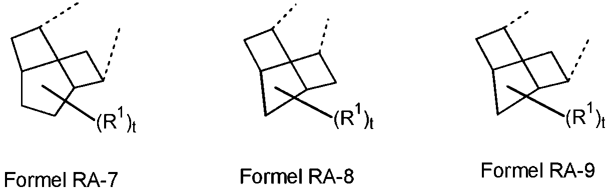 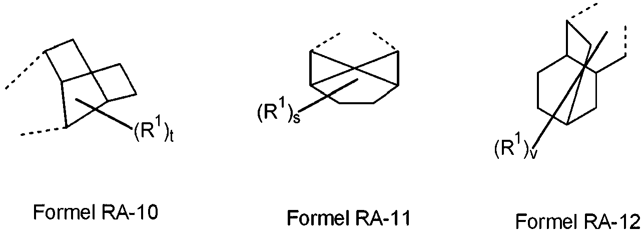 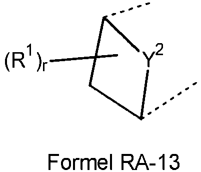 wobei R¹ die zuvor dargelegte Bedeutung hat, die gestrichelten Bindungen die Anbindungsstellen an die Atome der Gruppen, an die die zwei Reste R, R^{a}, R^{b}, R^{c}, R^{d}, R^{e}, R^{y} binden, darstellen, und die weiteren Symbole die folgende Bedeutung aufweisen:
   - Y²: ist bei jedem Auftreten gleich oder verschieden C(R¹)₂, (R¹)₂C-C(R¹)₂, (R¹)C=C(R¹), NR¹, NAr', O oder S, vorzugsweise C(R¹)₂, (R¹)₂C-C(R¹)₂, (R¹)C=C(R¹), O oder S;
   - R^{f}: ist bei jedem Auftreten gleich oder verschieden F, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C=C, Si(R²)₂, C=O, C=S, C=Se, C=NR², -C(=O)O-, -C(=O)NR²-, NR², P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann; dabei können zwei Reste R^{f} auch miteinander oder ein Rest R^{f} mit einem Rest R¹ oder mit einer weiteren Gruppe ein Ringsystem bilden;
   - r: ist 0, 1, 2, 3 oder 4, vorzugsweise 0, 1, oder 2, besonders bevorzugt 0 oder 1;
   - s: ist 0, 1, 2, 3, 4, 5 oder 6, vorzugsweise 0, 1, 2, 3, oder 4, besonders bevorzugt 0, 1 oder 2;
   - t: ist 0, 1, 2, 3, 4, 5, 6, 7 oder 8, vorzugsweise 0, 1, 2, 3, oder 4, besonders bevorzugt 0, 1 oder 2;
   - v: ist 0, 1, 2, 3, 4, 5, 6, 7, 8 oder 9, vorzugsweise 0, 1, 2, 3, oder 4, besonders bevorzugt 0, 1 oder 2,
   wobei Strukturen der Formeln RA-1, RA-3, RA-4 und RA-5 bevorzugt und Strukturen der Formeln RA-4 und RA-5 besonders bevorzugt sind.
10. Verbindung nach einem oder mehreren der Aspekte 1 bis 9, dass mindestens zwei Reste R, R^{a}, R^{b}, R^{c}, R^{d}, R^{e}, R^{y} mit den weiteren Gruppen, an die die zwei Reste R, R^{a}, R^{b}, R^{c}, R^{d}, R^{e}, R^{y} binden, einen kondensierten Ring bilden, wobei die zwei Reste R, R^{a}, R^{b}, R^{c}, R^{d}, R^{e}, R^{y} der Strukturen der Formel (RB) formen, 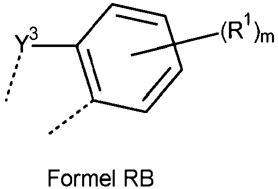 wobei R¹ die in Aspekt 1 dargelegte Bedeutung aufweist, die gestrichelten Bindungen die Anbindungsstellen darstellen, über die die zwei Reste R, R^{a}, R^{b}, R^{c}, R^{d}, R^{e}, R^{y} binden, der Index m 0, 1, 2, 3 oder 4, vorzugsweise 0, 1 oder 2 ist, und Y³ C(R¹)₂, NR¹, NAr', BR¹, BAr', O oder S ist, vorzugsweise C(R¹)₂, NAr' oder O.
11. Verbindung nach einem oder mehreren der Aspekte 1 bis 10, dadurch gekennzeichnet, dass R oder Ar gleich oder verschieden bei jedem Auftreten ausgewählt ist aus Phenyl, Biphenyl, Terphenyl, Quaterphenyl, Fluoren, Spirobifluoren, Naphthalin, Indol, Benzofuran, Benzothiophen, Carbazol, Dibenzofuran, Dibenzothiophen, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R^{e} substituiert sein können.
12. Verbindung gemäß mindestens einem der vorhergehenden Aspekte , dadurch gekennzeichnet, dass Verbindung in Bezug auf die Reste R^{a} und R^{c} symmetrisch ist.
13. Verbindung gemäß mindestens einem der vorhergehenden Aspekte , dadurch gekennzeichnet, dass der Rest R^{e} und/oder R^{d} mindestens eine Gruppe ausgewählt aus C(Ar')₃, C(R¹)₃, Si(Ar')₃, Si(R¹)₃, B(R¹)₂, vorzugsweise ausgewählt aus C(Ar')₃, C(R¹)₃, Si(Ar')₃, Si(R¹)₃ umfasst, vorzugsweise eine Fluorengruppe, die mit einem oder mehreren Resten R¹ substituiert sein kann, darstellt, umfasst oder mit einem Rest R^{d} beziehungsweise R^{e} bildet.
14. Verbindung gemäß mindestens einem der vorhergehenden Aspekte , dadurch gekennzeichnet, dass die Verbindung genau zwei oder genau drei Strukturen gemäß Formel (I) und/oder (I-1) bis (I-81) umfasst.
15. Oligomer, Polymer oder Dendrimer enthaltend eine oder mehrere Verbindungen nach einem der Aspekte 1 bis 14, wobei statt eines Wasserstoffatoms oder eines Substituenten eine oder mehrere Bindungen der Verbindungen zum Polymer, Oligomer oder Dendrimer vorhanden sind.
16. Formulierung, enthaltend mindestens eine Verbindung nach einem oder mehreren der Aspekte 1 bis 14 oder ein Oligomer, Polymer oder Dendrimer nach Aspekt 15 und mindestens eine weitere Verbindung, wobei die weitere Verbindung bevorzugt ausgewählt ist aus einem oder mehreren Lösemitteln.
17. Zusammensetzung, enthaltend mindestens eine Verbindung nach einem oder mehreren der Aspekte 1 bis 14 oder ein Oligomer, Polymer oder Dendrimer nach Anspruch 15 und mindestens eine weitere Verbindung ausgewählt aus der Gruppe bestehend aus fluoreszierenden Emittern, phosphoreszierenden Emittern, Emittern, die TADF zeigen, Hostmaterialien, Elektronentransportmaterialien, Elektroneninjektionsmaterialien, Lochleitermaterialien, Lochinjektionsmaterialien, Elektronenblockiermaterialien und Lochblockiermaterialien, vorzugsweise Hostmaterialien.
18. Zusammensetzung nach Aspekt 17, dadurch gekennzeichnet, dass die Zusammensetzung mindestens eine weitere Verbindung ein TADF-Hostmaterial darstellt und/oder mindestens eine weitere Verbindung ein phosphoreszierender Emitter (Triplettemitter) darstellt, wobei die weiteren Verbindungen mit einer Verbindung nach einem oder mehreren der Aspekte 1 bis 14 oder einem Oligomer, Polymer oder Dendrimer nach Aspekt 15 vorzugsweise ein Hyperfluoreszenz- und/oder Hyperphosphoreszenz-System bilden.
19. Verfahren zur Herstellung einer Verbindung nach einem oder mehreren der Aspekte 1 bis 14, dadurch gekennzeichnet, dass ein Grundgerüst mit einer aromatischen Aminogruppe synthetisiert wird und mindestens ein aromatischer oder heteroaromatischer Rest eingeführt wird, vorzugsweise mittels einer nukleophilen aromatischen Substitutionsreaktion oder einer Kupplungsreaktion.
20. Verwendung einer Verbindung nach einem oder mehreren der Aspekte 1 bis 14 oder ein Oligomer, Polymer oder Dendrimer nach Anspruch 15 in einer elektronischen Vorrichtung, vorzugsweise als blauer Emitter.
21. Elektronische Vorrichtung enthaltend mindestens eine Verbindung nach einem oder mehreren der Aspekte 1 bis 14 oder ein Oligomer, Polymer oder Dendrimer nach Aspekt 15, wobei die Verbindung nach einem oder mehreren der Aspekte 1 bis 14 oder das Oligomer, Polymer oder Dendrimer nach Aspekt 15 vorzugsweise als blauer Emitter in einer emittierenden Schicht enthalten ist.

## Patentansprüche

1. Verbindung umfassend mindestens eine Struktur der Formel (I), vorzugsweise Verbindung gemäß der Formel (I), 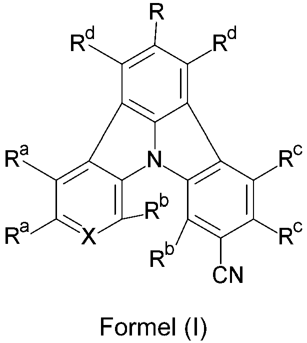 wobei für die verwendeten Symbole und Indizes gilt:
X steht bei jedem Auftreten gleich oder verschieden für N, C-CN, C-Y-R^{y} oder CR^{b}, vorzugsweise für N, C-CN, oder C-Y-R^{y}, besonders bevorzugt für C-CN;
Y ist bei jedem Auftreten gleich oder verschieden CO, P(=O)R^{a}, SO, SO₂, C(O)O, C(S)O, C(O)S, C(=O)NR^{a}, C(=O)NAr, vorzugsweise CO, P(=O)R^{a}, SO, SO₂, besonders bevorzugt CO;
R ist bei jedem Auftreten gleich oder verschieden H, D, OH, F, Cl, Br, I, CN, NO₂, N(Ar)₂, N(R^{e})₂, C(=O)N(Ar)₂, C(=O)N(R^{e})₂, C(Ar)₃, C(R^{e})₃, Si(Ar)₃, Si(R^{e})₃, B(Ar)₂, B(R^{e})₂, C(=O)Ar, C(=O)R^{e}, P(=O)(Ar)₂, P(=O)(R^{e})₂, P(Ar)₂, P(R^{e})₂, S(=O)Ar, S(=O)R^{e}, S(=O)₂Ar, S(=O)₂R^{e}, OSO₂Ar, OSO₂R^{e}, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R^{e} substituiert sein kann, wobei eine oder mehrere nicht benachbarte CH₂-Gruppen durch R^{e}C=CR^{e}, C=C, Si(R^{e})₂, C=O, C=S, C=Se, C=NR^{e}, -C(=O)O-, -C(=O)NR^{e}-, NR^{e}, P(=O)( R^{e}), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R^{e} substituiert sein kann, oder eine Aryloxy- oder Heteroaryloxygruppe mit 5 bis 60 aromatischen Ringatomen, die durch einen oder mehrere Reste R^{e} substituiert sein kann, oder eine Arylthio- oder Heteroarylthiogruppe mit 5 bis 60 aromatischen Ringatomen, die durch einen oder mehrere Reste R^{e} substituiert sein kann, oder eine Diarylamino-, Arylheteroarylamino-, Diheteroarylaminogruppe mit 5 bis 60 aromatischen Ringatomen, die durch einen oder mehrere Reste R^{e} substituiert sein kann, oder eine Arylalkyl- oder Heteroarylalkylgruppe mit 5 bis 60 aromatischen Ringatomen und 1 bis 10 C-Atomen im Alkylrest, die durch einen oder mehrere Reste R^{e} substituiert sein kann; dabei kann ein Rest R mit einer weiteren Gruppe, vorzugsweise R^{d} ein Ringsystem bilden;
Ar ist bei jedem Auftreten gleich oder verschieden ein aromatisches oder heteroaromatisches Ringsystem mit 5 bis 60 aromatischen Ringatomen, das mit einem oder mehreren Resten R^{e} substituiert sein kann, dabei können zwei Reste Ar, welche an dasselbe C-Atom, Si-Atom, N-Atom, P-Atom oder B-Atom binden, auch durch eine Einfachbindung oder eine Brücke, ausgewählt aus B(R^{e}), C(R^{e})₂, Si(R^{e})₂, C=O, C=NR^{e}, C=C(R^{e})₂, O, S, S=O, SO₂, N(R^{e}), P(R^{e}) und P(=O)R^{e}, miteinander verbrückt sein;
R^{a}, R^{c}, R^{d}, R^{e} ist bei jedem Auftreten gleich oder verschieden H, D, OH, F, Cl, Br, I, CN, NO₂, N(Ar')₂, N(R¹)₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; dabei können zwei Reste R^{a}, R^{c}, R^{d}, R^{e} auch miteinander oder einer weiteren Gruppe, vorzugsweise R oder R^{y} ein Ringsystem bilden;
R^{b} ist bei jedem Auftreten gleich oder verschieden H, D, OH, F, Cl, Br, I, CN, NO₂, C(=O)N(Ar')₂, C(=O)N(R¹)₂, C(Ar')₃, C(R¹)₃, Si(Ar')₃, Si(R¹)₃, B(Ar')₂, B(R¹)₂, C(=O)Ar', C(=O)R¹, P(=O)(Ar')₂, P(=O)(R¹)₂, P(Ar')₂, P(R¹)₂, S(=O)Ar', S(=O)R¹, S(=O)₂Ar', S(=O)₂R¹, OSO₂Ar', OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann; dabei können zwei Reste R^{b}, auch miteinander oder einer weiteren Gruppe, vorzugsweise R oder R^{y} ein Ringsystem bilden;
R^{y} ist bei jedem Auftreten gleich oder verschieden C(Ar')₃, C(R¹)₃, Si(Ar')₃, Si(R¹)₃, N(Ar')₂, N(R¹)₂, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können, wobei eine mit dem Rest Y verbundene CH₂-Gruppe nicht durch C=O, C=S, C=Se, C=NR¹, -C(=O)O-, -C(=O)NR¹-, P(=O)(R¹), SO oder SO₂ ersetzt sein kann, wobei eine mit dem Rest Y verbundene CH₂-Gruppe besonders bevorzugt nicht durch Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein kann, oder ein aromatisches oder heteroaromatisches Ringsystem mit 5 bis 60 aromatischen Ringatomen, das jeweils durch einen oder mehrere Reste R¹ substituiert sein kann; dabei können zwei Reste R^{y} auch miteinander oder ein Rest R^{y} mit einem Rest R^{a}, R^{b}, R^{c}, R^{d}, R^{e} ein Ringsystem bilden, vorzugsweise einem Rest R^{a};
Ar' ist bei jedem Auftreten gleich oder verschieden ein aromatisches oder heteroaromatisches Ringsystem mit 5 bis 60 aromatischen Ringatomen, das mit einem oder mehreren Resten R¹ substituiert sein kann, dabei können zwei Reste Ar', welche an dasselbe C-Atom, Si-Atom, N-Atom, P-Atom oder B-Atom binden, auch durch eine Einfachbindung oder eine Brücke, ausgewählt aus B(R¹), C(R¹)₂, Si(R¹)₂, C=O, C=NR¹, C=C(R¹)₂, O, S, S=O, SO₂, N(R¹), P(R¹) und P(=O)R¹, miteinander verbrückt sein;
R¹ ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C- Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch -R²C=CR²-, -C=C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei oder mehrere, vorzugsweise benachbarte Reste R¹ miteinander ein Ringsystem bilden, dabei können einer oder mehrere Reste R¹ mit einem weiteren Teil der Verbindung ein Ringsystem bilden;
-Ar" ist bei jedem Auftreten gleich oder verschieden ein aromatisches oder heteroaromatisches Ringsystem mit 5 bis 30 aromatischen Ringatomen, das mit einem oder mehreren Resten R² substituiert sein kann, dabei können zwei Reste Ar", welche an dasselbe C-Atom, Si-Atom, N-Atom, P-Atom oder B-Atom binden, auch durch eine Einfachbindung oder eine Brücke, ausgewählt aus B(R²), C(R²)₂, Si(R²)₂, C=O, C=NR², C=C(R²)₂, O, S, S=O, SO₂, N(R²), P(R²) und P(=O)R², miteinander verbrückt sein;
R² ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann, dabei können zwei oder mehrere, vorzugsweise benachbarte Substituenten R² miteinander ein Ringsystem bilden.

2. Verbindung nach Anspruch 1, **dadurch gekennzeichnet, dass** mindestens ein vorzugsweise mindestens zwei der Reste R, R^{a}, R^{b}, R^{c}, R^{d}, R^{e} ungleich H sind, vorzugsweise ungleich H, D, OH, NO₂, F, Cl, Br, I.

3. Verbindung nach Anspruch 1 oder 2, **dadurch gekennzeichnet, dass** mindestens ein vorzugsweise mindestens zwei der Reste R^{a}, R^{c} eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R¹ substituiert sein kann, darstellen, wobei eine oder mehrere nicht benachbarte CH₂-Gruppen durch R¹C=CR¹, C=C, Si(R¹)₂, C=O, C=S, C=Se, C=NR¹, -C(=O)O-, -C(=O)NR¹-, NR¹, P(=O)(R¹), -O-, -S-, SO oder SO₂ ersetzt sein können.

4. Verbindung nach einem oder mehreren der Ansprüche 1 bis 3, **dadurch gekennzeichnet, dass** der Rest R ein aromatisches oder heteroaromatisches Ringsystem mit 5 bis 13 aromatischen Ringatomen darstellt, das mit einem oder mehreren Resten R^{e} substituiert sein kann.

5. Verbindung nach einem oder mehreren der Ansprüche 1 bis 4, **dadurch gekennzeichnet, dass** zwei Reste R^{a} mit den weiteren Gruppen, an die die zwei Reste R^{a} binden, einen kondensierten Ring bilden, vorzugsweise einen aliphatischen oder heteroaliphatischen Ring mit 3 bis 20, vorzugsweise 5 bis 18 Ringatomen oder einen aromatischen oder heteroaromatischen Ring mit 5 bis 13 Ringatomen, besonders bevorzugt einen aliphatischen oder heteroaliphatischen Ring mit 3 bis 20, vorzugsweise 5 bis 18 Ringatomen.

6. Verbindung nach einem oder mehreren der Ansprüche 1 bis 5, **dadurch gekennzeichnet, dass** zwei Reste R^{c} mit den weiteren Gruppen, an die die zwei Reste R^{c} binden, einen kondensierten Ring bilden, vorzugsweise einen aliphatischen oder heteroaliphatischen Ring mit 3 bis 20, vorzugsweise 5 bis 18 Ringatomen oder einen aromatischen oder heteroaromatischen Ring mit 5 bis 13 Ringatomen, besonders bevorzugt einen aliphatischen oder heteroaliphatischen Ring mit 3 bis 20, vorzugsweise 5 bis 18 Ringatomen.

7. Verbindung nach einem oder mehreren der Ansprüche 1 bis 6, umfassend mindestens eine Struktur der Formeln (I-1) bis (I-83), vorzugsweise Verbindung gemäß einer der Formeln (I-1) bis (I-83), 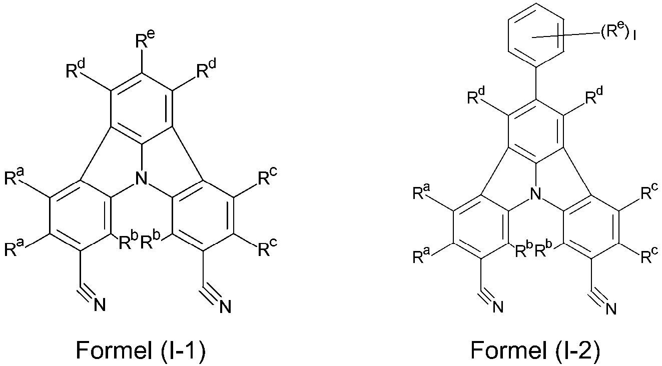 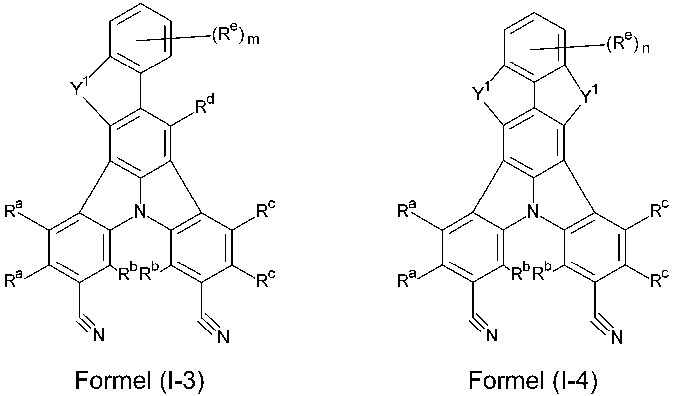 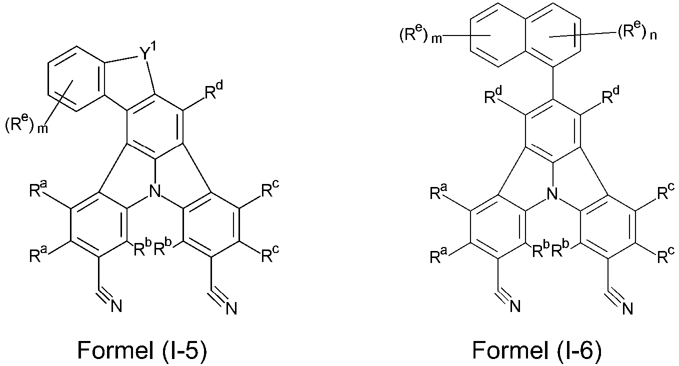 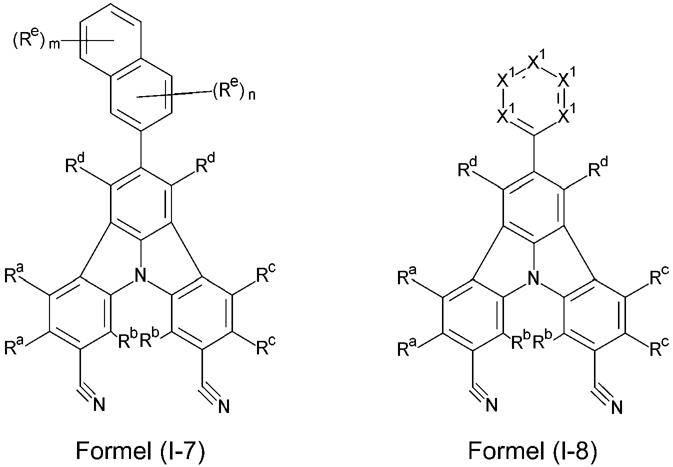 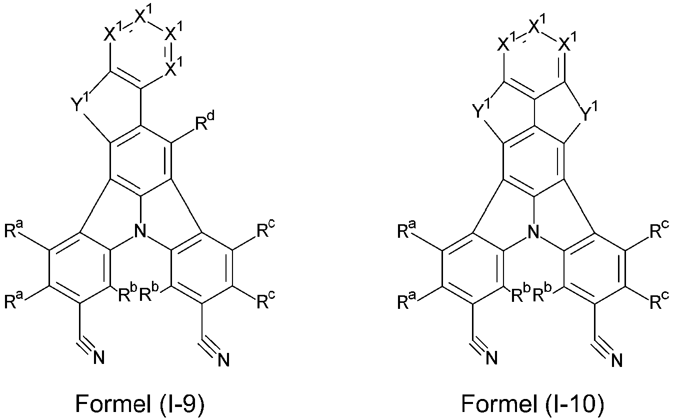 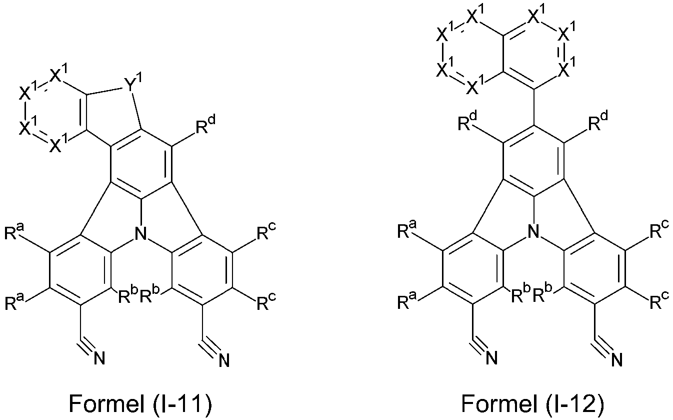 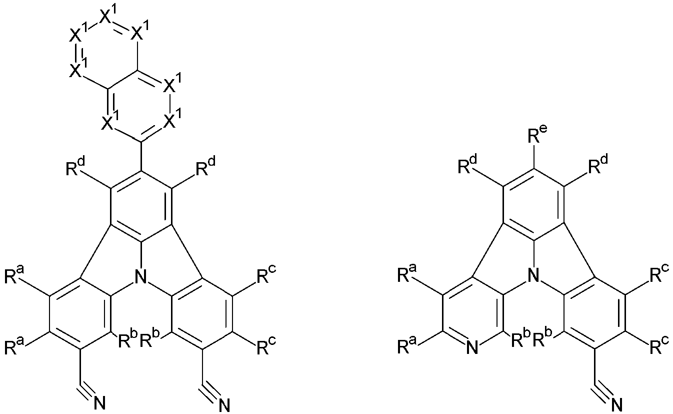 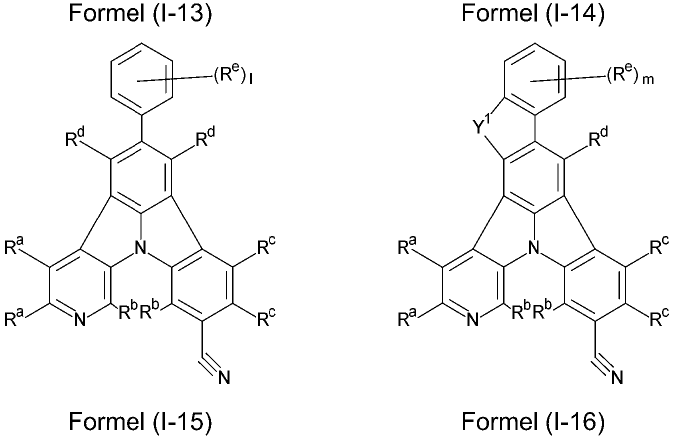 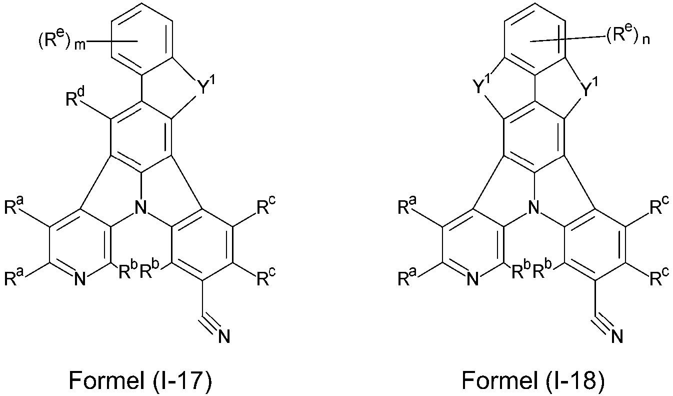 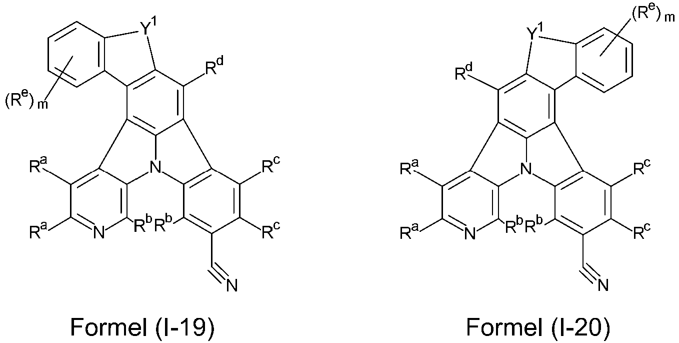 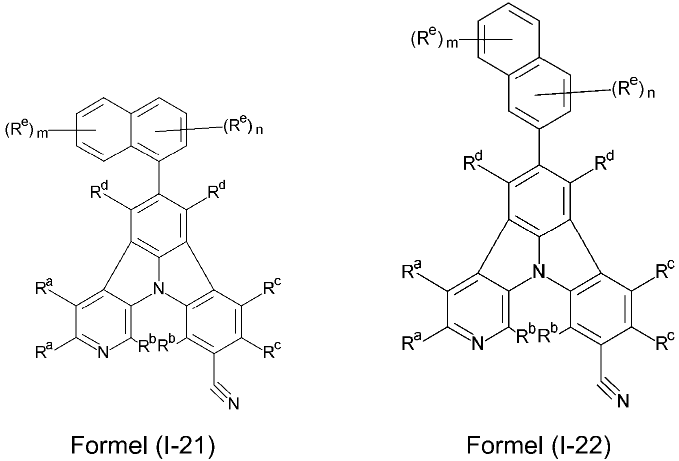 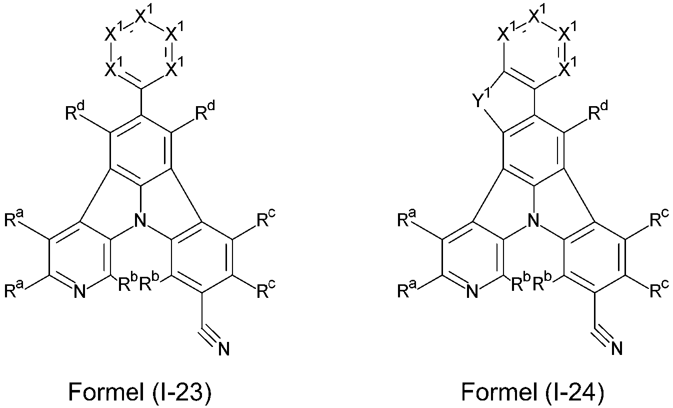 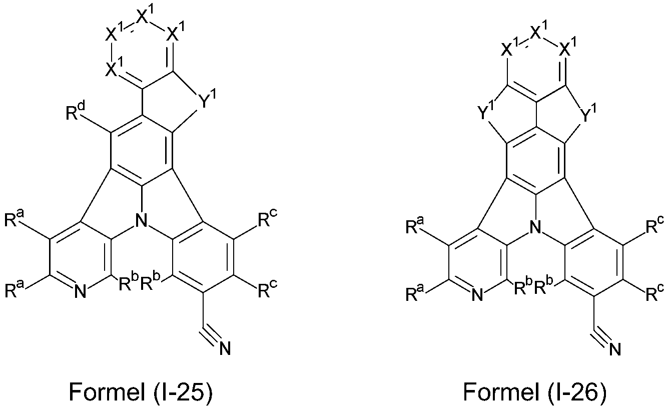 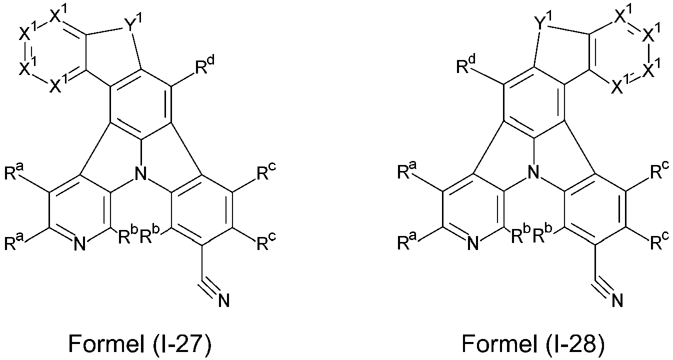 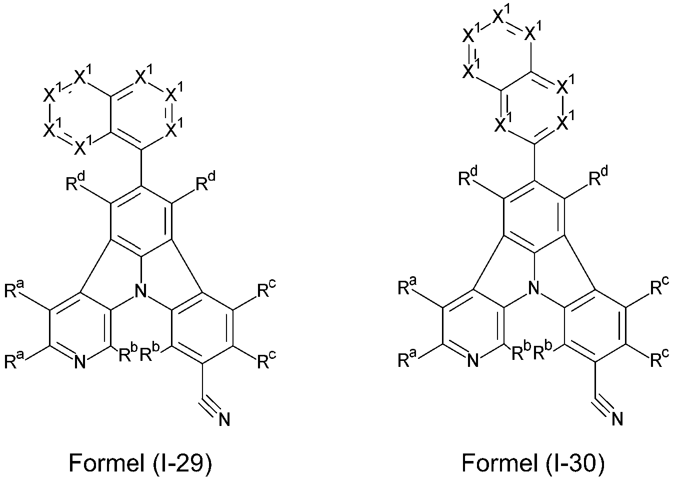 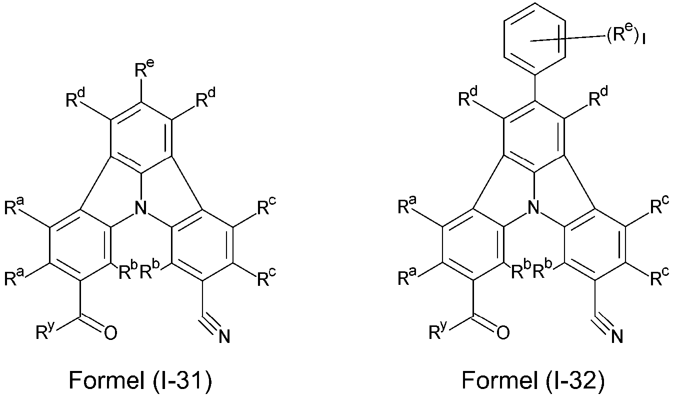 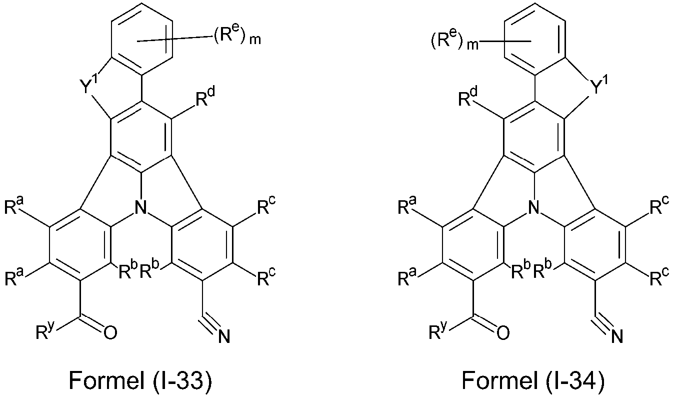 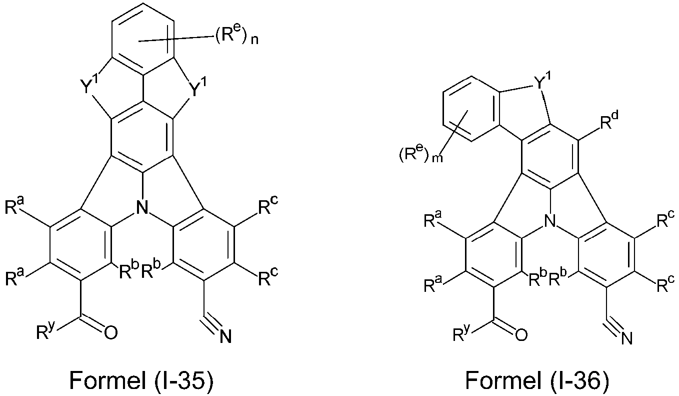 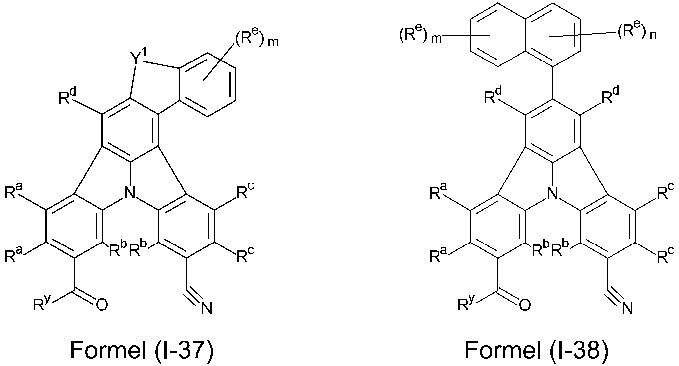 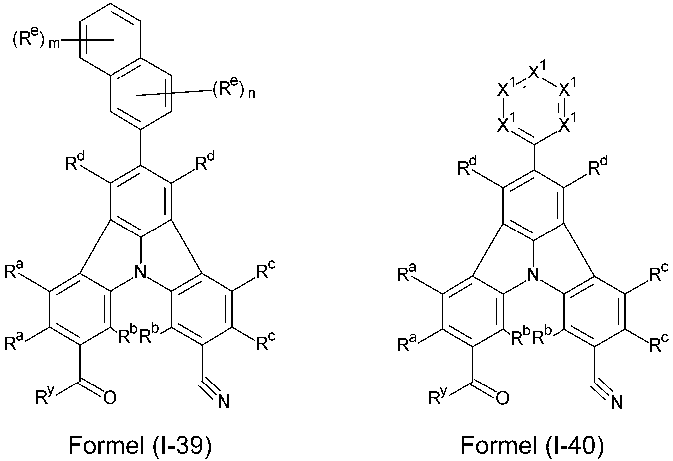 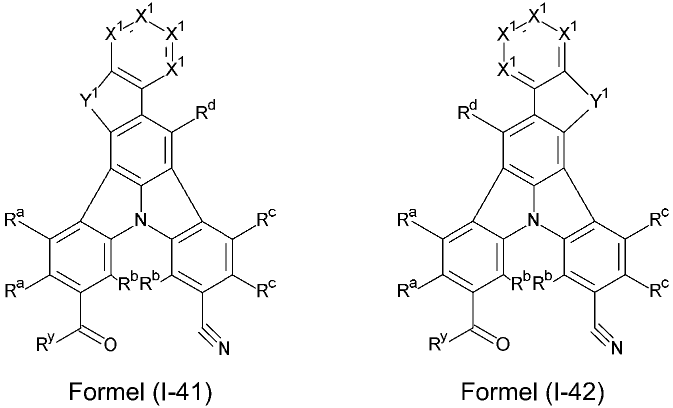 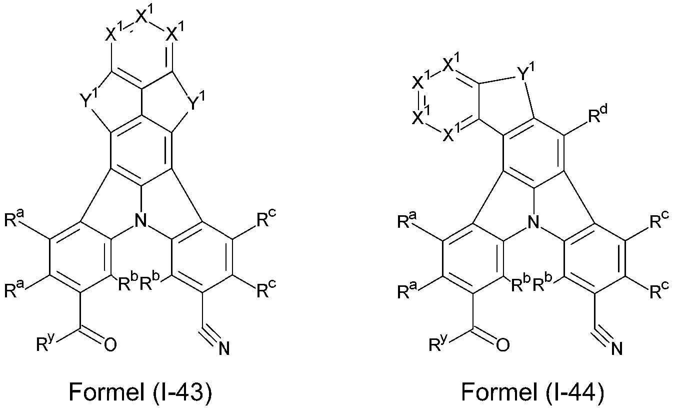 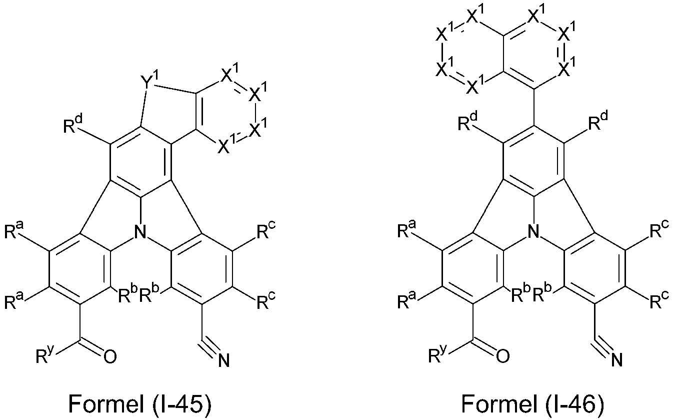 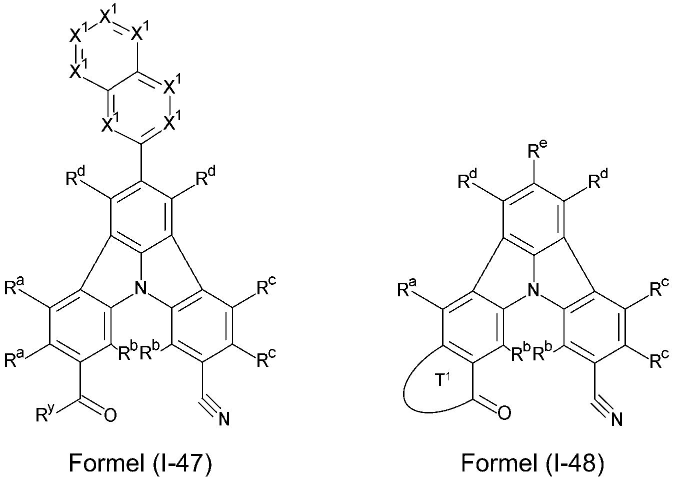 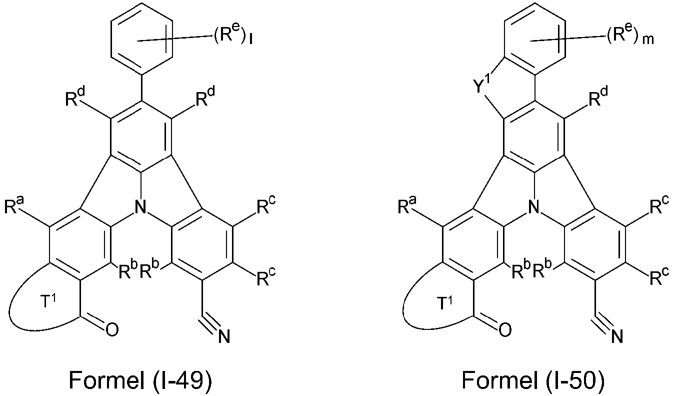 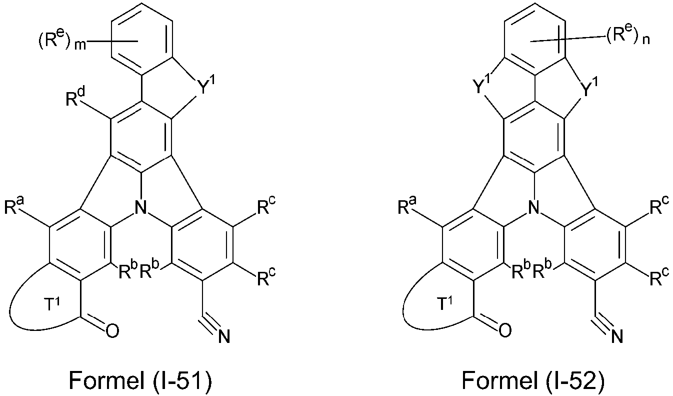 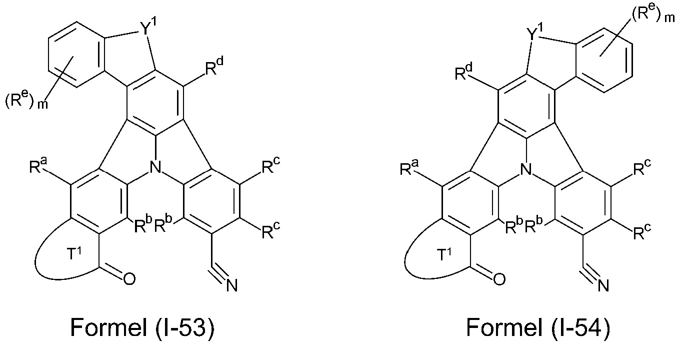 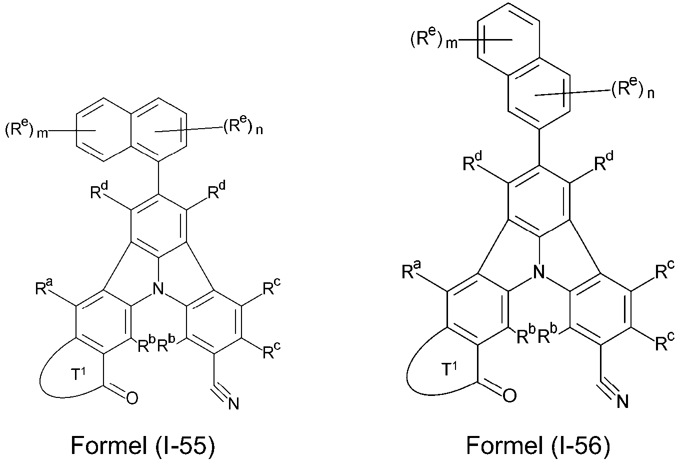 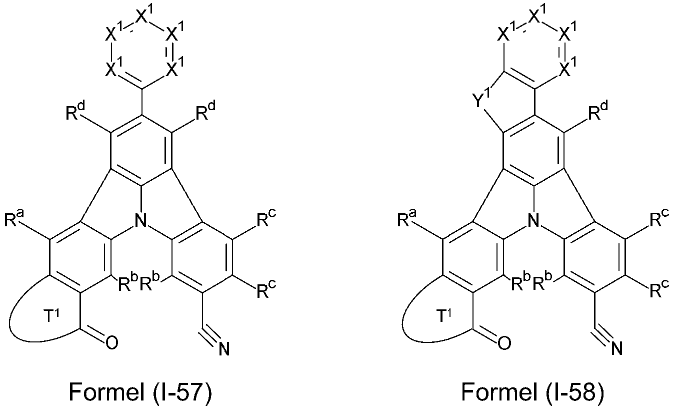 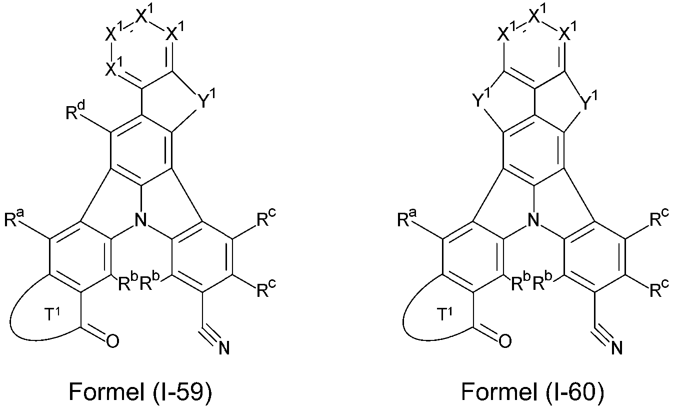 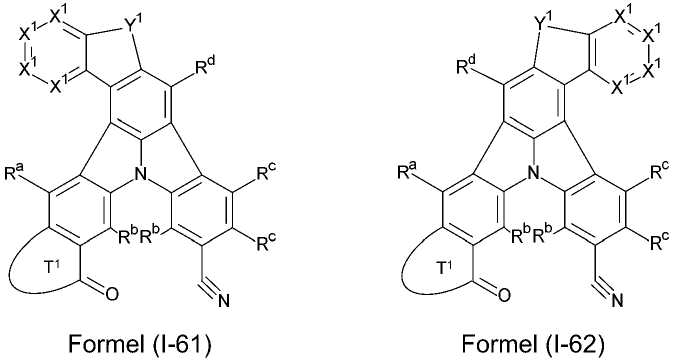 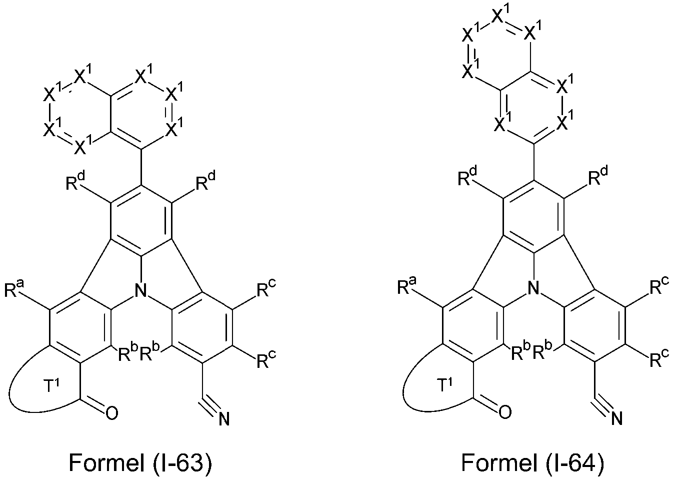 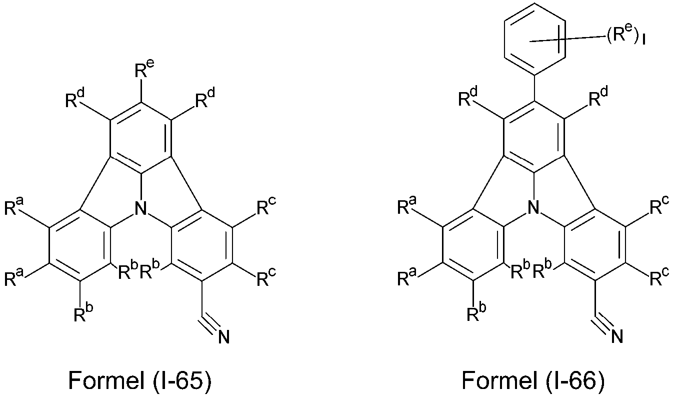 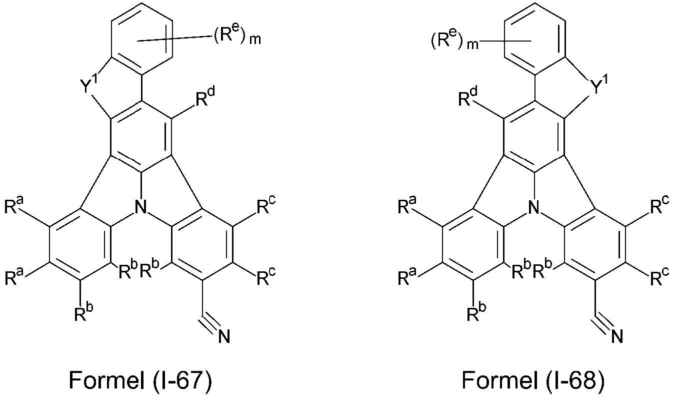 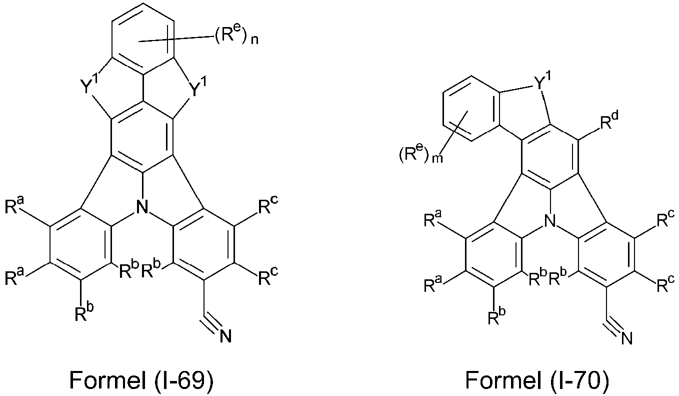 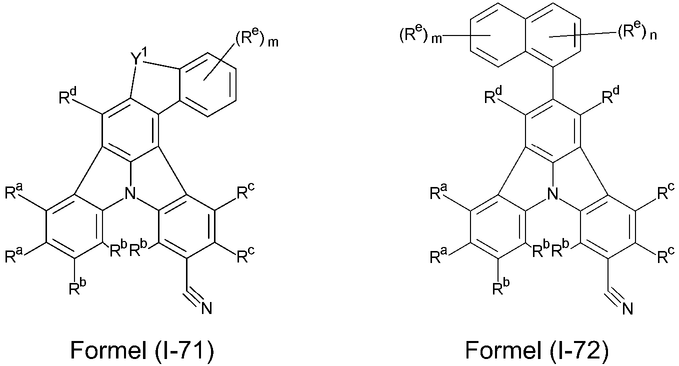 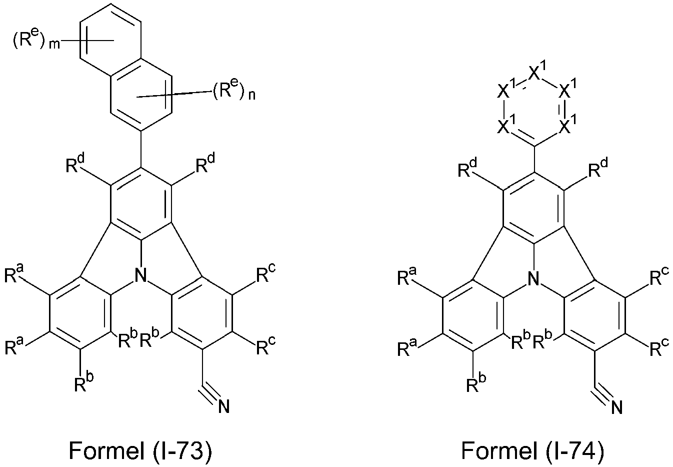 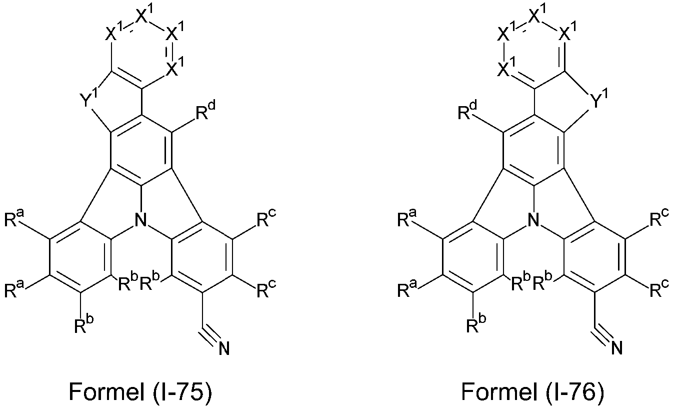 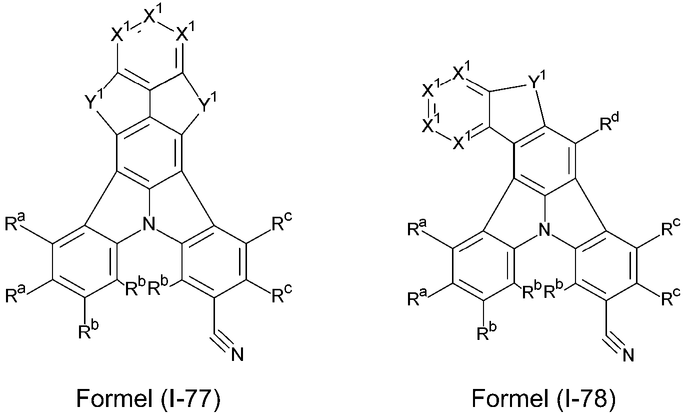 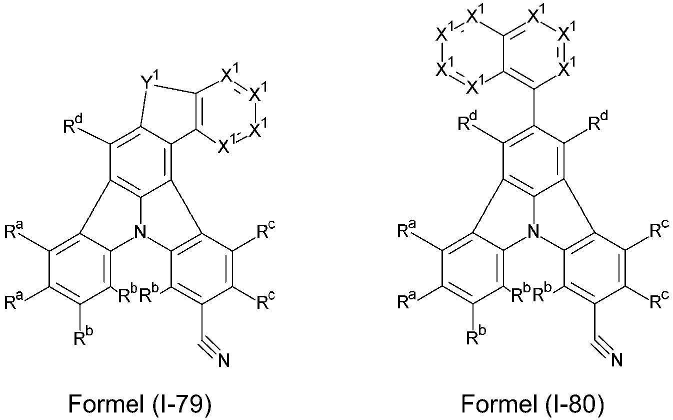 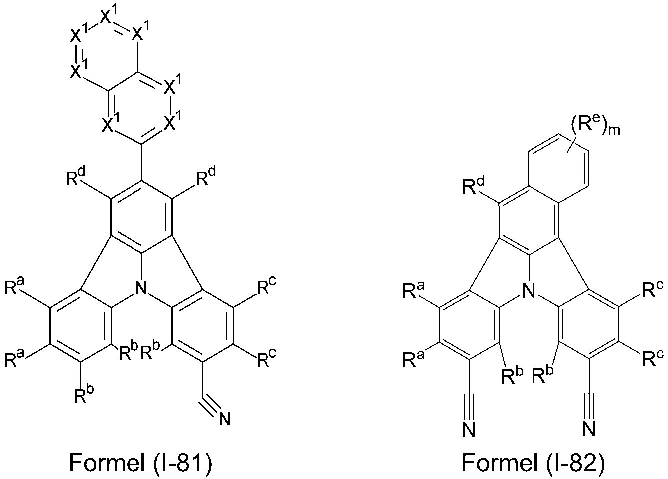 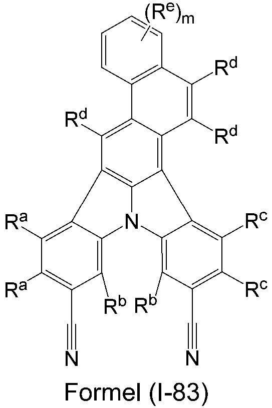 wobei die Symbole R^{a}, R^{b}, R^{c}, R^{d}, R^{e} und R^{y} die in Anspruch 1 genannten Bedeutungen aufweisen und die weiteren für die verwendeten Symbole und Indices gilt:
X¹ steht bei jedem Auftreten gleich oder verschieden für N oder CR^{e}, vorzugsweise für CR^{e} mit der Maßgabe, dass nicht mehr als zwei der Gruppen X¹ in einem Cyclus für N stehen;
Y¹ ist bei jedem Auftreten gleich oder verschieden C(R^{e})₂, (R^{e})₂C-C(R^{e})₂, (R^{e})C=C(R^{e}), NR^{e}, NAr', O, S, SO, SO₂, Se, P(O)R^{e}, BR^{e} oder Si(R^{e})₂, vorzugsweise C(R^{e})₂, (R^{e})₂C-C(R^{e})₂, (R^{e})C=C(R^{e}), O oder S, besonders bevorzugt C(R^{e})₂;
T¹ steht bei jedem Auftreten gleich oder verschieden für einen kondensierten Ring, vorzugsweise für einen aliphatischen oder heteroaliphatischen Ring mit 3 bis 20, vorzugsweise 5 bis 18 Ringatomen oder für einen aromatischen oder heteroaromatischen Ring mit 5 bis 13 Ringatomen, besonders bevorzugt für einen aliphatischen oder heteroaliphatischen Ring mit 3 bis 20, vorzugsweise 5 bis 18 Ringatomen, der mit einem oder mehreren Resten R¹ substituiert sein kann, wobei R¹ die in Anspruch 1 genannte Bedeutung aufweist;
n ist 0, 1, 2 oder 3, vorzugsweise 0, 1 oder 2;
m ist 0, 1, 2, 3 oder 4, vorzugsweise 0, 1 oder 2.

8. Verbindung nach einem oder mehreren der Ansprüche 1 bis 7, **dadurch gekennzeichnet, dass** mindestens zwei Reste R, R^{a}, R^{b}, R^{c}, R^{d}, R^{e}, R^{y} mit den weiteren Gruppen, an die die zwei Reste R, R^{a}, R^{b}, R^{c}, R^{d}, R^{e}, R^{y} binden, einen kondensierten Ring bilden, wobei die zwei Reste R, R^{a}, R^{b}, R^{c}, R^{d}, R^{e}, R^{y} mindestens eine Struktur der folgenden Formeln (Cy-1) bis (Cy-10) formen, 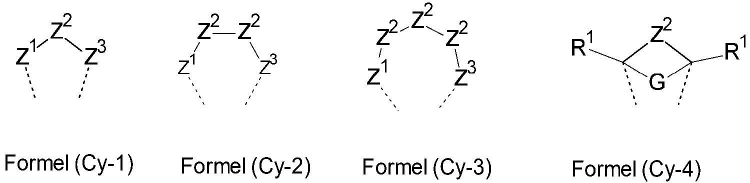 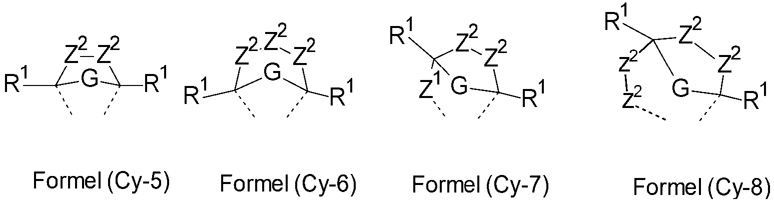 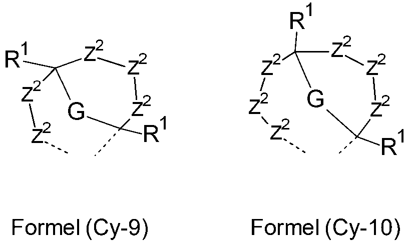 wobei R¹ und R² die in Anspruch 1 genannten Bedeutungen aufweisen, die gestrichelten Bindungen die Anbindungsstellen an die Atome der Gruppen, an die die zwei Reste R, R^{a}, R^{b}, R^{c}, R^{d}, R^{e}, R^{y} binden, darstellen und weiterhin gilt:
Z¹, Z³ ist gleich oder verschieden bei jedem Auftreten C(R³)₂, O, S, NR³ oder C(=O);
Z² ist C(R¹)₂, O, S, NR¹ oder C(=O), wobei zwei benachbarte Gruppen Z² für -CR¹=CR¹- oder eine ortho-verknüpfte Arylen- oder Heteroarylengruppe mit 5 bis 14 aromatischen Ringatomen, welche durch einen oder mehrere Reste R¹ substituiert sein kann, stehen können;
G ist eine Alkylengruppe mit 1, 2 oder 3 C-Atomen, welche mit einem oder mehreren Resten R¹ substituiert sein kann, -CR¹=CR¹- oder eine ortho-verknüpfte Arylen- oder Heteroarylengruppe mit 5 bis 14 aromatischen Ringatomen, welche durch einen oder mehrere Reste R¹ substituiert sein kann;
R³ ist bei jedem Auftreten gleich oder verschieden H, D, F, Cl, Br, I, CN, NO₂, N(Ar")₂, N(R²)₂, C(=O)Ar", C(=O)R², P(=O)(Ar")₂, P(Ar")₂, B(Ar")₂, B(R²)₂, C(Ar")₃, C(R²)₃, Si(Ar")₃, Si(R²)₃, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenylgruppe mit 2 bis 40 C-Atomen, die jeweils mit einem oder mehreren Resten R² substituiert sein kann, wobei eine oder mehrere nicht benachbarte CH₂-Gruppen durch -R²C=CR²-, -C=C-, Si(R²)₂, C=O, C=S, C=Se, C=NR², -C(=O)O-, -C(=O)NR²-, NR², P(=O)(R²), -O-, -S-, SO oder SO₂ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Aralkyl- oder Heteroaralkylgruppe mit 5 bis 60 aromatischen Ringatomen, die mit einem oder mehreren Resten R² substituiert sein kann, oder eine Kombination dieser Systeme; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem Rest R, R^{a}, R^{c}, R^{d}, R^{e} oder R¹ ein Ringsystem bilden;
mit der Maßgabe, dass in diesen Gruppen nicht zwei Heteroatome direkt aneinander gebunden sind und nicht zwei Gruppen C=O direkt aneinander gebunden sind.

9. Verbindung nach einem oder mehreren der Ansprüche 1 bis 8, **dadurch gekennzeichnet, dass** mindestens zwei Reste R, R^{a}, R^{b}, R^{c}, R^{d}, R^{e}, R^{y} mit den weiteren Gruppen, an die die zwei Reste R, R^{a}, R^{b}, R^{c}, R^{d}, R^{e}, R^{y} binden, einen kondensierten Ring bilden, wobei die zwei Reste R, R^{a}, R^{b}, R^{c}, R^{d}, R^{e}, R^{y} mindestens eine Struktur der Formeln (RA-1) bis (RA-13) formen 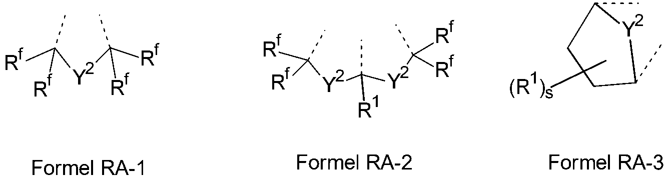 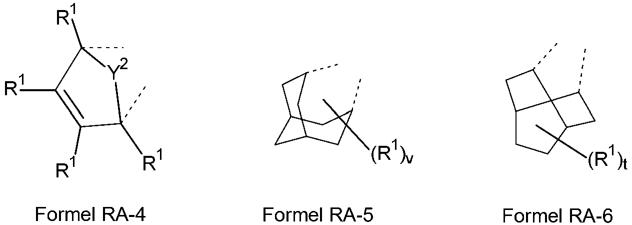 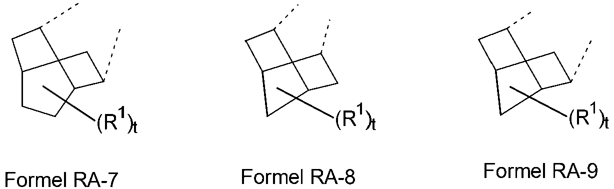 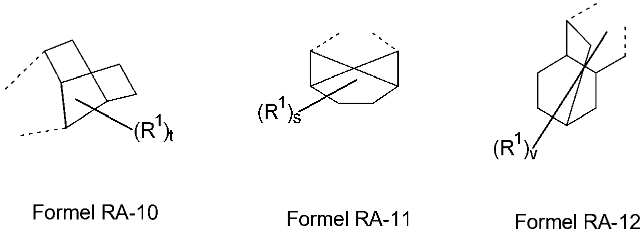 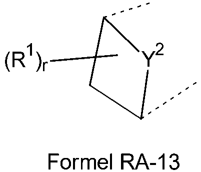 wobei R¹ die zuvor dargelegte Bedeutung hat, die gestrichelten Bindungen die Anbindungsstellen an die Atome der Gruppen, an die die zwei Reste R, R^{a}, R^{b}, R^{c}, R^{d}, R^{e}, R^{y} binden, darstellen, und die weiteren Symbole die folgende Bedeutung aufweisen:
Y² ist bei jedem Auftreten gleich oder verschieden C(R¹)₂, (R¹)₂C-C(R¹)₂, (R¹)C=C(R¹), NR¹, NAr', O oder S, vorzugsweise C(R¹)₂, (R¹)₂C-C(R¹)₂, (R¹)C=C(R¹), O oder S;
R^{f} ist bei jedem Auftreten gleich oder verschieden F, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C=C, Si(R²)₂, C=O, C=S, C=Se, C=NR², -C(=O)O-, -C(=O)NR²-, NR², P(=O)(R¹), -O-, -S-, SO oder SO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann; dabei können zwei Reste R^{f} auch miteinander oder ein Rest R^{f} mit einem Rest R¹ oder mit einer weiteren Gruppe ein Ringsystem bilden;
r ist 0, 1, 2, 3 oder 4, vorzugsweise 0, 1, oder 2, besonders bevorzugt 0 oder 1;
s ist 0, 1, 2, 3, 4, 5 oder 6, vorzugsweise 0, 1, 2, 3, oder 4, besonders bevorzugt 0, 1 oder 2;
t ist 0, 1, 2, 3, 4, 5, 6, 7 oder 8, vorzugsweise 0, 1, 2, 3, oder 4, besonders bevorzugt 0, 1 oder 2;
v ist 0, 1, 2, 3, 4, 5, 6, 7, 8 oder 9, vorzugsweise 0, 1, 2, 3, oder 4, besonders bevorzugt 0, 1 oder 2,
wobei Strukturen der Formeln RA-1, RA-3, RA-4 und RA-5 bevorzugt und Strukturen der Formeln RA-4 und RA-5 besonders bevorzugt sind.

10. Verbindung nach einem oder mehreren der Ansprüche 1 bis 9, dass mindestens zwei Reste R, R^{a}, R^{b}, R^{c}, R^{d}, R^{e}, R^{y} mit den weiteren Gruppen, an die die zwei Reste R, R^{a}, R^{b}, R^{c}, R^{d}, R^{e}, R^{y} binden, einen kondensierten Ring bilden, wobei die zwei Reste R, R^{a}, R^{b}, R^{c}, R^{d}, R^{e}, R^{y} der Strukturen der Formel (RB) formen, 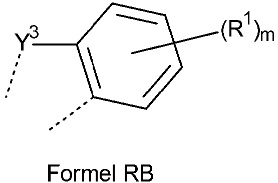 wobei R¹ die in Anspruch 1 dargelegte Bedeutung aufweist, die gestrichelten Bindungen die Anbindungsstellen darstellen, über die die zwei Reste R, R^{a}, R^{b}, R^{c}, R^{d}, R^{e}, R^{y} binden, der Index m 0, 1, 2, 3 oder 4, vorzugsweise 0, 1 oder 2 ist, und Y³ C(R¹)₂, NR¹, NAr', BR¹, BAr', O oder S ist, vorzugsweise C(R¹)₂, NAr' oder O.

11. Verbindung nach einem oder mehreren der Ansprüche 1 bis 10, **dadurch gekennzeichnet, dass** R oder Ar gleich oder verschieden bei jedem Auftreten ausgewählt ist aus Phenyl, Biphenyl, Terphenyl, Quaterphenyl, Fluoren, Spirobifluoren, Naphthalin, Indol, Benzofuran, Benzothiophen, Carbazol, Dibenzofuran, Dibenzothiophen, Indenocarbazol, Indolocarbazol, Pyridin, Pyrimidin, Pyrazin, Pyridazin, Triazin, Chinolin, Isochinolin, Chinazolin, Chinoxalin, Phenanthren oder Triphenylen, welche jeweils mit einem oder mehreren Resten R^{e} substituiert sein können; oder
**dadurch gekennzeichnet, dass** Verbindung in Bezug auf die Reste R^{a} und R^{c} symmetrisch ist; oder
**dadurch gekennzeichnet, dass** der Rest R^{e} und/oder R^{d} mindestens eine Gruppe ausgewählt aus C(Ar')₃, C(R¹)₃, Si(Ar')₃, Si(R¹)₃, B(R¹)₂, vorzugsweise ausgewählt aus C(Ar')₃, C(R¹)₃, Si(Ar')₃, Si(R¹)₃ umfasst, vorzugsweise eine Fluorengruppe, die mit einem oder mehreren Resten R¹ substituiert sein kann, darstellt, umfasst oder mit einem Rest R^{d} beziehungsweise R^{e} bildet.

12. Verbindung gemäß mindestens einem der vorhergehenden Ansprüche, **dadurch gekennzeichnet, dass** die Verbindung genau zwei oder genau drei Strukturen gemäß Formel (I) und/oder (I-1) bis (1-81) umfasst.

13. Formulierung, enthaltend mindestens eine Verbindung nach einem oder mehreren der Ansprüche 1 bis 12 oder ein Oligomer, Polymer oder Dendrimer davon und mindestens eine weitere Verbindung.

14. Organische Elektrolumineszenzvorrichtung (OLED), umfassend eine Kathode, eine Anode und mindestens eine emittierende Schicht enthaltend mindestens eine Verbindung nach einem oder mehreren der Ansprüche 1 bis 12 oder ein Oligomer, Polymer oder Dendrimer davon.

15. OLED nach Anspruch 14, **dadurch gekennzeichnet, dass** die Verbindung, ein Oligomer, Polymer oder Dendrimer davon, in einer emittierenden Schicht als Emitter eingesetzt wird; vorzugsweise umfasst die emittierende Schicht mindestens eine weitere Verbindung die ein TADF(thermally activated delayed fluorescence)-Hostmaterial darstellt und/oder mindestens eine weitere Verbindung die einen phosphoreszierenden Emitter (Triplettemitter) darstellt, wobei jede der weiteren Verbindungen mit einer Verbindung nach einem oder mehreren der Ansprüche 1 bis 12 oder einem Oligomer, Polymer oder Dendrimer davon ein Hyperfluoreszenz- und/oder Hyperphosphoreszenz-System bildet.
